(19) Europäisches Patentamt
European Patent Office
Office européen des brevets

(11) EP 1 921 157 B1

(12) EUROPEAN PATENT SPECIFICATION

(45) Date of publication and mention of the grant of the patent:
**26.05.2010 Bulletin 2010/21**

(51) Int Cl.:
*C12Q 1/68* (2006.01) *C12N 15/00* (2006.01)

(21) Application number: **07016747.3**

(22) Date of filing: **23.08.2002**

(54) **Processes for detecting toxic substances**

Verfahren zum Nachweis toxischer Substanzen

Processus de détection de substances toxiques

(84) Designated Contracting States:
**DE FR GB**

(30) Priority: **24.08.2001 JP 2001255379**

(43) Date of publication of application:
**14.05.2008 Bulletin 2008/20**

(62) Document number(s) of the earlier application(s) in accordance with Art. 76 EPC:
**02760723.3 / 1 426 439**

(73) Proprietor: **DAIKIN INDUSTRIES, LTD.**
**Osaka-shi, Osaka 530-8323 (JP)**

(72) Inventors:
- **Iwahashi, Hitoshi**
  **Tsukuba-shi**
  **Ibaraki 305-8561 (JP)**
- **Momose, Yuko**
  **Tsukuba-shi**
  **Ibaraki 305-8561 (JP)**
- **Kitagawa, Emiko**
  **Tsukuba-shi**
  **Ibaraki 305-8561 (JP)**
- **Takahashi, Junko**
  **Tsukuba-shi**
  **Ibaraki 305-0841 (JP)**

(74) Representative: **HOFFMANN EITLE**
**Patent- und Rechtsanwälte**
**Arabellastrasse 4**
**81925 München (DE)**

(56) References cited:
**WO-A-99/09202**

- **FUJITA K ET AL: "Hsp104 expression and morphological changes associated with disinfectants in saccharomyces cerevisiae: Environmental bioassay using stress response" WATER SCIENCE AND TECHNOLOGY, ELMSFORD, NY, US, vol. 38, no. 7, 1998, pages 237-243, XP002960456 ISSN: 0273-1223**
- **GASCH A P ET AL: "Genomic expression programs in the response of yeast cells to environmental changes" MOLECULAR BIOLOGY OF THE CELL, BETHESDA, MD, US, vol. 11, no. 12, December 2000 (2000-12), pages 4241-4257, XP002259051 ISSN: 1059-1524**
- **FUJITA K ET AL: "Induction of heat-shock proteins in the presence of thiuram in saccharomyces cerevisiae--for evaluating the potential toxic risks in pollutants" WATER RESEARCH, ELSEVIER SCIENCE PUBLISHERS, AMSTERDAM, NL, vol. 30, no. 9, September 1996 (1996-09), pages 2102-2106, XP004035344 ISSN: 0043-1354**
- **DATABASE EMBL [Online] 9 July 1996 (1996-07-09), "S.cerevisiae chromosome XV reading frame ORF YOR382w" XP002475245 retrieved from EBI accession no. EMBL:Z75290 Database accession no. Z75290**
- **FOURY FRANCOISE ET AL: "Mitochondrial control of iron homeostasis: A genome wide analysis of gene expression in a yeast frataxin-deficient strain" JOURNAL OF BIOLOGICAL CHEMISTRY, vol. 276, no. 11, 16 March 2001 (2001-03-16), pages 7762-7768, XP002475244 ISSN: 0021-9258**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KITAGAWA EMIKO ET AL: "Effects of the pesticide thiuram: Genome-wide screening of indicator genes by yeast DNA microarray" ENVIRONMENTAL SCIENCE AND TECHNOLOGY, vol. 36, no. 18, 15 September 2002 (2002-09-15), pages 3908-3915, XP001205322 ISSN: 0013-936X

**Description**

FILED OF THE INVENTION

This invention relates to biology-based processes for detecting toxic substances. It also relates to polynucleotides, vectors, and cells as used for the processes.

BACKGROUND ART

Environmental chemical fate search has been conducted every year for 24 years from 1974 through 1998 by Environment Agency, and revealed that about 40% of 775 chemical substances that have been searched so far are emitted into the environment. Chemical substances that are industrially produced at the present in Japan are estimated about 50,000, and the production scale and the kinds of chemical substances are increasing year by year. It is known that chemical substances that are accidentally produced by water treatment with chlorine and incineration pollute the environment. Although such facts allow us to predict that there are a large number of chemical substances that have been accumulated in the environment, it is extremely difficult to search and examine individually the all chemical substances.

Conventional bioassays (approaches to evaluate the harmful effects on biological materials on the basis of their responses) wherein inhibited growth and particular biological responses in individuals and cells of fishes, daphnia and shellfish are used as indicators make it possible to determine the presence or absence of the toxicity of chemical substances in the environment, but neither possible to evaluate the characters nor origins of the toxicity. The evaluation methods based on the activity of nitrite-forming bacteria or nitrate-forming bacteria (Japanese Patent Publication (kokai) No. 123705/1994, Japanese Patent Publication (kokai) No. 2000-206087) and the activity of iron bacteria have been proposed, and devices such as Acute toxiciants monitor (Fuji Electric Corporate Research and Development, Ltd. Japan) are marketed. In foreign countries, the devices for evaluation based on emission intensity of luminous bacteria are commercially available (MICROTOX, azur, Co., USA; LUMIS, drlange, Co. Germany). However, those devices still involve conventional bioassays, and never provide any detailed information of toxic chemical substances.

In Japan, the risk control of chemical substances is reconsidered every time a chemical substance pollution is newly found, and official regulations and self-imposed regulations are combined to organize the system for risk control. However, any system has not been yet organized that could quickly respond to the present complicated and diversified conditions including accidental productions and environmental emission of toxic chemical substance as typified by trihalomethane and dioxin. Animal experiments as used in the method for evaluation of toxic substance of "Law Concerning Examination and Manufacture, etc. of Chemical Substances" are expensive and time-consuming, and are not accepted across the world. Although, as such, the control system has been continuously discussed, it has not been successfully accomplished because there is no way to dissolve the problem. Thus, a method for detect readily chemical substances occurring in the environment is desired.

DISCLOSURE OF THE INVENTION

The inventors of the present application found that a toxic substance activates the promoter of a particular yeast gene to induce the transcription of mRNA from the polynucleotide encoding a marker protein operably linked to the promoter, and accomplished the present invention.

Specifically, the specification relates to a polynucleotide which comprises a polynucleotide sequence operably linked to a polynucleotide encoding a marker protein, wherein the polynucleotide sequence comprises a promoter from yeast genes that is selected from a group consisting of the following, or a promoter from a gene that is homologous to the yeast genes and is derived from other species; YBR072W, YCR102C, YCR107W, YDL218W, YDL243C, YDR453C, YDR533C, YFL014W, YFL056C, YFL057C, YGR110W, YJR155W, YKL071W, YKR076W, YLL060C, YLR460C, YMR090W, YNL331C, YNL332W, YNL335W, YOL150C, YOL165C, YPL171C, YPR167C, YBL048W, YBL064C, YBL107C, YBR008C, YBR173C, YBR256C, YBR296C, YDL021W, YFL022C, YFL024C, YFL061W, YGL121C, YGL158W, YGR043C, YHR029C, YHR112C, YHR139C, YHR179W, YHR209W, YIR030C, YJR010W, YJR048W, YKL001C, YKL107W, YKR075C, YKR097W, YLL056C, YLR297W, YLR303W, YML087C, YMR096W, YNL274C, YOL151W, YOR226C, YOR338W, YOR391C, YPL280W, YDR406W, YJL153C, YLR346C, YOR049C, YOR153W, YPL088W, YAL034C, YDL124W, YDL174C, YDR476C, YGL156W, YGR035C, YGR157W, YGR213C, YGR281W, YGR284C, YHL047C, YHR043C, YHR044C, YHR054C, YJR073C, YKL165C, YLR008C; YMR315W, YNL211C, YOL031C, YOL101C, YOR303W, YAL005C, YAR031W, YBL005W-A, YBL022C, YBL041W, YBL049W, YBL075C, YBL078C, YBR062C, YBR169C, YBR294W, YCL020W, YCL035C, YCL043C, YCL050C, YCL057W, YCR012W, YCR013C, YCR060W, YDL007W, YDL027C, YDL097C, YDL110C, YDL126C, YDL169C, YDR070C, YDR155C, YDR158W, YDR204W, YDR210W, YDR214W, YDR258C, YDR313C, YDR368W, YDR435C, YER012W, YER037W, YER091C, YER103W, YFL044C, YFR003C, YFR010W, YFR020W, YFR024C, YFR044C, YFR053C, YGL006W, YGL048C, YGL062W, YGL141W, YGL157W, YGL163C, YGL180W, YGL184C, YGR010W, YGR028W, YGR032W, YGR048W, YGR124W, YGR135W, YGR142W, YGR161C, YGR192C, YGR197C, YGR201C, YGR212W, YGR231C, YGR244C, YGR254W, YGR268C, YHL030W, YHR016C, YHR018C, YHR055C, YHR087W, YHR166C, YIL160C, YIR017C, YJL034W, YJL048C, YJL052W, YJL144W, YJL163C, YJR009C, YJR069C, YJR074W, YJR130C, YJR149W, YKL065C, YKL073W, YKL103C, YKL142W, YKL210W, YKL218C, YKRO11C, YKR018C, YKR046C, YKR049C, YLL024C, YLL026W, YLR027C, YLR080W, YLR107W, YLR121C, YLR132C, YLR133W, YLR155C, YLR158C, YLR161W, YLR195C, YLR217W, YLR328W, YLR336C, YLR345W, YLR370C, YLR423C, YML004C, YML092C, YML128C, YML130C, YML131W, YMR040W, YMR118C, YMR214W, YMR251W, YMR297W, YMR322C, YNL036W, YNL055C, YNL071W, YNL094W, YNL134C, YNL155W, YNL160W, YNL239W, YNL241C, YOL005C, YOR020C, YOR027W, YOR037W, YOR059C, YOR120W, YOR134W, YOR152C, YOR173W, YOR289W, YOR362C, YPL240C, YPR030W, YAL008W, YAL023C, YAL060W, YAL062W, YAR009C, YBL101C, YBR006W, YBR046C, YBR052C, YBR053C, YBR056W, YBR099C, YBR137W, YBR139W, YBR149W, YBR170C, YBR177C, YBR203W, YBR207W, YBR212W, YBR239C, YBR284W, YBR293W, YCL018W, YCL033C, YCL040W, YCL049C, YCR062W, YCR067C, YCR082W, YDL010W, YDL020C, YDL024C, YDL054C, YDL095W, YDL100C, YDL115C, YDL144C, YDL198C, YDL223C, YDL245C, YDL246C, YDR001C, YDR032C, YDR058C, YDR072C, YDR127W, YDR168W, YDR169C, YDR188W, YDR231C, YDR261C, YDR264C, YDR272W, YDR293C, YDR304C, YDR330W, YDR403W, YDR411C, YDR427W, YDR436W, YDR497C, YDR511W, YDR516C, YDR519W, YDR545W, YEL012W, YEL030W, YER004W, YER009W, YER021W, YER035W, YER053C, YER079W, YER094C, YER096W, YER125W, YER158C, YER163C, YER175C, YER177W, YER178W, YER185W, YFL006W, YFL010C, YFL016C, YFL029C, YFL030W, YFL031W, YFL032W, YFL038C, YFL041W, YFR004W, YFR047C, YFR050C, YFR052W, YGL011C, YGL013C, YGL037C, YGL047W, YGL053W, YGL091C, YGL094C, YGL127C, YGL150C, YGL199C, YGL207W, YGL248W, YGR008C, YGR037C, YGR055W, YGR101W, YGR130C, YGR154C, YGR194C, YGR221C, YGR232W, YGR248W, YGR253C, YGR256W, YHL008C, YHR027C, YHR053C, YHR057C, YHR111W, YHR138C, YHR161C, YHR164C, YHR169W, YHR174W, YHR176W, YHR199C, YIL010W, YIL034C, YIL041W, YIL045W, YIL087C, YIL107C, YIL142W, YIL155C, YIR034C, YIR036C, YIR037W, YIR038C, YIR039C, YJL001W, YJL031C, YJL035C, YJL053W, YJL057C, YJL066C, YJL068C, YJL082W, YJL099W, YJL102W, YJL151C, YJL152W, YJL161W, YJL164C, YJL171C, YJL172W, YJL210W, YJL219W, YJR008W, YJR045C, YJR046W, YJR106W, YJR117W, YJR137C, YKL007W, YKL026C, YKL035W, YKL091C, YKL104C, YKL117W, YKL145W, YKL146W, YKL151C, YKL152C, YKL153W, YKL193C, YKL195W, YKL213C, YKL215C, YLL028W, YLL039C, YLL058W, YLR054C, YLR103C, YLR120C, YLR136C, YLR149C, YLR152C, YLR178C, YLR259C, YLR299W, YLR324W, YLR327C, YLR348C, YLR350W, YLR356W, YLR362W, YLR387C, YLR429W, YML054C, YML070W, YML100W, YML117W, YML125C, YMR004W, YMR008C, YMR009W, YMR020W, YMR067C, YMR089C, YMR097C, YMR102C, YMR105C, YMR107W, YMR152W, YMR180C, YMR184W, YMR191W, YMR219W, YMR271C, YMR275C, YMR295C, YMR314W, YMR316W, YNL006W, YNL007C, YNL012W, YNL044W, YNL045W, YNL074C, YNL092W, YNL093W, YNL104C, YNL115C, YNL156C, YNL231C, YNL234W, YNL237W, YNL281W, YNL305C, YNL333W,YNRO10W, YNR019W, YNR033W, YNR059W, YNR068C, YNR069C, YOL032W, YOL036W, YOL047C, YOL071W, YOL082W, YOL083W, YOL117W, YOL119C, YOL126C, YOL131W, YOL153C, YOL162W, YOL163W, YOL164W, YOR019W, YOR035C, YOR036W, YOR099W, YOR117W, YOR124C, YOR130C, YOR132W, YOR157C, YOR185C, YOR197W, YOR259C, YOR261C, YOR273C, YOR288C, YOR332W, YOR336W, YOR347C, YPL017C, YPL087W, YPL106C, YPL109C, YPL149W, YPL154C, YPL196W, YPL206C, YPL222W, YPR023C, YPR024W, YPR026W, YPR067W, YPR103W, YPR108W, YPR151C, YAL012W, YBR029C, YBR222C, YCL009C, YCL027W, YCL064C, YCR098C, YDL222C, YDR055W, YDR077W, YDR502C, YEL001C, YEL042W, YER026C, YER106W, YGR136W, YGR138C, YHR137W, YHR142W, YIL023C, YIL153W, YJL073W, YJR004C, YJR054W, YKL039W, YKL086W, YKL163W, YKR091W, YLR109W, YLR194C, YLR250W, YMR095C, YMR189W, YNL106C, YNL169C, YNL322C, YOR181W, YOR198C, YOR208W, YOR247W, YPL089C, YAL038W, YAL053W, YBR023C, YBR214W, YBR295W, YCR048W, YDL072C, YDL204W, YDR085C, YDR098C, YDR259C, YDR380W, YDR388W, YDR391C, YDR432W, YDR481C, YDR510W, YER069W, YGL022W, YGL126W, YGL209W, YGL255W, YGR189C, YGR282C, YHL035C, YHR030C, YIL022W, YIL024C, YIL117C, YIL123W, YIL140W, YIL154C, YJL088W, YJL108C, YJL149W, YJL159W, YJL186W, YJR148W, YKL096W, YLR180W, YLR273C, YLR300W, YLR307W, YLR378C, YLR391W, YMR094W, YMR104C, YMR276W, YMR296C, YNL190W, YNL208W, YNL300W, YNR064C, YOL013C, YOL058W, YOR248W, YOR355W, YPL052W, YPL163C, YPR079W, YAR028W, YBR146W, YBR183W, YCL038C, YCR071C, YDL008W, YDR019C, YDR031W, YDR115W, YDR486C, YER038C, YER130C, YFL054C, YGL136C, YGR146C, YGR207C, YHL040C, YIL167W, YJL020C, YKR039W, YLR031W, YLR205C, YMR072W, YMR140W, YMR173W, YMR195W, YMR226C, YNL037C, YNR002C, YOL143C, YOR136W, YOR215C, YOR382W, YOR383C, YPL054W, YPL271W, YPR127W, YAL044C, YAL054C, YAR010C, YAR027W, YAR071W, YBL001C, YBL043W, YBL057C, YBR014C, YBR024W, YBR035C, YBR068C, YBR111C, YBR116C, YBR147W, YBR168W, YBR246W, YBR273C, YCR004C, YCR021C, YCR037C, YCR088W, YDL022W, YDL128W, YDL238C, YDR003W, YDR009W, YDR033W, YDR084C, YDR104C, YDR270W, YDR315C, YDR340W, YDR357C, YDR358W, YDR396W, YDR405W, YDR410C, YDR434W, YDR482C, YDR487C, YDR520C, YDR534C, YDR539W, YEL011W, YEL065W, YEL066W, YER039C, YER044C, YER067W, YER080W, YER107C, YFL020C, YFL028C, YFL043C, YFR015C, YGL001C, YGL008C, YGL068W, YGL073W, YGL104C, YGL113W, YGL154C, YGL167C, YGL229C, YGL242C, YGL249W, YGL253W, YGR052W, YGR060W, YGR065C, YGR106C, YGR111W, YGR220C, YGR257C, YHL023C, YHL048W, YHR004C, YHR037W, YHR071W, YHR092C, YHR190W, YIL007C, YIL033C, YIL070C, YIL088C, YIL111W, YIR002C, YIR016W, YIR035C, YIR043C, YJL012C, YJL083W, YJL089W, YJL116C, YJL131C, YJL132W, YJL196C, YJR061W, YJR086W, YJR142W, YJR161C, YKL008C, YKL013C, YKL041W, YKL067W, YKL138C, YKL139W, YKL150W, YKL175W, YKR006C, YKR014C, YKR070W, YLL023C, YLR023C, YLR093C, YLR118C, YLR142W, YLR225C, YLR241W, YLR251W, YLR252W, YLR270W, YML030W, YML110C, YMR021C, YMR027W, YMR148W, YMR181C, YMR262W, YMR272C, YMR298W, YNL011C, YNL130C, YNL214W, YNL259C, YOL129W, YOR042W, YOR052C, YOR137C, YOR149C, YOR165W, YOR270C, YOR285W, YOR367W, YPL018W, YPL156C, YPL186C, YPL203W, YPL216W, YPL255W, YPR006C, YPR073C, YPR098C, YBR050C, YBR145W, YBR299W, YDR518W, YEL020C, YFL062W, YGL039W, YGL134W, YJL217W, YJR159W, YLR126C, YNL249C, YNL284C, YNL336W, YOL157C, YOR344C, YOR381W, YPL265W, YPR124W, YBR074W, YBR109C, YBR126C, YBR201W, YCR005C, YDL248W, YDR041W, YDR105C, YDR268W, YDR452W, YEL075C, YER046W, YER050C, YER136W, YER159C, YGL250W, YGR019W, YGR042W, YGR053C, YGR066C, YGR247W, YGR255C, YGR295C, YHL044W, YHR145C, YIL058W, YIL065C, YIL083C, YIL098C, YIL172C, YJL030W, YJL185C, YJL213W, YJR029W, YJR099W, YJR122W, YJR125C, YKL190W, YKR020W, YLL025W, YLL051C, YLR043C, YLR090W, YLR100W, YLR108C, YLR290C, YML068W, YMR051C, YMR139W, YMR178W, YMR193W, YNL015W, YNL079C, YNL122C, YNL223W, YNL285W, YNL293W, YNR007C, YNR035C, YNR061C, YOL016C, YOL104C, YOR220W, YOR221C; YOR374W, YPL123C, YPR077C, YPR107C, YPR147C, YBR093C, YBR196C, YEL041W, YEL047C, YER023W, YER119C, YFL055W, YGR209C, YIL124W, YKL187C, YLL055W, YMR318C, YOL152W, YAL007C, YBR067C, YBR115C, YBR285W, YBR292C, YDL043C, YDL123W, YDL131W, YDL168W, YDL212W, YDR056C, YDR132C, YDR154C, YDR183W, YDR216W, YDR253C, YDR295C, YDR494W, YDR513W, YEL072W, YER045C, YER061C, YER181C, YFL052W, YFL058W, YFR030W, YGL089C, YGL096W, YGL114W, YGL193C, YGL202W, YGL204C, YGL259W, YGR006W, YGR070W, YGR088W, YHL034C, YHL036W, YHR048W, YHR104W, YHR163W, YIL060W, YIL136W, YIR024C, YJL036W, YJL045W, YJL060W, YJL101C, YJL155C, YJR085C, YJR109C, YJR156C, YKL070W, YKL161C, YKL221W, YKR071C, YLL009C, YLL050C, YLR092W, YLR145W, YLR156W, YLR163C, YLR220W, YLR280C, YLR311C, YLR390W, YML116W, YMR034C, YMR038C, YMR081C, YMR250W, YNL240C, YNL260C, YNL277W, YNR074C, YOL044W, YOL084W, YOL147C, YOL159C, YOR184W, YOR228C, YOR255W, YPL223C, YPR160W, YDL182W, YBR047W, YBR054W, YBR291C, YDR069C, YER124C, YER131W, YGR044C, YIL094C, YKR007W, YMR240C, YNR050C, YOR007C, YAL015C, YBL065W, YBR105C, YBR182C, YBR186W, YBR244W, YBR272C, YCL069W, YDL025C, YDL059C, YDL085W, YDL113C, YDL244W, YDR018C, YDR054C, YDR202C, YDR223W, YDR350C, YDR353W, YDR374C, YDR512C, YEL052W, YEL070W, YER098W, YFR017C, YGL046W, YGL067W, YGL098W, YGL117W, YGL146C, YGL240W, YGRO11W, YGR067C, YGR133W, YGR153W, YGR223C, YHR116W, YHR124W, YIL097W, YIL168W, YJL103C, YJL221C, YJR036C, YJR095W, YKL085W, YKL133C, YKL162C, YKL188C, YKL217W, YKR061W, YKR105C, YLL062C, YLR174W, YLR216C, YLR247C, YLR260W, YLR267W, YLR389C, YML007W, YMR041C, YMR177W, YMR253C, YNL009W, YNL117W, YNL128W, YNL183C, YNR073C, YOL133W, YOL158C, YOR133W, YOR225W, YOR227W, YPL161C, YPL166W, YPL202C, YPL224C, YPR015C, YPR086W, YPR201W, YAL061W, YAL067C, YAR007C, YBL033C, YBL056W, YBL086C, YBR026C, YBR073W, YBR101C, YBR117C, YBR123C, YBR213W, YBR269C, YBR280C, YCR036W, YDL132W, YDL149W, YDL200C, YDL234C, YDL242W, YDR099W, YDR177W, YDR256C, YDR392W, YDR394W, YDR531W, YEL071W, YER014W, YER042W, YER090W, YER184C, YFL059W, YFR042W, YFR046C, YFR049W, YGL026C, YGL058W, YGL185C, YGL227W, YGL252C, YGL254W, YGR089W, YGR112W, YGR134W, YGR276C, YHL019C, YHR012W, YHR017W, YHR028C, YHR106W, YHR109W, YHR156C, YIL036W, YIL046W, YIL143C, YIL152W, YIL159W, YIL164C, YJL071W, YJL094C, YJL154C, YJR056C, YJR072C, YJR110W, YJR139C, YKL025C, YKL034W, YKL064W, YKL171W, YKL196C, YKR012C, YKR068C, YKR069W, YLLOO1W, YLL057C, YLL061W, YLR064W, YLR070C, YLR099C, YLR144C, YLR157C, YLR160C, YLR164W, YLR364W, YLR421C, YML032C, YML042W, YML112W, YML118W, YMR114C, YMR115W, YMR258C, YNL181W, YNL191W, YNL212W, YNL213C, YNL250W, YNL265C, YNL312W, YNR032W, YOL038W, YOL049W, YOL064C, YOR088W, YOR155C, YOR257W, YOR265W, YOR377W, YOR386W, YPL031C, YPL113C, YPL124W, YPL151C, YPL249C, YPL260W, YPL274W, YPR048W, YPR061C, YPR093C, YPR125W, YPR158W, YPR168W, YPR169W, YPR174C, YPR180W, YPR193C, YPR200C, YAL014C, YAL017W, YAL049C, YBL019W, YBL058W, YBR001C, YBR013C, YBR018C, YBR037C, YBR045C, YBR051W, YBR063C, YBR128C, YBR129C, YBR204C, YBR241C, YBR255W, YBR281C, YCL044C, YCL055W, YCR014C, YCR019W, YCR024C, YCR105W, YDL065C, YDL089W, YDL143W, YDL173W, YDL193W, YDL197C, YDL206W, YDL230W, YDL233W, YDR040C, YDR071C, YDR078C, YDR109C, YDR140W, YDR194C, YDR212W, YDR221W, YDR257C, YDR271C, YDR287W, YDR294C, YDR316W, YDR329C, YDR338C, YDR369C, YDR421W, YDR425W, YDR485C, YDR488C, YDR504C, YDR505C, YDR506C, YDR515W, YEL005C, YEL037C, YEL044W, YER017C, YER048C, YER052C, YER078C, YER089C, YER092W, YER100W, YER162C, YER182W, YFL021W, YFL042C, YFR045W, YFR051C, YFR056C, YGL040C, YGL041C, YGL045W, YGL057C, YGL093W, YGL105W, YGL125W, YGL166W, YGL181W, YGL183C, YGL215W, YGL216W, YGL221C, YGL223C, YGR007W, YGR029W, YGR155W, YGR156W, YGR186W, YGR198W, YGR210C, YGR211W, YGR237C, YGR250C, YGR258C, YGR266W, YGR270W, YGR274C, YGR277C, YHL021C, YHL037C, YHL038C, YHR082C, YHR083W, YHR134W, YHR160C, YHR171W, YHR180W, YHR205W, YIL062C, YIL072W, YIL075C, YIL099W, YIL108W, YIL165C, YIL170W, YIR009W, YIR018W, YIR031C, YIR032C, YJL032W, YJL049W, YJL128C, YJL165C, YJR044C, YJR052W, YJR090C, YJR091C, YJR103W, YJR104C, YJR153W, YKL059C, YKL079W, YKL090W, YKL094W, YKL192C, YKL209C, YKR052C, YKR102W, YKR106W, YLL054C, YLR025W, YLR097C, YLR200W, YLR226W, YLR248W, YLR266C, YLR392C, YLR427W, YML013W, YML029W, YML041C, YML051W, YML078W, YML079W, YML088W, YML099C, YMR056C, YMR068W, YMR091C, YMR110C, YMR160W, YMR186W, YMR255W, YNL005C, YNL026W, YNL039W, YNL063W, YNL064C, YNL077W, YNL083W, YNL147W, YNL176C, YNL194C, YNL253W, YNL257C, YNL261W, YNL264C, YNL276C, YNR006W, YNR034W, YNR047W, YNR051C, YNR071C, YOL065C, YOL067C, YOR005C, YOR008C, YOR022C, YOR023C, YOR058C, YOR069W, YOR087W, YOR138C, YOR229W, YOR256C, YOR267C, YPL005W, YPL020C, YPL022W, YPL105C, YPL147W, YPL150W, YPL152W, YPL164C, YPL168W, YPL180W, YPL188W, YPL194W, YPR025C, YPR047W, YPR049C, YPR066W, YPR081C, YPR134W, YPR140W, YPR148C, YPR155C, YPR172W, YPR185W, YAL018C, YAR064W, YBR012C, YBR076W, YBR287W, YDR043C, YDR250C, YDR373W, YFR014C, YGL191W, YGR180C, YHR136C, YJL026W, YJL037W, YLR038C, YNL058C, YOR031W, YGR087C, YIL166C, YHR008C, YIL129C, YGL256W, YJR030C, YMR077C, YBR264C, YPL177C, YKR040C, YGL056C, YDR128W, YGR139W, YBL101W-A, YOR253W, YOL026C, YDR278C, YHR095W, YCL042W, YNL200C, YPL221W, YLR415C, YMR058W, YPR037C, YER072W, YML028W, YOR325W, YAL039C, YMR112C, YJR107W, YGL088W, YJR058C, YNL142W, YDR090C, YMR071C, YBL093C, YGR293C, YML055W, YDL017W, YDL210W, YGL055W, YCL025C, YDR080W, YDL181W, YNR030W, YJL017W, YIL127C, YDR281C, YDR366C, YFR026C, YJL212C, YPL215W, YEL019C, YBR132C, YHL018W, YNL196C, YPL038W, YAR047C, YPL262W, YHL006C, YPL225W, YBR124W, YOR148C, YKR053C, YBL044W, YER029C, YLR360W, YCL056C, YCR007C, YGR239C, YNL256W, YPR146C, YLR377C, YKL097C, YBR066C, YLR338W, YDL229W, YBR253W, YJR027W, YKL198C, YBL030C, YBR031W, YBR118W, YBR162C, YBR221C, YCR024C-A, YCR106W, YDL046W, YDR012W, YDR133C, YDR134C, YDR276C, YDR342C, YDR343C, YEL027W, YEL034W, YGR038W, YGR243W, YGR279C, YHR094C, YHR105W, YHR175W, YHR181W, YIL056W, YIL162W, YJL059W, YJL097W, YJL158C, YJR105W, YKL051W, YKL056C, YKL097W-A, YKL100C, YKL141W, YKR066C, YLR134W, YLR258W, YLR339C, YML058W, YMR083W, YMR203W, YNL209W, YNL307C, YOL030W, YOR178C, YPL028W, YPR028W, YPR113W, YPR149W, YPR150W, YPR183W, YAL016W, YBL099W, YBL100C, YBR011C, YBR096W, YBR100W, YBR127C, YBR283C, YBR286W, YCL008C, YCL058C, YCR030C, YCR034W, YCR069W, YDL015C, YDL023C, YDL061C, YDL086W, YDR038C, YDR039C, YDR050C, YDR151C,YDR178W, YDR233C, YDR284C, YDR298C, YDR345C, YDR359C, YDR382W, YDR385W, YDR400W, YDR407C, YDR538W, YEL024W, YEL033W, YEL063C, YER057C, YER081W, YER120W, YFL011W, YGL012W, YGL206C, YGR022C, YGR026W, YGR082W, YGR107W, YGR172C, YGR191W, YGR204W, YGR260W, YHL005C, YHL046C, YHR025W, YHR026W, YHR123W, YHR126C, YHR143W, YIL011W, YIL015W, YIL018W, YIL157C, YIR041W, YJL016W, YJL121C, YJL133W, YJL138C, YJL191W, YJR018W, YJR047C, YJR077C, YJR119C, YJR121W, YJR123W, YJR143C, YJR145C, YKL060C, YKL147C, YKL148C, YKL157W, YKL164C, YKL169C, YKR033C, YLL041C, YLL064C, YLR041W, YLR044C, YLR056W, YLR058C, YLR081W, YLR089C, YLR110C, YLR177W, YLR264W, YLR284C, YLR304C, YLR340W, YLR354C, YLR372W, YLR388W, YML022W, YMR007W, YMR011W, YMR015C, YMR092C, YMR101C, YMR156C, YMR205C, YMR215W, YMR261C, YMR323W, YNL069C, YNL135C, YNL195C, YNR076W, YOL039W, YOL073C, YOL086C, YOL120C, YOL156W, YOL161C, YOR002W, YOR009W, YOR010C, YOR085W, YOR108W, YOR128C, YOR129C, YOR142W, YOR161C, YOR176W, YOR230W, YOR298W, YPL004C, YPL036W, YPL048W, YPL057C, YPL059W, YPL061W,YPL135W, YPL179W, YPL218W, YPL220W, YPL246C, YPL272C, YPR063C, YPR080W, YPR181C, YBR290W, YCR010C, YCR091W, YDL107W, YDL129W, YDR066C, YDR529C, YFL026W, YGL018C, YGL059W, YNL144C, YOR003W, YAL037W, YAR023C, YBR003W, YBR020W, YBR044C, YBR091C, YBR185C, YBR282W, YCR015C, YCR038C, YCR043C, YDL119C, YDL146W, YDL220C, YDR057W, YDR123C, YDR125C, YDR222W, YDR225W, YDR277C, YDR286C, YDR347W, YDR408C, YDR438W, YDR479C, YDR483W, YEL039C, YEL057C, YEL073C, YER066W, YER076C, YER084W, YER121W, YER189W, YFL017C, YFL046W, YFR006W, YFR008W, YGL115W, YGL208W, YGL214W, YGL218W, YGR021W, YGR023W, YGR024C, YGR064W, YGR076C, YGR096W, YGR108W, YGR174C, YGR182C, YGR236C, YGR288W, YHL042W, YHR195W, YHR210C, YIL006W, YIL012W, YIL028W, YIL050W, YIL057C, YIL089W, YIL102C, YIL113W, YIL122W, YJL100W, YJL169W, YJL199C, YJR039W, YJR050W, YJR101W, YKL003C, YKL016C, YKL061W, YKL093W, YKL121W, YKL160W, YKL170W, YKL194C, YKR034W, YKR067W, YLR006C, YLR016C, YLR030W, YLR036C, YLR112W, YLR125W, YLR128W, YLR204W, YLR211C, YLR233C, YLR257W, YLR288C, YLR326W, YLR334C, YLR395C, YLR408C, YLR414C, YLR444C, YML050W, YML107C, YML120C, YMR031C, YMR053C, YMR073C, YMR162C, YMR204C, YMR206W, YMR284W, YNL010W, YNL025C, YNL127W, YNL139C, YNL217W, YOL116W, YOL118C, YOR053W, YOR100C, YOR103C, YOR122C, YOR150W, YOR187W, YOR251C, YOR312C, YOR327C, YOR348C, YOR352W, YOR388C, YOR394W, YPL001W, YPL033C, YPL066W, YPL148C, YPL230W, YPL275W, YPL276W, YPR005C, YPR014C, YPR192W, YPR194C, YBR005W, YER025W, YFL027C, YGL080W, YGL205W, YHL028W, YHR185C, YIL076W, YJL166W, YLR046C, YMR035W, YMR238W, YMR252C, YNL192W, YNL202W, YOL108C, YOR385W, YPR165W, YAR033W, YBL038W, YBR009C, YBR010W, YBR151W, YCL067C, YCR096C, YDL137W, YDL192W, YDR073W, YDR086C, YDR224C, YDR377W, YDR378C, YER015W, YGL187C, YHR162W, YJL167W, YJL216C, YKR009C, YLR165C, YMR197C, YNL157W, YOL002C, YOL109W, YOR180C, YPL010W, YPL233W, YBR036C, YDR297W, YGR149W, YGR224W, YNL043C, YPL067C, YPL170W, YCR046C, YDR387C, YFL050C, YGL051W, YHR132C, YIL112W, YJL141C, YKR098C, YLR052W, YLR206W, YML129C, YNL203C, YNR014W, YOL043C, YOL096C, YPR184W, YAL028W, YAL055W, YAR062W, YBL095W, YBL102W, YBR122C, YBR157C, YBR161W, YBR251W, YBR298C, YCR039C, YCR083W, YDL018C, YDL067C, YDL078C, YDL091C, YDL215C, YDL216C, YDR022C, YDR067C, YDR079W, YDR181C, YDR186C, YDR196C, YDR262W, YDR306C, YDR319C, YER188W, YGL004C, YGL035C, YGR036C, YGR062C, YGR120C, YGR131W, YGR141W, YGR167W, YGR287C, YHL024W, YHR080C, YHR097C, YIL077C, YJL046W, YJL070C, YJL096W, YJL113W, YJL146W, YJL180C, YJR019C, YJR049C, YKR058W, YLL005C, YLR078C, YLR151C, YLR271W, YLR295C, YLR351C, YLR375W, YMR023C, YMR025W, YMR135C, YMR210W, YMR267W, YMR278W, YMR293C, YNL073W, YNR037C, YNR040W, YNR072W, YOR028C, YOR316C, YOR328W, YOR363C, YPL039W, YPL040C, YPL099C, YPL107W, YPL134C, YPL138C, YPL140C.

The base sequences and the amino acid sequences of the yeast genes are disclosed in public databases such as MIPS in Germany: Munich Information Center for Protein Sequence, and SGD in USA: Saccharomyces Genome Database, and are known via the internet. The sequences of promoters are also disclosed in a public database of SCPD: The Promoter Database of *Saccharomyces cerevisiae.*

In addition to the promoters from yeast genes as described above, promoters from a gene that is homologous to the yeast genes and is derived from other species may be used. In this context, "a gene that is homologous to the yeast genes" means a gene that comprises a base sequence having a 50% or more, preferably 80% or more of the base sequences of yeast genes, wherein the base sequence encodes a protein having the same functions as the proteins encoded by the yeast genes.

A polynucleotide encoding a marker protein is operably linked to a polynucleotide of a promoter from the gene as described above so as to provide a polynucleotide construct. Processes to link a polynucleotide encoding a protein operably to a promoter is well known in the art. See, for example, R. W. Old, S. B. Primrose Principles of Gene Manipulation 5th Ed., BAIFUKAN CO., LTD, pp 138-165, pp.234-263,2000.

Examples of marker proteins include GFP (Green Fluorescence Protein) (Heim, R., Cubitt, A. B. and Tsien, R. Y. (1995) Nature 373, 663-664; Heim, R., Prasher DC. and Tsien, R. Y. (1994) Proc. Natl. Acad. Sci., 91, 12501-12504; Warg, S. and Hazerigg, T. (1994) Nature 639, 400-403; Youvan, D.C. and Michel-Beyerle, M.E. (1996) Nature Biotechnology 14 1219-1220; Chalfie, M., Tu, Y., Euskirchen, G., Ward, W. W. and Prasher, D.C. (1994) Science 263, 802-805), β-galactosidase (Canestro C, Albalat R, Escriva H, Gonzalez-Duarte R. Endogenous beta-galactosidase activity in amphioxus: a useful histochemical marker for the digestive system.Dev Genes Evol 2001 Mar 211(3):154-6), luciferases (Arch Toxicol 2002 Jun;76(5-6):257-61, Estrogenic activity of UV filters determined by an in vitro reporter gene assay and an in vivo transgenic zebrafish assay.Schreurs R, Lanser P, Seinen W, Van Der Burg B.), and acetyltransferase (J Recept Signal Transduct Res 2001 Feb;21(1):71-84, A simplified method for large scale quantification of ranscriptional activity and its use in studies of steroids and steroid receptors. Zhang S, Lu J, Iyama K, Lo SC, Danielsen M.).

The specification also relates to a vector that comprises a polynucleotide construct as described above.

A polynucleotide comprising a promoter sequence from yeast genes is obtained by preparing a primer that transcribes a likely necessary portion on the basis of the base sequence of the yeast genes as known in the public database, and amplifying the same by PCR using the genomic DNA of yeast as a template. Further, a plasmid that is capable of replicating in an intended cell is selected, and a base sequence of a marker protein is introduced into the plasmid. The polynucleotide comprising a promoter sequence as shown above is inserted upstream the marker gene to obtain an intended vector.

The specification also relates to a host cell that is transformed with the vector as described above. It is natural to use preferably human cells as a host cell, and cells from other mammalian such as mouse may be used. Also, cells from fishes, nematode or the like as used in bioassay so far may be used in view of environmental toxic evaluation. Further, it is preferred to use microbiological cells since cultivation of them are easy. It is more preferred to use yeast cells because the present method is based on the use of genes from yeast cells, and because yeast is grown irrespective of variable environmental conditions such as salt concentration. Transformation of cells are well known. For example, see Kaiser C, Michaelis S, Mitchell A: Lithium acetate yeast transformation, Methods in Yeast Genetics, A Cold Spring Harbor Laboratory Course Manual 1994 edition (Cold Spring Harbor Laboratory Press) pp.133-134,1994. The purpose may be attained without vector when the encoding region of the yeast gene is replaced with a polynucleotide sequence encoding a marker protein. The polynucleotide construct can be directly introduced into cells, and the method therefore is also well known.

The invention also relates to a process for detecting a toxic compound in a test material, which comprises:

(1) contacting the test material to the transformed cells as described above, and
(2) detecting the expression of mRNA encoding a marker protein.

The step to contact a test material to a cell comprises for example culturing the transformed cell in liquid at an appropriate condition for the cultivation of the cell, and adding the test material directly to the culture liquid.
Then, the expression level of a marker protein or a mRNA encoding the protein is determined.
The determination of the expression level of a marker protein may be conducted by destroying the cells, obtaining an extract containing the protein, and determining the level of marker protein in the extract. For example, when a marker protein is GFP, the fluorescence level in the protein extract is determined with a spectrofluorometer. Also, even when the cells are not destroyed, it is possible to determine them by observation and image processing with fluorescence microscopes and laser microscopes, determination with flow cytometry, and detection with evanescent lights.
The level of an expressed mRNA may be detected by 1) northern blotting (OGATA Nobukuni, NOJIMA Hiroshi: Genetic Engineering Keywords Book, revised 2nd ed., Yodosha, co.jp, 2000, pp299-301), 2) reverse transcription-PCR (RT-PCR) (NAKABEPPU Yusaku, et al.: Cell Technology, suppl. Tips series, Modified PCR Tips, Shujunsha Co.Ltd., 1999, pp25-43) and the like.
Procedures of northern blotting comprises electrophoresing the RNA, transferring the pattern to a filter, and hybridizing it with a specific probe labeled with an isotope, thereby analyzing the presences and the amount of mRNA in the sample as well as the length of the same. RT-PCR is a procedure for detection and quantitative determination of an intended RNA in a form of the amplified cDNA, which comprises forming cDNA from the RNA by reverse-transcription with reverse transcriptase, and conducting PCR using the cDNA as a starting material as well as specific primer sets and a thermostable DNA polymerase.
Toxic substances to be detected according to the present invention include, but are not limited to, $Na_2As$, $CdCl_2$, $HgCl_2$, $PbCl_2$, 4-nitroquinolin-N-oxide, 2,4,5-trichlorophenol, γ-hexachlorocyclohexane, manganese ethylenebis (dithiocarbamate), 2,4,5,6-tetrachloro-1,3-benzenedicarbonitrile, tetramethylthiuram disulfide, zinc N,N'-ethylenebis (dithiocarbamate), 8-methyl-N-vanillyl-6-nonenamide, gingerol, acrolein, dimethylsulfoxide, Roundup (trademark, herbicide) (N-(phosphomethyl) glycinate ammonium 41.0%, surfactant 59.0%), sodium dodecylbenzosulfonate, sodium lauryl sulfate , 2,4-dichlorophenoxyacetic acid, potassium cyanide, benzo(a)pyrene, formaldehyde, bisphenol-A, 2,5-dichlorophenol, methylmercury chloride, p-nonylphenol, pentachlorophenol, nickel (II) chloride, potassium bichromate , triphenyltin chloride, phenol, S-4-chlorobenzyl-N,N-diethylcarbamate, hexachlorophene, triclosan, and copper sulfate.
When the method as described above is conducted using two or more cells, both of which are transformed with a vector comprising a polynucleotide of a promoter from different yeast genes operably linked to a polynucleotide encoding a marker protein, then toxic substances can be further examined. For example, as shown in the working examples hereinafter, YLL057C can be used as a yeast gene promoter to detect 2,4-dichlorophenoxyacetic acid, arsenious acid or a salt thereof, cadmium salt, and cyanide or a salt thereof, and YLR303W can be used as a yeast gene promoter to detect 2,4-dichlorophenoxyacetic acid, arsenious acid or a salt thereof, cadmium salt, cyanide or a salt thereof, benzo(a)pyrene, formaldehyde, manganese ethylenebis (dithiocarbamate), and a mercuric salt. Accordingly, when for example the expression of a marker protein is induced by using YLR303W as yeast gene, and not induced by YLL057C, then toxic substances are identified as benzo(a)pyrene, mercuric salt, manganese ethylenebis(dithiocarbamate) or formaldehyde. When the expression of a marker protein is induced by using both yeast genes, then toxic substances are identified as 2,4-dichlorophenoxyacetic acid, arsenious acid or a salt thereof, cadmium salt, or cyanide or a salt thereof
The following examples are presented for purpose of further illustration of the invention, and such examples are not intended to be limiting the invention in any respect.

EXAMPLES

Example 1

The following experiment was conducted to examine which yeast gene is useful for the detection of a toxic substance.
Yeast *(Saccharomyces cerevisiae* S288C (α SUC2mal mel gap2 CUP1)) were cultured at 25°C on YPD medium (yeast extract 1%, polypepton 2%, glucose 2%). One of toxic chemical substances was added to the cell at logarithmic growth phase, and the cell was further cultured for two hours. Cell was cultured without any chemical substance in the same condition, and was used as control. Concentrations of the chemical substances were defined to inhibit the growth of the yeast but not lead to death.

| Chemical Substances | Concentrations |
|---|---|
| (1) $Na_2As$ | 0.3mM |
| (2) $CdCl_2$ | 0.3mM |
| (3) $HgCl_2$ | 0.7mM |
| (4) $PbCl_2$ | 2mM |
| (5) 4-nitroquinolin-N-oxide | 0.2μM |
| (6) 2,4,5-trichlorophenol | 16μM |
| (7) γ-hekachlorocyclohexane | 1.3mM |
| (8) manganese ethylenebis(dithiocarbamate) | 2ppm |
| (9) 2,4,5,6-tetrachloro-1,3- benzenedicarbonitrile | 10μM |
| (10) Tetramethylthiuram disulfide | 75μM |
| (11) zinc N,N'-ethylenebis(dithiocarbamate) | 2ppm |
| (12) 8-methyl-N-vanillyl-6-nonenamide 0.82mM | |
| (13) gingerol | 1.36mM |
| (14) acrolein | 0.20mM |
| (15) dimethylsulfoxide | 1.41M |
| (16) Roundup (trademark, herbicide) [1)] | 1500-fold dilution |
| (17) sodium dodecylbenzosulfonate | 0.02% |
| (18) sodium lauryl sulfate | 0.01% |

1) N- (phosphomethyl) glycinate ammonium41.0%, surfactant 59.0%

After the completion of cultivation, the culture was centrifuged to collect the cells. To the cells, sodium acetate buffer (50mM sodium acetate, 10mM EDTA, 1% SDS) was added, and the mixture was shaken at 65°C for five minutes, followed by returning to room temperature, and obtaining the supernatant, which procedure was repeated two times. To the supernatant, 1/2 amount of a solution of phenol/chloroform was added, and the mixture was centrifuged to give a supernatant, which was added with an equal amount of chloroform, and the mixture was centrifuged to give a supernatant. To the supernatant, an equal amount of isopropanol containing 0.3 M sodium acetate was added, and the mixture was allowed to stand at room temperature for 30 minutes, after which the mixture was centrifuged to give a sediment of the whole RNAs. Seventy % ethanol was added to the sediment, and the mixture was again centrifuged to give a sediment, which was then dried and dissolved in water. mRNA was isolated from the whole RNAs as followings. In view of the fact that a poly A chain is attached to the 3' terminus of mRNA, a polynucleotide having a poly T structure which was immobilized on the surface of latex beads was used to trap the mRNA, and then the mRNA was washed and eluted with spine column (Oligotex-dT30<Super>mRNA Purification Kit, Takara). Reverse transcription of the mRNA was conducted with a reverse transcriptase (Super Script II Reverse Transcriptase; catalogue No. 18064-014, GibcoBRL) using fluorescence-labeled nucleotides to give cDNAs that were introduced with Cy3-dUTP or Cy5-dUTP during the reverse transcription.

The labeled cDNAs were dissolved in TE buffer (10mM Tris-HCl/1mM EDTA, pH8.0), and the solution was dropped on the DNA chip containing the whole genes of yeast (DNA Chip Research Inc. Japan) so that the cDNAs were hybridized on the DNA chip at 65 ° C for over 12 hours. The fluoresces intensity of the DNA chip was read with a scanner, and the ratio relative to the fluorescence intensity resulting from the absence of chemical substance was estimated as the following, which is shown in Tables 1 to 9:

$$\frac{\text{The level of expressed mRNA in the presence of chemical substance}}{\text{The level of expressed mRNA in the absence of chemical substance}}$$

In the Tables, "Intensity" indicated in the rightmost column is a value of the level of expressed mRNA of each gene in control cells as divided by the average level of the expressions of the whole genes. A gene of which mRNA level is lower in control, and is higher in cases of addition of chemical substance, is especially useful in the detection of toxic substances.

**Table 1** Unknown protein yeast genes

| yeast genes | The level of expressed mRNA in the presence of chemical substance/ The level of expressed mRNA in the absence of chemical substance | | | | | | | | | | | | | | | | | | |
|---|---|---|---|---|---|---|---|---|---|---|---|---|---|---|---|---|---|---|---|
| | (1) | (2) | (3) | (4) | (5) | (6) | (7) | (8) | (9) | (10) | (11) | (12) | (13) | (14) | (15) | (16) | (17) | (18) | Intensity |
| YCR102C | 3.4 | 3.2 | 2.4 | 1.1 | 0.7 | 0.4 | 1.1 | 4.9 | 9.8 | 61.5 | 13.5 | 0.5 | 1.1 | 4.1 | 0.9 | 1.2 | 1.1 | 1.2 | 0.53 |
| YDL218W | 3.5 | 1.2 | 2.6 | 1.4 | 0.7 | 0.9 | 1.1 | 1.5 | 72.5 | 12.6 | 5.7 | 0.9 | 1.1 | 4.8 | 1.1 | 3.5 | 1.1 | 0.9 | 0.42 |
| YDR533C | 3.4 | 4.9 | 3.5 | 1.8 | 1.3 | 2.2 | 1.7 | 5.4 | 7.3 | 12.7 | 7.7 | 1.4 | 3.0 | 2.9 | 2.5 | 6.7 | 2.3 | 2.7 | 1.82 |
| YGR110W | 1.6 | 3.1 | 2.2 | 5.9 | 0.9 | 0.9 | 1.7 | 1.5 | 6.4 | 4.8 | 0.5 | 1.4 | 3.6 | 2.8 | 1.2 | 17.1 | 0.9 | 2.1 | 0.19 |
| YKL071W | 8.3 | 3.8 | 5.4 | 2.8 | 1.1 | 18.6 | 3.7 | 24.7 | 162.2 | 109.2 | 40.3 | 1.0 | 8.4 | 21.9 | 1.7 | 2.2 | 1.0 | 0.9 | 0.31 |
| YLR460C | 2.1 | 2.1 | 2.0 | 1.1 | 1.3 | 0.6 | 1.1 | 8.1 | 13.9 | 31.6 | 14.3 | 0.4 | 0.7 | 3.5 | 0.7 | 1.8 | 1.0 | 1.1 | 0.48 |
| YMR090W | 6.9 | 9.5 | 2.3 | 3.5 | 0.7 | 3.8 | 2.7 | 3.9 | 16.7 | 6.1 | 8.8 | 2.2 | 6.9 | 6.5 | 1.9 | 5.1 | 1.8 | 2.8 | 1.30 |
| YOL150C | 4.1 | 3.7 | 9.9 | 0.9 | 1.0 | 5.2 | 2.4 | 3.7 | 10.8 | 8.3 | 9.4 | 2.9 | 8.9 | 5.7 | 1.8 | 3.1 | 2.7 | 2.2 | 0.47 |
| YBL048W | 2.0 | 7.9 | 2.3 | 2.8 | 1.3 | 2.0 | 1.9 | 1.2 | 7.2 | 4.4 | 2.0 | 1.2 | 2.7 | 2.0 | 4.1 | 5.7 | 1.3 | 1.0 | 0.29 |
| YBL107C | 1.4 | 1.1 | 0.4 | 1.5 | 1.2 | 1.1 | 2.1 | 1.9 | 1.0 | 3.1 | 2.9 | 1.4 | 2.6 | 2.5 | 1.4 | 1.5 | 0.9 | 1.4 | 1.03 |
| YFL024C | 1.0 | 1.1 | 1.1 | 0.7 | 1.1 | 2.0 | 3.3 | 0.9 | 0.5 | 0.5 | 0.6 | 0.8 | 0.9 | 3.3 | 0.9 | 1.1 | 0.9 | 0.8 | 0.37 |
| YGL121C | 2.6 | 5.0 | 1.4 | 4.2 | 0.9 | 9.8 | 4.7 | 3.0 | 7.0 | 15.0 | 6.1 | 1.6 | 7.8 | 5.8 | 1.6 | 8.5 | 2.7 | 3.5 | 0.60 |
| YHR029C | 2.8 | 1.8 | 1.8 | 1.8 | 1.0 | 2.7 | 2.0 | 2.7 | 13.6 | 8.6 | 3.8 | 1.5 | 4.7 | 3.5 | 1.0 | 1.0 | 2.1 | 2.1 | 0.87 |
| YHR209W | 2.8 | 1.1 | 0.8 | 4.5 | 1.8 | 3.2 | 5.0 | 2.0 | 2.9 | 6.4 | 3.3 | 1.0 | 2.0 | 2.2 | 5.0 | 3.5 | 2.6 | 2.6 | 0.56 |
| YKL107W | 2.4 | 2.5 | 0.5 | 0.8 | 1.1 | 2.0 | 1.1 | 0.9 | 28.2 | 8.3 | 2.0 | 1.0 | 1.9 | 3.4 | 1.7 | 3.5 | 1.6 | 0.9 | 0.17 |
| YKR075C | 1.5 | 0.7 | 2.7 | 4.5 | 1.2 | 1.5 | 1.4 | 2.0 | 0.2 | 0.6 | 1.3 | 2.3 | 3.8 | 2.7 | 0.4 | 4.3 | 2.4 | 1.9 | 1.15 |
| YLL056C | 2.1 | 2.3 | 3.0 | 2.0 | 1.1 | 0.6 | 1.0 | 1.3 | 58.8 | 8.8 | 1.7 | 1.3 | 6.8 | 11.0 | 1.5 | 4.8 | 5.3 | 2.5 | 0.32 |
| YLR297W | 2.4 | 2.1 | 3.5 | 1.7 | 1.3 | 0.8 | 1.1 | 1.3 | 2.1 | 5.5 | 3.5 | 0.8 | 0.8 | 2.3 | 0.7 | 1.8 | 0.7 | 1.0 | 0.81 |
| YOR338W | 1.8 | 1.8 | 0.5 | 1.7 | 1.6 | 2.6 | 1.3 | 2.2 | 0.6 | 3.5 | 0.8 | 1.2 | 1.4 | 2.5 | 1.8 | 1.0 | 1.1 | 0.5 | 0.52 |
| YOR391C | 2.1 | 2.0 | 0.9 | 1.2 | 1.3 | 1.6 | 2.0 | 2.8 | 18.8 | 18.1 | 3.2 | 1.1 | 2.3 | 2.4 | 5.8 | 2.2 | 1.0 | 0.9 | 0.22 |
| YPL280W | 1.7 | 1.9 | 1.9 | 2.0 | 1.4 | 1.9 | 1.8 | 2.3 | 49.8 | 14.0 | 1.9 | 0.9 | 2.4 | 2.4 | 4.3 | 1.5 | 1.2 | 0.9 | 0.22 |
| YLR346C | 4.4 | 2.0 | 2.1 | 4.5 | 1.4 | 5.5 | 5.6 | 1.3 | 8.6 | 6.6 | 0.5 | 4.2 | 6.2 | 2.2 | 1.0 | 2.4 | 10.6 | 7.8 | 0.53 |
| YOR049C | 2.5 | 0.9 | 12.8 | 1.4 | 1.3 | 8.4 | 4.2 | 0.9 | 1.1 | 1.9 | 1.1 | 6.7 | 7.1 | 1.5 | 1.3 | 4.3 | 2.6 | 2.9 | 0.22 |
| YAL034C | 1.1 | 1.8 | 2.8 | 1.1 | 1.0 | 1.5 | 1.8 | 1.0 | 3.0 | 3.3 | 1.5 | 2.8 | 4.9 | 1.2 | 1.4 | 1.0 | 1.2 | 1.3 | 0.52 |
| YDR476C | 0.7 | 1.8 | 6.0 | 1.2 | 0.5 | 3.1 | 1.5 | 1.3 | 1.7 | 3.4 | 1.6 | 2.1 | 2.8 | 1.1 | 2.2 | 2.0 | 2.4 | 1.5 | 0.58 |
| YGR035C | 1.6 | 0.6 | 1.2 | 0.6 | 1.3 | 3.3 | 1.1 | 0.9 | 1.1 | 0.5 | 0.4 | 3.3 | 2.1 | 0.8 | 0.3 | 1.9 | 2.5 | 4.3 | 1.04 |
| YGR284C | 1.0 | 2.0 | 4.0 | 1.4 | 1.2 | 1.4 | 2.2 | 1.1 | 4.3 | 1.8 | 1.7 | 2.1 | 3.6 | 1.2 | 3.9 | 1.9 | 1.3 | 2.1 | 2.57 |
| YHR054C | 1.7 | 0.9 | 2.2 | 1.0 | 1.0 | 2.9 | 1.6 | 1.8 | 2.6 | 7.7 | 0.9 | 3.6 | 5.8 | 1.0 | 0.8 | 2.2 | 5.1 | 3.4 | 0.80 |
| YLR008C | 0.9 | 0.5 | 0.7 | 0.9 | 1.3 | 2.7 | 2.0 | 1.0 | 0.4 | 0.7 | 0.8 | 3.5 | 6.2 | 1.6 | 0.7 | 1.7 | 5.4 | 4.9 | 2.00 |

| | | | | | | | | | | | | | | | | | | |
|---|---|---|---|---|---|---|---|---|---|---|---|---|---|---|---|---|---|---|
| YMR315W | | 4.3 | 1.8 | 1.7 | | 4.2 | 2.9 | 2.0 | 2.4 | 2.6 | 3.2 | 2.0 | 2.5 | 1.3 | 2.5 | 1.6 | 2.1 | 2.8 | 1.76 |
| YNL211C | 1.2 | 1.0 | 0.8 | 1.0 | 1.0 | 7.4 | 3.1 | 1.1 | 0.8 | 1.1 | 1.1 | 3.2 | 9.9 | 1.1 | 0.8 | 9.3 | 4.2 | 6.8 | 0.52 |
| YOL031C | 0.9 | 1.9 | 1.0 | 1.7 | 1.2 | 1.0 | 2.5 | 2.1 | 2.3 | 1.4 | 1.3 | 2.1 | 2.4 | 1.1 | 4.4 | 1.5 | 1.2 | 1.9 | 1.20 |
| YOL101C | 0.9 | 0.8 | 0.4 | 1.7 | 1.6 | 0.5 | 0.7 | 0.9 | 0.1 | 0.3 | 0.9 | 2.4 | 0.2 | 0.8 | 0.6 | 3.5 | 0.6 | 0.6 | 1.37 |
| YAR031W | 1.5 | 1.1 | 1.1 | 1.7 | 1.1 | 2.9 | 1.6 | 1.2 | 2.7 | 1.6 | 1.4 | 1.2 | 3.3 | 1.4 | 0.9 | 3.5 | 1.6 | 2.5 | 1.00 |
| YBL049W | 2.9 | 11.8 | 2.1 | 2.9 | 1.1 | | 1.7 | 1.9 | 5.2 | 5.2 | 2.2 | 0.7 | 3.9 | 1.5 | 7.2 | 6.4 | 1.1 | 1.1 | 0.47 |
| YBR062C | 1.3 | 1.7 | 0.4 | 1.2 | 1.4 | 1.9 | 1.5 | 1.9 | 5.4 | 2.2 | 2.3 | 1.2 | 2.8 | 2.6 | 0.7 | 2.2 | 1.4 | 1.6 | 0.93 |
| YCR013C | 1.3 | 1.4 | 5.0 | 1.1 | 1.1 | 1.8 | 1.6 | 0.7 | 0.8 | 1.3 | 2.4 | 1.4 | 3.9 | 1.5 | 1.6 | 1.6 | 1.1 | 1.3 | 2.80 |
| YDL027C | 1.2 | 2.0 | 1.7 | 1.7 | 1.2 | 2.1 | 2.9 | 1.5 | 2.0 | 2.3 | 1.7 | 1.7 | 3.2 | 1.1 | 1.4 | 1.8 | 1.4 | 2.0 | 0.69 |
| YDL110C | 1.5 | 1.4 | 1.1 | 2.1 | 0.8 | 1.7 | 2.5 | 1.8 | 3.0 | 2.3 | 1.9 | 1.5 | 2.7 | 2.7 | 1.3 | 3.6 | 2.0 | 4.4 | 1.27 |
| YDL169C | 1.7 | 1.7 | 1.1 | 1.3 | 1.1 | 2.6 | 2.1 | 1.3 | 7.3 | 4.7 | 1.2 | 1.2 | 3.7 | 2.7 | 2.2 | 3.8 | 1.5 | 1.6 | 0.42 |
| YDR070C | 2.2 | 6.4 | 3.6 | 3.5 | 0.9 | 6.2 | 1.4 | 1.9 | 13.9 | 6.0 | 4.1 | 4.3 | 6.6 | 2.0 | 2.1 | 15.1 | 1.2 | 3.1 | 0.44 |
| YDR210W | 1.1 | 1.0 | 0.9 | 1.0 | 2.0 | 1.2 | 1.5 | 1.4 | 0.5 | 1.0 | 1.0 | 1.0 | 2.3 | 1.1 | 1.0 | 1.5 | 1.9 | 1.9 | 1.82 |
| YDR214W | 1.3 | 4.0 | 4.5 | 1.3 | 1.1 | 1.7 | 1.5 | 1.5 | 5.5 | 2.4 | 1.9 | 1.1 | 5.3 | 1.2 | 1.1 | 0.7 | 1.2 | 1.0 | 1.07 |
| YDR435C | 1.2 | 1.1 | 1.2 | 1.4 | 1.2 | 2.5 | 2.1 | 1.2 | 0.8 | 2.1 | 1.3 | 1.1 | 2.5 | 1.3 | 0.6 | 2.0 | 1.3 | 2.0 | 1.30 |
| YER037W | 2.0 | 2.1 | 0.8 | 1.1 | 1.3 | 3.2 | 2.6 | 1.0 | 1.0 | 5.5 | 1.5 | 1.2 | 3.0 | 1.7 | 0.8 | 1.8 | 1.7 | 2.6 | 1.17 |
| YFL044C | 1.0 | 1.5 | 0.8 | 2.3 | 1.6 | 1.3 | 1.6 | 1.4 | 5.2 | 3.7 | 2.2 | 1.2 | 4.7 | 1.2 | 1.0 | 1.8 | 1.3 | 1.4 | 0.50 |
| YFR003C | 1.2 | 1.5 | 0.8 | 1.6 | 1.0 | 2.2 | 1.4 | 1.4 | 8.3 | 3.1 | 1.4 | 0.9 | 2.5 | 2.1 | 1.0 | 1.4 | 1.3 | 1.8 | 1.01 |
| YFR020W | 1.0 | 0.9 | 1.2 | 1.0 | 0.9 | 1.4 | 1.1 | 0.9 | 3.7 | 3.5 | 1.4 | 0.9 | 2.3 | 1.8 | 2.6 | 1.0 | 1.2 | 0.9 | 0.61 |
| YFR044C | 0.9 | 1.3 | 3.8 | 1.1 | 0.9 | 2.1 | 1.2 | 0.8 | 2.1 | 2.2 | 1.9 | 1.2 | 3.1 | 0.8 | 1.5 | 1.2 | 1.0 | 1.0 | 1.99 |
| YGR010W | 1.2 | 1.6 | 0.9 | 1.2 | 0.8 | 1.2 | 1.7 | 1.6 | 13.6 | 8.9 | 1.5 | 0.9 | 5.2 | 1.9 | 1.3 | 1.4 | 0.8 | 1.3 | 0.55 |
| YGR142W | 2.7 | 6.2 | 15.3 | 1.3 | 1.3 | 0.9 | 1.4 | 1.5 | 36.8 | 12.2 | 2.3 | 2.0 | 8.9 | 0.9 | 1.3 | 0.3 | 1.9 | 1.5 | 1.28 |
| YGR161C | 2.0 | 2.2 | 5.4 | 3.8 | 1.0 | 1.4 | 1.4 | 1.3 | 2.0 | 3.8 | 0.8 | 1.7 | 2.8 | 0.9 | 1.7 | 1.1 | 1.5 | 1.8 | 0.65 |
| YGR212W | 1.5 | 1.7 | 1.5 | 1.3 | 1.2 | | | 1.0 | 1.1 | 3.8 | 0.9 | 1.5 | 10.0 | 1.1 | 1.1 | 2.1 | 3.9 | 2.2 | 0.28 |
| YGR268C | 1.1 | 2.3 | 2.0 | 2.5 | 1.1 | 1.1 | 1.0 | 1.1 | 4.1 | 1.6 | 0.8 | 1.0 | 2.8 | 1.0 | 1.5 | 1.2 | 1.5 | 1.4 | 0.48 |
| YHR016C | 0.8 | 1.5 | 1.7 | 1.5 | | 1.8 | 3.7 | 0.8 | 2.8 | 2.0 | 2.2 | 1.3 | 3.3 | 0.9 | 0.6 | 2.1 | 1.2 | 1.4 | 0.51 |
| YHR087W | 1.2 | 2.7 | 8.6 | 2.8 | 0.7 | 4.2 | 3.4 | 1.2 | 4.1 | 3.9 | 3.7 | 1.4 | 4.0 | 1.5 | 1.9 | 3.8 | 1.2 | 2.9 | 1.34 |
| YJL048C | 1.7 | 2.5 | 2.0 | 2.9 | 0.9 | 1.5 | 0.7 | 1.5 | 1.2 | 3.8 | 3.2 | 1.1 | 2.4 | 1.7 | 0.6 | 2.3 | 1.6 | 2.5 | 0.82 |
| YJL144W | 1.9 | 1.6 | 2.2 | 1.6 | 1.2 | 1.7 | 1.7 | 1.3 | 14.6 | 8.9 | 3.0 | 0.8 | 3.9 | 1.6 | 1.0 | 2.5 | 2.2 | 2.4 | 0.60 |
| YJL163C | 1.2 | 1.9 | 1.7 | 1.5 | 0.9 | 2.8 | 2.3 | 0.7 | 14.5 | 2.5 | 3.1 | 2.0 | 4.5 | 1.0 | 2.0 | 2.8 | 1.5 | 1.7 | 0.28 |
| YJR074W | 1.0 | 1.4 | 2.0 | 2.0 | 0.7 | 1.2 | 1.8 | 1.3 | 3.7 | 3.1 | 1.2 | 2.0 | 2.8 | 1.7 | 3.4 | 1.5 | 1.8 | 1.9 | 0.68 |
| YKL065C | 1.9 | 2.0 | 1.3 | 1.7 | 1.0 | 2.3 | 3.8 | 1.8 | 3.1 | 3.9 | 1.3 | 1.3 | 3.0 | 2.1 | 1.7 | 2.8 | 2.8 | 4.6 | 2.29 |

| | | | | | | | | | | | | | | | | | | | |
|---|---|---|---|---|---|---|---|---|---|---|---|---|---|---|---|---|---|---|---|
| YKR011C | 1.1 | 1.6 | 0.9 | 1.6 | 1.2 | 2.3 | 2.7 | 2.0 | 4.7 | 4.4 | 1.5 | 1.1 | 3.5 | 1.4 | 1.8 | 1.3 | 1.3 | 1.9 | 0.66 |
| YKR018C | 0.7 | 1.1 | 1.7 | 0.7 | 0.9 | 1.7 | 1.0 | 0.7 | 3.9 | 1.7 | 1.4 | 1.0 | 3.0 | 1.1 | 1.0 | 1.8 | 1.0 | 1.2 | 0.98 |
| YKR046C | 1.9 | 7.8 | 2.8 | 1.8 | 0.6 | 1.9 | 1.3 | 0.8 | 3.9 | 2.0 | 2.6 | 1.3 | 2.8 | 1.9 | 2.9 | 3.5 | 1.2 | 1.9 | 0.73 |
| YKR049C | 1.6 | 4.2 | 1.0 | 3.2 | 0.9 | 3.0 | 2.4 | 1.3 | 2.2 | 3.2 | 2.2 | 1.2 | 4.1 | 2.1 | 0.9 | 4.2 | 1.6 | 3.3 | 1.17 |
| YLR132C | 1.0 | 1.1 | 0.3 | 1.1 | 1.2 | 1.1 | 1.1 | 0.7 | 4.5 | 2.5 | 0.5 | 1.0 | 2.7 | 1.3 | 2.3 | 1.6 | 1.2 | 1.6 | 0.36 |
| YLR161W | 0.9 | 0.8 | 0.8 | 0.7 | 0.9 | 1.2 | 1.3 | 0.8 | 1.0 | 1.5 | 1.5 | 0.8 | 2.1 | 1.0 | 0.6 | 1.1 | 1.1 | 0.9 | 0.33 |
| YLR217W | 0.9 | 2.0 | 2.1 | 0.7 | 1.4 | 1.4 | 1.5 | 1.0 | 3.2 | 8.2 | | 0.9 | 3.8 | 0.9 | 0.9 | 0.8 | 1.0 | 1.2 | 0.35 |
| YLR328W | 1.3 | 1.2 | 1.3 | 0.9 | 0.9 | 4.8 | 5.4 | 1.1 | 0.9 | 1.2 | 1.4 | 1.2 | 4.3 | 0.5 | 0.8 | 1.4 | 1.1 | 0.8 | 1.04 |
| YML128C | 1.5 | 3.3 | 4.1 | 4.1 | 0.5 | 5.1 | 2.8 | 1.1 | 5.1 | 3.1 | 2.9 | 1.4 | 4.9 | 1.4 | 2.6 | 5.6 | 2.0 | 5.5 | 1.66 |
| YMR040W | 3.1 | 2.9 | 0.9 | 2.8 | 1.7 | 2.8 | 5.1 | 1.7 | 1.4 | 1.4 | 1.5 | 2.1 | 5.2 | 1.8 | 2.0 | 2.7 | 3.1 | 3.7 | 0.62 |
| YMR251W | 1.3 | 1.3 | 1.8 | 0.6 | 2.2 | 1.6 | 3.1 | 1.7 | 3.1 | 11.6 | 2.8 | 1.2 | 2.5 | 1.5 | 0.4 | 1.3 | 1.5 | 0.9 | 0.20 |
| YMR322C | 2.0 | 4.3 | 1.5 | 2.9 | 1.4 | 1.8 | 1.7 | 2.1 | 21.3 | 10.3 | 3.2 | 1.1 | 2.1 | 2.7 | 3.6 | 1.8 | 1.4 | 0.8 | 0.18 |
| YNL094W | 0.9 | 1.4 | 1.1 | 1.0 | 1.4 | 2.9 | 1.2 | 2.0 | 3.5 | 2.6 | 1.7 | 1.2 | 3.4 | 1.0 | 1.5 | 1.5 | 1.1 | 1.3 | 0.66 |
| YNL134C | 2.3 | 6.0 | 4.5 | 0.9 | 1.7 | 1.4 | 1.5 | 3.2 | 6.1 | 5.9 | 5.0 | 1.1 | 3.1 | 1.4 | 0.8 | 2.1 | 1.3 | 2.0 | 2.51 |
| YNL155W | 1.1 | 1.2 | 1.1 | 0.9 | 1.4 | 3.0 | 1.3 | 1.7 | 5.8 | 2.7 | 2.2 | 1.1 | 4.4 | 2.1 | 2.0 | 2.0 | 1.1 | 1.6 | 1.89 |
| YOR059C | 1.2 | 1.2 | 0.4 | 0.9 | 1.4 | 1.1 | 2.2 | 1.3 | 7.6 | 4.6 | 1.0 | 1.0 | 3.3 | 2.2 | 3.5 | 1.8 | 1.3 | 1.7 | 0.71 |
| YOR152C | 4.5 | 2.5 | 3.4 | 2.9 | 0.8 | 4.5 | 3.2 | 1.2 | 10.0 | 5.0 | 2.7 | 2.9 | 7.4 | 1.7 | 3.6 | 2.1 | 4.0 | 2.2 | 0.42 |
| YOR173W | 0.7 | 2.3 | 6.3 | 2.0 | 0.9 | 3.5 | 4.9 | 1.4 | 5.2 | 3.7 | 2.3 | 1.9 | 3.8 | 2.8 | 0.7 | 5.7 | 1.7 | 3.8 | 0.54 |
| YOR289W | 1.6 | 1.5 | 1.0 | 2.0 | 1.2 | 4.2 | 6.1 | 2.4 | 2.4 | 1.7 | 2.6 | 1.5 | 4.3 | 2.0 | 1.6 | 3.0 | 2.3 | 3.9 | 0.74 |
| YPR030W | 1.7 | 1.4 | 1.4 | 1.0 | 1.2 | 1.3 | 1.2 | 0.9 | 2.5 | 2.4 | 1.2 | 1.4 | 3.2 | 1.5 | 1.5 | 3.1 | 1.4 | 2.1 | 0.30 |
| YAL008W | 1.4 | 2.4 | 2.0 | 2.4 | 0.7 | 1.6 | 1.9 | 1.3 | 1.8 | 1.5 | 2.2 | 1.3 | 2.2 | 2.6 | 0.9 | 4.2 | 1.4 | 1.9 | 1.04 |
| YBR053C | 1.0 | 1.6 | 2.4 | 2.4 | 1.6 | 2.7 | 4.1 | 1.2 | 2.9 | 2.1 | 2.3 | 1.1 | 2.4 | 1.0 | 0.7 | 2.8 | 1.9 | 2.4 | 1.25 |
| YBR099C | 1.0 | 1.4 | 7.2 | 1.8 | 1.0 | 0.5 | 0.9 | 1.5 | 3.4 | 12.9 | 0.4 | 1.0 | 2.1 | 1.6 | 0.8 | 0.5 | 0.8 | 0.9 | 0.47 |
| YBR137W | 1.1 | 2.2 | 1.4 | 3.5 | 0.9 | 2.4 | 2.7 | 2.0 | 2.2 | 1.8 | 2.4 | 1.4 | 2.1 | 1.8 | 0.6 | 2.8 | 1.4 | 2.6 | 1.40 |
| YBR203W | 1.4 | 1.4 | 1.6 | 2.4 | 0.9 | 0.6 | 0.9 | 1.3 | 1.1 | 1.8 | 1.3 | 1.6 | 2.4 | 1.3 | 1.0 | 18.0 | 1.4 | 2.2 | 0.38 |
| YCL049C | 1.9 | 1.3 | 1.4 | 1.2 | 1.6 | 2.3 | 2.1 | 1.0 | 2.0 | 1.9 | 1.6 | 1.4 | 2.4 | 1.8 | 1.4 | 2.1 | 1.4 | 1.4 | 0.86 |
| YCR082W | 1.4 | 1.2 | 0.7 | 1.2 | 1.3 | 1.3 | 1.5 | 1.5 | 4.3 | 2.3 | 2.0 | 1.0 | 2.0 | 2.4 | 1.6 | 2.2 | 1.2 | 1.7 | 1.62 |
| YDL054C | 1.3 | 2.2 | 2.7 | 1.1 | 1.2 | 1.2 | 0.9 | 0.7 | 3.5 | 2.1 | 2.1 | 1.1 | 2.7 | 1.2 | 1.0 | 1.0 | 1.0 | 1.0 | 0.92 |
| YDL115C | 1.4 | 1.0 | 0.5 | 1.8 | 0.8 | 1.5 | 1.8 | 1.6 | 6.6 | 3.6 | 1.8 | 1.1 | 1.9 | 2.3 | 1.2 | 1.6 | 1.3 | 1.8 | 0.83 |
| YDL144C | 0.8 | 0.9 | 1.2 | 1.5 | 1.0 | 1.8 | 1.0 | 0.9 | 3.6 | 1.2 | 1.9 | 1.0 | 2.1 | 1.2 | 1.0 | 1.1 | 0.9 | 0.9 | 0.52 |
| YDL223C | 0.7 | 1.1 | 5.0 | 2.5 | 0.9 | 0.8 | 1.0 | 0.3 | 3.8 | 1.5 | 5.2 | 1.0 | 3.1 | 1.3 | 3.1 | 7.2 | 0.9 | 1.0 | 0.32 |
| YDR032C | 1.7 | 2.2 | 3.5 | 2.5 | 1.4 | 2.7 | 2.8 | 2.4 | 3.1 | 2.9 | 3.3 | 1.8 | 2.9 | 2.8 | 1.3 | 3.8 | 1.9 | 3.5 | 3.50 |

| | | | | | | | | | | | | | | | | | | | |
|---|---|---|---|---|---|---|---|---|---|---|---|---|---|---|---|---|---|---|---|
| YDR330W | 0.8 | 1.5 | 1.2 | 0.8 | 1.2 | 2.3 | 1.3 | 1.1 | 3.9 | 2.6 | 0.9 | 1.0 | 2.2 | 1.1 | 1.6 | 1.4 | 1.5 | 1.3 | 0.53 |
| YDR411C | 0.9 | 2.8 | 1.8 | 1.2 | 1.6 | 0.8 | 2.0 | 0.9 | 0.7 | 1.0 | 1.3 | 1.5 | 1.9 | 0.8 | 4.1 | 1.5 | 1.5 | 1.3 | 0.56 |
| YDR511W | 1.3 | 1.7 | 0.9 | 2.3 | 1.0 | 2.0 | 1.5 | 1.7 | 2.5 | 2.2 | 1.2 | 0.8 | 3.1 | 2.2 | 0.8 | 2.2 | 1.3 | 1.5 | 0.66 |
| YDR545W | 0.6 | 1.2 | 0.6 | 0.7 | 1.4 | 1.0 | 1.2 | 0.6 | 1.8 | 0.7 | 0.7 | 0.9 | 2.0 | 0.7 | 0.9 | 0.9 | 0.9 | 0.9 | 1.06 |
| YER004W | 1.0 | 1.5 | 1.9 | 1.4 | 1.5 | 1.2 | 1.4 | 1.1 | 4.5 | 2.9 | 2.2 | 1.7 | 4.6 | 1.8 | 1.4 | 2.3 | 1.4 | 1.2 | 1.48 |
| YER035W | 1.4 | 1.6 | 1.6 | 1.9 | 1.5 | 0.9 | 1.9 | 1.2 | 1.2 | 1.4 | 1.4 | 1.1 | 1.9 | 1.0 | 0.9 | 2.1 | 2.2 | 2.1 | 0.85 |
| YER079W | 1.1 | 1.6 | 1.9 | 2.0 | 0.8 | 1.5 | 1.9 | 1.4 | 5.3 | 3.4 | 2.1 | 0.9 | 2.1 | 1.6 | 0.5 | 1.4 | 1.0 | 1.6 | 0.69 |
| YER158C | 1.2 | 0.9 | 0.9 | 3.1 | 0.8 | 0.7 | 1.2 | 0.9 | 2.1 | 1.6 | 2.3 | 1.0 | 3.1 | 0.9 | 0.6 | 1.6 | 1.6 | 1.2 | 0.61 |
| YER163C | 1.1 | 2.4 | 1.2 | 1.6 | 0.8 | 2.0 | 1.5 | 1.4 | 5.4 | 3.4 | 1.6 | 1.0 | 2.9 | 1.9 | 1.6 | 2.4 | 1.2 | 1.2 | 0.62 |
| YER175C | 0.9 | 1.2 | 1.3 | 2.2 | 0.9 | 0.8 | 2.3 | 1.1 | 1.9 | 11.5 | 1.1 | 0.9 | 4.3 | 1.1 | 1.3 | 2.8 | 0.9 | 1.2 | 0.53 |
| YFL006W | 0.8 | 2.8 | 1.6 | 1.0 | 1.4 | 1.2 | 1.0 | 1.0 | 3.2 | 1.7 | 1.2 | 1.2 | 3.1 | 1.7 | 2.4 | 1.1 | 1.0 | 0.8 | 0.82 |
| YFL010C | 1.0 | 4.8 | 1.3 | 1.0 | 1.3 | 1.9 | 1.5 | 1.0 | 3.1 | 1.4 | 1.4 | 1.5 | 3.3 | 1.4 | 1.7 | 1.4 | 1.9 | 1.6 | 0.77 |
| YFL032W | 0.4 | 1.5 | 1.6 | 0.8 | 0.9 | 0.6 | 1.1 | 0.4 | 0.5 | 0.6 | 1.1 | 0.7 | 2.4 | 0.6 | 2.0 | 0.7 | 1.7 | 0.8 | 1.62 |
| YGL037C | 1.5 | 2.7 | 6.5 | 2.1 | 0.9 | 1.3 | 2.9 | 1.2 | 2.1 | 1.6 | 3.9 | 0.8 | 2.3 | 1.0 | 0.8 | 1.5 | 1.3 | 2.6 | 4.58 |
| YGL047W | 1.0 | 1.9 | 1.6 | 2.3 | 1.3 | 1.5 | 1.7 | 1.1 | 4.5 | 5.7 | 1.9 | 1.1 | 2.6 | 2.3 | 1.7 | 2.9 | 1.1 | 1.2 | 0.97 |
| YGL053W | 1.5 | 1.5 | 1.2 | 2.5 | 2.0 | 2.7 | 3.7 | 1.2 | 3.3 | 2.4 | 1.3 | 1.3 | 3.3 | 1.1 | 1.1 | 3.9 | 2.2 | 3.7 | 1.26 |
| YGL199C | 1.1 | 1.7 | 1.7 | 1.0 | 0.7 | 0.7 | 1.3 | 0.7 | | 0.1 | | 0.9 | 2.0 | 0.8 | 1.6 | 1.6 | 1.2 | 1.0 | 0.36 |
| YGR101W | 1.0 | 1.3 | 1.2 | 1.1 | 1.2 | 0.9 | 1.5 | 0.8 | 4.0 | 1.4 | 2.0 | 1.0 | 2.4 | 0.9 | 1.1 | 2.0 | 1.0 | 1.1 | 1.04 |
| YGR130C | 0.9 | 0.7 | 0.6 | 1.6 | 1.4 | 0.9 | 1.3 | 0.9 | 2.0 | 1.5 | 0.8 | 0.7 | 1.9 | 0.9 | 1.2 | 2.2 | 1.3 | 1.6 | 0.78 |
| YGR154C | 1.8 | 2.9 | 2.2 | 0.5 | 1.2 | 1.4 | 2.0 | 1.7 | 25.2 | 9.1 | 1.5 | 0.9 | 2.5 | 2.2 | 0.7 | 2.0 | 1.0 | 1.2 | 0.36 |
| YGR221C | 0.9 | 2.2 | 0.9 | 1.4 | 0.9 | 3.0 | 1.2 | 1.1 | | 0.7 | 0.5 | 1.6 | 2.3 | 1.4 | 1.5 | 2.7 | 1.3 | 1.3 | 0.29 |
| YHR138C | 2.8 | 1.8 | 2.2 | 2.8 | 1.5 | 3.8 | 3.0 | 1.4 | 5.3 | 4.6 | 1.6 | 1.8 | 4.0 | 3.4 | 2.2 | 3.3 | 5.0 | 7.1 | 1.89 |
| YHR199C | 1.9 | 2.0 | 3.2 | 2.0 | 1.2 | 1.1 | 1.1 | 1.0 | 8.6 | 6.0 | 1.9 | 1.1 | 2.1 | 2.0 | 2.8 | 3.3 | 1.1 | 0.9 | 0.63 |
| YIL041W | 1.0 | 1.2 | 2.1 | 1.3 | 0.5 | 1.0 | 0.8 | 0.9 | 1.8 | 1.4 | 1.4 | 1.0 | 2.3 | 1.3 | 1.3 | 0.7 | 0.8 | 1.1 | 1.33 |
| YIL087C | 1.3 | 1.9 | 3.4 | 1.6 | 1.4 | 4.1 | 1.8 | 1.1 | 2.4 | 1.8 | 2.8 | 1.1 | 2.4 | 1.3 | 0.4 | 5.6 | 1.7 | 1.6 | 0.68 |
| YJL057C | 1.5 | 1.2 | 1.6 | 1.7 | 1.5 | 1.4 | 2.7 | 1.5 | 7.3 | 6.1 | 2.0 | 1.0 | 2.5 | 1.6 | 1.4 | 1.9 | 1.4 | 1.5 | 0.51 |
| YJL066C | 0.9 | 1.2 | 1.2 | 2.5 | 0.8 | 1.4 | 1.6 | 0.8 | 2.0 | 2.1 | 0.8 | 1.0 | 2.2 | 1.0 | 1.3 | 2.8 | 1.4 | 1.5 | 0.43 |
| YJL082W | 1.4 | 0.8 | 2.4 | 1.1 | 1.7 | 0.2 | 0.4 | 0.9 | 1.4 | 2.9 | 1.0 | 1.3 | 2.1 | 0.5 | 3.2 | 0.2 | 0.6 | 0.3 | 2.07 |
| YJL151C | 1.0 | 2.4 | 3.2 | 1.2 | 1.3 | 2.2 | 1.4 | 0.8 | 0.5 | 1.6 | 1.0 | 1.3 | 2.4 | 1.3 | 1.0 | 2.5 | 3.5 | 2.4 | 1.69 |
| YJL152W | 0.9 | 1.5 | 1.8 | 1.3 | 1.6 | 1.5 | 1.2 | 0.7 | 0.3 | 1.0 | 1.0 | 1.0 | 1.8 | 1.4 | 0.8 | 1.6 | 2.5 | 1.7 | 0.80 |
| YJL161W | 1.7 | 2.2 | 1.5 | 4.6 | 1.1 | 8.0 | 3.6 | 1.4 | 3.1 | 2.1 | 3.7 | 1.0 | 2.5 | 2.2 | 0.6 | 4.4 | 1.9 | 2.1 | 0.44 |
| YJL171C | 2.3 | 0.7 | 2.8 | 1.4 | 1.0 | 2.7 | 1.4 | 0.9 | 1.0 | 1.7 | 2.2 | 1.0 | 2.9 | 1.0 | 1.8 | 1.0 | 3.6 | 2.9 | 1.94 |

| | | | | | | | | | | | | | | | | | | | |
|---|---|---|---|---|---|---|---|---|---|---|---|---|---|---|---|---|---|---|---|
| YJR008W | 1.6 | 1.5 | 1.2 | 1.7 | 1.4 | 2.2 | 2.9 | 1.1 | 1.8 | 1.9 | 1.4 | 1.3 | 2.0 | 1.2 | 0.9 | 3.3 | 1.5 | 2.7 | 0.87 |
| YKL151C | 1.2 | 3.1 | 1.7 | 2.5 | 1.0 | 2.0 | 5.6 | 1.0 | 3.3 | 3.5 | 2.3 | 1.0 | 2.0 | 1.3 | 1.4 | 3.7 | 1.4 | 1.8 | 1.02 |
| YKL153W | 1.1 | 1.4 | 2.0 | 0.7 | 1.0 | 2.9 | 1.1 | 0.7 | 0.8 | 1.4 | 2.1 | 1.1 | 2.4 | 1.1 | 1.5 | 1.1 | 0.9 | 1.3 | 1.60 |
| YKL195W | 0.8 | 0.5 | 0.4 | 1.1 | 1.9 | 2.5 | 2.1 | 1.2 | 3.6 | 2.1 | 1.0 | 1.1 | 2.4 | 1.6 | 0.9 | 1.7 | 1.4 | 1.6 | 1.58 |
| YLR054C | 2.0 | 1.5 | 0.4 | 2.4 | 1.3 | 1.2 | 1.1 | 1.1 | 2.7 | 1.1 | 0.7 | 1.1 | 3.2 | 1.1 | 0.7 | 0.9 | 1.6 | 1.1 | 0.14 |
| YLR149C | 1.4 | 1.9 | 3.4 | 2.3 | 0.8 | 1.4 | 1.6 | 1.1 | 6.2 | 5.2 | 2.8 | 2.0 | 3.0 | 1.2 | 1.3 | 2.8 | 1.3 | 3.0 | 0.33 |
| YLR152C | 0.7 | 1.3 | 1.1 | 0.9 | 1.1 | 2.1 | 1.4 | 1.2 | 0.6 | 1.1 | 3.6 | 1.0 | 2.8 | 1.2 | 0.8 | 2.8 | 1.5 | 1.8 | 0.46 |
| YLR324W | 0.7 | 1.2 | 0.9 | 1.0 | 1.3 | 1.2 | 1.0 | 1.0 | 3.1 | 1.4 | 0.9 | 0.8 | 2.3 | 1.4 | 1.6 | 1.7 | 1.2 | 1.2 | 0.49 |
| YLR350W | 1.2 | 1.9 | 3.2 | 2.3 | 0.8 | 1.6 | 1.2 | 1.0 | 0.6 | 1.4 | 0.9 | 1.8 | 4.5 | 2.8 | 1.6 | 2.9 | 1.6 | 1.1 | 1.52 |
| YLR387C | 0.9 | 1.6 | 0.8 | 0.6 | 1.3 | 1.9 | 1.1 | 1.4 | 7.8 | 3.0 | 0.9 | 1.1 | 3.6 | 1.7 | 1.9 | 1.0 | 1.0 | 1.4 | 1.20 |
| YML117W | 0.9 | 2.2 | 0.9 | 1.6 | 0.8 | 1.0 | 0.7 | 0.7 | 1.0 | 1.4 | 0.8 | 0.8 | 2.0 | 0.8 | 2.8 | 0.7 | 1.1 | 1.3 | 0.70 |
| YMR009W | 2.3 | 1.9 | 0.5 | 1.9 | 1.9 | 1.2 | 1.9 | 1.4 | 3.4 | 2.2 | 2.1 | 0.6 | 2.3 | 1.9 | 0.6 | 2.3 | 2.7 | 2.5 | 0.75 |
| YMR067C | 0.9 | 0.9 | 1.1 | 0.4 | 1.1 | 1.4 | 1.2 | 1.1 | 4.0 | 3.5 | 1.1 | 1.1 | 2.2 | 1.5 | 1.4 | 1.0 | 1.0 | 1.0 | 0.49 |
| YMR097C | 1.5 | 1.0 | 0.7 | 1.6 | 0.7 | 1.2 | 1.5 | 1.5 | 2.1 | 2.1 | 1.5 | 1.2 | 2.1 | 2.1 | 1.7 | 1.7 | 1.4 | 1.8 | 0.59 |
| YMR102C | 1.0 | 2.9 | 1.1 | 0.8 | 1.2 | 6.3 | 1.8 | 1.0 | 1.7 | 2.6 | 1.5 | 2.4 | 4.4 | 0.7 | 0.7 | 1.1 | 2.4 | 2.8 | 0.94 |
| YMR107W | 5.0 | 3.9 | 1.7 | 18.4 | 1.1 | 1.0 | 1.2 | 1.3 | 6.0 | 17.1 | 3.0 | 0.8 | 1.8 | 8.1 | 1.9 | 12.0 | 1.3 | 8.1 | 0.49 |
| YMR180C | 1.2 | 0.9 | 1.0 | 1.3 | 1.0 | 2.4 | 1.7 | 1.8 | 1.8 | 2.2 | 0.7 | 1.2 | 3.1 | 1.7 | 1.0 | 1.8 | 1.4 | 1.7 | 0.57 |
| YMR184W | 1.3 | 5.4 | 1.0 | 1.1 | 0.8 | 0.9 | 1.6 | 1.9 | 2.1 | 0.9 | 1.5 | 1.7 | 2.3 | 1.5 | 1.1 | 1.2 | 1.4 | 2.0 | 0.68 |
| YMR191W | 1.3 | 2.4 | 1.9 | 1.6 | 1.5 | 1.0 | 2.0 | 1.1 | 2.2 | 3.5 | 2.3 | 1.8 | 3.4 | 1.5 | 2.7 | 1.7 | 1.2 | 2.1 | 0.99 |
| YMR295C | 1.7 | 1.0 | 1.2 | 1.6 | 1.4 | 1.9 | 1.3 | 1.0 | 1.0 | 0.8 | 1.2 | 1.1 | 2.3 | 1.3 | 0.9 | 1.9 | 2.1 | 3.4 | 3.43 |
| YMR316W | 3.2 | 2.9 | 1.1 | 1.9 | 1.1 | 0.7 | 2.0 | 1.6 | 2.1 | 3.0 | 1.5 | 1.9 | 2.9 | 1.3 | 0.5 | 1.3 | 6.9 | 6.4 | 1.25 |
| YNL044W | 1.3 | 0.9 | 2.3 | 1.3 | 1.2 | 0.9 | 0.9 | 1.8 | 0.8 | 0.8 | 1.1 | 1.0 | 3.1 | 1.2 | 2.4 | 1.0 | 3.1 | 3.4 | 2.56 |
| YNL074C | 0.9 | 1.3 | 1.8 | 1.2 | 1.3 | 1.7 | 1.1 | 0.6 | 1.5 | 2.0 | 1.3 | 0.9 | 2.3 | 0.6 | 1.0 | 0.7 | 1.1 | 1.3 | 0.68 |
| YNL092W | 1.3 | 1.4 | 2.4 | 1.8 | 0.9 | 1.2 | 1.6 | 0.9 | 2.3 | 1.5 | 0.7 | 1.1 | 2.3 | 1.2 | 2.7 | 1.3 | 1.7 | 1.6 | 0.16 |
| YNL115C | 0.7 | 1.8 | 1.6 | 1.4 | 0.8 | 9.1 | 2.4 | 0.8 | 2.6 | 1.9 | 1.1 | 0.8 | 2.5 | 1.0 | 1.6 | 3.1 | 1.6 | 2.6 | 0.80 |
| YNL156C | 1.1 | 1.2 | 0.9 | 1.3 | 1.6 | 1.3 | 1.9 | 1.2 | 2.4 | 1.9 | 1.4 | 0.9 | 2.0 | 1.4 | 1.8 | 2.1 | 1.8 | 1.9 | 1.85 |
| YNL234W | 1.1 | 1.1 | 0.6 | 1.8 | 1.7 | 1.3 | 1.2 | 1.3 | 2.6 | 3.3 | 1.3 | 1.1 | 2.5 | 1.5 | 1.4 | 2.6 | 1.0 | 1.0 | 0.49 |
| YNL281W | 1.0 | 1.3 | 2.0 | 1.4 | 1.7 | 0.9 | 1.2 | 1.2 | 2.8 | 1.6 | 1.6 | 0.7 | 2.0 | 1.2 | 1.2 | 0.5 | 0.9 | 0.9 | 1.79 |
| YNL305C | 0.9 | 2.2 | 2.7 | 1.4 | 1.3 | 1.1 | 1.7 | 0.7 | 2.9 | 2.1 | 1.8 | 1.1 | 2.6 | 1.3 | 1.6 | 2.5 | 1.7 | 1.6 | 1.35 |
| YNR068C | 1.2 | 1.5 | 2.3 | 2.6 | 1.7 | 0.4 | 1.8 | 1.1 | 6.7 | 14.4 | 1.1 | 1.3 | 3.7 | 1.8 | 1.0 | 1.4 | 1.0 | 1.0 | 0.24 |
| YOL032W | 1.3 | 0.8 | 2.0 | 1.3 | 1.4 | 2.1 | 1.4 | 1.5 | 4.5 | 3.0 | 1.4 | 1.0 | 2.3 | 1.3 | 0.9 | 2.4 | 1.7 | 1.8 | 0.77 |
| YOL036W | 0.8 | 0.9 | 1.3 | 0.8 | 1.2 | 1.2 | 1.0 | 0.8 | 3.1 | 2.8 | 2.2 | 1.0 | 2.5 | 1.0 | 2.0 | 1.0 | 1.1 | 0.9 | 0.48 |

| | | | | | | | | | | | | | | | | | | |
|---|---|---|---|---|---|---|---|---|---|---|---|---|---|---|---|---|---|---|
| YOL047C | 1.4 | 1.1 | 1.1 | 2.5 | 1.4 | 1.5 | 1.3 | 1.1 | 0.6 | 2.3 | 0.9 | 0.8 | 2.0 | 1.2 | 12.3 | 1.3 | 1.5 | 1.0 | 0.19 |
| YOL071W | 1.3 | 2.0 | 1.7 | 2.0 | 0.9 | 1.7 | 2.0 | 1.6 | 4.4 | 2.3 | 4.3 | 1.0 | 2.5 | 2.5 | 0.7 | 4.6 | 1.3 | 2.0 | 1.08 |
| YOL082W | 1.0 | 1.6 | 1.6 | 2.1 | 1.5 | 1.8 | 2.5 | 1.1 | 3.4 | 2.0 | 1.6 | 1.0 | 2.4 | 1.4 | 1.0 | 2.4 | 1.6 | 2.5 | 0.60 |
| YOL083W | 1.1 | 2.3 | 1.2 | 2.8 | 1.4 | 4.5 | 3.1 | 1.3 | 2.7 | 2.3 | 1.3 | 1.2 | 2.3 | 1.4 | 1.0 | 3.8 | 1.4 | 3.2 | 0.41 |
| YOL117W | 1.0 | 1.1 | 0.5 | 1.4 | 1.7 | 2.0 | 1.6 | 1.1 | 1.3 | 4.1 | 1.0 | 0.9 | 2.2 | 1.7 | 1.1 | 2.2 | 1.3 | 1.4 | 0.21 |
| YOL131W | 1.0 | 1.5 | 0.6 | 1.0 | 1.5 | 1.1 | 1.3 | 1.1 | 8.4 | 8.1 | 1.4 | 0.9 | 3.1 | 2.3 | 1.4 | 0.7 | 1.1 | 0.9 | 0.15 |
| YOL162W | 2.0 | 6.9 | 1.7 | 1.4 | 0.9 | 1.2 | 2.0 | 1.6 | 12.0 | 6.8 | 4.3 | 1.4 | 3.8 | 1.4 | 0.6 | 0.9 | 1.2 | 0.9 | 0.26 |
| YOR019W | 2.9 | 1.2 | 0.8 | 4.7 | 1.4 | 1.3 | 2.8 | 1.1 | 3.5 | 7.1 | 1.7 | 1.0 | 2.2 | 1.4 | 0.7 | 2.6 | 1.7 | 2.8 | 0.40 |
| YOR197W | 0.8 | 1.0 | 1.1 | 1.4 | 0.6 | 1.3 | 0.9 | 0.9 | 0.8 | 0.7 | 1.0 | 1.0 | 2.1 | 0.7 | 1.7 | 1.0 | 1.5 | 1.5 | 1.30 |
| YPL087W | 1.0 | 5.1 | 3.0 | 7.3 | 0.6 | 4.3 | 4.2 | 1.0 | 1.0 | 2.2 | 2.5 | 1.8 | 4.4 | 1.2 | 1.3 | 1.8 | 2.1 | 2.7 | 1.49 |
| YPL196W | 1.3 | 1.6 | 1.3 | 1.7 | 1.5 | 2.0 | 2.4 | 1.2 | 10.9 | 2.8 | 1.7 | 0.9 | 2.1 | 1.6 | 0.7 | 4.3 | 1.4 | 2.3 | 0.92 |
| YPL206C | 1.0 | 2.9 | 1.4 | 1.9 | 0.7 | 1.8 | 1.2 | 1.0 | 1.7 | 1.1 | 1.5 | 0.8 | 2.5 | 1.1 | 0.8 | 1.2 | 1.4 | 1.9 | 1.22 |
| YPL222W | 0.9 | 3.5 | 0.8 | 1.3 | 1.2 | 2.2 | 1.1 | 0.7 | 7.4 | 3.4 | 1.2 | 1.3 | 3.0 | 1.1 | 0.9 | 2.2 | 1.1 | 0.9 | 0.26 |
| YPR023C | 1.2 | 1.4 | 1.2 | 0.8 | 1.1 | 1.4 | 1.2 | 0.9 | 4.4 | 2.8 | 1.7 | 0.9 | 2.0 | 0.9 | 0.8 | 1.4 | 1.0 | 1.1 | 0.81 |
| YPR151C | 2.3 | 2.0 | 1.5 | 4.1 | 1.3 | 1.6 | 4.4 | 1.4 | 3.0 | 9.5 | 1.4 | 0.9 | 2.0 | 1.4 | 0.7 | 2.8 | 1.3 | 1.4 | 0.16 |
| YDL222C | 0.9 | 1.1 | 1.5 | 3.9 | 1.0 | 1.4 | 1.0 | 0.7 | 1.9 | 1.0 | 2.6 | 1.0 | 2.2 | 1.3 | 7.1 | 6.5 | 0.9 | 1.3 | 0.50 |
| YEL001C | 1.0 | 1.0 | 1.4 | 0.7 | 0.9 | 1.3 | 1.1 | 1.2 | 2.0 | 1.2 | 1.2 | 1.1 | 0.9 | 1.3 | 3.0 | 1.2 | 0.7 | 1.3 | 2.81 |
| YER106W | 1.3 | 0.7 | 1.3 | 1.2 | 1.6 | 1.1 | 1.0 | 1.2 | 0.4 | 1.8 | 1.0 | 0.9 | 1.0 | 1.5 | 6.8 | 0.9 | 0.8 | 0.8 | 0.25 |
| YIL023C | 1.1 | 1.2 | 2.1 | 1.8 | 1.3 | 0.9 | 1.2 | 0.7 | 3.5 | 1.6 | 1.1 | 0.9 | 1.1 | 0.7 | 5.6 | 1.1 | 1.2 | 1.0 | 0.93 |
| YJR054W | 0.8 | 0.9 | 0.3 | 0.9 | 1.0 | 0.7 | 0.9 | 1.6 | | 0.6 | 0.7 | 0.9 | 0.4 | 1.2 | 3.1 | 0.6 | 0.9 | 0.9 | 0.52 |
| YKL086W | 1.0 | 1.1 | 2.4 | 2.6 | 0.8 | 1.0 | 0.7 | 1.1 | 8.2 | 20.5 | 0.7 | 0.6 | 1.3 | 2.9 | 5.0 | 0.6 | 0.9 | 1.0 | 0.28 |
| YKR091W | 1.5 | 2.3 | 0.9 | 3.5 | 1.6 | 1.0 | 1.3 | 1.3 | 1.0 | 1.6 | 1.1 | 0.9 | 1.0 | 1.9 | 6.3 | 1.6 | 1.6 | 1.3 | 0.45 |
| YLR194C | 2.1 | 2.2 | 1.3 | 3.6 | 1.0 | 0.9 | 1.0 | 1.0 | 0.9 | 1.1 | 1.4 | 1.6 | 4.0 | 1.2 | 3.6 | 0.9 | 1.0 | 0.9 | 0.60 |
| YMR095C | 1.1 | 2.0 | 1.6 | 1.0 | 0.9 | 0.7 | 1.2 | 1.2 | | 1.4 | 2.1 | 0.8 | 3.4 | 2.2 | 41.7 | 1.0 | 1.0 | 1.0 | 0.23 |
| YAL053W | 0.9 | 0.9 | 1.3 | 2.1 | 0.8 | 1.1 | 0.7 | 0.8 | 1.4 | 0.9 | 2.3 | 0.6 | 1.1 | 0.7 | 2.9 | 1.1 | 1.2 | 1.6 | 1.60 |
| YDL072C | 1.3 | 1.1 | 3.5 | 2.1 | 1.4 | 1.5 | 1.8 | 0.8 | 1.1 | 1.5 | 1.4 | 1.2 | 1.6 | 1.8 | 2.6 | 2.6 | 1.6 | 2.8 | 3.25 |
| YDL204W | 1.4 | 1.9 | 4.8 | 3.4 | 1.0 | 5.6 | 3.2 | 1.1 | 2.3 | 2.0 | 3.2 | 0.9 | 3.2 | 2.4 | 3.6 | 8.6 | 1.8 | 2.7 | 0.73 |
| YDR391C | 1.2 | 2.0 | 1.3 | 1.6 | 1.2 | 2.8 | 2.0 | 1.4 | 1.5 | 2.7 | 1.9 | 1.4 | 1.8 | 2.3 | 3.5 | 3.0 | 1.8 | 2.4 | 1.05 |
| YIL024C | 1.4 | 1.3 | 1.1 | 2.1 | 1.3 | 1.0 | 1.3 | 1.1 | 2.2 | 1.8 | 0.6 | 0.7 | 1.0 | 1.5 | 3.1 | 1.0 | 1.1 | 1.0 | 0.35 |
| YIL117C | 2.5 | 1.2 | 1.0 | 3.3 | 0.8 | 1.2 | 1.5 | 1.8 | 2.3 | 3.5 | 1.8 | 1.4 | 1.3 | 2.6 | 7.5 | 1.0 | 2.6 | 2.6 | 1.22 |
| YJL108C | 1.2 | 1.2 | 1.0 | 2.2 | 1.5 | 0.6 | 0.6 | 1.0 | 0.3 | 0.7 | 1.0 | 0.4 | 0.5 | 1.0 | 4.0 | 0.6 | 1.0 | 0.9 | 0.40 |
| YJL149W | 1.2 | 1.8 | 1.5 | 6.8 | 0.8 | 0.7 | 1.3 | 1.0 | 1.5 | 3.8 | 1.5 | 1.1 | 1.9 | 1.1 | 9.0 | 1.4 | 0.8 | 1.2 | 0.28 |

| | | | | | | | | | | | | | | | | | | | |
|---|---|---|---|---|---|---|---|---|---|---|---|---|---|---|---|---|---|---|---|
| YJL186W | 0.6 | 0.9 | 1.2 | 0.8 | 0.7 | 0.7 | 0.6 | 0.6 | 0.2 | 0.5 | 0.6 | 0.6 | 0.4 | 0.8 | 2.5 | 1.0 | 0.7 | 0.8 | 1.07 |
| YNL190W | 1.0 | 1.4 | 2.6 | 0.9 | 1.0 | 0.5 | 0.8 | 0.8 | 1.1 | 1.1 | 1.0 | 1.0 | 1.3 | 0.6 | 2.6 | 1.3 | 0.7 | 0.8 | 4.72 |
| YNL208W | 1.5 | 3.2 | 2.9 | 2.1 | 1.2 | 1.4 | 0.9 | 1.5 | 2.3 | 3.1 | 2.5 | 0.8 | 1.7 | 1.0 | 2.6 | 0.9 | 1.0 | 1.0 | 1.55 |
| YNL300W | 0.7 | 0.3 | 2.9 | 1.5 | 0.9 | 0.4 | 0.4 | 0.9 | | 0.2 | 0.9 | 0.8 | 0.7 | 0.8 | 3.3 | 1.0 | 1.0 | 0.8 | 0.39 |
| YNR064C | 0.9 | 0.6 | 0.9 | 1.8 | 1.5 | 2.1 | 0.8 | 0.9 | | 2.1 | 0.7 | 0.7 | 1.6 | 0.8 | 6.1 | 1.4 | 1.1 | 1.9 | 2.17 |
| YOR248W | 2.4 | 0.7 | 2.9 | 1.3 | 0.7 | 0.2 | 0.3 | 0.7 | 0.1 | 0.4 | 2.3 | 0.5 | 0.4 | 0.5 | 8.8 | 0.3 | 1.0 | 0.8 | 2.47 |
| YPL052W | 1.8 | 1.7 | 0.4 | 1.0 | 1.7 | 1.9 | 1.4 | 1.7 | 2.0 | 4.8 | 1.2 | 1.1 | 1.6 | 3.2 | 3.3 | 1.1 | 1.3 | 1.5 | 0.88 |
| YPR079W | 1.0 | 1.8 | 1.3 | 1.5 | 0.9 | 1.0 | 2.3 | 1.1 | 1.7 | 2.8 | 0.8 | 1.0 | 1.3 | 1.3 | 2.9 | 1.9 | 1.4 | 1.5 | 0.42 |
| YAR028W | 1.2 | 1.0 | 0.7 | 1.2 | 0.9 | 3.3 | 1.8 | 1.3 | 1.0 | 0.9 | 0.9 | 0.8 | 1.4 | 1.2 | 0.9 | 1.5 | 1.9 | 2.1 | 1.40 |
| YDR031W | 1.5 | 1.1 | 1.7 | 1.5 | 1.2 | 3.6 | 1.9 | 1.4 | 1.1 | 2.7 | 1.2 | 0.9 | 1.3 | 2.5 | 0.8 | 2.4 | 1.3 | 2.5 | 1.23 |
| YDR486C | 1.1 | 1.5 | 1.2 | 0.9 | 1.2 | 2.7 | 1.8 | 1.8 | 2.5 | 5.8 | 1.4 | 1.3 | 1.7 | 2.4 | 1.1 | 2.6 | 1.1 | 1.7 | 1.18 |
| YER038C | 1.2 | 1.4 | 0.6 | 1.2 | 1.4 | 2.8 | 1.5 | 1.1 | 0.7 | 2.5 | 0.6 | 0.9 | 2.2 | 1.3 | 0.8 | 1.6 | 1.2 | 1.4 | 0.51 |
| YGL136C | 1.1 | 1.0 | 1.2 | 1.1 | 1.0 | 3.1 | 0.7 | 1.1 | 1.3 | 1.0 | 1.4 | 1.1 | 1.1 | 2.3 | 0.9 | 1.4 | 1.3 | 1.1 | 0.64 |
| YGR146C | 1.6 | 2.0 | 2.0 | 2.0 | 0.9 | 4.1 | 2.7 | 1.4 | 0.8 | 3.8 | 0.9 | 1.4 | 1.7 | 1.3 | 1.4 | 1.8 | 1.3 | 0.9 | 0.75 |
| YJL020C | 0.7 | 1.2 | 0.8 | 0.8 | 1.0 | 2.1 | 1.3 | 0.6 | 1.3 | 1.0 | 0.6 | 0.7 | 1.6 | 1.2 | 1.4 | 0.8 | 2.0 | 1.3 | 0.95 |
| YLR031W | 1.3 | 1.0 | 0.7 | 1.3 | 1.3 | 3.0 | 1.5 | 0.9 | 0.9 | 1.4 | 0.7 | 1.0 | 1.6 | 1.5 | 0.7 | 1.6 | 1.2 | 1.2 | 0.30 |
| YLR205C | 1.2 | 5.1 | 0.7 | 1.3 | 1.3 | 4.4 | 9.0 | 1.7 | 1.5 | 5.7 | 0.4 | 1.3 | 1.4 | 2.4 | 2.5 | 2.3 | 2.0 | 1.4 | 0.29 |
| YMR140W | 1.0 | 0.9 | 1.2 | 0.7 | 1.1 | 17.1 | 4.3 | 0.8 | 1.8 | 2.3 | 0.7 | 0.9 | 1.6 | 1.2 | 1.4 | 2.8 | 1.4 | 2.0 | 0.54 |
| YMR195W | 1.7 | 3.1 | 1.2 | 2.2 | 1.1 | 2.6 | 1.1 | 1.0 | 0.2 | 0.7 | 1.1 | 0.9 | 0.7 | 1.3 | 0.6 | 2.5 | 1.6 | 2.2 | 1.28 |
| YOR215C | 1.5 | 1.3 | 0.7 | 1.8 | 1.8 | 2.6 | 2.6 | 1.7 | 2.1 | 1.3 | 1.6 | 1.1 | 1.3 | 1.7 | 0.8 | 3.3 | 1.3 | 2.9 | 2.08 |
| YOR382W | 0.7 | 7.6 | 3.3 | 0.5 | 1.2 | 13.1 | 28.4 | 2.0 | 1.1 | 12.8 | 2.5 | 2.8 | 1.7 | 0.4 | 2.1 | 4.3 | 1.9 | 1.9 | 0.79 |
| YPL054W | 4.2 | 5.3 | 4.9 | 3.1 | 1.1 | 2.3 | 1.8 | 1.3 | 2.5 | 9.2 | 1.9 | 1.3 | 1.5 | 1.4 | 1.0 | 1.3 | 1.5 | 1.4 | 0.25 |
| YPR127W | 1.1 | 1.4 | 1.0 | 1.6 | 1.1 | 3.2 | 1.7 | 1.2 | 1.9 | 2.4 | 1.6 | 0.9 | 1.3 | 1.4 | 0.4 | 2.4 | 1.3 | 1.2 | 0.65 |
| YAR027W | 1.8 | 1.0 | 1.3 | 1.4 | 1.2 | 2.3 | 2.2 | 1.3 | 2.3 | 1.7 | 1.7 | 1.2 | 2.6 | 1.3 | 1.2 | 2.8 | 2.0 | 4.7 | 2.15 |
| YBL057C | 1.1 | 0.9 | 0.6 | 1.0 | 1.3 | 2.2 | 1.1 | 1.4 | 1.6 | 1.2 | 0.9 | 1.0 | 1.0 | 1.6 | 1.0 | 1.1 | 1.2 | 1.2 | 1.88 |
| YBR116C | 0.9 | 4.8 | 2.4 | 2.3 | 1.1 | 2.8 | 1.5 | 1.1 | 9.0 | 8.7 | 3.1 | 1.1 | 2.0 | 2.7 | 1.7 | 9.9 | 0.9 | 1.0 | 0.45 |
| YBR147W | 2.2 | 1.1 | 1.5 | 3.2 | 1.2 | 2.2 | 1.2 | 1.9 | 0.9 | 3.6 | 4.0 | 1.3 | 1.2 | 1.1 | 2.1 | 6.0 | 1.3 | 1.9 | 0.63 |
| YBR168W | 0.9 | 0.8 | 1.0 | 1.3 | 1.6 | 3.5 | 1.6 | 1.0 | 1.1 | 1.7 | 0.8 | 0.9 | 1.9 | 1.8 | 0.9 | 1.0 | 1.1 | 1.5 | 0.56 |
| YBR246W | 1.0 | 0.8 | 1.7 | 0.6 | 1.1 | 3.1 | 1.1 | 0.9 | 0.5 | 0.9 | 0.8 | 0.9 | 1.1 | 1.3 | 0.9 | 1.8 | 1.4 | 1.7 | 0.82 |
| YBR273C | 0.8 | 0.6 | 0.6 | 1.3 | 1.9 | 2.2 | 1.4 | 1.4 | 2.2 | 2.0 | 0.6 | 0.8 | 2.1 | 0.9 | 0.8 | 0.9 | 1.4 | 1.4 | 1.33 |
| YDR003W | 2.0 | 2.4 | 1.6 | 1.5 | 1.8 | 1.8 | 1.8 | 1.1 | 6.6 | 3.6 | 1.8 | 0.8 | 1.5 | 1.2 | 1.0 | 1.8 | 1.4 | 2.0 | 0.95 |
| YDR340W | 1.2 | 1.4 | 0.7 | 0.8 | 1.1 | 1.9 | 2.3 | 1.3 | 1.4 | 2.4 | 1.4 | 0.9 | 1.3 | 2.0 | 1.4 | 1.0 | 1.1 | 1.6 | 1.90 |

| | | | | | | | | | | | | | | | | | | | |
|---|---|---|---|---|---|---|---|---|---|---|---|---|---|---|---|---|---|---|---|
| YDR357C | 1.3 | 1.5 | 0.3 | 1.3 | 2.0 | 1.9 | 2.2 | 1.7 | 1.0 | 0.9 | 1.1 | 0.6 | 1.0 | 1.4 | 0.5 | 2.2 | 1.3 | 1.7 | 0.73 |
| YDR396W | 0.7 | 1.3 | 0.6 | 0.7 | 1.4 | 2.0 | 1.5 | 1.4 | 0.3 | 1.2 | 1.0 | 1.2 | 0.6 | 1.4 | 0.9 | 1.3 | 1.1 | 1.3 | 0.63 |
| YDR434W | 0.8 | 0.8 | 1.5 | 1.2 | 1.0 | 2.2 | 1.4 | 0.9 | 0.8 | 1.2 | 0.8 | 0.9 | 1.2 | 0.9 | 1.3 | 0.8 | 1.2 | 1.5 | 1.53 |
| YDR482C | 1.1 | 0.9 | 0.7 | 1.1 | 1.5 | 1.7 | 1.2 | 1.0 | 0.9 | 0.8 | 0.8 | 0.8 | 1.1 | 1.0 | 0.5 | 1.7 | 1.6 | 1.2 | 0.85 |
| YDR520C | 0.9 | 2.1 | 0.9 | 0.6 | 1.3 | 2.3 | 1.2 | 1.0 | 0.8 | 0.7 | 0.6 | 1.2 | 1.4 | 1.0 | 1.3 | 0.9 | 1.1 | 1.0 | 0.46 |
| YDR534C | 0.8 | 4.8 | 1.8 | 0.9 | 1.0 | 2.1 | 9.7 | 1.0 | 0.6 | 7.5 | 1.7 | 1.5 | 1.8 | 0.7 | 4.2 | 1.0 | 0.6 | 1.0 | 0.34 |
| YDR539W | 0.7 | 2.0 | 1.1 | 1.0 | 1.8 | 2.1 | 2.3 | 1.3 | 0.7 | 1.2 | 1.3 | 1.2 | 1.3 | 1.1 | 1.5 | 1.6 | 1.2 | 1.4 | 0.61 |
| YER044C | 1.2 | 1.0 | 2.1 | 1.3 | 1.8 | 3.0 | 1.8 | 0.9 | 0.3 | 0.7 | 1.7 | 0.7 | 0.9 | 1.4 | 0.7 | 3.0 | 1.3 | 2.1 | 1.62 |
| YER067W | 4.0 | 1.9 | 5.2 | 6.0 | 1.1 | 2.1 | 4.9 | 2.1 | 1.8 | 3.8 | 2.2 | 1.1 | 1.2 | 1.7 | 0.5 | 3.5 | 1.5 | 3.0 | 1.57 |
| YER080W | 0.8 | 0.8 | 1.5 | 0.5 | 1.1 | 5.3 | 3.0 | 1.4 | 4.0 | 2.7 | 1.2 | 0.9 | 1.8 | 1.1 | 0.8 | 1.9 | 1.2 | 1.3 | 0.55 |
| YGL113W | 0.7 | 0.9 | 0.9 | 0.4 | 1.5 | 2.1 | 0.9 | 0.9 | 0.7 | 0.8 | 0.7 | 0.8 | 1.2 | 0.9 | 1.0 | 1.0 | 1.5 | 0.9 | 0.35 |
| YGL242C | 0.8 | 0.9 | 0.6 | 0.8 | 1.4 | 1.9 | 2.0 | 1.5 | 0.9 | 1.3 | 1.4 | 0.9 | 1.6 | 1.6 | 1.2 | 1.1 | 1.5 | 1.4 | 1.32 |
| YGR052W | 1.6 | 0.8 | 3.9 | 4.6 | 1.4 | 2.6 | 0.8 | 1.1 | 0.6 | 1.7 | 0.6 | 0.7 | 1.5 | 1.3 | 0.6 | 7.6 | 1.8 | 4.0 | 0.48 |
| YGR106C | 1.2 | 0.8 | 1.9 | 1.3 | 0.9 | 2.0 | 1.1 | 1.0 | 0.7 | 1.0 | 1.2 | 1.1 | 1.0 | 1.2 | 0.9 | 1.1 | 1.0 | 1.7 | 4.60 |
| YGR111W | 1.1 | 2.0 | 1.0 | 1.7 | 1.0 | 2.4 | 1.8 | 1.8 | 3.3 | 2.5 | 1.6 | 0.9 | 2.2 | 1.5 | 0.9 | 2.2 | 1.1 | 2.1 | 0.79 |
| YHL023C | 0.7 | 0.6 | 1.3 | 0.7 | 1.1 | 1.8 | 1.0 | 1.0 | 1.3 | 1.1 | 1.0 | 1.1 | 1.9 | 1.0 | 1.0 | 1.1 | 0.9 | 0.9 | 0.32 |
| YHL048W | 1.3 | 1.6 | 2.8 | 1.3 | 1.1 | 3.7 | 2.3 | 1.2 | 0.8 | 1.2 | 0.9 | 1.1 | 1.7 | 1.0 | 2.3 | 2.5 | 1.9 | 3.2 | 4.10 |
| YIL007C | 1.2 | 1.0 | 1.1 | 1.0 | 1.4 | 2.0 | 1.3 | 1.2 | 1.1 | 1.4 | 1.2 | 1.0 | 1.1 | 1.7 | 1.1 | 1.9 | 1.2 | 1.5 | 0.69 |
| YIR016W | 1.1 | 1.1 | 1.4 | 1.6 | 1.2 | 2.3 | 1.7 | 0.8 | 1.4 | 1.3 | 1.4 | 1.3 | 1.6 | 1.3 | 1.0 | 2.7 | 1.9 | 2.0 | 0.67 |
| YIR043C | 1.2 | 1.6 | 2.2 | 1.8 | 1.3 | 2.4 | 2.2 | 1.0 | 0.8 | 1.3 | 1.4 | 1.3 | 2.0 | 1.4 | 2.5 | 2.6 | 1.4 | 2.5 | 3.90 |
| YJL012C | 0.7 | 0.6 | 1.6 | 0.7 | 1.5 | 1.9 | 0.5 | 1.0 | 0.6 | 1.0 | 0.7 | 1.2 | 1.3 | 1.0 | 1.1 | 0.3 | 0.9 | 0.8 | 1.77 |
| YJL083W | 0.9 | 1.3 | 0.8 | 0.9 | 1.1 | 2.8 | 0.7 | 0.7 | 0.0 | 0.4 | 0.3 | 0.9 | 0.8 | 1.1 | 0.9 | 0.6 | 1.4 | 1.0 | 0.34 |
| YJL131C | 0.9 | 0.8 | 0.5 | 1.1 | 1.7 | 2.2 | 1.9 | 1.2 | 0.5 | 1.0 | 0.3 | 0.8 | 1.8 | 1.5 | 1.0 | 2.2 | 1.3 | 1.2 | 0.54 |
| YJR061W | 1.0 | 2.1 | 1.0 | 0.7 | 1.1 | 2.0 | 0.9 | 1.9 | 1.6 | 1.4 | 0.8 | 0.9 | 0.8 | 1.5 | 1.6 | 1.1 | 1.3 | 1.1 | 0.34 |
| YJR161C | 1.2 | 2.0 | 2.8 | 1.8 | 0.8 | 2.2 | 2.6 | 1.0 | 0.5 | 1.2 | 1.7 | 1.2 | 2.1 | 1.0 | 2.9 | 2.6 | 2.1 | 3.4 | 3.62 |
| YKL175W | 0.7 | 1.1 | 2.1 | 0.9 | 1.4 | 2.2 | 1.3 | 0.9 | 1.1 | 1.9 | 0.8 | 0.9 | 1.7 | 1.0 | 1.6 | 1.1 | 0.9 | 1.0 | 1.71 |
| YKR070W | 1.3 | 0.8 | 1.4 | 1.4 | 1.1 | 2.2 | 1.4 | 1.3 | 3.4 | 2.0 | 1.6 | 0.9 | 1.7 | 1.2 | 0.8 | 2.1 | 1.0 | 1.4 | 0.99 |
| YLL023C | 0.7 | 1.3 | 5.9 | 0.9 | 1.0 | 2.6 | 1.5 | 0.5 | 1.1 | 1.5 | 1.6 | 1.2 | 1.6 | 1.1 | 2.5 | 2.8 | 1.6 | 1.4 | 1.66 |
| YLR023C | 1.3 | 1.5 | 0.9 | 1.2 | 1.1 | 2.6 | 1.2 | 0.9 | 0.7 | 1.7 | 1.1 | 0.6 | 1.0 | 1.1 | 0.9 | 0.8 | 0.7 | 0.8 | 0.92 |
| YLR225C | 0.8 | 0.7 | 0.4 | 0.7 | 1.6 | 2.4 | 1.6 | 1.2 | 1.0 | 1.2 | 0.8 | 1.2 | 1.3 | 1.4 | 1.6 | 0.8 | 1.2 | 1.2 | 1.41 |
| YLR241W | 0.9 | 1.7 | 1.6 | 2.1 | 0.8 | 1.7 | 0.9 | 1.0 | 1.8 | 1.5 | 0.7 | 0.9 | 2.0 | 0.9 | 2.4 | 1.1 | 1.4 | 1.6 | 1.03 |
| YLR252W | 1.3 | 1.3 | 4.6 | 3.0 | 1.0 | 2.8 | 1.6 | 1.1 | 3.1 | 1.4 | 2.5 | 0.7 | 1.3 | 1.3 | 0.9 | 2.7 | 2.2 | 2.9 | 0.96 |

| | | | | | | | | | | | | | | | | | | | |
|---|---|---|---|---|---|---|---|---|---|---|---|---|---|---|---|---|---|---|---|
| YLR270W | 1.2 | 1.1 | 2.3 | 1.2 | 1.1 | 3.5 | 3.9 | 1.1 | 3.3 | 2.1 | 2.8 | 1.1 | 2.1 | 1.3 | 1.4 | 2.6 | 1.4 | 2.3 | 1.00 |
| YML030W | 1.3 | 0.8 | 0.8 | 1.4 | 1.9 | 3.0 | 2.1 | 1.4 | 0.8 | 0.8 | 1.2 | 0.7 | 1.0 | 1.3 | 0.6 | 2.7 | 1.2 | 1.3 | 1.35 |
| YMR148W | 1.0 | 1.0 | 1.6 | 0.9 | 1.2 | 2.9 | 0.8 | 0.6 | 1.0 | 0.8 | 1.0 | 0.5 | 1.0 | 1.0 | 0.7 | 1.4 | 1.1 | 0.9 | 0.28 |
| YMR181C | 1.3 | 1.6 | 1.8 | 1.1 | 1.2 | 2.1 | 2.0 | 0.9 | 1.9 | 1.4 | 1.7 | 1.4 | 2.8 | 1.0 | 1.4 | 2.8 | 1.1 | 2.0 | 0.79 |
| YMR298W | 1.3 | 1.3 | 2.9 | 0.8 | 1.5 | 2.3 | 1.3 | 1.1 | 2.4 | 2.6 | 1.7 | 0.9 | 1.0 | 2.0 | 2.0 | 1.7 | 1.4 | 1.6 | 1.37 |
| YNL011C | 1.1 | 1.5 | 0.8 | 0.9 | 0.9 | 2.9 | 2.9 | 1.5 | 3.8 | 1.6 | 1.4 | 0.6 | 1.2 | 1.2 | 0.8 | 3.1 | 1.2 | 1.8 | 0.68 |
| YOL129W | 1.0 | 1.1 | 2.5 | 1.2 | 1.6 | 2.2 | 1.6 | 0.7 | 1.2 | 1.2 | 0.7 | 0.8 | 1.8 | 1.5 | 1.0 | 1.9 | 2.6 | 2.4 | 2.61 |
| YOR042W | 0.6 | 1.4 | 0.7 | 0.7 | 1.3 | 2.1 | 1.1 | 0.9 | 2.1 | 1.2 | 0.9 | 0.8 | 1.3 | 1.2 | 1.1 | 1.1 | 1.4 | 1.1 | 0.54 |
| YOR052C | 1.5 | 1.3 | 0.5 | 2.4 | 2.3 | 2.4 | 1.4 | 1.4 | 1.4 | 1.3 | 0.8 | 1.0 | 2.6 | 1.6 | 1.2 | 1.3 | 1.2 | 2.3 | 2.64 |
| YOR137C | 1.1 | 1.0 | 0.9 | 1.2 | 1.3 | 2.1 | 1.6 | 0.6 | 0.7 | 0.7 | 0.9 | 0.8 | 1.6 | 0.9 | 1.4 | 1.0 | 1.6 | 1.5 | 0.78 |
| YPL156C | 1.4 | 1.4 | 0.9 | 2.9 | 1.2 | 2.4 | 3.2 | 1.2 | 0.5 | 2.8 | 0.9 | 1.5 | 1.7 | 1.6 | 0.8 | 2.4 | 1.6 | 1.9 | 0.70 |
| YPL186C | 1.6 | 1.7 | 1.4 | 4.5 | 1.3 | 3.2 | 3.2 | 1.9 | 2.4 | 1.5 | 3.7 | 1.0 | 1.4 | 2.0 | 0.7 | 6.2 | 1.2 | 2.9 | 0.60 |
| YPL216W | 0.7 | 0.8 | 1.3 | 0.9 | 0.9 | 1.8 | 0.8 | 0.9 | 1.8 | 1.9 | 0.9 | 0.9 | 1.1 | 1.2 | 1.0 | 1.1 | 1.1 | 0.9 | 0.47 |
| YPR098C | 1.6 | 2.6 | 1.7 | 1.9 | 2.0 | 3.0 | 2.9 | 1.1 | 1.4 | 1.7 | 1.9 | 0.9 | 1.2 | 1.8 | 0.9 | 2.9 | 2.2 | 2.2 | 1.10 |
| YFL062W | 1.3 | 2.7 | 1.7 | 2.4 | 1.6 | 1.8 | 3.5 | 1.1 | 0.6 | 1.2 | 2.2 | 1.1 | 1.6 | 1.1 | 1.6 | 2.7 | 2.1 | 3.0 | 3.43 |
| YJL217W | 0.6 | 0.7 | 3.1 | 2.5 | 0.7 | 1.7 | 3.2 | 4.5 | 0.7 | 1.7 | 4.6 | 0.7 | 0.5 | 0.7 | 0.8 | 5.1 | 1.4 | 2.5 | 2.19 |
| YLR126C | 0.9 | 1.5 | 0.4 | 0.8 | 1.3 | 1.7 | 3.4 | 1.8 | 1.3 | 3.4 | 1.3 | 1.4 | 1.6 | 1.4 | 1.4 | 1.4 | 1.3 | 1.2 | 0.61 |
| YNL249C | 1.3 | 1.7 | 0.3 | 1.5 | 1.2 | 0.7 | 2.3 | 1.7 | 2.6 | 1.7 | 1.4 | 1.0 | 1.4 | 1.3 | 0.9 | 1.3 | 1.1 | 1.1 | 0.56 |
| YNL336W | 1.2 | 1.0 | 3.4 | 1.3 | 1.3 | 1.4 | 2.5 | 0.9 | 0.7 | 1.9 | 0.8 | 1.1 | 1.3 | 1.1 | 1.9 | 2.8 | 2.0 | 3.1 | 3.74 |
| YBR074W | 0.7 | 1.3 | 0.9 | 0.8 | 1.3 | 1.5 | 2.2 | 0.7 | 0.4 | 0.5 | 0.5 | 1.0 | 1.2 | 1.5 | 0.8 | 1.3 | 1.4 | 1.0 | 0.52 |
| YDL248W | 1.1 | 1.7 | 2.8 | 1.7 | 1.5 | 2.0 | 2.5 | 1.1 | 0.9 | 1.4 | 1.4 | 1.2 | 2.0 | 0.9 | 1.8 | 1.9 | 1.6 | 2.4 | 4.35 |
| YDR105C | 0.7 | 1.1 | 2.3 | 1.0 | 0.8 | 1.4 | 2.1 | 0.8 | 2.3 | 1.7 | 1.4 | 0.9 | 1.8 | 1.1 | 0.9 | 1.4 | 1.1 | 1.4 | 1.09 |
| YEL075C | 1.1 | 1.1 | 0.7 | 1.0 | 1.1 | 1.4 | 1.9 | 0.7 | 0.6 | 0.7 | 0.7 | 1.1 | 1.7 | 1.9 | 0.8 | 1.2 | 1.4 | 1.1 | 1.19 |
| YER046W | 1.3 | 1.9 | 0.5 | 1.1 | 1.6 | 1.8 | 2.6 | 1.5 | 1.5 | 1.7 | 1.1 | 1.0 | 1.0 | 1.4 | 0.7 | 2.7 | 2.0 | 1.9 | 0.53 |
| YER050C | 1.3 | 0.9 | 0.5 | 2.3 | 2.2 | 1.0 | 2.2 | 1.5 | 1.4 | 1.6 | 1.4 | 0.7 | 1.0 | 2.1 | 1.0 | 2.1 | 1.0 | 1.8 | 1.40 |
| YGL250W | 1.1 | 1.4 | 1.9 | 1.8 | 1.6 | 1.4 | 2.0 | 1.1 | 1.5 | 1.2 | 2.4 | 0.7 | 1.2 | 1.0 | 1.1 | 2.3 | 1.2 | 1.9 | 0.45 |
| YGR042W | 1.5 | 1.1 | 1.2 | 2.0 | 1.5 | 2.2 | 2.2 | 1.8 | 1.3 | 3.2 | 1.6 | 1.1 | 1.6 | 2.2 | 1.4 | 2.6 | 1.3 | 1.9 | 0.79 |
| YGR053C | 1.3 | 0.7 | 0.6 | 2.7 | 2.1 | 1.9 | 3.4 | 1.5 | 4.5 | 2.2 | 1.6 | 0.7 | 1.2 | 1.7 | 0.7 | 2.7 | 1.5 | 2.4 | 0.39 |
| YGR066C | 1.6 | 2.5 | 1.3 | 2.5 | 1.2 | 1.7 | 2.1 | 1.3 | 2.3 | 5.0 | 2.6 | 0.8 | 1.2 | 1.9 | 0.7 | 2.6 | 1.0 | 1.1 | 0.17 |
| YGR247W | 0.8 | 1.1 | 0.8 | 1.0 | 1.0 | 0.9 | 1.8 | 0.9 | 1.1 | 1.1 | 1.1 | 0.6 | 0.8 | 1.0 | 0.4 | 1.2 | 1.0 | 1.0 | 0.45 |
| YGR295C | 1.0 | 1.6 | 2.8 | 2.0 | 0.9 | 1.9 | 2.8 | 1.1 | 0.5 | 1.2 | 1.1 | 1.1 | 1.9 | 1.0 | 2.4 | 2.4 | 1.6 | 2.8 | 4.78 |
| YHL044W | 0.9 | 1.3 | 1.5 | 1.4 | 0.7 | 1.9 | 2.0 | 1.4 | 1.1 | 1.9 | 0.7 | 1.2 | 1.6 | 1.7 | 1.1 | 3.9 | 0.8 | 1.2 | 0.45 |

| | | | | | | | | | | | | | | | | | | | |
|---|---|---|---|---|---|---|---|---|---|---|---|---|---|---|---|---|---|---|---|
| YHR145C | 1.5 | 1.6 | 0.6 | 0.9 | 1.4 | 1.3 | 2.1 | 1.6 | 0.8 | 2.4 | 2.0 | 0.9 | 1.5 | 1.8 | 1.3 | 1.1 | 1.3 | 1.1 | 1.70 |
| YIL058W | 2.0 | 0.9 | 1.4 | 0.5 | 1.3 | 1.4 | 2.2 | 0.8 | 1.1 | 1.0 | 0.0 | 0.6 | 1.0 | 2.0 | 0.9 | 2.1 | 1.4 | 1.3 | 0.39 |
| YIL065C | 1.0 | 1.3 | 0.8 | 1.6 | 1.9 | 1.6 | 2.0 | 1.4 | 3.8 | 2.1 | 1.1 | 0.9 | 1.8 | 1.7 | 0.8 | 2.4 | 2.1 | 1.4 | 0.94 |
| YIL083C | 0.8 | 1.0 | 0.9 | 0.8 | 1.2 | 1.4 | 2.2 | 1.0 | 1.0 | 1.1 | 1.2 | 1.0 | 1.0 | 1.0 | 0.9 | 1.4 | 0.9 | 1.1 | 0.76 |
| YJL185C | 0.9 | 1.0 | 1.9 | 1.6 | 1.0 | 1.4 | 2.3 | 1.0 | 0.9 | 1.2 | 1.2 | 0.8 | 1.0 | 1.4 | 1.6 | 2.3 | 1.0 | 1.1 | 0.30 |
| YJL213W | 0.8 | 1.1 | 0.2 | 1.2 | 1.5 | 2.6 | 2.1 | 1.2 | 1.3 | 1.5 | 0.7 | 0.7 | 1.0 | 0.8 | 2.8 | 3.6 | 2.1 | 2.8 | 0.33 |
| YKR020W | 1.1 | 1.8 | 0.9 | 1.6 | 1.0 | 4.5 | 2.2 | 1.5 | 1.9 | 2.8 | 1.0 | 0.8 | 1.7 | 1.4 | 1.2 | 2.5 | 1.3 | 2.0 | 0.44 |
| YLL025W | 0.8 | 1.3 | 1.8 | 0.8 | 1.1 | 1.5 | 1.9 | 1.0 | 1.5 | 2.0 | 1.4 | 1.0 | 1.3 | 1.0 | 1.2 | 1.7 | 1.0 | 0.9 | 0.40 |
| YLR108C | 3.2 | 1.8 | 4.7 | 1.8 | 1.9 | 1.3 | 2.6 | 2.0 | 7.4 | 10.4 | 2.6 | 0.8 | 1.9 | 3.4 | 1.2 | 2.3 | 1.0 | 1.5 | 0.39 |
| YLR290C | 1.3 | 1.2 | 0.8 | 1.9 | 2.1 | 1.7 | 2.3 | 1.1 | 1.2 | 1.1 | 1.5 | 0.8 | 1.0 | 1.3 | 0.8 | 3.2 | 1.3 | 2.4 | 1.49 |
| YML068W | 1.0 | 3.7 | 1.0 | 1.2 | 1.0 | 0.9 | 4.1 | 1.3 | 1.3 | 1.4 | 0.6 | 0.4 | 0.8 | 1.1 | 1.1 | 1.9 | 1.0 | 1.3 | 0.41 |
| YMR178W | 1.6 | 1.0 | 0.8 | 0.9 | 1.1 | 1.6 | 2.1 | 1.6 | 1.6 | 1.6 | 1.0 | 0.9 | 1.0 | 1.2 | 0.9 | 2.1 | 1.1 | 2.3 | 1.36 |
| YNL122C | 1.2 | 0.9 | 1.2 | 1.3 | 1.6 | 1.8 | 2.1 | 1.4 | 1.0 | 1.2 | 1.3 | 0.7 | 1.0 | 1.2 | 0.6 | 1.8 | 1.0 | 1.4 | 1.07 |
| YNL285W | 1.1 | 1.2 | 0.6 | 1.1 | 1.4 | 0.8 | 2.3 | 1.2 | 1.4 | 1.7 | 1.2 | 0.6 | 1.1 | 1.8 | 0.9 | 1.3 | 1.2 | 1.3 | 0.43 |
| YNL293W | 1.3 | 0.8 | 0.8 | 1.8 | 1.3 | 0.9 | 2.1 | 1.0 | 1.6 | 2.4 | 0.8 | 0.7 | 1.4 | 1.1 | 1.2 | 2.1 | 1.0 | 1.4 | 0.58 |
| YNR061C | 0.8 | 1.1 | 2.5 | 0.8 | 1.0 | 1.3 | 2.6 | 0.8 | 0.8 | 0.9 | 1.2 | 0.9 | 1.1 | 1.3 | 0.9 | 1.1 | 1.6 | 1.1 | 1.52 |
| YOR220W | 1.5 | 1.4 | 1.7 | 2.4 | 1.3 | 1.2 | 2.1 | 0.9 | 1.8 | 2.5 | 1.2 | 1.2 | 2.9 | 0.8 | 0.7 | 2.0 | 3.5 | 3.4 | 1.44 |
| YPR077C | 1.4 | 1.4 | 1.2 | 2.7 | 1.4 | 1.2 | 2.0 | 1.5 | 0.6 | 2.5 | 0.8 | 1.0 | 0.8 | 1.6 | 2.9 | 0.6 | 1.4 | 1.2 | 0.24 |
| YPR147C | 0.9 | 0.8 | 1.1 | 1.4 | 1.6 | 2.1 | 1.8 | 1.1 | 1.2 | 2.4 | 1.3 | 0.9 | 1.6 | 1.1 | 1.4 | 1.9 | 1.1 | 1.3 | 1.18 |
| YEL041W | 1.4 | 1.4 | 0.8 | 2.5 | 0.8 | 1.2 | 1.9 | 1.9 | 2.4 | 3.6 | 3.5 | 0.9 | 1.9 | 2.0 | 1.4 | 1.9 | 1.5 | 1.9 | 0.39 |
| YKL187C | 0.9 | 1.0 | 1.4 | 1.5 | 0.8 | 0.8 | 0.7 | 1.1 | 5.2 | 8.6 | 3.3 | 0.9 | 1.4 | 1.6 | 1.2 | 4.8 | 0.7 | 1.2 | 0.36 |
| YBR285W | 1.6 | 1.6 | 0.9 | 6.2 | 1.3 | 2.2 | 1.5 | 1.0 | 4.0 | 3.1 | 4.6 | 0.5 | 0.9 | 1.6 | 0.4 | 7.8 | 2.5 | 2.3 | 0.27 |
| YBR292C | 0.8 | 4.3 | 1.2 | 0.6 | 1.0 | 0.8 | 0.7 | 0.8 | 2.1 | 1.3 | 1.8 | 0.4 | 0.7 | 1.1 | 1.0 | 1.0 | 0.9 | 0.9 | 0.30 |
| YDL123W | 1.0 | 1.3 | 3.7 | 1.4 | 0.8 | 0.7 | 1.0 | 0.8 | 2.0 | 2.0 | 4.6 | 1.1 | 1.5 | 0.9 | 3.3 | 1.5 | 1.1 | 1.0 | 0.52 |
| YDR056C | 1.4 | 1.3 | 2.0 | 2.0 | 0.9 | 1.0 | 1.4 | 1.4 | 1.3 | 2.3 | 2.3 | 1.2 | 1.0 | 2.3 | 1.1 | 2.5 | 1.2 | 2.0 | 1.83 |
| YDR132C | 3.7 | 1.6 | 1.4 | 2.1 | 1.2 | 1.9 | 1.7 | 2.3 | 5.9 | 7.3 | 3.3 | 0.9 | 1.5 | 1.4 | 1.2 | 1.2 | 1.0 | 1.3 | 0.40 |
| YDR154C | 1.0 | 2.1 | 2.5 | 1.7 | 0.8 | 1.5 | 0.9 | 1.1 | 1.5 | 1.2 | 3.2 | 1.0 | 2.6 | 1.0 | 1.8 | 1.3 | 1.5 | 2.0 | 2.69 |
| YDR295C | 0.8 | 1.0 | 0.6 | 1.0 | 0.9 | 1.0 | 0.8 | 1.3 | 2.6 | 1.3 | 1.9 | 0.7 | 1.8 | 1.1 | 1.4 | 0.6 | 0.8 | 1.0 | 0.49 |
| YDR494W | 0.9 | 0.9 | 0.2 | 1.3 | 1.3 | 1.4 | 1.7 | 1.2 | 1.2 | 0.9 | 1.6 | 0.8 | 1.0 | 1.0 | 0.6 | 1.9 | 1.1 | 1.3 | 1.39 |
| YEL072W | 3.2 | 3.5 | 1.6 | 1.8 | 1.5 | 0.6 | 1.3 | 1.6 | 4.7 | 4.3 | 1.7 | 0.9 | 1.0 | 2.0 | 0.5 | 1.5 | 0.8 | 1.1 | 0.39 |
| YER045C | 1.6 | 2.1 | 1.2 | 1.0 | 0.9 | 0.8 | 1.1 | 1.4 | 2.0 | 1.5 | 2.3 | 0.8 | 1.2 | 1.3 | 1.4 | 1.6 | 1.1 | 1.1 | 0.34 |
| YER181C | 1.1 | 0.4 | 1.7 | 2.2 | 1.0 | 0.7 | 1.0 | 0.9 | | 0.8 | 0.1 | 0.6 | 1.3 | 1.0 | 0.7 | 0.7 | 0.7 | 1.1 | 0.42 |

| | | | | | | | | | | | | | | | | | | | |
|---|---|---|---|---|---|---|---|---|---|---|---|---|---|---|---|---|---|---|---|
| YGL114W | 1.5 | 0.8 | 3.6 | 1.3 | 1.3 | 0.7 | 1.0 | 1.1 | 2.7 | 5.6 | 3.2 | 0.6 | 1.2 | 1.1 | 1.2 | 1.4 | 0.9 | 0.8 | 0.60 |
| YGL193C | 1.1 | 0.9 | 0.9 | 1.4 | 0.7 | 0.7 | 1.0 | 0.7 | 0.8 | 1.0 | 2.1 | 0.7 | 1.1 | 1.0 | 1.0 | 1.7 | 1.1 | 0.9 | 0.62 |
| YGL204C | 0.9 | 2.2 | 1.2 | 1.2 | 0.7 | 0.3 | 1.3 | 0.8 | 1.0 | 0.9 | 1.9 | 0.8 | 1.4 | 1.8 | 0.8 | 1.1 | 0.9 | 0.9 | 0.38 |
| YGL259W | 1.1 | 1.6 | 1.4 | 1.5 | 1.1 | 0.8 | 1.6 | 1.3 | 2.6 | 2.2 | 2.7 | 1.1 | 1.9 | 1.1 | 1.3 | 4.1 | 0.9 | 0.9 | 0.49 |
| YIL060W | 1.0 | 2.3 | 0.8 | 1.0 | 1.2 | 0.7 | 0.9 | 1.8 | 0.9 | 3.0 | 1.9 | 0.7 | 0.6 | 0.9 | 1.3 | 1.1 | 1.2 | 0.9 | 1.22 |
| YJL036W | 1.3 | 1.0 | 0.6 | 1.6 | 0.7 | 1.1 | 1.5 | 1.5 | 6.6 | 3.5 | 1.7 | 1.2 | 2.2 | 1.5 | 1.1 | 2.1 | 1.4 | 1.6 | 0.88 |
| YJR085C | 1.0 | 1.9 | 2.8 | 4.5 | 0.6 | 1.5 | 1.4 | 0.9 | 3.7 | 2.6 | 2.9 | 1.1 | 1.9 | 1.9 | 0.5 | 2.0 | 1.4 | 1.9 | 2.19 |
| YKR071C | 3.6 | 1.6 | 1.0 | 1.1 | 1.9 | 0.6 | 0.9 | 2.1 | 4.7 | 4.9 | 2.3 | 0.8 | 1.5 | 1.6 | 0.7 | 0.8 | 0.9 | 1.0 | 1.05 |
| YLR145W | 0.9 | 1.1 | 0.6 | 1.5 | 1.1 | 0.6 | 0.9 | 1.1 | 1.9 | 1.2 | 2.4 | 0.8 | 0.7 | 1.6 | 1.0 | 2.5 | 1.0 | 0.8 | 0.62 |
| YLR156W | 1.3 | 1.0 | 0.7 | 0.8 | 0.8 | 0.6 | 1.3 | 1.0 | 1.1 | 2.2 | 1.9 | 0.8 | 1.9 | 1.0 | 1.0 | 1.7 | 1.1 | 0.9 | 0.31 |
| YLR280C | 0.6 | 1.4 | 0.4 | 0.6 | 1.3 | 0.9 | 1.5 | 1.0 | 0.5 | 1.3 | 2.1 | 0.8 | 1.6 | 0.5 | 0.6 | 1.2 | 1.2 | 0.9 | 0.19 |
| YLR311C | 1.5 | 1.9 | 2.8 | 2.9 | 0.9 | 0.4 | 1.1 | 1.0 | 0.9 | 3.0 | 7.8 | 0.9 | 0.9 | 1.6 | 0.5 | 1.9 | 1.3 | 1.1 | 0.33 |
| YMR034C | 1.5 | 1.3 | 1.0 | 6.5 | 1.3 | 1.1 | 1.9 | 1.2 | 0.7 | 2.7 | 2.1 | 0.9 | 1.8 | 1.4 | 1.7 | 1.3 | 1.8 | 1.6 | 0.43 |
| YNL240C | 0.8 | 0.8 | 0.8 | 0.6 | 0.8 | 0.7 | 0.7 | 0.9 | 5.4 | 3.1 | 3.4 | 0.7 | 1.1 | 0.8 | 1.4 | 0.8 | 0.8 | 0.7 | 0.37 |
| YNL260C | 1.7 | 1.8 | 0.5 | 1.0 | 1.6 | 0.8 | 1.1 | 1.2 | 3.8 | 2.0 | 2.5 | 0.6 | 0.7 | 1.4 | 0.7 | 1.2 | 0.9 | 1.0 | 0.75 |
| YNR074C | 1.3 | 1.7 | 3.1 | 1.4 | 1.2 | 0.7 | 1.4 | 1.5 | 3.9 | 3.0 | 2.7 | 0.6 | 1.5 | 1.7 | 0.8 | 0.9 | 0.9 | 0.8 | 0.66 |
| YOL084W | 1.0 | 1.4 | 1.6 | 3.6 | 0.6 | 1.6 | 0.8 | 1.0 | 2.6 | 3.4 | 6.1 | 1.7 | 6.4 | 0.9 | 1.6 | 6.6 | 1.3 | 1.6 | 0.28 |
| YOL159C | 1.9 | 2.4 | 1.7 | 2.0 | 0.9 | 1.0 | 2.1 | 1.2 | 1.1 | 2.4 | 6.6 | 1.2 | 1.4 | 1.8 | 1.1 | 2.3 | 2.0 | 1.6 | 0.61 |
| YOR228C | 1.3 | 1.1 | 1.8 | 1.4 | 1.2 | 1.0 | 1.2 | 0.7 | 1.4 | 1.1 | 2.8 | 0.9 | 1.1 | 1.0 | 0.6 | 3.4 | 1.0 | 1.4 | 0.44 |
| YOR255W | 2.5 | 2.7 | 1.0 | 1.5 | 1.0 | 0.5 | 0.9 | 1.2 | 0.3 | 0.8 | 2.0 | 0.7 | 1.6 | 1.3 | 4.9 | 1.2 | 1.0 | 1.0 | 0.19 |
| YBR047W | 2.7 | 2.4 | 1.1 | 1.5 | 0.9 | 1.1 | | 2.2 | 11.0 | 10.0 | 3.4 | 0.8 | 1.7 | 1.0 | 0.8 | 1.1 | 1.3 | 1.0 | 0.25 |
| YER124C | 0.5 | 17.3 | 0.7 | 1.2 | 1.4 | 2.1 | 1.0 | 1.8 | 0.4 | 1.4 | 1.5 | 0.6 | 0.7 | 0.5 | 0.5 | 1.5 | 0.9 | 1.1 | 2.22 |
| YKR007W | 0.8 | 0.9 | 0.8 | 0.9 | 1.1 | 1.6 | 1.5 | 1.9 | 1.1 | 1.8 | 1.3 | 0.8 | 0.9 | 1.2 | 1.2 | 1.1 | 0.8 | 0.9 | 0.73 |
| YOR007C | 1.1 | 3.3 | 1.4 | 0.9 | 1.1 | 1.2 | 0.4 | 2.1 | 5.6 | 2.5 | 1.8 | 0.7 | 0.7 | 0.8 | 1.3 | 1.0 | 1.2 | 0.8 | 2.26 |
| YBL065W | 1.2 | 2.9 | 1.0 | 3.5 | 1.1 | 0.7 | 1.0 | 1.9 | 20.8 | 4.2 | 1.3 | 0.7 | 1.8 | 1.9 | 3.1 | 0.9 | 1.1 | 0.9 | 0.15 |
| YDL113C | 1.0 | 1.4 | 1.2 | 1.2 | 1.0 | 1.2 | 2.2 | 1.7 | 3.8 | 3.0 | 1.1 | 1.3 | 1.5 | 1.2 | 1.2 | 1.7 | 1.2 | 1.5 | 0.58 |
| YDR018C | 1.5 | 1.3 | 3.3 | 2.6 | 1.3 | 1.5 | 1.4 | 1.4 | 2.3 | 4.4 | 1.4 | 1.1 | 1.8 | 1.7 | 1.0 | 2.6 | 0.9 | 1.2 | 0.22 |
| YDR202C | 1.1 | 1.2 | 1.1 | 1.3 | 1.3 | 1.7 | 1.6 | 1.4 | 4.8 | 2.4 | 1.3 | 0.8 | 1.2 | 2.0 | 0.5 | 2.8 | 1.0 | 1.6 | 0.79 |
| YDR223W | 1.3 | 4.6 | 2.0 | 1.9 | 1.1 | 1.4 | 1.0 | 1.0 | 2.7 | 4.6 | 1.4 | 1.0 | 1.5 | 1.2 | 1.2 | 3.5 | 0.9 | 1.0 | 0.29 |
| YDR350C | 0.9 | 1.5 | 0.4 | 1.3 | 1.3 | 1.3 | 1.5 | 0.9 | 2.8 | 2.5 | 0.9 | 0.9 | 1.0 | 1.1 | 1.6 | 1.3 | 1.4 | 1.2 | 0.53 |
| YDR374C | 1.9 | 2.9 | 1.5 | 0.8 | 1.3 | 1.1 | 0.9 | 1.8 | 10.4 | 3.9 | 1.4 | 1.0 | 2.5 | 4.0 | 2.0 | 1.3 | 1.0 | 1.0 | 0.36 |
| YDR512C | 1.8 | 4.3 | 2.5 | 2.3 | 0.7 | 2.1 | 2.0 | 1.6 | 3.6 | 4.0 | 1.8 | 1.1 | 1.9 | 2.7 | 1.1 | 3.0 | 1.3 | 2.0 | 0.82 |

| | | | | | | | | | | | | | | | | | | | |
|---|---|---|---|---|---|---|---|---|---|---|---|---|---|---|---|---|---|---|---|
| YFR017C | 1.1 | 1.4 | 4.4 | 4.4 | 1.2 | 1.2 | 1.3 | 0.8 | 1.0 | 7.1 | 3.1 | 1.8 | 1.7 | 1.3 | 1.4 | 3.7 | 0.9 | 1.1 | 0.49 |
| YGL046W | 1.6 | 1.2 | 1.5 | 1.4 | 0.9 | 1.2 | 1.0 | 1.2 | 0.8 | 4.5 | 1.5 | 1.1 | 1.6 | 0.9 | 2.0 | 1.2 | 1.5 | 1.3 | 0.42 |
| YGL067W | 1.6 | 1.3 | 1.7 | 1.1 | 0.6 | 1.0 | 1.1 | 1.2 | 1.0 | 2.3 | 1.7 | 0.9 | 3.7 | 1.6 | 0.7 | 1.0 | 0.9 | 1.1 | 0.63 |
| YGL098W | 1.2 | 0.8 | 0.5 | 1.7 | 0.8 | 0.7 | 1.2 | 1.3 | 1.2 | 2.4 | 1.3 | 0.8 | 0.8 | 1.1 | 0.7 | 1.3 | 1.1 | 1.5 | 0.77 |
| YGL117W | 2.3 | 0.6 | 0.5 | 2.0 | 0.9 | 0.9 | 1.3 | 1.9 | 0.5 | 5.8 | 1.4 | 1.5 | 1.4 | 2.0 | 1.4 | 0.6 | 2.1 | 1.1 | 1.05 |
| YGL146C | 0.9 | 1.0 | 1.1 | 3.3 | 1.3 | 1.2 | 1.3 | 1.2 | 0.9 | 3.2 | 1.3 | 0.9 | 1.1 | 1.2 | 1.1 | 2.2 | 1.0 | 1.0 | 0.43 |
| YGR011W | 1.1 | 1.5 | 1.0 | 1.3 | 0.9 | 1.1 | 1.8 | 1.4 | 7.4 | 9.4 | 2.3 | 0.8 | 1.6 | 3.9 | 1.8 | 1.6 | 0.9 | 1.0 | 0.40 |
| YGR153W | 1.5 | 0.9 | 1.0 | 1.1 | 1.6 | 0.9 | 1.4 | 1.8 | 2.7 | 2.5 | 1.0 | 0.7 | 1.1 | 1.9 | 1.3 | 1.1 | 1.3 | 1.7 | 0.39 |
| YGR223C | 1.8 | 1.2 | 1.5 | 1.3 | 1.3 | 1.2 | 1.2 | 1.4 | 6.2 | 3.8 | 1.9 | 0.8 | 1.8 | 1.6 | 1.2 | 1.8 | 1.1 | 1.8 | 0.66 |
| YHR116W | 0.9 | 1.2 | 0.9 | 2.1 | 1.6 | 1.5 | 1.8 | 1.1 | 1.5 | 2.7 | 0.9 | 0.8 | 1.9 | 1.4 | 1.2 | 1.9 | 0.9 | 1.5 | 0.60 |
| YIL097W | 1.2 | 1.0 | 1.3 | 1.2 | 1.2 | 2.0 | 1.6 | 1.7 | 4.9 | 3.4 | 1.4 | 1.1 | 1.4 | 1.4 | 1.2 | 2.1 | 1.1 | 1.7 | 0.52 |
| YKL133C | 1.3 | 12.7 | 2.4 | 2.0 | 1.0 | 1.7 | 3.2 | 1.5 | 4.1 | 5.9 | 1.4 | 1.3 | 1.4 | 1.6 | 1.7 | 3.8 | 1.2 | 2.4 | 0.35 |
| YKL162C | 1.3 | 1.0 | 1.9 | 0.9 | 1.1 | 1.7 | 1.4 | 1.5 | 6.7 | 4.7 | 1.6 | 1.1 | 1.1 | 1.9 | 1.6 | 2.0 | 0.8 | 1.1 | 0.25 |
| YLL062C | 6.5 | 6.7 | 4.6 | 1.1 | 0.8 | 0.9 | 0.7 | 1.8 | 14.9 | 5.4 | 1.7 | 0.6 | 1.4 | 1.7 | 1.6 | 1.0 | 0.9 | 0.9 | 0.29 |
| YLR247C | 0.8 | 1.5 | 1.0 | 1.0 | 1.2 | 1.2 | 1.2 | 1.3 | 2.5 | 2.9 | 1.6 | 1.0 | 1.8 | 1.0 | 1.4 | 1.4 | 1.2 | 1.3 | 0.48 |
| YLR267W | 0.9 | 1.7 | 1.3 | 1.4 | 1.2 | 1.3 | 1.4 | 1.4 | 1.1 | 4.6 | 1.7 | 0.8 | 1.5 | 2.3 | 0.7 | 3.6 | 1.2 | 2.8 | 0.24 |
| YMR041C | 1.2 | 2.8 | 1.4 | 1.2 | 1.1 | 0.5 | 0.5 | 1.4 | 6.5 | 6.0 | 1.6 | 1.0 | 1.1 | 0.6 | 0.5 | 0.3 | 0.8 | 0.4 | 0.84 |
| YMR253C | 0.9 | 1.7 | 2.0 | 1.3 | 0.8 | 1.7 | 2.3 | 1.0 | 1.5 | 2.8 | 1.0 | 0.5 | 1.2 | 1.3 | 0.9 | 1.3 | 1.2 | 1.4 | 0.42 |
| YOR225W | 1.1 | 2.8 | 1.4 | 1.0 | 1.3 | 0.7 | 0.8 | 0.8 | 0.8 | 3.3 | 1.2 | 0.5 | 0.6 | 2.2 | 0.7 | 0.6 | 0.9 | 0.9 | 0.26 |
| YPL166W | 1.0 | 1.4 | 1.4 | 1.4 | 1.2 | 0.7 | 1.8 | 1.2 | 1.5 | 3.1 | 1.4 | 0.8 | 1.4 | 0.8 | 1.2 | 2.3 | 1.1 | 1.6 | 0.39 |
| YPL202C | 1.1 | 1.6 | 1.0 | 0.7 | 1.9 | 1.4 | 1.6 | 1.1 | 1.8 | 3.5 | 1.0 | 0.7 | 1.1 | 0.9 | 1.0 | 1.2 | 1.1 | 1.0 | 0.56 |
| YBR101C | 1.3 | 2.2 | 1.7 | 1.1 | 1.2 | 0.8 | 0.6 | 1.0 | 6.9 | 3.0 | 1.3 | 0.7 | 1.6 | 0.7 | 0.5 | 0.3 | 0.9 | 1.0 | 1.83 |
| YBR269C | 1.2 | 5.7 | 2.2 | 1.1 | 1.3 | 1.8 | 1.3 | 0.8 | 2.7 | 2.6 | 1.2 | 1.0 | 1.3 | 1.4 | 0.4 | 1.6 | 1.5 | 1.6 | 0.58 |
| YBR280C | 1.2 | 1.0 | 2.7 | 2.0 | 1.3 | 1.6 | 1.7 | 1.0 | 5.3 | 1.9 | 1.3 | 1.3 | 2.8 | 1.3 | 1.0 | 3.2 | 1.1 | 1.7 | 0.33 |
| YDL234C | 1.9 | 0.9 | 0.5 | 1.4 | 1.0 | 1.1 | 1.9 | 1.3 | 4.6 | 2.1 | 1.5 | 0.9 | 3.5 | 0.7 | 1.0 | 2.5 | 2.3 | 3.6 | 0.94 |
| YDL242W | 1.3 | 1.3 | 1.6 | 1.5 | 1.3 | 0.7 | 1.1 | 0.5 | 4.0 | 4.1 | 1.3 | 1.0 | 1.0 | 1.0 | 1.2 | 1.3 | 0.9 | 1.0 | 0.38 |
| YDR531W | 0.8 | 0.9 | 0.7 | 1.0 | 2.0 | 1.2 | 1.6 | 1.4 | 2.9 | 1.5 | 1.3 | 0.9 | 1.4 | 1.2 | 0.9 | 1.2 | 1.5 | 1.2 | 1.32 |
| YFR042W | 1.5 | 1.2 | 1.9 | 1.9 | 1.8 | 0.8 | 1.6 | 1.0 | 2.8 | 2.6 | 1.7 | 1.1 | 1.9 | 1.7 | 1.4 | 1.4 | 1.7 | 1.7 | 1.17 |
| YFR046C | 1.1 | 2.3 | 1.1 | 1.6 | 1.2 | 1.0 | 1.0 | 1.5 | 5.8 | 1.5 | 1.0 | 0.9 | 1.1 | 1.4 | 0.9 | 1.3 | 1.2 | 1.2 | 0.29 |
| YGL227W | 1.0 | 0.8 | 0.9 | 0.7 | 1.2 | 1.1 | 1.2 | 1.0 | 2.4 | 1.9 | 1.1 | 0.8 | 1.0 | 1.3 | 0.9 | 1.5 | 1.1 | 1.3 | 0.59 |
| YGR089W | 0.8 | 0.8 | 0.9 | 0.5 | 1.1 | 1.2 | 0.9 | 0.8 | 4.0 | 1.4 | 0.9 | 0.7 | 0.8 | 1.1 | 0.8 | 0.9 | 0.9 | 1.1 | 0.50 |
| YGR134W | 1.1 | 1.5 | 0.5 | 1.3 | 1.3 | 1.0 | 1.0 | 1.5 | 2.4 | 1.9 | 0.9 | 0.7 | 1.5 | 1.1 | 1.1 | 0.7 | 0.9 | 0.8 | 0.33 |

| | | | | | | | | | | | | | | | | | | | |
|---|---|---|---|---|---|---|---|---|---|---|---|---|---|---|---|---|---|---|---|
| YHR017W | 0.8 | 1.0 | 1.3 | 1.3 | 1.4 | 1.1 | 1.5 | 1.5 | 2.9 | 1.7 | 1.5 | 1.0 | 1.2 | 1.2 | 0.8 | 2.5 | 1.1 | 1.7 | 0.90 |
| YIL152W | 1.1 | 1.3 | 1.0 | 1.7 | 1.0 | 0.8 | 1.2 | 1.0 | 3.1 | 1.8 | 0.9 | 1.1 | 1.4 | 1.7 | 0.9 | 1.5 | 1.1 | 1.0 | 0.81 |
| YIL164C | 1.2 | 1.2 | 1.0 | 1.2 | 1.0 | 1.1 | 2.0 | 1.2 | 4.2 | 3.0 | 1.0 | 1.0 | 1.8 | 2.0 | 0.9 | 2.5 | 1.4 | 1.4 | 0.54 |
| YJR056C | 1.0 | 2.4 | 0.8 | 1.2 | 0.9 | 0.5 | 0.9 | 1.2 | 3.5 | 1.4 | 0.8 | 0.8 | 0.9 | 1.4 | 1.6 | 1.3 | 0.9 | 1.1 | 0.44 |
| YJR072C | 0.7 | 2.5 | 1.0 | 0.8 | 1.1 | 0.7 | 0.8 | 1.3 | 3.1 | 1.6 | 0.9 | 1.0 | 1.1 | 1.2 | 0.6 | 0.9 | 0.8 | 0.8 | 0.78 |
| YKL034W | 0.8 | 1.4 | 1.0 | 0.9 | 1.1 | 1.9 | 1.4 | 0.7 | 2.6 | 3.7 | 1.2 | 1.0 | 1.9 | 1.0 | 1.6 | 1.5 | 1.3 | 1.2 | 0.41 |
| YKR012C | 0.7 | 1.4 | 1.0 | 1.0 | 1.2 | 1.4 | 1.0 | 1.0 | 3.9 | 1.2 | 0.8 | 0.7 | 1.5 | 1.4 | 1.2 | 0.9 | 1.0 | 0.9 | 0.61 |
| YLR064W | 1.1 | 1.1 | 2.9 | 1.3 | 0.8 | 1.2 | 0.8 | 1.0 | 3.3 | 2.1 | 1.6 | 0.7 | 1.6 | 1.4 | 1.5 | 1.0 | 0.9 | 1.0 | 1.49 |
| YLR364W | 3.3 | 8.0 | 1.2 | 1.5 | 1.3 | 1.0 | 1.0 | 1.1 | 8.3 | 1.3 | 1.5 | 0.7 | 1.0 | 3.0 | 1.9 | 1.0 | 1.0 | 0.9 | 0.37 |
| YLR421C | 1.1 | 1.3 | 0.9 | 1.2 | 0.9 | 2.6 | 1.5 | 1.5 | 4.5 | 3.3 | 1.2 | 0.9 | 1.6 | 1.6 | 1.6 | 1.9 | 1.2 | 1.8 | 2.07 |
| YML118W | 1.5 | 0.5 | 0.7 | 2.7 | 0.7 | 0.8 | 1.5 | 0.9 | 6.6 | 1.6 | 1.3 | 0.9 | 1.7 | 1.0 | 2.2 | 1.6 | 1.5 | 1.5 | 0.26 |
| YMR114C | 1.2 | 1.4 | 0.5 | 2.4 | 1.2 | 1.2 | 1.7 | 1.1 | 2.5 | 1.7 | 1.0 | 0.9 | 1.1 | 1.5 | 1.2 | 3.0 | 1.0 | 1.3 | 0.62 |
| YMR115W | 1.0 | 0.6 | 1.3 | 1.1 | 0.9 | 1.5 | 1.0 | 1.2 | 5.8 | 2.1 | 1.1 | 1.0 | 1.3 | 1.3 | 1.2 | 1.1 | 1.0 | 1.2 | 0.61 |
| YMR258C | 0.9 | 1.4 | 1.2 | 1.1 | 0.7 | 1.2 | 1.5 | 0.9 | 2.3 | 1.8 | 1.4 | 0.9 | 1.6 | 1.0 | 0.6 | 1.2 | 1.1 | 1.3 | 0.60 |
| YNL181W | 1.2 | 1.2 | 0.6 | 1.2 | 1.4 | 1.6 | 0.9 | 1.9 | 5.3 | 2.8 | 1.2 | 0.8 | 1.8 | 1.9 | 1.0 | 1.0 | 0.8 | 1.3 | 0.86 |
| YNL191W | 1.5 | 5.1 | 4.7 | 0.7 | 0.9 | 0.6 | 3.0 | 1.2 | 3.9 | 3.2 | 1.0 | 0.5 | 0.6 | 1.0 | 1.0 | 0.6 | 0.5 | 0.6 | 0.45 |
| YNL212W | 1.1 | 1.1 | 1.1 | 0.5 | 0.9 | 0.8 | 1.3 | 1.3 | 4.6 | 2.3 | 1.3 | 0.8 | 1.3 | 1.0 | 0.9 | 1.3 | 0.9 | 1.2 | 0.67 |
| YNL265C | 1.0 | 1.3 | 0.5 | 1.3 | 0.9 | 1.9 | 1.5 | 1.4 | 5.0 | 2.6 | 0.9 | 0.8 | 2.0 | 1.9 | 0.6 | 1.7 | 0.9 | 1.8 | 0.85 |
| YOR088W | 0.7 | 0.9 | 1.7 | 0.9 | 1.2 | 0.7 | 0.3 | 0.7 | 2.5 | 1.3 | 0.8 | 0.6 | 0.4 | 0.4 | 0.8 | 0.4 | 0.7 | 0.7 | 3.56 |
| YOR155C | 0.8 | 1.2 | 1.7 | 1.0 | 1.4 | | | 0.8 | 3.6 | 1.7 | 1.6 | 0.5 | | 1.5 | 0.9 | | 0.8 | 0.9 | 0.46 |
| YPL151C | 1.0 | 0.8 | 1.2 | 1.2 | 1.0 | 0.7 | 0.7 | 1.0 | 4.2 | 2.0 | 0.9 | 0.8 | 1.4 | 0.9 | 1.8 | 0.9 | 0.8 | 1.0 | 0.51 |
| YPL249C | 0.8 | 0.9 | 1.1 | 0.6 | 0.8 | 2.5 | 1.0 | 1.0 | 3.0 | 1.2 | 0.9 | 0.9 | 1.8 | 1.0 | 1.0 | 1.0 | 0.8 | 1.2 | 0.36 |
| YPL260W | 0.9 | 3.9 | 1.4 | 0.8 | 0.8 | 1.4 | 1.2 | 1.1 | 2.6 | 2.1 | 1.0 | 0.8 | 1.4 | 1.1 | 1.3 | 1.2 | 0.8 | 1.3 | 0.82 |
| YPR061C | 1.3 | 3.2 | 1.2 | 4.9 | 1.4 | 0.5 | 1.8 | 1.2 | 3.2 | 1.9 | 1.6 | 0.8 | 1.1 | 1.7 | 0.4 | 2.7 | 1.1 | 0.9 | 0.40 |
| YPR093C | 1.1 | 1.1 | 0.7 | 1.3 | 1.0 | 0.7 | 0.8 | 1.1 | 6.6 | 2.3 | 1.2 | 0.9 | 1.4 | 1.1 | 0.8 | 1.4 | 1.4 | 1.1 | 0.39 |
| YPR158W | 1.5 | 1.5 | 3.7 | 0.9 | 1.6 | 0.7 | 1.2 | 1.2 | 5.0 | 3.5 | 1.5 | 0.6 | 1.6 | 1.1 | 0.9 | 0.6 | 0.9 | 0.7 | 0.96 |
| YPR169W | 0.9 | 0.9 | 0.5 | 0.6 | 1.0 | 1.5 | 0.9 | 1.0 | 3.5 | 1.4 | 1.0 | 0.5 | 1.0 | 1.0 | 0.9 | 1.4 | 1.1 | 1.1 | 0.86 |
| YPR174C | 0.8 | 0.9 | 0.8 | 0.6 | 1.3 | 1.1 | 0.8 | 1.2 | 3.2 | 1.0 | 0.7 | 1.1 | 1.4 | 0.9 | 1.2 | 0.4 | 1.4 | 0.9 | 0.55 |
| YAL014C | 1.0 | 1.6 | 1.4 | 1.2 | 0.8 | 0.6 | 1.2 | 1.2 | 3.1 | 2.0 | 1.7 | 1.2 | 1.5 | 1.8 | 2.3 | 0.9 | 1.1 | 1.1 | 0.56 |
| YAL017W | 0.6 | 1.4 | 2.3 | 1.2 | 1.0 | 1.1 | 1.3 | 0.6 | 1.9 | 1.6 | 0.7 | 1.0 | 2.2 | 1.1 | 1.0 | 1.3 | 1.0 | 1.2 | 0.79 |
| YAL049C | 1.0 | 2.0 | 1.7 | 1.0 | 0.7 | 1.5 | 3.4 | 1.4 | 3.0 | 1.4 | 1.0 | 1.1 | 1.6 | 2.0 | 1.4 | 2.9 | 1.6 | 1.8 | 1.13 |
| YBR013C | 1.1 | 2.7 | 1.8 | 1.4 | 0.9 | 1.8 | 1.0 | 1.4 | 2.2 | 2.2 | 1.2 | 1.2 | 2.0 | 2.5 | 1.1 | 1.5 | 1.4 | 1.1 | 0.68 |

| | | | | | | | | | | | | | | | | | | | |
|---|---|---|---|---|---|---|---|---|---|---|---|---|---|---|---|---|---|---|---|
| YBR051W | 1.2 | 0.8 | 1.5 | 0.3 | 1.1 | | 1.1 | 1.1 | 1.5 | 1.5 | 0.1 | 0.7 | 1.2 | 1.0 | 0.8 | 1.2 | 1.1 | 0.8 | 0.40 |
| YBR063C | 0.8 | 0.8 | 0.4 | 0.6 | 1.2 | 1.6 | 1.6 | 1.3 | 2.2 | 1.6 | 0.6 | 1.1 | 1.7 | 1.7 | 1.6 | 1.0 | 1.2 | 1.2 | 0.38 |
| YBR129C | 1.1 | 0.8 | 0.6 | 1.0 | 1.2 | 1.9 | 1.0 | 1.4 | 2.3 | 1.4 | 1.4 | 1.0 | 1.3 | 1.6 | 0.9 | 1.7 | 1.0 | 1.5 | 1.25 |
| YBR255W | 1.1 | 1.6 | 0.4 | 0.7 | 1.2 | 1.0 | 1.1 | 1.8 | 1.8 | 1.5 | 1.5 | 0.7 | 1.4 | 1.1 | 1.0 | 1.5 | 1.0 | 1.0 | 0.27 |
| YBR281C | 0.8 | 1.1 | 3.0 | 0.5 | 0.8 | | 0.6 | 1.2 | 1.8 | 1.4 | 1.4 | 0.5 | 1.2 | 0.9 | 1.0 | 0.8 | 0.7 | 0.8 | 0.44 |
| YCL044C | 0.8 | 1.4 | 2.4 | 0.6 | 1.1 | 1.0 | 1.0 | 1.0 | 3.3 | 4.1 | 1.1 | 0.9 | 1.7 | 1.0 | 4.4 | 0.8 | 0.9 | 0.6 | 0.21 |
| YDL089W | 1.1 | 1.3 | 1.4 | 0.9 | 1.2 | 2.0 | 2.0 | 1.1 | 2.3 | 2.5 | 2.0 | 0.9 | 1.9 | 1.2 | 1.6 | 1.3 | 1.0 | 1.1 | 0.45 |
| YDL173W | 1.0 | 1.2 | 1.0 | 0.8 | 1.3 | 1.1 | 1.6 | 1.5 | 1.8 | 1.6 | 1.2 | 1.1 | 1.7 | 2.1 | 1.1 | 1.3 | 1.3 | 1.8 | 1.19 |
| YDL193W | 0.9 | 1.5 | 1.4 | 0.8 | 1.1 | 2.0 | 1.5 | 1.6 | 2.5 | 1.4 | 1.1 | 1.1 | 1.4 | 1.1 | 1.2 | 1.8 | 0.9 | 1.4 | 0.93 |
| YDL233W | 0.8 | 2.0 | 1.0 | 3.8 | 0.9 | 1.0 | 0.8 | 1.0 | 2.1 | 1.1 | 0.7 | 0.9 | 1.8 | 1.1 | 1.3 | 0.9 | 1.1 | 1.4 | 0.33 |
| YDR071C | 1.4 | 1.1 | 0.7 | 1.3 | 1.1 | 1.5 | 1.3 | 1.6 | 2.6 | 1.6 | 1.4 | 0.9 | 1.3 | 2.0 | 0.9 | 1.2 | 0.9 | 1.6 | 3.08 |
| YDR078C | 1.1 | 1.6 | 3.5 | 0.8 | 0.9 | 0.8 | 2.8 | 1.4 | 2.1 | 1.4 | 1.1 | 0.7 | 1.1 | 1.8 | 0.5 | 1.3 | 0.9 | 1.2 | 0.53 |
| YDR109C | 0.8 | 1.0 | 1.0 | 1.3 | 1.0 | 0.9 | 1.2 | 1.5 | 1.9 | 1.3 | 0.9 | 1.0 | 1.4 | 1.0 | 1.7 | 1.5 | 1.2 | 0.9 | 0.38 |
| YDR140W | 1.7 | 1.4 | 0.9 | 1.8 | 0.7 | 0.9 | 1.2 | 1.5 | 2.2 | 2.5 | 1.7 | 1.1 | 2.0 | 3.2 | 0.9 | 1.9 | 1.3 | 1.3 | 0.78 |
| YDR221W | 0.8 | 1.3 | 0.3 | 0.8 | 1.1 | 1.5 | 0.9 | 1.0 | 2.3 | 1.4 | 0.7 | 0.6 | 1.0 | 1.0 | 1.0 | 1.0 | 1.1 | 1.0 | 0.34 |
| YDR271C | 1.0 | 0.8 | 0.7 | 1.7 | 0.8 | 1.4 | 1.3 | 0.8 | 5.4 | 0.9 | 0.6 | 0.7 | 1.5 | 1.4 | 1.3 | 0.6 | 1.2 | 0.9 | 0.32 |
| YDR316W | 1.0 | 0.8 | 0.6 | 0.9 | 1.5 | 0.7 | 0.3 | 1.4 | 2.5 | 1.0 | 1.3 | 0.6 | 0.6 | 0.7 | 1.3 | 0.3 | 0.6 | 0.5 | 0.69 |
| YDR338C | 1.1 | 1.3 | 1.4 | 1.0 | 0.7 | 0.9 | 1.1 | 0.9 | 2.7 | 1.7 | 1.9 | 0.8 | 1.3 | 1.0 | 0.8 | 0.8 | 0.9 | 1.0 | 0.40 |
| YDR421W | 1.1 | 1.0 | 1.0 | 1.1 | 0.9 | 0.5 | 0.2 | 0.9 | 3.8 | 1.2 | 1.0 | 0.7 | 0.6 | 1.2 | 1.9 | 1.0 | 0.7 | 0.8 | 0.42 |
| YDR425W | 1.3 | 1.7 | 1.0 | 2.2 | 1.4 | 1.7 | 1.8 | 1.2 | 2.6 | 2.2 | 0.9 | 1.1 | 1.4 | 1.3 | 1.3 | 1.1 | 1.2 | 1.2 | 0.31 |
| YDR485C | 0.8 | 0.9 | 0.7 | 0.9 | 1.6 | 1.2 | 1.2 | 1.1 | 2.7 | 2.0 | 0.9 | 0.9 | 1.2 | 1.1 | 1.3 | 1.5 | 0.8 | 1.0 | 0.59 |
| YDR504C | 1.0 | 1.0 | 0.9 | 0.8 | 1.1 | 1.2 | 1.1 | 0.8 | 2.3 | 1.1 | 1.2 | 0.7 | 2.2 | 1.3 | 1.2 | 1.2 | 1.2 | 1.2 | 0.70 |
| YEL044W | 0.8 | 1.5 | 0.7 | 0.9 | 1.2 | 0.8 | 0.8 | 0.8 | 2.1 | 1.2 | 1.6 | 0.6 | 1.3 | 0.6 | 0.6 | 0.7 | 1.4 | 1.0 | 0.93 |
| YER092W | 1.3 | 1.3 | 1.2 | 1.1 | 1.2 | 1.5 | 1.3 | 1.1 | 1.9 | 1.6 | 0.9 | 0.9 | 1.2 | 1.2 | 1.0 | 1.9 | 1.4 | 1.7 | 1.13 |
| YER182W | 1.2 | 0.8 | 1.1 | 1.9 | 1.2 | 0.6 | 0.8 | 1.4 | 2.2 | 1.2 | 1.5 | 1.0 | 0.9 | 1.9 | 0.5 | 3.6 | 0.8 | 1.2 | 0.87 |
| YFL042C | 0.8 | 0.9 | 0.6 | 1.1 | 1.4 | 1.5 | 1.1 | 0.8 | 2.0 | 2.0 | 5.4 | 0.9 | 1.7 | 1.2 | 1.1 | 1.0 | 1.1 | 1.2 | 0.43 |
| YFR056C | 0.9 | 1.5 | 0.7 | 0.5 | 1.2 | 0.3 | 0.9 | 1.0 | 2.4 | 2.7 | 1.3 | 0.5 | 0.3 | 0.6 | 0.9 | 0.5 | 1.1 | 0.8 | 0.59 |
| YGL041C | 0.9 | 0.6 | 1.3 | 1.0 | 0.6 | 1.4 | 1.0 | 0.8 | 1.3 | 0.4 | 0.6 | 0.6 | 1.0 | 1.2 | 0.7 | 0.8 | 1.0 | 1.1 | 0.51 |
| YGL045W | 1.4 | 1.1 | 1.9 | 3.4 | 1.0 | 0.8 | 1.7 | 1.2 | 2.8 | 4.2 | 1.2 | 1.1 | 1.3 | 1.1 | 1.5 | 3.4 | 1.3 | 1.2 | 0.47 |
| YGL057C | 1.3 | 1.1 | 1.4 | 1.4 | 1.3 | 1.3 | 1.1 | 1.1 | 2.1 | 1.1 | 1.3 | 1.1 | 1.3 | 1.5 | 0.7 | 1.2 | 0.9 | 1.1 | 0.56 |
| YGL183C | 1.1 | 1.4 | 0.8 | 0.5 | 1.6 | 1.2 | 1.2 | 0.8 | 3.4 | 2.5 | 0.8 | 0.9 | 1.5 | 1.7 | 1.7 | 0.6 | 1.4 | 1.0 | 0.18 |
| YGL223C | 1.1 | 1.7 | 1.0 | 0.7 | 1.4 | 1.1 | 1.2 | 1.0 | 2.6 | 2.0 | 1.3 | 0.9 | 1.7 | 0.9 | 0.7 | 1.1 | 0.8 | 1.0 | 0.60 |

| | | | | | | | | | | | | | | | | | | | |
|---|---|---|---|---|---|---|---|---|---|---|---|---|---|---|---|---|---|---|---|
| YGR156W | 0.9 | 1.3 | 1.7 | 1.2 | 1.0 | 1.2 | 1.3 | 1.3 | 4.0 | 2.4 | 0.5 | 1.2 | 1.8 | 1.6 | 1.3 | 0.5 | 0.8 | 0.8 | 0.26 |
| YGR198W | 0.7 | 0.9 | 0.7 | 1.0 | 0.8 | 0.8 | 0.8 | 1.0 | 2.9 | 1.8 | 1.0 | 0.7 | 1.3 | 0.8 | 0.9 | 1.2 | 1.1 | 1.0 | 0.87 |
| YGR210C | 0.9 | 1.4 | 1.1 | 0.9 | 0.8 | 0.8 | 0.8 | 1.0 | 3.5 | 1.8 | 1.0 | 0.6 | 1.0 | 0.8 | 1.3 | 0.7 | 1.1 | 0.7 | 0.56 |
| YGR211W | 0.7 | 1.0 | 2.0 | 1.0 | 0.9 | 0.6 | 0.5 | 1.0 | 2.9 | 2.5 | 0.8 | 0.6 | 0.8 | 0.4 | 0.5 | 0.3 | 0.5 | 0.5 | 1.99 |
| YGR237C | 0.8 | 1.3 | 2.5 | 0.8 | 1.2 | 0.7 | 1.4 | 1.0 | 1.8 | 1.4 | 1.6 | 0.8 | 1.6 | 0.6 | 1.1 | 1.2 | 0.9 | 1.0 | 0.50 |
| YGR250C | 1.3 | 1.4 | 1.4 | 1.5 | 1.4 | 1.6 | 1.2 | 1.1 | 2.4 | 2.8 | 1.6 | 0.6 | 1.7 | 1.3 | 1.2 | 1.2 | 1.3 | 2.1 | 1.21 |
| YGR266W | 0.7 | 0.5 | 1.0 | 0.9 | 0.8 | 1.2 | 0.9 | 0.9 | 2.0 | 1.1 | 1.2 | 0.6 | 1.1 | 0.8 | 1.1 | 1.3 | 0.7 | 1.0 | 0.59 |
| YGR277C | 1.0 | 1.9 | 1.1 | 0.8 | 0.8 | 0.8 | 1.9 | 1.3 | 2.8 | 1.5 | 1.2 | 0.9 | 1.6 | 1.5 | 0.9 | 1.0 | 1.3 | 1.1 | 0.81 |
| YHL021C | 1.6 | 3.9 | 5.9 | 3.2 | 0.9 | 1.4 | 2.7 | 1.0 | 2.7 | 1.1 | 1.4 | 1.1 | 2.6 | 1.1 | 1.2 | 2.4 | 1.3 | 3.6 | 1.27 |
| YHL037C | 1.2 | 1.4 | 0.9 | | 1.0 | 0.7 | 1.0 | 1.1 | 0.4 | 1.1 | 1.0 | 0.6 | 1.0 | 0.9 | 0.8 | 0.7 | 0.9 | 0.9 | 0.28 |
| YHR083W | 1.0 | 0.9 | 1.4 | 1.3 | 0.9 | 0.9 | 1.0 | 1.0 | 2.0 | 2.1 | 1.3 | 0.9 | 1.1 | 1.6 | 1.3 | 2.4 | 0.8 | 1.0 | 0.90 |
| YHR134W | 1.1 | 0.7 | 0.4 | 1.1 | 1.1 | 2.0 | 1.3 | 1.2 | 3.5 | 1.5 | 0.7 | 1.0 | 1.2 | 1.6 | 1.0 | 1.2 | 1.3 | 1.3 | 0.81 |
| YHR180W | 1.5 | 0.7 | 1.1 | 1.5 | 0.9 | 1.1 | 1.1 | 1.2 | 4.8 | 3.5 | 1.4 | 0.7 | 1.3 | 1.5 | 0.8 | 1.3 | 0.8 | 1.0 | 0.36 |
| YIL108W | 1.0 | 1.7 | 2.2 | 0.7 | 1.3 | 0.6 | 0.9 | 0.8 | 2.4 | 2.0 | 1.2 | 0.7 | 1.3 | 1.0 | 1.8 | 0.7 | 1.2 | 0.7 | 0.51 |
| YIL165C | 1.2 | 2.3 | 0.9 | 1.6 | 1.6 | 0.8 | 1.5 | 1.1 | 3.2 | 3.4 | 1.1 | 0.9 | 1.5 | 1.3 | 0.8 | 1.9 | 1.4 | 1.3 | 0.74 |
| YJL032W | 1.1 | 1.0 | 0.8 | 0.9 | 0.9 | 0.6 | 0.9 | 1.3 | 3.3 | 2.0 | 1.8 | 0.5 | 1.4 | 1.6 | 1.0 | 1.2 | 1.1 | 1.1 | 0.33 |
| YJL049W | 1.3 | 0.8 | 0.5 | 1.8 | 1.6 | 1.5 | 1.2 | 1.5 | 2.0 | 1.9 | 1.5 | 0.8 | 0.9 | 1.8 | 0.8 | 1.6 | 0.9 | 1.1 | 0.75 |
| YJR044C | 1.1 | 1.2 | 5.1 | 1.0 | 1.0 | 1.5 | 2.0 | 1.0 | 2.2 | 1.6 | 1.9 | 0.7 | 2.0 | 1.3 | 1.6 | 2.7 | 1.8 | 1.6 | 1.18 |
| YKL059C | 0.8 | 1.0 | 0.9 | 0.7 | 1.3 | 0.9 | 1.0 | 1.0 | 2.6 | 1.2 | 0.9 | 0.9 | 1.3 | 1.1 | 1.1 | 0.8 | 1.1 | 0.9 | 0.53 |
| YKL090W | 1.0 | 0.9 | 1.0 | 1.2 | 1.1 | 1.0 | 1.1 | 1.2 | 3.9 | 1.6 | 2.3 | 0.6 | 0.8 | 1.4 | 0.8 | 1.6 | 0.7 | 1.0 | 0.37 |
| YKL094W | 1.2 | 1.6 | 1.2 | 1.9 | 0.9 | 1.0 | 1.1 | 1.3 | 3.1 | 1.8 | 1.3 | 0.9 | 1.7 | 1.3 | 1.0 | 2.0 | 1.2 | 1.9 | 1.60 |
| YLR097C | 1.1 | 1.5 | 1.0 | 1.6 | 1.6 | 1.7 | 1.8 | 1.4 | 2.2 | 1.1 | 1.7 | 0.8 | 1.1 | 1.7 | 0.9 | 2.5 | 1.1 | 1.7 | 0.76 |
| YLR226W | 1.0 | 1.8 | 0.3 | 1.0 | 1.7 | 1.1 | 1.9 | 1.3 | 2.1 | 1.4 | 0.8 | 0.7 | 0.7 | 1.1 | 1.3 | 0.9 | 1.1 | 1.0 | 0.56 |
| YLR392C | 1.1 | 0.9 | 0.8 | 1.9 | 1.0 | 1.2 | 1.0 | 1.3 | 2.4 | 2.1 | 1.4 | 0.6 | 1.4 | 1.1 | 0.5 | 2.1 | 0.8 | 1.6 | 0.46 |
| YLR427W | 0.6 | 1.0 | 0.2 | 0.5 | 1.6 | 1.0 | 1.2 | 1.1 | 2.1 | 1.3 | 0.8 | 0.9 | 2.5 | 0.8 | 0.8 | 1.3 | 1.0 | 1.0 | 0.49 |
| YML013W | 0.8 | 1.0 | 0.4 | 0.6 | 1.3 | 0.6 | 1.3 | 0.6 | 2.1 | 1.2 | 1.1 | 0.9 | 1.2 | 1.1 | 2.2 | 1.3 | 1.1 | 0.8 | 0.27 |
| YML029W | 0.6 | 1.5 | 1.3 | 1.0 | 1.2 | 1.6 | 1.1 | 0.9 | 2.3 | 1.8 | 1.2 | 1.1 | 2.0 | 1.6 | 0.8 | 1.1 | 1.2 | 1.0 | 0.46 |
| YML041C | 1.2 | 1.0 | 0.6 | 1.0 | 1.3 | 1.9 | 1.4 | 1.8 | 3.0 | 1.5 | 0.9 | 0.8 | 1.2 | 2.2 | 1.0 | 1.4 | 1.3 | 1.3 | 0.59 |
| YML079W | 1.0 | 1.7 | 1.1 | 1.5 | 0.8 | 1.4 | 1.2 | 1.2 | 1.9 | 1.4 | 1.2 | 1.1 | 1.6 | 1.4 | 0.3 | 2.0 | 1.4 | 1.4 | 1.18 |
| YMR068W | 0.9 | 0.7 | 0.8 | 1.5 | 1.6 | 1.1 | 1.5 | 0.8 | 2.4 | 1.4 | 1.1 | 0.7 | 1.4 | 1.1 | 2.2 | 0.8 | 1.3 | 1.1 | 0.24 |
| YMR160W | 0.8 | 1.3 | 1.2 | 1.2 | 1.4 | 2.2 | 1.0 | 0.9 | 2.2 | 1.8 | 1.0 | 1.0 | 1.6 | 1.1 | 1.5 | 1.4 | 0.9 | 1.3 | 0.37 |
| YNL026W | 0.7 | 1.0 | 1.3 | 0.8 | 1.4 | 1.1 | 1.5 | 1.0 | 2.7 | 1.8 | 1.1 | 0.9 | 1.7 | 0.8 | 0.8 | 1.5 | 1.0 | 1.4 | 0.86 |

| | | | | | | | | | | | | | | | | | | | |
|---|---|---|---|---|---|---|---|---|---|---|---|---|---|---|---|---|---|---|---|
| YNL063W | 0.9 | 1.9 | 0.9 | 1.4 | 1.1 | 1.1 | 1.4 | 1.5 | 3.0 | 1.8 | 1.1 | 1.0 | 1.5 | 1.1 | 0.7 | 1.8 | 0.9 | 1.0 | 0.54 |
| YNL176C | 1.1 | 1.3 | 2.9 | 1.0 | 0.8 | 0.7 | 0.7 | 0.9 | 2.4 | 2.1 | 1.2 | 0.6 | 0.6 | 0.9 | 0.8 | 1.3 | 0.6 | 0.8 | 0.66 |
| YNL194C | 1.6 | 0.7 | 2.0 | 15.2 | 0.7 | 0.7 | 1.5 | 0.9 | 2.2 | 6.4 | 17.1 | 0.5 | 1.5 | 3.0 | 0.5 | 4.3 | 0.8 | 2.4 | 0.34 |
| YNL253W | 1.3 | 1.3 | 0.6 | 1.0 | 1.5 | 1.3 | 1.3 | 1.1 | 2.8 | 2.6 | 0.8 | 0.8 | 1.1 | 1.4 | 0.5 | 1.1 | 1.1 | 1.2 | 0.54 |
| YNL276C | 1.3 | 13.1 | 1.9 | 0.3 | 1.1 | 1.3 | 0.9 | 0.7 | 3.1 | 1.3 | 0.7 | 0.9 | 1.5 | 1.5 | 0.5 | 0.8 | 1.1 | 0.8 | 0.22 |
| YNR051C | 0.7 | 0.5 | 1.1 | 0.6 | 1.3 | 1.8 | 1.3 | 0.7 | 2.4 | 1.4 | 1.5 | 0.9 | 2.1 | 1.0 | 1.2 | 0.6 | 0.7 | 0.8 | 1.58 |
| YOR022C | 1.3 | 1.1 | 1.1 | 1.2 | 1.4 | 1.2 | 1.6 | 1.2 | 2.0 | 1.4 | 1.7 | 0.8 | 1.7 | 1.0 | 0.8 | 1.8 | 1.1 | 1.1 | 0.36 |
| YOR087W | 0.7 | 1.0 | 1.8 | 1.3 | 0.8 | 0.3 | 0.9 | 0.8 | 2.8 | 1.3 | 1.3 | 0.8 | 1.2 | 0.9 | 0.8 | 1.2 | 0.8 | 1.1 | 0.61 |
| YOR138C | 0.8 | 1.5 | 0.7 | 0.8 | 0.8 | 1.0 | 1.1 | 0.8 | 2.1 | 3.7 | 0.7 | 0.9 | 1.8 | 0.9 | 1.0 | 1.1 | 1.1 | 1.1 | 0.47 |
| YOR267C | 0.8 | 0.7 | 1.0 | 0.9 | 1.2 | 1.6 | 0.9 | 0.7 | 2.4 | 2.2 | 0.4 | 0.9 | 1.4 | 0.9 | 1.3 | 0.8 | 1.3 | 1.0 | 0.58 |
| YPL005W | 1.0 | 1.0 | 1.0 | 1.0 | 1.4 | 0.8 | 2.0 | 1.0 | 2.6 | 2.8 | 1.1 | 0.7 | 1.2 | 1.0 | 1.2 | 1.6 | 1.2 | 1.1 | 0.32 |
| YPL150W | 0.7 | 0.9 | 1.6 | 0.9 | 1.0 | 0.6 | 1.2 | 1.1 | 3.1 | 2.0 | 1.1 | 1.0 | 2.1 | 1.0 | 1.1 | 1.2 | 1.4 | 1.1 | 0.47 |
| YPL152W | 1.4 | 1.1 | 1.4 | 1.8 | 1.4 | 1.2 | 1.3 | 1.1 | 3.6 | 2.7 | 1.4 | 0.9 | 1.4 | 1.2 | 2.3 | 1.4 | 1.2 | 1.9 | 0.42 |
| YPL168W | 1.1 | 1.5 | 0.5 | 1.4 | 1.0 | 0.8 | 1.2 | 1.2 | 2.1 | 1.6 | 0.7 | 0.7 | 1.0 | 1.0 | 0.8 | 1.3 | 1.1 | 1.2 | 0.41 |
| YPL180W | 0.9 | 2.0 | 0.7 | 0.6 | 1.2 | 1.5 | 1.0 | 1.0 | 1.9 | 1.8 | 1.0 | 0.8 | 1.5 | 1.0 | 0.7 | 1.0 | 1.0 | 0.9 | 0.30 |
| YPL188W | 1.3 | 1.2 | 2.9 | 1.0 | 0.9 | 0.6 | 1.3 | 1.6 | 2.5 | 2.6 | 1.0 | 0.6 | 0.9 | 1.4 | 0.8 | 1.7 | 0.7 | 0.9 | 0.55 |
| YPR049C | 1.0 | 2.5 | 1.5 | 1.0 | 0.7 | 1.2 | 1.1 | 1.1 | 2.4 | 2.1 | 0.9 | 0.9 | 1.6 | 1.4 | 1.5 | 1.7 | 1.0 | 1.1 | 0.34 |
| YPR148C | 1.1 | 1.2 | 0.8 | 1.3 | 0.9 | 1.0 | 1.6 | 1.5 | 2.7 | 1.7 | 0.9 | 1.2 | 1.2 | 1.1 | 1.7 | 1.7 | 0.9 | 2.0 | 1.25 |
| YPR172W | 1.1 | 1.6 | 2.2 | 1.2 | 1.0 | 1.7 | 1.2 | 1.0 | 2.1 | 1.3 | 1.9 | 0.8 | 1.3 | 1.6 | 1.5 | 1.9 | 1.1 | 1.4 | 0.45 |
| YAL018C | 2.0 | 1.9 | 1.1 | 11.3 | 0.8 | 0.7 | 1.0 | 1.6 | | 0.4 | 0.1 | 0.9 | 1.3 | 1.0 | 6.9 | 1.1 | 0.9 | 0.9 | 0.21 |
| YAR064W | 2.1 | 0.9 | 0.9 | 2.8 | 0.8 | 0.5 | 1.1 | 1.1 | 0.0 | 0.7 | 0.7 | 0.8 | 1.1 | 1.1 | 1.9 | 1.0 | 0.9 | 0.9 | 0.30 |
| YBR012C | 2.5 | 1.7 | 1.3 | 0.8 | 0.9 | 1.8 | 1.5 | 1.1 | 1.4 | 2.6 | 0.7 | 1.0 | 2.1 | 0.9 | 1.1 | 1.3 | 1.4 | 1.2 | 0.46 |
| YBR287W | 1.7 | 1.9 | 3.1 | 2.4 | 1.3 | 1.4 | 1.1 | 1.0 | 1.2 | 1.6 | 2.0 | 1.2 | 3.4 | 0.8 | 1.3 | 1.6 | 3.2 | 1.9 | 2.73 |
| YDR250C | 1.9 | 1.4 | 0.9 | 3.0 | 1.0 | 0.4 | 1.0 | 1.1 | | 0.5 | 0.6 | 0.8 | 1.2 | 0.9 | 0.7 | 1.7 | 0.9 | 0.9 | 0.32 |
| YJL037W | 1.8 | 1.4 | 1.4 | 2.4 | 1.5 | 1.8 | 1.2 | 1.2 | 1.8 | 2.4 | 1.6 | 0.8 | 1.1 | 2.0 | 1.6 | 1.0 | 1.3 | 0.9 | 0.26 |
| YNL058C | 2.0 | 1.0 | 2.1 | 1.7 | 1.0 | 0.4 | 0.7 | | 0.3 | 0.7 | 1.0 | 1.0 | 0.6 | 0.8 | 2.2 | 0.9 | 0.8 | 1.0 | 0.60 |
| YJR030C | 0.7 | 4.4 | 0.9 | 1.2 | 0.7 | 0.6 | 0.7 | 0.9 | 0.4 | 0.5 | 0.8 | 0.9 | 0.7 | 1.0 | 0.8 | 0.9 | 0.7 | 0.9 | 0.27 |
| YKR040C | 1.2 | 3.7 | 2.4 | 2.9 | 0.9 | 0.9 | 0.9 | 1.0 | 1.3 | 1.2 | 0.8 | 0.8 | 0.6 | 3.8 | 1.1 | 1.2 | 0.7 | 0.8 | 0.53 |
| YDR128W | 0.6 | 3.9 | 1.4 | 0.9 | 0.8 | 0.9 | 0.8 | 1.1 | 0.9 | 1.5 | 0.3 | 0.6 | 1.1 | 1.0 | 1.4 | 1.3 | 0.9 | 0.9 | 0.40 |
| YGR139W | 1.3 | 3.7 | 0.8 | 0.7 | 1.3 | 0.6 | 0.8 | 0.6 | | 0.6 | 0.9 | 0.5 | 0.4 | 0.9 | 2.3 | 0.8 | 1.0 | 0.8 | 0.22 |
| YOR253W | 0.9 | 2.8 | 1.0 | 1.4 | 0.8 | 0.5 | 1.3 | 1.2 | 0.4 | 1.0 | 0.9 | 0.7 | 0.5 | 1.3 | 1.1 | 0.9 | 0.9 | 0.9 | 1.01 |
| YOL026C | 1.2 | 2.7 | 0.7 | 1.4 | 1.9 | 1.0 | 1.8 | 1.1 | 1.2 | 1.1 | 1.2 | 0.9 | 1.2 | 1.3 | 0.9 | 2.4 | 2.1 | 1.4 | 0.65 |

| | | | | | | | | | | | | | | | | | | | |
|---|---|---|---|---|---|---|---|---|---|---|---|---|---|---|---|---|---|---|---|
| YDR278C | 1.0 | 3.2 | 0.9 | 1.0 | 1.1 | 1.0 | 0.7 | 0.5 | 0.6 | 0.7 | 0.4 | 0.6 | 0.7 | 0.9 | 0.7 | 0.5 | 0.7 | 0.8 | 0.78 |
| YHR095W | 1.1 | 2.6 | 1.5 | 2.1 | 1.1 | 1.0 | 0.8 | 1.0 | 1.5 | 1.2 | 1.3 | 0.6 | 0.7 | 0.9 | 0.8 | 0.9 | 0.8 | 0.7 | 0.67 |
| YCL042W | | 2.5 | 7.2 | | 1.1 | 0.6 | | 0.6 | 0.7 | 2.2 | 3.2 | | 7.5 | | | | 0.8 | | 1.94 |
| YNL200C | 1.0 | 3.0 | 3.6 | 2.0 | 1.0 | 0.4 | 1.2 | 0.7 | 1.9 | 1.2 | 3.1 | 0.9 | 1.1 | 0.6 | 0.8 | 0.9 | 1.2 | 0.9 | 1.40 |
| YPL221W | 0.9 | 3.0 | 1.0 | 1.8 | 1.4 | 1.1 | 1.3 | 0.9 | 0.3 | 1.3 | 1.0 | 0.8 | 1.0 | 0.8 | 1.0 | 1.2 | 1.5 | 1.2 | 1.51 |
| YLR415C | 1.1 | 2.4 | 6.7 | | 0.9 | 0.3 | 1.2 | 0.9 | | 0.3 | 0.8 | 0.8 | 1.0 | 0.8 | 1.0 | 1.9 | 0.8 | 1.0 | 0.19 |
| YOR325W | 1.0 | 2.6 | 1.5 | 0.3 | 0.9 | 0.3 | 1.2 | 0.9 | | | 0.6 | 0.9 | 0.6 | 1.3 | 0.7 | 1.4 | 0.8 | 1.0 | 0.19 |
| YGL088W | 0.9 | 2.1 | 0.9 | 1.1 | 0.6 | 0.8 | 1.0 | 0.5 | 0.5 | 0.8 | 0.8 | 0.6 | 0.7 | 1.4 | 0.4 | 0.3 | 0.5 | 0.7 | 1.80 |
| YDR090C | 0.9 | 3.0 | 0.8 | 1.2 | 1.8 | 1.4 | 1.5 | 0.8 | 1.0 | 1.5 | 0.7 | 0.6 | 0.8 | 0.9 | 1.1 | 0.9 | 1.1 | 1.1 | 0.79 |
| YMR071C | 1.0 | 2.0 | 1.1 | 1.9 | 1.4 | 1.2 | 1.8 | 1.1 | 1.6 | 1.8 | 1.2 | 0.7 | 1.3 | 1.2 | 0.8 | 1.4 | 1.2 | 1.9 | 2.39 |
| YGR293C | 0.5 | 1.9 | 1.6 | 0.0 | 1.1 | 0.7 | 1.3 | 1.3 | | 1.1 | 0.6 | 1.0 | 0.7 | 1.0 | 0.7 | 0.9 | 0.8 | 0.9 | 0.23 |
| YJL017W | 1.1 | 1.7 | 3.5 | 1.1 | 1.3 | 1.2 | 0.2 | 1.0 | 0.8 | 1.4 | 1.4 | 1.3 | 1.5 | 1.0 | 1.1 | 0.6 | 1.0 | 1.0 | 0.85 |
| YIL127C | 1.1 | 4.8 | 0.2 | 1.2 | 1.0 | 0.4 | 0.6 | 1.2 | 0.3 | 0.5 | 0.9 | 0.7 | 0.4 | 1.3 | 0.7 | 0.2 | 0.7 | 0.6 | 1.57 |
| YDR281C | 2.1 | 2.6 | 0.4 | 2.1 | 1.3 | 1.5 | 0.6 | 1.2 | 0.2 | 0.5 | 0.8 | 0.9 | 0.7 | 1.2 | 0.3 | 0.6 | 1.5 | 1.2 | 1.56 |
| YDR366C | 0.9 | 2.0 | 0.8 | 0.9 | 1.0 | 1.5 | 1.5 | 1.1 | 1.0 | 1.5 | 1.7 | 0.6 | 0.8 | 1.4 | 1.4 | 1.0 | 1.2 | 0.9 | 1.14 |
| YFR026C | 0.9 | 2.0 | 1.0 | 0.8 | 1.0 | 1.2 | 1.3 | 1.3 | 1.8 | 0.6 | 0.8 | 0.6 | 1.1 | 1.0 | 2.3 | 2.2 | 1.1 | 1.1 | 0.39 |
| YAR047C | 1.1 | 1.7 | 1.5 | 0.7 | 1.1 | 1.3 | 1.2 | 0.8 | 0.5 | 2.3 | 1.1 | 0.9 | 1.4 | 1.3 | 0.5 | 1.0 | 1.7 | 1.0 | 0.35 |
| YHL006C | 0.7 | 1.4 | 0.9 | 0.6 | 1.1 | 1.1 | 1.2 | 1.1 | 0.9 | 0.9 | 0.7 | 1.0 | 1.1 | 1.1 | 0.6 | 0.9 | 1.1 | 1.0 | 0.37 |
| YPL225W | 1.3 | 1.7 | 1.0 | 1.6 | 1.1 | 1.8 | 1.7 | 1.7 | 1.0 | 1.6 | 0.9 | 0.7 | 0.8 | 1.4 | 0.9 | 1.8 | 1.0 | 1.7 | 2.87 |
| YBR124W | 0.9 | 1.4 | 1.5 | 0.4 | 1.0 | 0.6 | 1.1 | 1.2 | 1.1 | 0.7 | 0.4 | 0.9 | 0.9 | 1.0 | 1.0 | 0.8 | 0.9 | 0.9 | 0.29 |
| YBL044W | 0.9 | 2.7 | 2.1 | 0.8 | 1.2 | 0.7 | 1.1 | 0.7 | 0.2 | 0.5 | 0.6 | 0.9 | 0.7 | 0.9 | 0.7 | 0.6 | 0.8 | 0.8 | 0.39 |
| YCL056C | 1.3 | 1.4 | 0.7 | 1.0 | 1.5 | 1.4 | 1.6 | 1.0 | 1.5 | 1.6 | 1.5 | 1.0 | 1.3 | 2.2 | 1.7 | 2.3 | 1.2 | 1.5 | 0.82 |
| YCR007C | 2.2 | 1.6 | 0.9 | 2.9 | 1.5 | 1.4 | 2.0 | 1.0 | 1.4 | 2.6 | 1.1 | 0.8 | 1.5 | 1.7 | 0.8 | 1.7 | 2.6 | 2.1 | 0.39 |
| YPR146C | 0.8 | 1.6 | 1.3 | 0.9 | 1.2 | 1.1 | 1.0 | 0.8 | 0.9 | 1.2 | 1.4 | 0.8 | 1.3 | 0.9 | 0.9 | 1.3 | 1.0 | 1.1 | 0.80 |
| YKL097C | 0.9 | 1.6 | 0.6 | 1.4 | 1.8 | 0.7 | 0.8 | 0.4 | 0.1 | | 0.6 | 0.6 | 0.9 | 1.0 | 0.8 | 0.5 | 1.1 | 0.5 | 0.21 |
| YBR066C | 1.9 | 1.3 | 1.6 | 1.6 | 1.3 | 1.3 | 1.3 | 1.2 | 0.3 | 0.9 | 1.2 | 0.7 | 0.6 | 1.1 | 0.4 | 3.2 | 1.2 | 1.2 | 0.83 |
| YLR338W | 1.1 | 1.6 | 1.4 | 1.1 | 0.9 | 1.1 | 0.9 | 0.8 | 7.9 | 0.5 | 0.9 | 0.6 | 1.2 | 0.8 | 1.5 | 0.9 | 0.8 | 0.6 | 0.33 |
| YBR162C | 0.6 | 0.5 | 3.1 | 0.8 | 1.3 | 0.6 | 0.3 | 0.8 | 0.1 | 0.4 | 0.8 | 0.7 | 0.7 | 0.5 | 1.9 | 0.4 | 1.2 | 1.3 | 3.96 |
| YDL046W | 1.2 | 1.4 | 5.1 | 1.3 | 0.9 | 1.8 | 1.2 | 0.7 | 1.6 | 2.3 | 2.6 | 1.0 | 1.5 | 1.2 | 2.3 | 2.6 | 1.2 | 1.6 | 1.94 |
| YDR133C | 0.9 | 1.1 | 2.9 | 2.0 | 1.2 | 0.7 | 0.2 | 0.5 | 0.1 | 0.4 | 1.3 | 0.3 | 0.1 | 0.6 | 0.6 | 1.1 | 0.6 | 0.9 | 4.02 |
| YGR038W | 0.9 | 1.2 | 3.5 | 0.8 | 1.3 | 1.3 | 1.1 | 0.7 | 1.4 | 1.0 | 1.4 | 1.0 | 1.2 | 1.4 | 1.1 | 1.3 | 1.1 | 1.1 | 1.27 |
| YGR243W | 1.4 | 2.2 | 9.4 | 5.0 | 1.0 | 1.5 | 1.9 | 1.3 | 0.7 | 3.0 | 1.7 | 1.2 | 1.1 | 1.9 | 0.8 | 2.8 | 2.1 | 4.4 | 1.11 |

| | | | | | | | | | | | | | | | | | | | |
|---|---|---|---|---|---|---|---|---|---|---|---|---|---|---|---|---|---|---|---|
| YHR105W | 0.9 | 0.8 | 2.4 | 6.3 | 1.5 | 0.2 | 0.9 | 1.0 | 1.1 | 3.4 | 0.7 | 0.7 | 1.4 | 1.2 | 0.9 | 1.8 | 1.0 | 0.9 | 0.32 |
| YHR181W | 0.9 | 0.7 | 2.4 | 1.4 | 1.0 | 0.9 | 0.8 | 0.9 | 0.5 | 1.3 | 1.3 | 0.8 | 0.7 | 1.6 | 1.4 | 1.5 | 0.9 | 0.9 | 1.40 |
| YJL097W | 1.0 | 1.1 | 3.7 | 1.6 | 0.9 | 1.6 | 1.0 | 0.8 | 0.3 | 0.7 | 1.5 | 1.0 | 0.7 | 1.0 | 1.1 | 1.2 | 1.2 | 1.1 | 1.69 |
| YKL051W | 1.0 | 1.1 | 4.7 | 0.8 | 0.8 | 1.0 | 0.6 | 0.8 | 1.3 | 1.1 | 1.8 | 0.8 | 1.2 | 0.7 | 1.5 | 0.9 | 1.5 | 1.4 | 1.07 |
| YKL100C | 0.7 | 1.1 | 5.0 | 1.6 | 0.6 | 1.8 | 1.2 | 0.7 | 1.3 | 1.3 | 1.6 | 0.9 | 1.3 | 1.0 | 1.7 | 2.0 | 0.7 | 1.5 | 1.14 |
| YLR339C | 0.7 | 0.8 | 3.1 | 0.8 | 1.3 | 0.4 | 0.4 | 0.5 | | 0.4 | 1.6 | 0.6 | 0.1 | 0.5 | 1.0 | 0.5 | 0.7 | 0.6 | 1.37 |
| YOL030W | 1.0 | 0.8 | 4.4 | 1.0 | 0.9 | 0.9 | 1.3 | 0.6 | 1.2 | 0.6 | 1.3 | 0.9 | 1.7 | 0.6 | 0.9 | 0.7 | 0.9 | 1.0 | 1.93 |
| YPR150W | 1.3 | 1.1 | 4.3 | 3.0 | 1.3 | 0.9 | 0.9 | 0.8 | 1.2 | 3.3 | 0.9 | 0.6 | 1.4 | 1.4 | 0.9 | 1.8 | 0.9 | 1.0 | 0.31 |
| YBL100C | 0.8 | 1.8 | 1.6 | 0.5 | 0.6 | 0.4 | 1.1 | 0.6 | 0.2 | 0.8 | 0.9 | 0.7 | 0.9 | 0.9 | 1.0 | 0.5 | 0.8 | 0.8 | 0.58 |
| YBR096W | 1.1 | 1.5 | 2.8 | 1.6 | 0.9 | 1.7 | 1.9 | 1.0 | 1.2 | 1.6 | 1.2 | 0.9 | 1.9 | 1.7 | 1.1 | 1.1 | 1.1 | 1.5 | 1.43 |
| YBR100W | 1.0 | 1.5 | 4.8 | 1.1 | 0.9 | 0.5 | 1.0 | 0.9 | 8.5 | 3.0 | 0.2 | 0.7 | 1.2 | 1.2 | 1.3 | 1.2 | 0.8 | 0.9 | 0.35 |
| YCL058C | 1.1 | 1.6 | 2.2 | 1.2 | 1.7 | 1.1 | 0.8 | 0.9 | 0.6 | 1.0 | 1.0 | 1.0 | 0.9 | 1.9 | 1.2 | 0.8 | 1.4 | 0.9 | 0.72 |
| YCR030C | 1.1 | 0.9 | 3.3 | 1.0 | 1.0 | 1.2 | 1.0 | 0.7 | 0.9 | 2.0 | 1.1 | 0.9 | 1.7 | 0.8 | 1.3 | 1.0 | 1.2 | 1.0 | 0.63 |
| YDL015C | 0.9 | 0.7 | 3.6 | 1.1 | 1.2 | 2.5 | 1.1 | 0.9 | 0.7 | 1.1 | 0.8 | 1.0 | 0.9 | 1.1 | 1.1 | 2.1 | 1.0 | 1.5 | 2.99 |
| YDL023C | 0.8 | 1.6 | 2.4 | 1.1 | 0.6 | 0.3 | 1.5 | 0.7 | 0.7 | 1.5 | 3.5 | 0.9 | 1.4 | 0.7 | 0.6 | 0.9 | 0.8 | 1.1 | 0.78 |
| YDL086W | 0.9 | 1.2 | 1.9 | 0.9 | 1.3 | 0.9 | 0.8 | 0.8 | 1.3 | 1.1 | 1.4 | 0.8 | 1.7 | 0.9 | 0.9 | 1.1 | 0.8 | 0.8 | 0.80 |
| YDR233C | 1.1 | 1.1 | 3.2 | 1.9 | 0.7 | 0.8 | 1.2 | 1.1 | 0.2 | 0.7 | 3.7 | 1.3 | 0.5 | 0.9 | 1.5 | 2.8 | 0.9 | 0.9 | 3.47 |
| YDR359C | 0.8 | 0.7 | 1.7 | 1.9 | 1.3 | 0.8 | 1.0 | 0.8 | 0.7 | 1.0 | 0.5 | 1.0 | 1.1 | 1.3 | 1.4 | 0.9 | 0.8 | 0.9 | 0.34 |
| YEL033W | 0.9 | 1.2 | 2.7 | 1.1 | 1.0 | 0.7 | 0.3 | 0.7 | 0.6 | 0.4 | 1.2 | 0.6 | 0.4 | 1.0 | 0.6 | 0.9 | 0.9 | 0.9 | 2.96 |
| YGR022C | 1.3 | 1.1 | 1.7 | 2.2 | 1.1 | 1.3 | 0.9 | 0.9 | 0.2 | | 0.4 | 0.7 | 1.2 | 1.1 | 0.7 | 0.6 | 0.9 | 0.8 | 0.50 |
| YGR026W | 0.8 | 1.1 | 2.9 | 0.9 | 0.9 | 1.5 | 0.9 | 0.9 | 0.8 | 0.7 | 1.3 | 0.5 | 0.6 | 1.0 | 1.2 | 1.4 | 1.0 | 1.2 | 2.19 |
| YGR107W | 1.0 | 0.8 | 1.7 | 0.9 | 1.1 | 0.8 | 1.0 | 0.9 | 0.1 | 0.0 | 0.6 | 0.6 | 0.9 | 1.0 | 1.1 | 0.8 | 1.2 | 0.8 | 0.43 |
| YHL005C | 0.9 | 2.7 | 2.7 | 0.2 | 1.2 | 1.1 | 0.8 | 0.9 | | 0.9 | | 0.7 | 0.7 | 1.0 | 0.7 | 0.7 | 1.0 | 0.8 | 0.33 |
| YHR126C | 1.1 | 0.9 | 2.8 | 0.6 | 1.0 | 1.2 | 1.1 | 0.6 | 0.4 | 0.7 | 1.9 | 1.0 | 0.8 | 0.8 | 1.4 | 1.7 | 1.1 | 1.3 | 1.31 |
| YHR143W | 0.5 | 0.9 | 4.9 | 1.0 | 1.2 | 2.1 | 0.5 | 1.5 | 0.2 | 0.7 | 1.2 | 0.5 | 0.5 | 0.8 | 0.3 | 0.9 | 0.9 | 0.7 | 3.58 |
| YIL157C | 1.1 | 1.3 | 2.1 | 1.7 | 0.8 | 1.3 | 1.2 | 1.0 | 0.9 | 0.9 | 1.3 | 0.8 | 1.0 | 1.0 | 0.5 | 1.8 | 1.1 | 1.5 | 1.46 |
| YIR041W | 1.6 | 1.0 | 2.4 | 1.4 | 1.3 | 1.0 | 1.6 | 0.9 | 1.5 | 2.5 | 1.2 | 1.3 | 1.1 | 1.1 | 1.3 | 1.3 | 1.1 | 0.9 | 0.66 |
| YJL016W | 1.3 | 1.6 | 2.7 | 1.3 | 1.4 | 1.5 | 1.4 | 0.9 | 0.6 | 0.9 | 1.3 | 0.9 | 1.3 | 1.6 | 1.1 | 2.5 | 1.2 | 1.5 | 0.88 |
| YJR018W | 0.8 | 1.2 | 1.8 | 0.7 | 1.1 | 1.0 | 0.9 | 0.7 | 0.4 | 0.7 | 0.9 | 0.8 | 1.5 | 0.9 | 1.4 | 0.8 | 1.0 | 0.7 | 0.35 |
| YKL147C | 1.0 | 1.3 | 1.9 | 0.9 | 0.8 | 0.7 | 0.8 | 0.7 | 1.0 | 0.5 | 0.3 | 0.9 | 1.0 | 1.0 | 0.7 | 1.1 | 0.9 | 0.9 | 0.28 |
| YKL169C | 1.0 | 1.0 | 1.6 | 1.8 | 0.8 | 1.2 | 1.5 | 1.2 | 0.3 | 0.5 | | 0.7 | 1.0 | 1.3 | 0.8 | 2.6 | 0.9 | 1.1 | 0.45 |
| YKR033C | 0.9 | 1.2 | 2.1 | 1.0 | 1.0 | 0.6 | 1.0 | 1.0 | 0.1 | 1.4 | 0.3 | 0.4 | 0.9 | 0.9 | 1.1 | 0.6 | 1.0 | 0.9 | 0.28 |

| | | | | | | | | | | | | | | | | | | | |
|---|---|---|---|---|---|---|---|---|---|---|---|---|---|---|---|---|---|---|---|
| YLL064C | 0.8 | 1.7 | 1.7 | 1.9 | 0.9 | 1.7 | 1.1 | 0.9 | 1.1 | 2.5 | 1.0 | 1.1 | 1.3 | 1.1 | 1.2 | 1.6 | 1.2 | 1.0 | 0.68 |
| YLR041W | 0.8 | 1.2 | 2.2 | 1.5 | 1.6 | 0.5 | 1.2 | 0.6 | | 0.4 | 1.1 | 0.4 | 0.3 | 0.5 | 1.3 | 0.5 | 0.7 | 0.9 | 0.69 |
| YLR177W | 0.8 | 1.2 | 1.8 | 2.2 | 0.8 | 1.1 | 1.3 | 0.9 | 1.0 | 2.3 | 0.9 | 1.1 | 1.5 | 1.0 | 0.5 | 1.3 | 1.1 | 1.5 | 1.25 |
| YMR007W | 1.1 | 0.9 | 2.0 | 0.7 | 1.3 | 1.7 | 3.1 | 0.7 | | 0.1 | 0.9 | 0.8 | 1.3 | 1.2 | 1.8 | 1.1 | 2.5 | 1.0 | 0.30 |
| YMR156C | 1.0 | 0.9 | 2.7 | 1.7 | 1.2 | 1.4 | 1.4 | 0.9 | 0.6 | 1.3 | 1.1 | 0.7 | 1.0 | 1.2 | 0.7 | 1.3 | 1.2 | 1.1 | 0.40 |
| YMR215W | 0.8 | 0.5 | 2.1 | 1.2 | 1.1 | 0.3 | 0.3 | 0.5 | 0.1 | 0.2 | 1.1 | 0.4 | 0.2 | 0.5 | 0.7 | 0.2 | 0.7 | 0.7 | 1.59 |
| YNL195C | 0.9 | 1.1 | 3.5 | 4.7 | 0.9 | 0.8 | 1.1 | 0.9 | 2.6 | 11.7 | 2.9 | 0.6 | 1.6 | 1.5 | 1.4 | 6.4 | 0.8 | 0.9 | 0.32 |
| YOL073C | 0.7 | 0.9 | 2.5 | 0.6 | 1.1 | 1.2 | 1.3 | 0.7 | 1.9 | 1.1 | 1.7 | 0.8 | 1.6 | 0.8 | 1.6 | 1.7 | 1.2 | 1.2 | 0.54 |
| YOR129C | 0.6 | 0.8 | 3.3 | 0.8 | 1.3 | 0.4 | 0.4 | 0.6 | 0.2 | 0.4 | 1.1 | 0.6 | 0.2 | 0.5 | 0.6 | 0.3 | 0.6 | 0.4 | 5.22 |
| YOR161C | 0.6 | 1.0 | 6.0 | 2.2 | 1.1 | 0.9 | 1.2 | 0.8 | 1.0 | 1.7 | 4.6 | 0.5 | 0.7 | 0.9 | 1.4 | 2.0 | 0.7 | 1.2 | 0.82 |
| YPL004C | 0.7 | 1.2 | 3.7 | 1.9 | 1.4 | 1.3 | 2.3 | 0.9 | 1.1 | 1.6 | 1.7 | 0.9 | 1.3 | 1.0 | 1.1 | 2.0 | 1.2 | 2.2 | 4.52 |
| YPL246C | 0.7 | 0.8 | 2.6 | 0.9 | 1.4 | 1.3 | 0.8 | 0.6 | 0.4 | 0.5 | 1.1 | 0.8 | 0.9 | 0.7 | 1.2 | 1.1 | 1.2 | 0.9 | 0.98 |
| YPL272C | 1.1 | 0.9 | 2.2 | 0.6 | 1.3 | 1.1 | 0.7 | 0.8 | 1.2 | 2.5 | 1.1 | 0.7 | 1.1 | 1.1 | 0.6 | 2.5 | 0.9 | 1.2 | 0.25 |
| YPR063C | 1.0 | 0.8 | 2.6 | 0.8 | 1.2 | 1.0 | 0.6 | 0.7 | 0.5 | 0.6 | 1.0 | 0.7 | 0.8 | 1.0 | 1.0 | 1.2 | 1.0 | 0.9 | 1.41 |
| YDL129W | 1.0 | 0.7 | 1.0 | 2.3 | 1.3 | 0.9 | 0.9 | 1.0 | 0.5 | 0.9 | 0.6 | 0.9 | 0.5 | 0.8 | 0.5 | 1.4 | 0.6 | 0.8 | 0.69 |
| YDR066C | 1.3 | 1.5 | 0.9 | 2.2 | 1.0 | 1.4 | 1.1 | 1.3 | 1.5 | 1.1 | 0.6 | 0.8 | 1.4 | 1.3 | 0.9 | 1.4 | 1.1 | 1.1 | 0.57 |
| YGL059W | 1.3 | 1.5 | 0.9 | 3.1 | 0.7 | 0.7 | 1.0 | 1.4 | 1.6 | 1.7 | 1.4 | 1.0 | 2.0 | 1.2 | 1.6 | 1.7 | 1.4 | 1.4 | 0.49 |
| YNL144C | 1.8 | 0.8 | 1.2 | 4.5 | 0.9 | 1.1 | 1.1 | 1.1 | 0.7 | 1.9 | 1.7 | 1.4 | 1.0 | 1.1 | 0.1 | 1.7 | 2.4 | 2.8 | 0.51 |
| YAL037W | 1.6 | 0.9 | 1.1 | 3.5 | 1.2 | 1.1 | 1.0 | 1.3 | 0.6 | 1.9 | 0.5 | 0.8 | 1.1 | 1.2 | 0.8 | 0.6 | 0.9 | 0.9 | 0.55 |
| YAR023C | 1.1 | 1.0 | 1.7 | 2.1 | 1.3 | 1.0 | 1.1 | 0.8 | 0.9 | 1.4 | 1.0 | 1.2 | 1.1 | 1.0 | 3.1 | 1.6 | 0.9 | 1.1 | 0.62 |
| YCR015C | 1.1 | 0.9 | 0.5 | 2.1 | 1.7 | 1.0 | 1.5 | 1.2 | 0.5 | 1.0 | 1.1 | 0.9 | 0.6 | 1.2 | 0.9 | 1.1 | 1.1 | 1.2 | 0.58 |
| YCR043C | 1.4 | 0.5 | 0.8 | 2.3 | 0.9 | 0.8 | 0.7 | 1.2 | 0.3 | 0.7 | 1.0 | 0.6 | 0.8 | 1.6 | 1.2 | 0.8 | 1.0 | 1.1 | 1.25 |
| YDL146W | 1.0 | 1.2 | 1.2 | 2.0 | 0.7 | 0.7 | 1.4 | 0.9 | 0.8 | 2.3 | 1.4 | 1.0 | 1.4 | 1.0 | 1.7 | 1.8 | 1.0 | 1.2 | 0.62 |
| YDR057W | 0.9 | 1.4 | 1.6 | 2.6 | 0.8 | 0.7 | 1.4 | 1.2 | 1.5 | 1.1 | 1.0 | 0.7 | 0.9 | 1.2 | 1.1 | 1.7 | 0.7 | 1.1 | 0.70 |
| YDR222W | 1.0 | 1.1 | 1.7 | 1.7 | 1.4 | 1.3 | 0.5 | 1.0 | 0.6 | 1.0 | 1.0 | 0.5 | 0.8 | 1.3 | 0.5 | 0.6 | 0.7 | 0.8 | 0.52 |
| YDR286C | 1.6 | 0.9 | 1.1 | 2.1 | 1.0 | 1.7 | 1.3 | 1.9 | 0.8 | 1.2 | 1.1 | 0.9 | 1.4 | 1.3 | 0.8 | 2.1 | 1.1 | 1.4 | 0.90 |
| YDR438W | 1.2 | 1.8 | 0.8 | 2.1 | 1.6 | 1.3 | 1.4 | 1.0 | 1.1 | 0.8 | 1.1 | 0.8 | 1.2 | 1.3 | 1.0 | 1.2 | 1.6 | 1.2 | 0.48 |
| YDR479C | 1.1 | 1.8 | 1.0 | 2.3 | 0.9 | 1.6 | 1.2 | 0.9 | 1.2 | 1.6 | 1.0 | 1.0 | 1.2 | 1.4 | 1.6 | 2.1 | 1.1 | 1.5 | 0.51 |
| YEL057C | 1.1 | 0.6 | 1.3 | 2.4 | 1.0 | 0.9 | 1.1 | 1.2 | 1.2 | 1.9 | 0.7 | 0.8 | 1.5 | 1.5 | 1.0 | 1.8 | 0.9 | 1.0 | 0.44 |
| YEL073C | 1.6 | 0.8 | 1.0 | 2.8 | 1.2 | 1.5 | 0.8 | 0.9 | 0.7 | 1.2 | 1.1 | 0.5 | 1.0 | 1.1 | 2.8 | 1.0 | 1.3 | 0.8 | 0.38 |
| YER084W | 0.8 | 1.1 | 1.0 | 3.9 | 0.9 | 0.6 | 0.8 | 0.9 | | 0.8 | | 0.8 | 0.7 | 0.9 | 2.6 | 1.5 | 0.8 | 0.9 | 0.30 |
| YER121W | 1.1 | 1.2 | 0.7 | 7.7 | 1.3 | 2.5 | 1.3 | 1.3 | 0.6 | 0.5 | 1.2 | 0.7 | 0.6 | 1.3 | 0.9 | 4.6 | 1.2 | 1.8 | 0.73 |

| | | | | | | | | | | | | | | | | | | | |
|---|---|---|---|---|---|---|---|---|---|---|---|---|---|---|---|---|---|---|---|
| YER189W | 0.9 | 0.8 | 0.9 | 1.9 | 1.3 | 0.5 | 0.9 | 0.7 | 0.4 | 0.5 | 0.7 | 0.8 | 0.7 | 0.9 | 0.9 | 0.8 | 0.8 | 0.8 | 0.43 |
| YFL017C | 1.3 | 1.2 | 0.9 | 2.3 | 1.5 | 1.3 | 1.5 | 1.4 | 0.8 | 0.6 | 1.1 | 1.0 | 0.7 | 1.1 | 0.6 | 1.8 | 1.5 | 1.8 | 1.21 |
| YFL046W | 1.3 | 0.9 | 0.7 | 2.1 | 1.7 | 0.7 | 1.3 | 1.3 | 0.7 | 0.7 | 1.3 | 0.8 | 0.7 | 1.3 | 1.0 | 1.1 | 1.4 | 1.6 | 0.68 |
| YFR008W | 1.1 | 1.0 | 0.8 | 2.2 | 1.1 | 1.0 | 1.1 | 0.9 | 1.5 | 1.4 | 0.7 | 0.9 | 0.8 | 1.1 | 0.8 | 1.5 | 1.0 | 1.0 | 1.26 |
| YGL214W | 0.9 | 1.0 | 1.2 | 2.6 | 1.2 | 0.9 | 0.8 | 0.9 | 0.4 | 0.7 | 0.4 | 0.8 | 0.6 | 1.1 | 0.9 | 0.8 | 1.0 | 0.8 | 0.61 |
| YGL218W | 1.4 | 0.9 | 1.4 | 3.4 | 1.2 | 1.1 | 0.8 | 1.1 | 1.3 | 0.4 | 0.1 | 0.8 | 1.3 | 0.9 | 1.1 | 0.7 | 1.0 | 1.0 | 0.53 |
| YGR021W | 0.9 | 1.5 | 1.0 | 8.6 | 1.0 | 0.9 | 1.3 | 1.4 | 0.7 | 0.6 | 0.6 | 0.9 | 0.9 | 1.2 | 0.8 | 2.1 | 1.0 | 1.0 | 0.58 |
| YGR024C | 1.2 | 0.9 | 0.7 | 2.6 | 1.0 | 0.7 | 1.3 | 1.4 | 0.4 | 1.2 | 1.0 | 0.7 | 0.5 | 1.4 | 1.0 | 1.2 | 0.8 | 1.3 | 1.61 |
| YGR064W | 1.1 | 1.2 | 1.6 | 2.3 | 0.7 | 0.6 | 1.1 | 1.0 | 0.1 | 0.8 | 1.7 | 0.8 | 0.9 | 2.1 | 1.0 | 1.3 | 0.8 | 0.9 | 0.50 |
| YGR182C | 1.6 | 1.2 | 1.2 | 2.3 | 1.1 | 0.9 | 0.7 | 1.1 | 0.5 | 0.7 | 0.9 | 0.7 | 0.7 | 2.2 | 0.4 | 2.0 | 1.0 | 1.7 | 1.95 |
| YGR236C | 1.6 | 1.6 | 0.4 | 4.1 | 1.2 | 1.2 | 1.0 | 1.2 | 1.5 | 0.7 | 1.0 | 0.9 | 1.2 | 1.2 | 0.7 | 7.9 | 1.5 | 1.9 | 0.30 |
| YHL042W | 1.2 | 0.8 | 1.3 | 51.1 | 1.0 | 0.7 | 1.2 | 1.0 | 0.8 | 1.8 | 0.7 | 0.6 | 1.3 | 1.2 | 1.1 | 1.9 | 1.1 | 0.9 | 0.37 |
| YIL012W | 1.2 | 5.8 | 1.5 | 2.8 | 1.5 | 1.5 | 0.9 | 0.9 | 1.7 | 2.8 | 0.9 | 0.6 | 0.4 | 1.1 | 1.1 | 0.9 | 1.0 | 0.9 | 0.28 |
| YIL028W | 1.0 | 1.1 | 0.8 | 5.4 | 1.4 | 0.7 | 1.1 | 0.8 | 0.8 | 1.7 | 1.1 | 0.5 | 1.0 | 1.1 | 0.9 | 1.1 | 1.3 | 1.0 | 0.27 |
| YIL057C | 1.3 | 1.8 | 1.1 | 4.6 | 1.3 | 1.2 | 1.2 | 1.4 | 0.1 | 5.0 | 0.8 | 0.6 | 0.9 | 1.5 | 0.8 | 11.1 | 1.1 | 3.6 | 0.26 |
| YIL089W | 1.3 | 1.3 | 0.7 | 3.9 | 1.6 | 1.6 | 1.6 | 1.1 | | 0.6 | 0.7 | 0.6 | 0.7 | 1.4 | 0.6 | 1.3 | 1.4 | 1.3 | 0.31 |
| YIL102C | 1.4 | 1.6 | 1.0 | 6.1 | 1.5 | 0.8 | 1.1 | 0.7 | | 0.3 | | 0.6 | 0.7 | 1.0 | 0.8 | 0.6 | 1.2 | 1.0 | 0.18 |
| YIL113W | 0.9 | 1.0 | 0.9 | 2.6 | 0.6 | 1.0 | 1.9 | 0.7 | 1.9 | 2.2 | 2.5 | 0.8 | 1.3 | 1.2 | 0.9 | 1.6 | 1.4 | 1.3 | 0.48 |
| YIL122W | 1.2 | 1.1 | 1.1 | 2.1 | 0.9 | 0.2 | 0.8 | 0.7 | 0.4 | 0.8 | 2.1 | 1.0 | 0.7 | 1.0 | 1.6 | 1.4 | 0.9 | 0.9 | 0.35 |
| YJL100W | 1.4 | 2.0 | 0.9 | 2.3 | 1.3 | 1.1 | 1.2 | 1.2 | 1.8 | 2.7 | 1.5 | 0.9 | 1.4 | 1.2 | 1.2 | 1.4 | 1.5 | 1.1 | 0.38 |
| YJL169W | 0.8 | 1.1 | 1.5 | 2.8 | 1.4 | 0.8 | 0.9 | 0.7 | 0.3 | 0.5 | 0.4 | 0.5 | 0.7 | 1.0 | 0.9 | 0.6 | 1.1 | 0.9 | 0.35 |
| YJL199C | 1.1 | 0.7 | 0.9 | 4.5 | 1.0 | 1.7 | 1.0 | 1.0 | 1.0 | 1.6 | 1.0 | 0.8 | 0.9 | 1.9 | 0.9 | 3.7 | 1.0 | 1.0 | 0.59 |
| YJR039W | 0.9 | 0.6 | 0.8 | 3.0 | 1.8 | 1.0 | 1.6 | 1.2 | 1.8 | 1.6 | 1.8 | 0.7 | 1.5 | 0.6 | 1.2 | 1.5 | 1.3 | 1.3 | 0.24 |
| YJR101W | 0.8 | 0.6 | 0.7 | 2.9 | 1.8 | 0.9 | 1.5 | 1.1 | 0.4 | 0.8 | 1.2 | 0.5 | 0.5 | 0.9 | 0.5 | 1.2 | 1.0 | 1.1 | 2.04 |
| YKL061W | 1.3 | 0.7 | 0.7 | 1.8 | 0.8 | 1.0 | 0.9 | 1.2 | 0.8 | 1.0 | 1.1 | 0.7 | 0.8 | 1.2 | 0.6 | 1.5 | 1.3 | 1.6 | 0.92 |
| YKL121W | 0.9 | 7.8 | 0.8 | 11.8 | 1.6 | 1.2 | 1.7 | 1.0 | 0.8 | 0.9 | 1.6 | 0.8 | 1.0 | 1.2 | 0.8 | 1.6 | 1.6 | 1.1 | 0.31 |
| YKL160W | 1.5 | 1.0 | 0.4 | 1.8 | 1.1 | 1.5 | 1.5 | 2.1 | 1.3 | 1.9 | 1.4 | 0.9 | 1.6 | 1.5 | 0.8 | 1.6 | 1.5 | 2.2 | 2.19 |
| YLR016C | 0.7 | 1.3 | 0.5 | 2.0 | 1.5 | 0.8 | 1.1 | 1.2 | 0.8 | 1.1 | 1.0 | 0.7 | 0.8 | 0.9 | 0.7 | 1.3 | 1.0 | 1.3 | 1.15 |
| YLR030W | 1.4 | 1.5 | 0.8 | 2.5 | 1.2 | 1.5 | 1.5 | 0.9 | 0.3 | 2.7 | 0.2 | 0.8 | 1.5 | 1.2 | 0.6 | 2.1 | 1.0 | 1.0 | 0.29 |
| YLR036C | 1.3 | 0.8 | 0.7 | 2.0 | 1.5 | 1.3 | 1.7 | 1.2 | 0.4 | 1.6 | 0.8 | 0.7 | 0.7 | 1.3 | 0.6 | 1.4 | 1.0 | 1.4 | 1.07 |
| YLR112W | 1.1 | 0.9 | 1.1 | 1.7 | 1.3 | 1.3 | 1.0 | 0.7 | | 0.7 | 0.5 | 0.5 | 0.7 | 0.8 | 0.4 | 0.4 | 1.0 | 0.8 | 0.39 |
| YLR125W | 1.4 | 2.0 | 0.5 | 3.1 | 1.8 | 0.7 | 1.3 | 1.2 | 0.5 | 1.7 | 1.1 | 0.5 | 0.8 | 1.3 | 0.7 | 1.6 | 1.2 | 1.2 | 0.29 |

| | | | | | | | | | | | | | | | | | | | |
|---|---|---|---|---|---|---|---|---|---|---|---|---|---|---|---|---|---|---|---|
| YLR128W | 1.3 | 1.4 | 0.8 | 1.8 | | 1.3 | 1.3 | 1.1 | 0.8 | 1.7 | 1.4 | 0.7 | 1.2 | 1.4 | 0.9 | 1.5 | 1.2 | 1.2 | 0.25 |
| YLR204W | 1.1 | 0.9 | 0.5 | 2.1 | 2.0 | 1.5 | 2.0 | 1.3 | 1.4 | 1.6 | 1.6 | 0.8 | 1.1 | 1.8 | 1.0 | 2.5 | 1.2 | 1.3 | 1.26 |
| YLR211C | 1.3 | 1.0 | 0.8 | 7.7 | 1.7 | 1.2 | 1.8 | 1.1 | 0.9 | 1.2 | 0.8 | 0.7 | 1.1 | 1.2 | 3.6 | 1.9 | 1.3 | 1.3 | 0.35 |
| YLR257W | 1.5 | 1.0 | 0.7 | 2.4 | 1.4 | 1.2 | 0.8 | 1.3 | 1.0 | 1.1 | 1.2 | 1.0 | 1.0 | 0.9 | 1.0 | 1.7 | 1.8 | 2.6 | 5.20 |
| YLR326W | 1.2 | 1.0 | 1.5 | 2.2 | 1.3 | 0.4 | 1.2 | 0.9 | 0.7 | 1.5 | 6.6 | 0.5 | 0.4 | 1.1 | 0.7 | 0.9 | 0.8 | 1.0 | 0.33 |
| YLR334C | 1.2 | 1.1 | 1.1 | 2.3 | 1.8 | 1.0 | 1.4 | 0.9 | 1.0 | 0.8 | 0.8 | 0.8 | 0.9 | 2.2 | 2.5 | 0.7 | 0.8 | 1.0 | 0.30 |
| YLR408C | 1.3 | 2.3 | 0.6 | 2.3 | 1.4 | 1.0 | 1.0 | 1.4 | 0.9 | 1.1 | 0.7 | 0.8 | 1.1 | 2.1 | 0.9 | 1.1 | 1.2 | 1.2 | 0.79 |
| YLR414C | 1.5 | 1.6 | 1.4 | 4.8 | 1.3 | 0.6 | 0.7 | 0.9 | 0.2 | 1.2 | 1.2 | 0.6 | 1.0 | 1.0 | 2.1 | 0.9 | 3.9 | 3.6 | 2.21 |
| YLR444C | 0.9 | 0.8 | 1.7 | 5.9 | 0.7 | 0.7 | 0.9 | 0.9 | 0.2 | 0.2 | 1.1 | 0.6 | 0.5 | 1.1 | 1.3 | 0.7 | 0.9 | 0.9 | 0.50 |
| YML050W | 1.5 | 1.2 | 1.3 | 2.1 | 0.8 | 0.5 | 0.9 | 1.1 | 1.3 | 1.6 | 1.2 | 0.8 | 0.9 | 1.9 | 1.0 | 1.5 | 0.8 | 1.1 | 0.42 |
| YML107C | 1.1 | 0.7 | 0.4 | 1.8 | 2.2 | 1.1 | 1.5 | 1.3 | 0.6 | 0.9 | 0.9 | 0.7 | 0.6 | 1.3 | 1.0 | 1.3 | 1.2 | 1.5 | 0.67 |
| YMR031C | 0.7 | 0.8 | 1.2 | 2.1 | 0.6 | 0.7 | 0.8 | 0.9 | 1.5 | 1.1 | 0.8 | 0.7 | 1.0 | 1.0 | 0.7 | 1.6 | 0.9 | 1.3 | 1.26 |
| YMR204C | 1.1 | 1.9 | 1.3 | 2.3 | 1.0 | 0.6 | 0.7 | 1.0 | 1.0 | 2.2 | 0.7 | 0.7 | 1.1 | 1.2 | 1.5 | 1.1 | 0.9 | 1.1 | 0.31 |
| YMR206W | 1.4 | 1.4 | 2.3 | 3.9 | 1.1 | 1.0 | 1.3 | 0.9 | 1.0 | 9.0 | 1.8 | 1.0 | 1.2 | 1.4 | 0.3 | 2.2 | 1.0 | 1.2 | 0.17 |
| YNL010W | 1.5 | 1.0 | 0.7 | 1.9 | 1.0 | 1.2 | 1.1 | 1.8 | 0.5 | 0.6 | 1.1 | 0.9 | 0.7 | 1.2 | 0.9 | 1.2 | 1.0 | 1.7 | 4.53 |
| YNL127W | 1.1 | 1.2 | 0.7 | 3.6 | 0.7 | 1.2 | 0.8 | 1.0 | 2.7 | 2.2 | 1.0 | 1.0 | 1.7 | 1.0 | 1.5 | 1.4 | 0.9 | 1.4 | 0.36 |
| YNL217W | 1.1 | 1.2 | 0.5 | 1.7 | 2.1 | 1.5 | 1.3 | 1.4 | 0.3 | 0.9 | 0.7 | 1.0 | 1.0 | 0.9 | 0.5 | 1.0 | 1.3 | 2.1 | 2.35 |
| YOL118C | 2.6 | 2.2 | 1.3 | 2.2 | | 1.5 | 3.2 | 1.1 | | 3.2 | 1.4 | 0.6 | 1.7 | 3.0 | 1.0 | 0.5 | 0.9 | 1.0 | 0.24 |
| YOR053W | 1.0 | 1.1 | 0.5 | 1.7 | 1.7 | 0.9 | 1.6 | 0.9 | 1.3 | 0.8 | 1.3 | 0.6 | 0.9 | 1.2 | 1.0 | 1.3 | 1.2 | 1.2 | 0.77 |
| YOR352W | 1.3 | 0.9 | 1.0 | 2.2 | 1.6 | 0.9 | 1.8 | 1.1 | 1.7 | 2.0 | 1.1 | 1.0 | 1.2 | 1.3 | 0.7 | 2.5 | 1.3 | 1.7 | 0.59 |
| YOR394W | 1.2 | 0.8 | 2.2 | 1.6 | 1.6 | 1.7 | 1.8 | 1.0 | 1.1 | 2.3 | 1.9 | 0.9 | 1.3 | 0.9 | 0.9 | 1.7 | 1.6 | 1.1 | 0.51 |
| YPL033C | 0.8 | 1.0 | 0.9 | 5.1 | 1.3 | 0.7 | 1.0 | 0.7 | 0.4 | 1.6 | 0.7 | 0.6 | 0.7 | 1.1 | 0.8 | 0.4 | 0.9 | 0.8 | 0.23 |
| YPL066W | 0.9 | 1.0 | 1.1 | 2.0 | 1.3 | 1.3 | 1.3 | 0.9 | 0.5 | 0.8 | 1.0 | 1.1 | 0.9 | 0.7 | 0.7 | 1.2 | 1.3 | 1.3 | 0.77 |
| YPR014C | 1.0 | 0.7 | 1.1 | 7.9 | 0.8 | 0.9 | 0.8 | 0.9 | | 0.9 | 0.5 | 0.7 | 0.7 | 1.0 | 0.9 | 0.8 | 0.9 | 1.1 | 0.29 |
| YBR005W | 1.9 | 2.6 | 1.1 | 2.9 | 0.8 | 0.7 | 1.2 | 0.9 | 0.4 | 1.3 | 0.8 | 1.7 | 1.9 | 0.9 | 1.1 | 1.7 | 2.9 | 4.6 | 1.00 |
| YFL027C | 0.8 | 0.8 | 1.1 | 1.3 | 1.1 | 0.3 | 0.4 | 1.1 | 1.8 | 1.3 | 1.3 | 0.6 | 1.1 | 0.3 | 1.4 | 1.2 | 5.1 | 5.2 | 1.74 |
| YGL080W | 1.3 | 1.5 | 1.1 | 1.3 | 1.5 | 1.4 | 1.5 | 1.2 | 1.1 | 1.2 | 0.8 | 1.0 | 0.7 | 1.6 | 0.9 | 1.7 | 1.3 | 2.9 | 1.70 |
| YMR252C | 1.1 | 1.0 | 1.5 | 1.3 | 1.3 | 1.0 | 1.3 | 1.1 | 0.9 | 1.0 | 1.4 | 0.8 | 1.1 | 1.1 | 1.1 | 2.1 | 1.5 | 2.2 | 0.86 |
| YOR385W | 2.8 | 1.2 | 0.2 | 2.5 | 1.5 | 1.0 | 1.2 | 1.0 | 1.5 | 2.2 | 1.2 | 0.6 | 2.8 | 0.8 | 0.8 | 1.2 | 4.6 | 5.9 | 1.17 |
| YAR033W | 1.0 | 0.8 | 1.5 | 1.1 | 1.1 | 1.4 | 2.1 | 1.2 | 1.3 | 1.6 | 1.5 | 0.9 | 1.5 | 1.3 | 0.9 | 2.0 | 1.1 | 3.0 | 1.15 |
| YBR151W | 0.8 | 0.8 | 1.3 | 1.2 | 1.2 | 1.7 | 1.3 | 0.8 | 0.8 | 0.9 | 0.9 | 0.9 | 1.1 | 0.8 | 1.0 | 3.0 | 1.4 | 2.4 | 1.83 |
| YHR162W | 1.2 | 1.2 | 0.9 | 1.8 | 1.2 | 1.3 | 1.7 | 1.4 | 1.0 | 1.7 | 1.3 | 1.2 | 1.5 | 2.0 | 1.1 | 1.4 | 1.3 | 2.7 | 3.64 |

| | | | | | | | | | | | | | | | | | | | |
|---|---|---|---|---|---|---|---|---|---|---|---|---|---|---|---|---|---|---|---|
| YLR165C | 1.4 | 0.8 | 1.3 | 1.1 | 1.1 | 1.5 | 1.4 | 1.1 | 1.7 | 1.0 | 1.4 | 0.9 | 1.2 | 1.4 | 0.5 | 1.6 | 1.1 | 2.1 | 0.78 |
| YNL157W | 1.4 | 1.1 | 0.7 | 1.5 | 1.9 | 0.9 | 1.3 | 1.1 | 1.7 | 1.4 | 1.3 | 0.9 | 1.6 | 1.5 | 1.0 | 1.0 | 1.9 | 2.3 | 1.41 |
| YOL002C | 0.9 | 0.7 | 0.5 | 1.0 | 2.0 | 0.9 | 0.4 | 0.8 | 0.1 | 0.3 | 0.8 | 1.1 | 0.2 | 0.5 | 0.6 | 1.7 | 1.5 | 2.1 | 5.33 |
| YPL233W | 1.2 | 0.9 | 0.5 | 1.1 | 1.4 | 0.6 | 1.0 | 1.0 | 0.3 | 0.6 | 0.9 | 0.6 | 1.0 | 1.0 | 0.4 | 1.0 | 1.9 | 1.9 | 0.48 |
| YGR149W | 1.4 | 1.3 | 1.8 | 1.9 | 1.0 | 1.7 | 1.8 | 0.8 | 0.6 | 0.6 | 1.8 | 1.2 | 2.6 | 1.0 | 2.4 | 1.3 | 2.0 | 1.7 | 0.71 |
| YNL043C | 1.0 | 4.4 | 1.6 | 1.3 | 0.8 | 0.6 | 0.9 | 0.9 | 0.4 | 0.4 | 0.7 | 0.9 | 2.1 | 1.2 | 1.6 | 0.8 | 2.7 | 1.6 | 0.68 |
| YPL067C | 1.2 | 1.6 | 2.8 | 1.4 | 1.0 | 0.8 | 1.2 | 1.2 | | 0.8 | 0.9 | 1.4 | 0.9 | 0.7 | 1.5 | 1.1 | 2.9 | 1.7 | 0.40 |
| YPL170W | 1.2 | 1.3 | 0.9 | 1.1 | 1.9 | 1.1 | 0.6 | 1.1 | 1.4 | 1.0 | 1.1 | 0.9 | 1.1 | 1.4 | 1.2 | 1.6 | 2.2 | 1.4 | 1.05 |
| YGL051W | 1.1 | 1.0 | 1.6 | 1.7 | 1.3 | 1.0 | 1.3 | 0.8 | 1.1 | 1.8 | 1.4 | 1.0 | 1.5 | 1.2 | 0.8 | 2.6 | 1.4 | 1.9 | 1.06 |
| YIL112W | 1.1 | 1.4 | 2.8 | 1.9 | 0.6 | 1.3 | 1.1 | 0.9 | 1.3 | 2.0 | 1.4 | 1.2 | 1.1 | 1.3 | 1.8 | 2.4 | 1.2 | 1.3 | 0.45 |
| YLR052W | 1.0 | 1.5 | 0.8 | 1.3 | 1.0 | 0.8 | 1.0 | 1.2 | 0.4 | 1.1 | 1.1 | 1.0 | 0.8 | 1.2 | 1.0 | 2.4 | 0.7 | 0.9 | 0.77 |
| YNL203C | 0.9 | 1.3 | 0.3 | 3.2 | 1.0 | 1.7 | 1.6 | 0.8 | | 0.3 | 1.1 | 0.9 | 1.2 | 2.7 | 1.2 | 5.6 | 1.3 | 1.7 | 0.21 |
| YNR014W | 1.0 | 0.8 | 1.7 | 1.2 | 1.2 | 0.7 | 1.7 | 0.9 | 0.3 | 2.3 | 4.4 | 0.9 | 1.5 | 4.9 | 1.0 | 2.4 | 1.1 | 1.0 | 0.31 |
| YAL028W | 0.9 | 0.7 | 1.1 | 2.0 | 1.0 | 0.6 | 1.2 | 0.8 | 1.6 | 1.3 | 1.0 | 1.0 | 1.5 | 1.6 | 2.2 | 1.7 | 1.0 | 1.0 | 0.30 |
| YBL095W | 0.8 | 1.4 | 1.2 | 1.4 | 1.1 | 0.9 | 1.1 | 0.9 | 1.0 | 0.6 | 1.2 | 0.8 | 1.0 | 1.0 | 0.4 | 2.2 | 1.3 | 1.9 | 0.72 |
| YBR157C | 0.7 | 1.0 | 0.4 | 1.4 | 1.3 | 1.2 | 1.6 | 0.7 | 0.6 | 0.3 | 0.6 | 0.6 | 0.5 | 0.9 | 0.2 | 1.9 | 1.3 | 0.8 | 0.69 |
| YDL091C | 1.1 | 1.3 | 1.8 | 1.4 | 0.7 | 0.8 | 1.3 | 1.3 | 1.6 | 1.5 | 1.5 | 1.0 | 1.3 | 1.5 | 1.9 | 2.4 | 1.1 | 1.6 | 0.47 |
| YDL216C | 1.1 | 1.1 | 0.7 | 2.0 | 1.6 | 1.6 | 1.2 | 1.6 | 1.3 | 1.3 | 0.7 | 0.9 | 1.2 | 1.1 | 1.0 | 1.8 | 1.0 | 1.6 | 0.42 |
| YDR067C | 1.3 | 1.2 | 0.7 | 2.7 | 0.9 | 1.1 | 1.3 | 1.8 | 1.0 | 1.3 | 0.9 | 0.9 | 1.2 | 1.5 | 1.5 | 2.4 | 1.3 | 1.4 | 0.76 |
| YDR186C | 0.7 | 0.9 | 0.7 | 0.7 | 1.3 | 0.7 | 1.4 | 1.2 | 1.2 | 1.6 | 0.7 | 1.1 | 1.1 | 0.8 | 0.8 | 2.3 | 1.1 | 0.9 | 0.50 |
| YDR196C | 0.9 | 0.9 | 0.7 | 1.1 | 1.0 | 1.7 | 1.7 | 1.5 | 1.6 | 1.1 | 1.3 | 0.7 | 1.2 | 1.0 | 0.7 | 2.7 | 1.2 | 1.9 | 1.54 |
| YDR262W | 1.3 | 1.8 | 0.8 | 1.2 | 1.7 | 1.3 | 1.7 | 1.2 | 0.9 | 1.7 | 1.6 | 0.8 | 0.9 | 1.7 | 1.5 | 3.8 | 1.0 | 1.4 | 1.11 |
| YDR306C | 0.9 | 1.2 | 0.7 | 1.8 | 1.7 | 1.2 | 1.6 | 1.3 | 2.0 | 1.4 | 2.1 | 0.9 | 1.2 | 1.1 | 1.2 | 2.4 | 1.0 | 1.5 | 0.77 |
| YDR319C | 1.3 | 0.9 | 0.8 | 1.1 | 1.2 | 1.2 | 1.7 | 1.3 | 1.1 | 1.1 | 1.3 | 1.0 | | 1.3 | 0.8 | 2.0 | 2.1 | 2.2 | 1.23 |
| YER188W | 1.3 | 0.9 | 1.2 | 1.9 | 0.9 | 1.5 | 1.2 | 0.7 | 0.4 | 0.9 | 1.2 | 0.9 | 1.1 | 1.1 | 0.3 | 2.5 | 1.0 | 1.1 | 0.61 |
| YGL004C | 0.8 | 1.4 | 1.3 | 1.0 | 1.0 | 1.4 | 2.9 | 1.4 | 1.8 | 2.2 | 1.0 | 0.8 | 1.3 | 0.9 | 0.6 | 2.2 | 1.1 | 1.4 | 0.48 |
| YGR141W | 0.8 | 1.6 | 1.6 | 1.7 | 1.1 | 1.5 | 1.5 | 0.9 | 0.3 | 1.0 | 0.6 | 0.6 | 1.0 | 0.7 | 2.6 | 2.1 | 1.1 | 1.3 | 0.65 |
| YHR080C | 0.6 | 1.8 | 1.9 | 0.7 | 0.8 | 1.8 | 1.1 | 1.1 | 1.9 | 1.2 | 0.7 | 1.2 | 2.0 | 0.9 | 0.9 | 2.3 | 1.1 | 1.3 | 0.49 |
| YHR097C | 1.6 | 0.7 | 0.7 | 0.9 | | 0.8 | 0.8 | 0.7 | 0.4 | 0.6 | 0.7 | 0.8 | 1.3 | 0.9 | 2.7 | 2.0 | 1.7 | 1.4 | 0.24 |
| YIL077C | 0.9 | 0.8 | 1.2 | 1.2 | 1.3 | 1.8 | 1.2 | 0.9 | 1.5 | 1.4 | 1.3 | 1.6 | 1.3 | 1.3 | 0.9 | 2.4 | 1.3 | 1.1 | 0.75 |
| YJL046W | 0.9 | 0.9 | 0.6 | 1.8 | 1.0 | 0.7 | 1.5 | 1.1 | 1.8 | 2.7 | 4.2 | 0.9 | 1.3 | 1.3 | 1.0 | 2.5 | 1.0 | 1.3 | 0.68 |
| YLL005C | 1.0 | 1.1 | 1.1 | 1.2 | 1.6 | 1.9 | 1.3 | 1.0 | 0.4 | 1.0 | 0.9 | 0.8 | 1.1 | 1.7 | 1.0 | 2.1 | 1.1 | 1.2 | 0.44 |

| | | | | | | | | | | | | | | | | | | | |
|---|---|---|---|---|---|---|---|---|---|---|---|---|---|---|---|---|---|---|---|
| YLR151C | 1.4 | 2.0 | 1.4 | 1.0 | 1.0 | | 1.4 | 1.4 | 1.5 | 0.9 | 1.1 | 0.5 | 0.7 | 1.3 | 0.7 | 2.4 | 1.0 | 1.4 | 0.30 |
| YLR271W | 1.6 | 1.2 | 0.8 | 1.8 | 1.4 | 1.4 | 1.7 | 1.5 | 1.0 | 1.0 | 1.3 | 0.8 | 1.2 | 1.2 | 1.0 | 3.0 | 1.5 | 1.8 | 0.78 |
| YMR025W | 1.2 | 1.2 | 0.7 | 1.5 | 1.3 | 1.3 | 1.4 | 1.2 | 1.0 | 1.6 | 1.2 | 0.9 | 1.2 | 1.8 | 1.1 | 2.4 | 1.0 | 1.5 | 0.42 |
| YMR135C | 1.0 | 1.0 | 1.1 | 1.9 | 1.8 | 1.0 | 1.0 | 1.0 | 1.1 | 1.4 | 1.1 | 1.0 | 1.3 | 1.0 | 1.4 | 2.3 | 1.5 | 1.3 | 1.06 |
| YMR210W | 1.0 | 1.1 | 1.8 | 1.9 | 0.6 | 0.8 | 1.1 | 1.1 | 1.5 | 2.2 | 0.8 | 0.9 | 1.0 | 1.0 | 1.0 | 2.2 | 0.8 | 1.5 | 0.56 |
| YNR040W | 0.8 | 4.9 | 0.5 | 0.9 | 0.9 | 1.6 | 2.1 | 1.3 | 1.1 | 0.8 | 1.2 | 0.6 | 0.8 | 1.5 | 0.7 | 2.1 | 1.1 | 1.0 | 0.30 |
| YPL039W | 1.2 | 0.9 | 0.6 | 1.3 | 1.4 | 1.5 | 1.5 | 1.4 | 1.1 | 1.0 | 0.9 | 0.9 | 1.1 | 1.1 | 0.6 | 2.4 | 1.1 | 1.1 | 0.52 |
| YPL099C | 1.1 | 1.9 | 0.7 | 2.0 | 0.9 | 1.2 | 1.4 | 1.4 | 2.4 | 1.7 | 0.8 | 0.9 | 1.1 | 1.3 | 0.5 | 2.1 | 1.0 | 1.7 | 0.92 |
| YPL107W | 1.2 | 0.8 | 1.1 | 1.3 | 1.0 | 1.1 | 1.2 | 1.5 | 1.4 | 1.3 | 1.1 | 0.7 | 0.8 | 1.7 | 0.7 | 1.8 | 1.0 | 1.1 | 0.59 |
| YPL138C | 1.1 | 1.7 | 1.1 | 1.8 | 0.7 | 0.5 | 1.1 | 1.3 | 1.1 | 1.5 | 0.5 | 0.9 | 0.6 | 1.3 | 2.7 | 2.6 | 0.8 | 1.1 | 0.38 |

## Table 2 Mitochondria-located protein genes

The level of expressed mRNA in the presence of chemical substance/ The level of expressed mRNA in the absence of chemical substance

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| YJR048W | 1.3 | 0.9 | 2.2 | 1.4 | 0.8 | 0.5 | 0.3 | 0.9 | 0.5 | 1.5 | 1.0 | 0.8 | 0.4 | 2.5 | 0.6 | 1.5 | 1.2 | 1.2 | 1.19 |
| YOR226C | 1.3 | 2.2 | 1.7 | 1.1 | 1.3 | 1.2 | 1.2 | 1.2 | 1.3 | 5.9 | 1.5 | 0.6 | 0.9 | 2.7 | 1.0 | 0.7 | 1.0 | 0.7 | 0.58 |
| YDL174C | 0.9 | 1.8 | 0.9 | 1.3 | 0.5 | 1.7 | 3.6 | 0.8 | 0.5 | 1.7 | 0.9 | 2.8 | 5.9 | 1.1 | 0.6 | 5.1 | 2.2 | 4.0 | 0.63 |
| YBL022C | 0.4 | 0.8 | 1.2 | 0.7 | 1.2 | 1.2 | 1.0 | 0.4 | 2.8 | 1.2 | 1.1 | 0.9 | 2.6 | 1.0 | 1.5 | 1.0 | 0.9 | 0.7 | 0.76 |
| YCL057W | 0.6 | 1.5 | 2.3 | 0.5 | 1.1 | 1.8 | 1.4 | 1.8 | 4.2 | 2.1 | 1.6 | 1.0 | 3.8 | 1.0 | 2.8 | 1.5 | 0.9 | 1.1 | 0.76 |
| YDR258C | 1.6 | 2.3 | 2.8 | 1.4 | 1.3 | 2.4 | 3.9 | 1.9 | 4.9 | 13.3 | 1.7 | 1.8 | 5.2 | 1.5 | 0.6 | 1.0 | 1.3 | 1.3 | 0.87 |
| YGR028W | 1.4 | 3.5 | 1.0 | 1.8 | 0.9 | 1.5 | 2.1 | 1.4 | 1.7 | 2.0 | 1.1 | 1.5 | 2.6 | 1.2 | 1.0 | 2.2 | 1.5 | 2.6 | 0.91 |
| YGR244C | 1.0 | 1.2 | 1.6 | 1.6 | 0.8 | 2.6 | 3.9 | 1.8 | 1.4 | 1.1 | 1.8 | 1.9 | 2.6 | 1.2 | 2.0 | 2.6 | 1.9 | 3.1 | 1.12 |
| YKL142W | 1.8 | 2.7 | 3.1 | 1.4 | 0.7 | 3.4 | 6.9 | 2.3 | 5.8 | 5.9 | 2.0 | 1.9 | 3.3 | 2.3 | 1.3 | 3.3 | 2.4 | 3.6 | 1.68 |
| YNL055C | 1.1 | 1.8 | 5.5 | 2.0 | 0.9 | 2.6 | 1.2 | 0.7 | 1.1 | 1.0 | 1.9 | 1.7 | 2.4 | 0.9 | 1.1 | 2.6 | 1.2 | 1.7 | 4.10 |
| YNL071W | 0.9 | 0.9 | 1.7 | 1.5 | 0.6 | 1.3 | 1.4 | 0.9 | 1.1 | 1.2 | 1.6 | 1.3 | 2.5 | 0.8 | 1.1 | 1.1 | 1.0 | 1.2 | 1.61 |
| YOR020C | 1.4 | 2.1 | 1.7 | 1.7 | 1.5 | 2.3 | 1.7 | 1.7 | 5.8 | 2.6 | 2.5 | 0.8 | 3.0 | 1.6 | 1.5 | 1.4 | 1.4 | 1.7 | 1.66 |
| YOR037W | 1.0 | 1.5 | 0.6 | 1.4 | 1.5 | 1.6 | 2.5 | 1.5 | 1.3 | 1.0 | 1.2 | 1.2 | 2.1 | 1.0 | 0.9 | 1.4 | 1.2 | 1.6 | 0.61 |
| YDL198C | 1.4 | 1.0 | 2.1 | 1.4 | 0.7 | 0.8 | 1.0 | 1.1 | 1.9 | 2.7 | 2.5 | 1.2 | 2.0 | 1.7 | 1.2 | 1.4 | 1.5 | 1.1 | 1.19 |
| YDR231C | 1.1 | 1.6 | 1.0 | 1.5 | 1.1 | 2.2 | 1.7 | 1.3 | 1.5 | 1.4 | 1.3 | 1.1 | 2.4 | 1.5 | 1.0 | 3.0 | 1.2 | 1.3 | 0.92 |
| YER178W | 0.8 | 0.9 | 3.6 | 1.0 | 0.7 | 2.5 | 1.6 | 0.8 | 1.7 | 1.3 | 2.5 | 1.3 | 2.4 | 0.9 | 1.8 | 1.0 | 1.1 | 0.9 | 2.18 |
| YFL016C | 0.8 | 1.0 | 1.6 | 0.9 | 1.3 | 1.3 | 1.4 | 0.7 | 6.4 | 2.9 | 1.2 | 1.0 | 2.8 | 0.8 | 0.8 | 0.6 | 1.2 | 0.9 | 1.25 |
| YGR008C | 2.1 | 3.0 | 1.7 | 3.2 | 0.9 | 2.9 | 3.7 | 1.9 | 3.1 | 2.4 | 2.6 | 2.0 | 3.4 | 1.3 | 1.5 | 2.3 | 1.7 | 4.2 | 3.03 |
| YIL155C | 1.0 | 0.7 | 1.4 | 3.7 | 1.3 | 1.4 | 2.2 | 1.0 | 2.5 | 2.9 | 1.3 | 1.4 | 2.0 | 1.3 | 1.4 | 3.8 | 1.1 | 1.4 | 0.51 |
| YJL102W | 1.1 | 0.7 | 0.4 | 0.8 | 1.3 | 1.6 | 0.6 | 0.9 | 3.1 | 2.2 | 0.7 | 0.8 | 2.6 | 1.5 | 1.5 | 0.6 | 1.0 | 1.1 | 0.22 |
| YJR045C | 0.5 | 1.9 | 3.9 | | 0.5 | 1.2 | 1.0 | 0.8 | 3.2 | 3.0 | 1.4 | 1.0 | 2.3 | 0.5 | 2.3 | 0.8 | 0.7 | 0.9 | 3.78 |
| YLR259C | 0.7 | 1.0 | 3.1 | 1.6 | 1.1 | 0.9 | 1.8 | 0.8 | 1.8 | 2.6 | 3.0 | 0.7 | 2.5 | 0.8 | 2.1 | 1.3 | 0.8 | 1.0 | 2.09 |
| YLR348C | 1.1 | 4.5 | 1.2 | 1.2 | 0.9 | 1.9 | 0.9 | 0.9 | 2.1 | 1.3 | 1.3 | 0.9 | 1.9 | 1.0 | 2.4 | 1.1 | 1.1 | 1.0 | 0.64 |
| YML054C | 1.5 | 1.8 | 1.3 | 3.4 | 1.3 | 1.8 | 1.2 | 1.5 | 4.1 | 1.8 | 1.4 | 1.8 | 2.8 | 2.1 | 1.1 | 7.8 | 1.1 | 1.6 | 0.25 |
| YMR089C | 0.8 | 1.1 | 0.9 | 0.9 | 0.8 | 1.2 | 1.2 | 1.3 | 5.6 | 2.5 | 1.3 | 0.8 | 2.3 | 0.9 | 1.8 | 1.1 | 0.9 | 1.1 | 0.69 |
| YMR152W | 0.9 | 1.1 | 1.8 | 0.9 | 0.8 | 7.5 | 0.8 | 0.8 | 2.7 | 1.3 | 1.2 | 1.3 | 1.9 | 1.1 | 0.6 | 1.0 | 1.5 | 0.9 | 0.71 |
| YNL104C | 1.1 | 1.3 | 3.0 | 0.9 | 0.8 | 0.8 | 0.6 | 0.9 | 1.1 | 2.0 | 2.1 | 1.1 | 2.1 | 0.9 | 2.5 | 1.5 | 0.9 | 0.9 | 1.69 |
| YOR130C | 0.8 | 1.7 | 1.4 | 1.0 | 1.8 | 1.6 | 1.3 | 0.8 | 2.0 | 1.7 | 0.6 | 0.9 | 2.1 | 1.3 | 1.0 | 0.8 | 1.5 | 1.1 | 0.51 |

| | | | | | | | | | | | | | | | | | | | |
|---|---|---|---|---|---|---|---|---|---|---|---|---|---|---|---|---|---|---|---|
| YPR024W | 0.7 | 1.0 | 1.3 | 1.0 | 1.3 | 1.0 | 1.4 | 0.7 | 3.0 | 1.7 | 0.9 | 0.8 | 2.5 | 0.7 | 1.0 | 1.6 | 1.1 | 0.8 | 0.84 |
| YPR067W | 1.6 | 1.2 | 1.7 | 0.9 | 1.3 | 1.3 | 1.5 | 1.4 | 3.5 | 3.4 | 1.8 | 1.0 | 3.0 | 1.6 | 1.3 | 0.8 | 1.0 | 1.0 | 0.66 |
| YBR029C | 0.7 | 0.2 | 1.8 | 1.0 | 1.4 | 0.8 | 0.4 | 0.7 | 2.0 | 1.1 | 0.7 | 1.3 | 1.7 | 0.9 | 3.5 | 0.9 | 0.6 | 0.8 | 1.56 |
| YCL009C | 0.6 | 0.9 | 2.0 | 0.5 | 0.7 | 1.8 | 1.3 | 1.1 | 0.9 | 1.6 | 1.5 | 1.7 | 1.6 | 0.7 | 6.5 | 0.9 | 1.1 | 0.8 | 0.68 |
| YER026C | 0.8 | 0.6 | 3.3 | 1.0 | 1.2 | 2.5 | 1.6 | 0.8 | 0.9 | 1.5 | 0.9 | 1.7 | 1.9 | 1.1 | 4.0 | 1.4 | 1.7 | 3.2 | 4.48 |
| YLR109W | 0.8 | 2.9 | 6.0 | 0.9 | 1.3 | 2.1 | 2.6 | 0.9 | 2.4 | 2.7 | 1.5 | 1.6 | 3.2 | 2.3 | 3.9 | 1.8 | 1.1 | 1.2 | 3.86 |
| YMR189W | 0.7 | 0.8 | 1.9 | 2.8 | 0.4 | 1.9 | 0.8 | 0.4 | 0.7 | 0.9 | 8.2 | 0.6 | 0.9 | 1.6 | 5.6 | 0.9 | 1.0 | 0.8 | 0.88 |
| YNL169C | 0.7 | 1.4 | 1.4 | 1.2 | 0.7 | 1.4 | 0.7 | 0.8 | 1.1 | 1.1 | 0.8 | 1.0 | 1.7 | 0.9 | 3.4 | 0.8 | 1.1 | 1.3 | 1.05 |
| YER069W | 1.3 | 0.8 | 3.3 | 1.4 | 1.5 | 1.3 | 1.0 | 1.5 | 0.8 | 10.4 | 1.5 | 0.8 | 1.4 | 1.3 | 2.4 | 1.1 | 1.1 | 0.9 | 0.25 |
| YIL022W | 0.8 | 0.7 | 1.1 | 1.6 | 0.8 | 1.2 | 0.7 | 0.8 | 1.1 | 1.3 | 1.0 | 0.9 | 1.2 | 0.6 | 2.9 | 1.1 | 0.8 | 0.7 | 0.52 |
| YBR146W | 0.9 | 0.8 | 0.7 | 1.5 | 1.3 | 2.6 | 1.5 | 1.0 | 0.9 | 1.1 | 1.2 | 1.0 | 1.7 | 1.2 | 1.0 | 2.9 | 1.0 | 1.2 | 1.61 |
| YDR019C | 1.4 | 0.5 | 1.7 | 4.0 | 0.9 | 4.8 | 1.4 | 0.7 | 0.4 | 0.7 | 1.8 | 0.7 | 1.0 | 1.6 | 1.8 | 1.8 | 1.2 | 1.6 | 2.48 |
| YGR207C | 1.5 | 1.1 | 1.0 | 1.1 | 1.5 | 2.8 | 1.7 | 1.9 | 2.1 | 2.6 | 1.4 | 1.1 | 1.0 | 2.2 | 0.6 | 1.8 | 1.2 | 1.8 | 1.49 |
| YMR072W | 1.1 | 1.0 | 1.4 | 1.0 | 1.7 | 2.4 | 1.4 | 1.0 | 3.0 | 1.2 | 1.9 | 0.8 | 2.1 | 1.2 | 0.8 | 1.7 | 0.9 | 1.9 | 2.06 |
| YNL037C | 1.4 | 1.8 | 1.7 | 1.1 | 1.7 | 2.8 | 2.8 | 1.0 | 0.6 | 3.2 | 1.4 | 1.3 | 1.9 | 1.3 | 0.9 | 1.4 | 0.9 | 1.3 | 2.05 |
| YOR136W | 1.0 | 0.9 | 3.5 | 1.0 | 1.5 | 3.9 | 3.2 | 1.1 | 0.3 | 3.4 | 1.4 | 1.2 | 1.5 | 0.9 | 1.2 | 1.3 | 0.8 | 1.3 | 3.20 |
| YPL271W | 1.2 | 3.2 | 4.1 | 1.2 | 1.4 | 3.3 | 1.2 | 0.9 | 0.6 | 1.2 | 1.7 | 0.8 | 1.2 | 1.3 | 0.8 | 1.4 | 1.2 | 1.3 | 1.34 |
| YAL044C | 1.5 | 1.1 | 1.0 | 3.0 | 0.8 | 2.2 | 1.0 | 0.8 | 0.4 | 0.6 | 1.4 | 0.6 | 1.1 | 1.4 | 0.7 | 1.0 | 1.4 | 1.8 | 4.07 |
| YAL054C | 1.2 | 1.1 | 1.4 | 1.6 | 1.1 | 2.2 | 1.1 | 1.1 | 8.9 | 4.1 | 1.3 | 1.0 | 1.8 | 1.3 | 0.9 | 2.5 | 1.5 | 1.4 | 0.24 |
| YBR024W | 1.0 | 1.0 | 1.1 | 2.4 | 1.0 | 2.2 | 1.9 | 1.4 | 1.3 | 1.4 | 1.3 | 1.1 | 1.7 | 1.9 | 0.8 | 2.3 | 1.0 | 1.2 | 1.14 |
| YDR405W | 0.9 | 1.1 | 4.7 | 1.0 | 1.1 | 2.4 | 1.2 | 0.9 | 0.6 | 1.0 | 1.0 | 1.2 | 1.3 | 1.1 | 2.5 | 1.3 | 1.3 | 0.9 | 0.44 |
| YGL068W | 1.0 | 0.6 | 1.1 | 1.4 | 0.9 | 2.6 | 1.5 | 0.9 | 0.7 | 0.8 | 1.0 | 1.1 | 1.0 | 0.9 | 0.6 | 1.8 | 1.8 | 1.0 | 3.35 |
| YGR220C | 1.1 | 0.6 | 0.9 | 1.1 | 1.3 | 2.1 | 1.4 | 1.5 | 0.8 | 1.5 | 1.1 | 0.9 | 1.1 | 1.5 | 1.1 | 2.3 | 0.9 | 1.1 | 1.42 |
| YHR037W | 0.7 | 0.9 | 1.3 | 1.6 | 0.9 | 1.8 | 1.2 | 1.0 | 0.9 | 2.2 | 1.7 | 1.1 | 1.1 | 1.1 | 1.1 | 2.0 | 1.0 | 1.4 | 1.15 |
| YIL070C | 1.2 | 1.0 | 0.8 | 1.4 | 1.3 | 2.3 | 1.6 | 1.1 | 0.3 | 0.6 | 1.0 | 0.8 | 0.7 | 1.3 | 1.0 | 2.4 | 0.7 | 1.1 | 1.69 |
| YIL111W | 1.6 | 1.0 | 1.8 | 2.7 | | 2.6 | 3.2 | 1.2 | 1.6 | 1.1 | 2.1 | 0.9 | 1.7 | 1.5 | 1.1 | 2.5 | 1.9 | 3.9 | 1.52 |
| YKL138C | 1.2 | 0.9 | 0.6 | 0.9 | 1.3 | 2.3 | 1.9 | 1.7 | 1.1 | 2.1 | 1.2 | 0.8 | 0.8 | 1.6 | 0.6 | 2.5 | 1.0 | 1.3 | 1.29 |
| YKL150W | 1.1 | 2.0 | 3.0 | 1.4 | 1.4 | 2.3 | 2.4 | 1.3 | 1.1 | 1.3 | 1.9 | 1.2 | 1.8 | 1.4 | 0.9 | 6.1 | 1.2 | 2.7 | 2.20 |
| YKR006C | 1.0 | 0.7 | 1.2 | 1.2 | 1.4 | 2.4 | 1.6 | 1.2 | 1.0 | 1.5 | 1.2 | 0.8 | 0.9 | 1.6 | 0.9 | 2.3 | 0.8 | 1.1 | 1.39 |
| YLR142W | 4.4 | 2.7 | 1.1 | 6.1 | 1.3 | 3.1 | 1.2 | 2.1 | 3.1 | 4.4 | 3.8 | 0.8 | 1.2 | 2.1 | 1.6 | 3.4 | 1.9 | 3.2 | 0.28 |
| YML110C | 1.1 | 0.8 | 1.9 | 1.6 | 0.8 | 2.6 | 2.0 | 1.4 | 2.7 | 2.2 | 1.7 | 1.1 | 1.8 | 1.4 | 1.3 | 2.3 | 1.1 | 1.6 | 2.01 |

| | | | | | | | | | | | | | | | | | | |
|---|---|---|---|---|---|---|---|---|---|---|---|---|---|---|---|---|---|---|
| YNL284C | 1.1 | 0.7 | 0.3 | 1.4 | 1.8 | 1.0 | 2.8 | 1.6 | 0.9 | 1.9 | 1.3 | 0.7 | 1.0 | 1.5 | 0.8 | 2.4 | 1.0 | 1.5 | 1.26 |
| YDR268W | 0.8 | 1.5 | 0.4 | 1.2 | 2.2 | 1.0 | 2.4 | 1.0 | 1.0 | 2.0 | 1.1 | 0.7 | 1.0 | 1.4 | 1.0 | 1.6 | 1.1 | 1.2 | 0.47 |
| YMR193W | 1.4 | 1.2 | 0.5 | 1.9 | 2.2 | 1.7 | 2.7 | 1.7 | 0.8 | 1.1 | 1.6 | 0.6 | 0.9 | 1.5 | 0.8 | 3.7 | 1.1 | 1.7 | 0.93 |
| YOR374W | 1.3 | 4.5 | 7.3 | 3.9 | 0.9 | 1.4 | 1.9 | 0.9 | 2.3 | 5.9 | 2.6 | 1.5 | 1.7 | 1.1 | 2.5 | 2.5 | 1.2 | 2.4 | 1.24 |
| YER061C | 0.9 | 0.9 | 1.2 | 2.5 | 0.8 | 0.4 | 0.9 | 0.7 | 0.3 | 0.7 | 2.2 | 0.7 | 0.9 | 1.0 | 0.8 | 1.8 | 1.2 | 1.2 | 0.84 |
| YIL136W | 1.1 | 1.0 | 1.2 | 2.5 | 1.0 | 1.0 | 1.8 | 1.1 | 3.4 | 1.7 | 4.8 | 0.9 | 1.7 | 1.7 | 1.5 | 4.5 | 1.3 | 3.2 | 0.95 |
| YLL009C | 1.0 | 1.2 | 0.6 | 1.9 | 1.5 | 1.2 | 1.7 | 1.0 | 1.3 | 1.7 | 3.5 | 0.7 | 0.9 | 1.4 | 1.1 | 2.8 | 0.9 | 1.2 | 1.66 |
| YLR163C | 1.0 | 0.9 | 0.7 | 1.4 | 1.6 | 1.3 | 1.7 | 1.4 | 2.6 | 2.9 | 2.2 | 0.7 | 1.4 | 1.3 | 1.2 | 1.4 | 0.9 | 1.1 | 0.55 |
| YBR291C | 2.0 | 0.9 | 1.0 | 1.5 | 0.9 | 1.1 | 0.9 | 2.2 | 0.9 | 1.0 | 1.1 | 0.9 | 1.0 | 1.7 | 0.5 | 1.5 | 0.9 | 1.3 | 1.19 |
| YIL094C | 1.5 | 0.4 | 0.8 | 0.7 | 1.4 | 1.2 | 1.3 | 3.6 | 0.3 | 0.4 | 1.8 | 1.0 | 0.7 | 1.0 | 0.5 | 0.9 | 0.9 | 1.0 | 2.26 |
| YAL015C | 1.2 | 1.1 | 1.7 | 1.9 | 1.1 | 0.7 | 1.2 | 1.1 | 4.0 | 2.7 | 1.4 | 1.0 | 1.2 | 1.6 | 0.7 | 2.0 | 1.1 | 1.2 | 0.61 |
| YJR095W | 1.2 | 20.5 | 1.9 | 6.7 | 1.2 | 1.5 | 2.0 | 0.9 | 0.5 | 6.3 | 0.6 | 0.7 | 0.8 | 1.3 | 0.8 | 0.8 | 1.3 | 0.9 | 0.23 |
| YKL085W | 1.4 | 2.3 | 1.6 | 1.2 | 1.2 | 1.9 | 1.5 | 1.2 | 1.9 | 3.0 | 1.8 | 0.8 | 1.5 | 1.0 | 0.5 | 1.7 | 0.9 | 1.3 | 2.16 |
| YMR177W | 1.4 | 0.8 | 1.1 | 1.0 | 1.6 | 0.8 | 1.0 | 1.2 | 2.4 | 3.8 | 1.4 | 0.6 | 0.5 | 1.2 | 0.8 | 0.5 | 0.9 | 0.9 | 0.55 |
| YPL224C | 1.0 | 2.2 | 1.3 | 2.5 | 0.6 | 1.3 | 2.2 | 1.3 | 3.2 | 4.2 | 0.8 | 1.1 | 1.3 | 1.4 | 1.0 | 2.5 | 1.3 | 1.9 | 0.64 |
| YER014W | 1.0 | 0.9 | 0.9 | 0.6 | 0.8 | 4.0 | | 1.2 | 3.5 | 1.1 | 0.9 | 1.1 | 1.5 | 1.2 | 2.3 | 0.8 | 1.0 | 0.9 | 0.43 |
| YFR049W | 1.5 | 1.5 | 1.8 | 1.2 | 1.3 | | | 1.3 | 2.6 | 1.5 | 1.7 | 1.2 | 1.4 | 1.2 | 0.7 | 0.9 | 1.3 | 1.0 | 0.83 |
| YGR112W | 1.0 | 2.9 | 1.1 | 1.3 | 0.9 | 1.1 | 1.3 | 1.3 | 2.6 | 1.3 | 1.3 | 0.7 | 1.1 | 1.1 | 1.2 | 2.8 | 0.9 | 1.2 | 0.31 |
| YLL001W | 0.9 | 0.7 | 1.2 | 1.2 | 0.6 | 1.0 | 0.8 | 1.0 | 3.5 | 2.4 | 0.9 | 0.7 | 1.7 | 1.2 | 0.9 | 1.3 | 0.9 | 1.3 | 0.70 |
| YML042W | 0.9 | 1.4 | 1.5 | 2.2 | 1.0 | 0.9 | 0.8 | 1.6 | 3.8 | 2.4 | 2.5 | 1.0 | 1.2 | 1.4 | 1.7 | 4.8 | 1.0 | 0.9 | 0.29 |
| YNL213C | 1.7 | 11.6 | 0.9 | 1.5 | 1.3 | 1.2 | 1.4 | 1.8 | 3.0 | 1.5 | 1.5 | 0.8 | 0.9 | 1.8 | 1.0 | 1.7 | 0.9 | 1.2 | 0.70 |
| YOR386W | 1.0 | 0.8 | 1.3 | 1.5 | 1.2 | 3.3 | 1.8 | 1.0 | 4.1 | 3.0 | 1.4 | 1.2 | 2.4 | 0.9 | 1.3 | 1.2 | 2.1 | 1.7 | 0.46 |
| YBR037C | 0.8 | 0.8 | 1.3 | 2.4 | 0.7 | 1.2 | 1.2 | 1.0 | 3.0 | 1.9 | 1.5 | 0.7 | 1.2 | 1.2 | 1.0 | 2.7 | 1.6 | 1.3 | 0.60 |
| YCR024C | 1.1 | 1.0 | 1.5 | 0.8 | 0.8 | 2.9 | 1.0 | 1.2 | 2.5 | 1.7 | 1.4 | 0.8 | 0.9 | 1.3 | 1.0 | 1.2 | 0.9 | 1.0 | 0.32 |
| YDR194C | 0.7 | 0.9 | 0.5 | 1.0 | 1.0 | 1.0 | 1.3 | 1.4 | 2.8 | 2.2 | 1.2 | 0.6 | 1.2 | 0.8 | 1.1 | 0.8 | 0.6 | 0.8 | 2.19 |
| YER017C | 0.8 | 1.1 | 1.4 | 1.3 | 1.0 | 0.5 | 1.1 | 0.5 | 2.7 | 1.8 | 0.9 | 1.0 | 1.5 | 1.1 | 2.1 | 1.0 | 0.9 | 1.0 | 0.43 |
| YGL125W | 1.0 | 3.2 | 4.3 | 0.7 | 0.9 | | 1.7 | 0.8 | 2.1 | 2.1 | 1.6 | 1.0 | 1.4 | 1.0 | 1.9 | 1.2 | 1.2 | 1.1 | 0.27 |
| YGR029W | 1.1 | 1.4 | 1.0 | 1.0 | 0.8 | 0.6 | 1.6 | 1.7 | 2.7 | 1.1 | 1.2 | 0.6 | 1.0 | 2.0 | 0.4 | 1.1 | 1.1 | 1.3 | 0.71 |
| YHL038C | 1.0 | 1.2 | 1.4 | 0.8 | 1.3 | 0.8 | 1.2 | 1.1 | 4.0 | 1.7 | 0.8 | 1.6 | 1.3 | 0.6 | 0.8 | 0.5 | 0.9 | 0.6 | 1.95 |
| YKL192C | 1.1 | 1.0 | 3.7 | 1.1 | 1.1 | 1.8 | 1.1 | 0.9 | 3.5 | 1.6 | 1.1 | 1.2 | 1.7 | 1.5 | 1.2 | 2.1 | 1.0 | 0.8 | 1.57 |
| YKR052C | 0.9 | 1.1 | 2.6 | 2.6 | 1.0 | 0.5 | 1.9 | 1.1 | 1.9 | 3.6 | 1.3 | 0.9 | 1.2 | 1.1 | 0.5 | 2.5 | 0.8 | 1.5 | 1.15 |

| | | | | | | | | | | | | | | | | | | |
|---|---|---|---|---|---|---|---|---|---|---|---|---|---|---|---|---|---|---|
| YML078W | 1.2 | 1.9 | 2.5 | 1.5 | 0.7 | 1.4 | 1.3 | 1.4 | 2.6 | 2.2 | 2.4 | 1.2 | 1.7 | 1.7 | 1.0 | 1.8 | 1.3 | 1.6 | 1.98 |
| YMR056C | 1.1 | 1.3 | 1.7 | 1.0 | 1.1 | 0.9 | 1.4 | 1.2 | 1.8 | 1.1 | 1.6 | 0.9 | 0.9 | 1.1 | 0.7 | 2.4 | 1.0 | 1.1 | 0.72 |
| YNL005C | 1.1 | 1.0 | 0.6 | 1.2 | 1.2 | 1.7 | 1.3 | 1.5 | 2.0 | 2.0 | 1.2 | 0.9 | 1.5 | 1.3 | 1.0 | 1.6 | 0.9 | 1.5 | 1.52 |
| YPR047W | 1.0 | 1.0 | 0.9 | 1.5 | 0.9 | 1.0 | 1.8 | 1.2 | 2.1 | 1.9 | 1.1 | 0.9 | 1.3 | 1.0 | 0.8 | 2.8 | 1.0 | 1.5 | 0.39 |
| YPR134W | 1.1 | 1.1 | 0.8 | 1.7 | 1.5 | 0.6 | 1.5 | 1.4 | 1.8 | 2.3 | 1.3 | 0.9 | 1.1 | 1.5 | 0.7 | 2.4 | 1.0 | 1.2 | 0.65 |
| YGL191W | 1.7 | 1.6 | 1.4 | 1.8 | 0.9 | 1.6 | 1.0 | 1.6 | 1.1 | 1.3 | 1.6 | 0.9 | 1.1 | 1.6 | 0.7 | 1.8 | 1.2 | 1.7 | 2.35 |
| YLR038C | 2.3 | 1.1 | 0.6 | 2.1 | 1.8 | 1.2 | 1.0 | 1.4 | 0.4 | 0.9 | 1.4 | 0.6 | 0.5 | 1.6 | 0.7 | 2.1 | 1.2 | 2.1 | 1.52 |
| YHR008C | 1.3 | 5.4 | 4.8 | 1.8 | 0.6 | 1.0 | 0.7 | 0.9 | 1.7 | 2.4 | 2.1 | 0.7 | 1.2 | 1.6 | 1.7 | 2.2 | 0.9 | 1.0 | 1.04 |
| YPR037C | 1.2 | 2.3 | 1.6 | 1.3 | 0.8 | 0.9 | 1.2 | 1.1 | 1.5 | 1.3 | 0.9 | 0.9 | 1.0 | 1.6 | 4.1 | 1.8 | 1.2 | 1.7 | 0.80 |
| YAL039C | 1.1 | 2.2 | 1.7 | 1.0 | 1.1 | 0.8 | 1.0 | 1.2 | 1.6 | 2.2 | 1.1 | 1.4 | 1.6 | 1.2 | 3.3 | 2.2 | 1.4 | 1.1 | 0.39 |
| YDL181W | 1.3 | 1.8 | 1.9 | 1.8 | 1.7 | 1.2 | 1.1 | 0.6 | 0.7 | 0.8 | 0.8 | 0.9 | 0.9 | 1.1 | 0.4 | 1.1 | 1.5 | 1.4 | 0.85 |
| YPL215W | 1.1 | 1.9 | 1.0 | 1.5 | 0.8 | 0.7 | 0.7 | 1.1 | 1.7 | 1.2 | 1.1 | 0.7 | 0.7 | 1.2 | 0.6 | 1.4 | 1.0 | 1.1 | 1.10 |
| YPL262W | 1.0 | 1.7 | 3.8 | 1.2 | 0.6 | 1.6 | 1.3 | 1.0 | 1.4 | 5.8 | 1.4 | 1.1 | 1.5 | 1.2 | 0.6 | 1.4 | 1.1 | 1.2 | 0.79 |
| YNL256W | 0.7 | 1.6 | 0.6 | 0.6 | 1.0 | 0.5 | 0.6 | 0.9 | 0.8 | 0.6 | 0.6 | 0.6 | 1.3 | 0.7 | 0.9 | 0.4 | 0.6 | 0.6 | 1.09 |
| YBL030C | 1.0 | 0.9 | 4.5 | 0.9 | 0.7 | 1.1 | 0.9 | 0.8 | 0.4 | 0.9 | 1.1 | 0.9 | 0.5 | 0.8 | 1.8 | 1.0 | 1.0 | 1.1 | 3.12 |
| YBR221C | 0.8 | 0.7 | 3.2 | 1.5 | 1.3 | 1.7 | 1.5 | 0.8 | 1.0 | 1.3 | 1.2 | 1.3 | 1.7 | 0.8 | 1.1 | 1.5 | 0.9 | 1.0 | 3.62 |
| YKL141W | 1.6 | 1.1 | 4.7 | 1.7 | 1.5 | 1.2 | 0.5 | 0.8 | 0.8 | 0.5 | 1.2 | 0.8 | 0.8 | 1.4 | 0.6 | 2.8 | 0.9 | 1.8 | 2.84 |
| YKR066C | 0.9 | 1.4 | 5.5 | 0.7 | 1.0 | 0.9 | 0.5 | 0.7 | 0.7 | 2.4 | 1.3 | 0.9 | 0.6 | 1.5 | 0.6 | 1.3 | 0.9 | 1.1 | 1.25 |
| YMR083W | 1.4 | 1.7 | 2.6 | 1.1 | 1.5 | 1.8 | 1.7 | 1.1 | 0.4 | 1.1 | 1.6 | 0.7 | 0.9 | 0.8 | 1.3 | 1.3 | 0.9 | 1.1 | 2.52 |
| YMR203W | 0.8 | 0.7 | 3.0 | 1.4 | 0.6 | 1.1 | 0.8 | 0.7 | 0.7 | 1.2 | 2.3 | 0.7 | 1.1 | 0.7 | 1.2 | 0.9 | 0.8 | 0.7 | 1.62 |
| YBL099W | 0.8 | 0.9 | 3.1 | 1.4 | 1.0 | 0.9 | 0.9 | 0.8 | 0.9 | 0.7 | 2.1 | 1.0 | 0.9 | 0.6 | 1.0 | 0.9 | 0.7 | 1.2 | 3.49 |
| YDR178W | 2.0 | 2.0 | 3.4 | 2.2 | 0.9 | 2.1 | 0.6 | 0.9 | 0.9 | 0.8 | 2.0 | 1.3 | 1.5 | 1.5 | 0.7 | 3.0 | 1.2 | 2.3 | 2.27 |
| YDR298C | 1.3 | 1.2 | 2.6 | 1.3 | 1.3 | 1.2 | 1.0 | 1.2 | 1.0 | 1.6 | 1.2 | 1.1 | 1.2 | 1.5 | 0.8 | 2.0 | 1.1 | 1.6 | 2.69 |
| YEL024W | 1.3 | 0.9 | 3.8 | 1.1 | 1.3 | 1.1 | 0.7 | 1.0 | 0.7 | 0.6 | 0.9 | 0.8 | 0.6 | 1.1 | 0.6 | 2.1 | 1.1 | 1.5 | 1.59 |
| YGR082W | 1.0 | 0.9 | 2.2 | 1.0 | 0.9 | 1.1 | 0.9 | 0.8 | 0.6 | 0.9 | 1.3 | 0.8 | 0.5 | 0.9 | 1.2 | 1.3 | 0.9 | 0.8 | 1.47 |
| YJL133W | 1.0 | 1.0 | 2.0 | 0.9 | 0.9 | 0.5 | 0.5 | 0.9 | 0.6 | 1.4 | 1.2 | 0.8 | 0.8 | 0.7 | 0.9 | 0.6 | 0.8 | 0.7 | 0.91 |
| YJR077C | 1.1 | 1.1 | 2.0 | 1.6 | 0.9 | 0.9 | 0.7 | 0.8 | 0.4 | 0.7 | 1.6 | 1.0 | 0.9 | 0.7 | 1.5 | 0.7 | 1.0 | 0.8 | 1.79 |
| YJR121W | 0.9 | 1.1 | 3.3 | 1.0 | 0.8 | 1.5 | 1.0 | 0.7 | 0.8 | 1.3 | 0.9 | 0.8 | 1.0 | 0.7 | 0.9 | 1.2 | 1.1 | 1.4 | 3.99 |
| YKL148C | 0.9 | 0.8 | 3.7 | 0.7 | 0.8 | 0.5 | 0.6 | 0.8 | 1.7 | 1.7 | 1.5 | 0.8 | 0.8 | 0.8 | 0.6 | 1.1 | 0.8 | 1.0 | 0.54 |
| YLL041C | 1.1 | 0.5 | 4.6 | 1.0 | 1.6 | 1.2 | 0.3 | 0.8 | 0.7 | 0.8 | 1.3 | 0.8 | 1.0 | 1.2 | 0.4 | 3.1 | 1.1 | 1.7 | 1.75 |
| YLR304C | 0.7 | 0.6 | 5.0 | 0.7 | 0.6 | 1.9 | 0.6 | 0.6 | 0.1 | 2.2 | 1.6 | 1.0 | 0.5 | 0.7 | 1.6 | 1.8 | 0.5 | 0.7 | 2.39 |

| | | | | | | | | | | | | | | | | | | | |
|---|---|---|---|---|---|---|---|---|---|---|---|---|---|---|---|---|---|---|---|
| YOR142W | 1.0 | 1.2 | 3.4 | 1.0 | 1.4 | 1.6 | 1.0 | 0.8 | 1.1 | 1.5 | 1.5 | 0.8 | 1.4 | 0.8 | 1.1 | 0.8 | 0.9 | 1.0 | 1.31 |
| YOR176W | 0.7 | 2.6 | 2.7 | 0.9 | 1.0 | 0.9 | 0.5 | 1.0 | 1.0 | 0.5 | 1.4 | 1.3 | 1.5 | 0.8 | 0.8 | 1.4 | 1.2 | 2.1 | 1.23 |
| YPL135W | 0.9 | 1.2 | 2.5 | 1.2 | 1.6 | 1.3 | 1.2 | 1.0 | 0.5 | 2.8 | 1.3 | 1.1 | 1.2 | 1.1 | 1.5 | 1.2 | 1.5 | 1.7 | 1.50 |
| YDR529C | 1.8 | 1.0 | 0.9 | 3.2 | 1.2 | 0.8 | 0.5 | 1.3 | 0.5 | 0.6 | 0.8 | 0.6 | 0.7 | 1.5 | 0.5 | 1.8 | 1.0 | 2.3 | 3.11 |
| YGL018C | 1.1 | 1.0 | 1.8 | 3.0 | 1.1 | 0.4 | 1.1 | 0.8 | 0.5 | 1.4 | 1.1 | 1.0 | 1.3 | 1.2 | 1.1 | 1.5 | 0.8 | 1.0 | 0.36 |
| YBR003W | 0.9 | 0.8 | 1.0 | 1.9 | 0.7 | 1.2 | 1.1 | 0.9 | 1.0 | 1.3 | 1.3 | 0.8 | 1.2 | 0.8 | 0.7 | 2.0 | 1.1 | 1.3 | 1.06 |
| YBR044C | 0.8 | 1.4 | 0.7 | 1.9 | 1.4 | 1.0 | 1.5 | 1.1 | 0.8 | 1.2 | 1.1 | 0.8 | 1.2 | 0.9 | 0.9 | 1.4 | 0.9 | 1.3 | 0.57 |
| YBR091C | 1.1 | 1.0 | 0.6 | 3.0 | 1.1 | 0.7 | 1.0 | 1.5 | 0.7 | 1.6 | 0.7 | 0.8 | 1.2 | 1.7 | 0.9 | 0.9 | 1.0 | 1.0 | 0.87 |
| YBR185C | 1.2 | 1.0 | 1.3 | 2.0 | 0.8 | 1.2 | 1.2 | 1.1 | 0.9 | 1.6 | 1.2 | 0.7 | 1.0 | 1.7 | 1.0 | 1.7 | 0.8 | 1.3 | 0.89 |
| YBR282W | 1.0 | 1.0 | 0.5 | 2.3 | 1.0 | 1.4 | 1.2 | 1.4 | 1.0 | 0.9 | 0.9 | 0.8 | 1.3 | 1.4 | 1.1 | 1.5 | 0.9 | 1.2 | 1.36 |
| YDR347W | 1.1 | 0.8 | 0.9 | 3.5 | 0.8 | 1.2 | 1.2 | 1.2 | 0.8 | 1.6 | 0.8 | 0.8 | 1.3 | 1.5 | 1.0 | 1.7 | 0.9 | 1.2 | 1.33 |
| YEL039C | 1.1 | 0.7 | 1.3 | 5.1 | 0.9 | 1.5 | 0.6 | 1.1 | 1.2 | 1.2 | 1.7 | 0.6 | 0.5 | 1.4 | 0.4 | 3.5 | 0.7 | 1.1 | 1.59 |
| YGR076C | 1.3 | 1.6 | 0.6 | 2.3 | 0.9 | 1.2 | 1.7 | 1.9 | 1.2 | 1.8 | 1.7 | 1.0 | 1.4 | 2.3 | 0.5 | 2.3 | 0.9 | 1.5 | 0.97 |
| YGR174C | 1.1 | 1.6 | 0.6 | 2.4 | 1.0 | 1.4 | 1.0 | 1.6 | 1.8 | 0.7 | 1.3 | 0.9 | 1.5 | 1.6 | 0.7 | 3.2 | 1.3 | 2.3 | 1.05 |
| YKL003C | 1.1 | 0.7 | 0.8 | 1.8 | 1.0 | 1.0 | 1.4 | 1.2 | 1.5 | 2.2 | 1.7 | 0.7 | 1.3 | 2.0 | 1.0 | 2.3 | 1.0 | 1.2 | 0.90 |
| YKL016C | 1.5 | 1.3 | 0.7 | 1.7 | 1.5 | 0.8 | 1.5 | 1.6 | 1.3 | 1.8 | 1.3 | 1.0 | 1.3 | 1.6 | 0.7 | 1.8 | 1.0 | 1.7 | 2.08 |
| YKL170W | 1.0 | 0.8 | 0.5 | 1.9 | 1.3 | 1.5 | 1.5 | 1.2 | 0.9 | 1.1 | 0.7 | 0.8 | 0.9 | 1.3 | 0.8 | 2.1 | 1.1 | 1.4 | 1.27 |
| YKL194C | 0.9 | 0.7 | 0.8 | 1.6 | 1.8 | 1.4 | 2.1 | 1.1 | 1.3 | 1.4 | 1.6 | 0.6 | 1.1 | 1.4 | 0.8 | 1.2 | 1.1 | 1.1 | 0.52 |
| YLR395C | 1.8 | 1.3 | 2.2 | 1.9 | 0.7 | 0.4 | | 1.0 | 1.0 | 0.9 | 1.5 | 0.6 | 0.5 | 1.7 | 0.4 | 1.2 | 1.2 | 2.3 | 1.76 |
| YML120C | 1.1 | 1.2 | 1.9 | 1.9 | 0.6 | 0.6 | 0.8 | 0.9 | 1.3 | 1.5 | 1.3 | 0.9 | 1.2 | 0.8 | 0.5 | 1.5 | 1.0 | 1.6 | 0.74 |
| YOR100C | 1.3 | 1.4 | 1.6 | 1.7 | 1.0 | 1.2 | 1.6 | 1.0 | 2.0 | 2.6 | 1.4 | 0.8 | 1.0 | 1.4 | 4.3 | 2.8 | 0.9 | 0.8 | 0.32 |
| YOR150W | 1.7 | 1.2 | 1.0 | 1.9 | 0.8 | 0.9 | 1.2 | 1.1 | 0.8 | 1.8 | 1.0 | 0.8 | 1.0 | 1.6 | 0.8 | 1.6 | 0.9 | 1.3 | 1.11 |
| YOR187W | 1.0 | 0.5 | 1.9 | 2.0 | 0.6 | 1.7 | 1.5 | 0.7 | 0.6 | 0.8 | 1.4 | 0.8 | 0.7 | 0.6 | 1.3 | 1.6 | 0.8 | 1.3 | 3.12 |
| YJL166W | 2.1 | 1.5 | 1.6 | 1.3 | 1.6 | 0.7 | 0.8 | 1.4 | 1.3 | 1.2 | 1.4 | 0.7 | 0.8 | 1.9 | 0.5 | 2.3 | 1.7 | 4.0 | 2.31 |
| YMR035W | 1.2 | 1.7 | 1.5 | 1.7 | 0.8 | 1.5 | 2.3 | 1.4 | 1.7 | 1.6 | 1.5 | 0.5 | 1.4 | 1.3 | 1.1 | 1.5 | 2.2 | 2.4 | 0.96 |
| YBL038W | 1.0 | 0.9 | 0.5 | 1.8 | 0.9 | 0.7 | 8.0 | 1.6 | 1.4 | 1.5 | 0.7 | 0.7 | 1.0 | 2.3 | 0.7 | 1.8 | 1.2 | 1.9 | 1.56 |
| YDR377W | 1.3 | 1.1 | 1.3 | 1.4 | 1.9 | 1.3 | 1.1 | 1.3 | 1.0 | 0.9 | 1.4 | 0.9 | 0.7 | 1.4 | 0.6 | 1.8 | 1.2 | 2.5 | 3.00 |
| YGL187C | 1.5 | 0.8 | 1.6 | 1.8 | 1.3 | 1.1 | 0.6 | 0.7 | 0.6 | 0.6 | 0.7 | 0.8 | 0.7 | 0.9 | 0.3 | 1.4 | 0.9 | 2.4 | 2.73 |
| YCR046C | 1.0 | 1.2 | 1.2 | 1.6 | 1.0 | 0.8 | 1.2 | 0.9 | 1.9 | 1.4 | 1.5 | 1.1 | 1.5 | 1.3 | 1.4 | 2.8 | 1.3 | 1.0 | 0.63 |
| YML129C | 1.1 | 0.9 | 0.9 | 1.2 | 1.7 | 1.7 | 1.8 | 1.2 | 1.4 | 2.2 | 1.2 | 0.8 | 1.0 | 1.4 | 1.1 | 2.9 | 1.3 | 1.3 | 1.04 |
| YOL096C | 1.1 | 1.0 | 1.2 | 1.6 | 1.2 | 1.3 | 1.7 | 1.0 | 1.3 | 1.4 | 1.2 | 1.2 | 1.4 | 1.4 | 1.0 | 2.3 | 1.2 | 1.2 | 0.64 |

| | | | | | | | | | | | | | | | | | | | |
|---|---|---|---|---|---|---|---|---|---|---|---|---|---|---|---|---|---|---|---|
| YBR122C | 0.9 | 0.9 | 0.3 | 1.1 | 1.5 | 1.1 | 2.0 | 1.3 | 1.1 | 1.4 | 1.2 | 0.7 | 0.8 | 1.2 | 0.7 | 1.9 | 0.9 | 1.5 | 1.53 |
| YBR251W | 1.0 | 0.9 | 1.0 | 1.2 | 1.3 | 2.0 | 1.4 | 1.3 | 0.5 | 1.1 | 1.5 | 1.1 | 1.0 | 1.6 | 0.8 | 3.0 | 0.9 | 1.4 | 1.13 |
| YCR083W | 1.1 | 2.8 | 1.5 | 1.8 | 1.6 | 2.4 | 1.8 | 1.5 | 1.8 | 1.8 | 1.8 | 1.1 | 1.8 | 2.4 | 1.0 | 3.1 | 1.3 | 1.3 | 0.98 |
| YDL067C | 1.8 | 1.0 | 1.6 | 1.3 | 2.0 | 0.9 | 0.8 | 1.1 | 0.9 | 0.9 | 1.4 | 1.0 | 0.6 | 1.4 | 0.7 | 1.8 | 1.2 | 1.7 | 1.85 |
| YDR079W | 1.3 | 1.1 | 0.8 | 1.1 | 1.2 | 1.7 | 1.3 | 2.6 | 0.9 | 0.8 | 1.3 | 1.1 | 1.3 | 1.5 | 0.6 | 2.6 | 1.1 | 1.3 | 1.06 |
| YGR062C | 0.9 | 1.0 | 0.7 | 1.4 | 0.7 | 0.9 | 1.3 | 0.8 | 1.0 | 1.4 | 1.6 | 1.0 | 1.5 | 1.2 | 0.5 | 1.8 | 0.9 | 1.1 | 0.54 |
| YJL096W | 1.0 | 1.0 | 0.8 | 1.3 | 1.9 | 1.0 | 1.7 | 1.3 | 1.0 | 1.4 | 1.2 | 1.0 | 0.7 | 2.1 | 1.0 | 2.2 | 1.1 | 1.2 | 1.21 |
| YJL180C | 0.9 | 1.6 | 1.1 | 1.3 | 1.5 | 1.3 | 1.6 | 1.0 | 1.4 | 1.4 | 1.4 | 0.8 | 1.2 | 1.4 | 0.6 | 2.0 | 0.9 | 1.4 | 1.08 |
| YLR295C | 1.4 | 1.1 | 1.1 | 1.7 | 2.2 | 1.1 | 1.1 | 1.2 | 1.4 | 1.2 | 1.3 | 0.5 | 0.8 | 1.2 | 0.5 | 2.4 | 0.9 | 1.5 | 1.07 |
| YMR023C | 1.4 | 1.3 | 0.5 | 1.9 | 1.7 | 1.1 | 1.9 | 1.4 | 1.5 | 2.6 | 0.8 | 0.7 | 1.1 | 1.6 | 0.7 | 2.1 | 1.1 | 1.4 | 0.48 |
| YMR267W | 0.8 | 1.8 | 0.7 | 0.9 | 1.0 | 1.4 | 1.2 | 1.5 | 0.4 | 0.8 | 1.0 | 0.8 | 0.7 | 1.0 | 0.9 | 2.6 | 0.9 | 1.3 | 0.94 |
| YNL073W | 0.8 | 1.4 | 0.9 | 1.7 | 0.6 | 0.5 | 1.1 | 1.0 | 0.9 | 1.8 | 1.1 | 0.7 | 1.2 | 0.7 | 0.7 | 1.8 | 0.8 | 1.0 | 0.52 |
| YOR316C | 0.7 | 2.5 | 1.5 | 1.5 | 1.0 | 0.9 | 1.4 | 0.8 | 1.6 | 2.0 | 2.4 | 1.1 | 1.6 | 1.0 | 1.5 | 2.2 | 0.8 | 1.0 | 0.93 |
| YPL040C | 1.1 | 1.0 | 0.8 | 1.7 | 0.8 | 1.1 | 0.8 | 1.1 | 0.2 | 1.2 | 0.9 | 0.9 | 1.0 | 1.1 | 1.1 | 2.7 | 0.8 | 1.1 | 0.33 |
| YPL134C | 1.5 | 1.0 | 1.6 | 1.6 | 1.4 | 1.2 | 1.2 | 1.3 | 0.6 | 0.8 | 1.7 | 0.8 | 1.0 | 1.4 | 1.1 | 3.3 | 1.3 | 1.3 | 0.70 |

## Table 3 DNA repair protein genes

The level of expressed mRNA in the presence of chemical substance/ The level of expressed mRNA in the absence of chemical substance

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| YFL014W | 3.4 | 5.1 | 5.7 | 11.0 | 1.0 | 9.3 | 5.5 | 3.4 | 13.1 | 5.8 | 5.0 | 4.3 | 15.2 | 7.3 | 6.3 | 14.2 | 1.5 | 8.8 | 2.14 |
| YGL163C | 0.9 | 0.8 | 0.6 | 4.3 | 1.5 | 0.9 | 1.0 | 1.4 | 5.8 | 2.3 | 0.4 | 0.9 | 3.1 | 1.6 | 1.8 | 1.0 | 0.9 | 0.8 | 0.26 |
| YKL145W | 0.8 | 1.4 | 1.2 | 1.0 | 1.4 | 2.1 | 1.6 | 1.5 | 3.5 | 2.7 | 1.8 | 1.3 | 2.7 | 1.0 | 1.3 | 1.2 | 1.1 | 1.5 | 2.29 |
| YIL153W | 0.8 | 1.4 | 1.3 | 2.9 | 1.0 | 0.9 | 1.6 | 0.5 | 4.3 | 1.1 | 1.0 | 1.0 | 1.2 | 1.0 | 2.8 | 1.3 | 0.9 | 0.7 | 0.32 |
| YIR002C | 0.8 | 1.1 | 0.5 | 0.6 | 1.2 | 2.2 | 1.3 | 1.2 | 1.7 | 1.4 | 0.8 | 1.3 | 1.2 | 1.1 | 1.1 | 1.4 | 1.1 | 1.2 | 0.58 |
| YAL015C | 1.2 | 1.1 | 1.7 | 1.9 | 1.1 | 0.7 | 1.2 | 1.1 | 4.0 | 2.7 | 1.4 | 1.0 | 1.2 | 1.6 | 0.7 | 2.0 | 1.1 | 1.2 | 0.61 |
| YBR073W | 0.7 | 1.0 | 0.9 | 0.7 | 1.2 | 0.7 | 0.6 | 1.1 | 2.5 | 1.8 | 0.5 | 0.9 | 1.4 | 1.0 | 1.3 | 0.8 | 0.7 | 0.8 | 0.63 |
| YDL200C | 1.4 | 2.0 | 1.2 | 1.8 | 1.1 | 0.6 | 1.7 | 1.5 | 2.4 | 1.8 | 1.2 | 1.1 | 1.1 | 1.8 | 0.7 | 1.8 | 0.9 | 1.6 | 0.79 |
| YGL058W | 1.1 | 1.1 | 0.6 | 0.7 | 1.0 | 1.0 | 0.0 | 1.3 | 3.7 | 1.8 | 0.8 | 1.3 | 1.0 | 1.3 | 1.3 | 0.6 | 1.1 | 0.7 | 1.07 |
| YIL143C | 0.9 | 0.8 | 1.1 | 0.9 | 0.8 | 1.4 | 0.8 | 0.9 | 7.1 | 3.5 | 1.5 | 0.9 | 1.7 | 1.0 | 1.3 | 1.1 | 0.9 | 0.9 | 0.56 |
| YML032C | 0.8 | 0.9 | 1.1 | 0.7 | 1.0 | 0.9 | 0.8 | 0.9 | 3.0 | 1.1 | 1.0 | 0.9 | 1.8 | 1.0 | 1.7 | 1.2 | 1.0 | 1.0 | 0.66 |
| YNL250W | 0.9 | 2.2 | 0.8 | 1.2 | 0.8 | 0.8 | 1.2 | 1.3 | 4.2 | 2.3 | 1.0 | 1.4 | 1.3 | 1.6 | 1.3 | 1.4 | 1.0 | 1.0 | 0.31 |
| YOR386W | 1.0 | 0.8 | 1.3 | 1.5 | 1.2 | 3.3 | 1.8 | 1.0 | 4.1 | 3.0 | 1.4 | 1.2 | 2.4 | 0.9 | 1.3 | 1.2 | 2.1 | 1.7 | 0.46 |
| YBL019W | 0.9 | 9.1 | 0.7 | 1.2 | 1.0 | 1.0 | 1.2 | 1.2 | 2.6 | 1.4 | 1.0 | 0.8 | 0.9 | 1.1 | 0.6 | 1.9 | 0.8 | 1.1 | 0.38 |
| YDR369C | 1.1 | 1.1 | 2.4 | 1.1 | 1.0 | 0.6 | 0.4 | 1.0 | 2.4 | 1.8 | 1.1 | 0.9 | 0.1 | 0.7 | 1.2 | 0.6 | 0.8 | 0.7 | 1.88 |
| YEL037C | 0.9 | 2.0 | 0.9 | 1.0 | 0.5 | 0.6 | 0.8 | 0.8 | 2.6 | 1.2 | 1.0 | 1.1 | 2.0 | 1.0 | 1.6 | 0.5 | 1.0 | 0.8 | 0.82 |
| YER162C | 1.0 | 1.2 | 1.0 | 1.1 | 0.9 | 0.7 | 0.5 | 0.9 | 2.8 | 1.6 | 0.5 | 0.4 | 0.8 | 0.8 | 1.0 | 1.0 | 1.2 | 0.9 | 0.45 |
| YGR258C | 0.7 | 0.9 | 0.7 | 0.7 | 1.5 | 2.2 | 1.5 | 0.8 | 2.5 | 1.5 | 0.3 | 0.9 | 1.9 | 1.2 | 1.1 | 1.2 | 1.0 | 0.9 | 0.37 |
| YJR052W | 1.1 | 1.0 | 1.1 | 1.0 | 1.0 | 1.4 | 1.2 | 1.7 | 4.5 | 2.3 | 1.1 | 1.1 | 1.8 | 1.4 | 1.4 | 1.4 | 1.0 | 1.3 | 0.36 |
| YOR005C | 1.2 | 1.8 | 1.0 | 0.8 | 1.3 | 1.3 | 1.4 | 1.3 | 2.4 | 1.5 | 1.2 | 0.9 | 2.1 | 1.2 | 0.8 | 1.4 | 1.1 | 1.0 | 0.29 |
| YPL022W | 0.7 | 0.8 | 0.7 | 1.2 | 0.8 | 5.2 | 0.8 | 1.0 | 2.1 | 1.6 | 0.8 | 0.7 | 1.4 | 0.9 | 0.9 | 1.0 | 0.9 | 0.9 | 0.72 |
| YPL164C | 1.1 | 0.7 | 1.1 | 1.0 | 1.2 | 1.7 | 0.9 | 1.0 | 2.1 | 2.0 | 1.1 | 0.9 | 1.0 | 0.9 | 1.3 | 1.4 | 1.0 | 1.0 | 0.25 |
| YPL194W | 0.9 | 1.4 | 0.4 | 1.1 | 1.3 | 1.1 | 1.5 | 1.3 | 9.6 | 3.1 | 0.9 | 0.9 | 1.4 | 1.1 | 1.4 | 0.9 | 1.0 | 0.8 | 0.22 |
| YPR025C | 1.1 | 0.8 | 0.6 | 0.6 | 1.6 | 0.9 | 1.4 | 1.3 | 1.9 | 1.7 | 0.9 | 0.7 | 1.1 | 1.3 | 1.1 | 1.3 | 1.1 | 1.5 | 0.87 |
| YGR180C | 3.1 | 1.1 | 1.9 | 1.0 | 2.0 | 0.6 | 0.5 | 1.0 | 1.4 | 1.5 | 1.0 | 0.9 | 0.9 | 0.9 | 1.3 | 1.2 | 1.0 | 1.9 | 3.90 |
| YEL019C | 1.1 | 1.6 | 0.3 | 0.7 | 0.9 | 1.5 | 1.2 | 0.9 | 0.7 | 0.7 | 1.2 | 1.0 | 1.0 | 1.7 | 1.5 | 0.3 | 0.8 | 0.9 | 0.27 |
| YLR288C | 1.2 | 0.8 | 1.6 | 1.8 | 1.7 | 1.2 | 1.3 | 1.5 | 0.8 | 1.3 | 1.4 | 0.6 | 0.7 | 1.2 | 0.9 | 1.5 | 1.2 | 1.3 | 0.31 |
| YMR284W | 1.1 | 1.0 | 0.5 | 2.0 | 0.8 | 0.8 | 0.8 | 1.1 | 1.6 | 1.8 | 1.1 | 0.6 | 1.0 | 1.2 | 0.7 | 1.3 | 1.1 | 1.3 | 0.54 |

| | | | | | | | | | | | | | | | | | | | |
|---|---|---|---|---|---|---|---|---|---|---|---|---|---|---|---|---|---|---|---|
| YMR035W | 1.2 | 1.7 | 1.5 | 1.7 | 0.8 | 1.5 | 2.3 | 1.4 | 1.7 | 1.6 | 1.5 | 0.5 | 1.4 | 1.3 | 1.1 | 1.5 | 2.2 | 2.4 | 0.96 |
| YOL043C | 1.1 | 0.9 | 0.8 | 1.7 | 1.2 | 0.8 | 1.0 | 0.9 | 1.0 | 1.8 | 1.2 | 0.8 | 0.9 | 1.5 | 0.6 | 2.5 | 1.0 | 1.0 | 0.38 |
| YGR231C | 1.0 | 1.2 | 0.7 | 1.0 | 1.3 | 1.9 | 2.1 | 1.1 | 3.2 | 2.3 | 1.6 | 1.3 | 3.9 | 2.2 | 1.3 | 2.5 | 1.0 | 1.5 | 1.74 |
| YHR164C | 0.9 | 1.3 | 0.9 | 0.7 | 0.9 | 1.4 | 0.8 | 1.1 | 1.3 | 3.1 | 0.6 | 1.2 | 2.0 | 1.3 | 2.2 | 1.1 | 0.8 | 1.0 | 0.44 |
| YJR046W | 1.0 | 1.2 | 1.2 | 1.5 | 0.8 | 2.0 | 0.9 | 0.9 | 6.0 | 2.4 | 0.6 | 0.9 | 3.0 | 1.0 | 1.1 | 0.8 | 0.7 | 0.7 | 0.45 |
| YLR103C | 0.8 | 1.5 | 1.1 | 0.4 | 1.0 | 1.1 | 1.3 | 1.4 | 0.8 | 0.5 | 0.9 | 1.6 | 3.0 | 1.0 | 0.8 | 1.5 | 1.1 | 1.4 | 1.32 |
| YMR072W | 1.1 | 1.0 | 1.4 | 1.0 | 1.7 | 1.6 | 1.4 | 1.0 | 3.0 | 1.2 | 1.9 | 0.8 | 2.1 | 1.2 | 0.8 | 1.7 | 0.9 | 1.9 | 2.06 |
| YDR054C | 1.3 | 1.0 | 0.7 | 1.4 | 0.7 | 1.9 | 1.1 | 1.5 | 2.9 | 3.2 | 1.9 | 1.0 | 2.5 | 1.1 | 1.6 | 1.4 | 0.8 | 1.4 | 1.18 |
| YAR007C | 0.7 | 1.9 | 0.8 | 0.5 | 1.5 | 1.6 | 0.8 | 1.1 | 4.1 | 1.7 | 0.9 | 0.9 | 1.7 | 1.4 | 1.2 | 0.9 | 0.8 | 1.0 | 1.03 |
| YGL058W | 1.1 | 1.1 | 0.6 | 0.7 | 1.0 | 1.5 | 0.0 | 1.3 | 3.7 | 1.8 | 0.8 | 1.3 | 1.0 | 1.3 | 1.3 | 0.6 | 1.1 | 0.7 | 1.07 |
| YIL036W | 0.9 | 1.3 | 1.3 | 1.8 | 0.9 | 1.8 | 1.4 | 1.1 | 3.6 | 2.7 | 1.2 | 1.2 | 1.9 | 1.1 | 0.8 | 1.3 | 1.1 | 1.3 | 0.66 |
| YNL213C | 1.7 | 11.6 | 0.9 | 1.5 | 1.3 | 1.7 | 1.4 | 1.8 | 3.0 | 1.5 | 1.5 | 0.8 | 0.9 | 1.8 | 1.0 | 1.7 | 0.9 | 1.2 | 0.70 |
| YNL312W | 0.9 | 1.0 | 1.6 | 0.7 | 1.4 | 1.7 | 0.5 | 1.2 | 3.7 | 2.5 | 1.4 | 0.9 | 0.7 | 1.1 | 0.8 | 0.8 | 1.2 | 1.1 | 2.05 |
| YNL261W | 1.0 | 1.3 | 0.6 | 1.3 | 0.7 | 1.3 | 1.0 | 1.1 | 2.3 | 1.5 | 0.8 | 0.8 | 0.8 | 1.4 | 0.7 | 1.1 | 0.8 | 1.0 | 0.91 |
| YGR180C | 3.1 | 1.1 | 1.9 | 1.0 | 2.0 | 1.0 | 0.5 | 1.0 | 1.4 | 1.5 | 1.0 | 0.9 | 0.9 | 0.9 | 1.3 | 1.2 | 1.0 | 1.9 | 3.90 |
| YJL026W | 2.5 | 2.4 | 2.9 | 1.0 | 1.7 | 1.0 | 0.5 | 1.2 | 1.0 | 1.1 | 1.3 | 1.0 | 1.2 | 1.0 | 1.1 | 1.6 | 1.4 | 2.1 | 3.74 |
| YDL017W | 0.8 | 2.3 | 1.2 | 1.3 | 1.2 | 1.1 | 1.1 | 1.2 | 0.8 | 1.8 | 0.8 | 0.8 | 0.9 | 1.3 | 1.3 | 1.0 | 0.8 | 1.0 | 0.38 |
| YML058W | 1.9 | 1.2 | 5.8 | 1.9 | 0.6 | 1.3 | 0.6 | 0.7 | 1.1 | 1.2 | 3.3 | 0.9 | 1.4 | 1.1 | 2.5 | 1.3 | 1.3 | 1.5 | 2.14 |
| YLR233C | 1.1 | 0.9 | 2.0 | 2.0 | 1.2 | 0.8 | 0.8 | 1.1 | 0.6 | 0.5 | 0.8 | 0.9 | 1.0 | 1.1 | 1.1 | 1.1 | 1.0 | 1.2 | 0.43 |
| YMR284W | 1.1 | 1.0 | 0.5 | 2.0 | 0.8 | 1.2 | 0.8 | 1.1 | 1.6 | 1.8 | 1.1 | 0.6 | 1.0 | 1.2 | 0.7 | 1.3 | 1.1 | 1.3 | 0.54 |
| YGL163C | 0.9 | 0.8 | 0.6 | 4.3 | 1.5 | 0.9 | 1.0 | 1.4 | 5.8 | 2.3 | 0.4 | 0.9 | 3.1 | 1.6 | 1.8 | 1.0 | 0.9 | 0.8 | 0.26 |
| YGL127C | 1.0 | 1.9 | 1.3 | 1.7 | 1.3 | 0.6 | 1.0 | 0.9 | 1.2 | 1.5 | 1.0 | 1.4 | 1.7 | 1.0 | 1.5 | 1.3 | 1.0 | 1.2 | 0.54 |
| YMR072W | 1.1 | 1.0 | 1.4 | 1.0 | 1.7 | 2.4 | 1.4 | 1.0 | 3.0 | 1.2 | 1.9 | 0.8 | 2.1 | 1.2 | 0.8 | 1.7 | 0.9 | 1.9 | 2.06 |
| YGL249W | 1.4 | 0.8 | 0.5 | 0.8 | 1.1 | 2.5 | 1.2 | 0.9 | 0.3 | 2.4 | 0.6 | 0.8 | 0.8 | 1.9 | 0.7 | 1.4 | 1.0 | 1.0 | 0.20 |
| YBR272C | 0.8 | 1.8 | 1.3 | 1.0 | 1.1 | | 1.9 | 1.1 | 1.7 | 2.7 | 1.1 | 0.5 | 1.3 | 1.0 | 0.5 | 1.7 | 1.1 | 1.2 | 0.52 |
| YDL059C | 2.6 | 5.1 | 1.4 | 1.7 | 1.2 | 1.2 | 1.1 | 1.2 | 10.5 | 7.8 | 2.0 | 1.3 | 1.7 | 4.7 | 0.7 | 1.5 | 0.8 | 0.9 | 0.63 |
| YAR007C | 0.7 | 1.9 | 0.8 | 0.5 | 1.5 | 0.6 | 0.8 | 1.1 | 4.1 | 1.7 | 0.9 | 0.9 | 1.7 | 1.4 | 1.2 | 0.9 | 0.8 | 1.0 | 1.03 |
| YBR073W | 0.7 | 1.0 | 0.9 | 0.7 | 1.2 | 0.7 | 0.6 | 1.1 | 2.5 | 1.8 | 0.5 | 0.9 | 1.4 | 1.0 | 1.3 | 0.8 | 0.7 | 0.8 | 0.63 |
| YML032C | 0.8 | 0.9 | 1.1 | 0.7 | 1.0 | 0.9 | 0.8 | 0.9 | 3.0 | 1.1 | 1.0 | 0.9 | 1.8 | 1.0 | 1.7 | 1.2 | 1.0 | 1.0 | 0.66 |
| YNL250W | 0.9 | 2.2 | 0.8 | 1.2 | 0.8 | 0.8 | 1.2 | 1.3 | 4.2 | 2.3 | 1.0 | 1.4 | 1.3 | 1.6 | 1.3 | 1.4 | 1.0 | 1.0 | 0.31 |
| YCR014C | 0.8 | 1.0 | 0.8 | 0.6 | 1.2 | 1.1 | 1.1 | 1.0 | 2.4 | 1.0 | 0.8 | 0.7 | 1.2 | 1.0 | 0.7 | 1.5 | 1.0 | 0.9 | 0.34 |

| | | | | | | | | | | | | | | | | | | | |
|---|---|---|---|---|---|---|---|---|---|---|---|---|---|---|---|---|---|---|---|
| YDR369C | 1.1 | 1.1 | 2.4 | 1.1 | 1.0 | 0.6 | 0.4 | 1.0 | 2.4 | 1.8 | 1.1 | 0.9 | 0.1 | 0.7 | 1.2 | 0.6 | 0.8 | 0.7 | 1.88 |
| YIL072W | 0.8 | 3.1 | 1.1 | 2.6 | 1.5 | 1.2 | 1.7 | 1.4 | 2.9 | 2.1 | 1.8 | 1.1 | 1.4 | 2.0 | 1.4 | 1.4 | 1.0 | 1.1 | 0.23 |
| YOR005C | 1.2 | 1.8 | 1.0 | 0.8 | 1.3 | 1.3 | 1.4 | 1.3 | 2.4 | 1.5 | 1.2 | 0.9 | 2.1 | 1.2 | 0.8 | 1.4 | 1.1 | 1.0 | 0.29 |
| YPL164C | 1.1 | 0.7 | 1.1 | 1.0 | 1.2 | 1.7 | 0.9 | 1.0 | 2.1 | 2.0 | 1.1 | 0.9 | 1.0 | 0.9 | 1.3 | 1.4 | 1.0 | 1.0 | 0.25 |
| YPL194W | 0.9 | 1.4 | 0.4 | 1.1 | 1.3 | 1.1 | 1.5 | 1.3 | 9.6 | 3.1 | 0.9 | 0.9 | 1.4 | 1.1 | 1.4 | 0.9 | 1.0 | 0.8 | 0.22 |
| YGR180C | 3.1 | 1.1 | 1.9 | 1.0 | 2.0 | 0.6 | 0.5 | 1.0 | 1.4 | 1.5 | 1.0 | 0.9 | 0.9 | 0.9 | 1.3 | 1.2 | 1.0 | 1.9 | 3.90 |
| YEL019C | 1.1 | 1.6 | 0.3 | 0.7 | 0.9 | 1.5 | 1.2 | 0.9 | 0.7 | 0.7 | 1.2 | 1.0 | 1.0 | 1.7 | 1.5 | 0.3 | 0.8 | 0.9 | 0.27 |
| YML058W | 1.9 | 1.2 | 5.8 | 1.9 | 0.6 | 0.6 | 0.6 | 0.7 | 1.1 | 1.2 | 3.3 | 0.9 | 1.4 | 1.1 | 2.5 | 1.3 | 1.3 | 1.5 | 2.14 |
| YLR288C | 1.2 | 0.8 | 1.6 | 1.8 | 1.7 | 1.2 | 1.3 | 1.5 | 0.8 | 1.3 | 1.4 | 0.6 | 0.7 | 1.2 | 0.9 | 1.5 | 1.2 | 1.3 | 0.31 |
| YMR284W | 1.1 | 1.0 | 0.5 | 2.0 | 0.8 | 0.8 | 0.8 | 1.1 | 1.6 | 1.8 | 1.1 | 0.6 | 1.0 | 1.2 | 0.7 | 1.3 | 1.1 | 1.3 | 0.54 |
| YMR096W | 1.7 | 1.7 | 1.1 | 0.8 | 1.0 | 2.2 | 1.9 | 2.0 | 3.3 | 3.7 | 1.5 | 1.4 | 8.4 | 4.7 | 28.9 | 2.2 | 2.2 | 1.0 | 0.62 |
| YGL091C | 2.0 | 1.8 | 1.0 | 1.7 | 0.8 | 1.6 | 1.3 | 1.8 | 10.8 | 6.0 | 2.1 | 1.2 | 2.5 | 3.1 | 0.7 | 1.0 | 1.4 | 1.8 | 0.96 |

## Table 4 Energy system protein genes

The level of expressed mRNA in the presence of chemical substance/ The level of expressed mRNA in the absence of chemical substance

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| YCR107W | 12.1 | 2.6 | 1.8 | 1.6 | 0.7 | 1.5 | 1.5 | 15.6 | 196.6 | 34.0 | 23.0 | 0.9 | 4.0 | 8.3 | 4.8 | 3.1 | 1.3 | 1.1 | 0.59 |
| YDL243C | 14.5 | 2.7 | 2.4 | 1.0 | 1.1 | 2.2 | 1.6 | 11.8 | 64.2 | 29.6 | 19.6 | 1.1 | 4.1 | 12.0 | 4.2 | 3.0 | 1.8 | 1.5 | 0.76 |
| YFL056C | 19.0 | 2.3 | 2.3 | 1.5 | 0.9 | 0.6 | 1.4 | 18.5 | 162.3 | 31.3 | 68.3 | 1.0 | 4.7 | 7.8 | 5.0 | 3.4 | 1.0 | 1.1 | 0.55 |
| YFL057C | 20.9 | 5.9 | 1.5 | 1.8 | 0.9 | 1.2 | 1.8 | 18.0 | 51.8 | 46.1 | 27.7 | 1.0 | 4.1 | 23.4 | 3.1 | 3.9 | 1.6 | 1.3 | 0.71 |
| YJR155W | 7.6 | 3.7 | 1.4 | 2.5 | 0.7 | 1.4 | 1.7 | 10.6 | 38.2 | 18.8 | 15.4 | 1.0 | 5.7 | 9.4 | 2.6 | 5.6 | 1.3 | 1.4 | 0.64 |
| YNL331C | 8.6 | 3.6 | 1.3 | 1.0 | 1.6 | 1.8 | 1.9 | 13.1 | 42.6 | 36.3 | 21.8 | 0.9 | 3.1 | 7.5 | 2.3 | 4.0 | 1.7 | 1.3 | 0.58 |
| YOL165C | 10.1 | 4.5 | 1.8 | 0.9 | 0.9 | 1.7 | 1.4 | 17.8 | 46.9 | 23.3 | 17.6 | 0.8 | 3.7 | 9.1 | 3.0 | 1.8 | 1.6 | 1.0 | 0.69 |
| YPL171C | 15.2 | 4.1 | 3.5 | 2.2 | 1.1 | 1.3 | 1.7 | 20.5 | 60.0 | 50.6 | 37.0 | 1.4 | 2.4 | 9.3 | 1.4 | 1.2 | 2.4 | 1.5 | 0.47 |
| YDL021W | 5.1 | 1.7 | 2.4 | 3.7 | 1.7 | 6.5 | 5.9 | 2.7 | 2.5 | 7.4 | 4.7 | 2.4 | 5.3 | 3.7 | 0.7 | 7.3 | 1.9 | 3.2 | 0.47 |
| YGR043C | 2.6 | 3.7 | 3.2 | 7.9 | 0.9 | 16.3 | 6.5 | 2.6 | 10.9 | 8.4 | 3.6 | 3.3 | 6.9 | 4.1 | 3.0 | 13.7 | 1.6 | 4.8 | 0.66 |
| YHR179W | 3.3 | 2.3 | 2.6 | 0.7 | 1.2 | 3.7 | 1.9 | 3.3 | 3.6 | 5.8 | 2.0 | 2.2 | 2.6 | 5.8 | 2.7 | 1.0 | 1.9 | 1.6 | 2.69 |
| YJR048W | 1.3 | 0.9 | 2.2 | 1.4 | 0.8 | 0.5 | 0.3 | 0.9 | 0.5 | 1.5 | 1.0 | 0.8 | 0.4 | 2.5 | 0.6 | 1.5 | 1.2 | 1.2 | 1.19 |
| YKR097W | 1.6 | 2.4 | 1.3 | 3.3 | 1.1 | 1.7 | 3.7 | 2.5 | 1.9 | 3.3 | 0.8 | 1.8 | 17.5 | 2.1 | 2.5 | 2.2 | 1.1 | 1.5 | 0.16 |
| YML087C | 1.8 | 1.6 | 0.8 | 2.5 | 0.9 | 0.9 | 1.1 | 1.3 | 1.9 | 4.9 | 1.6 | 0.8 | 1.5 | 2.1 | 0.3 | 2.0 | 0.7 | 0.8 | 0.52 |
| YPL088W | 3.3 | 1.3 | 0.6 | 2.5 | 1.5 | 7.1 | 3.7 | 1.9 | 0.6 | 2.8 | 1.1 | 3.6 | 4.8 | 1.6 | 3.2 | 4.3 | 9.1 | 8.3 | 0.69 |
| YDL174C | 0.9 | 1.8 | 0.9 | 1.3 | 0.5 | 1.7 | 3.6 | 0.8 | 0.5 | 1.7 | 0.9 | 2.8 | 5.9 | 1.1 | 0.6 | 5.1 | 2.2 | 4.0 | 0.63 |
| YCR012W | 1.2 | 1.5 | 5.1 | 1.2 | 1.1 | 2.5 | 1.4 | 0.8 | 1.4 | 1.5 | 1.8 | 1.2 | 4.1 | 0.9 | 1.4 | 1.4 | 0.8 | 1.3 | 4.48 |
| YFR053C | 1.8 | 1.4 | 3.0 | 2.2 | 0.9 | 3.2 | 2.1 | 2.8 | 0.6 | 1.1 | 1.5 | 1.7 | 3.0 | 0.6 | 0.6 | 3.0 | 1.4 | 2.2 | 4.17 |
| YGL062W | 0.6 | 1.3 | 1.1 | 0.6 | 0.9 | 1.0 | 1.3 | 0.8 | 1.6 | 2.5 | 0.7 | 1.6 | 4.5 | 1.2 | 2.7 | 1.5 | 1.1 | 0.9 | 0.77 |
| YGR192C | 1.4 | 1.0 | 3.8 | 1.0 | 1.1 | 1.7 | 1.7 | 1.1 | 0.9 | 1.3 | 2.3 | 2.2 | 3.4 | 1.0 | 1.9 | 1.1 | 1.1 | 1.3 | 7.49 |
| YGR244C | 1.0 | 1.2 | 1.6 | 1.6 | 0.8 | 2.6 | 3.9 | 1.8 | 1.4 | 1.1 | 1.8 | 1.9 | 2.6 | 1.2 | 2.0 | 2.6 | 1.9 | 3.1 | 1.12 |
| YGR254W | 1.2 | 1.3 | 3.8 | 1.3 | 1.2 | 1.9 | 1.5 | 1.4 | 0.8 | 1.2 | 1.3 | 1.7 | 3.1 | 0.6 | 2.4 | 1.4 | 1.3 | 1.2 | 7.01 |
| YIL160C | 0.8 | 2.4 | 2.3 | 3.5 | 1.0 | 1.6 | 1.1 | 1.2 | 3.2 | 3.8 | 2.4 | 1.0 | 2.3 | 1.5 | 5.6 | 8.8 | 2.4 | 3.0 | 0.27 |
| YJL052W | 1.6 | 0.9 | 4.0 | 1.8 | 0.7 | 2.4 | 2.1 | 1.5 | 1.6 | 2.0 | 4.3 | 2.4 | 5.6 | 1.3 | 2.3 | 2.3 | 1.2 | 1.9 | 6.19 |
| YJR009C | 1.1 | 1.7 | 5.6 | 1.0 | 1.2 | 1.6 | 1.6 | 1.1 | 1.1 | 1.3 | 1.1 | 1.5 | 2.7 | 1.0 | 2.1 | 1.5 | 1.1 | 1.3 | 5.86 |
| YLR345W | 1.4 | 1.4 | 1.4 | 1.2 | 1.2 | 1.6 | 1.2 | 1.4 | 4.5 | 4.0 | 2.5 | 1.6 | 3.5 | 1.5 | 1.0 | 1.2 | 1.2 | 1.4 | 2.65 |
| YMR118C | 1.3 | 2.9 | 1.4 | 1.6 | 1.2 | 2.0 | 1.0 | 1.0 | 9.3 | 1.4 | 0.7 | 0.8 | 4.2 | 0.9 | 0.9 | 8.1 | 0.8 | 1.0 | 0.38 |
| YNL071W | 0.9 | 0.9 | 1.7 | 1.5 | 0.6 | 1.3 | 1.4 | 0.9 | 1.1 | 1.2 | 1.6 | 1.3 | 2.5 | 0.8 | 1.1 | 1.1 | 1.0 | 1.2 | 1.61 |

| | | | | | | | | | | | | | | | | | | | |
|---|---|---|---|---|---|---|---|---|---|---|---|---|---|---|---|---|---|---|---|
| YNL241C | 1.3 | 2.5 | 4.3 | 1.0 | 0.8 | 0.9 | 3.2 | 0.9 | 3.4 | 7.4 | 3.0 | 2.0 | 4.9 | 1.1 | 7.0 | 2.8 | 1.0 | 1.0 | 0.68 |
| YPL240C | 0.7 | 1.4 | 2.4 | 0.9 | 1.3 | 1.2 | 1.4 | 1.2 | 3.5 | 2.6 | 1.0 | 0.8 | 2.9 | 0.7 | 1.2 | 0.7 | 0.8 | 1.0 | 4.83 |
| YAL060W | 1.1 | 1.8 | 3.2 | 2.7 | 1.2 | 4.2 | 3.3 | 0.9 | 0.6 | 2.5 | 2.4 | 0.8 | 2.2 | 0.9 | 0.9 | 3.2 | 1.1 | 1.8 | 2.39 |
| YCL040W | 0.9 | 5.6 | 10.1 | 2.0 | 0.5 | 3.5 | 2.9 | 0.7 | 0.9 | 3.0 | 8.2 | 2.3 | 5.6 | 0.7 | 3.4 | 3.1 | 1.4 | 1.7 | 1.98 |
| YDR001C | 1.0 | 2.2 | 2.6 | 1.0 | 1.1 | 2.2 | 1.5 | 0.9 | 2.2 | 3.0 | 0.7 | 1.1 | 2.8 | 1.0 | 4.4 | 1.9 | 1.1 | 1.6 | 0.75 |
| YDR231C | 1.1 | 1.6 | 1.0 | 1.5 | 1.1 | 2.2 | 1.7 | 1.3 | 1.5 | 1.4 | 1.3 | 1.1 | 2.4 | 1.5 | 1.0 | 3.0 | 1.2 | 1.3 | 0.92 |
| YER178W | 0.8 | 0.9 | 3.6 | 1.0 | 0.7 | 2.5 | 1.6 | 0.8 | 1.7 | 1.3 | 2.5 | 1.3 | 2.4 | 0.9 | 1.8 | 1.0 | 1.1 | 0.9 | 2.18 |
| YGR008C | 2.4 | 3.0 | 1.7 | 3.2 | 0.9 | 2.9 | 3.7 | 1.9 | 3.1 | 2.4 | 2.6 | 2.0 | 3.4 | 1.3 | 1.5 | 2.3 | 1.7 | 4.2 | 3.03 |
| YGR256W | 1.2 | 1.5 | 2.3 | 0.8 | 0.9 | 6.2 | 1.4 | 2.2 | 3.4 | 2.5 | 2.7 | 1.5 | 3.9 | 1.1 | 2.7 | 5.3 | 1.1 | 0.8 | 0.94 |
| YHL008C | 0.9 | 0.5 | 1.0 | 1.6 | 0.8 | 0.8 | 0.7 | 0.9 | 1.6 | 1.7 | 1.4 | 0.8 | 1.8 | 1.0 | 1.8 | 0.9 | 0.8 | 0.9 | 0.40 |
| YHR174W | 1.1 | 1.4 | 3.3 | 1.2 | 1.3 | 1.5 | 1.6 | 1.2 | 1.0 | 1.5 | 1.4 | 1.5 | 3.6 | 0.6 | 2.0 | 1.1 | 1.0 | 1.2 | 7.34 |
| YIL045W | 1.7 | 1.4 | 1.9 | 2.2 | 1.3 | 1.7 | 1.6 | 1.1 | 2.1 | 3.2 | 1.2 | 1.5 | 2.0 | 1.6 | 0.6 | 2.9 | 1.1 | 1.8 | 0.37 |
| YKL035W | 1.0 | 0.9 | 4.8 | 1.2 | 0.8 | 1.2 | 0.6 | 1.0 | 0.8 | 1.2 | 2.1 | 1.0 | 2.0 | 0.6 | 1.0 | 1.5 | 1.0 | 1.9 | 2.54 |
| YKL152C | 1.4 | 1.3 | 1.9 | 0.9 | 1.3 | 1.6 | 1.5 | 1.0 | 0.9 | 1.5 | 1.7 | 1.5 | 2.7 | 1.0 | 1.8 | 2.0 | 1.1 | 1.7 | 3.28 |
| YML054C | 1.5 | 1.8 | 1.3 | 3.4 | 1.3 | 1.8 | 1.2 | 1.5 | 4.1 | 1.8 | 1.4 | 1.8 | 2.8 | 2.1 | 1.1 | 7.8 | 1.1 | 1.6 | 0.25 |
| YML100W | 0.8 | 2.7 | 10.6 | 1.4 | 0.9 | 2.8 | 2.2 | 0.7 | 1.7 | 1.4 | 3.2 | 1.2 | 3.8 | 1.2 | 1.8 | 2.2 | 1.0 | 1.5 | 0.88 |
| YML125C | 0.8 | 0.9 | 0.7 | 0.7 | 0.9 | 1.3 | 1.0 | 1.3 | 2.6 | 1.6 | 1.0 | 1.0 | 2.2 | 1.6 | 1.9 | 0.7 | 1.2 | 0.9 | 2.21 |
| YMR089C | 1.0 | 1.1 | 0.9 | 0.9 | 0.8 | 1.2 | 1.2 | 1.3 | 5.6 | 2.5 | 1.3 | 0.8 | 2.3 | 0.9 | 1.8 | 1.1 | 0.9 | 1.1 | 0.69 |
| YMR105C | 2.0 | 3.0 | 5.0 | 4.2 | 0.9 | 2.8 | 2.8 | 1.1 | 0.6 | 2.9 | 3.0 | 1.7 | 3.3 | 1.0 | 0.9 | 2.0 | 1.6 | 2.6 | 1.21 |
| YNL237W | 1.3 | 1.3 | 1.5 | 1.0 | 1.5 | 4.6 | 4.9 | 1.1 | 0.9 | 2.1 | 1.5 | 1.8 | 3.2 | 1.3 | 1.6 | 4.4 | 1.3 | 1.1 | 0.21 |
| YOL126C | 1.1 | 0.8 | 1.0 | 1.6 | 0.7 | 1.5 | 1.4 | 0.9 | 0.8 | 2.4 | 1.8 | 1.1 | 2.6 | 1.2 | 1.4 | 2.5 | 1.6 | 2.4 | 0.59 |
| YOR347C | 0.9 | 0.9 | 2.1 | 2.2 | 0.7 | 0.9 | 0.9 | 0.8 | 0.5 | 0.9 | 1.4 | 1.2 | 1.8 | 0.9 | 2.0 | 1.4 | 1.7 | 1.3 | 1.31 |
| YPR026W | 0.9 | 1.2 | 5.0 | 1.3 | 0.8 | | 1.3 | 1.0 | 0.9 | 3.1 | 1.6 | 1.2 | 2.3 | 1.5 | 3.1 | 2.5 | 0.9 | 1.3 | 0.26 |
| YAL038W | 1.0 | 1.0 | 3.0 | 1.4 | 1.4 | 1.3 | 0.9 | 1.1 | 0.1 | 1.1 | 1.0 | 1.2 | 1.8 | 0.5 | 3.1 | 0.9 | 1.0 | 1.0 | 7.02 |
| YDR380W | 0.8 | 0.8 | 0.8 | 1.7 | 1.1 | 0.4 | 0.5 | 0.2 | 0.2 | 0.3 | 0.4 | 0.4 | 0.4 | 0.6 | 2.4 | 0.2 | 0.8 | 0.8 | 1.04 |
| YLR273C | 1.5 | 1.0 | 1.3 | 2.4 | | 1.2 | 1.2 | 1.3 | 2.8 | 2.4 | 1.4 | 0.9 | 1.7 | 1.3 | 2.6 | 1.5 | 1.0 | 1.0 | 0.20 |
| YGR207C | 1.5 | 1.1 | 1.0 | 1.1 | 1.5 | 2.8 | 1.7 | 1.9 | 2.1 | 2.6 | 1.4 | 1.1 | 1.0 | 2.2 | 0.6 | 1.8 | 1.2 | 1.8 | 1.49 |
| YNL037C | 1.4 | 1.8 | 1.7 | 1.1 | 1.7 | 2.8 | 2.8 | 1.0 | 0.6 | 3.2 | 1.4 | 1.3 | 1.9 | 1.3 | 0.9 | 1.4 | 0.9 | 1.3 | 2.05 |
| YOR136W | 1.0 | 0.9 | 3.5 | 1.0 | 1.5 | 3.9 | 3.2 | 1.1 | 0.3 | 3.4 | 1.4 | 1.2 | 1.5 | 0.9 | 1.2 | 1.3 | 0.8 | 1.3 | 3.20 |
| YPL271W | 1.2 | 3.2 | 4.1 | 1.2 | 1.4 | 3.3 | 1.2 | 0.9 | 0.6 | 1.2 | 1.7 | 0.8 | 1.2 | 1.3 | 0.8 | 1.4 | 1.2 | 1.3 | 1.34 |
| YAL054C | 1.2 | 1.1 | 1.4 | 1.6 | 1.1 | 2.2 | 1.1 | 1.1 | 8.9 | 4.1 | 1.3 | 1.0 | 1.8 | 1.3 | 0.9 | 2.5 | 1.5 | 1.4 | 0.24 |

| | | | | | | | | | | | | | | | | | | | |
|---|---|---|---|---|---|---|---|---|---|---|---|---|---|---|---|---|---|---|---|
| YEL011W | 2.0 | 1.6 | 1.7 | 6.3 | 1.1 | 2.8 | 2.7 | 0.9 | 2.5 | 1.6 | 3.7 | 1.5 | 1.5 | 1.5 | 0.4 | 2.4 | 1.4 | 2.5 | 0.89 |
| YFR015C | 1.2 | 0.8 | 3.2 | 2.1 | 1.2 | 3.0 | 7.3 | 0.7 | 0.3 | 1.8 | 3.9 | 0.4 | 2.5 | 3.1 | 0.4 | 2.4 | 1.3 | 1.2 | 0.66 |
| YGL253W | 0.8 | 1.0 | 3.2 | 0.7 | 1.3 | 2.4 | 1.7 | 1.1 | 0.3 | 1.4 | 1.0 | 1.2 | 1.5 | 0.5 | 0.8 | 0.4 | 0.7 | 0.8 | 4.63 |
| YIL111W | 1.9 | 1.0 | 1.8 | 2.7 | | 2.6 | 3.2 | 1.2 | 1.6 | 1.1 | 2.1 | 0.9 | 1.7 | 1.5 | 1.1 | 2.5 | 1.9 | 3.9 | 1.52 |
| YKL150W | 1.1 | 2.0 | 3.0 | 1.4 | 1.4 | 2.3 | 2.4 | 1.3 | 1.1 | 1.3 | 1.9 | 1.2 | 1.8 | 1.4 | 0.9 | 6.1 | 1.2 | 2.7 | 2.20 |
| YPR006C | 1.9 | 1.5 | 0.5 | 1.9 | 2.0 | 2.4 | 2.8 | 1.5 | 1.5 | 3.9 | 2.4 | 1.1 | 1.8 | 1.2 | 0.7 | 1.6 | 1.8 | 2.1 | 0.46 |
| YBR145W | 1.5 | 0.7 | 2.8 | 0.9 | 1.1 | 11.5 | 58.8 | 1.0 | 0.1 | 1.1 | 1.1 | 1.0 | 2.0 | 2.2 | 1.2 | 3.6 | 1.7 | 2.0 | 2.17 |
| YBR299W | 2.0 | 0.9 | 1.1 | 3.5 | 1.6 | 0.8 | 3.6 | 2.2 | 1.1 | 5.3 | 2.4 | 1.2 | 0.7 | 1.4 | 0.6 | 3.9 | 1.0 | 1.1 | 0.32 |
| YEL020C | 1.0 | 1.5 | 0.8 | 2.9 | 1.5 | 1.3 | 2.4 | 1.2 | 1.4 | 1.1 | 1.3 | 0.8 | 1.2 | 1.0 | 1.4 | 2.1 | 1.2 | 1.3 | 0.31 |
| YGL134W | 1.2 | 1.3 | 0.5 | 0.8 | 1.4 | 1.2 | 2.3 | 1.4 | 1.1 | 1.4 | 1.1 | 0.9 | 0.7 | 1.1 | 0.5 | 1.7 | 0.9 | 1.3 | 0.53 |
| YOL157C | 1.0 | 1.1 | 1.3 | 2.5 | 1.4 | 0.9 | 2.7 | 1.4 | 2.3 | 4.8 | 1.2 | 1.2 | 1.4 | 1.2 | 1.1 | 3.5 | 1.4 | 1.3 | 0.41 |
| YBR126C | 0.8 | 1.9 | 5.6 | 1.2 | 0.7 | 2.9 | 2.3 | 0.6 | 1.7 | | 1.1 | 1.3 | 2.1 | 0.7 | 1.0 | 1.7 | 1.5 | 1.3 | 1.96 |
| YCR005C | 1.2 | 1.9 | 2.0 | 1.2 | 0.9 | 1.6 | 4.4 | 1.2 | 1.5 | 1.5 | 2.1 | 0.5 | 0.7 | 0.8 | 0.7 | 0.7 | 1.6 | 1.7 | 2.38 |
| YIL172C | 1.1 | 1.1 | 1.6 | 1.7 | | 1.3 | 2.5 | 1.6 | 2.8 | 7.1 | 1.0 | 1.4 | 2.0 | 1.3 | 1.4 | 2.8 | 1.1 | 1.2 | 0.42 |
| YOR221C | 0.8 | 1.0 | 0.9 | 1.1 | 1.7 | 0.8 | 2.1 | 0.9 | 1.4 | 1.7 | 1.1 | 0.8 | 1.0 | 1.1 | 1.0 | 1.4 | 0.9 | 1.2 | 0.39 |
| YBR196C | 0.8 | 0.6 | 3.9 | 1.4 | 0.8 | 0.8 | 1.4 | 1.1 | 0.3 | 0.9 | 2.3 | 1.0 | 1.4 | 0.5 | 1.9 | 1.1 | 0.8 | 1.0 | 6.60 |
| YEL047C | 1.3 | 1.9 | 3.2 | 1.2 | 0.8 | 0.6 | 1.3 | 1.0 | 2.7 | 2.3 | 4.2 | 1.2 | 1.6 | 1.1 | 1.0 | 1.3 | 0.9 | 1.0 | 1.02 |
| YMR318C | 1.8 | 2.4 | 2.2 | 0.7 | 1.2 | 2.1 | 0.8 | 3.6 | 2.3 | 4.8 | 3.6 | 0.8 | 1.8 | 1.7 | 1.5 | 1.1 | 1.1 | 1.1 | 3.17 |
| YER061C | 0.9 | 0.9 | 1.2 | 2.5 | 0.8 | 0.4 | 0.9 | 0.7 | 0.3 | 0.7 | 2.2 | 0.7 | 0.9 | 1.0 | 0.8 | 1.8 | 1.2 | 1.2 | 0.84 |
| YJL045W | 1.8 | 2.2 | 1.6 | 5.3 | 0.7 | 0.6 | 0.7 | 0.9 | 9.7 | 1.6 | 2.3 | 1.1 | 1.6 | 1.2 | 3.4 | 2.4 | 0.9 | 0.9 | 0.42 |
| YLL009C | 1.0 | 1.2 | 0.6 | 1.9 | 1.5 | 1.2 | 1.7 | 1.0 | 1.3 | 1.7 | 3.5 | 0.7 | 0.9 | 1.4 | 1.1 | 2.8 | 0.9 | 1.2 | 1.66 |
| YPR160W | 1.4 | 3.8 | 3.6 | 3.3 | 0.7 | 4.5 | 1.8 | 0.9 | 0.9 | 1.3 | 4.4 | 2.1 | 2.2 | 1.1 | 1.4 | 5.3 | 1.0 | 2.9 | 1.42 |
| YDL085W | 1.2 | 1.9 | 1.2 | 2.0 | 1.2 | 1.2 | 1.4 | 1.0 | 7.8 | 3.4 | 2.7 | 0.8 | 1.7 | 1.2 | 0.7 | 4.5 | 0.9 | 0.9 | 0.23 |
| YJL221C | 1.1 | 1.0 | 1.1 | 1.3 | 0.9 | 6.6 | 2.5 | 1.8 | 2.7 | 4.4 | 0.8 | 1.1 | 1.4 | 1.1 | 1.1 | 3.3 | 1.1 | 1.4 | 0.41 |
| YKL085W | 1.5 | 2.3 | 1.6 | 1.2 | 1.2 | 1.9 | 1.5 | 1.2 | 1.9 | 3.0 | 1.8 | 0.8 | 1.5 | 1.0 | 0.5 | 1.7 | 0.9 | 1.3 | 2.16 |
| YLR174W | 1.2 | 1.5 | 1.7 | 2.1 | 0.9 | 0.9 | 1.6 | 1.1 | 2.5 | 8.3 | 1.3 | 1.3 | 1.8 | 1.7 | 0.8 | 4.6 | 0.9 | 1.2 | 0.41 |
| YNL009W | 1.1 | 1.9 | 2.0 | 1.4 | 1.4 | 1.1 | 1.2 | 1.1 | 1.4 | 3.3 | 1.3 | 0.9 | 1.3 | 1.3 | 2.7 | 3.3 | 1.2 | 2.3 | 0.45 |
| YNL117W | 0.9 | 4.7 | 1.7 | 0.8 | 0.8 | | 1.1 | 1.7 | 12.8 | 4.4 | 1.4 | 0.7 | 1.3 | 2.0 | 2.6 | 1.6 | 1.1 | 0.9 | 0.24 |
| YAL061W | 1.7 | 2.4 | 3.3 | 3.8 | 1.0 | 1.0 | 2.0 | 0.8 | 5.5 | 1.4 | 4.1 | 1.1 | 1.4 | 0.7 | 0.6 | 1.1 | 1.4 | 1.2 | 0.88 |
| YBR117C | 0.8 | 1.6 | 1.5 | 1.4 | 0.9 | 2.3 | 1.0 | 0.7 | 5.9 | 1.5 | 0.4 | 0.8 | 2.1 | 0.9 | 1.0 | 12.0 | 0.7 | 0.7 | 0.49 |
| YEL071W | 1.1 | 1.6 | 2.6 | 0.4 | 1.3 | 1.9 | 2.0 | 1.2 | 3.6 | 2.8 | 2.1 | 0.9 | 1.6 | 1.2 | 1.6 | 1.2 | 1.1 | 1.2 | 1.13 |

| | | | | | | | | | | | | | | | | | | | |
|---|---|---|---|---|---|---|---|---|---|---|---|---|---|---|---|---|---|---|---|
| YGR112W | 1.0 | 2.9 | 1.1 | 1.3 | 0.9 | 1.1 | 1.3 | 1.3 | 2.6 | 1.3 | 1.3 | 0.7 | 1.1 | 1.1 | 1.2 | 2.8 | 0.9 | 1.2 | 0.31 |
| YLR164W | 1.0 | 2.4 | 0.3 | 1.0 | 1.2 | 1.2 | 1.1 | 1.2 | 5.3 | 2.0 | 3.2 | 0.7 | 1.0 | 1.5 | 0.7 | 11.0 | 1.5 | 1.2 | 0.31 |
| YNR032W | 1.0 | 1.5 | 1.0 | 2.1 | 1.5 | 1.0 | 1.1 | 1.0 | 6.2 | 2.4 | 1.4 | 0.6 | 1.3 | 1.4 | 0.9 | 1.0 | 1.2 | 1.3 | 0.71 |
| YPL031C | 0.9 | 2.0 | 1.4 | 0.8 | 1.5 | 1.8 | 1.6 | 1.0 | 5.7 | 1.4 | 1.4 | 0.8 | 1.6 | 0.9 | 0.7 | 2.0 | 1.4 | 1.2 | 0.49 |
| YPR048W | 1.3 | 2.0 | 0.9 | 1.1 | 0.9 | 0.6 | 0.9 | 1.5 | 4.0 | 2.1 | 1.0 | 0.8 | 0.6 | 1.2 | 0.6 | 0.8 | 0.7 | 0.8 | 0.75 |
| YBL058W | 0.9 | 1.5 | 0.5 | 1.1 | 1.6 | 0.9 | 1.5 | 1.2 | 2.6 | 2.1 | 1.3 | 0.8 | 1.6 | 1.3 | 1.8 | 1.8 | 1.0 | 1.4 | 1.42 |
| YBR001C | 0.9 | 1.4 | 1.1 | 1.4 | 0.9 | 1.1 | 0.9 | 1.3 | 2.3 | 2.1 | 1.0 | 1.0 | 2.0 | 1.2 | 1.8 | 1.4 | 1.0 | 1.6 | 0.61 |
| YCR105W | 2.2 | 1.2 | 1.2 | 3.0 | 0.9 | 1.0 | 1.0 | 1.4 | 2.0 | 3.9 | 2.3 | 0.9 | 1.9 | 1.9 | 3.1 | 1.3 | 1.3 | 1.1 | 0.36 |
| YIR031C | 0.9 | 1.6 | 0.6 | 0.6 | 1.6 | 1.7 | 1.4 | 1.0 | 2.6 | 1.0 | 1.2 | 0.8 | 1.0 | 1.2 | 0.8 | 0.7 | 0.9 | 0.8 | 0.44 |
| YGL191W | 2.3 | 1.6 | 1.4 | 1.8 | 0.9 | 1.6 | 1.0 | 1.6 | 1.1 | 1.3 | 1.6 | 0.9 | 1.1 | 1.6 | 0.7 | 1.8 | 1.2 | 1.7 | 2.35 |
| YLR038C | 2.6 | 1.1 | 0.6 | 2.1 | 1.8 | 1.2 | 1.0 | 1.4 | 0.4 | 0.9 | 1.4 | 0.6 | 0.5 | 1.6 | 0.7 | 2.1 | 1.2 | 2.1 | 1.52 |
| YGR087C | 1.0 | 15.0 | 1.7 | 0.6 | 1.0 | 1.1 | 1.0 | 0.8 | 0.2 | 0.9 | 0.8 | 1.2 | 1.2 | 0.7 | 3.5 | 0.6 | 1.0 | 0.6 | 1.88 |
| YGL256W | 0.9 | 5.3 | 1.1 | 1.3 | 0.7 | 0.7 | 0.5 | 1.0 | 0.4 | 1.2 | 1.0 | 0.9 | 0.8 | 0.9 | 1.0 | 0.7 | 0.7 | 0.8 | 0.90 |
| YDL181W | 1.4 | 1.8 | 1.9 | 1.8 | 1.7 | 1.2 | 1.1 | 0.6 | 0.7 | 0.8 | 0.8 | 0.9 | 0.9 | 1.1 | 0.4 | 1.1 | 1.5 | 1.4 | 0.85 |
| YPL262W | 1.1 | 1.7 | 3.8 | 1.2 | 0.6 | 1.6 | 1.3 | 1.0 | 1.4 | 5.8 | 1.4 | 1.1 | 1.5 | 1.2 | 0.6 | 1.4 | 1.1 | 1.2 | 0.79 |
| YLR377C | 1.1 | 2.6 | 1.8 | 1.2 | 1.0 | 0.7 | 0.9 | 0.7 | 1.8 | 1.6 | 0.6 | 0.6 | 1.2 | 2.9 | 0.8 | 2.7 | 0.9 | 1.4 | 0.14 |
| YBR221C | 0.8 | 0.7 | 3.2 | 1.5 | 1.3 | 1.7 | 1.5 | 0.8 | 1.0 | 1.3 | 1.2 | 1.3 | 1.7 | 0.8 | 1.1 | 1.5 | 0.9 | 1.0 | 3.62 |
| YKL141W | 1.6 | 1.1 | 4.7 | 1.7 | 1.5 | 1.2 | 0.5 | 0.8 | 0.8 | 0.5 | 1.2 | 0.8 | 0.8 | 1.4 | 0.6 | 2.8 | 0.9 | 1.8 | 2.84 |
| YLR134W | 0.9 | 0.6 | 2.3 | 0.8 | 1.3 | 2.5 | 1.1 | 0.8 | 0.1 | 0.7 | 1.1 | 1.2 | 1.5 | 0.6 | 1.7 | 0.5 | 0.6 | 0.8 | 3.47 |
| YLR258W | 1.7 | 1.0 | 4.2 | 3.5 | 0.9 | 1.8 | 1.8 | 0.9 | 0.8 | 1.3 | 1.2 | 0.9 | 1.4 | 1.2 | 0.6 | 1.7 | 1.0 | 2.0 | 1.36 |
| YOR178C | 1.4 | 1.3 | 4.8 | 2.3 | 1.0 | 2.7 | 0.9 | 0.9 | 0.2 | 1.7 | 4.1 | 1.7 | 1.0 | 1.0 | 1.1 | 1.7 | 1.0 | 1.5 | 0.56 |
| YBL099W | 0.8 | 0.9 | 3.1 | 1.4 | 1.0 | 0.9 | 0.9 | 0.8 | 0.9 | 0.7 | 2.1 | 1.0 | 0.9 | 0.6 | 1.0 | 0.9 | 0.7 | 1.2 | 3.49 |
| YDR050C | 1.9 | 1.3 | 2.3 | 1.8 | 0.9 | 1.7 | 1.5 | 1.3 | 0.4 | 1.3 | 2.0 | 1.4 | 2.0 | 1.2 | 1.9 | 1.3 | 1.1 | 2.3 | 6.26 |
| YDR178W | 2.0 | 2.0 | 3.4 | 2.2 | 0.9 | 2.1 | 0.6 | 0.9 | 0.9 | 0.8 | 2.0 | 1.3 | 1.5 | 1.5 | 0.7 | 3.0 | 1.2 | 2.3 | 2.27 |
| YDR298C | 1.3 | 1.2 | 2.6 | 1.3 | 1.3 | 1.2 | 1.0 | 1.2 | 1.0 | 1.6 | 1.2 | 1.1 | 1.2 | 1.5 | 0.8 | 2.0 | 1.1 | 1.6 | 2.69 |
| YEL024W | 1.5 | 0.9 | 3.8 | 1.1 | 1.3 | 1.1 | 0.7 | 1.0 | 0.7 | 0.6 | 0.9 | 0.8 | 0.6 | 1.1 | 0.6 | 2.1 | 1.1 | 1.5 | 1.59 |
| YJL121C | 1.0 | 0.5 | 1.9 | 0.8 | 1.0 | 1.1 | 0.7 | 1.0 | 0.1 | 0.4 | 1.1 | 0.6 | 0.7 | 0.9 | 1.0 | 0.6 | 1.1 | 1.0 | 1.00 |
| YJR121W | 1.2 | 1.1 | 3.3 | 1.0 | 0.8 | 1.5 | 1.0 | 0.7 | 0.8 | 1.3 | 0.9 | 0.8 | 1.0 | 0.7 | 0.9 | 1.2 | 1.1 | 1.4 | 3.99 |
| YKL060C | 1.7 | 0.8 | 2.5 | 1.0 | 1.6 | 1.3 | 1.2 | 1.1 | 0.3 | 0.8 | 2.1 | 1.3 | 1.2 | 0.8 | 1.6 | 1.5 | 1.2 | 1.7 | 6.01 |
| YKL148C | 0.9 | 0.8 | 3.7 | 0.7 | 0.8 | 0.5 | 0.6 | 0.8 | 1.7 | 1.7 | 1.5 | 0.8 | 0.8 | 0.8 | 0.6 | 1.1 | 0.8 | 1.0 | 0.54 |
| YLL041C | 1.5 | 0.5 | 4.6 | 1.0 | 1.6 | 1.2 | 0.3 | 0.8 | 0.7 | 0.8 | 1.3 | 0.8 | 1.0 | 1.2 | 0.4 | 3.1 | 1.1 | 1.7 | 1.75 |

| | | | | | | | | | | | | | | | | | | | |
|---|---|---|---|---|---|---|---|---|---|---|---|---|---|---|---|---|---|---|---|
| YLR044C | 0.8 | 0.6 | 2.2 | 0.9 | 1.7 | 1.5 | 1.3 | 0.7 | 0.0 | 1.2 | 1.1 | 1.4 | 1.7 | 0.5 | 2.2 | 0.6 | 0.9 | 0.9 | 5.16 |
| YLR284C | 0.9 | 1.0 | 3.8 | 1.9 | 1.6 | 0.9 | 0.8 | 1.2 | 0.9 | 0.9 | 1.5 | 0.6 | 0.8 | 1.3 | 1.0 | 4.5 | 2.9 | 8.1 | 0.84 |
| YLR304C | 0.7 | 0.6 | 5.0 | 0.7 | 0.6 | 1.9 | 0.6 | 0.6 | 0.1 | 2.2 | 1.6 | 1.0 | 0.5 | 0.7 | 1.6 | 1.8 | 0.5 | 0.7 | 2.39 |
| YLR354C | 1.2 | 2.5 | 2.3 | 1.5 | 1.6 | 1.5 | 1.1 | 1.4 | 0.4 | 0.9 | 1.7 | 1.2 | 1.0 | 0.9 | 2.4 | 1.3 | 1.0 | 1.5 | 4.53 |
| YMR205C | 0.5 | 0.7 | 2.3 | 0.8 | 1.2 | 1.2 | 0.9 | 0.7 | 0.3 | 0.8 | 1.0 | 1.1 | 1.3 | 0.5 | 0.9 | 0.9 | 0.6 | 0.5 | 4.75 |
| YMR261C | 0.8 | 1.6 | 3.6 | 0.8 | 0.9 | 0.6 | 1.3 | 0.7 | 1.6 | 1.3 | 0.8 | 0.9 | 1.6 | 0.6 | 0.8 | 1.6 | 0.7 | 1.1 | 0.78 |
| YMR323W | 0.8 | 1.1 | 2.9 | 1.1 | 0.7 | 0.4 | 1.1 | 0.8 | 0.5 | 1.3 | 2.5 | 1.2 | 1.8 | 1.2 | 37.3 | 0.6 | 1.1 | 0.6 | 1.04 |
| YOL086C | 1.1 | 0.5 | 2.2 | 1.1 | 1.9 | 1.7 | 1.9 | 0.8 | 0.1 | 1.2 | 2.6 | 1.3 | 1.7 | 0.6 | 1.6 | 1.4 | 1.1 | 1.3 | 4.19 |
| YOR142W | 1.3 | 1.2 | 3.4 | 1.0 | 1.4 | 1.6 | 1.0 | 0.8 | 1.1 | 1.5 | 1.5 | 0.8 | 1.4 | 0.8 | 1.1 | 0.8 | 0.9 | 1.0 | 1.31 |
| YPL061W | 0.4 | 1.4 | 5.0 | 1.3 | 1.6 | 1.7 | 0.5 | 0.8 | 1.3 | 0.7 | 1.1 | 1.0 | 1.1 | 0.6 | 1.7 | 1.0 | 1.6 | 2.6 | 3.23 |
| YDL107W | 1.3 | 1.3 | 1.0 | 2.2 | 1.0 | 1.3 | 1.8 | 1.8 | 1.2 | 1.8 | 1.0 | 1.0 | 1.2 | 1.5 | 1.0 | 2.0 | 1.1 | 1.5 | 0.51 |
| YDR529C | 1.8 | 1.0 | 0.9 | 3.2 | 1.2 | 0.8 | 0.5 | 1.3 | 0.5 | 0.6 | 0.8 | 0.6 | 0.7 | 1.5 | 0.5 | 1.8 | 1.0 | 2.3 | 3.11 |
| YGL018C | 1.5 | 1.0 | 1.8 | 3.0 | 1.1 | 0.4 | 1.1 | 0.8 | 0.5 | 1.4 | 1.1 | 1.0 | 1.3 | 1.2 | 1.1 | 1.5 | 0.8 | 1.0 | 0.36 |
| YBR185C | 1.5 | 1.0 | 1.3 | 2.0 | 0.8 | 1.2 | 1.2 | 1.1 | 0.9 | 1.6 | 1.2 | 0.7 | 1.0 | 1.7 | 1.0 | 1.7 | 0.8 | 1.3 | 0.89 |
| YEL039C | 1.1 | 0.7 | 1.3 | 5.1 | 0.9 | 1.5 | 0.6 | 1.1 | 1.2 | 1.2 | 1.7 | 0.6 | 0.5 | 1.4 | 0.4 | 3.5 | 0.7 | 1.1 | 1.59 |
| YGR174C | 1.1 | 1.6 | 0.6 | 2.4 | 1.0 | 1.4 | 1.0 | 1.6 | 1.8 | 0.7 | 1.3 | 0.9 | 1.5 | 1.6 | 0.7 | 3.2 | 1.3 | 2.3 | 1.05 |
| YKL016C | 1.5 | 1.3 | 0.7 | 1.7 | 1.5 | 0.8 | 1.5 | 1.6 | 1.3 | 1.8 | 1.3 | 1.0 | 1.3 | 1.6 | 0.7 | 1.8 | 1.0 | 1.7 | 2.08 |
| YLR395C | 1.8 | 1.3 | 2.2 | 1.9 | 0.7 | 0.4 | | 1.0 | 1.0 | 0.9 | 1.5 | 0.6 | 0.5 | 1.7 | 0.4 | 1.2 | 1.2 | 2.3 | 1.76 |
| YML120C | 1.1 | 1.2 | 1.9 | 1.9 | 0.6 | 0.6 | 0.8 | 0.9 | 1.3 | 1.5 | 1.3 | 0.9 | 1.2 | 0.8 | 0.5 | 1.5 | 1.0 | 1.6 | 0.74 |
| YMR073C | 1.1 | 1.1 | 1.2 | 1.5 | 0.8 | 0.9 | 1.0 | 1.1 | 1.2 | 1.2 | 0.9 | 0.8 | 1.2 | 2.0 | 0.9 | 1.5 | 0.8 | 1.2 | 0.69 |
| YOR388C | 1.0 | 1.6 | 1.3 | 4.7 | 1.3 | 1.4 | 1.0 | 0.7 | 0.1 | 1.4 | 1.3 | 0.7 | 1.1 | 1.3 | 4.0 | 0.8 | 1.0 | 0.9 | 0.21 |
| YPL275W | 0.6 | 1.5 | 1.3 | 5.1 | 1.2 | 1.0 | 1.1 | 1.0 | 0.2 | 1.2 | 0.9 | 0.6 | 1.0 | 1.0 | 1.4 | 0.9 | 1.0 | 1.0 | 0.28 |
| YPL276W | 1.2 | 1.5 | 1.6 | 3.0 | 1.5 | 1.1 | 1.2 | 0.9 | -0.2 | 1.8 | -0.4 | 0.6 | 0.9 | 0.9 | 1.0 | 0.6 | 1.1 | 0.8 | 0.22 |
| YGL205W | 1.0 | 0.9 | 0.7 | 0.8 | 1.1 | 1.6 | 0.8 | 1.2 | 0.5 | 0.6 | 0.4 | 1.1 | 1.0 | 1.0 | 2.0 | 4.1 | 3.9 | 9.1 | 0.24 |
| YJL166W | 2.1 | 1.5 | 1.6 | 1.3 | 1.6 | 0.7 | 0.8 | 1.4 | 1.3 | 1.2 | 1.4 | 0.7 | 0.8 | 1.9 | 0.5 | 2.3 | 1.7 | 4.0 | 2.31 |
| YNL202W | 0.7 | 1.3 | 1.2 | 1.7 | 0.9 | 2.0 | 0.9 | 1.2 | 1.3 | 1.1 | 1.2 | 0.9 | 1.8 | 1.0 | 1.4 | 4.2 | 2.4 | 3.3 | 0.47 |
| YDR377W | 1.3 | 1.1 | 1.3 | 1.4 | 1.9 | 1.3 | 1.1 | 1.3 | 1.0 | 0.9 | 1.4 | 0.9 | 0.7 | 1.4 | 0.6 | 1.8 | 1.2 | 2.5 | 3.00 |
| YGL187C | 1.5 | 0.8 | 1.6 | 1.8 | 1.3 | 1.1 | 0.6 | 0.7 | 0.6 | 0.6 | 0.7 | 0.8 | 0.7 | 0.9 | 0.3 | 1.4 | 0.9 | 2.4 | 2.73 |
| YJL216C | 1.1 | 4.7 | 2.2 | 0.9 | 0.8 | | 1.5 | 1.9 | 1.5 | 1.3 | 1.1 | 1.0 | 1.1 | 1.1 | 1.2 | 1.5 | 1.2 | 2.0 | 0.25 |
| YKR009C | 1.0 | 1.2 | 1.5 | 0.9 | 1.0 | 1.8 | 1.0 | 0.9 | 2.4 | 2.1 | 1.2 | 1.0 | 1.8 | 1.0 | 1.0 | 4.6 | 1.9 | 2.5 | 0.27 |
| YCR046C | 1.0 | 1.2 | 1.2 | 1.6 | 1.0 | 0.8 | 1.2 | 0.9 | 1.9 | 1.4 | 1.5 | 1.1 | 1.5 | 1.3 | 1.4 | 2.8 | 1.3 | 1.0 | 0.63 |

| | | | | | | | | | | | | | | | | | | | |
|---|---|---|---|---|---|---|---|---|---|---|---|---|---|---|---|---|---|---|---|
| YML129C | 1.1 | 0.9 | 0.9 | 1.2 | 1.7 | 1.7 | 1.8 | 1.2 | 1.4 | 2.2 | 1.2 | 0.8 | 1.0 | 1.4 | 1.1 | 2.9 | 1.3 | 1.3 | 1.04 |
| YPR184W | 1.2 | 1.4 | 5.7 | 2.2 | 0.9 | 1.5 | 1.3 | 0.7 | 1.9 | 3.2 | 3.6 | 1.4 | 2.5 | 1.3 | 1.5 | 3.1 | 1.0 | 1.7 | 0.37 |
| YDL067C | 1.8 | 1.0 | 1.6 | 1.3 | 2.0 | 0.9 | 0.8 | 1.1 | 0.9 | 0.9 | 1.4 | 1.0 | 0.6 | 1.4 | 0.7 | 1.8 | 1.2 | 1.7 | 1.85 |
| YDL078C | 0.7 | 1.1 | 1.2 | 0.8 | 1.3 | 1.1 | 1.7 | 1.0 | 1.1 | 1.0 | 1.7 | 0.8 | 1.0 | 0.8 | 0.5 | 2.2 | 1.1 | 2.0 | 1.65 |
| YDR079W | 1.3 | 1.1 | 0.8 | 1.1 | 1.2 | 1.7 | 1.3 | 2.6 | 0.9 | 0.8 | 1.3 | 1.1 | 1.3 | 1.5 | 0.6 | 2.6 | 1.1 | 1.3 | 1.06 |
| YGR062C | 0.9 | 1.0 | 0.7 | 1.4 | 0.7 | 0.9 | 1.3 | 0.8 | 1.0 | 1.4 | 1.6 | 1.0 | 1.5 | 1.2 | 0.5 | 1.8 | 0.9 | 1.1 | 0.54 |
| YKR058W | 1.3 | 0.9 | 1.4 | 1.9 | 0.8 | 1.4 | 1.4 | 1.5 | 1.3 | 1.2 | 1.5 | 0.9 | 0.7 | 1.3 | 0.5 | 2.6 | 0.8 | 1.6 | 0.73 |
| YLR295C | 1.5 | 1.1 | 1.1 | 1.7 | 2.2 | 1.1 | 1.1 | 1.2 | 1.4 | 1.2 | 1.3 | 0.5 | 0.8 | 1.2 | 0.5 | 2.4 | 0.9 | 1.5 | 1.07 |
| YMR267W | 0.8 | 1.8 | 0.7 | 0.9 | 1.0 | 1.4 | 1.2 | 1.5 | 0.4 | 0.8 | 1.0 | 0.8 | 0.7 | 1.0 | 0.9 | 2.6 | 0.9 | 1.3 | 0.94 |

## Table 5 Transport facilitation protein genes

| yeast genes | The level of expressed mRNA in the presence of chemical substance/ The level of expressed mRNA in the absence of chemical substance | | | | | | | | | | | | | | | | | | |
|---|---|---|---|---|---|---|---|---|---|---|---|---|---|---|---|---|---|---|---|
| | (1) | (2) | (3) | (4) | (5) | (6) | (7) | (8) | (9) | (10) | (11) | (12) | (13) | (14) | (15) | (16) | (17) | (18) | Intensity |
| YBR008C | 3.0 | 2.4 | 4.9 | 0.7 | 0.9 | 1.0 | 0.9 | 2.8 | 54.6 | 21.1 | 9.4 | 0.8 | 2.0 | 4.1 | 0.8 | 3.1 | 2.0 | 1.2 | 0.37 |
| YBR296C | 7.0 | 4.3 | 2.2 | 2.5 | 1.4 | 1.4 | 0.2 | 1.1 | 0.4 | 1.8 | 1.5 | 1.6 | 2.0 | 2.3 | 1.7 | 0.3 | 3.8 | 4.0 | 0.54 |
| YDR406W | 0.8 | 1.2 | 3.1 | 1.1 | 1.0 | 5.9 | 1.8 | 0.6 | 1.1 | 1.7 | 1.4 | 3.9 | 7.7 | 0.5 | 0.8 | 5.5 | 2.7 | 5.6 | 0.53 |
| YOR153W | 1.6 | 0.9 | 5.1 | 1.1 | 1.0 | 7.4 | 2.6 | 0.5 | 3.2 | 1.2 | 1.0 | 4.0 | 11.8 | 0.3 | 3.6 | 1.6 | 2.5 | 3.1 | 1.91 |
| YGR281W | 1.2 | 1.0 | 1.3 | 2.2 | 0.6 | 1.9 | 0.9 | 0.7 | 2.7 | 1.4 | 0.7 | 2.4 | 12.9 | 0.9 | 2.1 | 1.1 | 2.8 | 3.1 | 1.04 |
| YHL047C | 0.6 | 4.4 | 1.0 | 1.0 | 1.2 | 8.4 | 16.8 | 1.5 | 1.2 | 11.9 | 1.0 | 2.6 | 3.8 | 0.5 | 1.1 | 2.3 | 1.2 | 1.2 | 0.74 |
| YBR294W | 6.3 | 14.4 | 0.9 | 2.6 | 1.4 | 1.2 | 1.1 | 1.0 | 5.2 | 5.2 | 1.2 | 1.0 | 3.1 | 1.3 | 1.0 | 0.9 | 1.0 | 0.9 | 0.18 |
| YGL006W | 2.5 | 1.3 | 1.3 | 2.6 | 1.0 | 1.0 | 1.3 | 0.9 | 3.0 | 1.2 | 0.8 | 0.9 | 4.5 | 0.9 | 1.1 | 1.7 | 2.4 | 4.1 | 1.37 |
| YGR197C | 1.1 | 2.4 | 2.5 | 1.0 | 0.7 | 0.5 | 1.3 | 0.8 | 16.7 | 3.2 | 1.1 | 1.2 | 2.4 | 1.5 | 1.0 | 1.5 | 1.7 | 1.9 | 0.37 |
| YJL034W | 0.7 | 1.7 | 3.0 | 0.8 | 1.2 | 1.1 | 1.3 | 1.5 | 2.8 | 1.1 | 1.3 | 1.6 | 6.1 | 0.6 | 7.9 | 0.6 | 1.1 | 1.0 | 4.58 |
| YNL055C | 1.1 | 1.8 | 5.5 | 2.0 | 0.9 | 2.6 | 1.2 | 0.7 | 1.1 | 1.0 | 1.9 | 1.7 | 2.4 | 0.9 | 1.1 | 2.6 | 1.2 | 1.7 | 4.10 |
| YBR052C | 1.6 | 1.6 | 1.5 | 2.6 | 1.4 | 2.1 | 3.0 | 1.3 | 2.3 | 2.5 | 1.7 | 1.3 | 2.1 | 1.5 | 0.7 | 3.1 | 1.8 | 3.1 | 2.77 |
| YBR207W | 0.8 | 1.7 | 0.8 | 2.0 | 1.7 | 1.6 | 4.3 | 0.9 | 1.7 | 2.2 | 1.1 | 0.9 | 2.2 | 0.9 | 1.4 | 1.4 | 1.2 | 1.4 | 1.39 |
| YBR293W | 1.9 | 3.1 | 1.0 | 1.8 | 0.8 | 0.9 | 0.9 | 0.9 | 5.5 | 2.4 | 1.1 | 1.0 | 3.0 | 1.4 | 3.1 | 1.6 | 1.0 | 0.9 | 0.94 |
| YDL198C | 1.4 | 1.0 | 2.1 | 1.4 | 0.7 | 0.8 | 1.0 | 1.1 | 1.9 | 2.7 | 2.5 | 1.2 | 2.0 | 1.7 | 1.2 | 1.4 | 1.5 | 1.1 | 1.19 |
| YDL245C | 1.0 | 2.4 | 1.6 | 2.2 | 1.3 | 1.1 | 1.2 | 1.2 | 0.4 | 2.8 | 1.5 | 1.0 | 2.0 | 1.2 | 1.8 | 1.5 | 1.0 | 1.1 | 0.28 |
| YDR497C | 0.7 | 0.5 | 0.6 | 1.3 | 0.7 | 0.6 | 0.5 | 0.6 | 0.4 | 0.8 | 0.7 | 0.9 | 2.7 | 0.6 | 10.1 | | 1.6 | 1.8 | 1.45 |
| YER053C | 1.6 | 1.8 | 1.9 | 1.7 | 0.6 | 2.8 | 2.8 | 1.3 | 1.3 | 3.9 | 2.4 | 1.7 | 4.1 | 1.3 | 1.1 | 2.8 | 1.2 | 2.3 | 1.83 |
| YFL041W | 0.7 | 1.6 | 1.4 | 1.2 | 1.2 | 2.0 | 3.4 | 1.0 | 0.8 | 5.1 | 0.9 | 1.1 | 1.8 | 1.0 | 0.9 | 1.4 | 1.1 | 0.9 | 0.89 |
| YGR055W | 2.5 | 5.7 | 12.0 | 0.7 | 1.3 | 1.1 | 0.6 | 1.1 | 5.0 | 2.4 | 1.5 | 0.9 | 1.9 | 1.2 | 2.1 | 0.6 | 0.7 | 0.7 | 1.42 |
| YJL219W | 1.2 | 2.5 | 3.1 | 1.6 | 1.2 | 1.5 | 0.9 | 1.5 | 4.0 | 2.3 | 2.3 | 1.1 | 4.0 | 1.1 | 2.6 | 1.4 | 1.6 | 1.2 | 0.91 |
| YJR106W | 0.9 | 3.8 | 1.5 | 1.1 | 0.9 | 1.2 | 1.0 | 0.9 | 2.0 | 1.6 | 1.6 | 0.9 | 3.5 | 0.9 | 1.2 | 1.9 | 1.2 | 1.3 | 0.29 |
| YKL146W | 0.8 | 1.3 | 1.3 | 0.9 | 1.0 | 1.3 | 0.9 | 0.8 | 4.6 | 2.8 | 0.9 | 1.1 | 2.7 | 1.0 | 1.5 | 1.3 | 1.0 | 0.9 | 0.42 |
| YLL028W | 0.6 | 0.9 | 4.6 | 0.5 | 0.9 | 1.7 | 1.0 | 0.7 | 1.2 | 2.3 | 0.7 | 1.3 | 4.1 | 0.7 | 3.1 | 0.6 | 1.2 | 1.2 | 1.02 |
| YLR348C | 1.1 | 4.5 | 1.2 | 1.2 | 0.9 | 1.9 | 0.9 | 0.9 | 2.1 | 1.3 | 1.3 | 0.9 | 1.9 | 1.0 | 2.4 | 1.1 | 1.1 | 1.0 | 0.64 |
| YOL119C | 2.6 | 4.8 | 1.5 | 2.3 | 1.5 | 0.9 | 2.2 | 2.0 | 5.0 | 7.4 | 3.4 | 0.9 | 1.6 | 2.4 | 4.7 | 2.8 | 1.4 | 1.0 | 0.37 |
| YOL163W | 1.5 | 3.2 | 2.1 | 3.3 | 0.9 | 0.5 | 0.9 | 1.1 | 7.5 | 6.2 | 1.6 | 1.0 | 2.4 | 1.2 | 1.0 | 1.4 | 0.8 | 0.8 | 0.30 |
| YOR035C | 1.2 | 1.2 | 0.3 | 1.2 | 1.0 | 1.9 | 1.3 | 1.0 | 1.3 | 1.7 | 1.0 | 1.1 | 2.5 | 0.9 | 3.8 | 1.8 | 1.6 | 2.1 | 0.52 |

| | | | | | | | | | | | | | | | | | | | |
|---|---|---|---|---|---|---|---|---|---|---|---|---|---|---|---|---|---|---|---|
| YOR130C | 0.8 | 1.7 | 1.4 | 1.0 | 1.8 | 1.6 | 1.3 | 0.8 | 2.0 | 1.7 | 0.6 | 0.9 | 2.1 | 1.3 | 1.0 | 0.8 | 1.5 | 1.1 | 0.51 |
| YOR273C | 0.7 | 1.0 | 1.7 | 1.0 | 1.1 | 0.4 | 0.4 | 0.6 | 0.6 | 0.5 | 1.5 | 0.8 | 2.6 | 0.5 | 0.8 | 0.6 | 3.2 | 3.1 | 1.20 |
| YOR332W | 1.1 | 1.0 | 1.0 | 1.0 | 0.9 | 1.8 | 0.9 | 1.5 | 1.8 | 1.1 | 1.5 | 1.0 | 2.2 | 1.1 | 1.2 | 1.2 | 1.3 | 1.7 | 3.58 |
| YCR098C | 1.6 | 3.0 | 1.7 | 2.0 | 1.4 | 1.6 | 1.2 | 1.0 | 1.4 | 2.4 | 1.3 | 1.3 | 1.4 | 1.1 | 11.0 | 0.8 | 1.7 | 1.6 | 0.22 |
| YGR138C | 1.1 | 1.6 | 1.0 | 1.2 | 0.8 | 0.8 | 0.7 | 1.0 | 0.5 | 1.0 | 0.7 | 0.6 | 0.6 | 0.9 | 4.5 | 0.7 | 0.7 | 0.7 | 1.24 |
| YBR295W | 1.4 | 1.5 | 3.3 | 1.1 | 0.8 | | 1.6 | 0.9 | 1.2 | 3.3 | 1.1 | 0.9 | 1.8 | 1.1 | 4.8 | 1.4 | 1.3 | 1.2 | 0.27 |
| YGL255W | 0.7 | 0.9 | 0.9 | 0.8 | 1.4 | 1.1 | 1.0 | 0.7 | 0.3 | 5.0 | 0.5 | 0.7 | 0.2 | 1.0 | 2.6 | 0.5 | 0.6 | 0.5 | 1.36 |
| YHL035C | 0.7 | 1.3 | 0.9 | 1.9 | 0.9 | 4.1 | 1.7 | 1.1 | 0.6 | 4.6 | 0.9 | 1.4 | 1.1 | 0.8 | 3.1 | 2.4 | 1.1 | 1.2 | 0.77 |
| YIL022W | 0.8 | 0.7 | 1.1 | 1.6 | 0.8 | 1.2 | 0.7 | 0.8 | 1.1 | 1.3 | 1.0 | 0.9 | 1.2 | 0.6 | 2.9 | 1.1 | 0.8 | 0.7 | 0.52 |
| YLR378C | 0.7 | 0.9 | 2.6 | 0.5 | 1.0 | 1.2 | 0.7 | 0.5 | 1.1 | 0.7 | 1.0 | 1.2 | 1.6 | 0.4 | 5.9 | 0.4 | 0.9 | 0.7 | 1.23 |
| YCL038C | 1.6 | 1.6 | 1.4 | 1.6 | 1.2 | 3.2 | 1.5 | 1.2 | 1.1 | 0.9 | 2.0 | 1.3 | 1.9 | 1.3 | 2.0 | 1.8 | 1.4 | 1.5 | 1.04 |
| YFL054C | 1.6 | 1.1 | 1.4 | 2.2 | 1.3 | 2.4 | 1.1 | 1.5 | 2.0 | 2.3 | 1.7 | 1.2 | 2.3 | 0.9 | 1.4 | 1.3 | 1.7 | 1.4 | 1.06 |
| YHL040C | 1.6 | 4.9 | 1.5 | 0.4 | 1.1 | 7.3 | 4.0 | 1.5 | 1.1 | 11.3 | 1.7 | 2.0 | 2.0 | 1.1 | 3.4 | 1.0 | 1.4 | 1.0 | 0.69 |
| YKR039W | 1.4 | 3.5 | 1.3 | 1.7 | 1.0 | 2.8 | 1.0 | 1.0 | 1.9 | 2.0 | 1.9 | 1.5 | 1.3 | 1.2 | 1.7 | 1.9 | 0.9 | 1.1 | 0.40 |
| YPL271W | 1.2 | 3.2 | 4.1 | 1.2 | 1.4 | 3.3 | 1.2 | 0.9 | 0.6 | 1.2 | 1.7 | 0.8 | 1.2 | 1.3 | 0.8 | 1.4 | 1.2 | 1.3 | 1.34 |
| YBR068C | 1.1 | 1.0 | 1.9 | 2.5 | 1.4 | 1.9 | | 1.0 | 0.8 | 2.5 | 0.8 | 0.8 | 2.2 | 1.8 | 1.6 | 5.5 | 1.1 | 1.3 | 1.99 |
| YCR037C | 0.7 | 1.0 | 0.8 | 0.8 | 1.3 | 1.8 | 1.0 | 0.7 | 0.6 | 0.6 | 0.8 | 0.7 | 1.3 | 0.8 | 1.0 | 1.1 | 1.4 | 0.9 | 0.74 |
| YDL128W | 0.8 | 1.1 | 2.1 | 1.0 | 1.0 | 2.3 | 0.9 | 0.8 | 0.5 | 0.7 | 1.2 | 0.9 | 1.6 | 0.7 | 1.1 | 1.0 | 0.9 | 1.0 | 2.80 |
| YDR270W | 0.7 | 1.5 | 1.4 | 1.3 | 1.0 | 2.2 | 1.7 | 1.2 | 2.9 | 3.6 | 1.0 | 1.0 | 1.8 | 1.0 | 1.4 | 1.1 | 0.9 | 0.7 | 0.38 |
| YEL065W | 0.3 | 3.8 | 1.2 | 0.4 | 1.4 | 2.3 | 4.3 | 0.9 | 0.1 | 4.9 | 0.6 | 1.7 | 0.8 | 0.4 | 2.2 | 2.6 | 0.7 | 0.6 | 2.10 |
| YGL008C | 0.6 | 0.7 | 3.1 | 0.9 | 0.8 | 1.9 | 1.4 | 0.7 | 0.0 | 0.4 | 0.7 | 2.4 | 1.9 | 1.2 | 1.0 | 2.1 | 1.1 | 1.0 | 4.08 |
| YGL104C | 0.8 | 2.0 | 2.1 | 1.8 | 1.0 | 2.1 | 1.2 | 0.8 | 4.6 | 2.1 | 1.8 | 1.1 | 2.0 | 1.0 | 0.9 | 1.7 | 1.3 | 1.3 | 0.58 |
| YGL167C | 0.9 | 0.7 | 1.6 | 1.3 | 1.2 | 2.0 | 1.2 | 0.8 | 1.3 | 1.1 | 0.8 | 1.1 | 1.5 | 0.8 | 1.3 | 1.3 | 1.4 | 1.4 | 1.41 |
| YGR065C | 0.8 | 1.5 | 2.6 | 1.1 | 1.2 | 2.4 | 2.4 | 1.0 | 1.2 | 2.6 | 1.1 | 0.5 | 0.8 | 1.3 | 1.2 | 1.1 | 0.7 | 0.7 | 0.68 |
| YHR092C | 2.3 | 1.0 | 7.5 | 2.5 | 1.4 | 1.7 | 0.5 | 1.5 | 0.2 | 0.9 | 1.5 | 1.0 | 0.5 | 1.6 | 0.2 | 1.2 | 0.9 | 1.3 | 6.09 |
| YIL088C | 0.9 | 2.1 | 2.1 | 1.3 | 1.6 | 2.2 | 1.7 | 0.8 | 1.0 | 1.7 | 1.8 | 0.9 | 1.5 | 1.2 | 1.2 | 1.8 | 1.2 | 1.4 | 1.89 |
| YNL259C | 1.6 | 3.5 | 1.1 | 1.2 | 1.1 | 3.9 | 3.8 | 1.5 | 1.6 | 2.1 | 1.6 | 0.7 | 1.1 | 1.4 | 0.7 | 1.7 | 2.6 | 2.4 | 1.22 |
| YOR270C | 0.7 | 0.5 | 2.0 | 0.8 | 0.8 | 1.8 | 1.0 | 0.6 | 0.4 | 0.9 | 1.5 | 0.7 | 1.3 | 0.7 | 1.1 | 0.8 | 0.9 | 0.9 | 3.49 |
| YPL265W | 0.7 | 0.4 | 2.3 | 1.1 | 1.0 | 2.3 | 8.9 | 2.3 | 0.2 | 1.2 | 1.8 | 0.9 | 1.1 | 1.1 | 1.2 | 2.0 | 0.5 | 0.4 | 1.42 |
| YPR124W | 0.2 | 0.4 | 0.3 | 1.4 | 2.0 | 2.0 | 2.9 | 2.7 | 0.2 | 1.5 | 2.0 | 0.8 | 0.6 | 0.4 | 1.1 | 2.4 | 1.0 | 1.3 | 2.57 |
| YER119C | 0.7 | 2.1 | 1.8 | 0.7 | 0.8 | 1.3 | 0.8 | 0.6 | 5.8 | 1.2 | 2.2 | 0.7 | 1.6 | 1.0 | 2.3 | 0.8 | 1.0 | 0.9 | 0.32 |

| | | | | | | | | | | | | | | | | | | | |
|---|---|---|---|---|---|---|---|---|---|---|---|---|---|---|---|---|---|---|---|
| YFL055W | 2.0 | 6.7 | 1.3 | 2.5 | 1.5 | 1.1 | 1.4 | 1.3 | 23.9 | 6.5 | 2.7 | 0.9 | 1.3 | 1.5 | 0.7 | 1.4 | 1.4 | 1.0 | 0.23 |
| YLL055W | 2.6 | 19.0 | 4.8 | 1.7 | 1.0 | 1.4 | 1.7 | 1.4 | 19.1 | 14.0 | 3.6 | 0.7 | 1.5 | 1.3 | 0.7 | 2.8 | 1.1 | 1.3 | 0.47 |
| YHL036W | 2.2 | 4.8 | 3.4 | 2.0 | 1.3 | 1.3 | 1.0 | 1.2 | 9.0 | 4.4 | 2.3 | 1.2 | 1.5 | 1.2 | 0.9 | 1.1 | 1.0 | 1.0 | 0.60 |
| YHR048W | 2.5 | 1.4 | 1.4 | 1.7 | 1.0 | 0.8 | 0.8 | 1.9 | 4.5 | 2.7 | 2.0 | 0.9 | 1.1 | 1.7 | 0.9 | 1.4 | 0.7 | 0.9 | 0.26 |
| YKL221W | 1.3 | 0.7 | 2.0 | 1.0 | 1.0 | 0.9 | 1.3 | 1.2 | 7.3 | 2.7 | 1.9 | 0.7 | 1.6 | 1.4 | 1.1 | 1.3 | 0.8 | 0.9 | 0.27 |
| YLR092W | 5.0 | 5.6 | 1.4 | 0.6 | 1.3 | 1.0 | 1.3 | 1.5 | 12.7 | 5.8 | 3.1 | 1.0 | 1.8 | 1.4 | 0.9 | 1.2 | 1.0 | 1.1 | 0.24 |
| YML116W | 4.1 | 1.3 | 1.5 | 1.4 | 1.2 | 0.9 | 1.4 | 2.2 | 1.4 | 3.1 | 4.3 | 0.5 | 0.8 | 2.0 | 1.9 | 1.0 | 1.0 | 1.0 | 0.94 |
| YBR291C | 2.0 | 0.9 | 1.0 | 1.5 | 0.9 | 1.1 | 0.9 | 2.2 | 0.9 | 1.0 | 1.1 | 0.9 | 1.0 | 1.7 | 0.5 | 1.5 | 0.9 | 1.3 | 1.19 |
| YCL069W | 0.9 | 4.5 | 0.9 | 0.8 | 0.9 | | 1.4 | 1.3 | 1.2 | 6.9 | 1.0 | 0.9 | 0.8 | 1.3 | 1.4 | 0.9 | 1.0 | 1.0 | 0.25 |
| YJR095W | 1.2 | 20.5 | 1.9 | 6.7 | 1.2 | 1.5 | 2.0 | 0.9 | 0.5 | 6.3 | 0.6 | 0.7 | 0.8 | 1.3 | 0.8 | 0.8 | 1.3 | 0.9 | 0.23 |
| YKL188C | 1.3 | 0.7 | 1.9 | 2.4 | 1.0 | 0.8 | 1.1 | 0.7 | 2.5 | 2.8 | 1.1 | 1.2 | 1.2 | 2.1 | 1.4 | 2.7 | 1.1 | 1.5 | 0.27 |
| YKL217W | 1.8 | 2.4 | 1.0 | 2.1 | 1.1 | 1.2 | 1.6 | 1.1 | 0.9 | 4.1 | 1.6 | 0.8 | 1.2 | 1.2 | 2.2 | 3.0 | 1.7 | 3.3 | 0.29 |
| YKR105C | 0.8 | 0.9 | 0.9 | 1.5 | 1.0 | 1.2 | 1.0 | 1.3 | 1.0 | 5.2 | 0.0 | 0.8 | 2.5 | 1.2 | 1.5 | 0.7 | 0.8 | 1.0 | 0.26 |
| YOL158C | 0.7 | 4.0 | 2.4 | 0.9 | 1.2 | 2.0 | 1.6 | 0.7 | 1.7 | 6.1 | 0.7 | 0.9 | 1.4 | 1.0 | 0.9 | 1.2 | 1.4 | 1.7 | 1.30 |
| YPL224C | 1.0 | 2.2 | 1.3 | 2.5 | 0.6 | 1.3 | 2.2 | 1.3 | 3.2 | 4.2 | 0.8 | 1.1 | 1.3 | 1.4 | 1.0 | 2.5 | 1.3 | 1.9 | 0.64 |
| YPR201W | 33.2 | 0.9 | 1.1 | 1.0 | 1.2 | 1.9 | 0.8 | 0.9 | 8.3 | 5.6 | 2.2 | 0.7 | 1.1 | 1.8 | 0.8 | 0.9 | 0.8 | 0.9 | 0.29 |
| YAL067C | 2.7 | 12.1 | 1.5 | 1.4 | 0.7 | 1.2 | 0.8 | 1.1 | 7.6 | 2.9 | 1.2 | 1.0 | 1.1 | 1.3 | 2.4 | 1.6 | 1.0 | 1.0 | 0.33 |
| YDL149W | 0.8 | 2.0 | 1.2 | 1.6 | 1.5 | 1.0 | 0.7 | 1.2 | 3.2 | 2.0 | 1.2 | 0.7 | 1.5 | 1.2 | 1.8 | 1.4 | 0.8 | 1.0 | 0.33 |
| YJL094C | 1.0 | 1.7 | 1.3 | 2.0 | 1.4 | 1.3 | 1.7 | 0.8 | 5.4 | 2.9 | 1.1 | 1.2 | 2.6 | 1.0 | 1.0 | 1.8 | 2.1 | 2.0 | 0.84 |
| YLL061W | 3.6 | 2.8 | 9.2 | 0.4 | 0.8 | 0.8 | 0.9 | 1.4 | 3.7 | 5.5 | 1.7 | 0.7 | 0.9 | 1.3 | 1.4 | 1.0 | 0.6 | 0.7 | 0.33 |
| YPL274W | 0.8 | 4.2 | 3.8 | 0.8 | 0.6 | 1.1 | 0.7 | 0.9 | 4.1 | 3.6 | 2.0 | 0.5 | 0.6 | 0.9 | 1.5 | 1.1 | 0.7 | 0.6 | 0.41 |
| YBR241C | 0.9 | 9.3 | 2.6 | 0.9 | 0.9 | 1.5 | 1.4 | 0.7 | 29.8 | 2.2 | 1.4 | 1.4 | 1.4 | 0.9 | 1.6 | 0.7 | 1.0 | 1.2 | 1.25 |
| YDL206W | 0.9 | 1.7 | 1.8 | 1.4 | 1.1 | 5.7 | 2.0 | 1.1 | 1.6 | 1.5 | 0.6 | 1.0 | 1.3 | 1.0 | 1.1 | 3.1 | 1.0 | 1.1 | 0.35 |
| YDR040C | 0.7 | 1.4 | 3.7 | 0.8 | 1.0 | 1.7 | 0.7 | 0.8 | 2.0 | 1.2 | 0.8 | 0.9 | 1.8 | 0.9 | 0.8 | 1.4 | 1.4 | 1.6 | 1.25 |
| YFR045W | 1.1 | 1.6 | 1.1 | 1.1 | 1.0 | 1.0 | 0.6 | 1.0 | 2.2 | 1.0 | 1.4 | 1.0 | 1.1 | 1.0 | 1.1 | 0.6 | 0.9 | 0.8 | 0.84 |
| YIL170W | 1.1 | 1.0 | 2.5 | 2.2 | 0.9 | 8.8 | 0.5 | 1.2 | 5.7 | 3.2 | 1.7 | 0.7 | 1.9 | 1.1 | 2.3 | 1.8 | 1.6 | 1.5 | 0.48 |
| YKL192C | 1.1 | 1.0 | 3.7 | 1.1 | 1.1 | 1.8 | 1.1 | 0.9 | 3.5 | 1.6 | 1.1 | 1.2 | 1.7 | 1.5 | 1.2 | 2.1 | 1.0 | 0.8 | 1.57 |
| YKL209C | 1.0 | 1.0 | 1.3 | 0.8 | 1.2 | 3.3 | 0.9 | 1.4 | 2.4 | 1.6 | 0.8 | 0.9 | 1.4 | 1.1 | 1.0 | 1.1 | 1.1 | 1.0 | 0.30 |
| YKR106W | 1.3 | 0.8 | 2.1 | 1.4 | 1.2 | 1.6 | 0.9 | 1.3 | 10.5 | 7.4 | 1.8 | 0.8 | 1.4 | 1.7 | | 2.8 | 0.9 | 0.8 | 0.16 |
| YMR056C | 1.1 | 1.3 | 1.7 | 1.0 | 1.1 | 0.9 | 1.4 | 1.2 | 1.8 | 1.1 | 1.6 | 0.9 | 0.9 | 1.1 | 0.7 | 2.4 | 1.0 | 1.1 | 0.72 |
| YPL147W | 1.0 | 0.8 | 2.3 | 1.2 | 1.2 | 1.1 | 1.0 | 0.9 | 2.4 | 2.4 | 1.3 | 0.9 | 1.5 | 1.0 | 2.4 | 2.5 | 1.5 | 3.6 | 0.33 |

| | | | | | | | | | | | | | | | | | | | |
|---|---|---|---|---|---|---|---|---|---|---|---|---|---|---|---|---|---|---|---|
| YIL166C | 1.9 | 12.5 | 2.1 | 1.2 | 1.0 | 1.9 | | 1.3 | 2.5 | 2.9 | 1.8 | 0.9 | 1.5 | 1.1 | 1.3 | 0.7 | 1.2 | 0.9 | 0.26 |
| YMR058W | 0.3 | 5.1 | 2.1 | 0.3 | 0.8 | 2.6 | 1.8 | 0.8 | 0.2 | 1.4 | 0.8 | 0.8 | 0.4 | 0.3 | 0.5 | 0.4 | 0.6 | 0.5 | 1.66 |
| YNL142W | 0.7 | 2.4 | 2.5 | 0.8 | 1.3 | 0.9 | 1.1 | 0.8 | 0.9 | 0.6 | 0.9 | 1.2 | 0.9 | 0.8 | 0.3 | 1.0 | 1.0 | 0.9 | 0.44 |
| YDL210W | 1.2 | 2.2 | 2.7 | 1.1 | 0.9 | 1.3 | 0.8 | 1.1 | 1.2 | 1.4 | 0.6 | 0.8 | 1.2 | 1.1 | 1.9 | 0.9 | 1.1 | 0.9 | 0.20 |
| YCL025C | 1.1 | 4.6 | 2.5 | 1.7 | 0.9 | 0.9 | 0.7 | 0.7 | 0.3 | 0.3 | 1.0 | 1.1 | 0.5 | 0.7 | 0.8 | 0.6 | 0.6 | 0.6 | 1.98 |
| YJL212C | 0.8 | 3.2 | 3.1 | 0.5 | 1.2 | 1.1 | 0.7 | 0.7 | 1.1 | 1.3 | 0.9 | 0.4 | 1.0 | 0.8 | 0.3 | 0.5 | 0.6 | 0.5 | 0.59 |
| YBR132C | 0.9 | 1.9 | 2.4 | 1.8 | 0.8 | 1.2 | 1.1 | 0.8 | 0.8 | 1.1 | 1.9 | 1.0 | 1.4 | 1.1 | 1.5 | 1.3 | 1.1 | 1.3 | 0.43 |
| YBL030C | 1.0 | 0.9 | 4.5 | 0.9 | 0.7 | 1.1 | 0.9 | 0.8 | 0.4 | 0.9 | 1.1 | 0.9 | 0.5 | 0.8 | 1.8 | 1.0 | 1.0 | 1.1 | 3.12 |
| YCR024C-A | 0.8 | 0.9 | 3.2 | | 0.9 | 1.2 | 0.8 | 0.8 | 0.2 | 0.7 | 1.5 | 1.0 | 0.5 | 0.8 | 1.3 | 1.5 | 0.9 | 1.4 | 4.02 |
| YDR342C | 2.8 | 1.1 | 12.2 | 5.7 | 1.6 | 1.1 | 0.8 | 1.2 | 0.2 | 2.2 | 2.9 | 1.0 | 0.6 | 0.9 | 0.5 | 2.4 | 1.0 | 2.2 | 5.23 |
| YDR343C | 1.2 | 1.0 | 20.6 | 4.6 | 1.3 | 1.3 | 0.7 | 1.2 | 0.3 | 2.1 | 2.3 | 1.0 | 0.8 | 0.8 | 0.5 | 2.8 | 1.1 | 2.3 | 5.81 |
| YEL027W | 1.2 | 0.6 | 3.4 | 1.1 | 1.0 | 1.1 | 0.8 | 0.9 | 0.9 | 1.1 | 0.9 | 0.9 | 0.7 | 1.4 | 1.5 | 1.6 | 1.0 | 1.4 | 4.75 |
| YHR094C | 0.7 | 1.2 | 5.3 | 1.6 | 1.1 | 1.6 | 0.8 | 1.2 | 0.3 | 1.2 | 0.6 | 0.9 | 0.7 | 0.6 | 2.7 | 0.9 | 1.4 | 1.4 | 4.82 |
| YHR175W | 0.8 | 2.0 | 2.4 | 1.5 | 0.9 | 1.0 | 1.6 | 0.9 | 0.6 | 2.0 | 1.1 | 1.4 | 1.2 | 0.7 | 1.3 | 2.3 | 1.3 | 1.2 | 1.01 |
| YIL056W | 1.1 | 0.9 | 6.2 | 1.4 | 1.2 | 1.0 | 0.6 | 0.7 | 1.2 | 1.6 | 0.7 | 1.0 | 1.5 | 1.0 | 0.5 | 0.9 | 1.5 | 1.2 | 0.71 |
| YMR203W | 0.8 | 0.7 | 3.0 | 1.4 | 0.6 | 1.1 | 0.8 | 0.7 | 0.7 | 1.2 | 2.3 | 0.7 | 1.1 | 0.7 | 1.2 | 0.9 | 0.8 | 0.7 | 1.62 |
| YBL099W | 0.8 | 0.9 | 3.1 | 1.4 | 1.0 | 0.9 | 0.9 | 0.8 | 0.9 | 0.7 | 2.1 | 1.0 | 0.9 | 0.6 | 1.0 | 0.9 | 0.7 | 1.2 | 3.49 |
| YBR127C | 0.8 | 0.7 | 2.2 | 1.1 | 1.4 | 1.1 | 0.9 | 1.1 | 0.6 | 1.3 | 1.1 | 0.9 | 0.9 | 0.6 | 1.3 | 1.4 | 0.8 | 1.0 | 4.59 |
| YDR038C | 0.6 | 1.3 | 3.1 | 0.9 | 0.9 | 1.8 | 0.7 | 0.9 | 1.4 | 0.9 | 0.9 | 0.7 | 2.0 | 0.8 | 0.8 | 1.4 | 1.3 | 1.5 | 1.32 |
| YDR039C | 0.6 | 1.6 | 3.2 | 0.9 | 1.3 | 1.1 | 0.8 | 0.6 | 1.8 | 1.0 | 0.9 | 0.8 | 1.8 | 0.9 | 1.2 | 1.4 | 1.1 | 1.6 | 1.39 |
| YDR298C | 1.3 | 1.2 | 2.6 | 1.3 | 1.3 | 1.2 | 1.0 | 1.2 | 1.0 | 1.6 | 1.2 | 1.1 | 1.2 | 1.5 | 0.8 | 2.0 | 1.1 | 1.6 | 2.69 |
| YDR345C | 0.8 | 0.9 | 5.6 | 2.6 | 1.3 | 1.2 | 1.1 | 1.2 | 0.2 | 1.1 | 1.5 | 1.4 | 1.2 | 0.8 | 0.8 | 1.0 | 1.3 | 1.8 | 5.65 |
| YEL063C | 0.7 | 0.8 | 2.4 | 1.3 | 1.2 | 1.3 | 0.7 | 0.6 | 0.8 | 1.7 | 0.8 | 1.1 | 1.1 | 0.8 | 1.3 | 1.0 | 0.7 | 0.7 | 1.12 |
| YFL011W | 1.2 | 0.7 | 3.7 | 3.3 | 1.3 | 1.0 | 0.8 | 0.8 | 0.3 | 1.6 | 2.1 | 0.9 | 0.8 | 0.8 | 1.7 | 1.2 | 0.8 | 1.0 | 1.25 |
| YGR082W | 1.0 | 0.9 | 2.2 | 1.0 | 0.9 | 1.1 | 0.9 | 0.8 | 0.6 | 0.9 | 1.3 | 0.8 | 0.5 | 0.9 | 1.2 | 1.3 | 0.9 | 0.8 | 1.47 |
| YGR191W | 0.8 | 0.7 | 1.6 | 1.5 | 0.8 | 1.1 | 0.7 | 0.7 | 0.1 | 0.7 | 1.6 | 0.7 | 1.0 | 0.7 | 1.5 | 0.7 | 0.8 | 0.8 | 1.58 |
| YGR260W | 0.6 | 0.7 | 3.4 | 0.8 | 0.8 | 0.9 | 0.9 | 0.5 | 0.3 | 0.4 | 1.3 | 0.6 | 0.5 | 0.7 | 1.8 | 1.0 | 0.9 | 0.9 | 1.71 |
| YHR026W | 0.9 | 1.0 | 1.9 | 1.0 | 1.1 | 1.3 | 0.9 | 1.6 | 1.1 | 0.9 | 1.4 | 0.7 | 0.7 | 1.3 | 2.4 | 1.2 | 0.9 | 1.1 | 3.40 |
| YJR077C | 1.1 | 1.1 | 2.0 | 1.6 | 0.9 | 0.9 | 0.7 | 0.8 | 0.4 | 0.7 | 1.6 | 1.0 | 0.9 | 0.7 | 1.5 | 0.7 | 1.0 | 0.8 | 1.79 |
| YJR121W | 0.9 | 1.1 | 3.3 | 1.0 | 0.8 | 1.5 | 1.0 | 0.7 | 0.8 | 1.3 | 0.9 | 0.8 | 1.0 | 0.7 | 0.9 | 1.2 | 1.1 | 1.4 | 3.99 |
| YLR081W | 1.3 | 0.8 | 2.8 | 3.6 | 1.0 | 0.9 | 0.7 | 0.9 | 0.1 | 2.2 | 2.6 | 1.0 | 0.6 | 1.0 | 0.7 | 1.2 | 0.7 | 0.9 | 1.46 |

| | | | | | | | | | | | | | | | | | | | |
|---|---|---|---|---|---|---|---|---|---|---|---|---|---|---|---|---|---|---|---|
| YMR011W | 1.3 | 1.0 | 9.4 | 5.5 | 0.9 | 0.6 | 0.8 | 1.1 | 0.0 | 0.7 | 1.5 | 0.8 | 0.4 | 0.6 | 0.6 | 1.2 | 1.3 | 1.6 | 4.95 |
| YOL156W | 1.1 | 0.7 | 2.5 | 1.1 | 1.2 | 1.2 | 1.0 | 1.0 | 2.3 | 2.1 | 1.1 | 0.9 | 1.7 | 0.9 | 1.9 | 0.9 | 0.9 | 0.9 | 0.53 |
| YPL036W | 0.5 | 0.8 | 2.8 | 0.9 | 1.3 | 0.7 | 0.3 | 0.5 | 0.1 | 0.3 | 0.8 | 0.5 | 0.3 | 0.6 | 1.0 | 0.7 | 0.4 | 0.5 | 3.66 |
| YGR096W | 1.5 | 1.0 | 1.1 | 4.6 | 1.2 | 1.7 | 0.9 | 1.0 | 0.8 | 0.8 | 0.8 | 0.8 | 1.1 | 1.2 | 0.9 | 0.8 | 0.8 | 1.0 | 0.49 |
| YIL006W | 1.0 | 1.1 | 1.4 | 2.9 | 0.8 | 0.3 | 0.8 | 0.6 | 0.9 | 1.1 | 1.0 | 0.9 | 0.8 | 1.0 | 1.5 | 1.7 | 0.8 | 0.9 | 0.28 |
| YKL016C | 1.5 | 1.3 | 0.7 | 1.7 | 1.5 | 0.8 | 1.5 | 1.6 | 1.3 | 1.8 | 1.3 | 1.0 | 1.3 | 1.6 | 0.7 | 1.8 | 1.0 | 1.7 | 2.08 |
| YKR067W | 0.8 | 2.6 | 1.5 | 2.5 | 1.3 | 1.8 | 2.0 | 0.8 | 1.6 | 2.3 | 1.3 | 1.0 | 1.3 | 1.3 | 1.3 | 2.0 | 0.8 | 1.0 | 0.58 |
| YMR162C | 0.8 | 1.1 | 1.1 | 2.0 | 0.9 | 1.0 | 0.8 | 0.8 | 0.7 | 1.0 | 1.7 | 0.7 | 0.9 | 1.0 | 1.0 | 0.8 | 0.8 | 1.1 | 0.32 |
| YOR348C | 1.1 | 0.7 | 1.2 | 2.0 | 0.9 | 0.7 | 0.8 | 0.8 | 0.5 | 0.4 | 1.1 | 0.7 | 1.1 | 1.2 | 1.6 | 1.7 | 0.9 | 0.9 | 0.18 |
| YPR192W | 0.9 | 1.1 | 0.9 | 5.0 | 1.2 | 0.9 | 0.9 | 0.6 | 0.7 | 1.3 | 0.7 | 0.5 | 1.1 | 0.7 | 1.7 | 2.6 | 1.6 | 0.9 | 0.28 |
| YPR194C | 2.2 | 1.4 | 0.5 | 2.9 | 1.3 | 1.5 | 0.7 | 1.0 | 0.5 | 0.6 | 0.7 | 0.7 | 1.0 | 1.0 | 0.4 | 0.8 | 1.0 | 0.9 | 0.27 |
| YDR086C | 1.3 | 1.0 | 1.2 | 1.4 | 1.0 | 0.8 | 2.0 | 1.9 | 0.6 | 0.8 | 1.1 | 0.7 | 0.7 | 1.5 | 2.1 | 1.3 | 1.4 | 1.9 | 2.60 |
| YGR224W | 0.9 | 1.2 | 1.2 | 1.2 | 1.4 | 0.8 | 0.4 | 0.6 | 0.9 | 0.9 | 0.8 | 0.6 | 0.8 | 0.9 | 1.1 | 1.0 | 2.8 | 1.3 | 0.27 |
| YDR387C | 0.8 | 0.9 | 1.2 | 2.2 | 0.9 | 1.2 | 1.3 | 0.7 | 1.4 | 2.0 | 2.0 | 0.9 | 1.3 | 0.9 | 1.4 | 2.7 | 1.2 | 1.1 | 0.86 |
| YFL050C | 0.7 | 1.0 | 1.1 | 2.2 | 1.0 | 0.9 | 0.8 | 0.9 | 0.5 | 1.6 | 1.2 | 0.9 | 1.2 | 1.0 | 1.4 | 2.3 | 0.8 | 1.0 | 0.36 |
| YBR298C | 1.1 | 1.0 | 1.6 | 2.3 | 1.4 | 0.4 | 1.7 | 0.5 | 0.3 | 1.1 | 1.5 | 1.1 | 0.8 | 0.9 | 0.2 | 2.1 | 0.9 | 0.8 | 0.88 |
| YLR295C | 1.4 | 1.1 | 1.1 | 1.7 | 2.2 | 1.1 | 1.1 | 1.2 | 1.4 | 1.2 | 1.3 | 0.5 | 0.8 | 1.2 | 0.5 | 2.4 | 0.9 | 1.5 | 1.07 |
| YNR072W | 1.1 | 1.5 | 0.5 | 0.7 | 1.0 | 0.9 | 1.5 | 0.9 | 0.7 | 1.8 | 1.4 | 1.0 | 1.5 | 1.0 | 1.8 | 2.1 | 1.3 | 1.0 | 0.26 |
| YOR316C | 0.7 | 2.5 | 1.5 | 1.5 | 1.0 | 0.9 | 1.4 | 0.8 | 1.6 | 2.0 | 2.4 | 1.1 | 1.6 | 1.0 | 1.5 | 2.2 | 0.8 | 1.0 | 0.93 |
| YOR328W | 1.0 | 1.0 | | 1.0 | 1.0 | 2.9 | | | | | 1.1 | 1.8 | | 0.8 | 3.3 | 2.1 | | | 0.19 |
| YPL134C | 1.5 | 1.0 | 1.6 | 1.6 | 1.4 | 1.2 | 1.2 | 1.3 | 0.6 | 0.8 | 1.7 | 0.8 | 1.0 | 1.4 | 1.1 | 3.3 | 1.3 | 1.3 | 0.70 |

**Table 6** Stress protein genes

| yeast genes | The level of expressed mRNA in the presence of chemical substance/ The level of expressed mRNA in the absence of chemical substance | | | | | | | | | | | | | | | | | | |
|---|---|---|---|---|---|---|---|---|---|---|---|---|---|---|---|---|---|---|---|
| | (1) | (2) | (3) | (4) | (5) | (6) | (7) | (8) | (9) | (10) | (11) | (12) | (13) | (14) | (15) | (16) | (17) | (18) | Intensity |
| YBR072W | 18.5 | 17.4 | 18.4 | 10.5 | 1.2 | 32.2 | 6.6 | 2.1 | 29.4 | 43.4 | 6.3 | 16.3 | 38.5 | 5.8 | 4.9 | 23.1 | 1.8 | 2.2 | 0.59 |
| YFL014W | 3.4 | 5.1 | 5.7 | 11.0 | 1.0 | 9.3 | 5.5 | 3.4 | 13.1 | 5.8 | 5.0 | 4.3 | 15.2 | 7.3 | 6.3 | 14.2 | 1.5 | 8.8 | 2.14 |
| YLL060C | 12.5 | 4.2 | 2.3 | 2.6 | 0.9 | 1.4 | 2.0 | 5.8 | 13.0 | 23.2 | 14.1 | 1.1 | 1.8 | 10.6 | 1.5 | 3.6 | 1.9 | 2.3 | 0.57 |
| YAL005C | 0.6 | 1.6 | 7.0 | 0.9 | 1.2 | 1.6 | 1.2 | 0.9 | 3.9 | 1.8 | 1.0 | 1.2 | 5.0 | 0.4 | 1.6 | 0.7 | 0.8 | 0.5 | 5.23 |
| YBL075C | 1.3 | 4.3 | 3.1 | 1.6 | 1.1 | 0.8 | 1.3 | 0.8 | 60.3 | 6.7 | 1.6 | 1.1 | 8.0 | 1.1 | 2.6 | 2.2 | 1.2 | 0.7 | 1.17 |
| YBR169C | 1.0 | 2.1 | 3.9 | 1.9 | 0.8 | 4.9 | 2.5 | 0.9 | 7.0 | 4.3 | 2.2 | 1.7 | 7.2 | 1.1 | 1.6 | 2.4 | 1.3 | 1.6 | 1.10 |
| YCL035C | 2.0 | 2.3 | 1.5 | 1.7 | 1.5 | 5.1 | 2.7 | 1.4 | 1.9 | 2.3 | 2.1 | 1.5 | 2.8 | 2.5 | 1.9 | 5.5 | 2.6 | 4.2 | 1.74 |
| YCR060W | | 0.7 | 0.9 | | 0.8 | 3.3 | 2.4 | 1.2 | 0.7 | 1.1 | 1.2 | 1.7 | 8.6 | 1.7 | | 3.3 | 1.2 | 1.7 | 1.04 |
| YDR155C | 1.1 | 1.5 | 3.2 | 1.8 | 0.8 | 1.5 | 1.2 | 1.4 | 1.3 | 1.3 | 3.8 | 1.4 | 3.3 | 1.0 | 2.5 | 1.6 | 1.2 | 2.5 | 4.99 |
| YDR258C | 1.6 | 2.3 | 2.8 | 1.4 | 1.3 | 2.4 | 3.9 | 1.9 | 4.9 | 13.3 | 1.7 | 1.8 | 5.2 | 1.5 | 0.6 | 1.0 | 1.3 | 1.3 | 0.87 |
| YER103W | 0.7 | 2.1 | 5.2 | 2.1 | 1.1 | 1.7 | 1.0 | 0.9 | 20.3 | 9.0 | 1.8 | 1.9 | 9.0 | 0.9 | 3.2 | 1.2 | 1.4 | 0.6 | 2.10 |
| YKL210W | 0.5 | 1.7 | 2.7 | 0.7 | 0.8 | 1.4 | 1.0 | 0.9 | 3.5 | 2.9 | 1.4 | 1.3 | 2.9 | 0.8 | 1.0 | 0.9 | 0.7 | 1.1 | 2.29 |
| YLL024C | 0.5 | 1.6 | 5.3 | 0.8 | 0.7 | 1.0 | 1.1 | 0.7 | 2.8 | 2.4 | 1.6 | 1.0 | 4.8 | 0.4 | 1.3 | 0.5 | 0.5 | 0.6 | 5.12 |
| YLL026W | 1.8 | 2.9 | 7.7 | 1.7 | 0.9 | 1.9 | 2.9 | 1.4 | 11.4 | 6.6 | 1.7 | 1.9 | 8.1 | 0.8 | 0.9 | 1.6 | 1.7 | 2.5 | 2.56 |
| YNL160W | 2.1 | 3.7 | 10.6 | 2.0 | 1.3 | 5.8 | 2.4 | 1.2 | 3.5 | 2.7 | 5.4 | 1.4 | 6.0 | 1.3 | 0.7 | 4.0 | 1.0 | 1.0 | 1.77 |
| YNL241C | 1.3 | 2.5 | 4.3 | 1.0 | 0.8 | 0.9 | 3.2 | 0.9 | 3.4 | 7.4 | 3.0 | 2.0 | 4.9 | 1.1 | 7.0 | 2.8 | 1.0 | 1.0 | 0.68 |
| YOR027W | 0.7 | 2.4 | 4.4 | 0.8 | 1.0 | 1.6 | 1.3 | 0.9 | 3.5 | 4.0 | 1.3 | 1.3 | 5.1 | 0.8 | 1.4 | 0.6 | 0.9 | 0.8 | 1.52 |
| YPL240C | 0.7 | 1.4 | 2.4 | 0.9 | 1.3 | 1.2 | 1.4 | 1.2 | 3.5 | 2.6 | 1.0 | 0.8 | 2.9 | 0.7 | 1.2 | 0.7 | 0.8 | 1.0 | 4.83 |
| YDR293C | 0.9 | 0.9 | 1.2 | 1.4 | 0.6 | 0.9 | 0.9 | 0.6 | 1.2 | 1.2 | 1.1 | 0.9 | 2.3 | 0.8 | 1.7 | 1.6 | 1.4 | 1.3 | 1.12 |
| YDR436W | 0.9 | 0.9 | 1.1 | 1.4 | 0.7 | 1.1 | 1.2 | 0.9 | 3.5 | 1.1 | 2.0 | 0.9 | 2.7 | 1.2 | 0.8 | 1.4 | 0.9 | 1.2 | 0.53 |
| YDR519W | 1.3 | 3.2 | 1.7 | 1.6 | 0.7 | 1.3 | 1.9 | 1.4 | 0.5 | 1.2 | 1.2 | 1.7 | 2.5 | 1.3 | 2.0 | 1.5 | 1.8 | 2.4 | 2.01 |
| YEL030W | 0.7 | 0.9 | 1.9 | 1.1 | 0.4 | 0.6 | 0.9 | 0.7 | 2.3 | 2.5 | 1.7 | 0.9 | 2.7 | 0.7 | 0.8 | 0.8 | 0.9 | 0.6 | 1.28 |
| YER125W | 0.6 | 1.1 | 1.5 | 0.9 | 1.4 | 0.7 | 0.8 | 0.7 | 0.6 | 1.2 | 1.0 | 0.7 | 1.9 | 0.7 | 0.8 | 1.0 | 1.0 | 1.0 | 1.48 |
| YFL016C | 0.8 | 1.0 | 1.6 | 0.9 | 1.3 | 1.3 | 1.4 | 0.7 | 6.4 | 2.9 | 1.2 | 1.0 | 2.8 | 0.8 | 0.8 | 0.6 | 1.2 | 0.9 | 1.25 |
| YFR052W | 1.1 | 1.6 | 1.3 | 1.5 | 1.4 | 3.2 | 2.2 | 1.5 | 3.7 | 3.0 | 0.9 | 1.8 | 3.8 | 3.3 | 1.4 | 2.7 | 1.2 | 1.9 | 1.62 |
| YHR057C | 1.1 | 1.2 | 0.7 | 0.9 | 1.3 | 1.3 | 1.3 | 1.0 | 2.6 | 1.2 | 1.6 | 1.1 | 1.9 | 1.9 | 1.2 | 2.2 | 1.6 | 1.3 | 1.07 |
| YIR037W | 1.4 | 2.2 | 2.0 | 2.3 | 0.6 | 2.7 | 1.8 | 1.3 | 6.2 | 3.8 | 3.0 | 1.3 | 2.4 | 2.3 | 1.0 | 4.0 | 1.7 | 2.9 | 1.97 |
| YIR038C | 1.3 | 3.5 | 4.6 | 2.0 | 0.7 | 2.5 | 2.0 | 1.3 | 4.6 | 4.5 | 3.4 | 1.1 | 2.5 | 2.5 | 1.2 | 6.0 | 2.8 | 2.0 | 1.11 |
| YJR045C | 0.5 | 1.9 | 3.9 | | 0.5 | 1.2 | 1.0 | 0.8 | 3.2 | 3.0 | 1.4 | 1.0 | 2.3 | 0.5 | 2.3 | 0.8 | 0.7 | 0.9 | 3.78 |

| | | | | | | | | | | | | | | | | | | | |
|---|---|---|---|---|---|---|---|---|---|---|---|---|---|---|---|---|---|---|---|
| YLL039C | 1.1 | 1.0 | 1.7 | 1.2 | 0.9 | 1.5 | 1.1 | 1.1 | 3.4 | 1.5 | 1.1 | 1.0 | 2.2 | 1.4 | 3.0 | 1.1 | 1.6 | 2.1 | 3.60 |
| YLR259C | 0.7 | 1.0 | 3.1 | 1.6 | 1.1 | 0.9 | 1.8 | 0.8 | 1.8 | 2.6 | 3.0 | 0.7 | 2.5 | 0.8 | 2.1 | 1.3 | 0.8 | 1.0 | 2.09 |
| YML070W | 0.9 | 1.7 | 2.5 | 1.9 | 1.4 | 2.2 | 3.1 | 1.3 | 4.5 | 3.9 | 1.5 | 1.3 | 3.3 | 1.3 | 0.9 | 2.6 | 1.2 | 1.3 | 1.03 |
| YPL106C | 0.6 | 1.7 | 2.4 | 1.1 | 1.2 | 1.6 | 1.1 | 1.2 | 4.9 | 1.8 | 1.3 | 1.1 | 3.1 | 0.9 | 1.1 | 0.5 | 0.5 | 0.5 | 3.34 |
| YPR026W | 0.9 | 1.2 | 5.0 | 1.3 | 0.8 | | 1.3 | 1.0 | 0.9 | 3.1 | 1.6 | 1.2 | 2.3 | 1.5 | 3.1 | 2.5 | 0.9 | 1.3 | 0.26 |
| YDR077W | 1.2 | 1.0 | 3.5 | 2.1 | 0.8 | 1.0 | 0.7 | 0.8 | 0.7 | 0.7 | 6.8 | 0.8 | 1.0 | 0.3 | 6.1 | 1.0 | 1.4 | 1.1 | 4.77 |
| YKL163W | 1.1 | 1.4 | 3.4 | 1.9 | 0.6 | 1.1 | 0.9 | 0.8 | 0.7 | 1.6 | 1.3 | 0.9 | 1.5 | 0.5 | 16.8 | 3.0 | 0.8 | 1.0 | 2.01 |
| YLR109W | 0.8 | 2.9 | 6.0 | 0.9 | 1.3 | 2.1 | 2.6 | 0.9 | 2.4 | 2.7 | 1.5 | 1.6 | 3.2 | 2.3 | 3.9 | 1.8 | 1.1 | 1.2 | 3.86 |
| YOR208W | 1.3 | 1.0 | 1.2 | 2.9 | 1.4 | 1.8 | 1.1 | 1.0 | 1.5 | 2.0 | 1.2 | 1.2 | 1.5 | 1.3 | 4.4 | 1.9 | 1.6 | 1.6 | 0.56 |
| YDR098C | 0.8 | 1.4 | 1.0 | 0.8 | 1.5 | 1.1 | 1.5 | 1.2 | 3.7 | 1.5 | 1.4 | 1.3 | 1.2 | 1.5 | 2.2 | 0.8 | 1.0 | 1.2 | 1.59 |
| YHR030C | 2.1 | 0.8 | 0.6 | 2.9 | 1.8 | 0.9 | 1.8 | 1.5 | 0.6 | 2.5 | 1.5 | 0.7 | 1.0 | 1.1 | 3.4 | 1.4 | 2.0 | 1.8 | 1.05 |
| YJL159W | 1.0 | 1.3 | 2.8 | 1.1 | 1.0 | 1.8 | 0.9 | 0.8 | 0.5 | 1.2 | 1.2 | 1.0 | 1.7 | 0.6 | 5.3 | 1.0 | 1.4 | 0.9 | 2.45 |
| YMR173W | 1.5 | 3.6 | 3.0 | 0.9 | 1.1 | 5.0 | 2.4 | 2.3 | 2.7 | 8.2 | 3.2 | 1.4 | 2.0 | 2.3 | 2.5 | 1.2 | 1.9 | 1.8 | 1.37 |
| YCR021C | 5.2 | 1.4 | 4.2 | 8.6 | 0.9 | 2.2 | 0.9 | 0.8 | 2.2 | 1.0 | 0.6 | 0.5 | 0.9 | 1.6 | 0.5 | 0.6 | 0.7 | 0.8 | 1.36 |
| YDL022W | 0.9 | 2.6 | 6.9 | 1.5 | 0.7 | 2.1 | 1.5 | 0.8 | 1.2 | 1.8 | 2.8 | 1.2 | 1.5 | 0.8 | 0.9 | 1.1 | 1.0 | 1.4 | 1.79 |
| YDR033W | 1.1 | 1.3 | 4.6 | 1.5 | 1.1 | 6.7 | 0.8 | 1.0 | 0.3 | 1.0 | 0.7 | 1.0 | 0.7 | 1.0 | 0.8 | 1.0 | 0.7 | 1.0 | 5.78 |
| YFL020C | 1.0 | 1.1 | 1.9 | 1.2 | 1.1 | 2.0 | 1.4 | 0.7 | 1.3 | 1.4 | 1.2 | 1.3 | 1.4 | 1.0 | 1.4 | 0.9 | 1.5 | 0.9 | 0.70 |
| YGL073W | 1.0 | 2.1 | 0.9 | 1.0 | 1.7 | 1.9 | 1.3 | 0.8 | 1.9 | 2.4 | 0.8 | 0.8 | 1.5 | 1.2 | 0.9 | 1.1 | 2.5 | 1.2 | 0.66 |
| YIL033C | 0.8 | 1.3 | 3.3 | 1.3 | 0.7 | 2.0 | 1.1 | 0.7 | 2.4 | 1.3 | 1.8 | 1.1 | 2.5 | 0.7 | 1.0 | 2.0 | 1.0 | 1.2 | 1.18 |
| YMR021C | 1.0 | 0.7 | 0.9 | 1.1 | 1.6 | 2.2 | 2.5 | 1.3 | 0.6 | 1.3 | 0.9 | 0.8 | 1.4 | 1.3 | 1.0 | 2.0 | 3.3 | 3.4 | 1.14 |
| YBR126C | 0.8 | 1.9 | 5.6 | 1.2 | 0.7 | 2.9 | 2.3 | 0.6 | 1.7 | | 1.1 | 1.3 | 2.1 | 0.7 | 1.0 | 1.7 | 1.5 | 1.3 | 1.96 |
| YBR067C | 1.6 | 2.5 | 2.8 | 0.9 | 1.1 | 1.1 | 1.1 | 1.1 | 1.7 | 1.0 | 3.1 | 1.0 | 0.9 | 0.7 | 3.3 | 2.5 | 0.5 | 0.4 | 1.76 |
| YDR513W | 2.2 | 2.5 | 2.3 | 2.6 | 0.9 | 2.1 | 1.6 | 1.6 | 4.6 | 3.1 | 2.0 | 0.9 | 1.8 | 2.0 | 1.3 | 3.8 | 1.3 | 3.2 | 3.10 |
| YGR088W | 1.3 | 1.2 | 7.7 | 2.4 | 1.0 | 1.3 | 0.8 | 0.8 | 1.5 | 3.2 | 3.9 | 1.1 | 1.2 | 1.3 | 0.7 | 5.6 | 0.9 | 2.0 | 0.75 |
| YHR104W | 1.0 | 4.1 | 15.7 | 1.9 | 1.1 | 1.2 | 1.5 | 1.1 | 4.8 | 2.3 | 2.6 | 1.0 | 1.2 | 1.5 | 1.6 | 1.3 | 1.9 | 1.9 | 1.57 |
| YKL161C | 4.2 | 0.8 | 0.9 | 2.2 | 1.4 | 2.0 | 2.0 | 1.9 | 1.4 | 1.7 | 3.1 | 0.9 | 1.3 | 1.4 | 1.3 | 1.1 | 1.4 | 1.9 | 0.45 |
| YPL223C | 4.8 | 20.0 | 1.2 | 5.1 | 0.9 | 1.5 | 1.1 | 1.2 | 29.4 | 16.5 | 1.8 | 0.9 | 2.3 | 2.5 | 1.5 | 19.0 | 1.0 | 1.3 | 0.31 |
| YBR054W | 1.9 | 1.8 | 5.4 | 1.7 | 0.8 | 1.1 | 0.6 | 2.1 | 0.4 | 1.2 | 1.8 | 0.7 | 1.5 | 1.0 | 0.6 | 0.4 | 0.7 | 0.7 | 3.02 |
| YAL015C | 1.2 | 1.1 | 1.7 | 1.9 | 1.1 | 0.7 | 1.2 | 1.1 | 4.0 | 2.7 | 1.4 | 1.0 | 1.2 | 1.6 | 0.7 | 2.0 | 1.1 | 1.2 | 0.61 |
| YDL025C | 1.7 | 5.0 | 3.5 | 1.5 | 0.6 | 0.6 | 1.0 | 1.1 | 3.6 | 4.5 | 1.5 | 0.9 | 1.2 | 1.7 | 1.0 | 1.7 | 1.0 | 1.4 | 0.56 |
| YML007W | 0.9 | 0.8 | 0.9 | 1.3 | 1.0 | 0.5 | 1.1 | 1.9 | 2.1 | 2.5 | 1.3 | 1.1 | 1.7 | 1.2 | 1.0 | 1.4 | 1.0 | 1.4 | 1.54 |
| YER042W | 3.0 | 2.2 | 3.1 | 1.0 | 1.1 | 1.6 | 1.1 | 1.7 | 6.8 | 2.1 | 1.8 | 1.1 | 1.4 | 5.2 | 1.0 | 1.9 | 0.7 | 1.1 | 1.80 |

| | | | | | | | | | | | | | | | | | | | |
|---|---|---|---|---|---|---|---|---|---|---|---|---|---|---|---|---|---|---|---|
| YOL064C | 1.4 | 2.1 | 1.1 | 0.6 | 0.9 | 1.4 | 1.2 | 1.0 | 8.7 | 2.7 | 1.0 | 0.9 | 2.0 | 0.9 | 1.0 | 1.7 | 1.2 | 1.3 | 1.32 |
| YGL181W | 0.9 | 0.9 | 1.2 | 1.1 | 1.1 | 1.9 | 1.3 | 1.2 | 2.7 | 2.3 | 1.2 | 1.0 | 2.8 | 1.0 | 1.5 | 1.3 | 1.0 | 1.3 | 0.98 |
| YJL128C | 0.8 | 1.4 | 0.4 | 0.8 | 1.2 | 1.3 | 1.8 | 0.9 | 2.0 | 1.7 | 0.6 | 1.0 | 1.7 | 0.8 | 1.2 | 0.9 | 1.0 | 1.0 | 0.42 |
| YJL165C | 1.0 | 0.8 | 1.3 | 1.6 | 1.1 | 0.5 | 0.8 | 0.7 | 2.8 | 1.0 | 1.7 | 0.9 | 1.5 | 0.9 | 1.0 | 1.5 | 1.3 | 1.8 | 0.87 |
| YJR090C | 0.8 | 1.0 | 0.6 | 0.8 | 1.2 | 1.2 | 0.8 | 0.8 | 1.9 | 1.2 | 0.4 | 0.8 | 2.2 | 1.0 | 1.6 | 0.8 | 1.1 | 1.0 | 0.60 |
| YMR186W | 0.6 | 1.4 | 2.0 | 0.9 | 1.3 | 1.3 | 1.2 | 0.8 | 2.6 | 1.3 | 1.1 | 1.0 | 2.4 | 0.6 | 1.3 | 0.6 | 0.7 | 1.1 | 6.64 |
| YNL064C | 0.9 | 1.8 | 1.8 | 1.1 | 0.9 | 0.8 | 0.6 | 1.4 | 2.4 | 1.8 | 0.9 | 0.8 | 1.1 | 0.8 | 0.7 | 0.3 | 0.7 | 0.7 | 3.79 |
| YOR008C | 0.7 | 0.8 | 1.4 | 1.1 | 1.4 | 1.1 | 0.8 | 0.8 | 1.9 | 1.0 | 1.0 | 0.7 | 0.9 | 0.9 | 0.9 | 1.2 | 0.8 | 1.3 | 1.75 |
| YPL194W | 0.9 | 1.4 | 0.4 | 1.1 | 1.3 | 1.1 | 1.5 | 1.3 | 9.6 | 3.1 | 0.9 | 0.9 | 1.4 | 1.1 | 1.4 | 0.9 | 1.0 | 0.8 | 0.22 |
| YBL093C | 1.0 | 2.0 | 0.4 | 1.4 | 1.0 | 0.8 | 1.3 | 1.6 | 0.7 | 1.1 | 1.3 | 1.1 | 2.1 | 1.0 | 0.5 | 0.9 | 1.4 | 1.3 | 1.47 |
| YKR053C | 0.9 | 2.0 | 1.0 | 1.2 | 1.0 | 1.1 | 1.7 | 0.9 | 0.8 | 1.8 | 0.5 | 0.7 | 0.7 | 1.3 | 0.3 | 0.9 | 2.8 | 2.4 | 0.35 |
| YJL158C | 0.9 | 0.6 | 2.8 | 1.5 | 1.4 | 0.5 | 0.4 | 0.7 | 0.2 | 0.5 | 1.9 | 1.1 | 0.4 | 0.5 | 1.7 | 0.6 | 1.4 | 1.4 | 3.48 |
| YER057C | 1.2 | 2.0 | 3.0 | 1.8 | 0.8 | 1.6 | 1.4 | 1.0 | 0.6 | 1.4 | 1.8 | 1.5 | 1.0 | 1.4 | 1.7 | 2.8 | 1.1 | 1.8 | 2.03 |
| YHL046C | 1.1 | 1.7 | 2.1 | 1.3 | 0.9 | 0.8 | 1.3 | 1.0 | 1.0 | 2.1 | 0.4 | 1.1 | 1.4 | 1.4 | 1.0 | 1.4 | 0.9 | 0.9 | 0.55 |
| YIL011W | 0.7 | 0.5 | 3.9 | 0.6 | 1.1 | 0.7 | 0.6 | 0.7 | 0.0 | 0.5 | 2.5 | 0.6 | 0.5 | 0.5 | 0.9 | 1.0 | 0.8 | 0.8 | 0.57 |
| YKL164C | 0.6 | 1.1 | 2.5 | 0.9 | 1.1 | 1.4 | 0.8 | 0.8 | 0.4 | 0.7 | 1.0 | 0.8 | 0.9 | 0.3 | 1.2 | 1.1 | 0.8 | 0.8 | 2.75 |
| YNR076W | 1.3 | 1.3 | 2.5 | 1.0 | 1.2 | 1.2 | 1.2 | 1.0 | 1.3 | 2.5 | 1.4 | 0.7 | 0.9 | 0.8 | 1.1 | 0.8 | 1.5 | 0.8 | 0.42 |
| YOL161C | 1.2 | 1.1 | 2.4 | 1.4 | 0.9 | 1.0 | 0.9 | 0.8 | 0.7 | 2.0 | 1.5 | 1.3 | 1.2 | 0.9 | 1.1 | 1.6 | 1.1 | 1.0 | 0.89 |
| YOR009W | 0.9 | 0.8 | 3.2 | 0.7 | 0.8 | 0.9 | 0.6 | 0.7 | 0.4 | 0.5 | 1.4 | 0.4 | 0.4 | 0.7 | 0.6 | 0.8 | 0.9 | 0.8 | 0.36 |
| YOR010C | 0.7 | 0.7 | 3.2 | 0.6 | 1.2 | 0.5 | 0.4 | 0.5 | 0.6 | 0.7 | 1.2 | 0.5 | 0.5 | 0.4 | 0.6 | 1.1 | 1.4 | 0.6 | 0.61 |
| YPL059W | 1.2 | 0.9 | 2.6 | 1.3 | 1.2 | 1.3 | 0.7 | 1.4 | 2.0 | 1.3 | 1.6 | 0.8 | 0.7 | 1.3 | 1.0 | 2.1 | 1.8 | 1.3 | 1.35 |
| YBR044C | 0.8 | 1.4 | 0.7 | 1.9 | 1.4 | 1.0 | 1.5 | 1.1 | 0.8 | 1.2 | 1.1 | 0.8 | 1.2 | 0.9 | 0.9 | 1.4 | 0.9 | 1.3 | 0.57 |
| YEL039C | 1.1 | 0.7 | 1.3 | 5.1 | 0.9 | 1.5 | 0.6 | 1.1 | 1.2 | 1.2 | 1.7 | 0.6 | 0.5 | 1.4 | 0.4 | 3.5 | 0.7 | 1.1 | 1.59 |
| YGL115W | 0.9 | 0.6 | 1.2 | 2.0 | 1.2 | 1.2 | 1.4 | 1.0 | 1.2 | 1.2 | 1.0 | 0.9 | 0.7 | 1.2 | 0.7 | 1.7 | 0.8 | 1.2 | 2.46 |
| YLR006C | 0.7 | 0.7 | 0.9 | 6.2 | 1.6 | 0.9 | 1.1 | 0.9 | 1.1 | 1.1 | 1.5 | 0.7 | 0.8 | 1.0 | 0.9 | 0.8 | 1.6 | 1.0 | 0.40 |
| YPR005C | 1.3 | 0.8 | 1.5 | 3.8 | 1.0 | 0.8 | 1.2 | 1.2 | 2.8 | 2.0 | 0.4 | 0.6 | 0.7 | 1.3 | 2.7 | 0.8 | 1.0 | 1.1 | 0.24 |
| YHL028W | 0.7 | 0.8 | 1.4 | 0.5 | 1.1 | 1.8 | 2.1 | 0.4 | 0.2 | 0.4 | 0.7 | 1.5 | 2.1 | 1.3 | 0.6 | 2.6 | 1.6 | 2.5 | 0.62 |
| YCR083W | 1.1 | 2.8 | 1.5 | 1.8 | 1.6 | 2.4 | 1.8 | 1.5 | 1.8 | 1.8 | 1.8 | 1.1 | 1.8 | 2.4 | 1.0 | 3.1 | 1.3 | 1.3 | 0.98 |
| YPL140C | 0.9 | 1.3 | 0.9 | 1.0 | 0.8 | 0.3 | 1.4 | 1.1 | 0.9 | 0.9 | 0.8 | 0.8 | 0.9 | 1.1 | 0.7 | 1.8 | 0.9 | 1.4 | 0.46 |

**Table 7 Metabolism protein genes**

The level of expressed mRNA in the presence of chemical substance/ The level of expressed mRNA in the absence of chemical substance

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| YCR107W | 12.0 | 2.6 | 1.8 | 1.6 | 0.7 | 1.5 | 1.5 | 15.6 | 196.6 | 34.0 | 23.0 | 0.9 | 4.0 | 8.3 | 4.8 | 3.1 | 1.3 | 1.1 | 0.59 |
| YDL243C | 14.4 | 2.7 | 2.4 | 1.0 | 1.1 | 2.2 | 1.6 | 11.8 | 64.2 | 29.6 | 19.6 | 1.1 | 4.1 | 12.0 | 4.2 | 3.0 | 1.8 | 1.5 | 0.76 |
| YFL014W | 3.4 | 7.2 | 5.7 | 11.0 | 1.0 | 9.3 | 5.5 | 3.4 | 13.1 | 5.8 | 5.0 | 4.3 | 15.2 | 7.3 | 6.3 | 14.2 | 1.5 | 8.8 | 2.14 |
| YFL056C | 19.0 | 2.3 | 2.3 | 1.5 | 0.9 | 0.6 | 1.4 | 18.5 | 162.3 | 31.3 | 68.3 | 1.0 | 4.7 | 7.8 | 5.0 | 3.4 | 1.0 | 1.1 | 0.55 |
| YFL057C | 20.9 | 5.8 | 1.5 | 1.8 | 0.9 | 1.2 | 1.8 | 18.0 | 51.8 | 46.1 | 27.7 | 1.0 | 4.1 | 23.4 | 3.1 | 3.9 | 1.6 | 1.3 | 0.71 |
| YJR155W | 10.6 | 3.7 | 1.4 | 2.5 | 0.7 | 1.4 | 1.7 | 10.6 | 38.2 | 18.8 | 15.4 | 1.0 | 5.7 | 9.4 | 2.6 | 5.6 | 1.3 | 1.4 | 0.64 |
| YNL331C | 8.6 | 3.6 | 1.3 | 1.0 | 1.6 | 1.8 | 1.9 | 13.1 | 42.6 | 36.3 | 21.8 | 0.9 | 3.1 | 7.5 | 2.3 | 4.0 | 1.7 | 1.3 | 0.58 |
| YNL332W | 1.1 | 1.5 | 1.1 | 2.0 | 1.6 | 1.1 | 2.1 | 1.5 | 3.3 | 2.1 | 2.6 | 1.2 | 2.1 | 3.0 | 5.4 | 2.2 | 1.0 | 1.0 | 0.24 |
| YOL165C | 10.1 | 4.5 | 1.8 | 0.9 | 0.9 | 1.7 | 1.4 | 17.8 | 46.9 | 23.3 | 17.6 | 0.8 | 3.7 | 9.1 | 3.0 | 1.8 | 1.6 | 1.0 | 0.69 |
| YPR167C | 5.3 | 5.8 | 1.9 | 0.8 | 1.4 | 2.6 | 1.2 | 5.5 | 76.6 | 9.0 | 1.9 | 1.3 | 1.9 | 4.2 | 0.9 | 0.9 | 1.7 | 1.4 | 0.54 |
| YBR256C | 3.0 | 1.4 | 0.6 | 1.3 | 1.6 | 2.0 | 2.1 | 2.3 | 18.1 | 6.8 | 3.2 | 1.7 | 3.5 | 5.6 | 1.6 | 3.0 | 2.6 | 3.3 | 1.97 |
| YBR296C | 7.0 | 11.2 | 2.2 | 2.5 | 1.4 | 1.4 | 0.2 | 1.1 | 0.4 | 1.8 | 1.5 | 1.6 | 2.0 | 2.3 | 1.7 | 0.3 | 3.8 | 4.0 | 0.54 |
| YDL021W | 4.8 | 1.7 | 2.4 | 3.7 | 1.7 | 6.5 | 5.9 | 2.7 | 2.5 | 7.4 | 4.7 | 2.4 | 5.3 | 3.7 | 0.7 | 7.3 | 1.9 | 3.2 | 0.47 |
| YFL061W | 1.5 | 1.4 | 2.1 | 3.0 | 1.0 | 0.5 | 1.2 | 1.2 | 3.2 | 9.9 | 1.1 | 0.9 | 1.2 | 6.0 | 2.1 | 1.0 | 1.2 | 0.9 | 0.31 |
| YGR043C | 2.5 | 4.7 | 3.2 | 7.9 | 0.9 | 16.3 | 6.5 | 2.6 | 10.9 | 8.4 | 3.6 | 3.3 | 6.9 | 4.1 | 3.0 | 13.7 | 1.6 | 4.8 | 0.66 |
| YHR112C | 1.8 | 3.1 | 0.7 | 1.4 | 1.3 | 2.9 | 3.4 | 1.7 | 10.5 | 4.4 | 1.5 | 1.9 | 3.9 | 2.4 | 2.0 | 2.7 | 1.6 | 1.8 | 0.55 |
| YIR030C | 1.4 | 1.0 | 0.9 | 1.3 | 0.9 | 0.5 | 1.2 | 1.4 | 5.4 | 2.1 | 1.4 | 0.9 | 1.3 | 2.9 | 0.7 | 0.8 | 0.9 | 1.0 | 0.42 |
| YJR010W | 9.1 | 7.3 | 4.9 | 0.8 | 1.1 | 2.5 | 1.4 | 3.1 | 30.0 | 11.1 | 2.9 | 1.4 | 3.2 | 5.4 | 1.7 | 1.7 | 1.2 | 1.0 | 0.56 |
| YKL001C | 6.8 | 21.4 | 5.6 | 1.8 | 0.8 | 0.9 | 1.1 | 3.4 | 10.3 | 3.4 | 2.6 | 2.1 | 1.6 | 6.2 | 1.1 | 1.8 | 2.7 | 1.7 | 0.91 |
| YKR097W | 1.3 | 2.4 | 1.3 | 3.3 | 1.1 | 1.7 | 3.7 | 2.5 | 1.9 | 3.3 | 0.8 | 1.8 | 17.5 | 2.1 | 2.5 | 2.2 | 1.1 | 1.5 | 0.16 |
| YLR303W | 7.3 | 9.8 | 12.1 | 1.1 | 1.1 | 3.0 | 3.6 | 5.3 | 18.6 | 5.6 | 9.8 | 4.3 | 9.9 | 3.8 | 5.8 | 3.4 | 1.5 | 1.4 | 1.42 |
| YNL274C | 1.4 | 18.7 | 2.6 | 1.9 | 0.7 | 4.2 | 2.0 | 2.3 | 6.3 | 5.3 | 4.0 | 1.1 | 2.3 | 3.3 | 2.5 | 6.4 | 1.4 | 2.6 | 0.85 |
| YOL151W | 6.7 | 3.8 | 9.1 | 1.3 | 1.3 | 8.4 | 4.4 | 6.7 | 22.8 | 17.0 | 9.0 | 4.6 | 16.9 | 4.0 | 2.8 | 3.7 | 4.0 | 5.6 | 1.12 |
| YOR226C | 1.3 | 2.2 | 1.7 | 1.1 | 1.3 | 1.2 | 1.2 | 1.2 | 1.3 | 5.9 | 1.5 | 0.6 | 0.9 | 2.7 | 1.0 | 0.7 | 1.0 | 0.7 | 0.58 |
| YJL153C | 1.3 | 1.7 | 1.7 | 17.6 | 0.7 | 0.8 | 2.3 | 1.3 | 3.9 | 2.5 | 0.7 | 10.2 | 38.0 | 1.4 | 37.0 | 1.0 | 6.5 | 1.8 | 0.27 |
| YOR153W | 1.6 | 0.9 | 5.1 | 1.1 | 1.0 | 7.4 | 2.6 | 0.5 | 3.2 | 1.2 | 1.0 | 4.0 | 11.8 | 0.3 | 3.6 | 1.6 | 2.5 | 3.1 | 1.91 |
| YPL088W | 3.3 | 1.3 | 0.6 | 2.5 | 1.5 | 7.1 | 3.7 | 1.9 | 0.6 | 2.8 | 1.1 | 3.6 | 4.8 | 1.6 | 3.2 | 4.3 | 9.1 | 8.3 | 0.69 |
| YDL124W | 2.1 | 8.5 | 5.7 | 1.7 | 0.7 | 3.4 | 2.9 | 2.1 | 3.3 | 5.3 | 3.4 | 2.4 | 6.0 | 1.6 | 3.4 | 3.6 | 2.2 | 2.6 | 2.43 |

| | | | | | | | | | | | | | | | | | | | |
|---|---|---|---|---|---|---|---|---|---|---|---|---|---|---|---|---|---|---|---|
| YDL174C | 0.9 | 1.8 | 0.9 | 1.3 | 0.5 | 1.7 | 3.6 | 0.8 | 0.5 | 1.7 | 0.9 | 2.8 | 5.9 | 1.1 | 0.6 | 5.1 | 2.2 | 4.0 | 0.63 |
| YGL156W | 1.3 | 2.5 | 1.8 | 2.2 | 1.4 | 2.1 | 2.1 | 1.4 | 5.6 | 2.4 | 2.1 | 2.6 | 6.0 | 1.6 | 3.0 | 4.1 | 1.4 | 1.9 | 0.29 |
| YGR157W | 1.0 | 0.9 | 5.2 | 0.8 | 1.2 | 1.0 | 0.8 | 0.7 | 3.3 | 2.0 | 1.0 | 1.8 | 4.8 | 0.7 | 4.6 | 1.0 | 0.8 | 1.3 | 2.10 |
| YHR043C | 1.3 | 1.8 | 1.2 | 1.7 | 0.9 | 2.2 | 2.9 | 3.0 | 1.2 | 1.0 | 1.6 | 2.2 | 2.7 | 1.3 | 2.1 | 2.2 | 1.4 | 1.7 | 1.38 |
| YHR044C | 1.3 | 1.3 | 1.7 | 1.5 | 1.2 | 3.3 | 2.6 | 2.0 | 1.1 | 1.1 | 1.7 | 2.3 | 4.3 | 1.3 | 1.5 | 2.4 | 1.3 | 1.4 | 1.12 |
| YJR073C | 1.1 | 2.4 | 2.7 | 2.7 | 0.6 | 2.2 | 2.9 | 1.0 | 6.8 | 2.8 | 2.8 | 4.1 | 10.0 | 2.3 | 28.9 | 3.7 | 2.9 | 2.6 | 0.99 |
| YOR303W | 1.1 | 2.2 | 0.5 | 1.0 | 1.2 | 1.8 | 1.2 | 1.6 | 0.9 | 5.2 | 0.9 | 1.9 | 3.3 | 1.6 | 4.1 | 1.2 | 1.2 | 0.8 | 1.47 |
| YBR294W | 6.3 | 12.0 | 0.9 | 2.6 | 1.4 | 1.2 | 1.1 | 1.0 | 5.2 | 5.2 | 1.2 | 1.0 | 3.1 | 1.3 | 1.0 | 0.9 | 1.0 | 0.9 | 0.18 |
| YCL043C | 0.6 | 3.7 | 2.5 | 0.7 | 0.9 | 1.3 | 1.0 | 0.8 | 1.6 | 1.2 | 1.7 | 1.5 | 4.1 | 0.6 | 5.7 | 0.8 | 0.9 | 1.0 | 2.37 |
| YCL050C | 1.0 | 1.1 | 1.9 | 0.7 | 1.4 | 1.2 | 1.2 | 1.2 | 3.0 | 2.9 | 1.3 | 1.2 | 2.8 | 1.2 | 0.8 | 0.7 | 1.0 | 1.0 | 2.20 |
| YCR012W | 1.1 | 1.5 | 5.1 | 1.2 | 1.1 | 2.5 | 1.4 | 0.8 | 1.4 | 1.5 | 1.8 | 1.2 | 4.1 | 0.9 | 1.4 | 1.4 | 0.8 | 1.3 | 4.48 |
| YDR158W | 1.1 | 0.5 | 1.8 | 0.8 | 1.3 | 1.0 | 0.8 | 1.2 | 1.1 | 1.7 | 1.6 | 1.0 | 2.6 | 0.9 | 1.0 | 0.6 | 1.0 | 0.9 | 3.54 |
| YDR204W | 0.9 | 2.3 | 3.0 | 1.4 | 1.0 | 2.2 | 2.2 | 1.1 | 5.7 | 3.7 | 2.2 | 1.2 | 2.9 | 1.1 | 0.6 | 2.8 | 1.1 | 1.7 | 0.62 |
| YDR313C | 1.2 | 1.2 | 1.2 | 1.4 | 1.3 | 1.4 | 1.5 | 1.2 | 4.7 | 2.6 | 1.5 | 1.2 | 2.3 | 1.3 | 0.8 | 2.9 | 1.1 | 1.3 | 0.84 |
| YDR368W | 2.0 | 2.2 | 2.2 | 1.8 | 1.0 | 3.0 | 2.1 | 1.7 | 3.8 | 3.8 | 1.8 | 1.4 | 2.6 | 2.0 | 1.2 | 2.3 | 1.1 | 2.0 | 1.70 |
| YER091C | 3.6 | 3.9 | 30.7 | 1.3 | 0.9 | 2.3 | 2.1 | 0.9 | 5.8 | 1.4 | 3.2 | 2.0 | 5.4 | 1.2 | 10.2 | 2.7 | 1.2 | 1.0 | 1.23 |
| YFR053C | 1.7 | 1.4 | 3.0 | 2.2 | 0.9 | 3.2 | 2.1 | 2.8 | 0.6 | 1.1 | 1.5 | 1.7 | 3.0 | 0.6 | 0.6 | 3.0 | 1.4 | 2.2 | 4.17 |
| YGL062W | 0.6 | 1.3 | 1.1 | 0.6 | 0.9 | 1.0 | 1.3 | 0.8 | 1.6 | 2.5 | 0.7 | 1.6 | 4.5 | 1.2 | 2.7 | 1.5 | 1.1 | 0.9 | 0.77 |
| YGL157W | 0.7 | 0.9 | 1.8 | 0.9 | 1.5 | 5.4 | 3.3 | 1.4 | 0.6 | 1.8 | 1.4 | 1.5 | 4.8 | 1.2 | 5.1 | 1.7 | 1.8 | 2.3 | 1.69 |
| YGL184C | 4.6 | 15.9 | 57.4 | 1.3 | 1.1 | 1.0 | 1.2 | 1.9 | 37.9 | 66.8 | 1.7 | 2.6 | 7.4 | 1.4 | 0.4 | 1.5 | 1.0 | 0.9 | 0.16 |
| YGR032W | 1.0 | 1.4 | 1.0 | 1.3 | 0.7 | 1.9 | 0.8 | 0.6 | 0.5 | 0.7 | 1.5 | 1.5 | 3.9 | 1.1 | 5.4 | 1.5 | 3.2 | 2.8 | 1.18 |
| YGR124W | 0.5 | 0.6 | 1.7 | 0.7 | 1.2 | 1.1 | 0.7 | 1.0 | 2.2 | 1.3 | 1.1 | 1.1 | 2.3 | 0.5 | 1.5 | 0.3 | 0.7 | 0.6 | 3.56 |
| YGR192C | 1.4 | 1.0 | 3.8 | 1.0 | 1.1 | 1.7 | 1.7 | 1.1 | 0.9 | 1.3 | 2.3 | 2.2 | 3.4 | 1.0 | 1.9 | 1.1 | 1.1 | 1.3 | 7.49 |
| YGR244C | 1.0 | 1.2 | 1.6 | 1.6 | 0.8 | 2.6 | 3.9 | 1.8 | 1.4 | 1.1 | 1.8 | 1.9 | 2.6 | 1.2 | 2.0 | 2.6 | 1.9 | 3.1 | 1.12 |
| YGR254W | 1.2 | 1.3 | 3.8 | 1.3 | 1.2 | 1.9 | 1.5 | 1.4 | 0.8 | 1.2 | 1.3 | 1.7 | 3.1 | 0.6 | 2.4 | 1.4 | 1.3 | 1.2 | 7.01 |
| YHR018C | 1.0 | 1.0 | 1.5 | 1.4 | 1.7 | 1.1 | 0.8 | 1.0 | 1.4 | 4.4 | 1.4 | 1.4 | 3.3 | 1.0 | 4.4 | 1.0 | 1.9 | 1.2 | 1.21 |
| YIL160C | 0.8 | 2.4 | 2.3 | 3.5 | 1.0 | 1.6 | 1.1 | 1.2 | 3.2 | 3.8 | 2.4 | 1.0 | 2.3 | 1.5 | 5.6 | 8.8 | 2.4 | 3.0 | 0.27 |
| YIR017C | 3.5 | 8.6 | 2.5 | 1.9 | 0.8 | 1.9 | 1.5 | 1.2 | 5.2 | 3.4 | 4.2 | 1.2 | 3.4 | 1.9 | 2.3 | 3.0 | 1.5 | 1.3 | 0.36 |
| YJL052W | 1.5 | 0.9 | 4.0 | 1.8 | 0.7 | 2.4 | 2.1 | 1.5 | 1.6 | 2.0 | 4.3 | 2.4 | 5.6 | 1.3 | 2.3 | 2.3 | 1.2 | 1.9 | 6.19 |
| YJR009C | 1.1 | 1.7 | 5.6 | 1.0 | 1.2 | 1.6 | 1.6 | 1.1 | 1.1 | 1.3 | 1.1 | 1.5 | 2.7 | 1.0 | 2.1 | 1.5 | 1.1 | 1.3 | 5.86 |
| YJR130C | 1.1 | 0.6 | 0.8 | 0.9 | 1.4 | 2.0 | 1.3 | 1.3 | 5.2 | 3.8 | 1.7 | 1.0 | 2.9 | 1.1 | 0.9 | 1.5 | 1.1 | 1.3 | 0.62 |

| | | | | | | | | | | | | | | | | | | | |
|---|---|---|---|---|---|---|---|---|---|---|---|---|---|---|---|---|---|---|---|
| YJR149W | 1.0 | 1.8 | 1.4 | 2.3 | 0.8 | 1.0 | 1.7 | 1.0 | 1.4 | 1.0 | 0.7 | 1.0 | 3.5 | 1.4 | 1.4 | 2.4 | 1.1 | 1.2 | 0.34 |
| YKL218C | 2.1 | 1.0 | 2.2 | 1.8 | 1.2 | 3.6 | 1.6 | 1.7 | 3.4 | 2.7 | 1.7 | 1.2 | 5.1 | 1.4 | 0.8 | 3.1 | 2.5 | 2.6 | 0.60 |
| YLR027C | 1.0 | 1.5 | 2.3 | 0.8 | 0.9 | 2.0 | 1.4 | 0.7 | 1.6 | 2.7 | 1.6 | 1.3 | 2.5 | 1.0 | 1.4 | 0.9 | 1.3 | 1.3 | 1.39 |
| YLR133W | 0.9 | 1.6 | 1.4 | 0.7 | 1.0 | 2.2 | 1.3 | 0.7 | 6.1 | 2.3 | 0.6 | 1.5 | 3.3 | 1.1 | 1.9 | 1.0 | 1.1 | 1.3 | 0.36 |
| YLR155C | 1.4 | 1.1 | 2.4 | 1.8 | 1.7 | 1.6 | 2.0 | 1.0 | 3.0 | 1.8 | 2.0 | 1.1 | 3.3 | 1.1 | 1.2 | 2.2 | 1.4 | 2.2 | 1.63 |
| YLR158C | 1.4 | 1.2 | 2.1 | 1.7 | 1.7 | 1.5 | 2.0 | 1.1 | 2.7 | s | 2.4 | 1.1 | 3.2 | 1.2 | 1.1 | 2.1 | 1.4 | 2.0 | 1.57 |
| YLR195C | 0.8 | 0.9 | 1.0 | 0.8 | 1.0 | 0.8 | 1.1 | 1.0 | 0.6 | 0.7 | 0.8 | 1.3 | 2.3 | 0.7 | 0.9 | 0.8 | 0.9 | 1.0 | 1.55 |
| YLR345W | 1.4 | 1.4 | 1.4 | 1.2 | 1.2 | 1.6 | 1.2 | 1.4 | 4.5 | 4.0 | 2.5 | 1.6 | 3.5 | 1.5 | 1.0 | 1.2 | 1.2 | 1.4 | 2.65 |
| YML004C | 0.8 | 1.6 | 2.5 | 1.0 | 0.7 | 1.7 | 2.1 | 1.9 | 3.9 | 2.2 | 2.2 | 1.1 | 2.4 | 1.4 | 2.1 | 4.5 | 1.5 | 2.4 | 2.41 |
| YML131W | 11.2 | 4.3 | 7.7 | 1.9 | 0.7 | 2.0 | 3.0 | 6.1 | 11.1 | 17.7 | 14.4 | 1.1 | 2.4 | 2.7 | 2.2 | 1.6 | 2.5 | 3.0 | 1.15 |
| YNL071W | 0.9 | 0.9 | 1.7 | 1.5 | 0.6 | 1.3 | 1.4 | 0.9 | 1.1 | 1.2 | 1.6 | 1.3 | 2.5 | 0.8 | 1.1 | 1.1 | 1.0 | 1.2 | 1.61 |
| YNL241C | 1.3 | 2.5 | 4.3 | 1.0 | 0.8 | 0.9 | 3.2 | 0.9 | 3.4 | 7.4 | 3.0 | 2.0 | 4.9 | 1.1 | 7.0 | 2.8 | 1.0 | 1.0 | 0.68 |
| YOR120W | 1.8 | 5.4 | 2.8 | 3.2 | 1.4 | 3.4 | 2.7 | 2.0 | 3.3 | 3.0 | 3.0 | 1.2 | 3.6 | 2.4 | 1.5 | 3.9 | 1.3 | 2.6 | 1.06 |
| YAL023C | 0.4 | 1.5 | 1.0 | 0.7 | 1.0 | 0.6 | 0.8 | 0.6 | 0.3 | 0.4 | 1.7 | 1.2 | 2.3 | 0.4 | 2.5 | 0.4 | 1.0 | 0.7 | 1.49 |
| YAL060W | 1.1 | 1.8 | 3.2 | 2.7 | 1.2 | 4.2 | 3.3 | 0.9 | 0.6 | 2.5 | 2.4 | 0.8 | 2.2 | 0.9 | 0.9 | 3.2 | 1.1 | 1.8 | 2.39 |
| YAL062W | 0.8 | 1.5 | 1.4 | 1.1 | 1.0 | 1.2 | 1.1 | 0.8 | 1.4 | 0.7 | 1.2 | 1.0 | 2.1 | 0.8 | 1.1 | 1.3 | 1.0 | 0.8 | 0.86 |
| YBR006W | 1.3 | 1.6 | 2.6 | 1.5 | 1.3 | 3.1 | 3.3 | 1.0 | 4.5 | 2.7 | 1.9 | 1.1 | 2.2 | 1.1 | 1.1 | 3.0 | 1.2 | 1.8 | 0.62 |
| YBR056W | 1.4 | 1.7 | 1.4 | 2.3 | 1.4 | 2.3 | 5.5 | 1.4 | 3.0 | 3.3 | 1.8 | 1.4 | 3.0 | 1.0 | 0.7 | 2.8 | 2.4 | 3.4 | 1.13 |
| YBR149W | 1.1 | 2.0 | 2.7 | 1.7 | 1.4 | 2.8 | 3.1 | 1.5 | 1.7 | 1.9 | 1.9 | 1.4 | 2.2 | 1.2 | 0.7 | 2.7 | 2.1 | 3.4 | 2.72 |
| YBR284W | 0.9 | 1.5 | 2.8 | 4.8 | 1.3 | 1.9 | 1.0 | 0.9 | 6.5 | 3.2 | 1.4 | 1.2 | 2.8 | 1.6 | 0.8 | 0.8 | 1.1 | 1.0 | 0.25 |
| YCL018W | 1.2 | 2.7 | 2.4 | 1.4 | 0.8 | 2.3 | 2.3 | 1.3 | 2.1 | 4.3 | 3.7 | 1.6 | 3.2 | 1.0 | 0.6 | 2.3 | 1.3 | 1.0 | 0.99 |
| YCL040W | 0.9 | 7.1 | 10.1 | 2.0 | 0.5 | 3.5 | 2.9 | 0.7 | 0.9 | 3.0 | 8.2 | 2.3 | 5.6 | 0.7 | 3.4 | 3.1 | 1.4 | 1.7 | 1.98 |
| YDL010W | 1.7 | 0.8 | 0.6 | 0.7 | 1.2 | 1.1 | 1.9 | 1.1 | 2.2 | 1.3 | 1.3 | 0.9 | 2.0 | 2.2 | 1.4 | 1.4 | 1.5 | 2.4 | 0.93 |
| YDL024C | 1.6 | 1.5 | 1.2 | 2.2 | 1.0 | 1.7 | 0.9 | 1.7 | 4.2 | 3.4 | 1.2 | 1.7 | 2.6 | 1.9 | 2.1 | 2.8 | 0.9 | 1.1 | 0.40 |
| YDL095W | 0.5 | 1.3 | 1.0 | 0.8 | 0.8 | 1.0 | 0.8 | 0.7 | 1.2 | 0.7 | 1.0 | 1.1 | 2.2 | 0.5 | 2.0 | 0.5 | 0.7 | 0.9 | 1.57 |
| YDL245C | 1.0 | 2.4 | 1.6 | 2.2 | 1.3 | 1.1 | 1.2 | 1.2 | 0.4 | 2.8 | 1.5 | 1.0 | 2.0 | 1.2 | 1.8 | 1.5 | 1.0 | 1.1 | 0.28 |
| YDL246C | 1.2 | 1.5 | 1.3 | 1.1 | 1.1 | 1.4 | 2.1 | 2.4 | 4.4 | 3.6 | 2.6 | 1.2 | 2.9 | 1.7 | 2.6 | 1.7 | 1.8 | 1.8 | 0.43 |
| YDR001C | 0.8 | 2.2 | 2.6 | 1.0 | 1.1 | 2.2 | 1.5 | 0.9 | 2.2 | 3.0 | 0.7 | 1.1 | 2.8 | 1.0 | 4.4 | 1.9 | 1.1 | 1.6 | 0.75 |
| YDR058C | 1.2 | 2.2 | 0.8 | 2.7 | 0.7 | 1.0 | 1.4 | 1.9 | 2.4 | 1.7 | 1.7 | 1.2 | 2.5 | 1.8 | 1.2 | 1.9 | 1.1 | 1.6 | 0.56 |
| YDR072C | 1.3 | 1.3 | 1.7 | 1.2 | 0.9 | 1.5 | 0.9 | 0.7 | 0.6 | 0.7 | 0.7 | 0.9 | 1.9 | 0.6 | 0.4 | 0.7 | 1.9 | 1.9 | 2.81 |
| YDR127W | 0.8 | 0.9 | 1.5 | 0.7 | 1.2 | 0.8 | 0.9 | 0.7 | 0.6 | 1.3 | 1.3 | 0.8 | 1.7 | 1.0 | 1.3 | 0.6 | 0.9 | 0.7 | 0.78 |

| | | | | | | | | | | | | | | | | | | | |
|---|---|---|---|---|---|---|---|---|---|---|---|---|---|---|---|---|---|---|---|
| YDR261C | 1.2 | 1.2 | 1.9 | 0.7 | 1.3 | 1.4 | 0.8 | 0.9 | 1.7 | 1.0 | 1.2 | 1.0 | 1.9 | 0.9 | 2.3 | 0.6 | 0.9 | 0.8 | 0.68 |
| YDR272W | 1.0 | 4.0 | 0.8 | 1.8 | 0.7 | 2.3 | 1.7 | 2.1 | 2.5 | 1.9 | 2.1 | 1.2 | 2.4 | 1.4 | 1.1 | 2.1 | 1.4 | 2.3 | 1.51 |
| YDR497C | 0.7 | 0.5 | 0.6 | 1.3 | 0.7 | 0.6 | 0.5 | 0.6 | 0.4 | 0.8 | 0.7 | 0.9 | 2.7 | 0.6 | 10.1 | | 1.6 | 1.8 | 1.45 |
| YDR516C | 1.2 | 1.5 | 7.8 | 2.1 | 1.3 | 1.8 | 3.9 | 1.3 | 1.8 | 5.2 | 2.8 | 1.1 | 2.5 | 0.7 | 0.4 | 1.7 | 1.1 | 1.2 | 1.66 |
| YER053C | 1.6 | 1.8 | 1.9 | 1.7 | 0.6 | 2.8 | 2.8 | 1.3 | 1.3 | 3.9 | 2.4 | 1.7 | 4.1 | 1.3 | 1.1 | 2.8 | 1.2 | 2.3 | 1.83 |
| YER096W | 1.1 | 2.3 | 2.0 | 2.0 | 1.3 | 1.2 | 0.9 | 1.0 | 7.5 | 3.5 | 1.1 | 0.9 | 2.3 | 3.1 | 1.4 | 2.0 | 1.0 | 1.2 | 0.22 |
| YER178W | 0.8 | 0.9 | 3.6 | 1.0 | 0.7 | 2.5 | 1.6 | 0.8 | 1.7 | 1.3 | 2.5 | 1.3 | 2.4 | 0.9 | 1.8 | 1.0 | 1.1 | 0.9 | 2.18 |
| YFL030W | 1.6 | 6.0 | 3.1 | 2.0 | 1.1 | 1.3 | 0.6 | 1.6 | 24.5 | 4.9 | 5.4 | 1.2 | 2.2 | 0.8 | 1.1 | 0.6 | 1.0 | 0.8 | 0.50 |
| YFL031W | 0.5 | 1.3 | 2.1 | 0.7 | 1.0 | 0.3 | 0.9 | 0.7 | 1.0 | 0.7 | 1.2 | 1.0 | 2.6 | 0.4 | 2.7 | 0.9 | 1.2 | 1.1 | 3.16 |
| YFR047C | 1.3 | 4.0 | 2.7 | 2.6 | 1.2 | 2.0 | 2.0 | 1.0 | 3.0 | 1.5 | 2.3 | 1.7 | 2.7 | 1.9 | 0.6 | 3.6 | 1.5 | 1.6 | 1.57 |
| YGL248W | 1.3 | 1.3 | 0.9 | 0.7 | 1.3 | 2.7 | 1.6 | 0.8 | 0.2 | 2.9 | 1.0 | 0.8 | 2.0 | 1.3 | 1.8 | 2.9 | 1.1 | 1.2 | 0.22 |
| YGR037C | 1.0 | 1.2 | 2.2 | 0.8 | 1.0 | 1.1 | 1.4 | 1.4 | 1.7 | 1.4 | 1.4 | 1.5 | 2.7 | 1.5 | 1.5 | 2.6 | 1.4 | 2.0 | 3.40 |
| YGR055W | 2.5 | 5.7 | 12.0 | 0.7 | 1.3 | 1.1 | 0.6 | 1.1 | 5.0 | 2.4 | 1.5 | 0.9 | 1.9 | 1.2 | 2.1 | 0.6 | 0.7 | 0.7 | 1.42 |
| YGR194C | 0.9 | 1.0 | 1.3 | 2.1 | 0.8 | 1.9 | 2.2 | 1.2 | 2.4 | 2.1 | 0.7 | 0.8 | 2.2 | 1.0 | 0.6 | 2.6 | 1.1 | 2.0 | 0.63 |
| YGR256W | 1.2 | 1.5 | 2.3 | 0.8 | 0.9 | 6.2 | 1.4 | 2.2 | 3.4 | 2.5 | 2.7 | 1.5 | 3.9 | 1.1 | 2.7 | 5.3 | 1.1 | 0.8 | 0.94 |
| YHR111W | 1.4 | 1.3 | 0.8 | 1.9 | 1.5 | 1.3 | 1.6 | 1.3 | 4.5 | 4.2 | 1.9 | 0.9 | 2.2 | 1.7 | 1.1 | 1.3 | 1.1 | 1.2 | 0.44 |
| YHR174W | 1.1 | 1.4 | 3.3 | 1.2 | 1.3 | 1.5 | 1.6 | 1.2 | 1.0 | 1.5 | 1.4 | 1.5 | 3.6 | 0.6 | 2.0 | 1.1 | 1.0 | 1.2 | 7.34 |
| YHR176W | 1.8 | 2.0 | 1.1 | 1.4 | 1.0 | 2.3 | 1.4 | 1.3 | 6.3 | 6.4 | 1.2 | 1.2 | 1.9 | 1.3 | 1.5 | 1.8 | 1.4 | 1.6 | 0.27 |
| YIL045W | 1.7 | 1.4 | 1.9 | 2.2 | 1.3 | 1.7 | 1.6 | 1.1 | 2.1 | 3.2 | 1.2 | 1.5 | 2.0 | 1.6 | 0.6 | 2.9 | 1.1 | 1.8 | 0.37 |
| YIL107C | 1.5 | 0.8 | 1.1 | 1.5 | 0.8 | 1.0 | 1.6 | 1.3 | 3.5 | 3.5 | 0.9 | 1.1 | 1.9 | 1.6 | 1.0 | 2.7 | 1.2 | 2.0 | 0.58 |
| YIL155C | 1.0 | 0.7 | 1.4 | 3.7 | 1.3 | 1.4 | 2.2 | 1.0 | 2.5 | 2.9 | 1.3 | 1.4 | 2.0 | 1.3 | 1.4 | 3.8 | 1.1 | 1.4 | 0.51 |
| YIR034C | 1.2 | 1.1 | 2.5 | 0.6 | 1.1 | 1.3 | 0.8 | 1.7 | 3.2 | 3.5 | 3.0 | 1.4 | 2.6 | 1.2 | 1.9 | 1.3 | 1.0 | 1.0 | 0.92 |
| YIR036C | 1.0 | 1.7 | 2.6 | 1.8 | 0.9 | 0.7 | 1.0 | 1.0 | 3.3 | 1.9 | 2.8 | 1.2 | 1.9 | 1.2 | 1.1 | 4.0 | 1.5 | 1.3 | 0.64 |
| YJL031C | 1.6 | 1.2 | 0.5 | 1.3 | 1.7 | 1.6 | 2.3 | 1.8 | 4.7 | 3.6 | 1.9 | 1.3 | 2.6 | 3.0 | 2.0 | 1.9 | 1.2 | 1.9 | 1.02 |
| YJL068C | 1.2 | 2.7 | 3.4 | 1.6 | 0.7 | 1.1 | 1.2 | 1.1 | 6.5 | 3.1 | 3.2 | 1.7 | 2.7 | 1.5 | 0.5 | 2.6 | 1.4 | 1.5 | 0.95 |
| YJL099W | 1.2 | 1.2 | 0.3 | 1.3 | 1.9 | 2.0 | 1.8 | 1.0 | 3.1 | 3.1 | 0.9 | 1.0 | 2.4 | 1.8 | 1.4 | 1.7 | 1.2 | 1.6 | 0.55 |
| YJL172W | 0.8 | 2.1 | 1.8 | 1.5 | 0.9 | 3.8 | 1.0 | 0.7 | 0.7 | 1.9 | 1.4 | 1.1 | 3.1 | 0.9 | 1.2 | 0.5 | 1.8 | 1.8 | 0.62 |
| YJL219W | 1.2 | 2.5 | 3.1 | 1.6 | 1.2 | 1.5 | 0.9 | 1.5 | 4.0 | 2.3 | 2.3 | 1.1 | 4.0 | 1.1 | 2.6 | 1.4 | 1.6 | 1.2 | 0.91 |
| YJR137C | 2.7 | 2.9 | 4.6 | 0.8 | 0.6 | 1.5 | 0.8 | 1.6 | 9.0 | 4.8 | 2.0 | 1.4 | 2.1 | 1.2 | 1.0 | 1.3 | 0.9 | 0.8 | 0.46 |
| YKL035W | 1.0 | 0.9 | 4.8 | 1.2 | 0.8 | 1.2 | 0.6 | 1.0 | 0.8 | 1.2 | 2.1 | 1.0 | 2.0 | 0.6 | 1.0 | 1.5 | 1.0 | 1.9 | 2.54 |
| YKL091C | 1.2 | 1.3 | 1.4 | 2.6 | 1.0 | 2.2 | 1.9 | 1.3 | 2.3 | 3.2 | 1.7 | 1.3 | 2.0 | 1.5 | 1.1 | 4.6 | 1.4 | 2.0 | 0.83 |

| | | | | | | | | | | | | | | | | | | | |
|---|---|---|---|---|---|---|---|---|---|---|---|---|---|---|---|---|---|---|---|
| YKL104C | 1.0 | 1.0 | 1.6 | 1.2 | 1.4 | 0.9 | 2.3 | 1.1 | 1.4 | 1.8 | 2.2 | 1.6 | 2.2 | 0.8 | 3.3 | 0.6 | 1.0 | 1.1 | 1.25 |
| YKL152C | 1.3 | 1.3 | 1.9 | 0.9 | 1.3 | 1.6 | 1.5 | 1.0 | 0.9 | 1.5 | 1.7 | 1.5 | 2.7 | 1.0 | 1.8 | 2.0 | 1.1 | 1.7 | 3.28 |
| YKL213C | 0.9 | 0.8 | 1.0 | 1.3 | 0.7 | 1.0 | 1.0 | 1.1 | 4.8 | 2.1 | 1.3 | 0.9 | 2.1 | 0.9 | 1.1 | 1.5 | 1.4 | 1.6 | 0.77 |
| YKL215C | 0.6 | 1.1 | 1.3 | 0.8 | 1.0 | 2.1 | 1.1 | 1.1 | 1.3 | 1.9 | 0.5 | 1.0 | 2.6 | 1.0 | 0.9 | 0.8 | 0.8 | 0.9 | 0.38 |
| YLL058W | 1.0 | 3.8 | 1.4 | 0.9 | 0.8 | 1.4 | 1.5 | 0.9 | 3.5 | 3.6 | 2.0 | 1.0 | 2.3 | 0.8 | 1.9 | 1.1 | 1.4 | 1.3 | 0.39 |
| YLR299W | 1.2 | 1.9 | 1.5 | 1.3 | 0.6 | 0.9 | 0.8 | 1.0 | 4.9 | 2.3 | 1.7 | 1.0 | 2.9 | 1.3 | 1.5 | 0.9 | 1.3 | 1.4 | 0.57 |
| YLR348C | 1.1 | 4.5 | 1.2 | 1.2 | 0.9 | 1.9 | 0.9 | 0.9 | 2.1 | 1.3 | 1.3 | 0.9 | 1.9 | 1.0 | 2.4 | 1.1 | 1.1 | 1.0 | 0.64 |
| YML054C | 1.5 | 1.8 | 1.3 | 3.4 | 1.3 | 1.8 | 1.2 | 1.5 | 4.1 | 1.8 | 1.4 | 1.8 | 2.8 | 2.1 | 1.1 | 7.8 | 1.1 | 1.6 | 0.25 |
| YML070W | 0.9 | 1.7 | 2.5 | 1.9 | 1.4 | 2.2 | 3.1 | 1.3 | 4.5 | 3.9 | 1.5 | 1.3 | 3.3 | 1.3 | 0.9 | 2.6 | 1.2 | 1.3 | 1.03 |
| YML100W | 0.8 | 2.7 | 10.6 | 1.4 | 0.9 | 2.8 | 2.2 | 0.7 | 1.7 | 1.4 | 3.2 | 1.2 | 3.8 | 1.2 | 1.8 | 2.2 | 1.0 | 1.5 | 0.88 |
| YMR008C | 0.7 | 0.9 | 1.4 | 1.0 | 1.1 | 1.7 | 0.9 | 0.5 | 0.3 | 0.6 | 1.3 | 1.1 | 3.7 | 0.7 | 2.0 | 1.2 | 3.0 | 1.9 | 1.59 |
| YMR020W | 1.3 | 1.0 | 0.9 | 1.7 | 1.4 | 1.6 | 2.7 | 1.2 | 2.3 | 2.9 | 1.5 | 1.1 | 3.0 | 1.1 | 1.8 | 2.8 | 2.3 | 2.2 | 0.81 |
| YMR105C | 1.9 | 3.0 | 5.0 | 4.2 | 0.9 | 2.8 | 2.8 | 1.1 | 0.6 | 2.9 | 3.0 | 1.7 | 3.3 | 1.0 | 0.9 | 2.0 | 1.6 | 2.6 | 1.21 |
| YMR271C | 1.6 | 1.6 | 1.3 | 2.0 | 1.1 | 4.2 | 3.4 | 1.8 | 8.0 | 4.4 | 1.7 | 1.3 | 3.6 | 1.8 | 0.6 | 5.7 | 1.3 | 2.2 | 0.70 |
| YNL012W | 1.3 | 1.3 | 1.8 | 0.8 | 1.3 | 1.7 | 2.0 | 1.8 | 3.3 | 3.2 | 1.6 | 1.3 | 2.9 | 1.0 | 1.6 | 1.2 | 1.3 | 1.5 | 0.24 |
| YNL045W | 0.8 | 1.3 | 2.0 | 1.3 | 1.1 | 2.2 | 1.8 | 0.7 | 1.3 | 1.1 | 2.1 | 0.9 | 2.6 | 0.8 | 0.8 | 2.4 | 1.1 | 1.1 | 0.80 |
| YNL104C | 1.1 | 1.3 | 3.0 | 0.9 | 0.8 | 0.8 | 0.6 | 0.9 | 1.1 | 2.0 | 2.1 | 1.1 | 2.1 | 0.9 | 2.5 | 1.5 | 0.9 | 0.9 | 1.69 |
| YNL231C | 1.5 | 0.8 | 0.8 | 0.5 | 2.1 | 1.8 | 2.5 | 1.5 | 2.2 | 1.3 | 1.3 | 1.8 | 2.9 | 0.9 | 0.9 | 0.7 | 3.1 | 4.0 | 2.13 |
| YNR019W | 1.0 | 1.3 | 1.6 | 1.2 | 0.8 | 1.3 | 0.8 | 0.7 | 1.8 | 1.9 | 0.7 | 1.0 | 2.1 | 1.0 | 1.6 | 0.8 | 2.4 | 2.0 | 0.37 |
| YNR033W | 0.6 | 0.8 | 0.8 | 1.0 | 1.3 | 1.6 | 1.2 | 1.1 | 3.6 | 4.2 | 0.4 | 1.1 | 3.8 | 0.9 | 1.3 | 2.2 | 0.9 | 1.1 | 0.72 |
| YNR059W | 1.1 | 0.9 | 0.2 | 1.4 | 1.4 | 0.8 | 1.6 | 1.3 | 1.8 | 1.4 | 1.0 | 0.8 | 2.4 | 1.0 | 1.0 | 2.0 | 2.6 | 2.2 | 0.45 |
| YOL126C | 1.0 | 0.8 | 1.0 | 1.6 | 0.7 | 1.5 | 1.4 | 0.9 | 0.8 | 2.4 | 1.8 | 1.1 | 2.6 | 1.2 | 1.4 | 2.5 | 1.6 | 2.4 | 0.59 |
| YOL153C | 1.0 | 1.2 | 2.1 | 3.9 | 1.2 | 1.9 | 3.3 | 1.0 | 2.5 | 3.2 | 2.4 | 1.9 | 2.9 | 1.5 | 1.2 | 5.4 | 1.1 | 1.7 | 0.37 |
| YOR099W | 0.8 | 1.3 | 2.1 | 1.3 | 1.5 | 0.9 | 1.0 | 0.9 | 0.6 | 0.7 | 1.2 | 1.1 | 1.6 | 0.7 | 1.5 | 1.1 | 1.2 | 2.3 | 3.94 |
| YOR130C | 0.8 | 1.7 | 1.4 | 1.0 | 1.8 | 1.6 | 1.3 | 0.8 | 2.0 | 1.7 | 0.6 | 0.9 | 2.1 | 1.3 | 1.0 | 0.8 | 1.5 | 1.1 | 0.51 |
| YOR336W | 0.7 | 1.4 | 0.7 | 1.0 | 1.3 | 1.7 | 1.2 | 0.8 | 1.7 | 1.5 | 1.4 | 1.0 | 2.1 | 1.1 | 0.6 | 0.9 | 1.0 | 1.0 | 0.44 |
| YOR347C | 0.9 | 0.9 | 2.1 | 2.2 | 0.7 | 0.9 | 0.9 | 0.8 | 0.5 | 0.9 | 1.4 | 1.2 | 1.8 | 0.9 | 2.0 | 1.4 | 1.7 | 1.3 | 1.31 |
| YPL017C | 1.0 | 2.5 | 0.8 | 1.0 | 1.3 | 1.5 | 1.4 | 1.3 | 3.0 | 6.7 | 0.4 | 1.0 | 1.9 | 1.6 | 1.0 | 1.3 | 1.3 | 0.8 | 0.19 |
| YPR026W | 0.9 | 1.2 | 5.0 | 1.3 | 0.8 | | 1.3 | 1.0 | 0.9 | 3.1 | 1.6 | 1.2 | 2.3 | 1.5 | 3.1 | 2.5 | 0.9 | 1.3 | 0.26 |
| YAL012W | 1.1 | 6.5 | 10.4 | 0.8 | 0.6 | 1.6 | 0.6 | 0.7 | 4.7 | 3.9 | 4.0 | 1.1 | 1.1 | 0.9 | 3.1 | 1.0 | 0.9 | 0.8 | 0.96 |
| YBR029C | 0.7 | 0.2 | 1.8 | 1.0 | 1.4 | 0.8 | 0.4 | 0.7 | 2.0 | 1.1 | 0.7 | 1.3 | 1.7 | 0.9 | 3.5 | 0.9 | 0.6 | 0.8 | 1.56 |

| | | | | | | | | | | | | | | | | | | | |
|---|---|---|---|---|---|---|---|---|---|---|---|---|---|---|---|---|---|---|---|
| YBR222C | 0.6 | 1.2 | 1.3 | 0.8 | 0.7 | 1.3 | 1.0 | 0.6 | 0.7 | 1.0 | 1.3 | 0.9 | 1.6 | 0.5 | 4.3 | 2.1 | 0.8 | 0.9 | 0.52 |
| YCL009C | 0.6 | 0.9 | 2.0 | 0.5 | 0.7 | 1.8 | 1.3 | 1.1 | 0.9 | 1.6 | 1.5 | 1.7 | 1.6 | 0.7 | 6.5 | 0.9 | 1.1 | 0.8 | 0.68 |
| YCL064C | 0.6 | 0.6 | 0.8 | 0.8 | 1.4 | 3.3 | 13.4 | 2.4 | 1.7 | 5.2 | 1.8 | 0.7 | 1.6 | 0.8 | 21.4 | 1.0 | 0.3 | 0.3 | 1.42 |
| YCR098C | 1.6 | 3.0 | 1.7 | 2.0 | 1.4 | 1.6 | 1.2 | 1.0 | 1.4 | 2.4 | 1.3 | 1.3 | 1.4 | 1.1 | 11.0 | 0.8 | 1.7 | 1.6 | 0.22 |
| YDR502C | 1.2 | 2.8 | 2.4 | 1.2 | 1.5 | 1.5 | 0.9 | 0.9 | 1.6 | 0.9 | 1.3 | 1.3 | 1.2 | 0.8 | 8.2 | 1.0 | 0.9 | 0.9 | 2.75 |
| YER026C | 0.8 | 0.6 | 3.3 | 1.0 | 1.2 | 2.5 | 1.6 | 0.8 | 0.9 | 1.5 | 0.9 | 1.7 | 1.9 | 1.1 | 4.0 | 1.4 | 1.7 | 3.2 | 4.48 |
| YHR137W | 0.7 | 1.0 | 2.4 | 1.7 | 1.0 | 0.7 | 0.4 | 0.4 | 0.2 | 0.9 | 0.6 | 0.6 | 0.6 | 0.8 | 2.8 | 0.4 | 0.8 | 1.3 | 1.40 |
| YMR189W | 0.7 | 0.8 | 1.9 | 2.8 | 0.4 | 1.9 | 0.8 | 0.4 | 0.7 | 0.9 | 8.2 | 0.6 | 0.9 | 1.6 | 5.6 | 0.9 | 1.0 | 0.8 | 0.88 |
| YNL106C | 1.0 | 1.4 | 0.8 | 1.5 | 0.8 | 1.0 | 0.9 | 0.8 | 0.7 | 1.4 | 0.9 | 0.8 | 0.9 | 1.1 | 3.3 | 1.2 | 1.1 | 0.9 | 0.37 |
| YNL169C | 0.7 | 1.4 | 1.4 | 1.2 | 0.7 | 1.4 | 0.7 | 0.8 | 1.1 | 1.1 | 0.8 | 1.0 | 1.7 | 0.9 | 3.4 | 0.8 | 1.1 | 1.3 | 1.05 |
| YNL322C | 0.8 | 0.9 | 1.9 | 0.8 | 1.4 | 1.1 | 0.9 | 0.7 | 0.7 | 0.8 | 0.6 | 0.7 | 0.8 | 1.2 | 3.7 | 0.9 | 0.9 | 1.0 | 0.75 |
| YAL038W | 1.0 | 1.0 | 3.0 | 1.4 | 1.4 | 1.3 | 0.9 | 1.1 | 0.1 | 1.1 | 1.0 | 1.2 | 1.8 | 0.5 | 3.1 | 0.9 | 1.0 | 1.0 | 7.02 |
| YBR023C | 0.5 | 0.8 | 1.3 | 1.1 | 0.8 | 0.5 | 0.7 | 0.5 | 0.3 | 0.4 | 0.9 | 0.8 | 0.5 | 0.8 | 2.8 | 0.7 | 0.7 | 0.9 | 0.93 |
| YCR048W | 0.5 | 1.1 | 1.0 | 0.6 | 0.9 | 0.2 | 0.9 | 0.8 | 0.8 | 0.5 | 1.2 | 0.9 | 1.3 | 0.9 | 3.1 | 1.1 | 1.2 | 0.8 | 0.29 |
| YDR380W | 0.8 | 0.8 | 0.8 | 1.7 | 1.1 | 0.4 | 0.5 | 0.2 | 0.2 | 0.3 | 0.4 | 0.4 | 0.4 | 0.6 | 2.4 | 0.2 | 0.8 | 0.8 | 1.04 |
| YER069W | 1.3 | 0.8 | 3.3 | 1.4 | 1.5 | 1.3 | 1.0 | 1.5 | 0.8 | 10.4 | 1.5 | 0.8 | 1.4 | 1.3 | 2.4 | 1.1 | 1.1 | 0.9 | 0.25 |
| YGL022W | 0.5 | 0.8 | 1.4 | 0.3 | 1.0 | 0.9 | 0.7 | 0.4 | 0.9 | 0.4 | 0.8 | 0.7 | 1.0 | 0.5 | 4.0 | 0.3 | 0.9 | 0.7 | 0.91 |
| YGL126W | 0.6 | 1.3 | 1.4 | 0.8 | 1.1 | 1.6 | | 0.7 | 1.6 | 0.7 | 1.0 | 0.6 | | | 2.8 | | 0.9 | 0.9 | 0.34 |
| YGL209W | 1.2 | 1.6 | 1.1 | 2.6 | 0.7 | 1.0 | 1.0 | 1.1 | 0.6 | 0.4 | 0.8 | 1.3 | 1.9 | 1.6 | 3.2 | 2.8 | 1.1 | 1.5 | 0.63 |
| YGR282C | 1.3 | 0.7 | 4.6 | 1.9 | 1.0 | 2.2 | 1.1 | 1.0 | 0.8 | 0.8 | 1.5 | 1.3 | 1.2 | 1.0 | 2.9 | 1.4 | 1.1 | 1.5 | 5.04 |
| YIL154C | 0.8 | 1.3 | 1.9 | 0.8 | 0.9 | 1.1 | 1.3 | 0.6 | 2.4 | 1.0 | 1.2 | 1.1 | 1.7 | 0.9 | 4.3 | 1.2 | 1.2 | 1.0 | 0.39 |
| YJL088W | 0.9 | 0.8 | 2.2 | 0.5 | 0.9 | 2.8 | | 1.3 | 3.2 | 4.1 | 0.9 | 0.7 | 1.0 | 2.0 | 5.7 | 0.7 | 1.4 | 0.9 | 0.27 |
| YJR148W | 0.7 | 1.4 | 2.0 | 3.5 | 0.6 | 2.0 | 2.1 | 0.6 | 0.7 | 3.0 | 0.6 | 0.8 | 1.1 | 1.3 | 3.2 | 1.3 | 1.3 | 1.7 | 1.50 |
| YLR180W | 1.3 | 1.4 | 2.4 | 1.0 | 0.8 | 1.2 | 0.6 | 0.9 | 0.8 | 0.9 | 1.7 | 0.6 | 0.6 | 0.6 | 2.5 | 0.5 | 0.8 | 0.7 | 3.23 |
| YLR273C | 1.4 | 1.0 | 1.3 | 2.4 | | 1.2 | 1.2 | 1.3 | 2.8 | 2.4 | 1.4 | 0.9 | 1.7 | 1.3 | 2.6 | 1.5 | 1.0 | 1.0 | 0.20 |
| YLR300W | 0.9 | 0.8 | 0.8 | 1.0 | 0.8 | 1.1 | 0.5 | 0.8 | 0.0 | 0.5 | 1.0 | 0.5 | 0.2 | 0.6 | 2.7 | 0.3 | 0.7 | 0.5 | 5.14 |
| YLR307W | 0.9 | 0.4 | 0.7 | 3.3 | 1.4 | 1.2 | 1.5 | 1.2 | 0.6 | 0.7 | 0.8 | 0.7 | 1.0 | 1.9 | 3.9 | 0.9 | 1.1 | 1.0 | 0.16 |
| YMR296C | 0.6 | 0.7 | 1.2 | 2.6 | 0.4 | 0.4 | 0.6 | 0.5 | 0.4 | 0.6 | 0.8 | 0.8 | 0.9 | 0.7 | 6.7 | 0.5 | 0.8 | 0.7 | 0.52 |
| YOL058W | 0.8 | 0.5 | 1.7 | 0.9 | 1.2 | 0.8 | 0.4 | 1.6 | 0.1 | 15.7 | 2.3 | 1.3 | 1.2 | 1.5 | 2.7 | 0.9 | 1.2 | 0.8 | 0.82 |
| YBR183W | 1.8 | 1.5 | 2.0 | 2.9 | 0.9 | 2.5 | 1.7 | 1.0 | 0.4 | 2.0 | 1.9 | 1.2 | 1.4 | 1.3 | 1.1 | 2.1 | 1.4 | 2.4 | 1.47 |
| YDR019C | 1.4 | 0.5 | 1.7 | 4.0 | 0.9 | 4.8 | 1.4 | 0.7 | 0.4 | 0.7 | 1.8 | 0.7 | 1.0 | 1.6 | 1.8 | 1.8 | 1.2 | 1.6 | 2.48 |

| | | | | | | | | | | | | | | | | | | | |
|---|---|---|---|---|---|---|---|---|---|---|---|---|---|---|---|---|---|---|---|
| YIL167W | 3.3 | 1.6 | 2.0 | 1.0 | 1.0 | 7.7 | 1.2 | 1.9 | 6.9 | 12.7 | 1.9 | 0.7 | 1.2 | 1.4 | 0.6 | 2.3 | 0.7 | 0.8 | 2.11 |
| YKR039W | 1.4 | 3.5 | 1.3 | 1.7 | 1.0 | 2.8 | 1.0 | 1.0 | 1.9 | 2.0 | 1.9 | 1.5 | 1.3 | 1.2 | 1.7 | 1.9 | 0.9 | 1.1 | 0.40 |
| YNL037C | 1.4 | 1.8 | 1.7 | 1.1 | 1.7 | 2.8 | 2.8 | 1.0 | 0.6 | 3.2 | 1.4 | 1.3 | 1.9 | 1.3 | 0.9 | 1.4 | 0.9 | 1.3 | 2.05 |
| YNR002C | 1.3 | 1.6 | 1.0 | 2.0 | 1.2 | 2.3 | 1.2 | 1.0 | 3.9 | 1.6 | 1.6 | 1.2 | 2.1 | 1.2 | 1.0 | 2.4 | 1.5 | 2.1 | 0.29 |
| YOL143C | 1.0 | 1.4 | 1.2 | 1.2 | 0.9 | 3.5 | 1.3 | 1.2 | 0.8 | 1.0 | 2.0 | 1.2 | 1.2 | 0.7 | 1.0 | 2.2 | 1.8 | 1.4 | 2.30 |
| YOR136W | 1.0 | 0.9 | 3.5 | 1.0 | 1.5 | 3.9 | 3.2 | 1.1 | 0.3 | 3.4 | 1.4 | 1.2 | 1.5 | 0.9 | 1.2 | 1.3 | 0.8 | 1.3 | 3.20 |
| YAL044C | 1.5 | 1.1 | 1.0 | 3.0 | 0.8 | 2.2 | 1.0 | 0.8 | 0.4 | 0.6 | 1.4 | 0.6 | 1.1 | 1.4 | 0.7 | 1.0 | 1.4 | 1.8 | 4.07 |
| YAL054C | 1.2 | 1.1 | 1.4 | 1.6 | 1.1 | 2.2 | 1.1 | 1.1 | 8.9 | 4.1 | 1.3 | 1.0 | 1.8 | 1.3 | 0.9 | 2.5 | 1.5 | 1.4 | 0.24 |
| YAR071W | 1.6 | 0.8 | 1.0 | 0.7 | 1.6 | 2.0 | 0.3 | 1.4 | 0.3 | 0.3 | 1.1 | 2.0 | 0.3 | 1.1 | 1.1 | 0.3 | 0.7 | 1.6 | 3.21 |
| YBL001C | 1.1 | 1.5 | 1.0 | 1.6 | 1.0 | 2.4 | 1.3 | 1.0 | 1.5 | 0.9 | 2.3 | 0.7 | 1.0 | 1.5 | 1.0 | 1.7 | 1.6 | 1.8 | 2.38 |
| YBR014C | 1.1 | 1.2 | 0.8 | 0.9 | 1.4 | 2.0 | 1.4 | 1.2 | 1.3 | 0.8 | 1.8 | 0.6 | 0.9 | 1.2 | 1.2 | 1.5 | 1.0 | 1.6 | 1.29 |
| YBR035C | 1.1 | 1.0 | 1.7 | 1.1 | 1.3 | 2.3 | 1.5 | 1.5 | 2.2 | 1.5 | 2.0 | 1.2 | 1.8 | 1.3 | 0.7 | 2.1 | 1.5 | 1.9 | 1.89 |
| YBR068C | 1.1 | 1.0 | 1.9 | 2.5 | 1.4 | 1.9 | | 1.0 | 0.8 | 2.5 | 0.8 | 0.8 | 2.2 | 1.8 | 1.6 | 5.5 | 1.1 | 1.3 | 1.99 |
| YBR111C | 1.0 | 0.9 | 1.0 | 1.7 | 1.3 | 3.0 | 2.2 | 1.7 | 1.9 | 1.9 | 1.5 | 1.2 | 1.6 | 1.6 | 0.7 | 3.5 | 1.4 | 2.4 | 3.20 |
| YCR037C | 0.7 | 1.0 | 0.8 | 0.8 | 1.3 | 1.8 | 1.0 | 0.7 | 0.6 | 0.6 | 0.8 | 0.7 | 1.3 | 0.8 | 1.0 | 1.1 | 1.4 | 0.9 | 0.74 |
| YDL022W | 0.9 | 2.6 | 6.9 | 1.5 | 0.7 | 2.1 | 1.5 | 0.8 | 1.2 | 1.8 | 2.8 | 1.2 | 1.5 | 0.8 | 0.9 | 1.1 | 1.0 | 1.4 | 1.79 |
| YDR009W | 1.4 | 0.8 | 1.5 | 1.0 | 1.2 | 2.4 | 1.4 | 1.2 | 1.3 | 2.0 | 0.1 | 1.0 | 1.2 | 1.5 | 3.2 | 1.0 | 1.2 | 1.0 | 0.21 |
| YDR410C | 0.8 | 2.0 | 1.3 | 0.9 | 1.1 | 2.5 | 1.4 | 0.9 | 1.2 | 1.5 | 1.6 | 0.9 | 1.2 | 1.3 | 1.2 | 0.8 | 1.0 | 1.0 | 1.33 |
| YDR487C | 2.2 | 0.9 | 1.3 | 1.1 | 1.3 | 2.3 | 1.3 | 1.8 | 2.7 | 2.3 | 2.1 | 1.3 | 1.5 | 2.5 | 1.0 | 1.5 | 1.5 | 1.9 | 2.61 |
| YEL011W | 2.0 | 1.6 | 1.7 | 6.3 | 1.1 | 2.8 | 2.7 | 0.9 | 2.5 | 1.6 | 3.7 | 1.5 | 1.5 | 1.5 | 0.4 | 2.4 | 1.4 | 2.5 | 0.89 |
| YFR015C | 1.2 | 0.8 | 3.2 | 2.1 | 1.2 | 3.0 | 7.3 | 0.7 | 0.3 | 1.8 | 3.9 | 0.4 | 2.5 | 3.1 | 0.4 | 2.4 | 1.3 | 1.2 | 0.66 |
| YGL001C | 1.0 | 1.0 | 1.4 | 1.1 | 1.5 | 2.8 | 1.4 | 1.1 | 0.8 | 0.8 | 1.6 | 0.9 | 1.0 | 1.1 | 0.9 | 2.2 | 1.9 | 2.1 | 2.53 |
| YGL104C | 0.8 | 2.0 | 2.1 | 1.8 | 1.0 | 2.1 | 1.2 | 0.8 | 4.6 | 2.1 | 1.8 | 1.1 | 2.0 | 1.0 | 0.9 | 1.7 | 1.3 | 1.3 | 0.58 |
| YGL154C | 1.3 | 1.4 | 1.5 | 0.9 | 1.0 | 1.7 | 0.7 | 1.2 | 2.0 | 1.4 | 0.9 | 0.9 | 2.0 | 1.1 | 0.8 | 0.7 | 1.0 | 1.1 | 0.57 |
| YGL253W | 0.8 | 1.0 | 3.2 | 0.7 | 1.3 | 2.4 | 1.7 | 1.1 | 0.3 | 1.4 | 1.0 | 1.2 | 1.5 | 0.5 | 0.8 | 0.4 | 0.7 | 0.8 | 4.63 |
| YGR060W | 0.7 | 0.6 | 0.9 | 0.7 | 1.9 | 2.5 | 0.5 | 1.0 | 0.5 | 0.4 | 0.8 | 1.0 | 0.6 | 0.8 | 1.4 | 2.1 | 1.3 | 1.6 | 4.19 |
| YHR037W | 0.7 | 0.9 | 1.3 | 1.6 | 0.9 | 1.8 | 1.2 | 1.0 | 0.9 | 2.2 | 1.7 | 1.1 | 1.1 | 1.1 | 1.1 | 2.0 | 1.0 | 1.4 | 1.15 |
| YHR092C | 2.3 | 1.0 | 7.5 | 2.5 | 1.4 | 1.7 | 0.5 | 1.5 | 0.2 | 0.9 | 1.5 | 1.0 | 0.5 | 1.6 | 0.2 | 1.2 | 0.9 | 1.3 | 6.09 |
| YHR190W | 0.8 | 3.9 | 1.2 | 1.2 | 1.8 | 1.7 | 1.9 | 1.2 | 2.4 | 1.9 | 1.3 | 1.1 | 1.7 | 1.3 | 1.3 | 1.8 | 1.3 | 1.8 | 2.15 |
| YIL033C | 0.8 | 1.3 | 3.3 | 1.3 | 0.7 | 2.0 | 1.1 | 0.7 | 2.4 | 1.3 | 1.8 | 1.1 | 2.5 | 0.7 | 1.0 | 2.0 | 1.0 | 1.2 | 1.18 |
| YIR035C | 1.4 | 1.8 | 0.9 | 1.6 | 0.7 | 2.1 | 1.3 | 1.3 | 0.7 | 1.2 | 1.3 | 0.9 | 1.1 | 1.1 | 1.4 | 1.0 | 1.1 | 0.9 | 1.51 |

| | | | | | | | | | | | | | | | | | | |
|---|---|---|---|---|---|---|---|---|---|---|---|---|---|---|---|---|---|---|
| YJL132W | 1.0 | 0.7 | 0.8 | 1.6 | 1.3 | 2.0 | 1.7 | 1.0 | 1.6 | 1.4 | 1.4 | 1.0 | 1.3 | 1.4 | 1.1 | 2.1 | 1.3 | 1.5 | 0.37 |
| YJL196C | 0.9 | 0.9 | 2.5 | 1.3 | 1.1 | 2.0 | 0.7 | 0.7 | 1.1 | 0.9 | 2.3 | 1.1 | 0.6 | 0.8 | 0.6 | 1.7 | 0.9 | 1.9 | 2.85 |
| YJR142W | 1.2 | 0.6 | 1.1 | 1.1 | 1.3 | 2.4 | 1.7 | 1.0 | 0.6 | 0.8 | 0.9 | 0.8 | 1.6 | 1.3 | 0.6 | 2.0 | 1.2 | 1.4 | 0.94 |
| YKL067W | 1.1 | 1.9 | 2.1 | 1.4 | 1.9 | 2.2 | 2.6 | 1.2 | 0.9 | 1.6 | 1.8 | 0.9 | 1.7 | 1.2 | 1.1 | 2.4 | 1.4 | 3.2 | 3.29 |
| YLR142W | 4.4 | 2.7 | 1.1 | 6.1 | 1.3 | 3.1 | 1.2 | 2.1 | 3.1 | 4.4 | 3.8 | 0.8 | 1.2 | 2.1 | 1.6 | 3.4 | 1.9 | 3.2 | 0.28 |
| YML110C | 1.1 | 0.8 | 1.9 | 1.6 | 0.8 | 2.6 | 2.0 | 1.4 | 2.7 | 2.2 | 1.7 | 1.1 | 1.8 | 1.4 | 1.3 | 2.3 | 1.1 | 1.6 | 2.01 |
| YMR272C | 0.7 | 1.0 | 1.6 | 0.7 | 1.1 | 2.6 | 1.0 | 0.8 | 0.4 | 0.6 | 0.8 | 1.2 | 1.2 | 0.8 | 1.1 | 1.5 | 1.3 | 1.4 | 1.66 |
| YNL130C | 0.6 | 0.7 | 2.4 | 0.7 | 0.8 | 2.4 | 1.3 | 0.8 | 1.7 | 2.4 | 1.5 | 0.9 | 1.9 | 1.0 | 2.5 | 0.7 | 0.7 | 1.2 | 1.18 |
| YPR006C | 1.9 | 1.5 | 0.5 | 1.9 | 2.0 | 2.4 | 2.8 | 1.5 | 1.5 | 3.9 | 2.4 | 1.1 | 1.8 | 1.2 | 0.7 | 1.6 | 1.8 | 2.1 | 0.46 |
| YBR050C | 2.4 | 1.9 | 1.9 | 3.1 | 1.0 | 4.1 | 2.6 | 1.7 | 5.8 | 3.2 | 1.1 | 1.0 | 1.3 | 1.3 | 0.5 | 1.7 | 1.5 | 1.8 | 0.41 |
| YBR145W | 1.5 | 0.7 | 2.8 | 0.9 | 1.1 | 11.5 | 58.8 | 1.0 | 0.1 | 1.1 | 1.1 | 1.0 | 2.0 | 2.2 | 1.2 | 3.6 | 1.7 | 2.0 | 2.17 |
| YBR299W | 1.8 | 0.9 | 1.1 | 3.5 | 1.6 | 0.8 | 3.6 | 2.2 | 1.1 | 5.3 | 2.4 | 1.2 | 0.7 | 1.4 | 0.6 | 3.9 | 1.0 | 1.1 | 0.32 |
| YEL020C | 1.0 | 1.5 | 0.8 | 2.9 | 1.5 | 1.3 | 2.4 | 1.2 | 1.4 | 1.1 | 1.3 | 0.8 | 1.2 | 1.0 | 1.4 | 2.1 | 1.2 | 1.3 | 0.31 |
| YGL039W | 0.8 | 1.4 | 2.1 | 1.3 | 1.3 | 1.7 | 4.2 | 1.3 | 0.7 | 2.8 | 1.1 | 1.3 | 1.5 | 0.9 | 1.5 | 1.5 | 0.6 | 1.0 | 1.09 |
| YGL134W | 0.9 | 1.3 | 0.5 | 0.8 | 1.4 | 1.2 | 2.3 | 1.4 | 1.1 | 1.4 | 1.1 | 0.9 | 0.7 | 1.1 | 0.5 | 1.7 | 0.9 | 1.3 | 0.53 |
| YJR159W | 1.4 | 2.3 | 1.3 | 1.7 | 0.9 | 1.4 | 2.3 | 2.7 | 5.2 | 2.8 | 2.2 | 1.5 | 2.1 | 2.0 | 2.3 | 5.2 | 1.7 | 2.3 | 0.30 |
| YOL157C | 1.0 | 1.1 | 1.3 | 2.5 | 1.4 | 0.9 | 2.7 | 1.4 | 2.3 | 4.8 | 1.2 | 1.2 | 1.4 | 1.2 | 1.1 | 3.5 | 1.4 | 1.3 | 0.41 |
| YOR344C | 1.1 | 1.1 | 0.4 | 0.7 | 1.9 | 2.0 | 2.4 | 1.4 | 0.3 | 0.9 | 0.6 | 1.3 | 1.8 | 0.6 | 0.7 | 0.6 | 1.3 | 1.3 | 2.11 |
| YPL265W | 0.7 | 0.4 | 2.3 | 1.1 | 1.0 | 2.3 | 8.9 | 2.3 | 0.2 | 1.2 | 1.8 | 0.9 | 1.1 | 1.1 | 1.2 | 2.0 | 0.5 | 0.4 | 1.42 |
| YBR126C | 0.8 | 1.9 | 5.6 | 1.2 | 0.7 | 2.9 | 2.3 | 0.6 | 1.7 | | 1.1 | 1.3 | 2.1 | 0.7 | 1.0 | 1.7 | 1.5 | 1.3 | 1.96 |
| YCR005C | 1.1 | 1.9 | 2.0 | 1.2 | 0.9 | 1.6 | 4.4 | 1.2 | 1.5 | 1.5 | 2.1 | 0.5 | 0.7 | 0.8 | 0.7 | 0.7 | 1.6 | 1.7 | 2.38 |
| YDR452W | 1.1 | 1.1 | 1.3 | 0.8 | 1.2 | 2.0 | 1.9 | 1.0 | 1.4 | 1.3 | 1.5 | 0.9 | 1.4 | 1.2 | 1.3 | 1.3 | 1.9 | 2.2 | 1.54 |
| YGR019W | 1.2 | 0.8 | 1.4 | 1.7 | 0.8 | 2.4 | 2.4 | 1.2 | 2.9 | | 1.1 | 1.2 | 2.1 | 1.3 | 2.2 | 2.4 | 1.3 | 2.0 | 0.79 |
| YGR255C | 0.9 | 1.4 | 1.4 | 1.5 | 1.3 | 3.2 | 1.8 | 1.0 | 2.5 | 1.8 | 2.0 | 1.3 | 2.5 | 0.9 | 1.0 | 1.1 | 1.1 | 1.1 | 0.81 |
| YIL098C | 1.4 | 1.6 | 0.5 | 2.0 | 1.7 | 1.7 | 2.2 | 1.6 | 1.9 | 1.9 | 1.4 | 0.8 | 1.3 | 1.9 | 0.7 | 2.0 | 1.2 | 1.6 | 0.75 |
| YIL172C | 1.1 | 1.1 | 1.6 | 1.7 | | 1.3 | 2.5 | 1.6 | 2.8 | 7.1 | 1.0 | 1.4 | 2.0 | 1.3 | 1.4 | 2.8 | 1.1 | 1.2 | 0.42 |
| YLR100W | 0.8 | 1.4 | 1.3 | 1.9 | 1.8 | 1.8 | 2.3 | 1.0 | 0.9 | 1.0 | 0.9 | 1.1 | 1.6 | 1.1 | 0.6 | 1.9 | 1.5 | 2.1 | 1.88 |
| YOR221C | 0.8 | 1.0 | 0.9 | 1.1 | 1.7 | 0.8 | 2.1 | 0.9 | 1.4 | 1.7 | 1.1 | 0.8 | 1.0 | 1.1 | 1.0 | 1.4 | 0.9 | 1.2 | 0.39 |
| YPL123C | 1.2 | 0.8 | 1.8 | 3.0 | 0.8 | 1.9 | 2.7 | 1.2 | 4.2 | 2.3 | 1.9 | 1.0 | 2.2 | 1.6 | 1.4 | 2.6 | 1.6 | 3.2 | 0.71 |
| YBR093C | 2.9 | 1.2 | 1.7 | 1.3 | 1.1 | 0.6 | 0.3 | 1.7 | 0.1 | 0.5 | 3.1 | 2.0 | 0.2 | 1.3 | 2.7 | 0.4 | 0.8 | 1.9 | 3.33 |
| YBR196C | 0.8 | 0.6 | 3.9 | 1.4 | 0.8 | 0.8 | 1.4 | 1.1 | 0.3 | 0.9 | 2.3 | 1.0 | 1.4 | 0.5 | 1.9 | 1.1 | 0.8 | 1.0 | 6.60 |

| | | | | | | | | | | | | | | | | | | | |
|---|---|---|---|---|---|---|---|---|---|---|---|---|---|---|---|---|---|---|---|
| YER023W | 0.8 | 0.6 | 1.2 | 1.2 | 0.8 | 1.3 | 0.7 | 0.9 | 1.1 | 0.6 | 2.1 | 0.8 | 1.2 | 0.7 | 1.2 | 1.6 | 1.5 | 1.4 | 1.77 |
| YFL055W | 2.0 | 6.7 | 1.3 | 2.5 | 1.5 | 1.1 | 1.4 | 1.3 | 23.9 | 6.5 | 2.7 | 0.9 | 1.3 | 1.5 | 0.7 | 1.4 | 1.4 | 1.0 | 0.23 |
| YIL124W | 0.9 | 0.8 | 2.8 | 1.6 | 0.9 | 1.2 | 1.3 | 0.9 | 1.0 | 0.7 | 2.8 | 1.1 | 0.9 | 1.1 | 0.6 | 3.1 | 1.2 | 2.1 | 2.39 |
| YMR318C | 1.4 | 2.4 | 2.2 | 0.7 | 1.2 | 2.1 | 0.8 | 3.6 | 2.3 | 4.8 | 3.6 | 0.8 | 1.8 | 1.7 | 1.5 | 1.1 | 1.1 | 1.1 | 3.17 |
| YBR067C | 1.6 | 2.5 | 2.8 | 0.9 | 1.1 | 1.1 | 1.1 | 1.1 | 1.7 | 1.0 | 3.1 | 1.0 | 0.9 | 0.7 | 3.3 | 2.5 | 0.5 | 0.4 | 1.76 |
| YBR115C | 0.6 | 0.7 | 1.4 | 0.4 | 0.7 | 0.9 | 1.1 | 1.3 | 0.8 | 1.6 | 1.8 | 0.9 | 2.3 | 0.7 | 1.1 | 0.5 | 0.6 | 0.6 | 0.64 |
| YDL131W | 1.1 | 0.9 | 1.8 | 1.0 | 1.0 | 0.9 | 0.7 | 2.2 | 0.8 | 2.7 | 2.2 | 1.2 | 1.0 | 0.9 | 1.1 | 0.9 | 0.7 | 0.6 | 1.81 |
| YDL168W | 2.3 | 2.0 | 2.1 | 0.9 | 1.2 | 1.4 | 1.1 | 1.7 | 8.2 | 4.7 | 1.9 | 0.6 | 1.3 | 1.6 | 1.2 | 1.1 | 0.9 | 0.8 | 1.08 |
| YDR216W | 1.3 | 1.1 | 2.0 | 2.6 | 0.8 | 1.3 | 1.0 | 1.0 | 2.5 | 2.7 | 2.1 | 1.0 | 2.4 | 0.9 | 2.5 | 1.6 | 1.0 | 1.8 | 0.44 |
| YDR253C | 5.7 | 6.4 | 1.4 | 3.2 | 0.9 | 0.8 | 1.0 | 1.7 | 14.8 | 6.3 | 2.3 | 1.0 | 2.2 | 3.6 | 1.2 | 1.6 | 1.0 | 1.0 | 0.38 |
| YDR513W | 2.2 | 2.5 | 2.3 | 2.6 | 0.9 | 2.1 | 1.6 | 1.6 | 4.6 | 3.1 | 2.0 | 0.9 | 1.8 | 2.0 | 1.3 | 3.8 | 1.3 | 3.2 | 3.10 |
| YER061C | 0.9 | 0.9 | 1.2 | 2.5 | 0.8 | 0.4 | 0.9 | 0.7 | 0.3 | 0.7 | 2.2 | 0.7 | 0.9 | 1.0 | 0.8 | 1.8 | 1.2 | 1.2 | 0.84 |
| YFL052W | 1.3 | 0.8 | 0.7 | 2.3 | 1.5 | 2.1 | 1.4 | 1.3 | 0.6 | 1.2 | 2.3 | 0.8 | 1.0 | 1.6 | 0.4 | 1.0 | 1.1 | 0.9 | 0.32 |
| YFL058W | 1.2 | 1.7 | 1.1 | 1.4 | 1.1 | 2.0 | 0.9 | 1.7 | 3.2 | 2.4 | 2.4 | 1.2 | 1.2 | 1.0 | 1.7 | 0.7 | 1.1 | 0.9 | 0.40 |
| YFR030W | 3.1 | 5.5 | 8.5 | 0.5 | 0.8 | 1.8 | 1.1 | 1.7 | 24.1 | 9.0 | 2.4 | 2.4 | 4.1 | 1.3 | 1.7 | 1.6 | 1.3 | 0.9 | 0.30 |
| YGL202W | 0.8 | 0.7 | 2.2 | 0.7 | 0.7 | 0.7 | 0.6 | 1.0 | 1.3 | 1.7 | 2.4 | 0.9 | 1.1 | 0.8 | 2.2 | 0.6 | 1.3 | 0.9 | 1.70 |
| YGR070W | 1.1 | 1.0 | 2.0 | 1.5 | 1.4 | | 1.4 | 1.8 | 1.1 | 1.5 | 1.8 | 0.6 | 1.4 | 1.0 | 2.6 | 2.3 | 1.2 | 1.3 | 0.40 |
| YHL036W | 2.2 | 4.8 | 3.4 | 2.0 | 1.3 | 1.3 | 1.0 | 1.2 | 9.0 | 4.4 | 2.3 | 1.2 | 1.5 | 1.2 | 0.9 | 1.1 | 1.0 | 1.0 | 0.60 |
| YHR104W | 1.0 | 4.1 | 15.7 | 1.9 | 1.1 | 1.2 | 1.5 | 1.1 | 4.8 | 2.3 | 2.6 | 1.0 | 1.2 | 1.5 | 1.6 | 1.3 | 1.9 | 1.9 | 1.57 |
| YJL045W | 1.8 | 2.2 | 1.6 | 5.3 | 0.7 | 0.6 | 0.7 | 0.9 | 9.7 | 1.6 | 2.3 | 1.1 | 1.6 | 1.2 | 3.4 | 2.4 | 0.9 | 0.9 | 0.42 |
| YJL060W | 1.8 | 2.6 | 9.6 | 1.0 | 1.4 | 1.3 | 1.3 | 0.8 | 5.3 | 2.8 | 1.6 | 1.5 | 2.1 | 1.7 | 1.4 | 1.5 | 1.2 | 1.1 | 0.95 |
| YJL155C | 2.1 | 5.0 | 0.8 | 2.4 | 0.8 | 0.8 | 2.0 | 1.5 | 3.8 | 2.8 | 2.4 | 1.0 | 6.6 | 1.2 | 1.2 | 2.3 | 1.8 | 3.1 | 0.60 |
| YJR109C | 0.8 | 0.7 | 1.4 | 0.9 | 0.7 | 0.9 | 0.8 | 1.0 | 1.8 | 4.3 | 1.5 | 1.4 | 3.1 | 0.9 | 1.3 | 0.5 | 1.1 | 0.6 | 0.84 |
| YJR156C | 1.6 | 3.2 | 2.4 | 1.8 | 0.9 | 0.9 | 1.3 | 1.5 | 3.4 | 2.8 | 3.0 | 1.1 | 1.6 | 2.4 | 3.0 | 2.3 | 0.8 | 1.1 | 0.24 |
| YLR092W | 5.0 | 7.5 | 1.4 | 0.6 | 1.3 | 1.0 | 1.3 | 1.5 | 12.7 | 5.8 | 3.1 | 1.0 | 1.8 | 1.4 | 0.9 | 1.2 | 1.0 | 1.1 | 0.24 |
| YMR081C | 3.5 | 1.4 | 2.0 | 5.8 | 1.7 | 1.1 | 0.9 | 1.7 | 0.3 | 2.2 | 2.5 | 1.1 | 0.7 | 1.8 | 0.2 | 3.0 | 1.9 | 3.0 | 0.62 |
| YMR250W | 0.9 | 5.1 | 5.6 | 1.3 | 0.6 | 4.5 | 3.0 | 0.9 | 6.1 | 2.4 | 4.3 | 1.2 | 2.6 | 1.1 | 2.0 | 5.0 | 1.2 | 3.0 | 0.84 |
| YNL277W | 5.7 | 10.3 | 5.3 | 0.6 | 1.1 | 1.7 | 1.4 | 2.2 | 55.0 | 10.9 | 3.2 | 1.3 | 1.8 | 1.5 | 0.6 | 1.3 | 0.9 | 0.7 | 0.27 |
| YOR184W | 1.1 | 0.9 | 2.1 | 2.1 | 1.0 | 0.6 | 1.2 | 0.7 | 0.9 | 0.7 | 2.7 | 0.7 | 0.6 | 0.7 | 1.0 | 0.9 | 1.2 | 1.2 | 3.19 |
| YPR160W | 1.4 | 3.8 | 3.6 | 3.3 | 0.7 | 4.5 | 1.8 | 0.9 | 0.9 | 1.3 | 4.4 | 2.1 | 2.2 | 1.1 | 1.4 | 5.3 | 1.0 | 2.9 | 1.42 |
| YDL182W | 0.8 | 0.7 | 1.3 | 0.8 | 1.3 | 1.0 | 1.0 | 2.9 | 0.8 | 2.4 | 2.0 | 1.4 | 1.5 | 0.8 | 0.7 | 1.1 | 0.6 | 0.6 | 2.31 |

| | | | | | | | | | | | | | | | | | | | |
|---|---|---|---|---|---|---|---|---|---|---|---|---|---|---|---|---|---|---|---|
| YBR291C | 2.0 | 0.9 | 1.0 | 1.5 | 0.9 | 1.1 | 0.9 | 2.2 | 0.9 | 1.0 | 1.1 | 0.9 | 1.0 | 1.7 | 0.5 | 1.5 | 0.9 | 1.3 | 1.19 |
| YIL094C | 1.5 | 0.4 | 0.8 | 0.7 | 1.4 | 1.2 | 1.3 | 3.6 | 0.3 | 0.4 | 1.8 | 1.0 | 0.7 | 1.0 | 0.5 | 0.9 | 0.9 | 1.0 | 2.26 |
| YNR050C | 1.7 | 0.5 | 1.2 | 0.6 | 1.3 | 0.9 | 1.0 | 2.2 | 0.4 | 0.7 | 1.5 | 1.0 | 1.1 | 0.6 | 0.3 | 1.4 | 0.6 | 0.7 | 2.13 |
| YDL244W | 1.1 | 3.5 | 1.8 | 1.6 | 0.9 | 1.3 | 1.7 | 1.8 | 2.5 | 3.7 | 1.2 | 1.2 | 2.0 | 2.0 | 2.9 | 1.5 | 1.1 | 1.0 | 0.25 |
| YDR054C | 1.3 | 1.0 | 0.7 | 1.4 | 0.7 | 1.0 | 1.1 | 1.5 | 2.9 | 3.2 | 1.9 | 1.0 | 2.5 | 1.1 | 1.6 | 1.4 | 0.8 | 1.4 | 1.18 |
| YDR353W | 0.9 | 1.6 | 3.4 | 1.6 | 0.6 | 0.9 | 1.0 | 1.1 | 2.0 | 5.1 | 1.4 | 0.8 | 0.7 | 1.6 | 1.3 | 1.0 | 0.9 | 1.3 | 3.20 |
| YEL070W | 1.4 | 0.9 | 1.6 | 3.4 | 1.3 | 1.2 | 1.3 | 1.2 | 1.9 | 13.0 | 1.1 | 1.1 | 1.1 | 2.1 | 3.2 | 1.9 | 0.9 | 1.0 | 0.27 |
| YIL168W | 2.8 | 1.4 | 1.0 | 1.2 | 1.4 | 0.7 | 1.3 | 1.6 | 7.8 | 19.3 | 1.9 | 0.8 | 0.9 | 1.5 | 1.2 | 1.2 | 1.0 | 1.1 | 0.39 |
| YJL221C | 1.1 | 1.0 | 1.1 | 1.3 | 0.9 | 6.6 | 2.5 | 1.8 | 2.7 | 4.4 | 0.8 | 1.1 | 1.4 | 1.1 | 1.1 | 3.3 | 1.1 | 1.4 | 0.41 |
| YJR095W | 1.2 | 20.5 | 1.9 | 6.7 | 1.2 | 1.5 | 2.0 | 0.9 | 0.5 | 6.3 | 0.6 | 0.7 | 0.8 | 1.3 | 0.8 | 0.8 | 1.3 | 0.9 | 0.23 |
| YKL085W | 1.4 | 2.3 | 1.6 | 1.2 | 1.2 | 1.9 | 1.5 | 1.2 | 1.9 | 3.0 | 1.8 | 0.8 | 1.5 | 1.0 | 0.5 | 1.7 | 0.9 | 1.3 | 2.16 |
| YKL188C | 1.3 | 0.7 | 1.9 | 2.4 | 1.0 | 0.8 | 1.1 | 0.7 | 2.5 | 2.8 | 1.1 | 1.2 | 1.2 | 2.1 | 1.4 | 2.7 | 1.1 | 1.5 | 0.27 |
| YKL217W | 1.8 | 2.4 | 1.0 | 2.1 | 1.1 | 1.2 | 1.6 | 1.1 | 0.9 | 4.1 | 1.6 | 0.8 | 1.2 | 1.2 | 2.2 | 3.0 | 1.7 | 3.3 | 0.29 |
| YKR061W | 2.0 | 0.9 | 0.5 | 1.9 | 1.2 | 1.2 | 1.4 | 1.5 | 1.5 | 2.6 | 1.1 | 0.9 | 0.9 | 1.6 | 0.9 | 0.8 | 1.5 | 1.6 | 0.70 |
| YLR174W | 1.2 | 1.5 | 1.7 | 2.1 | 0.9 | 0.9 | 1.6 | 1.1 | 2.5 | 8.3 | 1.3 | 1.3 | 1.8 | 1.7 | 0.8 | 4.6 | 0.9 | 1.2 | 0.41 |
| YLR260W | 1.1 | 1.5 | 1.5 | 2.4 | 1.2 | 1.0 | 1.2 | 1.2 | 2.6 | 3.2 | 0.6 | 0.8 | 1.9 | 1.2 | 1.0 | 1.0 | 1.1 | 1.2 | 0.44 |
| YNL009W | 1.1 | 1.9 | 2.0 | 1.4 | 1.4 | 1.1 | 1.2 | 1.1 | 1.4 | 3.3 | 1.3 | 0.9 | 1.3 | 1.3 | 2.7 | 3.3 | 1.2 | 2.3 | 0.45 |
| YNL117W | 0.9 | 4.7 | 1.7 | 0.8 | 0.8 | | 1.1 | 1.7 | 12.8 | 4.4 | 1.4 | 0.7 | 1.3 | 2.0 | 2.6 | 1.6 | 1.1 | 0.9 | 0.24 |
| YNL183C | 0.9 | 3.5 | 6.4 | 1.3 | 0.7 | 1.2 | 1.3 | 0.9 | 4.8 | 4.3 | 1.3 | 1.1 | 2.3 | 1.1 | 1.1 | 1.2 | 0.8 | 1.0 | 0.47 |
| YNR073C | 1.1 | 1.4 | 0.8 | 2.7 | 1.4 | 1.7 | 2.2 | 1.5 | 2.5 | 16.8 | 2.2 | 1.1 | 1.2 | 2.3 | 1.7 | 1.6 | 1.0 | 0.9 | 0.18 |
| YPL161C | 1.0 | 1.7 | 1.1 | 1.1 | 0.8 | 0.7 | 1.3 | 1.8 | 1.2 | 2.7 | 1.0 | 1.0 | 1.2 | 1.1 | 0.8 | 1.4 | 1.3 | 1.3 | 0.41 |
| YAL061W | 1.7 | 2.4 | 3.3 | 3.8 | 1.0 | 1.0 | 2.0 | 0.8 | 5.5 | 1.4 | 4.1 | 1.1 | 1.4 | 0.7 | 0.6 | 1.1 | 1.4 | 1.2 | 0.88 |
| YAL067C | 2.7 | 7.5 | 1.5 | 1.4 | 0.7 | 1.2 | 0.8 | 1.1 | 7.6 | 2.9 | 1.2 | 1.0 | 1.1 | 1.3 | 2.4 | 1.6 | 1.0 | 1.0 | 0.33 |
| YBL033C | 1.3 | 1.9 | 2.4 | 1.0 | 1.1 | 0.4 | 0.9 | 1.4 | 6.6 | 4.2 | 1.1 | 0.9 | 1.8 | 1.4 | 1.0 | 1.2 | 1.2 | 1.0 | 0.53 |
| YBL086C | 0.8 | 0.5 | 1.0 | 0.6 | 1.3 | 0.7 | 1.0 | 1.1 | 3.3 | 1.4 | 1.3 | 0.5 | 0.9 | 0.8 | 0.5 | 1.4 | 0.9 | 1.0 | 0.43 |
| YBR117C | 0.8 | 1.6 | 1.5 | 1.4 | 0.9 | 2.3 | 1.0 | 0.7 | 5.9 | 1.5 | 0.4 | 0.8 | 2.1 | 0.9 | 1.0 | 12.0 | 0.7 | 0.7 | 0.49 |
| YBR213W | 1.0 | 1.6 | 1.0 | 0.5 | 1.1 | 2.4 | 1.3 | 1.3 | 11.4 | 2.3 | 0.5 | 1.2 | 1.4 | 1.3 | 1.7 | 0.8 | 0.9 | 0.9 | 0.23 |
| YCR036W | 1.3 | 1.2 | 1.3 | 1.3 | 1.5 | 1.4 | 1.5 | 1.1 | 2.6 | 1.5 | 1.0 | 1.3 | 1.8 | 1.5 | 1.7 | 1.6 | 1.6 | 1.7 | 1.35 |
| YDL132W | 0.8 | 1.2 | 1.1 | 0.7 | 1.3 | 0.9 | 1.4 | 1.0 | 3.8 | 2.4 | 1.3 | 0.7 | 2.0 | 0.9 | 1.7 | 1.0 | 0.9 | 1.1 | 0.74 |
| YER014W | 1.0 | 0.9 | 0.9 | 0.6 | 0.8 | 4.0 | | 1.2 | 3.5 | 1.1 | 0.9 | 1.1 | 1.5 | 1.2 | 2.3 | 0.8 | 1.0 | 0.9 | 0.43 |
| YER042W | 3.0 | 2.2 | 3.1 | 1.0 | 1.1 | 1.6 | 1.1 | 1.7 | 6.8 | 2.1 | 1.8 | 1.1 | 1.4 | 5.2 | 1.0 | 1.9 | 0.7 | 1.1 | 1.80 |

| | | | | | | | | | | | | | | | | | | | |
|---|---|---|---|---|---|---|---|---|---|---|---|---|---|---|---|---|---|---|---|
| YER090W | 1.0 | 1.1 | 2.0 | 0.7 | 1.3 | 0.7 | 0.9 | 1.4 | 3.7 | 2.5 | 1.8 | 1.3 | 1.2 | 1.2 | 1.5 | 0.8 | 1.1 | 0.9 | 0.97 |
| YGL026C | 0.7 | 9.3 | 1.9 | 0.6 | 1.2 | 1.7 | 0.9 | 0.8 | 3.7 | 1.5 | 3.4 | 1.3 | 1.8 | 0.9 | 2.4 | 0.7 | 1.5 | 0.8 | 0.87 |
| YGL252C | 1.1 | 2.4 | 1.4 | 0.7 | 1.3 | 0.8 | 1.4 | 1.2 | 2.7 | 1.7 | 0.9 | 0.9 | 1.2 | 1.0 | 1.3 | 1.2 | 1.0 | 1.3 | 0.91 |
| YGL254W | 1.2 | 1.1 | 0.8 | 0.6 | 1.1 | 0.7 | 1.3 | 1.4 | 3.1 | 1.8 | 1.1 | 1.2 | 2.4 | 1.3 | 1.1 | 1.4 | 0.8 | 1.2 | 0.53 |
| YGR276C | 0.9 | 0.8 | 0.3 | 1.3 | 1.5 | 0.7 | 1.9 | 1.5 | 4.5 | 2.2 | 0.9 | 1.0 | 1.8 | 1.0 | 0.8 | 1.3 | 0.9 | 1.1 | 0.97 |
| YHR106W | 0.9 | 1.7 | 1.7 | 1.5 | 1.7 | 1.3 | 1.2 | 1.0 | 2.8 | 2.5 | 1.4 | 0.7 | 1.0 | 1.2 | 0.9 | 2.4 | 1.3 | 1.1 | 1.73 |
| YIL046W | 1.5 | 2.7 | 1.6 | 0.7 | 1.4 | 0.9 | 1.0 | 1.0 | 10.7 | 2.5 | 1.2 | 1.0 | 1.7 | 1.1 | 1.3 | 1.8 | 9.0 | 5.8 | 0.68 |
| YJL071W | 1.0 | 1.5 | 1.3 | 1.6 | 1.4 | 1.0 | 1.3 | 1.1 | 2.8 | 2.2 | 1.3 | 1.1 | 1.0 | 1.0 | 1.5 | 1.2 | 0.9 | 0.9 | 0.41 |
| YJR139C | 1.4 | 2.6 | 2.9 | 1.1 | 1.2 | 1.8 | 1.3 | 1.1 | 4.1 | 1.9 | 1.5 | 1.0 | 1.5 | 1.4 | 0.7 | 1.2 | 1.0 | 1.3 | 4.47 |
| YKR069W | 3.4 | 6.3 | 5.0 | 3.1 | 0.7 | 0.5 | 1.0 | 1.2 | 10.9 | 9.0 | 2.0 | 1.4 | 1.4 | 1.7 | 1.1 | 1.8 | 1.0 | 1.0 | 0.25 |
| YLL061W | 3.6 | 2.8 | 9.2 | 0.4 | 0.8 | 0.8 | 0.9 | 1.4 | 3.7 | 5.5 | 1.7 | 0.7 | 0.9 | 1.3 | 1.4 | 1.0 | 0.6 | 0.7 | 0.33 |
| YLR070C | 1.0 | 1.3 | 1.4 | 1.1 | 0.7 | 1.1 | 1.3 | 1.0 | 3.5 | 1.6 | 0.4 | 0.7 | 2.0 | 1.3 | 0.5 | 2.1 | 1.0 | 0.9 | 0.31 |
| YLR099C | 2.0 | 2.2 | 1.4 | 0.8 | 0.9 | 0.4 | 0.2 | 1.2 | 3.6 | 1.1 | 1.2 | 0.9 | 0.8 | 0.7 | 0.9 | 0.7 | 0.7 | 0.5 | 0.92 |
| YLR157C | 1.1 | 1.1 | 3.4 | 1.1 | 1.3 | 1.9 | 1.9 | 1.5 | 5.1 | 1.8 | 1.9 | 1.2 | 1.8 | 1.1 | 1.1 | 2.1 | 1.6 | 2.1 | 1.70 |
| YLR160C | 1.2 | 1.4 | 3.9 | 1.1 | 1.3 | 1.8 | 1.9 | 2.0 | 4.8 | 1.8 | 1.6 | 1.2 | 2.0 | 1.2 | 1.0 | 2.0 | 1.6 | 2.2 | 1.59 |
| YLR164W | 1.0 | 2.4 | 0.3 | 1.0 | 1.2 | 1.2 | 1.1 | 1.2 | 5.3 | 2.0 | 3.2 | 0.7 | 1.0 | 1.5 | 0.7 | 11.0 | 1.5 | 1.2 | 0.31 |
| YML042W | 0.9 | 1.4 | 1.5 | 2.2 | 1.0 | 0.9 | 0.8 | 1.6 | 3.8 | 2.4 | 2.5 | 1.0 | 1.2 | 1.4 | 1.7 | 4.8 | 1.0 | 0.9 | 0.29 |
| YOL049W | 0.9 | 1.1 | 1.3 | 0.9 | 1.4 | 0.6 | 1.1 | 1.0 | 3.0 | 1.9 | 1.8 | 0.8 | 0.9 | 0.9 | 0.7 | 1.3 | 1.0 | 1.3 | 1.61 |
| YOL064C | 1.4 | 2.1 | 1.1 | 0.6 | 0.9 | 1.4 | 1.2 | 1.0 | 8.7 | 2.7 | 1.0 | 0.9 | 2.0 | 0.9 | 1.0 | 1.7 | 1.2 | 1.3 | 1.32 |
| YOR377W | 0.6 | 0.6 | 0.6 | 1.0 | 1.4 | 1.6 | 1.6 | 1.0 | 3.8 | 1.1 | 1.2 | 0.9 | 1.3 | 0.9 | 1.2 | 1.3 | 1.1 | 1.1 | 0.46 |
| YPL031C | 0.9 | 2.0 | 1.4 | 0.8 | 1.5 | 1.8 | 1.6 | 1.0 | 5.7 | 1.4 | 1.4 | 0.8 | 1.6 | 0.9 | 0.7 | 2.0 | 1.4 | 1.2 | 0.49 |
| YPL113C | 1.4 | 1.7 | 0.4 | 2.4 | 1.3 | 1.0 | 1.9 | 1.4 | 5.5 | 2.0 | 1.5 | 1.1 | 1.7 | 1.6 | 0.9 | 2.5 | 1.2 | 1.7 | 0.28 |
| YPL274W | 0.8 | 4.2 | 3.8 | 0.8 | 0.6 | 1.1 | 0.7 | 0.9 | 4.1 | 3.6 | 2.0 | 0.5 | 0.6 | 0.9 | 1.5 | 1.1 | 0.7 | 0.6 | 0.41 |
| YPR048W | 1.3 | 2.0 | 0.9 | 1.1 | 0.9 | 0.6 | 0.9 | 1.5 | 4.0 | 2.1 | 1.0 | 0.8 | 0.6 | 1.2 | 0.6 | 0.8 | 0.7 | 0.8 | 0.75 |
| YBR001C | 0.9 | 1.4 | 1.1 | 1.4 | 0.9 | 1.1 | 0.9 | 1.3 | 2.3 | 2.1 | 1.0 | 1.0 | 2.0 | 1.2 | 1.8 | 1.4 | 1.0 | 1.6 | 0.61 |
| YBR018C | 0.9 | 1.1 | 1.9 | 2.9 | 0.9 | 0.7 | 1.4 | 0.6 | 1.3 | 1.7 | 5.8 | 1.0 | 1.0 | 2.4 | 1.3 | 6.2 | 0.8 | 0.9 | 0.20 |
| YBR204C | 1.0 | 1.0 | 1.5 | 1.8 | 1.1 | 0.9 | 1.7 | 1.1 | 1.8 | 1.9 | 2.2 | 1.0 | 1.7 | 1.4 | 1.2 | 2.3 | 1.1 | 1.5 | 0.84 |
| YBR241C | 0.9 | 9.3 | 2.6 | 0.9 | 0.9 | 1.5 | 1.4 | 0.7 | 29.8 | 2.2 | 1.4 | 1.4 | 1.4 | 0.9 | 1.6 | 0.7 | 1.0 | 1.2 | 1.25 |
| YCR105W | 2.2 | 1.2 | 1.2 | 3.0 | 0.9 | 1.0 | 1.0 | 1.4 | 2.0 | 3.9 | 2.3 | 0.9 | 1.9 | 1.9 | 3.1 | 1.3 | 1.3 | 1.1 | 0.36 |
| YDR287W | 1.1 | 8.4 | 1.9 | 1.2 | 1.2 | 1.6 | 1.2 | 1.2 | 2.5 | 1.8 | 1.5 | 1.1 | 1.5 | 1.2 | 1.6 | 2.7 | 1.5 | 1.3 | 0.52 |
| YDR294C | 0.7 | 1.1 | 1.9 | 1.2 | 0.9 | 0.7 | 1.0 | 1.0 | 2.0 | 2.9 | 1.2 | 1.1 | 1.6 | 1.0 | 2.0 | 1.7 | 0.9 | 1.1 | 1.02 |

| | | | | | | | | | | | | | | | | | | | |
|---|---|---|---|---|---|---|---|---|---|---|---|---|---|---|---|---|---|---|---|
| YER052C | 1.1 | 1.0 | 3.6 | 1.1 | 0.6 | 0.6 | 0.5 | 1.0 | 2.7 | 2.4 | 1.1 | 0.9 | 2.1 | 0.7 | 1.2 | 0.2 | 0.8 | 0.4 | 1.54 |
| YFL021W | 0.8 | 1.4 | 2.4 | 1.7 | 1.1 | 0.8 | 0.8 | 0.6 | 2.7 | 1.2 | 1.3 | 0.9 | 1.5 | 1.1 | 1.3 | 1.0 | 0.8 | 0.5 | 0.40 |
| YGL040C | 0.7 | 1.2 | 1.4 | 0.9 | 1.3 | 2.0 | 1.1 | 1.0 | 2.1 | 1.9 | 0.9 | 0.6 | 1.6 | 1.3 | 0.7 | 1.5 | 0.7 | 1.0 | 1.77 |
| YGL125W | 1.0 | 3.2 | 4.3 | 0.7 | 0.9 | | 1.7 | 0.8 | 2.1 | 2.1 | 1.6 | 1.0 | 1.4 | 1.0 | 1.9 | 1.2 | 1.2 | 1.1 | 0.27 |
| YGR007W | 1.3 | 1.7 | 0.7 | 1.6 | 0.6 | 0.8 | 1.2 | 1.1 | 2.2 | 2.3 | 0.9 | 0.8 | 1.4 | 1.8 | 1.9 | 1.3 | 1.1 | 1.4 | 0.74 |
| YGR155W | 1.3 | 4.4 | 1.2 | 0.6 | 1.3 | 1.6 | 1.1 | 1.4 | 2.4 | 1.7 | 1.3 | 1.0 | 1.4 | 0.7 | 0.5 | 1.0 | 0.5 | 0.6 | 4.15 |
| YIL099W | 0.9 | 1.6 | 1.5 | 0.4 | 0.8 | | 101.4 | 1.0 | 2.2 | 1.8 | 0.9 | 0.9 | 1.4 | 1.6 | 5.5 | 1.2 | 1.0 | 1.1 | 0.18 |
| YIL170W | 1.1 | 1.0 | 2.5 | 2.2 | 0.9 | 8.8 | 0.5 | 1.2 | 5.7 | 3.2 | 1.7 | 0.7 | 1.9 | 1.1 | 2.3 | 1.8 | 1.6 | 1.5 | 0.48 |
| YIR031C | 0.8 | 1.6 | 0.6 | 0.6 | 1.6 | 1.7 | 1.4 | 1.0 | 2.6 | 1.0 | 1.2 | 0.8 | 1.0 | 1.2 | 0.8 | 0.7 | 0.9 | 0.8 | 0.44 |
| YIR032C | 1.1 | 1.9 | 1.3 | 2.6 | 1.0 | 0.6 | 1.0 | 1.2 | 2.6 | 2.3 | 1.4 | 1.0 | 1.4 | 1.6 | 0.6 | 1.6 | 0.9 | 1.1 | 0.40 |
| YJL128C | 0.8 | 1.4 | 0.4 | 0.8 | 1.2 | 1.3 | 1.8 | 0.9 | 2.0 | 1.7 | 0.6 | 1.0 | 1.7 | 0.8 | 1.2 | 0.9 | 1.0 | 1.0 | 0.42 |
| YJR090C | 0.8 | 1.0 | 0.6 | 0.8 | 1.2 | 1.2 | 0.8 | 0.8 | 1.9 | 1.2 | 0.4 | 0.8 | 2.2 | 1.0 | 1.6 | 0.8 | 1.1 | 1.0 | 0.60 |
| YJR103W | 1.1 | 3.8 | 1.2 | 1.6 | 0.7 | 0.6 | 0.6 | 0.8 | 1.8 | 3.0 | 1.1 | 0.6 | 0.7 | 0.7 | 3.2 | 2.3 | 0.9 | 1.0 | 0.74 |
| YJR153W | 1.1 | 1.7 | 1.1 | 0.8 | 0.9 | 0.4 | 1.3 | 0.9 | 2.4 | 1.6 | 2.0 | 0.7 | 1.3 | 1.7 | 2.9 | 1.6 | 0.8 | 1.0 | 0.18 |
| YKL192C | 1.1 | 1.0 | 3.7 | 1.1 | 1.1 | 1.8 | 1.1 | 0.9 | 3.5 | 1.6 | 1.1 | 1.2 | 1.7 | 1.5 | 1.2 | 2.1 | 1.0 | 0.8 | 1.57 |
| YLR025W | 1.2 | 5.1 | 0.7 | 1.1 | 1.9 | 1.5 | 1.7 | 1.5 | 2.4 | 1.5 | 1.1 | 0.9 | 1.0 | 1.7 | 1.5 | 2.2 | 1.2 | 1.3 | 1.15 |
| YML051W | 1.0 | 1.4 | 1.8 | 0.9 | 1.0 | 0.7 | 0.9 | 0.9 | 2.0 | 1.5 | 1.4 | 0.7 | 1.2 | 1.1 | 1.4 | 1.1 | 0.7 | 1.0 | 0.57 |
| YML099C | 0.6 | 1.0 | 1.1 | 1.3 | 1.0 | 0.5 | 1.0 | 0.7 | 2.2 | 1.7 | 1.6 | 1.1 | 1.3 | 0.9 | 1.0 | 1.6 | 0.7 | 0.8 | 0.48 |
| YMR056C | 1.1 | 1.3 | 1.7 | 1.0 | 1.1 | 0.9 | 1.4 | 1.2 | 1.8 | 1.1 | 1.6 | 0.9 | 0.9 | 1.1 | 0.7 | 2.4 | 1.0 | 1.1 | 0.72 |
| YNL257C | 0.7 | 1.0 | 0.9 | 0.8 | 0.9 | 2.0 | 1.1 | 1.1 | 2.1 | 1.4 | 1.0 | 1.1 | 1.7 | 0.8 | 1.0 | 1.4 | 0.9 | 1.3 | 0.63 |
| YNL264C | 1.0 | 2.4 | 1.3 | 1.0 | 0.8 | 1.4 | 1.5 | 1.1 | 3.3 | 1.3 | 1.0 | 1.1 | 1.5 | 1.2 | 1.1 | 1.3 | 0.7 | 1.1 | 0.48 |
| YNR071C | 1.4 | 1.0 | 0.9 | 3.2 | 1.1 | 0.9 | 1.4 | 1.2 | 3.1 | 7.0 | 1.3 | 0.6 | 1.7 | 1.1 | 1.8 | 1.0 | 1.0 | 0.9 | 0.16 |
| YOL065C | 1.0 | 1.7 | 1.6 | 1.3 | 0.8 | 1.3 | 1.9 | 1.0 | 2.1 | 1.8 | 1.0 | 0.8 | 1.3 | 1.1 | 0.7 | 2.6 | 1.1 | 1.5 | 0.43 |
| YOL067C | 0.8 | 0.9 | 0.9 | 1.4 | 0.7 | 0.3 | 0.7 | 1.0 | 2.1 | 1.3 | 1.3 | 0.7 | 1.7 | 0.7 | 1.2 | 1.1 | 1.4 | 0.9 | 0.55 |
| YPL147W | 1.0 | 0.8 | 2.3 | 1.2 | 1.2 | 1.1 | 1.0 | 0.9 | 2.4 | 2.4 | 1.3 | 0.9 | 1.5 | 1.0 | 2.4 | 2.5 | 1.5 | 3.6 | 0.33 |
| YDR043C | 2.8 | 1.1 | 1.2 | 6.9 | 1.7 | 1.4 | 1.3 | 1.2 | 1.0 | 2.7 | 1.3 | 0.9 | 0.7 | 1.8 | 1.2 | 1.4 | 1.9 | 1.2 | 0.66 |
| YGR180C | 3.1 | 1.1 | 1.9 | 1.0 | 2.0 | 0.6 | 0.5 | 1.0 | 1.4 | 1.5 | 1.0 | 0.9 | 0.9 | 0.9 | 1.3 | 1.2 | 1.0 | 1.9 | 3.90 |
| YJL026W | 2.5 | 2.4 | 2.9 | 1.0 | 1.7 | 1.4 | 0.5 | 1.2 | 1.0 | 1.1 | 1.3 | 1.0 | 1.2 | 1.0 | 1.1 | 1.6 | 1.4 | 2.1 | 3.74 |
| YGR087C | 1.0 | 15.0 | 1.7 | 0.6 | 1.0 | 1.1 | 1.0 | 0.8 | 0.2 | 0.9 | 0.8 | 1.2 | 1.2 | 0.7 | 3.5 | 0.6 | 1.0 | 0.6 | 1.88 |
| YGL256W | 0.9 | 5.3 | 1.1 | 1.3 | 0.7 | 0.7 | 0.5 | 1.0 | 0.4 | 1.2 | 1.0 | 0.9 | 0.8 | 0.9 | 1.0 | 0.7 | 0.7 | 0.8 | 0.90 |
| YAL039C | 1.1 | 2.2 | 1.7 | 1.0 | 1.1 | 0.8 | 1.0 | 1.2 | 1.6 | 2.2 | 1.1 | 1.4 | 1.6 | 1.2 | 3.3 | 2.2 | 1.4 | 1.1 | 0.39 |

| | | | | | | | | | | | | | | | | | | | |
|---|---|---|---|---|---|---|---|---|---|---|---|---|---|---|---|---|---|---|---|
| YJR107W | 1.0 | 3.1 | 1.5 | 1.8 | 0.7 | 1.5 | 1.3 | 1.3 | 1.4 | 0.9 | 1.3 | 1.1 | 1.7 | 1.4 | 1.1 | 1.1 | 1.4 | 1.3 | 0.41 |
| YNL142W | 0.7 | 2.4 | 2.5 | 0.8 | 1.3 | 0.9 | 1.1 | 0.8 | 0.9 | 0.6 | 0.9 | 1.2 | 0.9 | 0.8 | 0.3 | 1.0 | 1.0 | 0.9 | 0.44 |
| YDL210W | 1.2 | 2.2 | 2.7 | 1.1 | 0.9 | 1.3 | 0.8 | 1.1 | 1.2 | 1.4 | 0.6 | 0.8 | 1.2 | 1.1 | 1.9 | 0.9 | 1.1 | 0.9 | 0.20 |
| YGL055W | 1.1 | 1.8 | 0.3 | 2.5 | 1.5 | 1.6 | 0.6 | 1.4 | 0.2 | 0.7 | 0.9 | 1.3 | 0.2 | 0.7 | 0.5 | 3.0 | 1.0 | 1.5 | 5.60 |
| YCL025C | 1.1 | 4.6 | 2.5 | 1.7 | 0.9 | 0.9 | 0.7 | 0.7 | 0.3 | 0.3 | 1.0 | 1.1 | 0.5 | 0.7 | 0.8 | 0.6 | 0.6 | 0.6 | 1.98 |
| YBR132C | 0.9 | 1.9 | 2.4 | 1.8 | 0.8 | 1.2 | 1.1 | 0.8 | 0.8 | 1.1 | 1.9 | 1.0 | 1.4 | 1.1 | 1.5 | 1.3 | 1.1 | 1.3 | 0.43 |
| YHL018W | 0.8 | 1.5 | 0.5 | 0.5 | 0.9 | 0.4 | 1.2 | 0.8 | 0.8 | 0.6 | 1.2 | 0.7 | 0.8 | 1.0 | 0.9 | 0.9 | 1.0 | 0.9 | 0.37 |
| YPL038W | 0.8 | 1.5 | 0.2 | 1.1 | 1.4 | 0.5 | 1.0 | 1.2 | 0.9 | 0.7 | 1.1 | 0.6 | 0.6 | 0.9 | 0.6 | 0.8 | 0.9 | 0.9 | 0.64 |
| YKR053C | 0.9 | 2.0 | 1.0 | 1.2 | 1.0 | 1.1 | 1.7 | 0.9 | 0.8 | 1.8 | 0.5 | 0.7 | 0.7 | 1.3 | 0.3 | 0.9 | 2.8 | 2.4 | 0.35 |
| YNL256W | 0.7 | 1.6 | 0.6 | 0.6 | 1.0 | 0.5 | 0.6 | 0.9 | 0.8 | 0.6 | 0.6 | 0.6 | 1.3 | 0.7 | 0.9 | 0.4 | 0.6 | 0.6 | 1.09 |
| YLR377C | 1.1 | 2.6 | 1.8 | 1.2 | 1.0 | 0.7 | 0.9 | 0.7 | 1.8 | 1.6 | 0.6 | 0.6 | 1.2 | 2.9 | 0.8 | 2.7 | 0.9 | 1.4 | 0.14 |
| YBR253W | 1.3 | 4.4 | 0.4 | 1.1 | 1.4 | 1.3 | 1.4 | 1.8 | 1.8 | 1.5 | 1.1 | 1.0 | 0.8 | 1.2 | 0.9 | 1.3 | 1.3 | 1.1 | 0.96 |
| YBL030C | 1.0 | 0.9 | 4.5 | 0.9 | 0.7 | 1.1 | 0.9 | 0.8 | 0.4 | 0.9 | 1.1 | 0.9 | 0.5 | 0.8 | 1.8 | 1.0 | 1.0 | 1.1 | 3.12 |
| YBR221C | 0.8 | 0.7 | 3.2 | 1.5 | 1.3 | 1.7 | 1.5 | 0.8 | 1.0 | 1.3 | 1.2 | 1.3 | 1.7 | 0.8 | 1.1 | 1.5 | 0.9 | 1.0 | 3.62 |
| YDR342C | 2.8 | 1.1 | 12.2 | 5.7 | 1.6 | 1.1 | 0.8 | 1.2 | 0.2 | 2.2 | 2.9 | 1.0 | 0.6 | 0.9 | 0.5 | 2.4 | 1.0 | 2.2 | 5.23 |
| YDR343C | 1.2 | 1.0 | 20.6 | 4.6 | 1.3 | 1.3 | 0.7 | 1.2 | 0.3 | 2.1 | 2.3 | 1.0 | 0.8 | 0.8 | 0.5 | 2.8 | 1.1 | 2.3 | 5.81 |
| YEL034W | 0.9 | 0.6 | 3.3 | 0.9 | 1.4 | 0.9 | 0.4 | 0.9 | 0.8 | 1.0 | 1.1 | 0.9 | 0.8 | 0.9 | 0.8 | 0.9 | 0.9 | 0.9 | 5.44 |
| YHR094C | 0.7 | 1.2 | 5.3 | 1.6 | 1.1 | 1.6 | 0.8 | 1.2 | 0.3 | 1.2 | 0.6 | 0.9 | 0.7 | 0.6 | 2.7 | 0.9 | 1.4 | 1.4 | 4.82 |
| YIL162W | 1.3 | 1.5 | 6.8 | 2.4 | 1.2 | 1.0 | 1.2 | 1.6 | 0.8 | 3.7 | 2.8 | 2.0 | 1.0 | 1.0 | 1.5 | 1.7 | 0.9 | 1.4 | 1.22 |
| YJR105W | 0.6 | 0.7 | 3.0 | 0.8 | 0.7 | 0.3 | 0.5 | 0.8 | 0.9 | 0.5 | 1.0 | 0.8 | 0.8 | 0.5 | 1.2 | 0.7 | 1.1 | 0.9 | 3.75 |
| YLR134W | 0.8 | 0.6 | 2.3 | 0.8 | 1.3 | 2.5 | 1.1 | 0.8 | 0.1 | 0.7 | 1.1 | 1.2 | 1.5 | 0.6 | 1.7 | 0.5 | 0.6 | 0.8 | 3.47 |
| YLR258W | 1.5 | 1.0 | 4.2 | 3.5 | 0.9 | 1.8 | 1.8 | 0.9 | 0.8 | 1.3 | 1.2 | 0.9 | 1.4 | 1.2 | 0.6 | 1.7 | 1.0 | 2.0 | 1.36 |
| YML058W | 1.9 | 1.2 | 5.8 | 1.9 | 0.6 | 0.6 | 0.6 | 0.7 | 1.1 | 1.2 | 3.3 | 0.9 | 1.4 | 1.1 | 2.5 | 1.3 | 1.3 | 1.5 | 2.14 |
| YMR083W | 1.4 | 1.7 | 2.6 | 1.1 | 1.5 | 1.8 | 1.7 | 1.1 | 0.4 | 1.1 | 1.6 | 0.7 | 0.9 | 0.8 | 1.3 | 1.3 | 0.9 | 1.1 | 2.52 |
| YOR178C | 1.4 | 1.3 | 4.8 | 2.3 | 1.0 | 2.7 | 0.9 | 0.9 | 0.2 | 1.7 | 4.1 | 1.7 | 1.0 | 1.0 | 1.1 | 1.7 | 1.0 | 1.5 | 0.56 |
| YPL028W | 0.7 | 0.9 | 2.6 | 0.9 | 1.0 | 1.2 | 1.3 | 0.9 | 1.2 | 0.8 | 1.2 | 1.4 | 0.9 | 0.8 | 0.6 | 2.2 | 1.4 | 1.8 | 4.35 |
| YPR113W | 1.1 | 0.8 | 3.7 | 1.3 | 2.0 | 1.1 | 1.2 | 0.9 | 0.4 | 0.8 | 2.0 | 0.7 | 0.7 | 1.3 | 1.0 | 2.3 | 1.2 | 1.9 | 2.85 |
| YPR183W | 0.9 | 1.5 | 3.5 | 0.8 | 0.9 | 1.2 | 1.2 | 1.0 | 0.9 | 0.6 | 2.5 | 1.2 | 1.6 | 0.7 | 0.9 | 0.8 | 1.2 | 1.0 | 1.17 |
| YBR011C | 1.3 | 1.8 | 2.5 | 1.1 | 0.9 | 2.0 | 1.1 | 1.3 | 2.2 | 1.0 | 1.7 | 1.1 | 1.5 | 0.9 | 2.1 | 1.7 | 0.8 | 1.1 | 3.68 |
| YCR034W | 0.8 | 0.4 | 2.5 | 0.9 | 0.9 | 0.3 | 0.3 | 0.9 | 0.1 | 0.2 | 0.8 | 0.5 | 0.2 | 0.6 | 0.9 | 0.3 | 0.9 | 0.7 | 3.77 |
| YDR050C | 1.6 | 1.3 | 2.3 | 1.8 | 0.9 | 1.7 | 1.5 | 1.3 | 0.4 | 1.3 | 2.0 | 1.4 | 2.0 | 1.2 | 1.9 | 1.3 | 1.1 | 2.3 | 6.26 |

| | | | | | | | | | | | | | | | | | | | |
|---|---|---|---|---|---|---|---|---|---|---|---|---|---|---|---|---|---|---|---|
| YDR178W | 2.0 | 2.0 | 3.4 | 2.2 | 0.9 | 2.1 | 0.6 | 0.9 | 0.9 | 0.8 | 2.0 | 1.3 | 1.5 | 1.5 | 0.7 | 3.0 | 1.2 | 2.3 | 2.27 |
| YDR284C | 0.8 | 1.5 | 3.0 | 2.9 | 0.8 | 1.8 | 1.4 | 0.9 | 0.9 | 1.0 | 1.3 | 0.9 | 1.2 | 1.3 | 0.9 | 1.7 | 1.5 | 1.5 | 1.44 |
| YDR345C | 0.8 | 0.9 | 5.6 | 2.6 | 1.3 | 1.2 | 1.1 | 1.2 | 0.2 | 1.1 | 1.5 | 1.4 | 1.2 | 0.8 | 0.8 | 1.0 | 1.3 | 1.8 | 5.65 |
| YDR400W | 1.0 | 2.0 | 2.2 | 0.7 | 1.0 | 1.1 | 0.6 | 1.4 | 1.1 | 1.4 | 1.3 | 0.4 | 0.7 | 0.9 | 0.8 | 1.1 | 1.1 | 0.8 | 0.56 |
| YEL063C | 0.7 | 0.8 | 2.4 | 1.3 | 1.2 | 1.3 | 0.7 | 0.6 | 0.8 | 1.7 | 0.8 | 1.1 | 1.1 | 0.8 | 1.3 | 1.0 | 0.7 | 0.7 | 1.12 |
| YER081W | 1.6 | 1.9 | 2.7 | 1.4 | 1.1 | 1.1 | 0.5 | 0.6 | 1.8 | 1.1 | 1.1 | 1.0 | 0.6 | 1.6 | 0.5 | 0.8 | 1.1 | 1.7 | 2.31 |
| YER120W | 0.9 | 1.1 | 2.5 | 0.8 | 0.8 | 1.5 | 0.9 | 0.6 | 0.8 | 0.9 | 1.1 | 1.3 | 1.0 | 0.9 | 1.5 | 1.0 | 1.2 | 1.0 | 1.52 |
| YFL011W | 1.2 | 0.7 | 3.7 | 3.3 | 1.3 | 1.0 | 0.8 | 0.8 | 0.3 | 1.6 | 2.1 | 0.9 | 0.8 | 0.8 | 1.7 | 1.2 | 0.8 | 1.0 | 1.25 |
| YGL012W | 1.0 | 0.8 | 3.0 | 1.2 | 1.5 | 1.6 | 0.9 | 0.8 | 0.3 | 0.9 | 0.6 | 0.6 | 0.3 | 0.8 | 0.6 | 1.2 | 0.9 | 1.1 | 4.88 |
| YGR191W | 0.8 | 0.7 | 1.6 | 1.5 | 0.8 | 1.1 | 0.7 | 0.7 | 0.1 | 0.7 | 1.6 | 0.7 | 1.0 | 0.7 | 1.5 | 0.7 | 0.8 | 0.8 | 1.58 |
| YGR204W | 0.5 | 1.1 | 2.7 | 1.3 | 0.6 | 0.6 | 0.8 | 0.4 | 0.9 | 2.3 | 2.1 | 1.0 | 1.3 | 0.5 | 1.6 | 0.6 | 1.0 | 0.7 | 1.49 |
| YHR025W | 0.6 | 0.8 | 2.4 | 0.6 | 0.9 | 0.8 | 0.8 | 0.8 | 0.2 | 0.4 | 2.1 | 0.9 | 0.7 | 0.6 | 2.1 | 0.5 | 1.0 | 0.6 | 0.68 |
| YHR123W | 0.7 | 1.2 | 1.8 | 1.0 | | 1.0 | 0.9 | 0.8 | 0.8 | 1.1 | 1.2 | 0.7 | 1.5 | 0.8 | 2.3 | 0.5 | 1.1 | 1.0 | 0.85 |
| YJL121C | 1.0 | 0.5 | 1.9 | 0.8 | 1.0 | 1.1 | 0.7 | 1.0 | 0.1 | 0.4 | 1.1 | 0.6 | 0.7 | 0.9 | 1.0 | 0.6 | 1.1 | 1.0 | 1.00 |
| YJR077C | 1.1 | 1.1 | 2.0 | 1.6 | 0.9 | 0.9 | 0.7 | 0.8 | 0.4 | 0.7 | 1.6 | 1.0 | 0.9 | 0.7 | 1.5 | 0.7 | 1.0 | 0.8 | 1.79 |
| YJR143C | 0.6 | 0.6 | 2.1 | 0.8 | 1.6 | 0.6 | 0.5 | 0.7 | 0.1 | 0.3 | 1.3 | 0.6 | 0.3 | 0.6 | 1.1 | 0.5 | 0.7 | 0.8 | 3.24 |
| YKL060C | 1.6 | 0.8 | 2.5 | 1.0 | 1.6 | 1.3 | 1.2 | 1.1 | 0.3 | 0.8 | 2.1 | 1.3 | 1.2 | 0.8 | 1.6 | 1.5 | 1.2 | 1.7 | 6.01 |
| YKL148C | 0.9 | 0.8 | 3.7 | 0.7 | 0.8 | 0.5 | 0.6 | 0.8 | 1.7 | 1.7 | 1.5 | 0.8 | 0.8 | 0.8 | 0.6 | 1.1 | 0.8 | 1.0 | 0.54 |
| YKL157W | 0.7 | 1.0 | 2.3 | 1.3 | 1.5 | 1.5 | 1.9 | 1.1 | 1.7 | 1.9 | 1.5 | 1.0 | 2.3 | 0.8 | 1.2 | 1.7 | 1.2 | 1.5 | 1.22 |
| YLR044C | 0.8 | 0.6 | 2.2 | 0.9 | 1.7 | 1.5 | 1.3 | 0.7 | 0.0 | 1.2 | 1.1 | 1.4 | 1.7 | 0.5 | 2.2 | 0.6 | 0.9 | 0.9 | 5.16 |
| YLR056W | 1.0 | 0.6 | 2.5 | 0.8 | 1.6 | 1.2 | 0.8 | 0.9 | 0.0 | 0.5 | 0.8 | 0.9 | 0.2 | 0.7 | 0.9 | 2.1 | 1.1 | 1.1 | 3.61 |
| YLR058C | 0.9 | 0.8 | 5.5 | 2.6 | 0.7 | 0.9 | 0.5 | 0.3 | 0.2 | 0.1 | 2.6 | 0.4 | 0.3 | 0.7 | 1.5 | 0.4 | 0.8 | 0.8 | 2.71 |
| YLR081W | 1.3 | 0.8 | 2.8 | 3.6 | 1.0 | 0.9 | 0.7 | 0.9 | 0.1 | 2.2 | 2.6 | 1.0 | 0.6 | 1.0 | 0.7 | 1.2 | 0.7 | 0.9 | 1.46 |
| YLR089C | 0.9 | 1.2 | 2.1 | 0.9 | 0.8 | 1.5 | 1.6 | 0.7 | 0.5 | 0.8 | 1.7 | 0.6 | 1.0 | 0.7 | 1.7 | 1.4 | 1.2 | 1.3 | 1.27 |
| YLR284C | 0.9 | 1.0 | 3.8 | 1.9 | 1.6 | 0.9 | 0.8 | 1.2 | 0.9 | 0.9 | 1.5 | 0.6 | 0.8 | 1.3 | 1.0 | 4.5 | 2.9 | 8.1 | 0.84 |
| YLR304C | 0.7 | 0.6 | 5.0 | 0.7 | 0.6 | 1.9 | 0.6 | 0.6 | 0.1 | 2.2 | 1.6 | 1.0 | 0.5 | 0.7 | 1.6 | 1.8 | 0.5 | 0.7 | 2.39 |
| YLR354C | 1.1 | 2.5 | 2.3 | 1.5 | 1.6 | 1.5 | 1.1 | 1.4 | 0.4 | 0.9 | 1.7 | 1.2 | 1.0 | 0.9 | 2.4 | 1.3 | 1.0 | 1.5 | 4.53 |
| YLR372W | 0.7 | 0.3 | 1.7 | 0.8 | 1.4 | 0.5 | 0.2 | 0.9 | 0.0 | 0.1 | 0.9 | 0.6 | 0.1 | 0.6 | 0.7 | 0.2 | 0.5 | 0.6 | 4.46 |
| YML022W | 0.9 | 0.6 | 2.0 | 1.1 | 1.3 | 0.8 | 0.6 | 1.1 | 0.3 | 0.6 | 1.2 | 0.6 | 0.2 | 0.9 | 0.6 | 0.6 | 0.5 | 0.7 | 4.93 |
| YMR011W | 1.3 | 1.0 | 9.4 | 5.5 | 0.9 | 0.6 | 0.8 | 1.1 | 0.0 | 0.7 | 1.5 | 0.8 | 0.4 | 0.6 | 0.6 | 1.2 | 1.3 | 1.6 | 4.95 |
| YMR015C | 0.7 | 0.7 | 1.9 | 0.9 | 1.7 | 1.3 | 0.4 | 0.7 | 0.1 | 0.3 | 1.0 | 0.9 | 0.4 | 0.8 | 1.2 | 1.5 | 1.0 | 1.0 | 2.51 |

| | | | | | | | | | | | | | | | | | | | |
|---|---|---|---|---|---|---|---|---|---|---|---|---|---|---|---|---|---|---|---|
| YMR205C | 0.5 | 0.7 | 2.3 | 0.8 | 1.2 | 1.2 | 0.9 | 0.7 | 0.3 | 0.8 | 1.0 | 1.1 | 1.3 | 0.5 | 0.9 | 0.9 | 0.6 | 0.5 | 4.75 |
| YMR261C | 0.7 | 1.6 | 3.6 | 0.8 | 0.9 | 0.6 | 1.3 | 0.7 | 1.6 | 1.3 | 0.8 | 0.9 | 1.6 | 0.6 | 0.8 | 1.6 | 0.7 | 1.1 | 0.78 |
| YMR323W | 0.8 | 1.1 | 2.9 | 1.1 | 0.7 | 0.4 | 1.1 | 0.8 | 0.5 | 1.3 | 2.5 | 1.2 | 1.8 | 1.2 | 37.3 | 0.6 | 1.1 | 0.6 | 1.04 |
| YOL086C | 1.1 | 0.5 | 2.2 | 1.1 | 1.9 | 1.7 | 1.9 | 0.8 | 0.1 | 1.2 | 2.6 | 1.3 | 1.7 | 0.6 | 1.6 | 1.4 | 1.1 | 1.3 | 4.19 |
| YOL156W | 1.1 | 0.7 | 2.5 | 1.1 | 1.2 | 1.2 | 1.0 | 1.0 | 2.3 | 2.1 | 1.1 | 0.9 | 1.7 | 0.9 | 1.9 | 0.9 | 0.9 | 0.9 | 0.53 |
| YOR002W | 0.8 | 1.1 | 1.9 | 0.9 | 1.1 | 1.1 | 1.0 | 0.9 | 0.8 | 0.9 | 1.2 | 0.5 | 0.6 | 0.9 | 1.5 | 0.8 | 0.9 | 1.1 | 1.49 |
| YOR085W | 0.7 | 0.8 | 1.9 | 1.0 | 1.0 | 0.6 | 0.6 | 0.7 | 0.5 | 0.6 | 1.1 | 0.8 | 1.0 | 0.7 | 1.8 | 0.8 | 0.9 | 0.9 | 1.86 |
| YOR108W | 0.8 | 0.9 | 2.3 | 0.6 | 1.1 | 1.6 | 1.2 | 0.9 | 0.4 | 1.1 | 1.4 | 0.7 | 1.3 | 0.9 | 0.7 | 1.0 | 1.1 | 1.1 | 1.07 |
| YOR128C | 0.9 | 0.7 | 2.1 | 2.1 | 1.1 | 1.0 | 0.7 | 0.6 | 0.3 | 0.4 | 0.8 | 0.5 | 0.4 | 0.7 | 1.6 | 0.5 | 1.2 | 1.2 | 2.14 |
| YOR142W | 1.0 | 1.2 | 3.4 | 1.0 | 1.4 | 1.6 | 1.0 | 0.8 | 1.1 | 1.5 | 1.5 | 0.8 | 1.4 | 0.8 | 1.1 | 0.8 | 0.9 | 1.0 | 1.31 |
| YOR176W | 0.7 | 2.6 | 2.7 | 0.9 | 1.0 | 0.9 | 0.5 | 1.0 | 1.0 | 0.5 | 1.4 | 1.3 | 1.5 | 0.8 | 0.8 | 1.4 | 1.2 | 2.1 | 1.23 |
| YPL057C | 1.9 | 1.1 | 3.2 | 3.1 | 1.9 | 0.6 | 0.6 | 1.0 | 0.4 | 1.4 | 1.6 | 1.0 | 1.3 | 0.7 | 1.1 | 2.5 | 2.2 | 2.0 | 2.08 |
| YPL135W | 0.9 | 1.2 | 2.5 | 1.2 | 1.6 | 1.3 | 1.2 | 1.0 | 0.5 | 2.8 | 1.3 | 1.1 | 1.2 | 1.1 | 1.5 | 1.2 | 1.5 | 1.7 | 1.50 |
| YCR010C | 1.6 | 1.6 | 1.6 | 4.2 | 1.7 | 1.3 | 1.3 | 0.8 | 0.9 | 4.7 | 1.1 | 0.8 | 1.0 | 1.2 | 1.2 | 2.8 | 1.9 | 1.5 | 0.26 |
| YBR003W | 0.9 | 0.8 | 1.0 | 1.9 | 0.7 | 1.2 | 1.1 | 0.9 | 1.0 | 1.3 | 1.3 | 0.8 | 1.2 | 0.8 | 0.7 | 2.0 | 1.1 | 1.3 | 1.06 |
| YBR020W | 1.0 | 0.5 | 1.9 | 1.9 | 1.0 | 0.8 | 0.7 | 1.0 | -0.4 | 1.1 | 1.0 | 0.8 | 1.1 | 1.0 | 1.3 | 0.7 | 0.8 | 0.9 | 0.27 |
| YDR123C | 1.2 | 0.8 | 0.4 | 2.1 | 1.6 | 0.7 | 0.9 | 1.3 | 0.5 | 0.6 | 0.6 | 0.5 | 0.9 | 0.7 | 1.2 | 0.8 | 1.3 | 1.0 | 0.30 |
| YDR277C | 1.6 | 1.2 | 1.0 | 3.0 | 0.8 | 1.1 | 1.2 | 1.7 | 0.2 | 0.9 | 1.2 | 0.9 | 1.2 | 1.3 | 0.4 | 2.3 | 1.2 | 1.7 | 0.89 |
| YDR408C | 1.0 | 1.0 | 1.4 | 4.1 | 0.9 | 0.9 | 0.8 | 0.7 | 0.3 | 0.5 | 1.7 | 0.6 | 0.5 | 0.9 | 1.0 | 1.0 | 1.1 | 1.1 | 2.16 |
| YDR483W | 1.1 | 1.0 | 1.3 | 2.0 | 0.7 | 1.5 | 0.8 | 1.0 | 0.4 | 1.1 | 1.4 | 0.8 | 1.3 | 0.8 | 1.5 | 1.2 | 1.2 | 1.4 | 3.24 |
| YGL115W | 0.9 | 0.6 | 1.2 | 2.0 | 1.2 | 1.2 | 1.4 | 1.0 | 1.2 | 1.2 | 1.0 | 0.9 | 0.7 | 1.2 | 0.7 | 1.7 | 0.8 | 1.2 | 2.46 |
| YGR096W | 1.5 | 1.0 | 1.1 | 4.6 | 1.2 | 1.7 | 0.9 | 1.0 | 0.8 | 0.8 | 0.8 | 0.8 | 1.1 | 1.2 | 0.9 | 0.8 | 0.8 | 1.0 | 0.49 |
| YGR288W | 0.9 | 0.5 | 1.0 | 2.6 | 0.8 | | 1.4 | 1.0 | 1.6 | 2.6 | 1.3 | 0.9 | 1.3 | 1.1 | 0.8 | 1.7 | 1.2 | 1.3 | 0.41 |
| YHR210C | 1.3 | 1.0 | 0.6 | 5.2 | 1.8 | 0.7 | 1.9 | 1.3 | -0.1 | 0.9 | 1.5 | 0.5 | 0.4 | 1.0 | 0.5 | 1.5 | 1.4 | 1.2 | 0.33 |
| YIL006W | 1.0 | 1.1 | 1.4 | 2.9 | 0.8 | 0.3 | 0.8 | 0.6 | 0.9 | 1.1 | 1.0 | 0.9 | 0.8 | 1.0 | 1.5 | 1.7 | 0.8 | 0.9 | 0.28 |
| YKR034W | 0.9 | 1.5 | 1.1 | 4.7 | 0.9 | 0.9 | 0.7 | 1.5 | 0.5 | 0.6 | 0.0 | 0.7 | 1.2 | 1.1 | 1.7 | 0.6 | 0.8 | 1.0 | 0.26 |
| YLR006C | 0.7 | 0.7 | 0.9 | 6.2 | 1.6 | 0.9 | 1.1 | 0.9 | 1.1 | 1.1 | 1.5 | 0.7 | 0.8 | 1.0 | 0.9 | 0.8 | 1.6 | 1.0 | 0.40 |
| YNL025C | 0.9 | 1.4 | 1.4 | 12.8 | 0.7 | 0.5 | 1.2 | 1.0 | 1.1 | 1.8 | 1.1 | 0.8 | 1.3 | 1.2 | 1.4 | 2.9 | 0.7 | 1.3 | 0.37 |
| YOL116W | 1.1 | 1.0 | 0.9 | 2.8 | 1.7 | 0.7 | 1.5 | 1.3 | 0.6 | 1.8 | 1.0 | 0.6 | 0.8 | 0.9 | 0.7 | 1.9 | 1.1 | 1.4 | 0.47 |
| YOR103C | 1.1 | 0.8 | 1.3 | 1.8 | 1.5 | 1.0 | 1.2 | 1.0 | 0.8 | 0.9 | 1.0 | 1.2 | 0.8 | 1.2 | 1.4 | 1.3 | 1.1 | 1.5 | 2.84 |
| YOR251C | 1.1 | 1.0 | 1.3 | 1.8 | 0.8 | 0.9 | 1.0 | 1.1 | 0.8 | 0.8 | 1.0 | 0.9 | 1.0 | 1.1 | 0.6 | 1.2 | 0.9 | 1.3 | 1.34 |

| | | | | | | | | | | | | | | | | | | |
|---|---|---|---|---|---|---|---|---|---|---|---|---|---|---|---|---|---|---|
| YOR348C | 1.1 | 0.7 | 1.2 | 2.0 | 0.9 | 0.7 | 0.8 | 0.8 | 0.5 | 0.4 | 1.1 | 0.7 | 1.1 | 1.2 | 1.6 | 1.7 | 0.9 | 0.9 | 0.18 |
| YPL148C | 1.2 | 0.8 | 0.9 | 2.3 | 1.1 | 0.6 | 1.0 | 0.9 | 0.7 | 1.5 | 3.5 | 0.7 | 0.6 | 1.6 | 0.6 | 1.6 | 1.0 | 1.1 | 0.48 |
| YGL205W | 0.9 | 0.9 | 0.7 | 0.8 | 1.1 | 1.6 | 0.8 | 1.2 | 0.5 | 0.6 | 0.4 | 1.1 | 1.0 | 1.0 | 2.0 | 4.1 | 3.9 | 9.1 | 0.24 |
| YNL192W | 1.1 | 1.2 | 1.5 | 1.6 | 0.9 | 0.6 | 1.0 | 0.8 | 0.6 | 1.8 | 1.1 | 1.1 | 2.7 | 0.7 | 2.1 | 0.8 | 2.3 | 2.8 | 1.31 |
| YOL108C | 1.4 | 1.5 | 0.4 | 1.9 | 2.1 | 0.9 | 1.1 | 1.3 | 1.6 | 0.9 | 1.1 | 1.0 | 1.3 | 0.9 | 1.5 | 1.8 | 1.5 | 2.7 | 0.87 |
| YPR165W | 1.3 | 0.7 | 1.6 | 1.0 | 1.5 | 1.8 | 1.0 | 1.0 | 0.9 | 0.7 | 1.0 | 0.8 | 1.0 | 1.0 | 0.9 | 1.0 | 2.3 | 2.7 | 3.64 |
| YDR073W | 1.7 | 0.9 | 0.7 | 1.4 | 1.2 | 1.3 | 1.2 | 1.3 | 0.5 | 1.2 | 1.0 | 0.8 | 0.6 | 1.2 | 0.8 | 1.6 | 1.8 | 2.3 | 1.27 |
| YER015W | 0.7 | 0.9 | 1.1 | 1.5 | 1.3 | 0.9 | 1.2 | 1.1 | 0.4 | 0.9 | 1.0 | 0.9 | 0.8 | 1.1 | 0.8 | 3.0 | 1.6 | 2.6 | 0.41 |
| YJL167W | 0.9 | 1.4 | 1.4 | 1.1 | 0.7 | 1.3 | 1.5 | 1.5 | 0.9 | 0.9 | 1.2 | 1.3 | 0.9 | 0.9 | 1.6 | 1.4 | 1.4 | 2.1 | 2.94 |
| YJL216C | 0.8 | 4.7 | 2.2 | 0.9 | 0.8 | | 1.5 | 1.9 | 1.5 | 1.3 | 1.1 | 1.0 | 1.1 | 1.1 | 1.2 | 1.5 | 1.2 | 2.0 | 0.25 |
| YKR009C | 1.0 | 1.2 | 1.5 | 0.9 | 1.0 | 1.8 | 1.0 | 0.9 | 2.4 | 2.1 | 1.2 | 1.0 | 1.8 | 1.0 | 1.0 | 4.6 | 1.9 | 2.5 | 0.27 |
| YOR180C | 0.8 | 1.1 | 1.0 | 1.0 | 1.0 | 0.7 | 0.8 | 0.9 | 0.3 | 0.4 | 0.6 | 0.8 | 0.9 | 1.0 | 1.1 | 2.4 | 0.9 | 2.3 | 0.55 |
| YBR036C | 0.8 | 1.7 | 1.8 | 1.2 | 1.2 | 1.3 | 1.3 | 0.7 | 1.0 | 1.0 | 1.5 | 1.0 | 1.9 | 0.8 | 1.5 | 1.6 | 2.1 | 1.8 | 2.05 |
| YDR297W | 1.5 | 0.8 | 0.8 | 1.1 | 0.9 | 1.4 | 0.6 | 1.5 | 0.3 | 0.7 | 1.4 | 1.0 | 0.8 | 0.9 | 1.4 | 1.4 | 1.9 | 1.9 | 1.73 |
| YDR387C | 0.8 | 0.9 | 1.2 | 2.2 | 0.9 | 1.2 | 1.3 | 0.7 | 1.4 | 2.0 | 2.0 | 0.9 | 1.3 | 0.9 | 1.4 | 2.7 | 1.2 | 1.1 | 0.86 |
| YOL096C | 1.1 | 1.0 | 1.2 | 1.6 | 1.2 | 1.3 | 1.7 | 1.0 | 1.3 | 1.4 | 1.2 | 1.2 | 1.4 | 1.4 | 1.0 | 2.3 | 1.2 | 1.2 | 0.64 |
| YPR184W | 1.2 | 1.4 | 5.7 | 2.2 | 0.9 | 1.5 | 1.3 | 0.7 | 1.9 | 3.2 | 3.6 | 1.4 | 2.5 | 1.3 | 1.5 | 3.1 | 1.0 | 1.7 | 0.37 |
| YBR298C | 1.1 | 1.0 | 1.6 | 2.3 | 1.4 | 0.4 | 1.7 | 0.5 | 0.3 | 1.1 | 1.5 | 1.1 | 0.8 | 0.9 | 0.2 | 2.1 | 0.9 | 0.8 | 0.88 |
| YDL078C | 0.7 | 1.1 | 1.2 | 0.8 | 1.3 | 1.1 | 1.7 | 1.0 | 1.1 | 1.0 | 1.7 | 0.8 | 1.0 | 0.8 | 0.5 | 2.2 | 1.1 | 2.0 | 1.65 |
| YDL215C | 1.0 | 1.5 | 1.0 | 1.6 | 1.5 | 0.9 | 1.9 | 1.1 | 1.7 | 2.4 | 0.8 | 0.8 | 1.5 | 1.0 | 1.1 | 2.2 | 1.1 | 1.6 | 0.91 |
| YGL035C | 0.7 | 1.4 | 4.3 | 1.4 | 1.1 | 0.6 | 1.2 | 1.3 | 0.9 | 1.5 | 1.3 | 0.9 | 1.1 | 1.1 | 1.0 | 2.3 | 0.9 | 1.2 | 0.61 |
| YGR287C | 1.3 | 1.0 | 1.3 | 1.5 | 0.8 | 0.8 | 2.2 | 1.3 | 2.6 | 4.2 | 1.5 | 1.1 | 1.7 | 1.1 | 2.3 | 3.0 | 1.0 | 1.1 | 0.38 |
| YJL070C | 1.3 | 0.9 | 1.1 | 1.2 | 1.3 | 2.0 | 1.4 | 1.1 | 1.0 | 1.1 | 1.3 | 1.0 | 1.8 | 1.3 | 3.5 | 2.2 | 1.1 | 1.2 | 0.34 |
| YLR351C | 1.0 | 1.4 | 1.3 | 1.5 | 1.3 | 1.4 | 1.4 | 1.2 | 0.8 | 1.2 | 1.1 | 0.9 | 0.9 | 1.4 | 1.2 | 2.0 | 1.3 | 1.8 | 1.75 |
| YLR375W | 0.9 | 1.5 | 0.9 | 0.9 | 1.0 | 1.3 | 0.9 | 1.0 | 1.6 | 1.2 | 1.6 | 0.9 | 1.6 | 0.9 | 1.3 | 2.2 | 1.0 | 1.0 | 1.05 |
| YMR267W | 0.8 | 1.8 | 0.7 | 0.9 | 1.0 | 1.4 | 1.2 | 1.5 | 0.4 | 0.8 | 1.0 | 0.8 | 0.7 | 1.0 | 0.9 | 2.6 | 0.9 | 1.3 | 0.94 |
| YMR278W | 0.7 | 1.7 | 1.4 | 1.0 | 1.2 | 1.3 | 1.8 | 1.1 | 1.4 | 1.7 | 1.1 | 1.0 | 2.5 | 0.8 | 1.8 | 2.5 | 1.0 | 1.5 | 0.62 |
| YMR293C | 0.9 | 1.5 | 1.6 | 2.2 | 0.6 | 0.5 | 1.2 | 1.0 | 0.7 | 1.3 | 0.4 | 0.9 | 0.8 | 1.3 | 0.7 | 2.2 | 0.8 | 1.0 | 0.37 |
| YNR072W | 1.1 | 1.5 | 0.5 | 0.7 | 1.0 | 0.9 | 1.5 | 0.9 | 0.7 | 1.8 | 1.4 | 1.0 | 1.5 | 1.0 | 1.8 | 2.1 | 1.3 | 1.0 | 0.26 |
| YOR363C | 0.8 | 2.0 | 0.9 | 1.3 | 0.7 | 1.2 | 0.7 | 1.1 | 1.2 | 1.6 | 1.2 | 0.9 | 1.3 | 0.9 | 1.1 | 2.1 | 1.3 | 1.8 | 0.40 |

## Table 8 Detoxification protein genes

| yeast genes | The level of expressed mRNA in the presence of chemical substance/ The level of expressed mRNA in the absence of chemical substance | | | | | | | | | | | | | | | | | | |
|---|---|---|---|---|---|---|---|---|---|---|---|---|---|---|---|---|---|---|---|
| | (1) | (2) | (3) | (4) | (5) | (6) | (7) | (8) | (9) | (10) | (11) | (12) | (13) | (14) | (15) | (16) | (17) | (18) | Intensity |
| YDR453C | 1.1 | 2.7 | 4.4 | 3.0 | 0.7 | 1.7 | 1.0 | 1.3 | 4.5 | 6.1 | 2.3 | 0.9 | 1.4 | 3.8 | 2.3 | 5.6 | 1.0 | 1.5 | 1.53 |
| YLL060C | 12.5 | 4.2 | 2.3 | 2.6 | 0.9 | 1.4 | 2.0 | 5.8 | 13.0 | 23.2 | 14.1 | 1.1 | 1.8 | 10.6 | 1.5 | 3.6 | 1.9 | 2.3 | 0.57 |
| YBL064C | 2.6 | 2.8 | 3.9 | 1.7 | 0.8 | 5.2 | 2.5 | 2.4 | 4.5 | 3.8 | 2.9 | 1.7 | 4.1 | 5.0 | 1.3 | 5.0 | 1.2 | 2.3 | 1.67 |
| YBR008C | 3.0 | 2.4 | 4.9 | 0.7 | 0.9 | 1.0 | 0.9 | 2.8 | 54.6 | 21.1 | 9.4 | 0.8 | 2.0 | 4.1 | 0.8 | 3.1 | 2.0 | 1.2 | 0.37 |
| YOR153W | 1.6 | 0.9 | 5.1 | 1.1 | 1.0 | 7.4 | 2.6 | 0.5 | 3.2 | 1.2 | 1.0 | 4.0 | 11.8 | 0.3 | 3.6 | 1.6 | 2.5 | 3.1 | 1.91 |
| YHL047C | 0.6 | 4.4 | 1.0 | 1.0 | 1.2 | 8.4 | 16.8 | 1.5 | 1.2 | 11.9 | 1.0 | 2.6 | 3.8 | 0.5 | 1.1 | 2.3 | 1.2 | 1.2 | 0.74 |
| YCL035C | 2.0 | 2.3 | 1.5 | 1.7 | 1.5 | 5.1 | 2.7 | 1.4 | 1.9 | 2.3 | 2.1 | 1.5 | 2.8 | 2.5 | 1.9 | 5.5 | 2.6 | 4.2 | 1.74 |
| YGR197C | 1.1 | 2.4 | 2.5 | 1.0 | 0.7 | 0.5 | 1.3 | 0.8 | 16.7 | 3.2 | 1.1 | 1.2 | 2.4 | 1.5 | 1.0 | 1.5 | 1.7 | 1.9 | 0.37 |
| YHR055C | 4.4 | 1.8 | 0.8 | 1.2 | 2.7 | 1.6 | 1.1 | 2.1 | 3.9 | 3.3 | 1.8 | 1.2 | 3.0 | 1.5 | 0.5 | 0.8 | 0.9 | 0.6 | 3.96 |
| YNL239W | 1.0 | 2.4 | 2.4 | 1.2 | 1.2 | 1.8 | 2.1 | 1.3 | 2.5 | 6.0 | 1.9 | 1.5 | 3.6 | 1.3 | 1.7 | 1.5 | 1.1 | 1.4 | 0.68 |
| YNL241C | 1.3 | 2.5 | 4.3 | 1.0 | 0.8 | 0.9 | 3.2 | 0.9 | 3.4 | 7.4 | 3.0 | 2.0 | 4.9 | 1.1 | 7.0 | 2.8 | 1.0 | 1.0 | 0.68 |
| YBR293W | 1.9 | 3.1 | 1.0 | 1.8 | 0.8 | 0.9 | 0.9 | 0.9 | 5.5 | 2.4 | 1.1 | 1.0 | 3.0 | 1.4 | 3.1 | 1.6 | 1.0 | 0.9 | 0.94 |
| YDL100C | 1.0 | 1.2 | 1.1 | 0.8 | 1.0 | 2.1 | 1.6 | 1.7 | 5.4 | 3.2 | 2.8 | 1.5 | 2.5 | 1.4 | 1.0 | 1.3 | 1.2 | 1.4 | 2.60 |
| YER185W | 2.0 | 3.1 | 2.1 | 7.0 | 1.6 | 1.2 | 1.1 | 1.0 | 2.9 | 1.2 | 1.6 | 1.1 | 2.1 | 1.6 | 1.7 | 0.6 | 2.6 | 1.1 | 0.26 |
| YGL013C | 0.9 | 1.2 | 0.8 | 0.7 | 1.1 | 2.3 | 0.9 | 1.4 | 3.0 | 1.5 | 0.9 | 1.0 | 2.3 | 0.8 | 1.0 | 0.9 | 1.1 | 1.0 | 0.43 |
| YHR053C | 3.5 | 2.1 | 0.7 | 1.1 | 2.9 | 1.3 | 1.2 | 2.2 | 4.5 | 2.9 | 1.4 | 1.1 | 1.9 | 1.5 | 0.3 | 0.7 | 1.0 | 0.5 | 3.99 |
| YIR038C | 1.3 | 3.5 | 4.6 | 2.0 | 0.7 | 2.5 | 2.0 | 1.3 | 4.6 | 4.5 | 3.4 | 1.1 | 2.5 | 2.5 | 1.2 | 6.0 | 2.8 | 2.0 | 1.11 |
| YKL026C | 2.7 | 2.0 | 1.2 | 4.9 | 1.0 | 1.8 | 2.5 | 1.8 | 7.5 | 3.0 | 3.0 | 1.0 | 2.3 | 1.8 | 0.8 | 6.6 | 1.6 | 3.3 | 0.61 |
| YLL028W | 0.6 | 0.9 | 4.6 | 0.5 | 0.9 | 1.7 | 1.0 | 0.7 | 1.2 | 2.3 | 0.7 | 1.3 | 4.1 | 0.7 | 3.1 | 0.6 | 1.2 | 1.2 | 1.02 |
| YOR273C | 0.7 | 1.0 | 1.7 | 1.0 | 1.1 | 0.4 | 0.4 | 0.6 | 0.6 | 0.5 | 1.5 | 0.8 | 2.6 | 0.5 | 0.8 | 0.6 | 3.2 | 3.1 | 1.20 |
| YGR138C | 1.1 | 1.6 | 1.0 | 1.2 | 0.8 | 0.8 | 0.7 | 1.0 | 0.5 | 1.0 | 0.7 | 0.6 | 0.6 | 0.9 | 4.5 | 0.7 | 0.7 | 0.7 | 1.24 |
| YOR247W | 1.7 | 0.6 | 3.6 | 1.4 | 0.6 | 0.2 | 0.5 | 0.7 | 0.1 | 0.4 | 2.9 | 0.6 | 0.3 | 0.4 | 7.3 | 0.2 | 1.2 | 0.9 | 2.23 |
| YPL163C | 0.6 | 0.7 | 1.4 | 0.9 | 0.6 | 0.6 | 0.4 | 0.5 | 0.1 | 0.4 | 0.5 | 0.9 | 0.4 | 0.6 | 5.3 | 0.6 | 1.4 | 1.4 | 1.05 |
| YHL040C | 1.6 | 4.4 | 1.5 | 0.4 | 1.1 | 7.3 | 4.0 | 1.5 | 1.1 | 11.3 | 1.7 | 2.0 | 2.0 | 1.1 | 3.4 | 1.0 | 1.4 | 1.0 | 0.69 |
| YEL065W | 0.3 | 3.8 | 1.2 | 0.4 | 1.4 | 2.3 | 4.3 | 0.9 | 0.1 | 4.9 | 0.6 | 1.7 | 0.8 | 0.4 | 2.2 | 2.6 | 0.7 | 0.6 | 2.10 |
| YIR002C | 0.8 | 1.1 | 0.5 | 0.6 | 1.2 | 2.2 | 1.3 | 1.2 | 1.7 | 1.4 | 0.8 | 1.3 | 1.2 | 1.1 | 1.1 | 1.4 | 1.1 | 1.2 | 0.58 |
| YNL259C | 1.6 | 4.5 | 1.1 | 1.2 | 1.1 | 3.9 | 3.8 | 1.5 | 1.6 | 2.1 | 1.6 | 0.7 | 1.1 | 1.4 | 0.7 | 1.7 | 2.6 | 2.4 | 1.22 |
| YBR145W | 1.5 | 0.7 | 2.8 | 0.9 | 1.1 | 11.5 | 58.8 | 1.0 | 0.1 | 1.1 | 1.1 | 1.0 | 2.0 | 2.2 | 1.2 | 3.6 | 1.7 | 2.0 | 2.17 |
| YLR043C | 1.4 | 1.4 | 2.0 | 1.8 | 2.0 | 1.4 | 2.6 | 1.1 | 2.8 | 2.5 | 1.4 | 0.8 | 2.0 | 2.1 | 0.5 | 1.8 | 1.5 | 2.2 | 2.12 |

| | | | | | | | | | | | | | | | | | | | |
|---|---|---|---|---|---|---|---|---|---|---|---|---|---|---|---|---|---|---|---|
| YGR209C | 1.6 | 3.1 | 3.5 | 1.5 | 1.9 | 1.9 | 1.7 | 1.6 | 2.4 | 6.3 | 3.1 | 0.9 | 1.4 | 5.0 | 1.3 | 1.9 | 1.3 | 2.4 | 3.17 |
| YDL168W | 2.3 | 2.0 | 2.1 | 0.9 | 1.2 | 1.4 | 1.1 | 1.7 | 8.2 | 4.7 | 1.9 | 0.6 | 1.3 | 1.6 | 1.2 | 1.1 | 0.9 | 0.8 | 1.08 |
| YDR513W | 2.2 | 2.5 | 2.3 | 2.6 | 0.9 | 2.1 | 1.6 | 1.6 | 4.6 | 3.1 | 2.0 | 0.9 | 1.8 | 2.0 | 1.3 | 3.8 | 1.3 | 3.2 | 3.10 |
| YGR088W | 1.3 | 1.2 | 7.7 | 2.4 | 1.0 | 1.3 | 0.8 | 0.8 | 1.5 | 3.2 | 3.9 | 1.1 | 1.2 | 1.3 | 0.7 | 5.6 | 0.9 | 2.0 | 0.75 |
| YHR048W | 2.5 | 1.4 | 1.4 | 1.7 | 1.0 | 0.8 | 0.8 | 1.9 | 4.5 | 2.7 | 2.0 | 0.9 | 1.1 | 1.7 | 0.9 | 1.4 | 0.7 | 0.9 | 0.26 |
| YJL101C | 2.1 | 1.9 | 2.1 | 1.2 | 1.4 | 1.0 | 1.0 | 1.3 | 3.1 | 3.6 | 2.7 | 0.7 | 1.0 | 1.0 | 0.5 | 1.3 | 0.9 | 1.0 | 1.13 |
| YML116W | 4.1 | 1.3 | 1.5 | 1.4 | 1.2 | 0.9 | 1.4 | 2.2 | 1.4 | 3.1 | 4.3 | 0.5 | 0.8 | 2.0 | 1.9 | 1.0 | 1.0 | 1.0 | 0.94 |
| YMR038C | 1.9 | 1.7 | 1.8 | 0.8 | 1.5 | 1.0 | 1.1 | 1.9 | 2.4 | 2.6 | 2.7 | 0.9 | 1.7 | 1.1 | 0.6 | 1.5 | 1.2 | 1.3 | 1.76 |
| YBR244W | 0.6 | 1.4 | 2.0 | 0.9 | 1.7 | 1.0 | 0.6 | 1.0 | 1.4 | 3.0 | 1.5 | 0.5 | 0.4 | 3.7 | 0.4 | 0.7 | 1.2 | 1.2 | 3.42 |
| YCL069W | 0.9 | 15.7 | 0.9 | 0.8 | 0.9 | | 1.4 | 1.3 | 1.2 | 6.9 | 1.0 | 0.9 | 0.8 | 1.3 | 1.4 | 0.9 | 1.0 | 1.0 | 0.25 |
| YKR105C | 0.8 | 0.9 | 0.9 | 1.5 | 1.0 | 1.2 | 1.0 | 1.3 | 1.0 | 5.2 | 0.0 | 0.8 | 2.5 | 1.2 | 1.5 | 0.7 | 0.8 | 1.0 | 0.26 |
| YOL158C | 0.7 | 4.0 | 2.4 | 0.9 | 1.2 | 2.0 | 1.6 | 0.7 | 1.7 | 6.1 | 0.7 | 0.9 | 1.4 | 1.0 | 0.9 | 1.2 | 1.4 | 1.7 | 1.30 |
| YDR256C | 0.8 | 1.4 | 0.7 | 1.3 | 1.2 | 0.5 | 0.6 | 0.8 | 5.2 | 2.0 | 1.4 | 0.8 | 1.1 | 1.6 | 1.0 | 4.3 | 1.0 | 2.0 | 0.30 |
| YGL254W | 1.2 | 1.1 | 0.8 | 0.6 | 1.1 | 0.7 | 1.3 | 1.4 | 3.1 | 1.8 | 1.1 | 1.2 | 2.4 | 1.3 | 1.1 | 1.4 | 0.8 | 1.2 | 0.53 |
| YKL064W | 0.6 | 0.9 | 0.4 | 0.8 | 1.2 | 1.5 | 1.1 | 0.9 | 2.4 | 1.6 | 0.7 | 0.7 | 1.6 | 1.1 | 0.7 | 1.4 | 1.3 | 1.3 | 0.60 |
| YLL057C | 2.2 | 59.9 | 1.8 | 0.9 | 1.1 | 1.0 | 1.6 | 1.4 | 121.8 | 2.9 | 1.3 | 0.7 | 1.4 | 1.1 | 1.4 | 0.9 | 3.0 | 1.3 | 0.19 |
| YPR200C | 47.7 | 1.7 | 0.8 | 5.0 | 1.6 | 0.8 | 1.3 | 1.4 | 4.7 | 3.8 | 2.0 | 0.4 | 0.6 | 2.0 | 0.7 | 1.2 | 1.1 | 1.0 | 0.27 |
| YJR104C | 1.4 | 1.8 | 2.9 | 1.2 | 1.5 | 1.4 | 1.1 | 1.5 | 1.9 | 2.8 | 2.6 | 0.9 | 0.9 | 2.0 | 1.3 | 2.9 | 0.9 | 1.1 | 3.50 |
| YKR106W | 1.3 | 0.8 | 2.1 | 1.4 | 1.2 | 1.6 | 0.9 | 1.3 | 10.5 | 7.4 | 1.8 | 0.8 | 1.4 | 1.7 | | 2.8 | 0.9 | 0.8 | 0.16 |
| YOR031W | 2.6 | 1.7 | 1.3 | 3.4 | 1.4 | 0.9 | 1.3 | 0.9 | 0.7 | 1.3 | 1.2 | 0.8 | 1.8 | 1.5 | 1.4 | 6.2 | 1.7 | 2.4 | 0.52 |
| YHR008C | 1.3 | 6.9 | 4.8 | 1.8 | 0.6 | 1.0 | 0.7 | 0.9 | 1.7 | 2.4 | 2.1 | 0.7 | 1.2 | 1.6 | 1.7 | 2.2 | 0.9 | 1.0 | 1.04 |
| YML028W | 1.0 | 2.2 | 4.4 | 1.3 | 1.6 | 1.2 | 0.8 | 0.8 | 0.8 | 1.8 | 1.0 | 1.6 | 1.6 | 2.0 | 2.2 | 1.3 | 1.1 | 1.5 | 4.85 |
| YEL027W | 1.2 | 0.6 | 3.4 | 1.1 | 1.0 | 1.1 | 0.8 | 0.9 | 0.9 | 1.1 | 0.9 | 0.9 | 0.7 | 1.4 | 1.5 | 1.6 | 1.0 | 1.4 | 4.75 |
| YKR066C | 0.9 | 1.4 | 5.5 | 0.7 | 1.0 | 0.9 | 0.5 | 0.7 | 0.7 | 2.4 | 1.3 | 0.9 | 0.6 | 1.5 | 0.6 | 1.3 | 0.9 | 1.1 | 1.25 |
| YDR538W | 0.9 | 1.1 | 2.4 | 1.2 | 1.0 | 1.0 | 1.2 | 1.0 | 0.3 | 0.8 | 0.9 | 0.7 | 0.7 | 0.9 | 1.0 | 0.9 | 1.0 | 0.9 | 0.53 |
| YMR015C | 0.7 | 0.7 | 1.9 | 0.9 | 1.7 | 1.3 | 0.4 | 0.7 | 0.1 | 0.3 | 1.0 | 0.9 | 0.4 | 0.8 | 1.2 | 1.5 | 1.0 | 1.0 | 2.51 |
| YOR251C | 1.1 | 1.0 | 1.3 | 1.8 | 0.8 | 0.9 | 1.0 | 1.1 | 0.8 | 0.8 | 1.0 | 0.9 | 1.0 | 1.1 | 0.6 | 1.2 | 0.9 | 1.3 | 1.34 |
| YLR046C | 0.7 | 1.7 | 0.9 | 0.8 | 1.0 | 3.8 | 1.5 | 0.5 | 1.0 | 1.4 | 0.9 | 0.8 | 1.2 | 1.3 | 0.8 | 1.3 | 5.2 | 5.4 | 0.86 |
| YGR224W | 0.9 | 1.2 | 1.2 | 1.2 | 1.4 | 0.8 | 0.4 | 0.6 | 0.9 | 0.9 | 0.8 | 0.6 | 0.8 | 0.9 | 1.1 | 1.0 | 2.8 | 1.3 | 0.27 |
| YFL050C | 0.7 | 1.0 | 1.1 | 2.2 | 1.0 | 0.9 | 0.8 | 0.9 | 0.5 | 1.6 | 1.2 | 0.9 | 1.2 | 1.0 | 1.4 | 2.3 | 0.8 | 1.0 | 0.36 |
| YCR083W | 1.1 | 2.8 | 1.5 | 1.8 | 1.6 | 2.4 | 1.8 | 1.5 | 1.8 | 1.8 | 1.8 | 1.1 | 1.8 | 2.4 | 1.0 | 3.1 | 1.3 | 1.3 | 0.98 |

**Table 9 Genes belonging to other category**

The level of expressed mRNA in the presence of chemical substance/ The level of expressed mRNA in the absence of chemical substance

| yeast genes | (1) | (2) | (3) | (4) | (5) | (6) | (7) | (8) | (9) | (10) | (11) | (12) | (13) | (14) | (15) | (16) | (17) | (18) | Intensity |
|---|---|---|---|---|---|---|---|---|---|---|---|---|---|---|---|---|---|---|---|
| YKR076W | 5.0 | 2.6 | 3.5 | 2.2 | 1.0 | 4.7 | 4.9 | 6.2 | 43.4 | 43.1 | 18.6 | 1.5 | 5.1 | 14.7 | 3.2 | 9.6 | 1.8 | 2.7 | 0.27 |
| YNL335W | 1.2 | 0.7 | 1.5 | 2.3 | 1.5 | 1.6 | 1.3 | 1.7 | 1.6 | 5.3 | 1.1 | 0.8 | 0.9 | 4.6 | 1.7 | 1.0 | 1.2 | 1.1 | 0.22 |
| YBR173C | 1.1 | 2.4 | 3.4 | 1.6 | 0.7 | 1.3 | 1.6 | 1.2 | 6.0 | 5.1 | 2.5 | 1.7 | 3.8 | 3.0 | 15.4 | 2.1 | 0.9 | 1.6 | 1.15 |
| YFL022C | 0.8 | 0.9 | 1.5 | 0.6 | 0.7 | 0.9 | 0.3 | 0.8 | 0.5 | 0.5 | 1.2 | 0.6 | 0.6 | 3.1 | 1.1 | 1.3 | 0.9 | 0.8 | 1.07 |
| YGL158W | 1.1 | 2.8 | 1.9 | 12.9 | 1.0 | 0.4 | 1.3 | 1.3 | -0.4 | 0.9 | 0.2 | 1.3 | 1.0 | 2.3 | 2.3 | 8.2 | 0.8 | 1.1 | 0.18 |
| YHR139C | 3.0 | 4.6 | 6.8 | 9.3 | 1.3 | 1.9 | 1.5 | 1.3 | 107.2 | 46.6 | 3.5 | 1.6 | 7.0 | 2.5 | 1.9 | 17.1 | 1.4 | 1.3 | 0.34 |
| YGR213C | 5.4 | 2.9 | 1.0 | 6.8 | 1.2 | 4.1 | 7.0 | 2.0 | 7.5 | 6.9 | 1.6 | 10.6 | 30.4 | 1.0 | 6.5 | 5.2 | 12.8 | 8.5 | 0.22 |
| YKL165C | 0.5 | 2.0 | 1.1 | 0.6 | 0.9 | 0.6 | 2.1 | 1.5 | 0.4 | 0.4 | 0.3 | 2.4 | 3.2 | 0.8 | 2.1 | 1.2 | 0.9 | 1.0 | 1.08 |
| YBL005W-A | 1.0 | 0.9 | 0.5 | 0.9 | 1.3 | 1.2 | 1.4 | 1.0 | 0.8 | 1.5 | 0.8 | 1.6 | 2.9 | 1.4 | 0.9 | 1.1 | 1.3 | 1.0 | 0.48 |
| YBL041W | 1.2 | 1.4 | 0.9 | 1.0 | 1.6 | 1.7 | 1.6 | 1.7 | 2.8 | 2.6 | 1.6 | 1.6 | 2.6 | 2.0 | 1.3 | 2.3 | 1.3 | 2.0 | 3.03 |
| YBL078C | 2.3 | 2.7 | 1.3 | 2.6 | 0.7 | 2.4 | 3.1 | 1.6 | 12.3 | 15.2 | 4.6 | 3.1 | 5.0 | 3.6 | 2.5 | 4.4 | 1.8 | 2.8 | 0.65 |
| YCL020W | 1.1 | 2.5 | 0.5 | 1.1 | 0.5 | 1.0 | 1.5 | 1.7 | 0.9 | 2.4 | 1.3 | 1.5 | 3.0 | 1.7 | 2.5 | 1.3 | 0.9 | 1.3 | 1.46 |
| YDL007W | 0.8 | 1.4 | 1.3 | 0.9 | 1.0 | 1.1 | 1.2 | 1.4 | 3.8 | 2.4 | 1.3 | 1.1 | 2.4 | 1.4 | 1.4 | 0.8 | 0.8 | 1.5 | 2.43 |
| YDL097C | 0.9 | 1.7 | 0.9 | 1.2 | 0.8 | 1.3 | 1.6 | 1.5 | 4.5 | 2.3 | 1.5 | 1.3 | 4.0 | 1.4 | 1.1 | 1.1 | 1.0 | 1.3 | 2.06 |
| YDL126C | 0.5 | 1.4 | 2.3 | 0.7 | 0.9 | 1.2 | 1.5 | 0.8 | 2.7 | 2.0 | 1.4 | 1.3 | 4.3 | 0.6 | 1.7 | 0.7 | 0.9 | 0.9 | 3.24 |
| YER012W | 1.4 | 1.2 | 1.2 | 1.0 | 1.9 | 2.4 | 2.4 | 1.8 | 6.7 | 2.7 | 1.9 | 1.5 | 2.9 | 4.6 | 1.7 | 2.2 | 1.5 | 1.3 | 1.84 |
| YFR010W | 1.1 | 1.1 | 0.8 | 1.2 | 0.9 | 1.3 | 1.4 | 1.6 | 4.3 | 3.4 | 1.5 | 1.2 | 3.7 | 1.1 | 1.5 | 1.3 | 0.9 | 1.4 | 1.89 |
| YFR024C | 0.8 | 25.0 | 2.3 | 1.1 | 0.7 | 1.4 | 3.0 | 0.9 | 5.3 | 4.1 | 1.9 | 1.5 | 2.8 | 1.2 | 0.9 | 1.9 | 1.0 | 1.4 | 1.06 |
| YGL048C | 1.0 | 1.3 | 0.7 | 1.3 | 1.8 | 1.5 | 2.0 | 1.5 | 2.4 | 2.1 | 1.5 | 1.3 | 2.5 | 1.2 | 1.0 | 1.6 | 1.4 | 2.0 | 3.07 |
| YGL141W | 0.8 | 1.2 | 1.1 | 0.7 | 1.4 | 3.0 | 1.7 | 0.8 | 4.5 | 1.9 | 0.6 | 1.4 | 3.6 | 1.2 | 1.5 | 1.2 | 1.4 | 1.2 | 0.44 |
| YGL180W | 1.3 | 1.3 | 1.7 | 0.6 | 1.2 | 1.1 | 2.4 | 1.2 | 7.2 | 3.7 | 1.3 | 1.4 | 2.6 | 1.0 | 1.3 | 1.7 | 1.0 | 1.1 | 0.28 |
| YGR048W | 0.7 | 2.7 | 1.4 | 1.3 | 1.0 | 1.0 | 1.9 | 1.4 | 4.4 | 3.1 | 1.1 | 1.1 | 2.5 | 1.3 | 0.8 | 1.1 | 0.9 | 1.2 | 0.93 |
| YGR135W | 1.0 | 1.0 | 1.0 | 1.1 | 1.6 | 1.5 | 2.2 | 1.8 | 1.5 | 2.3 | 1.6 | 1.2 | 2.8 | 2.6 | 1.5 | 1.5 | 1.0 | 1.9 | 3.10 |
| YGR201C | 1.7 | 11.9 | 2.4 | 2.0 | 0.8 | 3.1 | 2.6 | 1.6 | 5.4 | 3.5 | 1.6 | 1.9 | 8.6 | 1.6 | 0.5 | 14.4 | 1.4 | 2.1 | 0.60 |
| YHL030W | 0.5 | 0.8 | 1.0 | 0.7 | 0.7 | 1.9 | 0.9 | 0.8 | 2.7 | 2.5 | 1.1 | 1.1 | 3.8 | 1.0 | 2.0 | 0.5 | 0.8 | 0.9 | 0.66 |
| YHR166C | 0.9 | 1.1 | 1.0 | 0.7 | 1.0 | 1.8 | 1.5 | 1.0 | 2.6 | 1.2 | 0.6 | 1.7 | 6.9 | 1.2 | 0.9 | 1.1 | 1.4 | 1.5 | 1.35 |
| YJR069C | 0.8 | 0.8 | 0.9 | 0.6 | 0.9 | 1.2 | 1.3 | 0.9 | 0.7 | 0.6 | 0.9 | 1.1 | 3.9 | 0.6 | 0.4 | 0.8 | 1.0 | 0.9 | 1.73 |
| YKL073W | 0.6 | 1.4 | 0.5 | 0.6 | 1.2 | 0.5 | 2.1 | 1.6 | 1.3 | 1.1 | 0.5 | 1.5 | 3.3 | 0.7 | 1.6 | 1.0 | 1.2 | 1.5 | 1.29 |
| YKL103C | 1.6 | 4.1 | 2.5 | 2.1 | 0.9 | 2.7 | 2.8 | 1.6 | 8.3 | 9.9 | 2.8 | 1.3 | 3.8 | 2.0 | 2.1 | 1.2 | 1.9 | 2.2 | 0.56 |

| | | | | | | | | | | | | | | | | | | | |
|---|---|---|---|---|---|---|---|---|---|---|---|---|---|---|---|---|---|---|---|
| YLR080W | 1.0 | 1.4 | 1.2 | 1.7 | 1.1 | 2.5 | 2.8 | 1.3 | 2.3 | 1.5 | 1.9 | 0.8 | 3.6 | 1.2 | 0.7 | 3.0 | 1.6 | 2.2 | 0.41 |
| YLR107W | 1.2 | 0.9 | 0.5 | 1.2 | 1.7 | 2.3 | 1.6 | 1.1 | 1.8 | 1.2 | 0.8 | 1.0 | 2.9 | 1.6 | 1.1 | 1.6 | 1.8 | 2.4 | 0.94 |
| YLR121C | 1.7 | 1.7 | 1.1 | 1.2 | 1.0 | 1.6 | | 1.3 | 2.2 | 2.0 | 2.2 | 1.1 | 4.0 | 1.2 | 10.7 | 1.5 | 2.7 | 1.4 | 0.30 |
| YLR336C | 0.6 | 0.8 | 0.7 | 0.4 | 0.9 | 0.8 | 1.8 | 1.0 | -0.3 | 0.5 | 0.5 | 1.9 | 4.6 | 0.6 | 0.8 | 1.3 | 1.5 | 1.0 | 0.71 |
| YLR370C | 1.1 | 1.0 | 1.1 | 1.4 | 1.4 | 4.1 | 2.1 | 0.8 | 1.5 | 1.9 | 1.0 | 1.2 | 3.1 | 1.9 | 1.5 | 1.6 | 1.6 | 2.0 | 1.41 |
| YLR423C | 1.2 | 1.2 | 0.7 | 1.9 | 1.5 | 1.2 | 3.8 | 2.0 | 1.2 | 1.0 | 1.0 | 1.7 | 3.9 | 1.2 | 1.3 | 1.9 | 1.2 | 1.6 | 0.34 |
| YML092C | 1.1 | 3.1 | 2.4 | 1.1 | 0.9 | 1.9 | 1.2 | 1.2 | 6.4 | 4.0 | 2.5 | 1.2 | 2.3 | 1.6 | 1.4 | 2.2 | 1.3 | 1.5 | 1.72 |
| YML130C | 1.5 | 2.7 | 5.3 | 1.2 | 0.9 | 0.6 | 2.9 | 2.7 | 7.8 | 4.2 | 1.9 | 2.3 | 5.8 | 1.3 | 2.4 | 0.8 | 1.2 | 1.2 | 1.59 |
| YMR214W | 0.8 | 3.8 | 1.0 | 1.4 | 0.8 | 0.9 | 1.0 | 1.1 | 2.1 | 1.0 | 0.7 | 1.0 | 2.4 | 1.1 | 4.8 | 0.7 | 1.1 | 1.0 | 0.88 |
| YMR297W | 0.7 | 2.0 | 3.6 | 1.9 | 0.6 | 1.8 | 1.2 | 0.8 | 1.9 | 1.0 | 2.5 | 1.2 | 4.2 | 0.5 | 2.4 | 1.9 | 1.1 | 1.4 | 4.10 |
| YNL036W | 2.0 | 1.9 | 3.2 | 1.0 | 1.6 | 1.1 | 2.0 | 2.1 | 6.6 | 5.0 | 1.7 | 1.4 | 3.3 | 5.1 | 1.6 | 1.3 | 1.4 | 1.9 | 2.04 |
| YOL005C | 1.4 | 1.4 | 1.1 | 0.8 | 1.6 | 1.1 | 1.1 | 1.0 | 0.9 | 1.0 | 1.6 | 1.2 | 2.9 | 1.2 | 0.9 | 1.0 | 1.2 | 1.2 | 1.71 |
| YOR134W | 2.0 | 3.6 | 1.0 | 9.5 | 0.9 | 1.4 | 1.1 | 1.5 | 1.5 | 8.2 | 1.9 | 2.8 | 4.4 | 1.3 | 1.6 | 7.7 | 2.2 | 4.6 | 0.28 |
| YOR362C | 1.0 | 1.7 | 1.0 | 0.8 | 1.7 | 1.4 | 1.6 | 1.4 | 4.3 | 3.0 | 1.3 | 1.3 | 2.5 | 1.4 | 1.0 | 1.5 | 1.1 | 1.8 | 2.55 |
| YAR009C | 0.8 | 1.3 | 0.6 | 1.0 | 1.4 | 1.6 | 2.7 | 1.4 | 0.9 | 1.4 | 0.9 | 1.3 | 2.0 | 1.0 | 2.1 | 0.6 | 0.8 | 1.2 | 7.11 |
| YBL101C | 1.0 | 1.1 | 1.9 | 0.9 | 1.0 | 0.9 | 0.8 | 0.8 | 3.0 | 1.4 | 0.8 | 1.1 | 3.5 | 0.9 | 2.7 | 1.4 | 1.0 | 1.1 | 0.39 |
| YBR046C | 1.4 | 1.4 | 1.7 | 1.8 | 1.3 | 2.6 | 3.9 | 1.6 | 4.6 | 6.2 | 2.9 | 1.2 | 2.0 | 1.3 | 2.1 | 3.0 | 1.2 | 2.0 | 0.63 |
| YBR139W | 1.2 | 1.4 | 3.1 | 1.4 | 0.8 | 2.4 | 1.9 | 0.7 | 2.3 | 1.6 | 2.3 | 1.2 | 2.7 | 1.1 | 5.6 | 2.3 | 1.2 | 2.0 | 1.21 |
| YBR170C | 0.8 | 1.2 | 0.6 | 1.4 | 1.5 | 1.4 | 1.9 | 1.3 | 6.1 | 4.2 | 1.5 | 1.2 | 2.7 | 1.3 | 1.9 | 1.3 | 1.1 | 1.4 | 0.72 |
| YBR177C | 0.8 | 1.1 | 4.0 | 1.2 | 1.2 | 2.3 | 0.9 | 0.9 | 2.1 | 1.7 | 0.6 | 1.2 | 3.1 | 1.3 | 1.4 | 0.4 | 0.8 | 0.9 | 0.76 |
| YBR212W | 0.7 | 1.2 | 2.6 | 1.2 | 1.0 | 1.4 | 0.9 | 0.6 | 2.7 | 2.3 | 1.1 | 0.9 | 2.2 | 0.7 | 1.0 | 1.5 | 1.1 | 1.0 | 0.95 |
| YBR239C | 1.0 | 1.3 | 0.9 | 1.5 | 0.8 | 1.5 | 1.0 | 1.3 | 0.4 | 1.2 | 0.7 | 1.1 | 1.9 | 1.1 | 1.0 | 2.7 | 1.0 | 0.9 | 0.31 |
| YCL033C | 1.1 | 2.0 | 1.7 | 1.7 | 1.2 | 4.6 | 2.4 | 1.1 | 2.3 | 1.4 | 1.9 | 1.6 | 2.0 | 1.8 | 0.8 | 3.6 | 2.0 | 2.0 | 1.14 |
| YCR062W | 0.8 | 1.9 | 2.7 | 2.8 | 1.4 | 1.9 | 0.9 | 0.7 | 0.7 | 1.3 | 1.2 | 1.3 | 2.2 | 1.0 | 2.3 | 1.1 | 1.1 | 1.0 | 0.41 |
| YCR067C | 0.9 | 0.7 | 1.2 | 1.3 | 0.7 | 0.8 | 0.8 | 0.7 | 1.8 | 1.6 | 1.2 | 0.9 | 2.0 | 0.9 | 2.1 | 1.4 | 1.1 | 0.9 | 0.62 |
| YDL020C | 2.3 | 1.2 | 3.4 | 1.1 | 1.2 | 2.5 | 1.0 | 2.2 | 5.6 | 2.9 | 1.9 | 1.1 | 3.4 | 1.2 | 1.5 | 1.8 | 1.5 | 1.7 | 1.36 |
| YDR168W | 1.2 | 1.4 | 1.0 | 0.8 | 1.5 | 1.3 | 2.4 | 2.1 | 3.7 | 2.3 | 1.5 | 1.2 | 2.4 | 1.4 | 1.9 | 1.4 | 1.2 | 1.4 | 1.28 |
| YDR169C | 1.0 | 1.4 | 1.1 | 1.3 | 1.3 | 1.2 | 1.1 | 1.0 | 1.6 | 1.4 | 0.9 | 0.9 | 1.9 | 0.9 | 1.4 | 0.9 | 1.4 | 1.1 | 0.40 |
| YDR188W | 0.8 | 1.2 | 1.0 | 0.8 | 0.8 | 0.8 | 0.9 | 1.0 | 2.9 | 1.6 | 0.9 | 0.9 | 1.7 | 0.7 | 1.3 | 0.9 | 0.7 | 0.8 | 1.78 |
| YDR264C | 0.5 | 2.6 | 0.9 | 1.4 | 0.9 | 1.8 | 3.2 | 0.8 | 1.1 | 3.4 | 0.9 | 1.1 | 2.1 | 0.7 | 2.3 | 1.5 | 1.2 | 1.4 | 1.63 |
| YDR304C | 1.0 | 1.8 | 2.7 | 1.3 | 1.5 | 2.1 | 2.0 | 1.3 | 3.9 | 1.9 | 1.2 | 1.3 | 2.3 | 1.7 | 1.0 | 4.0 | 1.6 | 1.8 | 2.23 |
| YDR403W | 1.0 | 1.2 | 1.6 | 1.0 | 1.2 | 1.1 | 1.2 | 1.2 | 4.1 | 3.6 | 1.0 | 1.0 | 2.0 | 1.1 | 0.9 | 0.9 | 0.9 | 1.0 | 0.35 |

| | | | | | | | | | | | | | | | | | | | |
|---|---|---|---|---|---|---|---|---|---|---|---|---|---|---|---|---|---|---|---|
| YDR427W | 0.8 | 1.1 | 0.8 | 0.8 | 1.5 | 1.9 | 1.4 | 1.2 | 2.3 | 2.0 | 1.2 | 1.1 | 2.1 | 1.0 | 1.3 | 1.1 | 1.0 | 1.5 | 2.37 |
| YEL012W | 1.3 | 1.8 | 1.0 | 1.8 | 1.7 | 1.6 | 4.7 | 1.4 | 4.6 | 4.2 | 2.2 | 1.2 | 2.2 | 1.9 | 1.1 | 2.5 | 1.5 | 2.2 | 0.74 |
| YER009W | 1.3 | 0.6 | 2.4 | 1.2 | 1.2 | 2.0 | 2.8 | 1.0 | 0.7 | 1.0 | 1.4 | 1.1 | 2.0 | 1.6 | 0.9 | 1.5 | 2.0 | 2.1 | 3.35 |
| YER021W | 0.8 | 1.0 | 0.9 | 0.9 | 1.4 | 1.1 | 1.5 | 1.2 | 3.0 | 2.2 | 1.3 | 1.0 | 1.8 | 1.3 | 0.8 | 1.4 | 0.8 | 1.5 | 2.02 |
| YER094C | 1.0 | 1.3 | 0.9 | 1.1 | 1.9 | 1.1 | 1.8 | 1.3 | 2.3 | 1.7 | 1.3 | 1.1 | 2.1 | 1.5 | 0.8 | 1.5 | 1.3 | 1.8 | 3.01 |
| YER177W | 1.4 | 1.5 | 2.1 | 1.5 | 0.9 | 1.6 | 1.2 | 1.1 | 1.8 | 1.7 | 1.9 | 1.1 | 2.2 | 0.9 | 1.4 | 1.0 | 0.9 | 2.0 | 5.81 |
| YFL029C | 1.0 | 1.2 | 1.2 | 1.8 | 1.7 | 1.0 | 1.6 | 1.5 | 4.3 | 2.9 | 2.1 | 1.1 | 1.8 | 1.9 | 1.3 | 1.2 | 1.2 | 1.4 | 0.49 |
| YFL038C | 1.0 | 1.2 | 0.7 | 1.3 | 1.5 | 1.3 | 1.7 | 1.4 | 2.1 | 1.9 | 1.7 | 1.1 | 2.1 | 1.5 | 1.5 | 2.1 | 1.2 | 1.6 | 2.31 |
| YFR004W | 1.0 | 1.5 | 0.8 | 1.4 | 1.0 | 1.5 | 1.3 | 1.4 | 3.6 | 2.6 | 1.3 | 1.2 | 2.0 | 2.1 | 1.1 | 1.3 | 1.0 | 1.4 | 2.44 |
| YFR050C | 0.9 | 1.2 | 1.1 | 1.3 | 1.7 | 1.5 | 1.4 | 1.4 | 3.1 | 2.1 | 1.4 | 1.5 | 2.1 | 1.6 | 1.5 | 1.5 | 1.1 | 1.7 | 1.70 |
| YGL011C | 1.2 | 1.5 | 1.1 | 1.1 | 1.8 | 1.6 | 1.5 | 1.3 | 2.0 | 2.2 | 1.5 | 1.0 | 2.1 | 3.0 | 1.3 | 1.7 | 1.3 | 1.5 | 1.78 |
| YGL094C | 0.8 | 1.3 | 0.3 | 0.9 | 1.3 | 1.0 | 0.8 | 1.0 | 1.4 | 1.1 | 0.6 | 0.8 | 2.3 | 0.9 | 1.9 | 1.0 | 1.0 | 0.9 | 0.51 |
| YGL150C | 0.7 | 0.8 | 1.2 | 1.0 | 0.8 | 1.1 | 0.8 | 0.8 | 1.4 | 0.9 | 0.4 | 0.8 | 2.4 | 1.0 | 1.3 | 0.9 | 0.9 | 0.8 | 0.62 |
| YGL207W | 0.5 | 0.6 | 0.9 | 0.6 | 1.3 | 0.9 | 0.6 | 1.0 | 1.4 | 1.1 | 0.7 | 0.7 | 2.5 | 0.8 | 1.2 | 0.7 | 0.9 | 0.8 | 0.88 |
| YGR232W | 1.2 | 1.2 | 1.2 | 1.7 | 1.5 | 1.9 | 2.0 | 1.1 | 3.4 | 2.2 | 1.3 | 1.1 | 2.4 | 2.1 | 0.9 | 1.8 | 1.3 | 1.2 | 0.96 |
| YGR248W | 1.3 | 1.7 | 3.2 | 4.0 | 0.5 | 2.3 | 1.1 | 1.2 | 2.8 | 3.2 | 2.3 | 1.1 | 2.0 | 1.5 | 1.5 | 7.9 | 1.0 | 1.5 | 0.52 |
| YGR253C | 1.3 | 1.4 | 0.7 | 1.3 | 2.0 | 1.4 | 1.6 | 2.0 | 4.0 | 3.1 | 2.4 | 1.1 | 3.4 | 1.8 | 1.1 | 1.8 | 1.5 | 2.1 | 2.01 |
| YHR027C | 0.5 | 0.8 | 1.2 | 0.6 | 1.1 | 0.4 | 0.9 | 0.6 | 3.6 | 2.2 | 1.1 | 1.0 | 2.1 | 0.7 | 1.5 | 0.9 | 0.8 | 1.0 | 2.06 |
| YHR161C | 0.6 | 1.1 | 1.3 | 2.1 | 1.5 | 1.2 | 1.8 | 0.7 | 2.5 | 2.0 | 1.2 | 1.0 | 1.8 | 1.0 | 1.4 | 1.7 | 1.4 | 1.5 | 0.86 |
| YHR169W | 0.6 | 0.5 | 0.4 | 0.4 | 1.1 | 1.2 | 0.9 | 0.9 | 0.0 | 0.2 | 0.4 | 1.1 | 3.3 | 1.1 | 0.6 | 1.0 | 1.6 | 1.3 | 0.81 |
| YIL010W | 1.0 | 1.2 | 1.4 | 0.8 | 1.2 | 4.4 | 1.5 | 0.9 | 1.4 | 2.2 | 1.4 | 1.4 | 2.3 | 1.7 | 1.1 | 1.7 | 1.5 | 1.5 | 0.70 |
| YIL034C | 1.0 | 0.8 | 1.5 | 3.0 | 0.7 | 1.7 | 1.2 | 0.9 | 2.1 | 1.3 | 1.6 | 1.0 | 2.6 | 1.1 | 1.0 | 1.7 | 1.2 | 1.5 | 0.99 |
| YIL142W | 0.7 | 1.1 | 0.9 | 1.1 | 0.8 | 1.1 | 0.8 | 1.1 | 4.4 | 3.0 | 1.7 | 1.1 | 1.8 | 1.0 | 1.3 | 0.7 | 0.9 | 0.8 | 1.84 |
| YIR039C | 1.4 | 1.8 | 4.3 | 4.2 | 1.5 | 3.2 | 4.1 | 1.1 | 4.4 | 4.1 | 3.8 | 1.4 | 2.9 | 1.9 | 2.0 | 4.2 | 1.2 | 1.1 | 0.45 |
| YJL001W | 0.9 | 1.7 | 1.2 | 2.0 | | 1.7 | 1.6 | 1.3 | 3.1 | 2.8 | 1.5 | 1.6 | 2.9 | 2.5 | 1.2 | 1.8 | 1.0 | 1.6 | 3.00 |
| YJL035C | 0.9 | 1.3 | 0.8 | 0.6 | 1.4 | 0.7 | 1.3 | 1.5 | 1.7 | 1.4 | 0.8 | 1.1 | 2.4 | 1.6 | 0.7 | 0.6 | 1.0 | 1.0 | 0.82 |
| YJL053W | 1.1 | 0.9 | 1.7 | 1.3 | 1.1 | 2.0 | 1.7 | 1.3 | 2.2 | 2.2 | 1.6 | 1.1 | 1.9 | 1.3 | 1.1 | 1.7 | 1.3 | 1.6 | 0.80 |
| YJL164C | 1.1 | 1.1 | 1.0 | 1.9 | 1.1 | 2.7 | 3.1 | 0.9 | 2.1 | 0.8 | 1.5 | 1.0 | 2.1 | 0.9 | 0.8 | 2.1 | 1.3 | 1.7 | 1.04 |
| YJL210W | 1.2 | 1.4 | 1.6 | 2.0 | 0.6 | 0.6 | 1.0 | 1.0 | 2.0 | 0.9 | 2.4 | 1.0 | 2.2 | 0.9 | 0.8 | 2.3 | 0.9 | 1.0 | 0.70 |
| YJR117W | 1.0 | 2.1 | 2.3 | 0.7 | 1.2 | 1.3 | 1.0 | 0.8 | 4.7 | 2.3 | 1.0 | 1.2 | 2.8 | 0.8 | 2.2 | 0.7 | 0.8 | 0.9 | 1.34 |
| YKL007W | 1.2 | 1.6 | 1.2 | 0.9 | 1.0 | 0.9 | 1.5 | 1.2 | 2.3 | 2.4 | 1.8 | 1.3 | 2.5 | 0.7 | 1.0 | 1.0 | 1.4 | 1.3 | 1.13 |
| YKL117W | 1.0 | 1.3 | 0.8 | 1.0 | 1.8 | 1.0 | 1.3 | 1.5 | 2.9 | 2.1 | 1.6 | 1.0 | 2.4 | 1.6 | 1.6 | 1.8 | 1.2 | 1.8 | 3.73 |

| | | | | | | | | | | | | | | | | | | | |
|---|---|---|---|---|---|---|---|---|---|---|---|---|---|---|---|---|---|---|---|
| YKL193C | 1.3 | 0.8 | 1.1 | 1.4 | 1.1 | 0.9 | 2.1 | 1.4 | 1.0 | 1.6 | 1.7 | 0.9 | 2.1 | 1.2 | 1.2 | 2.4 | 1.2 | 2.3 | 0.71 |
| YLR120C | 1.2 | 1.9 | 2.4 | 1.6 | 1.1 | 1.9 | 1.5 | 0.9 | 1.8 | 1.6 | 1.2 | 1.6 | 4.8 | 0.8 | 2.7 | 1.7 | 3.5 | 2.9 | 1.53 |
| YLR136C | 1.3 | 9.0 | 0.6 | 3.3 | 1.6 | 1.7 | 3.9 | 1.1 | 0.9 | 6.0 | 1.2 | 1.3 | 2.9 | 0.7 | 2.1 | 1.2 | 1.8 | 1.4 | 0.41 |
| YLR178C | 1.7 | 6.9 | 8.8 | 4.6 | 1.0 | 4.2 | 3.3 | 0.9 | 4.3 | 4.1 | 3.7 | 2.2 | 7.5 | 2.8 | 2.3 | 10.6 | 2.0 | 3.6 | 1.03 |
| YLR327C | 5.5 | 3.3 | 14.4 | 8.2 | 1.3 | 5.1 | 5.7 | 2.2 | 3.0 | 8.3 | 5.0 | 1.4 | 2.8 | 1.5 | 0.8 | 2.8 | 3.3 | 5.3 | 2.23 |
| YLR356W | 1.0 | 2.9 | 4.9 | 1.2 | 1.1 | 3.4 | 1.1 | 0.7 | 2.3 | 1.6 | 3.1 | 1.5 | 2.5 | 2.2 | 2.8 | 0.9 | 1.1 | 1.0 | 0.41 |
| YLR362W | 1.1 | 1.2 | 1.0 | 3.0 | 1.5 | 0.8 | 1.9 | 1.4 | 3.5 | 3.3 | 1.5 | 0.8 | 2.4 | 1.2 | 1.3 | 1.9 | 1.2 | 1.3 | 0.35 |
| YLR429W | 0.7 | 1.0 | 0.6 | 0.9 | 1.4 | 0.8 | 1.3 | 1.0 | 0.9 | 1.3 | 0.9 | 1.1 | 2.3 | 0.7 | 1.5 | 1.0 | 1.0 | 0.9 | 0.86 |
| YMR004W | 0.9 | 0.9 | 1.3 | 1.3 | 1.0 | 0.5 | 1.2 | 1.0 | 94.2 | 3.4 | 2.5 | 1.0 | 2.2 | 2.2 | 1.3 | 1.3 | 0.9 | 1.3 | 0.52 |
| YMR219W | 0.7 | 1.1 | 1.0 | 0.7 | 1.6 | 0.8 | | 1.1 | 2.3 | 0.9 | 1.5 | 1.5 | 2.1 | 0.5 | 1.7 | 0.8 | 1.1 | 0.9 | 0.23 |
| YMR275C | 0.7 | 0.7 | 1.2 | 0.6 | 0.7 | 1.4 | 0.8 | 0.8 | 2.4 | 2.1 | 1.1 | 0.7 | 2.1 | 0.8 | 1.7 | 1.0 | 0.9 | 0.7 | 0.79 |
| YMR314W | | 1.2 | 0.9 | 1.3 | | 1.3 | 1.1 | 1.2 | 5.9 | 2.7 | 1.8 | 1.0 | 2.6 | 1.0 | 1.2 | 1.6 | 1.3 | 1.6 | 1.67 |
| YNL006W | 1.1 | 2.1 | 1.3 | 1.5 | 0.9 | 1.0 | 1.6 | 1.1 | 4.0 | 4.0 | 1.1 | 0.7 | 2.3 | 0.8 | 0.7 | 0.9 | 1.0 | 1.3 | 1.20 |
| YNL007C | 0.7 | 2.4 | 5.1 | 0.8 | 1.2 | 0.6 | 1.2 | 0.8 | 3.0 | 1.7 | 1.3 | 0.7 | 2.1 | 0.4 | 0.8 | 0.5 | 1.1 | 1.0 | 3.62 |
| YNL093W | 2.1 | 6.4 | 0.4 | 4.5 | 1.2 | 1.6 | 1.4 | 0.8 | 1.7 | 2.0 | 1.6 | 1.1 | 2.2 | 1.1 | 0.6 | 4.9 | 3.0 | 3.1 | 0.32 |
| YNL333W | 1.0 | 1.4 | 1.7 | 2.3 | 1.5 | 1.5 | 2.9 | 2.1 | 2.3 | 2.1 | 2.0 | 1.0 | 2.2 | 1.9 | 1.0 | 1.4 | 1.4 | 1.1 | 0.49 |
| YNR010W | 1.3 | 1.2 | 0.5 | 2.8 | 1.8 | 1.3 | 1.5 | 1.1 | 1.7 | 0.9 | 1.4 | 0.8 | 2.8 | 1.3 | 0.8 | 1.2 | 1.7 | 2.3 | 0.37 |
| YNR069C | 1.5 | 1.3 | 0.7 | 0.4 | 1.4 | 1.7 | 1.4 | 1.4 | 5.5 | 5.8 | 2.5 | 1.2 | 3.4 | 2.1 | 1.0 | 0.8 | 1.1 | 0.9 | 0.18 |
| YOL164W | 0.9 | 4.4 | 1.0 | 1.5 | 1.6 | 1.2 | 2.2 | 1.0 | 4.9 | 3.6 | 1.0 | 1.1 | 3.4 | 1.3 | 1.0 | 1.1 | 1.1 | 1.3 | 0.45 |
| YOR036W | 1.8 | 0.9 | 1.4 | 1.6 | 1.1 | 1.7 | 1.8 | 1.4 | 1.2 | 1.9 | 1.3 | 1.2 | 2.1 | 1.2 | 1.2 | 1.3 | 1.6 | 1.7 | 0.98 |
| YOR117W | 0.8 | 1.3 | 1.7 | 0.8 | 1.2 | 1.1 | 3.3 | 1.0 | 3.5 | 1.8 | 1.2 | 2.5 | 2.3 | 1.0 | 1.4 | 1.2 | 1.4 | 0.9 | 1.42 |
| YOR124C | 0.6 | 1.1 | 1.3 | 0.8 | 1.1 | 0.9 | 1.1 | 1.3 | 2.0 | 1.9 | 1.2 | 0.9 | 2.1 | 0.8 | 1.3 | 0.9 | 1.0 | 1.0 | 0.65 |
| YOR132W | 0.9 | 1.6 | 0.8 | 2.8 | 1.1 | 1.6 | 1.4 | 1.2 | 2.8 | 1.6 | 1.3 | 0.7 | 2.5 | 0.7 | 1.5 | 1.6 | 1.2 | 1.6 | 0.46 |
| YOR157C | 1.0 | 1.3 | 1.2 | 1.3 | 0.7 | 1.2 | 1.2 | 1.2 | 5.7 | 2.6 | 1.6 | 1.3 | 2.4 | 2.2 | 1.9 | 0.9 | 1.2 | 1.2 | 1.23 |
| YOR185C | 1.1 | 1.3 | 1.9 | 1.1 | 1.2 | 3.0 | 1.3 | 1.4 | 1.9 | 2.9 | 1.2 | 1.5 | 2.2 | 2.1 | 1.0 | 2.2 | 0.9 | 1.1 | 1.67 |
| YOR259C | 0.8 | 1.1 | 0.8 | 0.9 | 2.0 | 0.8 | 1.3 | 1.1 | 2.7 | 1.5 | 1.0 | 0.8 | 1.8 | 1.2 | 1.3 | 1.4 | 0.9 | 1.5 | 2.33 |
| YOR261C | 1.0 | 1.4 | 0.6 | 0.9 | 1.5 | 1.4 | 1.9 | 2.6 | 5.5 | 2.5 | 2.1 | 0.9 | 2.5 | 1.8 | 1.3 | 2.0 | 1.0 | 1.9 | 2.80 |
| YOR288C | 0.9 | 1.3 | 1.1 | 1.1 | 1.1 | 1.0 | 2.0 | 1.9 | 0.9 | 1.0 | 1.4 | 1.4 | 2.4 | 0.8 | 1.8 | 1.4 | 1.5 | 1.6 | 0.79 |
| YPL109C | 1.1 | 2.5 | 0.5 | 1.2 | 1.2 | 1.2 | 1.8 | 1.1 | 1.2 | 1.5 | -0.2 | 0.9 | 1.8 | 1.3 | 1.3 | 1.9 | 1.2 | 1.2 | 0.21 |
| YPL149W | 1.5 | 1.5 | 0.6 | 2.8 | 0.8 | 0.9 | 1.2 | 1.1 | 5.6 | 3.7 | 1.2 | 0.9 | 2.3 | 1.0 | 0.8 | 1.2 | 2.2 | 2.9 | 1.39 |
| YPL154C | 0.7 | 3.3 | 4.2 | 1.2 | 1.2 | 3.5 | 1.5 | 0.8 | 2.8 | 1.7 | 1.8 | 1.5 | 3.1 | 0.8 | 4.0 | 1.8 | 1.6 | 2.0 | 3.78 |
| YPR103W | 0.7 | 1.1 | 1.3 | 0.7 | 1.7 | 1.2 | 1.6 | 1.0 | 5.5 | 2.0 | 0.9 | 1.2 | 2.8 | 1.8 | 1.0 | 1.1 | 1.2 | 1.5 | 1.93 |

| | | | | | | | | | | | | | | | | | | | |
|---|---|---|---|---|---|---|---|---|---|---|---|---|---|---|---|---|---|---|---|
| YPR108W | 0.8 | 1.0 | 1.3 | 0.6 | 1.3 | 1.5 | 1.6 | 1.6 | 3.7 | 2.8 | 1.8 | 1.0 | 2.6 | 1.0 | 1.3 | 1.2 | 1.0 | 1.3 | 2.25 |
| YCL027W | 0.7 | 2.0 | 1.0 | 1.0 | 1.2 | 1.3 | 1.0 | 0.6 | 0.7 | 16.3 | 0.9 | 0.8 | 1.1 | 1.2 | 3.6 | 1.1 | 0.7 | 0.8 | 0.30 |
| YDR055W | 1.7 | 2.1 | 2.2 | 2.8 | 1.7 | 3.9 | 1.5 | 1.6 | 0.8 | 2.4 | 2.2 | 1.4 | 3.1 | 0.9 | 5.7 | 3.4 | 2.0 | 1.9 | 1.55 |
| YEL042W | 0.6 | 0.9 | 1.7 | 0.9 | 0.7 | 0.5 | 0.5 | 0.7 | 0.2 | 0.3 | 1.6 | 1.0 | 0.4 | 0.6 | 4.8 | 0.6 | 0.7 | 0.8 | 1.21 |
| YGR136W | 0.9 | 1.2 | 1.3 | 0.7 | 1.2 | 1.1 | 1.5 | 1.3 | 2.1 | 2.2 | 1.3 | 1.2 | 1.7 | 1.1 | 2.6 | 2.2 | 1.3 | 1.8 | 1.42 |
| YHR142W | 1.5 | 0.8 | 2.1 | 0.8 | 1.3 | 1.2 | 0.9 | 1.3 | 0.4 | 0.9 | 1.1 | 1.3 | 1.2 | 1.2 | 4.8 | 1.0 | 1.3 | 1.3 | 1.17 |
| YJL073W | 0.8 | 0.8 | 2.5 | 0.7 | 1.2 | 2.0 | | 1.0 | 1.7 | 0.7 | 0.5 | 1.0 | 1.6 | 1.1 | 2.6 | 0.8 | 1.1 | 1.0 | 0.43 |
| YJR004C | 0.2 | 0.8 | 3.4 | 0.6 | 1.1 | 1.2 | 0.4 | 0.4 | 0.3 | 0.7 | 0.7 | 0.8 | 0.3 | 0.7 | 3.2 | 0.3 | 0.4 | 0.4 | 2.12 |
| YKL039W | 0.8 | 1.1 | 2.4 | 0.8 | 0.7 | 4.9 | 2.0 | 0.8 | 1.3 | 3.3 | 1.0 | 0.9 | 1.9 | 0.8 | 3.1 | 1.5 | 1.2 | 1.3 | 1.13 |
| YLR250W | 1.5 | 1.0 | 1.0 | 1.4 | 1.4 | 1.1 | 1.9 | 1.3 | 2.7 | 2.4 | 1.4 | 1.0 | 1.2 | 2.5 | 2.6 | 2.1 | 1.4 | 2.2 | 2.90 |
| YOR181W | 1.0 | 0.9 | 1.1 | 1.4 | 0.7 | 0.9 | 0.7 | 0.7 | 0.6 | 0.5 | 1.0 | 0.8 | 1.2 | 0.9 | 3.4 | 1.0 | 1.1 | 0.6 | 0.39 |
| YOR198C | 1.0 | 0.7 | 1.4 | 1.0 | 0.8 | 0.9 | 1.6 | 1.3 | 2.4 | 1.4 | 1.4 | 1.5 | 1.8 | 1.1 | 3.8 | 0.8 | 1.1 | 1.5 | 1.76 |
| YPL089C | 1.2 | 1.0 | 0.5 | 1.8 | 1.4 | 0.8 | 0.9 | 0.8 | 0.4 | 2.5 | 0.9 | 0.7 | 1.3 | 0.8 | 2.6 | 0.9 | 1.5 | 1.1 | 0.37 |
| YBR214W | 0.9 | 2.1 | 5.7 | 2.0 | 1.1 | 0.8 | 1.1 | 0.7 | 2.2 | 3.5 | 3.2 | 1.2 | 2.1 | 0.9 | 5.1 | 1.3 | 1.1 | 1.2 | 0.51 |
| YDR085C | 1.9 | 1.8 | 1.6 | 3.4 | 0.9 | 2.1 | 4.1 | 2.0 | 0.4 | 2.3 | 1.7 | 1.6 | 1.1 | 1.6 | 3.1 | 2.3 | 1.3 | 1.8 | 0.37 |
| YDR259C | 1.4 | 1.0 | 0.4 | 1.4 | 1.0 | 0.5 | 2.8 | 1.4 | 1.0 | 1.8 | 1.2 | 1.0 | 2.0 | 1.5 | 2.9 | 0.9 | 1.0 | 0.8 | 0.28 |
| YDR388W | 0.7 | 0.7 | 1.2 | 1.0 | 0.7 | 1.0 | 1.1 | 0.8 | 1.4 | 0.9 | 1.6 | 0.8 | 1.7 | 0.6 | 2.6 | 1.0 | 1.2 | 1.3 | 0.99 |
| YDR432W | 0.5 | 0.8 | 1.0 | 1.4 | 1.6 | 0.6 | 0.5 | 0.5 | 0.7 | 0.8 | 0.6 | 0.7 | 1.4 | 0.5 | 2.6 | 0.6 | 0.7 | 0.7 | 2.38 |
| YDR481C | 0.7 | 2.3 | 1.3 | 0.7 | 1.0 | 0.9 | 0.7 | 1.0 | 1.9 | 1.6 | 1.2 | 0.9 | 1.5 | 0.9 | 3.5 | 1.3 | 1.2 | 1.4 | 1.43 |
| YDR510W | 1.0 | 1.3 | 1.5 | 1.3 | 0.7 | 1.0 | 1.6 | 1.5 | 2.7 | 2.5 | 1.6 | 1.4 | 2.0 | 1.9 | 2.2 | 1.5 | 1.1 | 1.5 | 1.57 |
| YGR189C | 1.1 | 0.9 | 1.5 | 3.5 | 0.7 | 0.5 | 0.6 | 1.0 | 0.3 | 0.9 | 1.5 | 1.3 | 1.2 | 0.9 | 9.3 | 0.6 | 2.3 | 1.7 | 1.51 |
| YIL123W | 1.0 | 0.5 | 1.2 | 1.5 | 0.6 | 0.5 | 0.5 | 0.6 | 0.1 | 0.2 | 1.2 | 0.8 | 0.4 | 0.3 | 4.8 | 0.7 | 1.3 | 1.0 | 1.67 |
| YIL140W | 0.7 | 1.0 | 1.5 | 1.4 | 0.8 | 0.5 | 0.5 | 0.7 | 0.7 | 0.6 | 0.9 | 1.0 | 0.5 | 0.9 | 3.3 | 0.6 | 0.7 | 1.0 | 0.62 |
| YKL096W | 1.4 | 0.6 | 8.1 | 1.6 | 0.6 | 0.6 | 0.2 | 0.5 | 0.0 | 0.5 | 3.5 | 0.3 | 0.1 | 0.5 | 2.3 | 0.4 | 0.5 | 0.7 | 2.04 |
| YLR391W | | 0.7 | 2.9 | 0.8 | 1.2 | 1.5 | 0.7 | 0.7 | 0.4 | 0.7 | 1.4 | 1.0 | 0.9 | 0.5 | 3.9 | 1.3 | 1.4 | 0.9 | 1.72 |
| YMR094W | 1.0 | 0.5 | 1.3 | 1.7 | 0.9 | 0.5 | 0.8 | 1.0 | -0.1 | 0.9 | 1.0 | 0.9 | 1.4 | 2.8 | 6.6 | 1.0 | 1.0 | 1.0 | 0.23 |
| YMR104C | 1.9 | 14.3 | 2.2 | 2.6 | 0.7 | 1.9 | 1.5 | 0.9 | 0.6 | 3.2 | 1.1 | 1.0 | 1.5 | 1.1 | 2.5 | 2.3 | 2.8 | 1.9 | 0.51 |
| YMR276W | 0.6 | 1.4 | 1.5 | 0.7 | 1.3 | 0.8 | 1.1 | 0.8 | 1.8 | 1.7 | 1.9 | 1.2 | 1.9 | 1.2 | 3.3 | 0.9 | 1.2 | 0.7 | 0.47 |
| YOL013C | 0.9 | 1.0 | 1.0 | 1.5 | 0.7 | 1.1 | 1.7 | 1.0 | 0.8 | 1.0 | 1.4 | 1.1 | 1.8 | 0.9 | 2.7 | 1.3 | 1.1 | 1.4 | 0.65 |
| YOR355W | 0.7 | 0.6 | 1.1 | 0.8 | 1.0 | 1.6 | 1.1 | 0.8 | 0.1 | 0.8 | 1.2 | 0.9 | 1.1 | 0.6 | 3.2 | 0.8 | 0.8 | 0.9 | 0.98 |
| YCR071C | 1.1 | 0.7 | 0.9 | 0.8 | 1.2 | 2.5 | 1.4 | 0.9 | 0.6 | 1.2 | 1.1 | 1.1 | 1.3 | 1.7 | 1.1 | 1.5 | 1.2 | 1.3 | 0.96 |
| YDL008W | 1.1 | 1.0 | 1.0 | 1.1 | 1.8 | 2.7 | 2.0 | 1.2 | 2.3 | 3.0 | 0.9 | 1.2 | 2.0 | 2.2 | 0.7 | 1.3 | 1.5 | 1.6 | 0.95 |

| | | | | | | | | | | | | | | | | | | | |
|---|---|---|---|---|---|---|---|---|---|---|---|---|---|---|---|---|---|---|---|
| YDR115W | 1.3 | 0.9 | 0.6 | 1.5 | 1.4 | 2.3 | 1.6 | 1.3 | 1.1 | 1.0 | 1.2 | 0.9 | 1.1 | 1.5 | 0.8 | 2.2 | 1.2 | 1.4 | 1.50 |
| YER130C | 0.9 | 1.6 | 0.5 | 0.8 | 1.7 | 2.7 | 1.5 | 1.2 | 0.5 | 1.3 | 0.9 | 1.0 | 1.6 | 0.8 | 1.1 | 0.7 | 3.0 | 2.5 | 1.36 |
| YMR226C | 1.1 | 1.8 | 1.4 | 1.6 | 0.9 | 2.6 | 1.8 | 1.6 | 2.6 | 2.2 | 1.4 | 1.1 | 1.6 | 1.6 | 2.0 | 1.7 | 1.6 | 2.0 | 2.03 |
| YOR383C | 0.9 | 4.3 | 2.6 | 0.9 | 0.9 | 6.8 | 8.0 | 1.0 | 1.2 | 4.5 | 1.4 | 1.1 | 1.2 | 0.6 | 1.1 | 3.0 | 1.4 | 1.1 | 0.68 |
| YAR010C | 0.8 | 1.2 | 1.2 | 0.8 | 1.0 | 2.3 | 1.9 | 1.3 | 1.2 | 1.1 | 0.9 | 1.0 | 1.4 | 1.0 | 1.8 | 0.7 | 1.5 | 1.0 | 4.69 |
| YBL043W | 1.2 | 1.2 | 1.1 | 1.3 | 1.1 | 26.9 | 1.5 | 2.1 | 0.5 | 14.7 | 1.5 | 0.9 | 1.8 | 1.9 | 1.8 | 2.3 | 2.3 | 1.8 | 0.47 |
| YCR004C | 1.7 | 1.0 | 12.0 | | 1.2 | 1.8 | 1.4 | 1.2 | 4.7 | 2.1 | 1.9 | 0.8 | 1.3 | 3.7 | 0.8 | 4.9 | 1.4 | 3.5 | 3.02 |
| YCR088W | 0.9 | 1.1 | 1.2 | 0.9 | 1.1 | 2.0 | 1.2 | 0.5 | 0.8 | 0.9 | 1.0 | 1.3 | 1.9 | 0.9 | 2.8 | 0.7 | 1.4 | 0.9 | 0.47 |
| YDL238C | 0.7 | 1.0 | 1.1 | 1.7 | 0.9 | 2.1 | 1.4 | 5.8 | 1.7 | 2.7 | 1.1 | 0.9 | 2.0 | 1.2 | 1.3 | 1.7 | 1.1 | 1.3 | 0.35 |
| YDR084C | 0.9 | 0.9 | 1.5 | 1.1 | 1.2 | 2.1 | 0.9 | 0.7 | 0.6 | 0.6 | 0.7 | 0.7 | 1.0 | 1.0 | 3.9 | 1.0 | 2.0 | 1.3 | 1.12 |
| YDR104C | 0.8 | 1.1 | 1.2 | 1.2 | 1.3 | 1.7 | 1.0 | 1.3 | 2.8 | 2.7 | 1.0 | 1.1 | 1.6 | 1.4 | 1.4 | 0.9 | 0.9 | 1.0 | 0.34 |
| YDR315C | 1.0 | 1.4 | 1.0 | 1.3 | 0.9 | 9.4 | 1.4 | 1.4 | 1.1 | 1.6 | 1.1 | 0.6 | 1.6 | 1.4 | 1.5 | 1.5 | 1.2 | 1.2 | 0.48 |
| YDR358W | 0.7 | 1.1 | 0.7 | 1.1 | 1.4 | 2.5 | 2.8 | 1.2 | 2.9 | 3.2 | 1.0 | 1.1 | 3.2 | 1.2 | 0.9 | 2.2 | 1.9 | 1.9 | 0.50 |
| YEL066W | 2.0 | 2.9 | 1.5 | 2.2 | 0.8 | 2.2 | 1.8 | 1.4 | 1.3 | 2.4 | 1.9 | 0.8 | 1.6 | 1.9 | 1.0 | 1.9 | 1.0 | 1.1 | 0.64 |
| YER039C | 1.0 | 1.2 | 1.0 | 4.1 | 1.5 | 1.6 | 1.3 | 0.7 | 1.2 | 0.8 | 1.2 | 0.9 | 2.1 | 1.2 | 1.2 | 1.8 | 1.7 | 1.1 | 0.42 |
| YER107C | 0.9 | 0.5 | 1.0 | 0.6 | 1.3 | 2.5 | 1.0 | 0.8 | 0.4 | 0.5 | 0.7 | 0.7 | 0.9 | 1.1 | 0.7 | 0.9 | 1.1 | 1.2 | 0.92 |
| YFL028C | 1.2 | 1.1 | 0.7 | 1.5 | 1.7 | 1.7 | 2.0 | 1.5 | 2.7 | 1.8 | 1.3 | 1.1 | 1.4 | 1.6 | 0.8 | 1.9 | 1.2 | 1.5 | 1.28 |
| YFL043C | 1.0 | 1.2 | 0.6 | 1.2 | 1.2 | 1.8 | 1.8 | 1.0 | 2.2 | 3.8 | 1.1 | 0.9 | 1.5 | 1.5 | 1.1 | 2.0 | 1.5 | 1.2 | 0.38 |
| YGL229C | 0.6 | 0.7 | 0.9 | 0.8 | 1.0 | 2.2 | 2.2 | 0.7 | 1.8 | 1.7 | 1.1 | 1.1 | 1.7 | 1.0 | 0.2 | 1.8 | 1.1 | 0.9 | 0.44 |
| YGR257C | 0.7 | 0.8 | 0.9 | 1.1 | 1.3 | 2.1 | 2.4 | 1.0 | 1.5 | 1.9 | 1.2 | 0.9 | 2.0 | 0.9 | 1.3 | 1.3 | 1.1 | 1.3 | 0.84 |
| YHR004C | 0.9 | 1.1 | 0.8 | 1.1 | 1.5 | 2.0 | 1.5 | 0.9 | 1.1 | 0.6 | 0.8 | 0.9 | 1.1 | 0.8 | 1.2 | 2.4 | 1.4 | 1.6 | 0.84 |
| YHR071W | 1.5 | 1.1 | 0.6 | 1.6 | 1.5 | 2.0 | 1.0 | 1.7 | 1.0 | 7.2 | 1.8 | 0.8 | 2.3 | 1.6 | 3.0 | 1.7 | 2.6 | 1.0 | 0.79 |
| YJL089W | 1.1 | 1.5 | 1.0 | 0.8 | 1.1 | 1.6 | 1.1 | 1.1 | 1.6 | 3.9 | 0.6 | 1.2 | 1.8 | 1.8 | 6.4 | 1.2 | 1.2 | 1.1 | 0.21 |
| YJL116C | 0.9 | 1.8 | 1.3 | 1.9 | 1.3 | 3.4 | 0.8 | 1.8 | 2.4 | 2.6 | 2.9 | 1.1 | 4.3 | 2.6 | 1.0 | 1.3 | 1.2 | 0.7 | 1.01 |
| YJR086W | 1.2 | 1.1 | 0.8 | 1.4 | 1.3 | 1.9 | 1.7 | 1.1 | 1.4 | 1.1 | 1.0 | 0.8 | 0.9 | 2.2 | 2.0 | 1.4 | 1.4 | 1.6 | 1.24 |
| YKL008C | 1.2 | 0.6 | 2.0 | 0.8 | 0.9 | 3.8 | 1.4 | 1.2 | 0.3 | 0.4 | 1.5 | 1.5 | 1.7 | 0.8 | 1.3 | 2.1 | 1.4 | 1.9 | 2.11 |
| YKL013C | 1.4 | 0.9 | 1.4 | 1.1 | 1.9 | 1.9 | 1.8 | 0.9 | 1.5 | 1.4 | 1.1 | 0.7 | 1.3 | 1.4 | 1.2 | 1.5 | 1.6 | 1.7 | 1.55 |
| YKL041W | 1.2 | 0.8 | 1.1 | 1.0 | 1.3 | 2.2 | 1.0 | 1.6 | 1.6 | 1.5 | 1.2 | 0.8 | 1.3 | 1.3 | 1.0 | 2.5 | 1.1 | 1.3 | 0.91 |
| YKL139W | 0.9 | 0.7 | 1.4 | 0.8 | 2.8 | 3.5 | 2.6 | 1.2 | 1.6 | 1.5 | 1.4 | 0.8 | 1.6 | 1.0 | 1.0 | 1.3 | 1.9 | 0.8 | 0.62 |
| YKR014C | 1.1 | 1.3 | 0.7 | 1.1 | 1.5 | 2.1 | 1.7 | 1.6 | 2.4 | 2.0 | 1.1 | 1.1 | 1.5 | 1.8 | 1.4 | 2.4 | 1.0 | 1.7 | 2.05 |
| YLR093C | 1.3 | 0.8 | 0.7 | 1.2 | 1.4 | 2.9 | 1.9 | 1.2 | 1.5 | 1.3 | 1.0 | 1.0 | 1.3 | 1.3 | 0.9 | 2.4 | 1.7 | 3.2 | 1.90 |
| YLR118C | 1.0 | 1.1 | 1.6 | 1.0 | 1.0 | 2.5 | 1.5 | 1.0 | 1.1 | 1.3 | 1.2 | 1.0 | 1.4 | 1.5 | 0.8 | 3.5 | 1.3 | 1.9 | 1.17 |

| | | | | | | | | | | | | | | | | | | | |
|---|---|---|---|---|---|---|---|---|---|---|---|---|---|---|---|---|---|---|---|
| YLR251W | 1.8 | 0.8 | 4.2 | 4.6 | 1.6 | 2.1 | 2.6 | 1.2 | 2.1 | 1.3 | 4.1 | 0.8 | 1.7 | 1.3 | 0.8 | 2.5 | 2.1 | 3.8 | 0.78 |
| YMR027W | 0.8 | 2.6 | 0.7 | 0.8 | 1.1 | 2.1 | 2.0 | 0.9 | 1.1 | 1.4 | 1.1 | 0.9 | 2.4 | 0.9 | 4.0 | 0.8 | 1.3 | 1.2 | 4.05 |
| YMR262W | 1.0 | 1.3 | 1.9 | 1.1 | 1.2 | 2.0 | 1.9 | 1.0 | 3.0 | 1.2 | 1.3 | 0.8 | 1.7 | 0.9 | 0.3 | 1.0 | 1.0 | 1.2 | 0.65 |
| YNL214W | 1.2 | 0.9 | 1.1 | 1.1 | 1.4 | 2.3 | 1.3 | 1.1 | 1.5 | 1.5 | 0.6 | 0.8 | 1.2 | 1.7 | 0.9 | 2.0 | 1.1 | 1.2 | 0.47 |
| YOR149C | 0.7 | 0.9 | 1.0 | 0.8 | 1.1 | 2.3 | 1.0 | 0.7 | 0.4 | 1.0 | 1.0 | 0.9 | 1.3 | 0.9 | 1.3 | 1.1 | 1.0 | 1.2 | 0.76 |
| YOR165W | 0.9 | 1.5 | 0.7 | 0.8 | 0.8 | 2.2 | 0.9 | 1.1 | 0.8 | 1.3 | 1.3 | 0.7 | 0.9 | 0.8 | 0.8 | 1.0 | 0.9 | 1.0 | 1.03 |
| YOR285W | 1.4 | 6.0 | 3.5 | 1.9 | 1.7 | 2.1 | 4.0 | 1.2 | 4.0 | 2.9 | 2.3 | 0.9 | 2.1 | 1.6 | 2.0 | 2.9 | 2.3 | 3.3 | 2.62 |
| YOR367W | 1.0 | 0.9 | 1.7 | 0.9 | 1.1 | 3.3 | 1.9 | 1.1 | 1.1 | 1.1 | 1.4 | 0.5 | 0.8 | 1.5 | 1.1 | 1.3 | 1.4 | 1.4 | 0.60 |
| YPL018W | 1.0 | 1.4 | 1.0 | 0.5 | 1.8 | 2.1 | 0.9 | 1.1 | 1.4 | 1.2 | 1.3 | 1.1 | 2.2 | 1.3 | 0.9 | 0.8 | 1.3 | 0.8 | 0.22 |
| YPL203W | 1.3 | 1.0 | 2.2 | 1.0 | 2.1 | 1.8 | 2.1 | 1.1 | 1.8 | 2.1 | 1.5 | 0.9 | 1.5 | 1.0 | 0.6 | 2.3 | 1.3 | 1.7 | 0.79 |
| YPL255W | 0.8 | 0.3 | 1.1 | 0.6 | 0.9 | 2.0 | 0.6 | 0.9 | 0.1 | 0.1 | 0.7 | 0.6 | 0.6 | 0.7 | 0.6 | 0.7 | 0.8 | 0.7 | 0.55 |
| YPR073C | 1.2 | 1.9 | 0.5 | 1.4 | 1.8 | 2.0 | 2.5 | 1.5 | 1.3 | 2.6 | 0.9 | 0.8 | 1.4 | 1.5 | 0.8 | 1.8 | 1.4 | 1.8 | 1.55 |
| YDR518W | 0.8 | 1.7 | 1.1 | 1.2 | 1.6 | 1.2 | 2.7 | 1.1 | 0.2 | 1.2 | 1.0 | 1.5 | 1.8 | 0.8 | 2.5 | 1.7 | 1.4 | 1.6 | 0.93 |
| YOR381W | 0.8 | 2.0 | 0.8 | 1.8 | 1.0 | 1.0 | 3.6 | 0.8 | 1.2 | 3.5 | 2.6 | 1.1 | 1.7 | 0.8 | 0.6 | 2.5 | 0.8 | 0.9 | 0.38 |
| YBR109C | 1.6 | 1.3 | 2.4 | 1.1 | 1.0 | 1.4 | 2.4 | 1.7 | 3.8 | 3.3 | 1.5 | 1.4 | 2.0 | 2.0 | 1.4 | 1.9 | 1.4 | 1.9 | 2.15 |
| YBR201W | 1.2 | 4.3 | 1.2 | 2.4 | 1.2 | 1.0 | 3.4 | 2.2 | 0.8 | 1.0 | 1.1 | 1.7 | 1.9 | 1.1 | 1.6 | 1.5 | 1.8 | 1.6 | 0.75 |
| YDR041W | 1.2 | 1.1 | 0.6 | 1.8 | 2.0 | 1.6 | 2.4 | 1.2 | 1.3 | 1.3 | 1.2 | 0.8 | 0.9 | 1.4 | 1.0 | 2.4 | 1.5 | 2.5 | 1.85 |
| YER136W | 0.9 | 1.1 | 1.1 | 1.4 | 1.6 | 1.3 | 2.1 | 1.3 | 1.4 | 1.6 | 1.7 | 0.9 | 1.3 | 0.8 | 1.2 | 1.6 | 1.3 | 1.7 | 2.31 |
| YER159C | 1.1 | 0.9 | 0.2 | 1.6 | 2.1 | 1.3 | 2.0 | 1.4 | 0.2 | 0.9 | 1.6 | 0.9 | 0.9 | 1.1 | 2.2 | 1.1 | 1.6 | 1.9 | 0.99 |
| YJL030W | 1.6 | 1.4 | 0.5 | 1.0 | 1.3 | 1.1 | 1.9 | 1.6 | 2.3 | 1.9 | 1.4 | 1.0 | 1.4 | 2.1 | 1.5 | 1.9 | 1.3 | 1.5 | 0.87 |
| YJR029W | 0.8 | 1.3 | 0.6 | 0.8 | | 1.0 | 2.4 | 0.9 | 1.1 | 1.5 | 0.6 | 1.0 | 2.1 | 1.1 | 1.8 | 0.7 | 0.8 | 1.1 | 5.84 |
| YJR099W | 1.4 | 0.9 | 1.1 | 1.3 | 1.5 | 2.1 | 2.0 | 1.4 | 1.4 | 2.4 | 1.3 | 0.7 | 1.5 | 2.1 | 0.5 | 2.0 | 1.1 | 1.7 | 0.94 |
| YJR122W | 2.0 | 1.4 | 2.5 | 1.1 | 1.3 | 1.4 | 1.8 | 1.4 | 5.3 | 6.1 | 2.4 | 0.9 | 1.3 | 1.4 | 0.8 | 1.9 | 1.0 | 1.1 | 0.43 |
| YJR125C | 1.0 | 1.0 | 1.2 | 1.0 | 1.2 | 1.3 | 1.9 | 0.9 | 1.8 | 1.3 | 1.1 | 0.9 | 1.5 | 1.1 | 1.3 | 2.0 | 1.2 | 1.4 | 0.90 |
| YKL190W | 1.1 | 1.3 | 0.4 | 0.9 | | 1.5 | 2.0 | 1.1 | 1.8 | 1.9 | 1.5 | 0.9 | 1.3 | 1.7 | 1.4 | 1.8 | 1.3 | 1.3 | 1.14 |
| YLL051C | 0.8 | 1.1 | 1.0 | 1.5 | 1.4 | 1.2 | 2.0 | 0.9 | 0.9 | 1.8 | 0.7 | 1.1 | 1.3 | 0.8 | 1.2 | 0.8 | 1.0 | 1.0 | 0.81 |
| YLR090W | 0.7 | 1.1 | 0.8 | 1.3 | 1.2 | 1.3 | 2.2 | 0.9 | 1.8 | 1.4 | 1.0 | 0.8 | 1.7 | 1.0 | 0.8 | 1.9 | 0.8 | 1.0 | 0.67 |
| YMR051C | 0.9 | 2.8 | 0.8 | 1.3 | 1.7 | 1.2 | 2.6 | 0.9 | 1.0 | 1.4 | 1.1 | 0.9 | 1.3 | 1.2 | 1.7 | 1.0 | 1.8 | 1.2 | 3.91 |
| YMR139W | 1.0 | 3.4 | 2.5 | 1.5 | 1.2 | 1.5 | 2.2 | 0.9 | 2.8 | 2.4 | 1.3 | 0.9 | 1.6 | 1.3 | 0.9 | 2.3 | 1.1 | 2.0 | 0.89 |
| YNL015W | 2.3 | 6.7 | 1.8 | 2.4 | 1.8 | 1.7 | 3.5 | 2.2 | 2.1 | 4.3 | 2.1 | 2.8 | 3.9 | 1.6 | 1.4 | 5.3 | 3.0 | 5.2 | 1.03 |
| YNL079C | 1.1 | 1.0 | 0.4 | 1.0 | 1.7 | 1.1 | 1.9 | 1.6 | 1.5 | 1.2 | 1.3 | 1.2 | 1.8 | 1.0 | 1.5 | 1.4 | 1.0 | 1.9 | 3.26 |
| YNL223W | 1.1 | 1.2 | 0.6 | 1.7 | 1.0 | 1.8 | 2.2 | 1.4 | 3.3 | 1.9 | 1.3 | 1.3 | 2.0 | 1.2 | 1.0 | 2.1 | 1.0 | 1.5 | 0.34 |

| | | | | | | | | | | | | | | | | | | | |
|---|---|---|---|---|---|---|---|---|---|---|---|---|---|---|---|---|---|---|---|
| YNR007C | 1.2 | 1.8 | 0.7 | 1.0 | 1.5 | 1.3 | 1.9 | 1.2 | 2.5 | 2.0 | 0.7 | 0.9 | 1.5 | 1.5 | 0.6 | 2.0 | 1.4 | 1.6 | 0.49 |
| YNR035C | 0.8 | 1.2 | 1.7 | 1.0 | 0.8 | 1.1 | 2.6 | 1.1 | 2.9 | 1.5 | 1.5 | 1.1 | 1.7 | 1.0 | 1.3 | 1.6 | 1.4 | 1.8 | 1.60 |
| YOL016C | 1.3 | 2.0 | 0.4 | 2.0 | 1.6 | 0.9 | 3.0 | 1.3 | 1.9 | 1.6 | 1.3 | 1.4 | 3.0 | 0.7 | 0.5 | 1.6 | 4.3 | 3.8 | 2.10 |
| YOL104C | 1.0 | 1.7 | 0.2 | 1.8 | 1.9 | 1.4 | 2.6 | 1.4 | 0.2 | 1.7 | 1.2 | 0.8 | 0.6 | 2.2 | 1.3 | 0.5 | 1.1 | 0.9 | 0.32 |
| YPR107C | 1.4 | 1.0 | 1.2 | 1.9 | 1.5 | 1.3 | 2.0 | 1.3 | 2.4 | 2.4 | 1.2 | 0.7 | 1.4 | 1.7 | 1.0 | 1.4 | 1.2 | 1.5 | 0.81 |
| YOL152W | 0.2 | 0.6 | 1.0 | 0.8 | 0.7 | 1.8 | 2.4 | 4.1 | 0.9 | 1.2 | 5.9 | 0.8 | 0.5 | 0.4 | 0.8 | 1.2 | 1.1 | 1.2 | 0.91 |
| YAL007C | 1.3 | 0.9 | 0.9 | 1.5 | 0.6 | 0.7 | 1.5 | 1.7 | 1.1 | 0.9 | 2.0 | 1.3 | 2.0 | 1.3 | 2.5 | 0.8 | 1.2 | 1.5 | 1.74 |
| YDL043C | 1.0 | 0.8 | 0.8 | 1.2 | 1.1 | 0.5 | 0.9 | 1.3 | 0.8 | 0.8 | 3.9 | 0.9 | 0.8 | 1.2 | 0.8 | 1.2 | 0.8 | 0.8 | 0.78 |
| YDL212W | 1.0 | 1.2 | 1.6 | 1.2 | 1.1 | 0.4 | 0.7 | 0.9 | 0.4 | 0.6 | 2.0 | 0.8 | 0.5 | 1.0 | 1.1 | 0.9 | 1.1 | 1.7 | 4.27 |
| YDR183W | 1.2 | 0.7 | 0.9 | 1.4 | 1.5 | 1.4 | 1.8 | 1.8 | 2.5 | 2.9 | 1.8 | 1.1 | 1.4 | 1.9 | 1.4 | 1.3 | 0.8 | 1.0 | 0.61 |
| YGL089C | 0.4 | 0.7 | 2.0 | 1.0 | 1.5 | 0.5 | 0.4 | 1.0 | 0.0 | 1.9 | 2.3 | 0.4 | 0.2 | 1.4 | 0.9 | 0.2 | 0.3 | 0.6 | 3.00 |
| YGL096W | 1.9 | 2.9 | 1.2 | 2.5 | 0.9 | 0.5 | 1.8 | 1.7 | 2.1 | 3.6 | 1.7 | 1.1 | 1.2 | 1.4 | 0.8 | 1.7 | 1.1 | 1.1 | 0.54 |
| YGR006W | 1.2 | 1.1 | 0.9 | 2.9 | 0.5 | 0.5 | 1.0 | 1.2 | 0.7 | 0.9 | 1.6 | 0.8 | 1.4 | 1.1 | 1.2 | 1.5 | 0.9 | 1.0 | 0.40 |
| YHL034C | 0.7 | 1.0 | 0.7 | 0.8 | 1.9 | 1.1 | 1.4 | 1.4 | 1.9 | 1.9 | 2.2 | 0.8 | 1.6 | 1.0 | 1.4 | 1.6 | 1.2 | 1.5 | 2.11 |
| YHR163W | 0.9 | 1.2 | 1.3 | 1.2 | 0.8 | 1.9 | 0.9 | 1.3 | 1.4 | 1.1 | 2.9 | 0.7 | 1.1 | 1.5 | 0.7 | 1.2 | 1.1 | 1.2 | 1.71 |
| YIR024C | 1.5 | 0.9 | 0.9 | 2.0 | 1.1 | 0.8 | 1.5 | 1.4 | 5.6 | 2.9 | 6.3 | 1.1 | 1.6 | 2.6 | 0.9 | 2.2 | 0.9 | 1.4 | 0.75 |
| YKL070W | 1.0 | 0.8 | 1.7 | 0.9 | 1.3 | 1.1 | 1.1 | 1.5 | 15.0 | 11.0 | 10.4 | 0.6 | 1.0 | 2.2 | 0.8 | 0.9 | 1.0 | 0.9 | 0.29 |
| YLL050C | 1.4 | 1.0 | 1.2 | 1.7 | | 1.9 | 1.4 | 1.3 | 1.0 | 1.0 | 2.3 | 0.9 | 1.8 | 1.2 | 1.6 | 1.3 | 1.2 | 2.1 | 3.43 |
| YLR220W | 0.9 | 0.6 | 3.0 | 1.0 | 0.9 | 0.3 | 0.4 | 1.0 | 0.4 | 0.4 | 2.4 | 0.7 | 0.5 | 1.4 | 0.7 | 1.1 | 0.8 | 0.8 | 2.28 |
| YLR390W | 0.9 | 0.9 | 1.2 | 1.7 | 1.2 | 0.7 | 1.5 | 0.8 | 1.7 | 1.7 | 1.9 | 1.0 | 1.1 | 1.1 | 0.5 | 2.0 | 1.0 | 0.9 | 0.51 |
| YOL044W | 1.0 | 1.4 | 1.0 | 1.3 | 1.0 | 1.2 | 1.2 | 1.1 | 1.6 | 1.4 | 2.1 | 0.7 | 1.3 | 1.1 | 0.6 | 1.3 | 1.6 | 1.9 | 0.65 |
| YOL147C | 1.6 | 1.1 | 2.4 | 1.5 | 0.7 | 0.9 | 0.6 | 1.5 | 0.5 | 0.7 | 2.8 | 0.7 | 0.8 | 0.9 | 0.8 | 2.4 | 2.0 | 1.7 | 1.09 |
| YDR069C | 1.4 | 0.8 | 3.0 | 1.5 | 1.6 | 0.7 | 1.0 | 1.8 | 1.8 | 4.5 | 0.8 | 0.7 | 1.3 | 1.2 | 1.0 | 1.3 | 1.0 | 1.3 | 0.33 |
| YER131W | 1.5 | 0.3 | 0.9 | 0.7 | 1.6 | 0.9 | 0.6 | 2.0 | 0.0 | 0.6 | 1.0 | 0.9 | 0.2 | 0.8 | 0.9 | 0.4 | 0.9 | 0.9 | 5.32 |
| YGR044C | 0.8 | 1.3 | 0.7 | 1.2 | 1.1 | 1.4 | 1.3 | 1.8 | 0.9 | 0.8 | 2.1 | 0.8 | 1.4 | 1.0 | 0.5 | 3.3 | 1.1 | 2.6 | 1.43 |
| YMR240C | 1.1 | 0.6 | 2.5 | 0.7 | 1.4 | 0.8 | 1.1 | 2.3 | 0.5 | 1.1 | 1.4 | 0.6 | 0.8 | 1.1 | 1.0 | 1.0 | 1.1 | 1.1 | 0.39 |
| YBR105C | 1.7 | 1.5 | 0.9 | 2.0 | 0.8 | 1.8 | 2.0 | 1.2 | 1.1 | 3.3 | 1.1 | 1.2 | 1.1 | 1.7 | 1.4 | 1.3 | 1.3 | 1.1 | 0.96 |
| YBR182C | 2.0 | 1.9 | 1.0 | 2.4 | 1.4 | 1.0 | 1.7 | 1.3 | 0.6 | 3.2 | 1.5 | 1.3 | 1.2 | 1.2 | 3.1 | 1.1 | 3.4 | 1.6 | 0.41 |
| YBR186W | 1.0 | 1.0 | 1.1 | 1.7 | 1.0 | 0.5 | 0.9 | 0.9 | 1.0 | 2.4 | 0.9 | 0.8 | 0.7 | 1.1 | 0.9 | 1.2 | 0.7 | 1.0 | 0.31 |
| YEL052W | 1.1 | 1.0 | 2.0 | 2.0 | 1.3 | 1.2 | 1.2 | 1.2 | 3.0 | 3.4 | 1.7 | 1.1 | 1.5 | 1.1 | 0.7 | 2.1 | 0.9 | 1.0 | 0.90 |
| YER098W | 0.9 | 0.9 | 0.5 | 1.1 | 1.4 | 1.6 | 1.3 | 1.7 | 2.8 | 3.3 | 1.4 | 1.0 | 1.1 | 1.3 | 0.9 | 1.7 | 1.2 | 1.5 | 0.35 |
| YGL240W | 0.9 | 1.2 | 0.8 | 1.8 | 1.2 | 1.1 | 1.2 | 0.8 | 2.0 | 3.4 | 0.9 | 0.8 | 1.7 | 1.3 | 1.1 | 1.0 | 1.2 | 0.8 | 0.48 |

| | | | | | | | | | | | | | | | | | | | |
|---|---|---|---|---|---|---|---|---|---|---|---|---|---|---|---|---|---|---|---|
| YGR067C | 2.2 | 1.4 | 1.2 | 4.6 | 1.6 | 1.3 | 1.5 | 1.5 | 0.7 | 6.7 | 1.0 | 0.7 | 1.0 | 0.9 | 1.2 | 1.7 | 1.2 | 1.0 | 0.11 |
| YGR133W | 1.0 | 1.1 | 0.7 | 0.6 | 1.3 | 1.2 | 1.2 | 1.2 | 1.3 | 3.6 | 1.6 | 0.9 | 1.2 | 1.4 | 1.0 | 1.5 | 1.0 | 1.0 | 0.35 |
| YHR124W | 1.1 | 1.1 | 1.5 | 2.0 | 0.9 | 0.8 | 1.2 | 1.1 | 3.0 | 6.8 | 1.1 | 1.2 | 1.7 | 2.7 | 0.8 | 1.8 | 1.3 | 1.1 | 0.24 |
| YJL103C | 1.3 | 1.8 | 3.8 | 1.3 | 1.1 | 1.0 | 0.7 | 0.8 | 2.3 | 2.4 | 1.5 | 0.8 | 1.4 | 1.0 | 1.3 | 0.9 | 1.4 | 1.2 | 0.44 |
| YJR036C | 1.2 | 1.2 | 1.0 | 4.3 | 1.5 | 0.9 | 1.0 | 1.0 | 1.4 | 3.0 | 1.1 | 0.9 | 1.1 | 1.3 | 1.8 | 2.0 | 0.9 | 1.2 | 0.31 |
| YLR216C | 1.2 | 2.3 | 3.3 | 1.1 | 1.2 | 1.2 | 5.2 | 1.3 | 14.1 | 4.1 | 1.8 | 0.7 | 1.2 | 1.1 | 1.2 | 1.5 | 1.2 | 1.3 | 1.44 |
| YLR389C | 0.8 | 6.4 | 2.3 | 0.6 | 0.7 | 0.2 | 1.0 | 1.0 | 2.4 | 2.8 | 0.9 | 0.8 | 1.9 | 0.8 | 1.0 | 1.4 | 1.0 | 1.0 | 0.60 |
| YNL128W | 1.3 | 4.6 | 1.1 | 0.8 | 1.2 | 1.1 | 1.0 | 1.3 | 2.9 | 2.4 | 0.9 | 0.9 | 1.4 | 1.4 | 1.1 | 0.6 | 0.9 | 0.9 | 0.21 |
| YOL133W | 1.0 | 1.6 | 1.4 | 1.2 | 0.9 | 1.6 | 1.8 | 1.0 | 4.7 | 3.8 | 1.5 | 0.6 | 1.0 | 1.4 | 1.3 | 2.0 | 1.3 | 1.3 | 0.87 |
| YOR133W | 1.1 | 1.3 | 1.6 | 1.3 | 1.3 | 0.9 | 1.1 | 0.8 | 3.1 | 4.9 | 1.3 | 0.8 | 1.3 | 1.1 | 1.8 | 1.3 | 1.2 | 1.2 | 0.26 |
| YOR227W | 1.1 | 2.4 | 3.2 | 0.9 | 0.5 | 1.0 | 1.0 | 0.7 | 2.9 | 3.0 | 1.2 | 1.0 | 1.7 | 1.1 | 3.7 | 2.2 | 0.8 | 1.3 | 0.35 |
| YPR015C | 1.3 | 1.3 | 1.5 | 1.7 | 1.1 | 0.7 | 1.3 | 1.2 | 5.6 | 5.3 | 1.0 | 0.7 | 1.5 | 1.5 | 1.6 | 2.0 | 1.0 | 1.0 | 0.32 |
| YPR086W | 1.0 | 1.4 | 0.7 | 0.7 | 1.2 | 1.1 | 1.6 | 1.3 | 3.0 | 2.9 | 1.3 | 0.8 | 1.0 | 1.0 | 1.3 | 1.4 | 1.2 | 1.2 | 1.37 |
| YBL056W | 0.7 | 0.7 | 1.1 | 1.2 | 1.8 | 1.6 | 1.4 | 1.3 | 3.6 | 2.2 | 0.7 | 0.9 | 2.7 | 0.9 | 1.0 | 1.7 | 1.1 | 1.5 | 2.49 |
| YBR026C | 1.1 | 1.7 | 2.8 | 0.9 | 1.2 | 0.1 | | 1.1 | 3.4 | 1.6 | 1.6 | 1.2 | 1.5 | 1.5 | 0.7 | 0.5 | 0.8 | 0.9 | 0.59 |
| YBR123C | 0.8 | 0.7 | 1.6 | 1.8 | 1.1 | 1.3 | 0.9 | 0.9 | 3.2 | 2.5 | 1.1 | 1.0 | 1.4 | 1.1 | 1.0 | 1.2 | 0.8 | 1.0 | 0.62 |
| YDR099W | 0.9 | 1.0 | 2.0 | 1.3 | 1.1 | 2.2 | 1.3 | 1.1 | 2.4 | 1.3 | 1.1 | 0.9 | 1.5 | 0.9 | 1.9 | 1.4 | 1.2 | 1.7 | 3.51 |
| YDR177W | 1.2 | 1.7 | 1.0 | 1.0 | 1.0 | 1.4 | 1.3 | 1.6 | 3.5 | 1.9 | 1.3 | 1.2 | 1.7 | 2.4 | 0.8 | 1.3 | 1.3 | 1.5 | 1.77 |
| YDR392W | 0.9 | 2.9 | 1.1 | 1.3 | 1.2 | 0.6 | 1.1 | 1.1 | 4.3 | 1.5 | 1.2 | 0.8 | 1.4 | 1.3 | 1.1 | 1.4 | 1.0 | 1.2 | 0.63 |
| YDR394W | 0.9 | 1.2 | 1.3 | 0.8 | 1.2 | 1.1 | 1.2 | 1.3 | 3.8 | 2.0 | 1.8 | 0.6 | 1.0 | 1.2 | 1.1 | 1.0 | 1.1 | 0.8 | 0.97 |
| YER184C | 2.3 | 1.6 | 1.1 | 2.3 | 1.0 | 0.6 | 0.9 | 1.1 | 4.6 | 3.2 | 1.3 | 0.8 | 1.6 | 1.4 | 1.4 | 1.2 | 1.6 | 2.3 | 0.29 |
| YFL059W | 1.2 | 1.4 | 1.2 | 2.2 | 1.1 | 1.0 | 1.3 | 1.5 | 4.1 | 4.2 | 1.5 | 1.2 | 2.3 | 2.4 | 0.9 | 1.6 | 1.3 | 1.1 | 0.57 |
| YGL185C | 1.3 | 1.9 | 1.9 | 1.9 | 0.8 | 0.8 | 1.9 | 1.5 | 4.1 | 2.3 | 1.5 | 1.5 | 1.7 | 1.5 | 0.9 | 15.8 | 1.3 | 1.6 | 0.23 |
| YHL019C | 1.1 | 0.8 | 1.2 | 0.6 | 1.0 | 1.2 | 1.1 | 1.0 | 2.1 | 1.4 | 1.3 | 0.8 | 0.8 | 0.8 | 1.2 | 1.3 | 1.0 | 1.0 | 0.55 |
| YHR012W | 1.1 | 1.0 | 1.2 | 1.0 | | 1.6 | 1.2 | 1.2 | 4.9 | 2.2 | 1.5 | 0.9 | 1.7 | 1.7 | 1.0 | 1.0 | 1.2 | 1.4 | 1.11 |
| YHR028C | 0.8 | 1.2 | 1.4 | 0.8 | 1.0 | 0.9 | 1.2 | 0.8 | 3.7 | 2.2 | 1.4 | 0.7 | 0.6 | 1.1 | 1.1 | 0.7 | 1.0 | 1.0 | 0.92 |
| YHR109W | 0.8 | 1.0 | 0.0 | 0.7 | 1.3 | 1.4 | 1.4 | 1.2 | 2.4 | 1.8 | 0.7 | 1.0 | 1.4 | 1.5 | 0.9 | 1.3 | 0.9 | 1.1 | 0.22 |
| YHR156C | 1.3 | 1.3 | 0.9 | 2.1 | 1.9 | 1.0 | 1.2 | 1.2 | 3.6 | 1.9 | 0.6 | 0.7 | 1.0 | 1.9 | 1.2 | 1.4 | 0.9 | 1.3 | 0.47 |
| YIL159W | 1.1 | 1.6 | 1.3 | 0.7 | 1.2 | 2.3 | 0.6 | 1.1 | 5.9 | 1.6 | 0.8 | 0.9 | 1.5 | 2.0 | 1.0 | 0.7 | 0.9 | 0.8 | 0.29 |
| YJL154C | 0.6 | 2.7 | 0.7 | 0.6 | 1.2 | 1.3 | 1.5 | 1.0 | 2.3 | 2.0 | 0.5 | 0.9 | 1.6 | 0.8 | 1.2 | 1.2 | 1.2 | 1.1 | 0.62 |
| YJR110W | 1.0 | 0.6 | 1.0 | 1.4 | 1.3 | 0.9 | 0.9 | 1.1 | 4.9 | 2.3 | 1.2 | 0.7 | 0.9 | 1.1 | 1.2 | 1.1 | 0.8 | 1.0 | 0.58 |
| YKL025C | 0.7 | 1.1 | 1.1 | 0.9 | 1.1 | 0.9 | 0.9 | 0.9 | 2.8 | 1.7 | 1.1 | 0.8 | 1.3 | 0.9 | 1.2 | 1.1 | 0.9 | 1.0 | 0.54 |

| | | | | | | | | | | | | | | | | | | |
|---|---|---|---|---|---|---|---|---|---|---|---|---|---|---|---|---|---|---|
| YKL171W | 0.9 | 1.3 | 1.2 | 2.1 | 0.9 | 0.4 | 0.9 | 0.8 | 8.2 | 1.8 | 2.8 | 1.1 | 1.5 | 1.1 | 1.2 | 2.1 | 0.9 | 1.2 | 0.45 |
| YKL196C | 1.2 | 1.1 | 0.5 | 0.9 | 1.3 | 0.9 | 1.5 | 1.3 | 4.3 | 2.0 | 1.6 | 1.0 | 1.8 | 2.5 | 1.9 | 1.9 | 1.3 | 2.0 | 2.41 |
| YKR068C | 1.2 | 1.5 | 1.0 | 1.8 | 1.1 | 1.4 | 1.4 | 1.3 | 2.4 | 1.9 | 1.0 | 1.0 | 1.1 | 1.6 | 0.8 | 1.4 | 1.4 | 1.7 | 1.72 |
| YLR144C | 0.8 | 1.5 | 4.0 | 1.3 | 0.7 | 0.9 | 0.7 | 1.1 | 2.8 | 1.5 | 1.0 | 0.7 | 2.3 | 1.2 | 1.9 | 1.7 | 1.0 | 1.0 | 0.44 |
| YML112W | 1.0 | 1.0 | 1.1 | 2.0 | 1.0 | 1.4 | 0.9 | 1.3 | 3.2 | 2.4 | 1.0 | 1.0 | 1.3 | 1.8 | 0.8 | 0.9 | 0.8 | 0.9 | 0.49 |
| YOL038W | 0.9 | 1.0 | 0.4 | 1.6 | 1.8 | 1.2 | 1.4 | 1.4 | 3.5 | 2.7 | 2.0 | 0.9 | 1.8 | 2.1 | 1.7 | 1.6 | 1.1 | 1.4 | 1.29 |
| YOR257W | 1.1 | 2.5 | 0.8 | 1.1 | 1.1 | 0.8 | 1.3 | 1.5 | 3.4 | 1.4 | 1.0 | 0.7 | 0.8 | 1.5 | 0.7 | 1.6 | 1.1 | 1.6 | 0.68 |
| YOR265W | 1.4 | 1.0 | 0.8 | 1.0 | 1.2 | 1.4 | 1.3 | 1.4 | 3.5 | 2.2 | 1.3 | 0.8 | 1.8 | 2.5 | 1.2 | 1.1 | 1.0 | 1.2 | 0.93 |
| YPL124W | 1.3 | 1.3 | 0.7 | 0.7 | 1.6 | 0.8 | 0.9 | 1.8 | 2.2 | 1.0 | 0.9 | 0.7 | 0.9 | 1.6 | 0.6 | 0.8 | 1.3 | 1.2 | 0.41 |
| YPR125W | 0.6 | 1.3 | 0.9 | | 1.2 | 0.7 | 1.3 | 1.2 | 4.1 | 1.8 | 0.9 | 1.1 | 1.9 | 1.6 | 2.0 | 0.6 | 0.8 | 0.7 | 0.73 |
| YPR168W | 0.9 | 1.0 | 0.2 | 0.5 | 1.3 | 0.4 | 1.1 | 1.1 | 4.7 | 2.7 | 1.2 | 0.5 | 1.0 | 1.1 | 1.2 | 1.3 | 0.9 | 0.8 | 0.29 |
| YPR180W | 0.9 | 1.2 | 1.6 | 1.0 | 1.0 | 0.8 | 2.4 | 1.3 | 2.3 | 1.1 | 0.5 | 0.7 | 1.3 | 1.2 | 1.3 | 1.0 | 1.0 | 1.4 | 0.61 |
| YPR193C | 1.0 | 2.2 | 0.6 | 1.1 | 1.4 | 1.3 | 1.4 | 1.1 | 3.5 | 4.1 | 0.1 | 0.9 | 1.6 | 1.6 | 0.9 | 1.6 | 1.1 | 0.9 | 0.17 |
| YBR045C | 0.9 | 1.0 | 2.2 | 0.7 | 1.0 | 0.8 | 0.8 | 1.0 | 3.6 | 1.1 | 0.4 | 1.3 | 1.3 | 1.0 | 2.0 | 0.8 | 1.1 | 0.9 | 1.29 |
| YBR128C | 1.0 | 1.1 | 2.1 | 1.0 | 1.1 | 1.5 | 2.1 | 1.2 | 2.8 | 1.2 | 1.3 | 0.7 | 1.3 | 1.4 | 1.4 | 2.0 | 1.2 | 1.8 | 0.44 |
| YCL055W | 0.7 | 2.2 | 0.7 | 1.3 | 1.2 | 1.4 | 1.0 | 1.3 | 2.1 | 1.5 | 1.4 | 0.9 | 0.9 | 1.8 | 1.5 | 0.7 | 0.8 | 1.1 | 0.66 |
| YCR019W | 1.0 | 1.6 | 1.0 | 0.8 | 1.4 | 1.5 | 1.1 | 1.5 | 2.8 | 1.6 | 2.0 | 1.0 | 1.6 | 1.2 | 0.7 | 1.4 | 1.0 | 1.0 | 0.45 |
| YDL065C | 1.2 | 0.9 | 1.0 | 0.7 | 1.4 | 1.1 | 1.3 | 1.4 | 2.4 | 1.5 | 1.4 | 0.6 | 0.7 | 1.4 | 1.3 | 1.5 | 1.1 | 1.2 | 1.08 |
| YDL143W | 0.8 | 0.8 | 1.2 | 1.1 | 1.0 | 1.0 | 0.9 | 1.2 | 2.1 | 1.8 | 1.3 | 1.1 | 1.9 | 0.9 | 1.4 | 0.9 | 0.8 | 0.8 | 1.86 |
| YDL197C | 1.0 | 1.1 | 0.9 | 2.1 | 1.5 | 1.2 | 1.0 | 1.0 | 2.6 | 1.5 | 1.1 | 0.8 | 1.4 | 1.1 | 1.3 | 0.9 | 1.3 | 0.8 | 0.31 |
| YDL230W | 1.0 | 0.8 | 1.2 | 1.3 | 1.4 | 0.9 | 1.1 | 0.9 | 2.6 | 1.2 | 0.9 | 0.6 | 1.3 | 1.0 | 1.4 | 1.7 | 1.0 | 1.2 | 0.42 |
| YDR212W | 0.8 | 1.2 | 1.3 | 0.8 | 1.4 | 1.4 | 1.0 | 1.3 | 3.5 | 1.7 | 1.8 | 0.8 | 2.0 | 1.0 | 1.2 | 0.7 | 0.6 | 1.0 | 2.13 |
| YDR257C | 0.8 | 5.3 | 0.6 | 0.8 | 1.6 | 1.1 | 1.0 | 1.0 | 3.0 | 1.6 | 1.3 | 0.7 | 1.0 | 1.0 | 1.3 | 1.0 | 1.1 | 0.8 | 0.62 |
| YDR329C | 0.9 | 0.8 | 1.0 | 1.7 | 1.9 | 1.2 | 1.6 | 1.2 | 2.5 | 1.0 | 1.0 | 0.8 | 1.1 | 1.1 | 1.0 | 1.6 | 1.2 | 1.6 | 0.95 |
| YDR488C | 1.0 | 0.3 | 0.9 | 0.9 | 0.9 | 0.9 | 0.7 | 0.9 | 2.1 | 0.4 | 0.4 | 0.6 | 1.3 | 0.8 | 3.5 | 0.7 | 0.9 | 0.9 | 0.32 |
| YDR505C | 0.8 | 1.0 | 1.0 | 1.4 | 1.2 | 1.1 | 1.2 | 0.8 | 2.5 | 1.0 | 1.0 | 0.7 | 1.6 | 0.9 | 1.1 | 1.6 | 1.0 | 1.5 | 0.84 |
| YDR506C | 0.9 | 0.7 | 1.3 | 0.7 | 0.9 | 0.9 | 0.6 | 1.0 | 1.9 | 1.4 | 1.1 | 0.9 | 0.3 | 0.5 | 0.9 | 0.4 | 0.6 | 0.5 | 1.90 |
| YDR515W | 0.9 | 1.1 | 0.8 | 0.8 | 1.5 | 0.8 | 0.6 | 1.4 | 5.8 | 2.7 | 1.0 | 0.8 | 0.7 | 0.8 | 1.3 | 0.5 | 0.7 | 0.5 | 1.02 |
| YEL005C | 1.1 | 1.3 | 0.8 | 1.1 | 1.6 | 1.9 | 2.0 | 1.2 | 2.3 | 1.6 | 1.6 | 0.8 | 1.2 | 1.7 | 0.8 | 3.4 | 1.1 | 1.4 | 0.45 |
| YER048C | 0.8 | 1.2 | 0.4 | 1.4 | 1.8 | 1.9 | 1.9 | 1.6 | 2.3 | 1.1 | 1.6 | 0.8 | 1.1 | 1.0 | 0.6 | 1.8 | 1.3 | 1.6 | 1.08 |
| YER078C | 0.9 | 0.8 | 1.8 | 0.8 | 0.9 | 1.4 | 1.3 | 1.1 | 2.3 | 1.5 | 1.3 | 1.0 | 1.5 | 1.0 | 0.9 | 1.0 | 0.9 | 0.9 | 0.49 |
| YER089C | 0.5 | 0.5 | 1.4 | 1.1 | 0.8 | 0.6 | 0.9 | 0.5 | 2.0 | 2.3 | 1.2 | 0.7 | 1.3 | 0.6 | 1.0 | 0.8 | 0.7 | 0.6 | 0.75 |

| | | | | | | | | | | | | | | | | | | | |
|---|---|---|---|---|---|---|---|---|---|---|---|---|---|---|---|---|---|---|---|
| YER100W | 1.1 | 1.1 | 0.7 | 0.7 | 0.9 | 0.5 | 0.6 | 1.2 | 2.3 | 1.6 | 1.1 | 0.8 | 0.7 | 0.8 | 1.0 | 1.1 | 1.1 | 0.7 | 0.95 |
| YFR051C | 0.5 | 1.1 | 2.2 | 0.5 | 0.5 | 0.2 | 1.0 | 0.8 | 2.5 | 1.4 | 1.3 | 1.2 | 1.5 | 0.8 | 3.0 | 0.8 | 1.1 | 0.9 | 0.53 |
| YGL093W | 0.8 | 0.9 | 1.2 | 0.6 | 0.9 | 1.0 | 0.8 | 0.8 | 4.5 | 1.6 | 1.1 | 0.6 | 0.7 | 0.8 | 1.4 | 1.0 | 0.9 | 1.2 | 0.56 |
| YGL105W | 0.9 | 0.8 | 1.1 | 0.8 | 1.3 | 1.5 | 0.7 | 1.2 | 1.8 | 1.4 | 1.4 | 1.1 | 1.0 | 1.1 | 0.9 | 1.0 | 0.8 | 0.9 | 3.01 |
| YGL166W | 1.0 | 1.4 | 0.6 | 1.5 | 1.3 | 1.0 | 1.5 | 1.2 | 1.8 | 2.4 | 1.0 | 1.1 | 1.9 | 1.1 | 0.9 | 1.2 | 2.1 | 1.9 | 0.73 |
| YGL215W | 0.7 | 1.2 | 1.2 | 0.9 | 1.0 | 0.9 | 1.0 | 0.7 | 2.2 | 1.8 | 0.8 | 0.7 | 1.0 | 0.8 | 1.0 | 1.2 | 0.7 | 0.9 | 1.31 |
| YGL216W | 0.7 | 1.3 | 0.8 | 1.0 | 1.2 | 1.5 | 1.0 | 1.0 | 2.6 | 0.5 | 0.5 | 1.0 | 1.4 | 1.0 | 1.1 | 0.8 | 1.1 | 0.9 | 0.37 |
| YGL221C | 1.1 | 1.2 | 1.0 | 1.1 | 0.9 | 2.2 | 1.3 | 1.6 | 3.4 | 1.9 | 1.4 | 1.1 | 1.7 | 1.7 | 0.7 | 1.3 | 1.1 | 1.4 | 1.64 |
| YGR186W | 0.9 | 0.7 | 0.4 | 1.1 | 1.5 | 0.7 | 1.3 | 1.3 | 2.6 | 1.9 | 1.0 | 0.6 | 1.1 | 1.1 | 1.3 | 1.4 | 1.3 | 1.2 | 0.79 |
| YGR270W | 0.9 | 1.0 | 0.8 | 0.8 | 0.7 | 0.9 | 0.9 | 0.9 | 2.3 | 1.3 | 0.7 | 0.8 | 1.3 | 0.9 | 1.6 | 1.0 | 0.9 | 0.9 | 0.51 |
| YGR274C | 0.7 | 0.5 | 0.3 | 0.9 | 1.8 | 0.7 | 1.3 | 1.2 | 2.2 | 1.8 | 0.9 | 0.7 | 1.1 | 0.8 | 1.2 | 0.7 | 1.1 | 1.0 | 0.62 |
| YHR082C | 0.7 | 1.1 | 1.6 | 0.9 | 1.6 | 0.8 | 1.1 | 0.8 | 2.6 | 1.7 | 0.9 | 0.7 | 1.7 | 0.9 | 1.4 | 0.7 | 1.1 | 0.9 | 0.60 |
| YHR160C | 1.0 | 0.5 | 1.6 | 1.6 | 1.0 | 1.0 | 1.1 | 1.2 | 2.3 | 1.6 | -0.2 | 0.8 | 1.8 | 1.2 | 2.1 | 4.4 | 1.0 | 1.5 | 0.32 |
| YHR171W | 1.2 | 0.8 | 1.1 | 1.2 | 1.3 | 2.0 | 1.9 | 0.9 | 2.1 | 1.6 | 1.2 | 0.8 | 1.3 | 1.0 | 0.7 | 1.6 | 1.2 | 1.5 | 0.41 |
| YHR205W | 0.7 | 1.3 | 0.6 | 0.8 | 1.3 | 1.0 | 0.7 | 0.5 | 2.0 | 0.6 | 0.9 | 0.9 | 1.7 | 0.9 | 1.5 | 0.8 | 0.9 | 0.8 | 0.34 |
| YIL062C | 1.1 | 1.0 | 1.3 | 1.2 | 1.2 | 0.9 | 0.4 | 1.1 | 2.8 | 2.3 | 1.7 | 0.8 | 1.1 | 2.0 | 0.9 | 0.4 | 1.1 | 1.1 | 1.72 |
| YIL075C | 0.5 | 0.7 | 1.5 | 0.6 | 1.1 | 0.8 | 0.8 | 0.7 | 1.9 | 2.3 | 0.8 | 0.8 | 0.7 | 0.8 | 1.6 | 0.8 | 0.8 | 0.8 | 1.54 |
| YIR009W | 0.8 | 0.8 | 0.9 | 2.8 | 1.5 | 1.4 | 1.3 | 1.3 | 2.6 | 1.5 | 1.1 | 0.7 | 0.8 | 1.6 | 1.1 | 1.0 | 1.7 | 1.0 | 0.48 |
| YIR018W | 1.2 | 1.0 | 0.9 | 1.1 | 1.4 | 2.0 | 1.5 | 1.5 | 4.0 | 2.1 | 1.2 | 1.1 | 1.5 | 1.2 | 1.5 | 1.0 | 1.6 | 1.1 | 0.44 |
| YJR091C | 0.8 | 1.2 | 2.6 | 1.4 | 1.4 | 0.8 | 1.1 | 0.8 | 2.4 | 2.3 | 1.8 | 0.8 | 1.4 | 0.8 | 1.4 | 0.7 | 1.2 | 0.9 | 0.57 |
| YKL079W | 0.9 | 0.8 | 1.5 | 1.0 | 1.4 | 0.8 | 1.7 | 1.2 | 2.3 | 2.0 | 1.3 | 0.9 | 1.2 | 0.9 | 1.1 | 1.6 | 1.1 | 1.2 | 0.64 |
| YKR102W | 0.8 | 1.6 | 1.4 | 0.8 | 0.9 | | | 0.7 | 2.4 | 1.4 | 3.3 | 0.8 | 2.7 | 1.8 | 1.1 | 1.5 | 1.5 | 0.9 | 0.19 |
| YLL054C | 1.2 | 0.6 | 0.5 | 1.2 | 1.4 | 1.9 | 1.1 | 1.1 | 2.4 | 1.4 | 0.5 | 0.7 | 1.7 | 1.2 | 0.9 | 0.9 | 1.0 | 1.0 | 0.33 |
| YLR200W | 1.4 | 0.9 | 0.5 | 1.2 | 1.7 | 1.2 | 1.9 | 1.4 | 2.2 | 2.6 | 1.1 | 0.9 | 1.3 | 2.3 | 0.9 | 1.2 | 1.1 | 1.5 | 1.12 |
| YLR248W | 0.9 | 0.7 | 1.1 | 1.4 | 1.9 | 1.2 | 1.2 | 1.1 | 2.3 | 1.1 | 1.0 | 0.7 | 1.2 | 0.9 | 1.2 | 1.7 | 1.1 | 1.7 | 1.58 |
| YLR266C | 0.9 | 1.3 | 0.7 | 1.5 | 0.7 | 1.0 | 1.6 | 1.1 | 2.2 | 2.6 | 1.1 | 1.0 | 1.3 | 1.0 | 0.8 | 1.7 | 0.8 | 1.4 | 0.74 |
| YML088W | 0.9 | 1.6 | 0.3 | 0.5 | 1.5 | 0.7 | 1.1 | 1.1 | 4.0 | 2.2 | 0.5 | 1.0 | 1.3 | 1.1 | 1.4 | 0.8 | 0.8 | 0.8 | 0.58 |
| YMR091C | 1.0 | 0.7 | 0.8 | 1.0 | 0.7 | 0.8 | 0.8 | 1.3 | 2.1 | 1.3 | 0.9 | 0.8 | 0.9 | 1.5 | 0.9 | 1.3 | 0.9 | 1.2 | 1.03 |
| YMR110C | 1.0 | 1.4 | 1.8 | 1.9 | 1.3 | 2.1 | 1.9 | 0.9 | 2.3 | 1.6 | 1.6 | 0.9 | 1.8 | 0.8 | 0.9 | 2.7 | 1.5 | 2.5 | 1.63 |
| YMR255W | 1.2 | 1.4 | 0.7 | 1.8 | 1.3 | 0.9 | 1.5 | 1.4 | 2.8 | 2.4 | 1.2 | 0.9 | 1.1 | 1.6 | 1.0 | 1.3 | 1.5 | 2.0 | 0.97 |
| YNL039W | 0.7 | 0.8 | 0.3 | 0.4 | 2.1 | 0.8 | 1.1 | 1.2 | 2.1 | 1.8 | 0.4 | 0.9 | 0.8 | 0.8 | 1.3 | 1.3 | 0.9 | 1.1 | 0.61 |
| YNL077W | 1.5 | 1.1 | 2.3 | 1.2 | 1.5 | 0.9 | 1.2 | 1.0 | 4.4 | 5.1 | 1.0 | 0.7 | 1.5 | 0.5 | 0.3 | 0.6 | 1.3 | 1.0 | 0.78 |

| | | | | | | | | | | | | | | | | | | | |
|---|---|---|---|---|---|---|---|---|---|---|---|---|---|---|---|---|---|---|---|
| YNL083W | 0.8 | 1.4 | 1.3 | 1.2 | 0.8 | 0.7 | 0.9 | 1.1 | 3.1 | 1.6 | 0.9 | 0.9 | 1.2 | 0.9 | 0.8 | 1.8 | 0.8 | 1.1 | 0.37 |
| YNL147W | 1.3 | 2.4 | 0.6 | 0.7 | 2.0 | 1.5 | 1.4 | 1.8 | 2.1 | 2.9 | 1.2 | 0.8 | 1.0 | 2.0 | 1.2 | 1.7 | 1.0 | 1.6 | 1.32 |
| YNR006W | 1.1 | 1.8 | 0.5 | 0.9 | 1.5 | 0.9 | 1.5 | 0.9 | 2.2 | 2.3 | 0.8 | 0.9 | 1.6 | 0.9 | 1.2 | 1.1 | 1.8 | 1.2 | 0.40 |
| YNR034W | 0.9 | 1.4 | 5.3 | 1.6 | 1.0 | 0.9 | 1.3 | 1.0 | 3.0 | 2.1 | 1.3 | 0.9 | 1.4 | 0.9 | 1.1 | 2.3 | 0.7 | 1.1 | 0.64 |
| YNR047W | 0.7 | 1.0 | 1.7 | 0.5 | 1.1 | 1.0 | 1.0 | 0.8 | 2.0 | 1.2 | 0.6 | 0.9 | 1.5 | 0.9 | 1.2 | 1.5 | 0.9 | 1.0 | 0.44 |
| YOR023C | 0.7 | 1.2 | 1.3 | 0.8 | 1.2 | 1.8 | 1.1 | 0.6 | 3.5 | 1.3 | 0.9 | 1.1 | 1.9 | 0.9 | 1.6 | 0.8 | 1.1 | 0.8 | 0.37 |
| YOR058C | 2.0 | 1.4 | 2.0 | 0.6 | 0.9 | 0.9 | 0.8 | 1.7 | 6.4 | 4.5 | 1.4 | 0.7 | 1.7 | 1.7 | 1.1 | 1.6 | 0.9 | 1.0 | 0.31 |
| YOR069W | 0.8 | 1.6 | 0.7 | 1.3 | 1.0 | 0.5 | 1.2 | 1.1 | 1.9 | 1.4 | 2.8 | 0.9 | 1.1 | 1.3 | 1.4 | 1.5 | 0.8 | 1.1 | 0.69 |
| YOR229W | 0.9 | 0.9 | 1.0 | 0.8 | 1.5 | 1.3 | 1.1 | 0.8 | 2.5 | 0.8 | 0.7 | 0.8 | 1.3 | 1.0 | 1.2 | 0.9 | 0.9 | 1.0 | 0.58 |
| YOR256C | 1.0 | 0.8 | 0.9 | 1.5 | 1.7 | 0.9 | 1.4 | 1.1 | 2.7 | 1.8 | 1.7 | 0.7 | 1.6 | 1.1 | 1.1 | 1.5 | 1.2 | 1.5 | 0.53 |
| YPL020C | 1.1 | 0.7 | 0.7 | 1.3 | 0.8 | 0.8 | 1.1 | 1.1 | 2.3 | 1.6 | 1.1 | 0.9 | 1.1 | 1.1 | 1.6 | 1.2 | 1.3 | 1.4 | 0.91 |
| YPL105C | 0.6 | 0.8 | 0.6 | 0.7 | 1.1 | 1.2 | 0.7 | 1.0 | 3.4 | 1.4 | 0.6 | 0.7 | 1.0 | 0.6 | 1.1 | 0.5 | 0.8 | 0.6 | 0.80 |
| YPR066W | 1.2 | 2.2 | 1.1 | 2.2 | 1.3 | 0.7 | 1.3 | 0.8 | 2.3 | 1.9 | 0.9 | 0.8 | 1.4 | 1.2 | 0.7 | 1.6 | 1.1 | 1.2 | 0.41 |
| YPR081C | 0.9 | 1.0 | 1.0 | 1.1 | 0.8 | 1.1 | 1.1 | 1.0 | 4.2 | 2.6 | 0.9 | 1.0 | 1.4 | 1.1 | 1.3 | 1.8 | 0.9 | 1.3 | 0.34 |
| YPR140W | 0.9 | 1.2 | 0.8 | 1.8 | 0.9 | 3.0 | 1.9 | 1.1 | 2.1 | 1.3 | 0.8 | 0.8 | 1.3 | 1.4 | 1.7 | 2.4 | 0.8 | 1.3 | 0.47 |
| YPR155C | 0.9 | 2.5 | 1.5 | 1.4 | 0.9 | 0.9 | 1.4 | 1.0 | 2.0 | 1.3 | 0.9 | 0.9 | 1.3 | 1.2 | 0.5 | 3.4 | 0.8 | 1.6 | 0.40 |
| YPR185W | 0.7 | 0.9 | 0.3 | 0.4 | 1.1 | 0.9 | 1.2 | 1.0 | 2.5 | 1.7 | 0.9 | 0.8 | 1.7 | 1.0 | 1.1 | 1.3 | 1.3 | 1.1 | 0.68 |
| YBR076W | 2.3 | 1.1 | 1.4 | 5.0 | 0.9 | 0.8 | 1.0 | 0.7 | 0.4 | 1.8 | 0.8 | 0.9 | 1.9 | 1.2 | 1.1 | 0.5 | 1.4 | 1.2 | 0.36 |
| YDR373W | 1.7 | 0.8 | 0.5 | 1.3 | 1.8 | 0.9 | 1.4 | 1.9 | 2.5 | 2.8 | 1.7 | 0.5 | 1.0 | 2.1 | 1.6 | 1.4 | 1.2 | 1.6 | 1.08 |
| YFR014C | 2.1 | 2.4 | 0.8 | 2.6 | 0.7 | 0.8 | 1.4 | 1.3 | 1.0 | 1.6 | 1.8 | 0.9 | 1.3 | 1.0 | 0.6 | 2.3 | 1.2 | 2.9 | 0.94 |
| YHR136C | 2.4 | 13.6 | 1.4 | 2.1 | 2.4 | 1.4 | 0.5 | 1.3 | 0.1 | 2.0 | 2.0 | 1.2 | 0.3 | 1.7 | 0.5 | 1.3 | 1.6 | 2.5 | 1.33 |
| YIL129C | 0.8 | 5.8 | 1.0 | 1.2 | | 0.3 | | | 2.1 | 1.8 | 1.5 | | 1.9 | 0.9 | 1.0 | 1.0 | 1.3 | 0.8 | 0.25 |
| YMR077C | 1.1 | 3.9 | 1.1 | 1.8 | 1.0 | 0.5 | 1.4 | 1.7 | 1.9 | 2.5 | 1.5 | 0.9 | 0.8 | 1.6 | 1.7 | 2.3 | 0.9 | 1.3 | 0.59 |
| YBR264C | 1.2 | 3.7 | 1.5 | 1.5 | 0.7 | 0.9 | 1.3 | 1.2 | 0.9 | 1.1 | 1.0 | 1.0 | 0.9 | 1.6 | 1.1 | 1.8 | 0.9 | 1.3 | 0.61 |
| YPL177C | 1.2 | 3.7 | 0.9 | 1.3 | 0.7 | 0.8 | 0.8 | 1.3 | 0.5 | 1.0 | 1.4 | 0.8 | 0.8 | 1.3 | 0.6 | 1.9 | 1.2 | 1.1 | 0.75 |
| YGL056C | 0.7 | 3.5 | 1.3 | 1.0 | 0.6 | 0.4 | 1.0 | 0.9 | 0.4 | 0.6 | 0.0 | 1.1 | 0.8 | 1.1 | 1.2 | 1.5 | 0.9 | 1.1 | 0.47 |
| YBL101W-A | 1.0 | 5.7 | 0.7 | 0.6 | 1.0 | 1.4 | 1.3 | 1.5 | 1.2 | 2.1 | 1.2 | 1.1 | 2.0 | 1.5 | 2.1 | 1.1 | 1.3 | 1.2 | 1.24 |
| YER072W | 1.6 | 2.3 | 1.6 | 1.3 | 1.2 | 1.5 | 0.7 | 1.2 | 0.5 | 1.1 | 1.7 | 1.1 | 0.6 | 1.5 | 0.9 | 1.1 | 1.7 | 1.7 | 1.69 |
| YMR112C | 1.2 | 4.2 | 0.9 | 1.2 | 1.0 | 0.7 | 1.5 | 1.6 | 1.4 | 1.3 | 0.9 | 1.0 | 0.9 | 1.8 | 0.9 | 1.4 | 1.1 | 1.6 | 0.57 |
| YJR058C | 1.3 | 2.0 | 1.3 | 1.5 | 1.1 | 1.6 | 1.3 | 1.2 | 1.9 | 2.6 | 1.2 | 0.7 | 0.8 | 1.8 | 0.8 | 1.2 | 1.3 | 1.2 | 1.00 |
| YML055W | 1.7 | 2.3 | 0.6 | 2.0 | 0.9 | 1.2 | 1.7 | 1.7 | 1.0 | 1.4 | 1.4 | 1.0 | 1.7 | 1.9 | 0.6 | 1.4 | 1.2 | 1.5 | 1.08 |
| YDR080W | 0.9 | 1.8 | 0.5 | 0.6 | 1.2 | 1.5 | 1.6 | 1.2 | 1.7 | 2.6 | 1.2 | 1.0 | 2.0 | 1.1 | 1.2 | 1.7 | 1.2 | 1.3 | 0.68 |

| | | | | | | | | | | | | | | | | | | |
|---|---|---|---|---|---|---|---|---|---|---|---|---|---|---|---|---|---|---|
| YNR030W | 0.6 | 2.1 | 2.2 | 0.7 | 0.8 | 1.2 | 1.0 | 0.6 | 1.0 | 1.4 | 1.2 | 0.8 | 1.5 | 0.6 | 1.1 | 0.4 | 0.9 | 0.8 | 1.19 |
| YNL196C | 1.1 | 1.8 | 1.4 | 1.3 | 0.9 | 1.2 | 1.0 | 0.7 | 0.5 | 2.2 | 0.7 | 1.0 | 2.2 | 1.2 | 0.8 | 2.1 | 1.1 | 1.2 | 0.50 |
| YOR148C | 1.4 | 1.4 | 0.5 | 1.8 | 0.9 | 0.9 | 1.2 | 1.5 | 0.8 | 0.8 | 1.3 | 0.9 | 1.4 | 1.4 | 1.0 | 1.3 | 1.0 | 1.3 | 1.08 |
| YER029C | 1.0 | 1.6 | 0.4 | 1.9 | 1.7 | 1.2 | 1.1 | 1.5 | 1.4 | 1.1 | 0.8 | 0.9 | 0.9 | 1.4 | 1.1 | 1.7 | 0.9 | 1.3 | 1.20 |
| YLR360W | 0.9 | 2.0 | 0.5 | 0.7 | 1.9 | 1.4 | 1.7 | 1.3 | 0.9 | 1.3 | 0.9 | 0.9 | 0.7 | 1.1 | 0.8 | 2.1 | 1.2 | 1.4 | 0.53 |
| YGR239C | 1.3 | 1.3 | 0.7 | 1.3 | 1.2 | 2.3 | 1.2 | 1.5 | 0.5 | 3.8 | 1.4 | 1.0 | 1.0 | 1.4 | 1.3 | 1.1 | 1.1 | 1.3 | 0.49 |
| YDL229W | 0.9 | 1.9 | 0.4 | 0.6 | 1.2 | 1.0 | 1.3 | 1.2 | 1.4 | 0.9 | 1.6 | 0.9 | 1.0 | 1.3 | 1.2 | 1.0 | 1.2 | 1.1 | 0.76 |
| YJR027W | 0.8 | 1.9 | 0.8 | 0.7 | | 1.5 | 2.0 | 1.0 | 1.2 | 1.3 | 1.1 | 0.8 | 1.3 | 1.1 | 2.3 | 0.7 | 0.7 | 1.1 | 4.88 |
| YKL198C | 1.2 | 1.4 | 0.8 | 0.2 | 0.9 | 1.0 | 1.2 | 0.6 | 0.0 | 0.8 | 1.4 | 0.7 | 1.9 | 6.6 | 2.6 | 0.7 | 1.0 | 0.8 | 0.17 |
| YBR031W | 1.1 | 0.8 | 4.1 | 1.4 | 1.4 | 0.7 | 0.5 | 1.0 | 0.1 | 1.0 | 0.9 | 1.0 | 0.3 | 0.4 | 0.9 | 0.7 | 1.0 | 0.8 | 7.58 |
| YBR118W | 1.3 | 0.8 | 2.9 | 1.2 | 1.0 | 1.3 | 1.0 | 1.3 | 0.5 | 0.9 | 1.0 | 0.8 | 0.8 | 0.7 | 1.6 | 0.6 | 1.2 | 0.9 | 8.91 |
| YCR106W | 0.9 | 1.1 | 2.8 | 1.1 | 0.8 | 1.4 | 0.9 | 0.8 | 2.0 | 1.2 | 1.2 | 0.8 | 1.2 | 0.8 | 1.3 | 1.2 | 1.1 | 1.2 | 0.76 |
| YDR012W | 0.9 | 0.8 | 3.9 | 1.2 | 1.1 | 0.9 | 0.5 | 1.1 | 0.3 | 0.7 | 0.9 | 1.2 | 0.2 | 0.4 | 0.8 | 0.7 | 1.0 | 0.7 | 7.07 |
| YDR134C | 1.0 | 0.7 | 3.9 | 1.9 | 0.8 | 1.1 | 0.4 | 0.6 | 0.1 | 0.5 | 1.9 | 0.6 | 0.4 | 0.5 | 0.8 | 0.9 | 0.7 | 1.1 | 5.84 |
| YDR276C | 1.5 | 1.6 | 6.1 | 1.4 | 0.7 | 1.1 | 1.0 | 1.1 | 1.1 | 2.7 | 1.4 | 0.9 | 1.1 | 1.3 | 2.2 | 3.6 | 1.3 | 2.4 | 2.48 |
| YGR279C | 0.9 | 0.7 | 3.6 | 1.0 | 1.2 | 1.6 | 0.8 | 0.6 | 0.3 | 0.7 | 0.9 | 0.7 | 0.3 | 0.4 | 1.3 | 0.8 | 1.0 | 0.8 | 3.77 |
| YJL059W | 1.0 | 1.7 | 3.1 | 0.8 | 0.9 | 1.6 | 1.1 | 0.8 | 1.7 | 1.6 | 1.8 | 1.0 | 1.6 | 1.3 | 0.9 | 0.8 | 1.1 | 1.0 | 0.39 |
| YKL056C | 1.3 | 0.7 | 2.6 | 1.4 | 1.7 | 0.9 | 0.5 | 1.0 | 0.1 | 0.7 | 1.1 | 0.8 | 0.1 | 0.7 | 0.9 | 0.6 | 0.8 | 1.2 | 6.68 |
| YKL097W-A | 1.5 | 1.3 | 4.2 | 1.3 | 1.2 | 0.9 | 0.2 | 0.7 | 0.0 | 0.4 | 1.6 | 0.7 | 0.2 | 0.6 | 2.0 | 0.8 | 1.1 | 1.4 | 4.01 |
| YNL209W | 0.9 | 0.9 | 2.7 | 0.9 | 1.3 | 0.9 | 0.5 | 1.1 | 0.2 | 0.8 | 0.9 | 0.9 | 0.5 | 0.6 | 0.9 | 0.3 | 0.6 | 0.6 | 8.06 |
| YNL307C | 0.8 | 0.6 | 3.1 | 1.3 | 1.0 | 1.9 | 0.9 | 0.9 | 1.0 | 0.9 | 1.0 | 1.0 | 1.0 | 0.7 | 0.6 | 1.0 | 1.0 | 1.3 | 2.79 |
| YPR028W | 1.0 | 1.1 | 4.0 | 1.7 | 1.0 | 1.4 | 0.8 | 0.7 | 0.5 | 0.8 | 1.4 | 0.6 | 1.7 | 0.8 | 0.9 | 1.3 | 1.2 | 1.7 | 4.06 |
| YPR149W | 1.0 | 1.1 | 5.9 | 1.7 | 1.7 | 1.6 | 2.1 | 0.6 | 1.2 | 1.8 | 1.1 | 0.7 | 1.4 | 1.3 | 1.0 | 2.2 | 1.1 | 1.1 | 2.65 |
| YAL016W | 0.8 | 1.0 | 2.8 | 0.9 | 0.9 | 0.9 | 1.1 | 1.0 | 1.2 | 1.9 | 0.9 | 0.8 | 1.4 | 0.9 | 1.1 | 1.1 | 0.8 | 1.3 | 1.00 |
| YBR283C | 0.6 | 0.7 | 1.9 | 1.2 | 1.4 | 2.2 | 0.8 | 0.8 | 1.5 | 1.2 | 2.0 | 0.8 | 0.8 | 0.6 | 1.4 | 0.9 | 0.9 | 0.9 | 2.62 |
| YBR286W | 0.7 | 1.4 | 3.8 | 1.2 | 1.3 | 1.5 | 1.3 | 0.9 | 1.2 | 1.6 | 1.0 | 1.1 | 1.8 | 1.5 | 1.3 | 2.6 | 0.8 | 1.6 | 2.98 |
| YCL008C | 0.8 | 1.1 | 1.8 | 1.2 | 1.2 | 0.9 | 0.9 | 0.7 | 0.9 | 1.4 | 0.9 | 0.9 | 1.0 | 0.9 | 1.4 | 0.6 | 0.9 | 0.9 | 0.47 |
| YCR069W | 1.0 | 1.2 | 2.0 | 0.9 | 1.1 | 1.4 | 1.1 | 0.7 | 0.8 | 0.7 | 1.3 | 1.4 | 1.6 | 1.0 | 2.4 | 1.2 | 1.2 | 1.3 | 0.97 |
| YDL061C | 1.4 | 0.5 | 2.9 | 0.9 | 1.3 | 1.1 | 0.5 | 1.0 | 0.0 | 0.5 | 1.4 | 1.0 | 0.1 | 0.7 | 1.0 | 0.6 | 1.1 | 1.0 | 4.40 |
| YDR151C | 1.3 | 1.0 | 2.3 | 1.8 | 0.8 | 0.7 | 0.8 | 1.5 | 2.6 | 2.8 | 2.1 | 0.9 | 2.8 | 1.1 | 0.6 | 1.2 | 0.8 | 1.1 | 0.96 |
| YDR382W | 1.3 | 0.5 | 2.4 | 1.2 | 1.1 | 0.6 | 0.4 | 1.2 | 0.0 | 0.4 | 1.2 | 0.7 | 0.1 | 0.7 | 1.1 | 0.4 | 1.0 | 1.0 | 6.40 |
| YDR385W | 0.6 | 0.4 | 3.5 | 0.9 | 1.1 | 0.5 | 0.4 | 0.7 | 0.2 | 0.5 | 0.9 | 0.9 | 0.3 | 0.5 | 0.8 | 0.3 | 0.6 | 0.4 | 7.27 |

| | | | | | | | | | | | | | | | | | | | |
|---|---|---|---|---|---|---|---|---|---|---|---|---|---|---|---|---|---|---|---|
| YDR407C | 0.7 | 1.0 | 2.0 | 0.7 | 0.8 | 1.2 | 1.4 | 0.9 | 1.5 | 1.7 | 1.2 | 1.0 | 1.3 | 0.8 | 1.3 | 1.0 | 0.9 | 0.9 | 0.52 |
| YGL206C | 0.4 | 3.5 | 2.0 | 0.5 | 0.6 | 0.8 | 0.8 | 0.5 | 0.7 | -0.7 | 0.9 | 0.7 | 1.1 | 0.8 | 1.3 | 0.6 | 0.8 | 1.0 | 1.04 |
| YGR172C | 0.6 | 1.3 | 2.5 | 1.3 | 0.6 | 0.9 | 1.0 | 0.8 | 0.4 | 1.1 | 0.8 | 0.8 | 0.7 | 1.1 | 1.9 | 1.0 | 1.1 | 1.1 | 0.94 |
| YIL015W | 1.1 | 1.7 | 2.0 | 2.5 | 0.9 | 0.4 | 0.9 | 0.9 | 0.3 | 1.1 | 0.8 | 0.9 | 0.8 | 5.9 | 1.1 | 0.8 | 0.8 | 1.0 | 0.17 |
| YIL018W | 1.0 | 0.4 | 2.3 | 1.4 | | 0.9 | 0.4 | 0.8 | 0.0 | 0.3 | 1.2 | 0.6 | 0.0 | 0.6 | 0.6 | 0.4 | 0.8 | 0.8 | 6.27 |
| YJL138C | 1.0 | 0.7 | 2.3 | 1.4 | 0.9 | 0.8 | 0.8 | 1.2 | 0.2 | 0.7 | 1.2 | 0.8 | 0.3 | 0.7 | 0.9 | 0.6 | 0.9 | 1.0 | 7.38 |
| YJL191W | 1.0 | 0.4 | 2.2 | 0.7 | | 1.3 | 1.0 | 0.8 | 0.0 | 0.4 | 1.1 | 1.0 | 0.9 | 1.1 | 0.7 | 2.2 | 1.0 | 1.0 | 1.86 |
| YJR047C | 1.1 | 0.8 | 2.6 | 1.1 | 1.1 | 1.1 | 0.4 | 0.9 | 0.7 | 0.5 | 1.1 | 0.9 | 0.9 | 0.9 | 0.6 | 0.8 | 0.8 | 0.9 | 5.29 |
| YJR119C | 0.9 | 1.0 | 2.0 | 1.0 | 0.9 | 1.4 | 1.1 | 1.6 | 0.5 | 1.3 | 1.1 | 0.9 | 1.2 | 1.3 | 1.5 | 1.0 | 0.7 | 0.9 | 0.30 |
| YJR123W | 1.2 | 0.6 | 2.2 | 1.0 | 1.3 | 1.0 | 0.4 | 1.2 | 0.0 | 0.5 | 0.9 | 0.8 | 0.1 | 0.6 | 1.1 | 0.4 | 0.8 | 0.9 | 7.84 |
| YJR145C | 0.8 | 0.7 | 2.3 | 1.3 | | 0.4 | 0.4 | 1.0 | 0.0 | 0.4 | 0.8 | 0.6 | 0.0 | 0.6 | 0.8 | 0.5 | 0.8 | 0.9 | 4.82 |
| YLR110C | 1.4 | 0.8 | 2.7 | 1.2 | 1.7 | 0.9 | 0.4 | 0.8 | 0.3 | 0.8 | 3.2 | 0.7 | 0.5 | 0.5 | 1.7 | 0.8 | 0.9 | 1.1 | 3.99 |
| YLR264W | 1.1 | 0.4 | 2.2 | 1.0 | 1.1 | 0.7 | 0.4 | 1.0 | 0.0 | 0.3 | 0.8 | 1.1 | 0.3 | 1.2 | 1.2 | 0.5 | 1.0 | 1.1 | 3.91 |
| YLR340W | 0.8 | 0.4 | 2.6 | 1.0 | 0.6 | 0.7 | 0.3 | 0.9 | 0.0 | 0.5 | 1.4 | 0.9 | 0.1 | 0.5 | 1.3 | 0.2 | 0.9 | 0.7 | 6.55 |
| YLR388W | 1.6 | 0.4 | 1.9 | 1.0 | 2.5 | 0.7 | 0.5 | 1.2 | 0.2 | 0.7 | 1.0 | 0.6 | 0.1 | 0.8 | 0.6 | 0.5 | 0.8 | 1.0 | 4.48 |
| YMR092C | 0.6 | 1.2 | 1.9 | 1.3 | 0.8 | 0.9 | 1.2 | 0.9 | 1.5 | 1.7 | 1.2 | 0.9 | 1.7 | 0.8 | 1.1 | 1.2 | 0.9 | 1.3 | 1.43 |
| YMR101C | 1.1 | 1.8 | 1.9 | 2.9 | 0.9 | 0.4 | 0.9 | 0.8 | -4.0 | 1.6 | -0.3 | 0.7 | 0.8 | 1.1 | 1.0 | 0.9 | 0.7 | 0.8 | 0.16 |
| YNL069C | 1.2 | 0.3 | 2.8 | 2.3 | 0.7 | 0.4 | 0.3 | 1.0 | 0.0 | 0.4 | 1.0 | 0.6 | 0.1 | 0.9 | 1.0 | 0.3 | 0.8 | 0.6 | 4.03 |
| YNL135C | 1.1 | 0.9 | 2.0 | 1.4 | 1.7 | 0.9 | 0.8 | 0.5 | 0.7 | 0.5 | 0.7 | 0.8 | 0.9 | 0.5 | 1.0 | 1.4 | 1.2 | 1.7 | 3.05 |
| YOL039W | 1.2 | 0.3 | 3.9 | 0.7 | 1.3 | 0.4 | 0.3 | 1.0 | 0.0 | 0.9 | 1.1 | 0.7 | 0.1 | 0.5 | 0.8 | 0.4 | 0.7 | 0.7 | 4.94 |
| YOL120C | 1.0 | 0.3 | 2.3 | 0.8 | 1.5 | 0.3 | 0.4 | 0.8 | 0.0 | 0.3 | 1.0 | 0.7 | 0.1 | 0.6 | 0.6 | 0.3 | 0.6 | 0.8 | 4.42 |
| YOR230W | 1.2 | 2.3 | 3.0 | 1.0 | 0.9 | 1.1 | 0.6 | 0.6 | 0.6 | 0.7 | 1.6 | 1.4 | 1.5 | 0.7 | 0.6 | 1.6 | 1.2 | 1.6 | 1.76 |
| YOR298W | 0.9 | 0.9 | 3.2 | 0.5 | 1.5 | 0.9 | 0.6 | 0.7 | 0.6 | 1.0 | 0.1 | 0.7 | 1.1 | 2.1 | 1.2 | 0.5 | 1.0 | 0.8 | 0.41 |
| YPL048W | 0.7 | 0.7 | 2.6 | 1.2 | 0.7 | 0.6 | 0.6 | 0.8 | 1.6 | 0.6 | 1.7 | 1.0 | 0.9 | 0.6 | 1.5 | 1.3 | 1.2 | 1.0 | 2.11 |
| YPL179W | 1.0 | 1.5 | 2.4 | 1.0 | 1.1 | 1.6 | 1.2 | 0.8 | 1.2 | 1.1 | 1.1 | 1.0 | 1.3 | 0.8 | 1.2 | 1.4 | 1.3 | 1.3 | 1.38 |
| YPL218W | 1.5 | 0.9 | 2.5 | 1.7 | 1.1 | 1.5 | 1.3 | 1.4 | 1.1 | 1.2 | 1.1 | 1.2 | 1.0 | 1.5 | 1.1 | 1.7 | 1.1 | 1.8 | 2.98 |
| YPL220W | 1.3 | 0.9 | 2.3 | 1.4 | 1.1 | 1.3 | 0.6 | 1.2 | 0.0 | 0.5 | 1.3 | 0.5 | 0.1 | 0.6 | 0.6 | 0.5 | 1.1 | 1.1 | 8.33 |
| YPR080W | 1.3 | 1.3 | 2.8 | 1.2 | 1.4 | 1.4 | 0.9 | 1.3 | 0.7 | 1.1 | 1.0 | 1.0 | 1.3 | 0.6 | 1.5 | 0.6 | 1.1 | 1.0 | 8.03 |
| YPR181C | 0.5 | 0.8 | 2.3 | 0.6 | 1.0 | 2.0 | 1.3 | 0.5 | 1.0 | 0.7 | 1.3 | 1.0 | 1.7 | 2.4 | 1.5 | 1.6 | 1.8 | 1.6 | 1.84 |
| YBR290W | 1.3 | 1.4 | 0.8 | 3.0 | 1.0 | 1.1 | 1.4 | 1.5 | 2.0 | 1.8 | 1.2 | 1.0 | 1.7 | 1.4 | 0.9 | 1.3 | 1.2 | 2.1 | 1.34 |
| YCR091W | 1.3 | 1.1 | 0.6 | 2.2 | 1.3 | 1.3 | 1.3 | 1.1 | 0.8 | 1.2 | 0.8 | 1.1 | 1.3 | 1.3 | 0.7 | 2.7 | 1.3 | 1.4 | 0.22 |
| YFL026W | 1.0 | 0.8 | 1.2 | 5.0 | 1.4 | 1.0 | 1.2 | 1.3 | 0.6 | 1.5 | 1.3 | 0.8 | 1.1 | 1.1 | 1.0 | 1.0 | 1.0 | 1.0 | 0.27 |

| | | | | | | | | | | | | | | | | | | | |
|---|---|---|---|---|---|---|---|---|---|---|---|---|---|---|---|---|---|---|---|
| YOR003W | 0.9 | 1.1 | 1.3 | 3.3 | 1.6 | 1.1 | 1.1 | 0.9 | 1.6 | 1.3 | 0.7 | 0.8 | 1.0 | 1.2 | 2.8 | 1.3 | 1.0 | 1.1 | 0.30 |
| YCR038C | 0.9 | 1.1 | 0.9 | 2.5 | 0.9 | 0.9 | 0.9 | 0.9 | 0.7 | 0.7 | 1.1 | 0.8 | 1.0 | 1.1 | 0.7 | 1.0 | 0.9 | 1.1 | 0.31 |
| YDL119C | 1.0 | 0.8 | 0.6 | 2.2 | 0.9 | 0.6 | 1.3 | 1.1 | 1.3 | 1.1 | 1.5 | 0.9 | 1.1 | 1.2 | 0.5 | 1.2 | 1.0 | 1.0 | 0.77 |
| YDL220C | 0.9 | 1.3 | 1.1 | 1.6 | 1.2 | 1.8 | 1.1 | 0.8 | 0.2 | 1.3 | 0.8 | 1.0 | 1.6 | 0.9 | 1.6 | 0.8 | 1.1 | 0.9 | 0.24 |
| YDR125C | 1.1 | 0.9 | 1.0 | 1.7 | 1.1 | 0.8 | 1.0 | 1.2 | 0.7 | 1.9 | 1.0 | 0.8 | 0.9 | 1.0 | 0.8 | 1.7 | 0.9 | 1.2 | 0.35 |
| YDR225W | 1.0 | 0.6 | 0.6 | 1.6 | 1.9 | 0.5 | 0.4 | 0.8 | 0.3 | 0.7 | 2.7 | 0.4 | 0.2 | 0.8 | 0.1 | 0.8 | 1.1 | 1.8 | 5.54 |
| YER066W | 0.9 | 0.7 | 0.9 | 2.1 | 1.5 | 1.3 | 1.3 | 0.7 | 1.7 | 1.4 | 1.5 | 0.7 | 0.9 | 1.1 | 0.5 | 3.3 | 1.1 | 1.1 | 0.66 |
| YER076C | 0.8 | 0.9 | 1.2 | 1.9 | 1.1 | 0.6 | 0.8 | 0.7 | 0.2 | 1.2 | 0.7 | 0.7 | 1.1 | 0.9 | 0.8 | 1.3 | 1.0 | 1.0 | 0.33 |
| YFR006W | 0.9 | 1.8 | 1.1 | 3.4 | 0.7 | 0.8 | 1.4 | 1.0 | -1.6 | 0.7 | 1.2 | 1.0 | 0.9 | 0.9 | 0.8 | 1.2 | 0.9 | 1.3 | 1.54 |
| YGL208W | 0.9 | 1.8 | 1.1 | 2.2 | 0.7 | 0.6 | 1.1 | 0.7 | 1.4 | 1.3 | 1.3 | 0.8 | 1.5 | 0.9 | 1.8 | 3.1 | 0.8 | 1.3 | 0.32 |
| YGR023W | 1.0 | 0.9 | 1.1 | 4.2 | 1.0 | 1.2 | 1.0 | 1.2 | 1.2 | 3.2 | 2.4 | 0.9 | 1.4 | 1.3 | 1.1 | 0.9 | 1.2 | 1.5 | 0.69 |
| YGR108W | 0.9 | 3.9 | 0.3 | 3.6 | 0.8 | 0.1 | 0.3 | 0.5 | 0.0 | 0.1 | 0.5 | 0.4 | 0.3 | 0.8 | 0.2 | 0.4 | 0.7 | 1.0 | 0.93 |
| YHR195W | 1.1 | 1.5 | 0.6 | 2.4 | 1.5 | 1.0 | 1.3 | 1.6 | 1.8 | 0.8 | 1.4 | 0.9 | 1.2 | 1.2 | 0.7 | 3.7 | 1.0 | 1.5 | 1.12 |
| YIL050W | 1.0 | 1.3 | 1.3 | 2.4 | 0.9 | 0.7 | 1.1 | 1.2 | 1.3 | 1.9 | 0.9 | 1.0 | 1.0 | 1.3 | 0.6 | 2.7 | 1.0 | 1.1 | 0.52 |
| YJR050W | 1.0 | 0.6 | 0.7 | 2.1 | 1.2 | 1.4 | 1.1 | 1.8 | 2.0 | 1.3 | 0.8 | 0.8 | 1.9 | 1.3 | 0.9 | 1.5 | 0.9 | 1.1 | 0.85 |
| YKL093W | 1.0 | 1.6 | 1.4 | 3.6 | 0.8 | 0.8 | 0.7 | 0.9 | 1.5 | 1.6 | 0.9 | 0.9 | 1.2 | 1.1 | 0.8 | 2.9 | 0.8 | 1.0 | 0.38 |
| YMR053C | 1.1 | 1.0 | 0.8 | 2.1 | 0.9 | 1.3 | 0.9 | 1.0 | 2.2 | 1.4 | 1.3 | 0.9 | 1.5 | 1.3 | 2.4 | 2.3 | 1.1 | 1.6 | 0.31 |
| YNL139C | 0.8 | 1.1 | 0.8 | 2.0 | 1.9 | 1.0 | 1.0 | 0.9 | 0.6 | 0.7 | 0.8 | 0.5 | 1.2 | 0.7 | 1.4 | 1.1 | 1.1 | 1.0 | 0.47 |
| YOR122C | 1.0 | 0.7 | 1.8 | 1.9 | 1.7 | 1.3 | 1.2 | 1.0 | 1.1 | 0.9 | 1.5 | 0.8 | 1.0 | 1.2 | 1.6 | 1.5 | 1.0 | 2.2 | 3.06 |
| YOR312C | 1.2 | 0.4 | 1.0 | 1.9 | 0.7 | 0.6 | 0.6 | 1.2 | 0.0 | 0.3 | 0.8 | 0.7 | 0.1 | 0.7 | 0.7 | 0.5 | 1.2 | 1.0 | 5.24 |
| YOR327C | 2.3 | 1.8 | 0.5 | 1.9 | 1.0 | 1.4 | 1.4 | 1.9 | 1.4 | 2.2 | 2.1 | 1.0 | 2.1 | 1.5 | 0.9 | 1.5 | 2.2 | 2.1 | 1.46 |
| YPL001W | 0.9 | 0.9 | 1.0 | 2.5 | 1.7 | 0.7 | 1.0 | 1.2 | 0.7 | 1.0 | 1.3 | 0.7 | 0.8 | 1.3 | 1.3 | 0.8 | 0.9 | 1.2 | 0.74 |
| YPL230W | 1.2 | 1.7 | 1.8 | 4.0 | 0.8 | 1.0 | 1.1 | 1.0 | 0.9 | 2.7 | 3.1 | 1.0 | 2.0 | 1.3 | 0.7 | 2.0 | 1.0 | 1.6 | 0.47 |
| YER025W | 0.6 | 0.7 | 0.6 | 0.6 | 1.5 | 1.0 | 1.5 | 1.1 | 0.4 | 0.6 | 1.2 | 0.7 | 0.6 | 2.1 | 0.8 | 2.6 | 1.8 | 2.3 | 1.96 |
| YHR185C | 0.9 | 1.1 | 1.4 | 0.7 | 1.4 | 1.0 | 1.3 | 0.8 | -0.6 | 0.9 | 0.7 | 0.8 | 0.4 | 1.7 | 1.3 | 3.2 | 1.4 | 3.0 | 1.64 |
| YIL076W | 1.3 | 0.6 | 1.1 | 1.0 | 1.8 | 0.6 | 0.5 | 1.3 | 1.0 | 1.4 | 1.1 | 0.8 | 0.8 | 1.4 | 1.0 | 0.8 | 1.2 | 2.5 | 3.73 |
| YMR238W | 1.9 | 1.3 | 1.1 | 1.3 | 1.5 | 1.3 | 1.2 | 0.9 | 1.3 | 1.4 | 1.2 | 1.0 | 1.5 | 1.2 | 2.3 | 1.4 | 2.2 | 2.4 | 1.36 |
| YBR009C | 1.6 | 0.8 | 0.4 | 1.7 | 1.4 | 1.0 | 0.5 | 1.2 | 0.6 | 0.6 | 0.8 | 0.5 | 0.4 | 1.1 | 0.2 | 0.4 | 1.0 | 2.4 | 7.00 |
| YBR010W | 1.0 | 0.8 | 0.5 | 2.0 | 0.8 | 1.1 | 0.8 | 1.3 | 0.5 | 0.7 | 1.0 | 0.7 | 0.4 | 1.0 | 0.3 | 0.8 | 1.1 | 2.3 | 7.25 |
| YCL067C | 1.2 | 1.1 | 0.3 | 1.7 | 1.1 | 1.7 | 1.3 | 1.7 | 1.4 | 1.9 | 1.6 | 1.0 | 1.4 | 2.1 | 2.2 | 1.7 | 0.8 | 2.2 | 3.15 |
| YCR096C | 1.1 | 0.9 | 0.4 | 1.6 | 2.1 | 1.2 | 1.7 | 1.5 | 1.2 | 1.6 | 1.4 | 1.0 | 1.0 | 2.1 | 2.1 | 1.8 | 1.0 | 2.1 | 2.34 |
| YDL137W | 1.4 | 1.1 | 1.2 | 1.2 | 1.8 | 1.0 | 2.0 | 1.1 | 1.1 | 1.3 | 1.5 | 1.6 | 1.7 | 1.4 | 1.3 | 2.3 | 1.3 | 2.2 | 4.95 |

| | | | | | | | | | | | | | | | | | | | |
|---|---|---|---|---|---|---|---|---|---|---|---|---|---|---|---|---|---|---|---|
| YDL192W | 1.4 | 1.0 | 0.6 | 1.2 | 1.4 | 1.0 | 1.7 | 1.5 | 0.8 | 0.6 | 1.3 | 1.1 | 0.9 | 1.2 | 1.3 | 1.3 | 1.1 | 2.3 | 5.57 |
| YDR224C | 1.1 | 0.7 | 0.8 | 0.9 | 1.2 | 1.0 | 0.8 | 1.4 | 1.1 | 1.3 | 1.1 | 0.7 | 0.5 | 1.7 | 0.3 | 1.1 | 1.2 | 2.2 | 5.62 |
| YDR378C | 1.3 | 1.3 | 1.4 | 1.3 | 1.0 | 1.0 | 1.4 | 1.5 | 0.5 | 1.3 | 0.8 | 0.9 | 0.8 | 1.4 | 0.8 | 1.0 | 1.7 | 2.2 | 2.40 |
| YMR197C | 1.3 | 1.6 | 1.4 | 1.9 | 1.1 | 1.6 | 1.5 | 1.4 | 1.9 | 2.5 | 1.0 | 1.1 | 1.5 | 4.1 | 1.7 | 1.9 | 1.2 | 2.2 | 1.20 |
| YOL109W | 2.0 | 2.0 | 1.5 | 1.8 | 1.0 | 1.3 | 1.2 | 1.5 | 0.1 | 1.1 | 1.0 | 0.8 | 0.5 | 1.4 | 1.0 | 0.8 | 1.6 | 2.2 | 4.13 |
| YPL010W | 1.0 | 1.1 | 0.9 | 1.0 | 1.4 | 1.0 | 1.9 | 1.5 | 1.7 | 1.5 | 1.3 | 1.1 | 1.1 | 1.3 | 1.2 | 1.3 | 1.3 | 2.0 | 3.25 |
| YHR132C | 0.8 | 0.7 | 1.7 | 1.4 | 1.0 | 0.7 | 0.8 | 0.8 | 0.9 | 1.1 | 1.1 | 0.8 | 1.2 | 0.9 | 1.4 | 2.3 | 0.8 | 1.3 | 1.52 |
| YJL141C | 1.0 | 0.8 | 1.0 | 1.6 | 1.5 | 1.1 | 1.1 | 0.9 | 1.2 | 1.3 | 1.2 | 0.8 | 1.4 | 0.8 | 0.8 | 2.2 | 1.4 | 1.6 | 0.94 |
| YKR098C | 1.0 | 1.1 | 1.2 | 1.7 | 1.1 | 1.8 | 1.7 | 1.3 | 1.4 | 2.7 | 1.9 | 1.1 | 1.4 | 1.4 | 0.9 | 3.9 | 1.1 | 1.6 | 0.55 |
| YLR206W | 0.7 | 1.6 | 1.4 | 1.1 | 1.1 | 0.5 | 0.7 | 0.8 | 1.5 | 1.0 | 1.3 | 0.8 | 1.4 | 0.8 | 2.4 | 2.3 | 1.1 | 1.2 | 0.72 |
| YAL055W | 1.2 | 1.0 | 0.9 | 1.0 | 1.4 | 1.3 | 1.5 | 1.9 | 1.0 | 1.4 | 1.3 | 1.2 | 1.5 | 1.6 | 1.1 | 2.6 | 1.3 | 1.3 | 0.62 |
| YAR062W | 1.2 | 1.8 | 1.4 | 0.7 | 1.0 | 0.6 | 1.3 | 0.9 | 0.4 | 0.9 | -1.1 | 0.8 | 1.2 | 1.1 | 1.3 | 2.4 | 0.9 | 1.0 | 0.32 |
| YBL102W | 0.8 | 1.0 | 1.4 | 1.2 | 0.9 | 2.5 | 1.6 | 0.8 | 2.3 | 1.1 | 1.2 | 0.9 | 1.7 | 1.1 | 1.1 | 2.0 | 2.4 | 1.5 | 1.26 |
| YBR161W | 1.2 | 0.8 | 2.3 | 0.7 | 1.1 | | 3.0 | 0.9 | 0.4 | 0.4 | 1.1 | 0.6 | 1.0 | 0.9 | 1.5 | 2.1 | 1.5 | 2.2 | 0.72 |
| YCR039C | 1.9 | 2.1 | 0.3 | 0.9 | 1.9 | 1.5 | 1.4 | 1.6 | 1.8 | 2.2 | 1.2 | 0.8 | 1.0 | 1.7 | 1.9 | 2.1 | 1.0 | 2.1 | 1.73 |
| YDL018C | 1.3 | 0.9 | 0.9 | 1.1 | 1.8 | 1.4 | 1.2 | 1.3 | 1.0 | 1.0 | 0.9 | 0.7 | 1.0 | 1.5 | 1.1 | 3.1 | 1.5 | 1.7 | 0.84 |
| YDR022C | 1.1 | 1.2 | 0.8 | 0.9 | 1.5 | 2.2 | 1.8 | 1.4 | 0.9 | 1.4 | 0.9 | 0.7 | 1.3 | 2.1 | 1.0 | 2.1 | 1.3 | 1.2 | 0.47 |
| YDR181C | 1.1 | 1.2 | 0.8 | 1.4 | 0.9 | 0.7 | 1.0 | 1.2 | 2.0 | 0.9 | 0.8 | 0.7 | 0.7 | 1.3 | 1.2 | 2.0 | 0.8 | 1.2 | 0.48 |
| YGR036C | 0.9 | 0.8 | 0.9 | 1.1 | 1.3 | 1.3 | 1.0 | 0.8 | 0.9 | 0.9 | 0.9 | 1.0 | 1.3 | 1.0 | 0.7 | 2.0 | 1.3 | 1.1 | 1.12 |
| YGR120C | 1.2 | 1.8 | 0.5 | 1.0 | 1.8 | 1.5 | 1.6 | 1.3 | 0.3 | 1.2 | 1.1 | 0.7 | 0.5 | 1.0 | 1.2 | 1.9 | 1.1 | 1.2 | 0.36 |
| YGR131W | 0.8 | 2.1 | 1.9 | 1.1 | 0.9 | 1.9 | 1.1 | 1.1 | 1.9 | 5.8 | 1.1 | 0.5 | 1.4 | 2.0 | 0.8 | 2.6 | 1.3 | 1.0 | 0.41 |
| YGR167W | 1.1 | 1.1 | 0.6 | 1.5 | 0.9 | 1.7 | 1.4 | 1.4 | 1.4 | 1.9 | 1.1 | 1.1 | 1.6 | 1.7 | 1.6 | 2.8 | 1.3 | 1.7 | 1.79 |
| YHL024W | 1.2 | 0.8 | 1.6 | 1.1 | 1.5 | 1.2 | 1.3 | 0.8 | 2.1 | 3.6 | 1.1 | 1.6 | 1.6 | 2.1 | 2.9 | 3.8 | 1.4 | 1.4 | 0.58 |
| YJL113W | 1.2 | 1.9 | 1.2 | 1.2 | | 0.7 | 1.2 | 1.3 | 1.3 | 2.1 | 1.2 | 0.7 | 0.9 | 1.3 | 1.7 | 2.1 | 0.9 | 1.5 | 0.41 |
| YJL146W | 1.3 | 1.9 | 1.2 | 2.0 | 0.8 | 0.7 | 1.3 | 1.0 | 1.0 | 1.7 | -1.1 | 1.1 | 0.8 | 1.5 | 1.2 | 1.8 | 1.0 | 1.3 | 0.30 |
| YJR019C | 0.8 | 1.8 | 1.9 | 1.6 | 1.1 | 0.8 | 0.9 | 0.7 | 1.1 | 0.6 | 2.4 | 1.1 | 1.4 | 0.8 | 1.1 | 2.6 | 1.8 | 1.4 | 0.33 |
| YJR049C | 1.0 | 1.2 | 1.0 | 1.0 | 0.7 | 0.6 | 1.0 | 1.2 | 1.4 | 1.4 | 1.6 | 1.0 | 2.1 | 1.2 | 1.2 | 1.7 | 0.9 | 1.2 | 0.44 |
| YLR078C | 0.9 | 1.1 | 0.7 | 1.4 | | 0.8 | 1.2 | 1.1 | 0.8 | 1.1 | 2.9 | 1.0 | 0.8 | 1.2 | 0.6 | 2.2 | 1.2 | 1.2 | 1.02 |
| YNR037C | 0.9 | 0.9 | 0.9 | 1.7 | 1.2 | 0.7 | 1.7 | 1.1 | 1.3 | 1.7 | 1.5 | 0.7 | 2.0 | 1.2 | 0.8 | 1.9 | 1.4 | 1.6 | 1.34 |
| YOR028C | 1.4 | 0.7 | 1.1 | 2.3 | 1.6 | 1.0 | 1.2 | 1.5 | 1.2 | 2.0 | 1.5 | 0.6 | 0.8 | 1.3 | 1.1 | 3.7 | 0.8 | 1.7 | 0.78 |

The tables show that the expressed mRNA of about 700 of 2400 unknown yeast genes was induced by any one of chemical substances such as heavy metals, agricultural chemicals, surfactants (Table 1), as well as the expressed mRNA of 167 mitochondria-located genes (Table 2), 52 DNA repair genes (Table 3), 161 energy genes (Table 4), 142 transport facilitation protein genes (Table 5), 90 stress protein genes (Table 6), 142 metabolism genes (Table 7), 60 detoxification genes (Table 8), and 507 genes belonging to other category (Table 9). Here, when the value of the following is 2 or more, then it is considered significant:

$$\frac{\text{The level of expressed mRNA in the presence of chemical substance}}{\text{The level of expressed mRNA in the absence of chemical substance.}}$$

The inventors of the present application understood that the induction of certain yeast genes by toxic substances is caused by the activation of the promoters of the genes by the toxic substances. Thus, the inventors prepared a vector that comprised a polynucleotide sequence comprising the promoter of the yeast gene, which sequence is operably linked to a polynucleotide encoding a marker protein; and transformed yeast cells with the vector. Such cells enable us to detect readily toxic substances by detecting the expressed marker proteins (hereinafter, such detection may be referred to as "promoter assay".). The working examples hereinafter illustrate the preparation of such vectors, transformation of yeast cells by use of said vectors, and the detection of toxic substances by use of the transformed cells.

Promoter assay is a method for determining variations of the level of intracellular genes without destroying cells on the basis of the level of expressed marker genes instead of the expressed mRNA. The gene selected to detect a chemical substance is expressed in the absence of chemical substances, and thus a marker protein also occurs in the absence of chemical substances. In the method according to the present invention, the behavior of yeast genes on the addition of test materials is determined on the basis of the level of expressed marker protein so that the presence or the absence, and the kind of toxic chemical substances are predicted. Thus, it is desired that the production of marker proteins is lower in the absence of chemical substances, and the production of marker proteins is higher in the presence of chemical substances. Under the circumstance, yeast gene as used in promoter assay is selected, of which intensity (level of expressed gene in control cells/average level of expressed whole genes) is preferably 1.5 or less, more preferably 1 or less, even more preferably 0.5 or less, and of which expression magnification (expressed mRNA in the presence of chemical substance/expressed mRNA in the absence of chemical substance) is preferably 3 or more, more preferably 10 or more, even more preferably 20 or more.

Example 2

Primers for PCR to amplify the polynucleotide comprising the promoter of yeast gene YKL071w were prepared. Primers were designed using a primer design software, Oligo4.0-S, Sequencher I Mackintosh version. The base sequence of the upper primer was:
CGCAATAATACTGGAAACATCAA (SEQ ID No: 7),
whereas the base sequence of the lower primer was:
ATCGACTTTGTTTGCTTAGAAT (SEQ ID No: 8).
For PCR, yeast chromosome *(Saccharomyces cerevisiae* S288C, Cat.40802, Research Genetics, Inc.) was used as template, and the commercially available kit (KOD DNA Polymerase; Code KOD-101, Toyobo) was used as reagents.

Type YEp shuttle vector pYES2 (pYES2, Cat no: V825-20, Invirtogen Corporation, USA), which can be replicated both in *E.coli.* and yeast was used as vector (R. W. Old, S. B. Primrose Principles of Gene Manipulation 5th Ed., BAIFUKAN CO., LTD, pp138-165, pp.234-263, 2000). The portion of vector pQBI 63 (Cat no.54-0082, Wako Pure Chemical Industries, Ltd.) corresponding to a marker protein GFP was used as the polynucleotide encoding GFP (SEQ ID No: 6). First, the GFP polynucleotide was inserted into the multiple cloning sites of pYES2 to give a vector. Thereafter, the GAL 1 promoter of pYES2 was replaced with the polynucleotide comprising the promoter sequence of the intended yeast gene YKL071w (SEQ ID No: 1) to give an intended plasmid vector. Suitable restriction enzymes were selected, and the insertion of the polynucleotide comprising GFP and the promoter sequence was conducted.

Next, the yeast *Saccharomyces cerevisiae* W303 was transformed with the resultant plasmid vector according to the following procedures:

1) Incubating the yeast *Saccharomyces cerevisiae* W303 in 200 ml of SD medium under shaking until OD 660 reaches 0.5;
2) Suspending the collected cells in 5ml of TE-buffer;
3) Adding 250 μL of 2.5M lithium acetate;

4) Dispending each 300 μl, adding 10μl of above plasmid vector thereto, then incubating the suspensions at 30 ° C for 30 minutes;
5) Adding 700 μl of 50% PEG4000, and incubating the mixture under shaking at 30 °C for 60 minutes;
6) Giving heat shock (42°C, 5 minutes), and then immediately cooling the mixture;
7) Washing the mixture twice with 1M sorbitol; and
8) Seeding it on agar plates made of minimum essential medium.

The transformations were confirmed on selective medium (SD medium (Yeast nitrogen base without amino acids (Difco 0919-15) + glucose + amino acids (adenine, histidine, tryptophan)). Colonies that were grown on agar plate of selective medium were further checked for auxotrophy for amino acids.

The transformed yeast cells named SC-YKL071w-pQBI has been deposited as the International Deposition at National Institute of Advanced Industrial Science and Technology, International Patent Organism Depositary of Tsukuba Central 6, 1-1-1 Higashi, Tsukuba, Ibaraki, Japan under Accession No. FERM BP-8161 on August 19, 2002.

Example 3

Primers for PCR to amplify the polynucleotide comprising the promoter of yeast gene YCR102c were prepared. Primers were designed using a primer design software, Oligo4.0-S, Sequencher I

Mackintosh version. The base sequence of the upper primer was:
AGGTGCGATAGTGGGGAATAAGA (SEQ ID No: 9),
whereas the base sequence of the lower primer was:
GGTTTCTGGAATTGCAACTTGC (SEQ ID No: 10).
For PCR, yeast chromosome *(Saccharomyces cerevisiae* S288C, Cat.40802, Research Genetics, Inc.) was used as template, and the commercially available kit (KOD DNA Polymerase; Code KOD-101, Toyobo) was used as reagents.

Type YEp shuttle vector pYES2 (pYES2, Cat no: V825-20, Invirtogen Corporation, USA), which can be replicated both in *E.coli.* and yeast was used as vector (R. W. Old, S. B. Primrose Principles of Gene Manipulation 5th Ed., BAIFUKAN CO., LTD, pp138-165, pp.234-263, 2000). The portion of vector pQBI 63 (Cat no.54-0082, Wako Pure Chemical Industries, Ltd.) corresponding to a marker protein GFP was used as the polynucleotide encoding GFP (SEQ ID No: 6). First, the GFP polynucleotide was inserted into the multiple cloning sites of pYES2 to give a vector. Thereafter, the GAL1 promoter of pYES2 was replaced with the polynucleotide comprising the promoter sequence of the intended yeast gene YCR102c (SEQ ID No: 2) to give an intended plasmid vector. Suitable restriction enzymes were selected, and the insertion of the polynucleotide comprising GFP and the promoter sequence was conducted.

Next, the yeast *Saccharomyces cerevisiae* W303 was transformed with the resultant plasmid vector according to the following procedures:

1) Incubating the yeast *Saccharomyces cerevisiae* W303 in 200 ml of SD medium under shaking until OD 660 reaches 0.5;
2) Suspending the collected cells in 5ml of TE-buffer;
3) Adding 250 μL of 2.5M lithium acetate;
4) Dispending each 300 μl, adding 10μl of above plasmid vector thereto, then incubating the suspensions at 30 ° C for 30 minutes;
5) Adding 700 μl of 50% PEG4000, and incubating the mixture under shaking at 30 ° C for 60 minutes;
6) Giving heat shock (42°C, 5 minutes), and then immediately cooling the mixture;
7) Washing the mixture twice with 1M sorbitol; and
8) Seeding it on agar plates made of minimum essential medium.

The transformations were confirmed on selective medium (SD medium (Yeast nitrogen base without amino acids (Difco 0919-15) + glucose + amino acids (adenine, histidine, tryptophan)). Colonies that were grown on agar plate of selective medium were further checked for auxotrophy for amino acids.

The transformed yeast cells named SC-YCR102c-pQBI has been deposited as the International Deposition at National Institute of Advanced Industrial Science and Technology, International Patent Organism Depositary of Tsukuba Central 6, 1-1-1 Higashi, Tsukuba, Ibaraki, Japan under Accession No. FERM BP-8159 on August 19, 2002.

Example 4

Primers for PCR to amplify the polynucleotide comprising the promoter of yeast gene YOR382w were prepared. Primers were designed using a primer design software, Oligo4.0-S, Sequencher I Mackintosh version. The base sequence of the upper primer was: GCTTTTCTCGCTTCGTTATCACC (SEQ ID No: 11),

whereas the base sequence of the lower primer was:
TATTATTGTTTTGTGATGGCTT (SEQ ID No: 12).
For PCR, yeast chromosome *(Saccharomyces cerevisiae* S288C, Cat.40802, Research Genetics, Inc.) was used as template, and the commercially available kit (KOD DNA Polymerase; Code KOD-101, Toyobo) was used as reagents.
Type YEp shuttle vector pYES2 (pYES2, Cat no: V825-20, Invirtogen Corporation, USA), which can be replicated both in *E.coli*. and yeast was used as vector (R. W. Old, S. B. Primrose Principles of Gene Manipulation 5th Ed., BAIFUKAN CO., LTD, pp 138-165, pp.234-263, 2000). The portion of vector pQBI 63 (Cat no.54-0082, Wako Pure Chemical Industries, Ltd.) corresponding to a marker protein GFP was used as the polynucleotide encoding GFP (SEQ ID No: 6). First, the GFP polynucleotide was inserted into the multiple cloning sites of pYES2 to give a vector. Thereafter, the GAL1 promoter of pYES2 was replaced with the polynucleotide comprising the promoter sequence of the intended yeast gene YOR382w (SEQ ID No: 3) to give an intended plasmid vector. Suitable restriction enzymes were selected, and the insertion of the polynucleotide comprising GFP and the promoter sequence was conducted.
Next, the yeast *Saccharomyces cerevisiae* W303 was transformed with the resultant plasmid vector according to the following procedures:

1) Incubating the yeast *Saccharomyces cerevisiae* W303 in 200 ml of SD medium under shaking until OD 660 reaches 0.5;
2) Suspending the collected cells in 5ml of TE-buffer;
3) Adding 250 μL of 2.5M lithium acetate;
4) Dispending each 300 μl, adding 10μl of above plasmid vector thereto, then incubating the suspensions at 30 ° C for 30 minutes;
5) Adding 700 μl of 50% PEG4000, and incubating the mixture under shaking at 30 ° C for 60 minutes;
6) Giving heat shock (42°C, 5 minutes), and then immediately cooling the mixture;
7) Washing the mixture twice with 1M sorbitol; and
8) Seeding it on agar plates made of minimum essential medium.

The transformations were confirmed on selective medium (SD medium (Yeast nitrogen base without amino acids (Difco 0919-15) + glucose + amino acids (adenine, histidine, tryptophan)). Colonies that were grown on agar plate of selective medium were further checked for auxotrophy for amino acids.
The transformed yeast cells named SC-YOR382W-pQBI has been deposited as the International Deposition at National Institute of Advanced Industrial Science and Technology, International Patent Organism Depositary of Tsukuba Central 6, 1-1-1 Higashi, Tsukuba, Ibaraki, Japan under Accession No. FERM BP-8160 on August 19, 2002.

Example 5

Primers for PCR to amplify the polynucleotide comprising the promoter of yeast gene YLL057c were prepared. Primers were designed using a primer design software, Oligo4.0-S, Sequencher I Mackintosh version. The base sequence of the upper primer was:
GCTAACGAACAGGATGGTATTGA (SEQ ID No: 13),
whereas the base sequence of the lower primer was:
ATTTTAACTGGGTTACTGTGCT (SEQ ID No: 14).
For PCR, yeast chromosome *(Saccharomyces cerevisiae* S288C, Cat.40802, Research Genetics, Inc.) was used as template, and the commercially available kit (KOD DNA Polymerase; Code KOD-101, Toyobo) was used as reagents.
Type YEp shuttle vector pYES2 (pYES2, Cat no: V825-20, Invirtogen Corporation, USA), which can be replicated both in *E.coli.* and yeast was used as vector (R. W. Old, S. B. Primrose Principles of Gene Manipulation 5th Ed., BAIFUKAN CO., LTD, pp 138-165, pp.234-263, 2000). The portion of vector pQBI 63 (Cat no.54-0082, Wako Pure Chemical Industries, Ltd.) corresponding to a marker protein GFP was used as the polynucleotide encoding GFP (SEQ ID No: 6). First, the GFP polynucleotide was inserted into the multiple cloning sites of **pYES2** to give a vector. Thereafter, the GAL1 promoter of pYES2 was replaced with the polynucleotide comprising the promoter sequence of the intended yeast gene YLL057c (SEQ ID No: 4) to give an intended plasmid vector. Suitable restriction enzymes were selected, and the insertion of the polynucleotide comprising GFP and the promoter sequence was conducted.
Next, the yeast *Saccharomyces cerevisiae* W303 was transformed with the resultant plasmid vector according to the following procedures:

1) Incubating the yeast *Saccharomyces cerevisiae* W303 in 200 ml of SD medium under shaking until OD 660 reaches 0.5;
2) Suspending the collected cells in 5ml of TE-buffer;

3) Adding 250 μL of 2.5M lithium acetate;
4) Dispending each 300 μl, adding 10μl of above plasmid vector thereto, then incubating the suspensions at 30°C for 30 minutes;
5) Adding 700 μl of 50% PEG4000, and incubating the mixture under shaking at 30 ° C for 60 minutes;
6) Giving heat shock (42°C, 5 minutes), and then immediately cooling the mixture;
7) Washing the mixture twice with 1M sorbitol; and
8) Seeding it on agar plates made of minimum essential medium.

The transformations were confirmed on selective medium (SD medium (Yeast nitrogen base without amino acids (Difco 0919-15) + glucose + amino acids (adenine, histidine, tryptophan)). Colonies that were grown on agar plate of selective medium were further checked for auxotrophy for amino acids.
The transformed yeast cells named SC-YLL057C-pQBI was deposited at National Institute of Advanced Industrial Science and Technology, International Patent Organism Depositary of Tsukuba Central 6, 1-1-1 Higashi, Tsukuba, Ibaraki, Japan under Accession No. FERM P-18439 on July 27, 2001, and then transferred into the International Deposition under Budapest Treaty as FERM BP-8158 on August 19, 2002.

Example 6

Primers for PCR to amplify the polynucleotide comprising the promoter of yeast gene YLR303w were prepared. Primers were designed using a primer design software, Oligo4.0-S, Sequencher I Mackintosh version. The base sequence of the upper primer was:
TCGTTTTCTACTTTCTTCTGCTG (SEQ ID No: 15),
whereas the base sequence of the lower primer was:
TGTATGGATGGGGGTAATAGAA (SEQ ID No: 16).
For PCR, yeast chromosome *(Saccharomyces cerevisiae* S288C, Cat.40802, Research Genetics, Inc.) was used as template, and the commercially available kit (KOD DNA Polymerase; Code KOD-101, Toyobo) was used as reagents.
Type YEp shuttle vector pYES2 (pYES2, Cat no: V825-20, Invirtogen Corporation, USA), which can be replicated both in *E.coli.* and yeast was used as vector (R. W. Old, S. B. Primrose Principles of Gene Manipulation 5th Ed., BAIFUKAN CO., LTD, pp 138-165, pp.234-263, 2000). The portion of vector pQBI 63 (Cat no.54-0082, Wako Pure Chemical Industries, Ltd.) corresponding to a marker protein GFP was used as the polynucleotide encoding GFP (SEQ ID No: 6). First, the GFP polynucleotide was inserted into the multiple cloning sites of pYES2 to give a vector. Thereafter, the GAL1 promoter of pYES2 was replaced with the polynucleotide comprising the promoter sequence of the intended yeast gene YLR303w (SEQ ID No: 5) to give an intended plasmid vector. Suitable restriction enzymes were selected, and the insertion of the polynucleotide comprising GFP and the promoter sequence was conducted.
Next, the yeast *Saccharomyces cerevisiae* W303 was transformed with the resultant plasmid vector according to the following procedures:

1) Incubating the yeast *Saccharomyces cerevisiae* W303 in 200 ml of SD medium under shaking until OD 660 reaches 0.5;
2) Suspending the collected cells in 5ml of TE-buffer;
3) Adding 250 μL of 2.5M lithium acetate;
4) Dispending each 300 μl, adding 10μl of above plasmid vector thereto, then incubating the suspensions at 30°C for 30 minutes;
5) Adding 700 μl of 50% PEG4000, and incubating the mixture under shaking at 30 °C for 60 minutes;
6) Giving heat shock (42°C, 5 minutes), and then immediately cooling the mixture;
7) Washing the mixture twice with 1M sorbitol; and
8) Seeding it on agar plates made of minimum essential medium.

The transformations were confirmed on selective medium (SD medium (Yeast nitrogen base without amino acids (Difco 0919-15) + glucose + amino acids (adenine, histidine, tryptophan)). Colonies that were grown on agar plate of selective medium were further checked for auxotrophy for amino acids.
The transformed yeast cells named SC-YLR303W-pQBI was deposited at National Institute of Advanced Industrial Science and Technology, International Patent Organism Depositary of Tsukuba Central 6, 1-1-1 Higashi, Tsukuba, Ibaraki, Japan under Accession No. FERM P-18438 on July 27, 2001, and then transferred into the International Deposition under Budapest Treaty as FERM BP-8157 on
August 19,2002.

Example 7

The cells of SC-YKL071W-pQBI as prepared in Example 2 were contacted to one of the following compounds. The yeast cells SC-YKL071W-pQBI were incubated at 25 °C in SD medium (Yeast nitrogen base without amino acids (Difco 0919-15) + glucose + amino acids (adenine, histidine, tryptophan)). One of toxic chemical substances as shown below was added to the cells at logarithmic growth phase, and then the cells were further incubated for two hours. Cells were incubated without any chemical substance in the same condition, and was used as control.
(1) benzo(a)pyrene, (2) bisphenol-A, (3) (2-ethylhexyl)phthalate, (4) 2,5-dichlorophenol, (5) 2,4-dichlorophenoxyacetic acid, (6) formaldehyde, (7) methylmercury chloride, (8) 4-nitroquinolin-N-oxide, (9) p-nonylphenol, (10) pentachlorophenol, (11) sodium arsenite, (12) Tetramethylthiuram disulfide, (13) tributyltin chloride, (14) 2,4,5-trichlorophenol, (15) Trp-P-2 (acetate), (16) paraquat, (17) cadmium chloride, (18) γ-hexachlorocyclohexane, (19) malathon, (20) manganese ethylenebis(dithiocarbamate), (21) nickel (II) chloride, (22) potassium bichromate, (23) triphenyltin chloride, (24) phenol, (25) S-4-Chlorobenzyl-N,N-diethylthiocarbamate, (26) hexachlorophene, (27) triclosan, (28) mercury(II) chloride, (29) copper sulfate (II), (30) potassium cyanide, and (31) dimethylsulfoxide.
After contacted, the yeast cells were washed once with physiological saline, and then immobilized with a physiological saline containing 5% formalin, after which the fluorescence was determined by flow cytometry (EPICS XL: BECKMAN COULTER). Fluorometric range of the control was first defined. When the number of the cells having a higher fluorescence than the control was under 1%, "-" was indicated as not showing any fluorescence, whereas when the number was between 1% and 2%, and 2% or more, then "+" and "++" were indicated as showing a fluorescence, respectively. The results are shown in Table 10.

Table 10

| Chemical substances | Concentrations | Fluorescens |
|---|---|---|
| (1) benzo(a)pyrene | 0.2 mM | - |
| (2) bisphenol-A | 0.4 mM | - |
| (3) (2-ethylhexyl)phthalate | 83.3 mM | - |
| (4) 2,5-dichlorophenol | 0.3 mM | - |
| (5) 2,4-dichlorophenoxyacetic acid | 0.3 mM | - |
| (6) formaldehyde | 0.2 mM | - |
| (7) methylmercury chloride | 0.2 μM | - |
| (8) 4-nitroquinolin-N-oxide | 0.6 μM | - |
| (9) p-nonylphenol | 10 μM | - |
| (10) pentachlorophenol | 50 μM | - |
| (11) sodium arsenite | 0.3 mM | - |
| (12) Tetramethylthiuram disulfide | 20 μM | + |
| (13) tributyltin chloride | 0.4 μM | - |
| (14) 2,4,5-trichlorophenol | 30 mM | - |
| (15) Trp-P-2 (acetate) | 0.2 mM | - |
| (16) paraquat | 16.7 mM | - |
| (17) cadmium chloride | 40 μM | - |
| (18) γ-hexachlorocyclohexane | 6.7 mM | - |
| (19) malathon | 22.2 mM | - |
| (20) manganese ethylenebis (dithiocarbamate) | 0.8 mM | - |
| (21) nickel (II) chloride | 3.3 mM | - |
| (22) potassium bichromate | 0.3 mM | - |
| (23) triphenyltin chloride | 10 μM | - |
| (24) phenol | 5.6 mM | - |
| (25) S-4-Chlorobenzyl-N,N-diethylthiocarbamate | 0.7 mM | - |
| (26) hexachlorophene | 30 μM | - |
| (27) triclosan | 730 μM | - |
| (28) mercury(II) chloride | 50 μM | - |
| (29) copper sulfate (II) | 3.3 mM | - |
| (30) potassium cyanide | 16.7 mM | - |
| (31) dimethylsulfoxide | 3.7 % | - |

Table 10 shows that tetramethylthiuram disulfide induced the expression of GFP.

Example 8

The cells of SC-YCR102C-pQBI as prepared in Example 3 were contacted to one of the following compounds. The yeast cells SC-YCR102C-pQBI were incubated at 25 ° C in SD medium (Yeast nitrogen base without amino acids (Difco 0919-15) + glucose + amino acids (adenine, histidine, tryptophan)). One of toxic chemical substances as shown below was added to the cells at logarithmic growth phase, and then the cells were further incubated for two hours. Cells were incubated without any chemical substance in the same condition, and was used as control. (1) benzo(a)pyrene, (2) bisphenol-A, (3) (2-ethylhexyl)phthalate, (4) 2,5-dichlorophenol, (5) 2,4-dichlorophenoxyacetic acid, (6) formaldehyde, (7) methylmercury chloride, (8) 4-nitroquinolin-N-oxide, (9) p-nonylphenol, (10) pentachlorophenol, (11) sodium arsenite, (12) Tetramethylthiuram disulfide, (13) tributyltin chloride, (14) 2,4,5-trichlorophenol, (15) Trp-P-2 (acetate), (16) paraquat, (17) cadmium chloride, (18) y-hexachlorocyclohexane, (19) malathon, (20) manganese ethylenebis(dithiocarbamate), (21) nickel (II) chloride, (22) potassium bichromate, (23) triphenyltin chloride, (24) phenol, (25) S-4-Chlorobenzyl-N,N-diethylthiocarbamate, (26) hexachlorophene, (27) triclosan, (28) mercury(II) chloride, (29) copper sulfate (II), (30) potassium cyanide, and (31) dimethylsulfoxide.

After contacted, the yeast cells were washed once with physiological saline, and then immobilized with a physiological saline containing 5% formalin, after which the fluorescence was determined by flow cytometry (EPICS XL: BECKMAN COULTER). Fluorometric range of the control was first defined. When the number of the cells having a higher fluorescence than the control was under 1%, "-" was indicated as not showing any fluorescence, whereas when the number was between 1% and 2%, and 2% or more, then "+" and "++" were indicated as showing a fluorescence, respectively. The results are shown in Table 11.

Table 11

| Chemical substances | Concentrations | Fluorescence |
|---|---|---|
| (1) benzo(a)pyrene | 0.2 mM | - |
| (2) bisphenol-A | 0.4 mM | - |
| (3) (2-ethylhexyl)phthalate | 83.3 mM | - |
| (4) 2,5-dichlorophenol | 0.3 mM | - |
| (5) 2,4-dichlorophenoxyacetic acid | 0.3 mM | - |
| (6) formaldehyde | 0.2 mM | - |
| (7) methylmercury chloride | 0.2 μM | - |
| (8) 4-nitroquinolin-N-oxide | 0.6 μM | - |
| (9) p-nonylphenol | 10 μM | - |
| (10) pentachlorophenol | 50 μM | - |
| (11) sodium arsenite | 0.3 mM | - |
| (12) Tetramethylthiuram disulfide | 20 μM | + |
| (13) tributyltin chloride | 0.4 μM | - |
| (14) 2,4,5-trichlorophenol | 30 mM | - |
| (15) Trp-P-2 (acetate) | 0.2 mM | - |
| (16) paraquat | 16.7 mM | - |
| (17) cadmium chloride | 40 μM | - |
| (18) γ-hexachlorocyclohexane | 6.7 mM | - |
| (19) malathon | 22.2 mM | - |
| (20) manganese ethylenebis (dithiocarbamate) | 0.8 mM | , - |
| (21) nickel (II) chloride | 3.3 mM | - |
| (22) potassium bichromate | 0.3 mM | - |
| (23) triphenyltin chloride | 10 μM | - |
| (24) phenol | 5.6 mM | - |
| (25) S-4-Chlorobenzyl-N,N-diethylthiocarbamate | 0.7 mM | - |
| (26) hexachlorophene | 30 μM | - |
| (27) triclosan | 730 μM | - |
| (28) mercury(II) chloride | 50 μM | - |
| (29) copper sulfate (II) | 3.3 mM | - |

(continued)

| Chemical substances | Concentrations | Fluorescence |
|---|---|---|
| (30) potassium cyanide | 16.7 mM | - |
| (31) dimethylsulfoxide | 3.7 % | - |

Table 11 shows that tetramethylthiuram disulfide induced the expression of GFP.

Example 9

The cells of SC-YOR382W-pQBI as prepared in Example 4 were contacted to one of the following compounds. The yeast cells SC-YOR382W-pQBI were incubated at 25 ° C in SD medium (Yeast nitrogen base without amino acids (Difco 0919-15) + glucose + amino acids (adenine, histidine, tryptophan)). One of toxic chemical substances as shown below was added to the cells at logarithmic growth phase, and then the cells were further incubated for two hours. Cells were incubated without any chemical substance in the same condition; and was used as control.
(1) benzo(a)pyrene, (2) bisphenol-A, (3) (2-ethylhexyl)phthalate, (4) 2,5-dichlorophenol, (5) 2,4-dichlorophenoxyacetic acid, (6) formaldehyde, (7) methylmercury chloride, (8) 4-nitroquinolin-N-oxide, (9) p-nonylphenol, (10) pentachlorophenol, (11) sodium arsenite, (12) Tetramethylthiuram disulfide, (13) tributyltin chloride, (14) 2,4,5-trichlorophenol, (15) Trp-P-2 (acetate), (16) paraquat, (17) cadmium chloride, (18) γ-hexachlorocyclohexane, (19) malathon, (20) manganese ethylenebis(dithiocarbamate), (21) nickel (II) chloride, (22) potassium bichromate, (23) triphenyltin chloride, (24) phenol, (25) S-4-Chlorobenzyl-N,N-diethylthiocarbamate, (26) hexachlorophene, (27) triclosan, (28) mercury(II) chloride, (29) copper sulfate (II), (30) potassium cyanide, and (31) dimethylsulfoxide.

After contacted, the yeast cells were washed once with physiological saline, and then immobilized with a physiological saline containing 5% formalin, after which the fluorescence was determined by flow cytometry (EPICS XL: BECKMAN COULTER). Fluorometric range of the control was first defined. When the number of the cells having a higher fluorescence than the control was under 1%, "-" was indicated as not showing any fluorescence, whereas when the number was between 1% and 2%, and 2% or more, then "+" and "++" were indicated as showing a fluorescence, respectively. The results are shown in Table 12.

Table 12

| Chemical sbstances | Concentrations | Fluorescence |
|---|---|---|
| (1) benzo(a)pyrene | 0.2 mM | - |
| (2) bisphenol-A | 0.4 mM | - |
| (3) (2-ethylhexyl)phthalate | 83.3 mM | - |
| (4) 2,5-dichlorophenol | 0.3 mM | ++ |
| (5) 2,4-dichlorophenoxyacetic acid | 0.3 mM | - |
| (6) formaldehyde | 0.2 mM | - |
| (7) methylmercury chloride | 0.2 μM | - |
| (8) 4-nitroquinolin-N-oxide | 0.6 μM | ++ |
| (9) p-nonylphenol | 10 μM | ++ |
| (10) pentachlorophenol | 50 μM | - |
| (11) sodium arsenite | 0.3 mM | - |
| (12) Tetramethylthiuram disulfide | 20 μM | - |
| (13) tributyltin chloride | 0.4 μM | - |
| (14) 2,4,5-trichlorophenol | 30 mM | ++ |
| (15) Trp-P-2 (acetate) | 0.2 mM | ++ |
| (16) paraquat | 16.7 mM | - |
| (17) cadmium chloride | 40 μM | - |
| (18) γ-hexachlorocyclohexane | 6.7 mM | - |
| (19) malathon | 22.2 mM | ++ |
| (20) manganese ethylenebis (dithiocarbamate) | 0.8 mM | ++ |
| (21) nickel (II) chloride | 3.3 mM | ++ |
| (22) potassium bichromate | 0.3 mM | ++ |
| (23) triphenyltin chloride | 10 μM | - |
| (24) phenol | 5.6 mM | ++ |

(continued)

| Chemical sbstances | Concentrations | Fluorescence |
|---|---|---|
| (25) S-4-Chlorobenzyl-N,N-diethylthiocarbamate | 0.7 mM | - |
| (26) hexachlorophene | 30 μM | - |
| (27) triclosan | 730 μM | - |
| (28) mercury(II) chloride | 50 μM | - |
| (29) copper sulfate (II) | 3.3 mM | - |
| (30) potassium cyanide | 16.7 mM | - |
| (31) dimethylsulfoxide | 3.7 % | ++ |

Table 12 shows that 2,4-dichlorophenoxyacetic acid, 4-nitroquinolin-N-oxide, p-nonylphenol, 2,4,5-trichlorophenol, Trp-P-2 (acetate), malathon, manganese ethylenebis(dithiocarbamate), nickel (II) chloride, potassium bichromate, phenol, and dimethylsulfoxide induced the expression of GFP.

Example 10

The cells of SC-YLL057C-pQBI as prepared in Example 5 were contacted to one of the following compounds. The yeast cells SC-YLL057C-pQBI were incubated at 25 ° C in SD medium (Yeast nitrogen base without amino acids (Difco 0919-15) + glucose + amino acids (adenine, histidine, tryptophan)). One of toxic chemical substances as shown below was added to the cells at logarithmic growth phase, and then the cells were further incubated for two hours. Cells were incubated without any chemical substance in the same condition, and was used as control.

(1) benzo(a)pyrene, (2) bisphenol-A, (3) (2-ethylhexyl)phthalate, (4) 2,5-dichlorophenol, (5) 2,4-dichlorophenoxyacetic acid, (6) formaldehyde, (7) methylmercury chloride, (8) 4-nitroquinolin-N-oxide, (9) p-nonylphenol, (10) pentachlorophenol, (11) sodium arsenite, (12) Tetramethylthiuram disulfide, (13) tributyltin chloride, (14) 2,4,5-trichlorophenol, (15) Trp-P-2 (acetate), (16) paraquat, (17) cadmium chloride, (18) γ-hexachlorocyclohexane, (19) malathon, (20) manganese ethylenebis(dithiocarbamate), (21) nickel (II) chloride, (22) potassium bichromate, (23) triphenyltin chloride, (24) phenol, (25) S-4-Chlorobenzyl-N,N-diethylthiocarbamate, (26) hexachlorophene, (27) triclosan, (28) mercury(II) chloride, (29) copper sulfate (II), (30) potassium cyanide, and (31) dimethylsulfoxide.

After contacted, the yeast cells were washed once with physiological saline, and then immobilized with a physiological saline containing 5% formalin, after which the fluorescence was determined by flow cytometry (EPICS XL: BECKMAN COULTER). Fluorometric range of the control was first defined. When the number of the cells having a higher fluorescence than the control was under 1%, "-" was indicated as not showing any fluorescence, whereas when the number was between 1% and 2%, and 2% or more, then "+" and "++" were indicated as showing a fluorescence, respectively. The results are shown in Table 13.

Table 13

| Chemical substances | Concentrations | Fluorescence |
|---|---|---|
| (1) benzo(a)pyrene | 0.2 mM | - |
| (2) bisphenol-A | 0.4 mM | - |
| (3) (2-ethylhexyl)phthalate | 83.3 mM | - |
| (4) 2,5-dichlorophenol | 0.3 mM | - |
| (5) 2,4-dichlorophenoxyacetic acid | 0.3 mM | ++ |
| (6) formaldehyde | 0.2 mM | - |
| (7) methylmercury chloride | 0.2 μM | - |
| (8) 4-nitroquinolin-N-oxide | 0.6 μM | - |
| (9) p-nonylphenol | 10 μM | - |
| (10) pentachlorophenol | 50 μM | - |
| (11) sodium arsenite | 0.3 mM | ++ |
| (12) Tetramethylthiuram disulfide | 20 μM | - |
| (13) tributyltin chloride | 0.4 μM | - |
| (14) 2,4,5-trichlorophenol | 30 mM | - |
| (15) Trp-P-2 (acetate) | 0.2 mM | - |
| (16) paraquat | 16.7 mM | - |
| (17) cadmium chloride | 40 μM | ++ |

(continued)

| Chemical substances | Concentrations | Fluorescence |
|---|---|---|
| (18) γ-hexachlorocyclohexane | 6.7 mM | - |
| (19) malathon | 22.2 mM | - |
| (20) manganese ethylenebis (dithiocarbamate) | 0.8 mM | - |
| (21) nickel (II) chloride | 3.3 mM | - |
| (22) potassium bichromate | 0.3 mM | - |
| (23) triphenyltin chloride | 10 μM | - |
| (24) phenol | 5.6 mM | - |
| (25) S-4-Chlorobenzyl-N,N-diethylthiocarbamate | 0.7 mM | - |
| (26) hexachlorophene | 30 μM | - |
| (27) triclosan | 730 μM | - |
| (28) mercury(II) chloride | 50 μM | - |
| (29) copper sulfate (II) | 3.3 mM | - |
| (30) potassium cyanide | 16.7 mM | ++ |
| (31) dimethylsulfoxide | 3.7 % | - |

Table 13 shows that 2,4-dichlorophenoxyacetic acid, sodium arsenite, cadmium chloride, and potassium cyanide induced the expression of GFP.

Example 11

The cells of SC-YCR303W-pQBI as prepared in Example 6 were contacted to one of the following compounds. The yeast cells SC-YCR303W-pQBI were incubated at 25 °C in SD medium (Yeast nitrogen base without amino acids (Difco 0919-15) + glucose + amino acids (adenine, histidine, tryptophan)). One of toxic chemical substances as shown below was added to the cells at logarithmic growth phase, and then the cells were further incubated for two hours. Cells were incubated without any chemical substance in the same condition, and was used as control. (1) benzo(a)pyrene, (2) bisphenol-A, (3) (2-ethylhexyl)phthalate, (4) 2,5-dichlorophenol, (5) 2,4-dichlorophenoxyacetic acid, (6) formaldehyde, (7) methylmercury chloride, (8) 4-nitroquinolin-N-oxide, (9) p-nonylphenol, (10) pentachlorophenol, (11) sodium arsenite, (12) Tetramethylthiuram disulfide, (13) tributyltin chloride, (14) 2,4,5-trichlorophenol, (15) Trp-P-2 (acetate), (16) paraquat, (17) cadmium chloride, (18) γ-hexachlorocyclohexane, (19) malathon, (20) manganese ethylenebis(dithiocarbamate), (21) nickel (II) chloride, (22) potassium bichromate, (23) triphenyltin chloride, (24) phenol, (25) S-4-Chlorobenzyl-N,N-diethylthiocarbamate, (26) hexachlorophene, (27) triclosan, (28) mercury(II) chloride, (29) copper sulfate (II), (30) potassium cyanide, and (31) dimethylsulfoxide.

After contacted, the yeast cells were washed once with physiological saline, and then immobilized with a physiological saline containing 5% formalin, after which the fluorescence was determined by flow cytometry (EPICS XL: BECKMAN COULTER). Fluorometric range of the control was first defined. When the number of the cells having a higher fluorescence than the control was under 1%, "-" was indicated as not showing any fluorescence, whereas when the number was between 1% and 2%, and 2% or more, then "+" and "++" were indicated as showing a fluorescence, respectively. The results are shown in Table 14.

Table 14

| Chemical substances | Concentrations | Fluorescence |
|---|---|---|
| (1) benzo(a)pyrene | 0.2 mM | ++ |
| (2) bisphenol-A | 0.4 mM | - |
| (3) (2-ethylhexyl)phthalate | 83.3 mM | - |
| (4) 2,5-dichlorophenol | 0.3 mM | - |
| (5) 2,4-dichlorophenoxyacetic acid | 0.3 mM | ++ |
| (6) formaldehyde | 0.2 mM | ++ |
| (7) methylmercury chloride | 0.2 μM | - |
| (8) 4-nitroquinolin-N-oxide | 0.6 μM | - |
| (9) p-nonylphenol | 10 μM | - |

(continued)

| Chemical substances | Concentrations | Fluorescence |
|---|---|---|
| (10) pentachlorophenol | 50 μM | - |
| (11) sodium arsenite | 0.3 mM | ++ |
| (12) Tetramethylthiuram disulfide | 20 μM | - |
| (13) tributyltin chloride | 0.4 μM | - |
| (14) 2,4,5-trichlorophenol | 30 mM | - |
| (15) Trp-P-2 (acetate) | 0.2 mM | - |
| (16) paraquat | 16.7 mM | - |
| (17) cadmium chloride | 40 μM | ++ |
| (18) γ-hexachlorocyclohexane | 6.7 mM | - |
| (19) malathon | 22.2 mM | - |
| (20) manganese ethylenebis (dithiocarbamate) | 0.8 mM | + |
| (21) nickel (II) chloride | 3.3 mM | - |
| (22) potassium bichromate | 0.3 mM | - |
| (23) triphenyltin chloride | 10 μM | - |
| (24) phenol | 5.6 mM | - |
| (25) S-4-Chlorobenzyl-N,N-diethylthiocarbamate | 0.7 mM | - |
| (26) hexachlorophene | 30 μM | - |
| (27) triclosan | 730 μM | - |
| (28) mercury(II) chloride | 50 μM | ++ |
| (29) copper sulfate (II) | 3.3 mM | - |
| (30) potassium cyanide | 16.7 mM | ++ |
| (31) dimethylsulfoxide | 3.7 % | - |

Table 14 shows that benzo(a)pyrene, 2,4-dichlorophenoxyacetic acid, formaldehyde, sodium arsenite, cadmium chloride , manganese ethylenebis(dithiocarbamate), mercury(II) chloride, and potassium cyanide induced the expression of GFP.

Preferred Embodiments

1. A polynucleotide which comprises a polynucleotide sequence operably linked to a polynucleotide encoding a marker protein, wherein the polynucleotide sequence comprises a promoter from yeast genes that is selected from a group consisting of the following, or a promoter from a gene that is homologous to the yeast genes and is derived from other species:

YBR072W, YCR102C, YCR107W, YDL218W, YDL243C, YDR453C, YDR533C, YFL014W, YFL056C, YFL057C, YGR110W, YJR155W, YKL071W, YKR076W, YLL060C, YLR460C, YMR090W, YNL331C, YNL332W, YNL335W, YOL150C, YOL165C, YPL171C, YPR167C, YBL048W, YBL064C, YBL107C, YBR008C, YBR173C, YBR256C, YBR296C, YDL021W, YFL022C, YFL024C, YFL061W, YGL121C, YGL158W, YGR043C, YHR029C, YHR112C, YHR139C, YHR179W, YHR209W, YIR030C, YJR010W, YJR048W, YKL001C, YKL107W, YKR075C, YKR097W, YLL056C, YLR297W, YLR303W, YML087C, YMR096W, YNL274C, YOL151W, YOR226C, YOR338W, YOR391C, YPL280W, YDR406W, YJL153C, YLR346C, YOR049C, YOR153W, YPL088W, YAL034C, YDL124W, YDL174C, YDR476C, YGL156W, YGR035C, YGR157W, YGR213C, YGR281W, YGR284C, YHL047C, YHR043C, YHR044C, YHR054C, YJR073C, YKL165C, YLR008C, YMR315W, YNL211C, YOL031C, YOL101C, YOR303W, YAL005C, YAR031W, YBL005W-A, YBL022C, YBL041W, YBL049W, YBL075C, YBL078C, YBR062C, YBR169C, YBR294W, YCL020W, YCL035C, YCL043C, YCL050C, YCL057W, YCR012W, YCR013C, YCR060W, YDL007W, YDL027C, YDL097C, YDL110C, YDL126C, YDL169C, YDR070C, YDR155C, YDR158W, YDR204W, YDR210W, YDR214W, YDR258C, YDR313C, YDR368W, YDR435C, YER012W, YER037W, YER091C, YER103W, YFL044C, YFR003C, YFR010W, YFR020W, YFR024C, YFR044C, YFR053C, YGL006W, YGL048C, YGL062W, YGL141W, YGL157W, YGL163C, YGL180W, YGL184C, YGR010W, YGR028W, YGR032W, YGR048W, YGR124W, YGR135W, YGR142W, YGR161C,YGR192C, YGR197C, YGR201C, YGR212W, YGR231C, YGR244C, YGR254W, YGR268C, YHL030W, YHR016C, YHR018C, YHR055C, YHR087W, YHR166C, YIL160C, YIR017C, YJL034W, YJL048C, YJL052W, YJL144W, YJL163C, YJR009C, YJR069C, YJR074W, YJR130C, YJR149W, YKL065C, YKL073W, YKL103C, YKL142W, YKL210W, YKL218C, YKR011C, YKR018C, YKR046C, YKR049C, YLL024C, YLL026W, YLR027C, YLR080W, YLR107W, YLR121C, YLR132C, YLR133W, YLR155C, YLR158C, YLR161W, YLR195C, YLR217W, YLR328W, YLR336C, YLR345W, YLR370C, YLR423C, YML004C, YML092C, YML128C, YML130C, YML131W, YMR040W, YMR118C, YMR214W, YMR251W, YMR297W, YMR322C, YNL036W, YNL055C, YNL071W, YNL094W, YNL134C, YNL155W, YNL160W, YNL239W, YNL241C, YOL005C, YOR020C, YOR027W, YOR037W, YOR059C, YOR120W, YOR134W, YOR152C, YOR173W, YOR289W, YOR362C, YPL240C, YPR030W, YAL008W, YAL023C, YAL060W, YAL062W, YAR009C, YBL101C, YBR006W, YBR046C, YBR052C, YBR053C, YBR056W, YBR099C, YBR137W, YBR139W, YBR149W, YBR170C, YBR177C, YBR203W, YBR207W, YBR212W, YBR239C, YBR284W, YBR293W, YCL018W, YCL033C, YCL040W, YCL049C, YCR062W, YCR067C, YCR082W, YDL010W, YDL020C, YDL024C, YDL054C, YDL095W, YDL100C, YDL115C, YDL144C, YDL198C, YDL223C, YDL245C, YDL246C, YDR001C, YDR032C, YDR058C, YDR072C, YDR127W, YDR168W, YDR169C, YDR188W, YDR231C, YDR261C, YDR264C, YDR272W, YDR293C, YDR304C, YDR330W, YDR403W, YDR411C, YDR427W, YDR436W, YDR497C, YDR511W, YDR516C, YDR519W, YDR545W, YEL012W, YEL030W, YER004W, YER009W, YER021W, YER035W, YER053C, YER079W, YER094C, YER096W, YER125W, YER158C, YER163C, YER175C, YER177W,YER178W, YER185W, YFL006W, YFL010C, YFL016C, YFL029C, YFL030W, YFL031W, YFL032W, YFL038C, YFL041W, YFR004W, YFR047C, YFR050C, YFR052W, YGL011C, YGL013C, YGL037C, YGL047W, YGL053W, YGL091C, YGL094C, YGL127C, YGL150C, YGL199C, YGL207W, YGL248W, YGR008C, YGR037C, YGR055W, YGR101W, YGR130C, YGR154C, YGR194C, YGR221C, YGR232W, YGR248W, YGR253C, YGR256W, YHL008C, YHR027C, YHR053C, YHR057C, YHR111W, YHR138C, YHR161C, YHR164C, YHR169W, YHR174W, YHR176W, YHR199C, YIL010W, YIL034C, YIL041W, YIL045W, YIL087C, YIL107C, YIL142W, YIL155C, YIR034C, YIR036C, YIR037W, YIR038C, YIR039C, YJL001W, YJL031C, YJL035C, YJL053W, YJL057C, YJL066C, YJL068C, YJL082W, YJL099W, YJL102W, YJL151C, YJL152W, YJL161W, YJL164C, YJL171C, YJL172W, YJL210W, YJL219W, YJR008W, YJR045C, YJR046W, YJR106W, YJR117W, YJR137C, YKL007W, YKL026C, YKL035W, YKL091C, YKL104C, YKL117W, YKL145W, YKL146W, YKL151C, YKL152C, YKL153W, YKL193C, YKL195W, YKL213C, YKL215C, YLL028W, YLL039C, YLL058W, YLR054C, YLR103C, YLR120C YLR136C, YLR149C, YLR152C, YLR178C, YLR259C, YLR299W, YLR324W, YLR327C, YLR348C, YLR350W, YLR356W, YLR362W, YLR387C, YLR429W, YML054C, YML070W, YML100W, YML117W, YML125C, YMR004W, YMR008C, YMR009W, YMR020W, YMR067C, YMR089C, YMR097C, YMR102C, YMR105C, YMR107W, YMR152W, YMR180C, YMR184W, YMR191W, YMR219W, YMR271C, YMR275C, YMR295C, YMR314W, YMR316W, YNL006W, YNL007C, YNL012W, YNL044W, YNL045W, YNL074C, YNL092W, YNL093W, YNL104C, YNL115C,YNL156C, YNL231C, YNL234W, YNL237W, YNL281W, YNL305C, YNL333W, YNR010W, YNR019W, YNR033W, YNR059W, YNR068C, YNR069C, YOL032W, YOL036W, YOL047C, YOL071W, YOL082W, YOL083W, YOL117W, YOL119C, YOL126C, YOL131W, YOL153C, YOL162W, YOL163W, YOL164W, YOR019W, YOR035C, YOR036W, YOR099W, YOR117W, YOR124C, YOR130C, YOR132W, YOR157C, YOR185C, YOR197W, YOR259C, YOR261C, YOR273C, YOR288C, YOR332W, YOR336W, YOR347C, YPL017C, YPL087W, YPL106C, YPL109C, YPL149W, YPL154C, YPL196W, YPL206C, YPL222W, YPR023C, YPR024W, YPR026W, YPR067W, YPR103W, YPR108W, YPR151C, YAL012W, YBR029C, YBR222C, YCL009C, YCL027W, YCL064C, YCR098C, YDL222C, YDR055W, YDR077W, YDR502C, YELOO1C, YEL042W, YER026C, YER106W, YGR136W, YGR138C, YHR137W, YHR142W, YIL023C, YIL153W, YJL073W, YJR004C, YJR054W, YKL039W, YKL086W, YKL163W, YKR091W, YLR109W, YLR194C, YLR250W, YMR095C, YMR189W, YNL106C, YNL169C, YNL322C, YOR181W, YOR198C, YOR208W, YOR247W, YPL089C, YAL038W, YAL053W, YBR023C, YBR214W, YBR295W, YCR048W, YDL072C, YDL204W, YDR085C, YDR098C, YDR259C, YDR380W, YDR388W, YDR391C, YDR432W, YDR481C, YDR510W, YER069W, YGL022W, YGL126W, YGL209W, YGL255W, YGR189C, YGR282C, YHL035C, YHR030C, YIL022W, YIL024C, YIL117C, YIL123W, YIL140W, YIL154C, YJL088W, YJL 108C, YJL149W, YJL159W, YJL186W, YJR148W, YKL096W, YLR180W, YLR273C, YLR300W, YLR307W, YLR378C, YLR391W, YMR094W, YMR104C, YMR276W, YMR296C, YNL190W, YNL208W, YNL300W, YNR064C, YOL013C, YOL058W, YOR248W, YOR355W, YPL052W, YPL163C, YPR079W, YAR028W, YBR146W, YBR183W, YCL038C, YCR071C, YDL008W, YDR019C, YDR031W, YDR115W, YDR486C, YER038C, YER130C, YFL054C, YGL136C, YGR146C, YGR207C, YHL040C, YIL167W, YJL020C, YKR039W, YLR031W, YLR205C, YMR072W, YMR140W, YMR173W, YMR195W, YMR226C, YNL037C, YNR002C, YOL143C, YOR136W, YOR215C, YOR382W, YOR383C, YPL054W, YPL271W, YPR127W, YAL044C, YAL054C, YARO10C, YAR027W, YAR071W, YBL001C, YBL043W, YBL057C, YBR014C, YBR024W, YBR035C, YBR068C, YBR111C, YBR116C, YBR147W, YBR168W, YBR246W, YBR273C, YCR004C, YCR021C, YCR037C, YCR088W, YDL022W, YDL128W, YDL238C, YDR003W, YDR009W, YDR033W, YDR084C, YDR104C, YDR270W, YDR315C, YDR340W, YDR357C, YDR358W, YDR396W, YDR405W, YDR410C, YDR434W, YDR482C, YDR487C, YDR520C, YDR534C, YDR539W, YEL011W, YEL065W, YEL066W, YER039C, YER044C, YER067W, YER080W, YER107C, YFL020C, YFL028C, YFL043C, YFR015C, YGL001C, YGL008C, YGL068W, YGL073W, YGL104C, YGL113W, YGL154C, YGL167C, YGL229C, YGL242C, YGL249W, YGL253W, YGR052W, YGR060W, YGR065C, YGR106C, YGR111W, YGR220C, YGR257C, YHL023C, YHL048W, YHR004C, YHR037W, YHR071W, YHR092C, YHR190W, YIL007C, YIL033C, YIL070C, YIL088C, YIL111W, YIR002C, YIR016W, YIR035C, YIR043C, YJL012C, YJL083W, YJL089W, YJL116C, YJL131C, YJL132W, YJL196C, YJR061W, YJR086W, YJR142W,YJR161C, YKL008C, YKL013C, YKL041W, YKL067W, YKL138C, YKL139W, YKL150W, YKL175W, YKR006C, YKR014C, YKR070W, YLL023C, YLR023C, YLR093C, YLR118C, YLR142W, YLR225C, YLR241W, YLR251W, YLR252W, YLR270W, YML030W, YML110C, YMR021C, YMR027W, YMR148W, YMR181C, YMR262W, YMR272C, YMR298W, YNL011C, YNL130C, YNL214W, YNL259C, YOL 129W, YOR042W, YOR052C, YOR137C, YOR149C, YOR165W, YOR270C, YOR285W, YOR367W, YPL018W, YPL156C, YPL186C, YPL203W, YPL216W, YPL255W, YPR006C, YPR073C, YPR098C, YBR050C, YBR145W, YBR299W, YDR518W, YEL020C, YFL062W, YGL039W, YGL134W, YJL217W, YJR159W, YLR126C, YNL249C, YNL284C, YNL336W, YOL157C, YOR344C, YOR381W, YPL265W, YPR124W, YBR074W, YBR109C, YBR126C, YBR201W, YCR005C, YDL248W, YDR041W, YDR105C, YDR268W, YDR452W, YEL075C, YER046W, YER050C, YER136W, YER159C, YGL250W, YGR019W, YGR042W, YGR053C, YGR066C, YGR247W, YGR255C, YGR295C, YHL044W, YHR145C, YIL058W, YIL065C, YIL083C, YIL098C, YIL172C, YJL030W, YJL185C, YJL213W, YJR029W, YJR099W, YJR122W, YJR125C, YKL190W, YKR020W, YLL025W, YLL051C, YLR043C, YLR090W, YLR100W, YLR108C, YLR290C, YML068W, YMR051C, YMR139W, YMR178W, YMR193W, YNL015W, YNL079C, YNL122C, YNL223W, YNL285W, YNL293W, YNR007C, YNR035C, YNR061C, YOL016C, YOL104C, YOR220W, YOR221C, YOR374W, YPL123C, YPR077C, YPR107C, YPR147C, YBR093C, YBR196C, YEL041W, YEL047C, YER023W, YER119C, YFL055W, YGR209C, YIL124W, YKL187C, YLL055W, YMR318C, YOL152W, YAL007C, YBR067C, YBR115C, YBR285W, YBR292C, YDL043C, YDL123W, YDL131W, YDL168W, YDL212W, YDR056C, YDR132C, YDR154C, YDR183W, YDR216W, YDR253C, YDR295C, YDR494W, YDR513W, YEL072W, YER045C, YER061C, YER181C, YFL052W, YFL058W, YFR030W, YGL089C, YGL096W, YGL114W, YGL193C, YGL202W, YGL204C, YGL259W, YGR006W, YGR070W, YGR088W, YHL034C, YHL036W, YHR048W, YHR104W, YHR163W, YIL060W, YIL136W, YIR024C, YJL036W, YJL045W, YJL060W, YJL101C, YJL155C, YJR085C, YJR109C, YJR156C, YKL070W, YKL161C, YKL221W, YKR071C, YLL009C, YLL050C, YLR092W, YLR145W, YLR156W, YLR163C, YLR220W, YLR280C, YLR311C, YLR390W, YML116W, YMR034C, YMR038C, YMR081C, YMR250W, YNL240C, YNL260C, YNL277W, YNR074C, YOL044W, YOL084W, YOL147C, YOL159C, YOR184W, YOR228C, YOR255W, YPL223C, YPR160W, YDL182W, YBR047W, YBR054W, YBR291C, YDR069C, YER124C, YER131W, YGR044C, YIL094C, YKR007W, YMR240C, YNR050C, YOR007C, YAL015C, YBL065W, YBR105C, YBR182C, YBR186W, YBR244W, YBR272C, YCL069W, YDL025C, YDL059C, YDL085W, YDL113C, YDL244W, YDR018C, YDR054C, YDR202C, YDR223W, YDR350C, YDR353W, YDR374C, YDR512C, YEL052W, YEL070W, YER098W, YFR017C, YGL046W, YGL067W, YGL098W, YGL117W, YGL146C, YGL240W, YGR011W, YGR067C, YGR133W, YGR153W, YGR223C, YHR116W, YHR124W, YIL097W, YIL168W, YJL103C, YJL221C, YJR036C, YJR095W, YKL085W, YKL133C, YKL162C, YKL188C, YKL217W, YKR061W, YKR105C, YLL062C, YLR174W, YLR216C, YLR247C, YLR260W, YLR267W, YLR389C, YML007W, YMR041C, YMR177W, YMR253C, YNL009W, YNL117W, YNL128W, YNL183C, YNR073C, YOL133W, YOL158C, YOR133W, YOR225W, YOR227W, YPL161C, YPL166W, YPL202C, YPL224C, YPR015C, YPR086W, YPR201W, YAL061W, YAL067C, YAR007C, YBL033C, YBL056W, YBL086C, YBR026C, YBR073W, YBR101C, YBR117C, YBR123C, YBR213W, YBR269C, YBR280C, YCR036W, YDL132W, YDL149W, YDL200C, YDL234C, YDL242W, YDR099W, YDR177W, YDR256C, YDR392W, YDR394W, YDR531W, YEL071W, YER014W, YER042W, YER090W, YER184C, YFL059W, YFR042W, YFR046C, YFR049W, YGL026C, YGL058W, YGL185C, YGL227W, YGL252C, YGL254W, YGR089W, YGR112W, YGR134W, YGR276C, YHL019C, YHR012W, YHR017W, YHR028C, YHR106W, YHR109W, YHR156C, YIL036W, YIL046W, YIL143C, YIL152W, YIL159W, YIL164C, YJL071W, YJL094C, YJL154C, YJR056C, YJR072C, YJR110W, YJR139C, YKL025C, YKL034W, YKL064W, YKL171W, YKL196C, YKR012C, YKR068C, YKR069W, YLL001W, YLL057C, YLL061W, YLR064W, YLR070C, YLR099C, YLR144C, YLR157C, YLR160C, YLR164W, YLR364W, YLR421C, YML032C, YML042W, YML112W, YML118W, YMR114C, YMR115W, YMR258C, YNL181W, YNL191W, YNL212W, YNL213C, YNL250W, YNL265C, YNL312W, YNR032W, YOL038W, YOL049W, YOL064C, YOR088W, YOR155C, YOR257W, YOR265W, YOR377W, YOR386W, YPL031C, YPL113C, YPL124W, YPL151C, YPL249C, YPL260W, YPL274W, YPR048W, YPR061C, YPR093C, YPR125W, YPR158W, YPR168W, YPR169W, YPR174C, YPR180W, YPR193C, YPR200C, YAL014C, YAL017W, YAL049C, YBL019W, YBL058W, YBR001C, YBR013C, YBR018C, YBR037C, YBR045C, YBR051W, YBR063C, YBR128C, YBR129C, YBR204C, YBR241C, YBR255W, YBR281C, YCL044C, YCL055W, YCR014C, YCR019W, YCR024C, YCR105W, YDL065C, YDL089W, YDL143W, YDL173W, YDL193W, YDL197C, YDL206W, YDL230W, YDL233W, YDR040C, YDR071C, YDR078C, YDR109C, YDR140W, YDR194C, YDR212W, YDR221W, YDR257C, YDR271C, YDR287W, YDR294C, YDR316W, YDR329C, YDR338C, YDR369C, YDR421W, YDR425W, YDR485C, YDR488C, YDR504C, YDR505C, YDR506C, YDR515W, YEL005C, YEL037C, YEL044W, YER017C, YER048C, YER052C, YER078C, YER089C, YER092W, YER100W, YER162C, YER182W, YFL021W, YFL042C, YFR045W, YFR051C, YFR056C, YGL040C, YGL041C, YGL045W, YGL057C, YGL093W, YGL105W, YGL125W, YGL166W, YGL181W, YGL183C, YGL215W, YGL216W, YGL221C, YGL223C, YGR007W, YGR029W, YGR155W, YGR156W, YGR186W, YGR198W, YGR210C, YGR211W, YGR237C, YGR250C, YGR258C, YGR266W, YGR270W, YGR274C, YGR277C, YHL021C, YHL037C, YHL038C, YHR082C, YHR083W, YHR134W, YHR160C, YHR171W, YHR180W, YHR205W, YIL062C, YIL072W, YIL075C, YIL099W, YIL108W, YIL165C, YIL170W, YIR009W, YIR018W, YIR031C, YIR032C, YJL032W, YJL049W, YJL128C, YJL165C, YJR044C, YJR052W, YJR090C, YJR091C, YJR103W, YJR104C, YJR153W, YKL059C, YKL079W, YKL090W, YKL094W, YKL192C, YKL209C, YKR052C, YKR102W, YKR106W, YLL054C, YLR025W, YLR097C, YLR200W, YLR226W, YLR248W, YLR266C, YLR392C, YLR427W, YML013W, YML029W, YML041C, YML051W, YML078W, YML079W, YML088W, YML099C, YMR056C, YMR068W, YMR091C, YMR110C, YMR160W, YMR186W, YMR255W, YNL005C, YNL026W, YNL039W, YNL063W, YNL064C, YNL077W, YNL083W, YNL147W, YNL176C, YNL194C, YNL253W, YNL257C, YNL261W, YNL264C, YNL276C, YNR006W, YNR034W, YNR047W, YNR051C, YNR071C, YOL065C, YOL067C, YOR005C, YOR008C, YOR022C, YOR023C, YOR058C, YOR069W, YOR087W, YOR138C, YOR229W, YOR256C, YOR267C, YPL005W, YPL020C, YPL022W, YPL105C, YPL147W, YPL150W, YPL152W, YPL164C, YPL168W, YPL180W, YPL188W, YPL194W, YPR025C, YPR047W, YPR049C, YPR066W, YPR081C, YPR134W, YPR140W, YPR 148C, YPR155C, YPR 172W, YPR185W, YAL018C, YAR064W, YBR012C, YBR076W, YBR287W, YDR043C, YDR250C, YDR373W, YFR014C, YGL191W, YGR180C, YHR136C, YJL026W, YJL037W, YLR038C, YNL058C, YOR031W, YGR087C, YIL166C, YHR008C, YIL129C, YGL256W, YJR030C, YMR077C, YBR264C, YPL177C, YKR040C, YGL056C, YDR128W, YGR139W, YBL101W-A, YOR253W, YOL026C, YDR278C, YHR095W, YCL042W, YNL200C, YPL221W, YLR415C, YMR058W, YPR037C, YER072W, YML028W, YOR325W, YAL039C, YMR112C, YJR107W, YGL088W, YJR058C, YNL142W, YDR090C, YMR071C, YBL093C, YGR293C, YML055W, YDL017W, YDL210W, YGL055W, YCL025C, YDR080W, YDL181W, YNR030W, YJL017W, YIL127C; YDR281C, YDR366C, YFR026C, YJL212C, YPL215W, YEL019C, YBR132C, YHL018W, YNL196C, YPL038W, YAR047C, YPL262W, YHL006C, YPL225W, YBR124W, YOR148C, YKR053C, YBL044W, YER029C, YLR360W, YCL056C, YCR007C, YGR239C, YNL256W, YPR146C, YLR377C, YKL097C, YBR066C, YLR338W, YDL229W, YBR253W, YJR027W, YKL198C, YBL030C, YBR031W, YBR118W, YBR162C, YBR221C, YCR024C-A, YCR106W, YDL046W, YDR012W, YDR133C, YDR134C, YDR276C, YDR342C, YDR343C, YEL027W, YEL034W, YGR038W, YGR243W, YGR279C, YHR094C, YHR105W, YHR175W, YHR181W, YIL056W, YIL162W, YJL059W, YJL097W, YJL158C, YJR105W, YKL051W, YKL056C, YKL097W-A, YKL100C, YKL141W, YKR066C, YLR134W, YLR258W, YLR339C, YML058W, YMR083W, YMR203W, YNL209W, YNL307C, YOL030W, YOR178C, YPL028W, YPR028W, YPR113W, YPR149W, YPR150W, YPR183W, YAL016W, YBL099W, YBL100C, YBR011C, YBR096W, YBR100W, YBR127C, YBR283C, YBR286W, YCL008C, YCL058C, YCR030C, YCR034W, YCR069W, YDL015C, YDL023C, YDL061C, YDL086W, YDR038C, YDR039C, YDR050C, YDR151C, YDR178W, YDR233C, YDR284C, YDR298C, YDR345C, YDR359C, YDR382W, YDR385W, YDR400W, YDR407C, YDR538W, YEL024W, YEL033W, YEL063C, YER057C, YER081W, YER120W, YFL011W, YGL012W, YGL206C, YGR022C, YGR026W, YGR082W, YGR107W, YGR172C, YGR191W, YGR204W, YGR260W, YHL005C, YHL046C, YHR025W, YHR026W, YHR123W, YHR126C, YHR143W, YIL011W, YIL015W, YIL018W, YIL157C, YIR041W, YJL016W, YJL121C, YJL133W, YJL138C, YJL191W, YJR018W, YJR047C, YJR077C, YJR119C, YJR121W, YJR123W, YJR143C, YJR145C, YKL060C, YKL147C, YKL148C, YKL157W, YKL164C, YKL169C, YKR033C, YLL041C, YLL064C, YLR041W, YLR044C, YLR056W, YLR058C, YLR081W, YLR089C, YLR110C, YLR177W, YLR264W, YLR284C, YLR304C; YLR340W, YLR354C, YLR372W, YLR388W, YML022W, YMR007W, YMRO11W, YMR015C, YMR092C, YMR101C, YMR156C, YMR205C, YMR215W, YMR261C, YMR323W, YNL069C, YNL135C, YNL195C, YNR076W, YOL039W, YOL073C, YOL086C, YOL120C, YOL156W, YOL161C, YOR002W, YOR009W, YOR010C, YOR085W, YOR108W, YOR128C, YOR129C, YOR142W, YOR161C, YOR176W, YOR230W, YOR298W, YPL004C, YPL036W, YPL048W, YPL057C, YPL059W, YPL061W, YPL135W, YPL179W, YPL218W, YPL220W, YPL246C, YPL272C, YPR063C, YPR080W, YPR181C, YBR290W, YCR010C, YCR091W, YDL107W, YDL129W, YDR066C, YDR529C, YFL026W, YGL018C, YGL059W, YNL144C, YOR003W, YAL037W, YAR023C, YBR003W, YBR020W, YBR044C, YBR091C, YBR185C, YBR282W, YCR015C, YCR038C, YCR043C, YDL119C, YDL146W, YDL220C, YDR057W, YDR123C, YDR125C, YDR222W, YDR225W, YDR277C, YDR286C, YDR347W, YDR408C, YDR438W, YDR479C, YDR483W, YEL039C, YEL057C, YEL073C, YER066W, YER076C, YER084W, YER121W, YER189W, YFL017C, YFL046W, YFR006W, YFR008W, YGL115W, YGL208W, YGL214W, YGL218W, YGR021W, YGR023W, YGR024C, YGR064W, YGR076C, YGR096W, YGR108W, YGR174C, YGR182C, YGR236C, YGR288W, YHL042W, YHR195W, YHR210C, YIL006W, YIL012W, YIL028W, YIL050W, YIL057C, YIL089W, YIL102C, YIL113W, YIL122W, YJL100W, YJL169W, YJL199C, YJR039W, YJR050W, YJR101W, YKL003C, YKL016C, YKL061W, YKL093W, YKL121W, YKL160W, YKL170W, YKL194C, YKR034W, YKR067W, YLR006C, YLR016C, YLR030W, YLR036C, YLR112W, YLR125W, YLR128W, YLR204W, YLR211C, YLR233C, YLR257W, YLR288C, YLR326W, YLR334C, YLR395C, YLR408C, YLR414C, YLR444C, YML050W, YML107C, YML120C, YMR031C, YMR053C, YMR073C, YMR162C, YMR204C, YMR206W, YMR284W, YNL010W, YNL025C, YNL127W, YNL139C, YNL217W, YOL116W, YOL118C, YOR053W, YOR100C, YOR103C, YOR122C, YOR150W, YOR187W, YOR251C, YOR312C, YOR327C, YOR348C, YOR352W, YOR388C, YOR394W, YPL001W, YPL033C, YPL066W, YPL148C, YPL230W, YPL275W, YPL276W, YPR005C, YPR014C, YPR192W, YPR194C, YBR005W, YER025W, YFL027C, YGL080W, YGL205W, YHL028W, YHR185C, YIL076W, YJL166W, YLR046C, YMR035W, YMR238W, YMR252C, YNL192W, YNL202W, YOL108C, YOR385W, YPR165W, YAR033W, YBL038W, YBR009C, YBR010W, YBR151W, YCL067C, YCR096C, YDL137W, YDL192W, YDR073W, YDR086C, YDR224C, YDR377W, YDR378C, YER015W, YGL187C, YHR162W, YJL167W, YJL216C, YKR009C, YLR165C, YMR197C, YNL157W, YOL002C, YOL109W, YOR180C, YPL010W, YPL233W, YBR036C, YDR297W, YGR149W, YGR224W, YNL043C, YPL067C, YPL170W, YCR046C, YDR387C, YFL050C, YGL051W, YHR132C, YIL112W, YJL141C, YKR098C, YLR052W, YLR206W, YML129C, YNL203C, YNR014W, YOL043C, YOL096C, YPR184W, YAL028W, YAL055W, YAR062W, YBL095W, YBL102W, YBR122C, YBR157C, YBR161W, YBR251W, YBR298C, YCR039C, YCR083W, YDL018C, YDL067C, YDL078C, YDL091C, YDL215C, YDL216C, YDR022C, YDR067C, YDR079W, YDR181C, YDR186C, YDR196C, YDR262W, YDR306C, YDR319C, YER188W, YGL004C, YGL035C, YGR036C, YGR062C, YGR120C, YGR131W, YGR141W, YGR167W, YGR287C, YHL024W, YHR080C, YHR097C, YIL077C, YJL046W, YJL070C, YJL096W, YJL113W, YJL146W, YJL180C, YJR019C, YJR049C, YKR058W, YLL005C, YLR078C, YLR151C, YLR271W, YLR295C, YLR351C, YLR375W, YMR023C, YMR025W, YMR135C, YMR210W, YMR267W, YMR278W, YMR293C, YNL073W, YNR037C, YNR040W, YNR072W, YOR028C, YOR316C, YOR328W, YOR363C, YPL039W, YPL040C, YPL099C, YPL107W, YPL134C, YPL138C, and YPL140C.

2. The polynucleotide according to item 1, wherein the yeast gene belongs to a category of unknown yeast genes.
3. The polynucleotide according to item 1, wherein the yeast gene belongs to a category of mitochondria genes.
4. The polynucleotide according to item 1, wherein the yeast gene belongs to a category of DNA repair genes.
5. The polynucleotide according to item 1, wherein the yeast gene belongs to a category of energy genes.
6. The polynucleotide according to item 1, wherein the yeast gene belongs to a category of transport facilitation genes.
7. The polynucleotide according to item 1, wherein the yeast gene belongs to a category of stress protein genes.
8. The polynucleotide according to item 1, wherein the yeast gene belongs to a category of metabolism genes.
9. The polynucleotide according to item 1, wherein the yeast gene belongs to a category of detoxification genes.
10. The polynucleotide according to item 2, wherein the unknown yeast gene is YKL071W.
11. The polynucleotide according to item 2, wherein the unknown yeast gene is YCR102C.
12. The polynucleotide according to item 2, wherein the unknown yeast gene is YOR382W.
13. The polynucleotide according to item 8, wherein the metabolism genes is YLR303W.
14. The polynucleotide according to item 9, wherein the detoxification genes is YLL057C.
15. A vector that comprises the polynucleotide according to any one of items 1 to 14.
16. A cell that is transformed with the polynucleotide according to any one of items 1 to 14 or the vector according to claim 15.
17. A process for detecting a toxic compound in a test material, which comprises:

(1) contacting the test material to the cells according to item 16, and
(2) detecting the expression of mRNA encoding a marker protein.

18. The process according to item 17, wherein the expression of mRNA is confirmed by the expression of a marker protein.
19. The process according to item 17, wherein the expression of mRNA is detected by northern blotting.
20. The process according to item 17, wherein mRNA is amplified by reverse transcription-PCR (RT-PCR), and the expression of mRNA is detected.
21. A process of identifying a toxic compound, which comprises conducting the process according to any one of items 17 to 20 against each of two or more of the cell according to item 16.

22. The process according to item 21, wherein two or more of the cell according to item 16 is selected from a group consisting of YLL057C, YLR303W, YKL071W, YCR102C, and YOR382W.

SEQUENCE LISTING

(110) National Institute of Advanced Industrial Science and Technology
(110) DAIKIN INDUSTRIES; LTD.

(120) Processes for Detecting Toxic Substances

(130) 664319

(140)02760723.3
(141)2002-08-23

(150)JP 2001255379
(151)2001-08-24

(160) 16

(210) 1
(211) 1682
(212) DNA
(213) Saccharomyces cerevisiae

(400) 1

```
cgcaataata ctggaaacat caacgaatgc tataataatg cgcatttact ttgggcaaaa   60
gaactattta aaattacgca ttacgtagtt tattttgaca ttatgatcat aaacatgcgc  120
aaatcctaag atcgtgaatt acctctactg cgagaataca aattataaca tatgactaac  180
gttacacaac attgtattac atttctgact aatttgaagt ctgggagtta attgtagtag  240
cctactaaat agcgcggtta tttataaagc taacattacc atgttacatg acatgctatt  300
tctagcgagg taaaagcatt gctccggctt ttcgaagata agtattcctt caatttccaa  360
atgtcacaac ataaaatttt gatttgcgtt atctctcgtt ctctttaatc actacaatag  420
catcaaccaa gtcaataaaa catgttgtga agagactacg cagacgtgac ggtcatctag  480
caactctcca tcttttgatg cagccatcca cagtttatcg tgagttatgg caaaatccaa  540
aaatgtaccg taccatttcc tctttaatcc tgtattcaat aagcaagttc cccatttcaa  600
ttgatctaca catcactgcg cgttaaagcg cacctaattt attcggtagg aaacagaaat  660
agtctgcaaa gtcattcaag caccttttctt taccaaatga aaggatttaa tagtacctat  720
gaaacaatag ttcgaacttt tgccatttcc cggttttttt ggccagcttg tataaaagtg  780
caccttaccc ttatattggg ctcttattga atgccttccg aagaactgac tattcaaaaa  840
atagaaacaa gtacgtcaat aaaaaatttt gcaattctac gaataattat tcctgtttct  900
ttaacctggt aaaaaaaagt acaaacactt aagcttttttg aaacagcttt attttgcttc  960
```

```
attaaatagc taggataaga aatccctcat ccgaaaggtt ttgtatctaa ctaccctaga 1020
gaacatttgt cctgatcagg ttcatttgga gtttatattt tttagaagct caaagtttgt 1080
tggactcatt accatggaag aaaaaaagaa gatactacga aatattggtt tctcaggtta 1040
aataagggac accattttcc tattaggcta gtcgagctta gttcttctaa tttcttcaga 1200
tcttctataa tttcctatct tctacctgat gtgtgcatga tatatctatg agctcctgat 1260
attgcttgtt ttactttagc ttgcatgact tgcaataatc taatcatata tgttcccgat 1320
taatatactg tgcacaaatt gcaggacata taatttttcc gtggattata tcttcgatta 1380
acgtccgcgg gtctcataaa aagcaaacca acttcgcaat tccctagaaa tacctcaata 1440
gaaagttatt tgtaatgaga ttagtaatga gattagcaat gagattagta atgagattag 1500
taatgagatt agtaatgtga ttagtaatgc atagcggtat aaatggtagt actaataagt 1560
aagatagtat accagttata ataaataggc ggcgatgctt caaaactaat ttttgacgtt 1620
tttaagaata aagcctttac cagtggcata aatcagtaga attctaagca aacaaagtcg 1680
at                                                                1682
```

(210) 2
(211) 1072
(212) DNA
(213) Saccharomyces cerevisiae

(400) 2

```
aggtgcgata gtggggaata agaattggaa atccgcttga taatcaggga aaacttaaag   60
aacggggtgt taaaagtctg tgaagtatag atcaaactcg cactagctag caatactcat  120
gagaacctag gtgagtgtaa agcagtgaac cttctttaga aagcagagaa aaatcctcca  180
agttaacagt cttccatact tactcagtat ggatcaagtt ccacagtttg tccacttctg  240
tagaataaac ttgtagtgct tttccgatga aaagggatag agtctttaaa agacttgatc  300
ttggcatttt tatcctcctt caagcagaat ggctccagta gagttgttct gaacgcttct  360
gggcagtgag cagcattgaa gaaaaagtac tcagcattcc aagctttgtc ttcatataat  420
tataaattaa aactcatagc caccaatttc caggttttgg tcgcaatatg ctgcagaatc  480
aatggcttgt ttcgttacta tactggtccg acggagttta cttactctaa atttgctgaa  540
atagcaaggc cacacgccat acaattaaca atagttcatt cagttgaaag gtttccgaga  600
tagaaagtgc ggtagtagag aagaacaata aacgtatcaa actttgcgca gtcatggaat  660
ttccaggaaa catttttagg ttttcatatg acagcagttc ctgtccgaga gctgtgcctt  720
cttgtctcag ctgactaaga tgtctcggat ccgcccggct ggcgcgcggc tccgtctagt  780
gggataggct ttcaacacat aggaaaccct tcagatgatg agaacacatc gaatcccaga  840
gagtaatatc accaagaata aagcaaccat gatagacaat attactaagt gttcctcaac  900
agatattgag aaaagttctt ataaagcggg caggcttttt tctatttttt tctttttctt  960
tatctagctt aagatccttc taaccatatt ctataaaatg gcaagctgaa tacagattat 1020
caacgacaat tacatataac ctacaaacag gcaagttgca attccagaaa cc         1072
```

(210) 3
(211) 1477
(212) DNA
(213) Saccharomyces cerevisiae

(400) 3

gcttttctcg cttcgttatc acctaaccac taagaacata ctaataatac aaaattttta 60
tccacttttt accaacagat tttcatgaaa tatccatctt ctttccgaat tctgtataaa 120
atgaaagaga gatttttgc tctcaatcca ggctcaaggc tcttgagaac aggtaattgt 180
tgtttagcac gttttcaaca catttatcag aaaacgacag ttaaaaataa gacgagcggc 240
gttatcatcc tgatagtcac taaatgaaaa gcaggtactt ttgaagagca catgtaatat 300
ttgctcaact gataatacct tcattttata cacacttcta attttttttt taaaatcaga 360
tacgcattga tgttcaatgt agcattacca tcacaagaca aaaaatcaga attttacaag 420
caaaagcgct tatctttaag gaccaacata ttagatgaat tcttaagggt tgccaaaacg 480
caagacaaca ggcaaaataa tttcgtttct cagtaccgaa atgacgaaat atactgaggc 540
aaatgcgatc atcatgcctt tgcgccaaga aactcccttg tgaagaactt caaaccgaaa 600
tgggaaaact ttgagttatt gacagggaat acggaggggga agatcacact taaatccgta 660
tgagccgcgc acataatggt attcaaatac acaagaacat tcatgagcta tttttcatcc 720
gtgcaaacga atttactaca attggaccag agggcaccat aactggagac tttgctactg 780
actcaacgtt gatgatgcga gtagtgggtg tactgtgatt tgctcatttt ttttttttata 840
gaaagattcg attaatgaaa gtcacaggag acatttttac atagacattc cgtatatgtt 900
gcgggtatcg cggatgcgga ttagtgatgc ctttaactac atttcataga tttctgtata 960
ccaattgaaa tgagtgaagt aagctcctac agtgaaatat ctgggtgcta ctgacgccaa 1020
gccctacagc gatcggaatg cgggaacgga agttaacggg gcttccagaa cggcggaagc 1080
gaattgaacg aggacggcaa acaaaaacac ccaaaatttc attacttaga atgaccctca 1140
agagcagggt gcaatttatc aagcgatcat tgaactaact aagttcatat cctgtatagg 1200
atttaaaaca atgcacccta agttcaaatg cacccccccct cgccccgcag cggacccttg 1260
aacagagaac tgtttcgagg ttcacccaat tggatcactt gtataatttg taatcgagtt 1320
cggataagat gtatacgaat ctaactgggt gcagtataat tagcatttta tattacctag 1380
caatatatgt ataaaacagg aatgtgtgcg tgcttcaggc agaattttac ggtccttgta 1440
aaaaagtcta tcataaagcc atcacaaaac aataata 1477

(210) 4
(211) 948
(212) DNA
(213) Saccharomyces cerevisiae

(400) 4

gctaacgaac aggatggtat tgatgaagat tccaaaattt tgcgtgctgt cactgaacag 60
gttgccttga gttacaaaga caaaggtgtt tcagcaagaa ttgtcagact cccattctcg 120

gttcatggca agggggacaa ggcttttgta ccaatattaa tgaatattgc caaagctgcc 180
ggaaaatctg gctatgtcgg acaaggcaca aacgcttggg cggctgtaca tcgtttggat 240
acggctcctc tgttcagact tgttttagag aaaggaaaaa caggacaagt gtatcattgc 300
gttggtgaac aaggtatacc attcaaagat attgcgcgtg tgattggaga aattttgaat 360
gttcccgtgg cctctatccc tgttgatgac gcggaaagtc attttggctt cctcacttgt 420
tttgtcacta gagatggccc agtttcaagc gaaggtacca gaaaagagct gggatggcag 480
ccacaacaaa tcggtcttct tgaagatatc cgtgcgaact atagcttaaa ctgacttgaa 540
gcttatatta tgcgtttttt ctaagagcgc catagcatgt aacgtttttt acatactagt 600
tagctttatc tatatagtct actgtgcagc ttaaaatacc caactcatgc gtctcattgg 660
acgagctctt ggcccttgga aaggtgctat attagtatat aggggaatga tgacaaaagc 720
ctcaatgtgg cttgagtcga tttcttattt ggcgccacag ggcacatgga gtttatttat 780
catactacta acataaagaa ggtatgtagg caatacaaca agaatgctgg aaaagttaag 840
gagtcagtaa cagttcttga tgacagaaac atataaaaag gtgtactatt gctgtataat 900
cggccctttc atacttgtac aaacatagca cagtaaccca gttaaaat 948

(210) 5
(211) 968
(212) DNA
(213) Saccharomyces cerevisiae

(400) 5

```
tcgttttcta ctttcttctg ctgctataat aagcacctat gggatctata tagtattttt  60
ataacgatag actttataaa agaaaatacc taagtgaaaa tttggtgaat tttgagataa 120
ttgttgggat tccattttta ataaggcaat aatattaggt atgtagaata tactagaagt 180
tctcctcgag gatttaggaa tccataaaag ggaatctgca attctacaca attctataaa 240
tattattatc atcgttttat atgttaatat tcattgatcc tattacatta tcaatccttg 300
cgtttcagct tccactaatt tagatgacta tttctcatca tttgcgtcat cttctaacac 360
cgtatatgat aatatactag taacgtaaat actagttagt agatgatagt tgatttttat 420
tccaacacta agaaataatt tcgccatttc ttgaatgtat ttaaagatat ttaatgctat 480
aatagacatt taaatccaat tcttccaaca tacaatggga gtttggccga gtggtttaag 540
gcgtcagatt taggtggatt taacctctaa aatctctgat atcttcggat gcaagggttc 600
gaatccctta gctctcatta tttttgctt tttctcttga ggtcacatga tcgcaaaatg 660
gcaaatggca cgtgaagctg tcgatattgg ggaactgtgg tggttggcaa atgactaatt 720
aagttagtca aggcgccatc ctcatgaaaa ctgtgtaaca taataaccga agtgtcgaaa 780
aggtggcacc ttgtccaatt gaacacgctc gatgaaaaaa ataagatata tataaggtta 840
agtaaagcgt ctgttagaaa ggaagttttt ccttttttctt gctctcttgt cttttcatct 900
actatttcct tcgtgtaata cagggtcgtc agatacatag atacaattct attacccccca 960
tccataca                                                           968
```

(210) 6

(211) 720

(212) DNA

(213) Aequorea victoria

(400) 6

```
atggctagca aaggagaaga actcttcact ggagttgtcc caattcttgt tgaattagat  60
ggtgatgtta acggccacaa gttctctgtc agtggagagg gtgaaggtga tgcaacatac 120
ggaaaactta ccctgaagtt catctgcact actggcaaac tgcctgttcc atggccaaca 180
ctagtcacta ctctgtgcta tggtgttcaa tgcttttcaa gatacccgga tcatatgaaa 240
cggcatgact ttttcaagag tgccatgccc gaaggttatg tacaggaaag gaccatcttc 300
ttcaaagatg acggcaacta caagacacgt gctgaagtca agtttgaagg tgataccctt 360
gttaatagaa tcgagttaaa aggtattgac ttcaaggaag atggcaacat tctgggacac 420
aaattggaat acaactataa ctcacacaat gtatacatca tggcagacaa acaaaagaat 480
ggaatcaaag tgaacttcaa gacccgccac aacattgaag atggaagcgt tcaactagca 540
gaccattatc aacaaaatac tccaattggc gatggccctg tccttttacc agacaaccat 600
tacctgtcca cacaatctgc cctttcgaaa gatcccaacg aaaagagaga ccacatggtc 660
cttcttgagt ttgtaacagc tgctgggatt acacatggca tggatgaact gtacaactga 720
```

(210) 7
(211) 23
(212) DNA
(213) Saccharomyces cerevisiae

(400) 7
cgcaataata ctggaaacat caa

(210) 8
(211) 22
(212) DNA
(213) Saccharomyces cerevisiae

(400) 8
atcgactttg tttgcttaga at

(210) 9
(211) 23
(212) DNA
(213) Saccharomyces cerevisiae

(400) 9
aggtgcgata gtggggaata aga

(210) 10
(211) 22
(212) DNA
(213) Saccharomyces cerevisiae

(400) 10
ggtttctgga attgcaactt gc

(210) 11
(211) 23
(212) DNA
(213) Saccharomyces cerevisiae

(400) 11
gcttttctcg cttcgttatc acc

(210) 12
(211) 22
(212) DNA
(213) Saccharomyces cerevisiae

(400) 12
tattattgtt ttgtgatggc tt

(210) 13
(211) 23
(212) DNA
(213) Saccharomyces cerevisiae

(400) 13
gctaacgaac aggatggtat tga

(210) 14
(211) 22

(212) DNA
(213) Saccharomyces cerevisiae

(400) 14
attttaactg ggttactgtg ct

(210) 15
(211) 23
(212) DNA
(213) Saccharomyces cerevisiae

(400) 15
tcgttttcta ctttcttctg ctg

(210) 16
(211) 22
(212) DNA
(213) Saccharomyces cerevisiae

(400) 16
tgtatggatg ggggtaatag aa

## Claims

**1.** A process for detecting a toxic compound in a test material, wherein said toxic compound is selected from the group consisting of 2,5-dichlorophenol, 4-nitroquinolin-N-oxide, p-nonylphenol, 2,4,5-trichlorophenol, Trp-P-2(acetate), malathon, manganese ethylenebis (dithiocarbamate), nickel (II) chloride, potassium bichromate, phenol and dimethylsulfoxide, wherein said process comprises:

(1) contacting the test material to a cell that is transformed with a polynucleotide or with a vector comprising said polynucleotide, whereby said polynucleotide comprises a polynucleotide sequence operably linked to a polynucleotide encoding a marker protein, and wherein said polynucleotide sequence comprises the promoter of the yeast gene YOR382W, wherein YOR382W is defined by SEQ ID NO:3, and
(2) detecting the expression of mRNA encoding a marker protein.

**2.** The process according to claim 1, wherein the expression of mRNA is confirmed by the expression of a marker protein.

**3.** The process according to claim 1, wherein the expression of mRNA is detected by northern blotting

**4.** The process according to claim 1, wherein mRNA is amplified by reverse transcription-PCR (RT-PCR), and the expression of mRNA is detected.

## Patentansprüche

**1.** Verfahren zum Detektieren einer toxischen Verbindung in einem Testmaterial, wobei die toxische Verbindung ausgewählt ist aus der Gruppe, bestehend aus 2,5-Dichlorphenol, 4-Nitrochinolin-N-oxid, p-Nonylphenol, 2,4,5-Trichlorphenol, Trp-P-2(acetat), Malathon, Manganethylenbis(dithiocarbamat), Nickel-(II)-chlorid, Kaliumbichromat, Phenol und Dimethylsulfoxid, wobei das Verfahren folgendes umfasst:

(1) Kontaktieren des Testmaterials mit einer Zelle, die mit einem Polynukleotid oder mit einem Vektor, der das Polynukleotid umfasst, transformiert ist, wobei das Polynukleotid eine Polynukleotidsequenz umfasst, die funktionell mit einem Polynukleotid verknüpft ist, das für ein Markerprotein kodiert, und wobei die Polynukleotidsequenz den Promotor des Hefegens YOR382W umfasst, wobei YOR382W durch SEQ ID Nr.: 3 definiert ist, und
(2) Detektieren der Expression der mRNA, die für ein Markerprotein kodiert.

**2.** Verfahren gemäß Anspruch 1, wobei die Expression der mRNA durch die Expression eines Markerproteins bestätigt wird.

3. Verfahren gemäß Anspruch 1, wobei die Expression der mRNA durch Northern-Blott detektiert wird.

4. Verfahren gemäß Anspruch 1, wobei mRNA durch reverse Transkription-PCR (RT-PCR) amplifiziert und die Expression der mRNA detektiert wird.

## Revendications

1. Procédé de détection d'un composé toxique dans un matériel à tester, où ledit composé toxique est sélectionné dans le groupe constitué du 2,5-dichlorophénol, du N-oxyde de 4-nitroquinoléine, du p-nonylphénol, du 2,4,5-trichlorophénol, du Trp-P-2 (acétate), du malathon, du manganèse éthylènebis (dithiocarbamate), du chlorure de nickel (II), du bichromate de potassium, du phénol et du diméthylsulfoxyde, où ledit procédé comprend :

   (1) la mise en contact du matériel à tester avec une cellule transformée par un polynucléotide ou par un vecteur comprenant ledit polynucléotide, ledit polynucléotide comprenant une séquence polynucléotidique liée fonctionnellement à un polynucléotide codant une protéine marqueuse et où ladite séquence polynucléotidique comprend le promoteur du gène de levure YOR382W, où YOR382W est défini par SEQ ID NO:3, et
   (2) la détection de l'expression de l'ARNm codant une protéine marqueuse.

2. Procédé selon la revendication 1, où l'expression de l'ARNm est confirmée par l'expression d'une protéine marqueuse.

3. Procédé selon la revendication 1, où l'expression de l'ARNm est détectée par buvardage de Northern.

4. Procédé selon la revendication 1, où l'ARNm est amplifié par PCR à transcription inverse (RT-PCR) et l'expression de l'ARNm est détectée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6123705 A
- JP 2000206087 A
- JP 2001255379 B
- JP 151 A


### Non-patent literature cited in the description


- Principles of Gene Manipulation. **R. W. Old ; S. B. Primrose.** Principles of Gene Manipulation. BAIFUKAN CO., LTD, 2000, 138-165234-263
- **Heim, R. ; Cubitt, A. B. ; Tsien, R. Y.** *Nature,* 1995, vol. 373, 663-664
- **Heim, R. ; Prasher DC. ; Tsien, R. Y.** *Proc. Natl. Acad. Sci.,* 1994, vol. 91, 12501-12504
- **Warg, S. ; Hazerigg, T.** *Nature,* 1994, vol. 639, 400-403
- **Youvan, D.C. ; Michel-Beyerle, M.E.** *Nature Biotechnology,* 1996, vol. 14, 1219-1220
- **Chalfie, M. ; Tu, Y. ; Euskirchen, G. ; Ward, W. W. ; Prasher, D.C.** *Science,* 1994, vol. 263, 802-805
- **Canestro C ; Albalat R ; Escriva H ; Gonzalez-Duarte R.** Endogenous beta-galactosidase activity in amphioxus: a useful histochemical marker for the digestive system. *Dev Genes Evol,* 21 March 2001, vol. 1 (3), 154-6
- *Arch Toxicol,* June 2002, vol. 76 (5-6), 257-61
- *J Recept Signal Transduct Res,* February 2001, vol. 21 (1), 71-84
- Lithium acetate yeast transformation. **Kaiser C ; Michaelis S ; Mitchell A.** Methods in Yeast Genetics, A Cold Spring Harbor Laboratory Course Manual. Cold Spring Harbor Laboratory Press, 1994, 133-134
- **OGATA Nobukuni ; NOJIMA Hiroshi.** Genetic Engineering Keywords Book. Yodosha, co.jp, 2000, 299-301
- Cell Technology. **NAKABEPPU Yusaku et al.** Tips series, Modified PCR Tips. Shujunsha Co.Ltd, 1999, 25-43
- **R. W. Old ; S. B. Primrose.** Principles of Gene Manipulation. BAIFUKAN CO., LTD, 2000, 138-165234-263
- **R. W. Old ; S. B. Primrose.** Principles of Gene Manipulation. BAIFUKAN CO., LTD, 2000, 234-263